(19) Europäisches Patentamt
European Patent Office
Office européen des brevets

(11) EP 2 441 773 A1

(12) **EUROPEAN PATENT APPLICATION**

(43) Date of publication:
**18.04.2012 Bulletin 2012/16**

(51) Int Cl.:
***C07K 14/415*** *(2006.01)* ***C12N 15/82*** *(2006.01)*

(21) Application number: **11165505.6**

(22) Date of filing: **10.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority:
**22.08.2008 US 91008 P**
**25.08.2008 US 91517 P**
**22.08.2008 US 91067 P**
**21.08.2008 US 90623 P**
**22.08.2008 EP 08162831**
**22.08.2008 EP 08162817**
**22.08.2008 EP 08162847**
**20.08.2008 EP 08162687**

(62) Document number(s) of the earlier application(s) in accordance with Art. 76 EPC:
**09781667.2 / 2 315 774**

(71) Applicant: **BASF Plant Science GmbH**
**67056 Ludwigshafen (DE)**

(72) Inventors:
- **Hatzfeld, Yves**
  **F-59000, Lille (FR)**
- **Reuzeau, Christophe**
  **24350, La Chapelle Gonaguet (FR)**
- **Frankard, Valerie**
  **1410, Waterloo (BE)**
- **Sanz Molinero, Ana Isabel**
  **28035, Madrid (ES)**

(74) Representative: **Saelens, Claire**
**CropDesign N.V.**
**Technologiepark 3**
**9052 Zwijnaarde (BE)**

Remarks:
This application was filed on 10-05-2011 as a divisional application to the application mentioned under INID code 62.

(54) **Plants having enhanced yield-related traits and a method for making the same**

(57) The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding a bHLH9 (basic-Helix-Loop-Helix group 9) polypeptide. The present invention also concerns plants having modulated expression of a nucleic acid encoding a bHLH9 polypeptide, which plants have enhanced yield-related traits relative to control plants. The invention also provides hitherto unknown BHLH9-encoding nucleic acids and constructs comprising the same, useful in performing the methods of the invention.

Furthermore, the present invention relates generally to the field of molecular biology and concerns a method for improving various plant growth characteristics by modulating expression in a plant of a nucleic acid encoding an IMB1 (Imbibition-inducible 1) polypeptide. The present invention also concerns plants having modulated expression of a nucleic acid encoding an IMB1 polypeptide, which plants have improved growth characteristics relative to corresponding wild type plants or other control plants. The invention also provides constructs useful in the methods of the invention.

Yet furthermore, the present invention relates generally to the field of molecular biology and concerns a method for improving various plant growth characteristics by modulating expression in a plant of a nucleic acid encoding a PCD-like (Pterin-4-alpha-carbinolamine dehydratase-like). The present invention also concerns plants having modulated expression of a nucleic acid encoding a PCD-like, which plants have improved growth characteristics relative to corresponding wild type plants or other control plants. The invention also provides constructs useful in the methods of the invention.

Furthermore the present invention relates generally to the field of molecular biology and concerns a method for increasing various plant yield-related traits by increasing expression in a plant of a nucleic acid sequence encoding a pseudo response regulator type 2 (PRR2) polypeptide. The present invention also concerns plants having increased expression of a nucleic acid sequence encoding a PRR2 polypeptide, which plants have in-

EP 2 441 773 A1

creased yield-related traits relative to control plants. The invention additionally relates to nucleic acid sequences, nucleic acid constructs, vectors and plants containing said nucleic acid sequences.

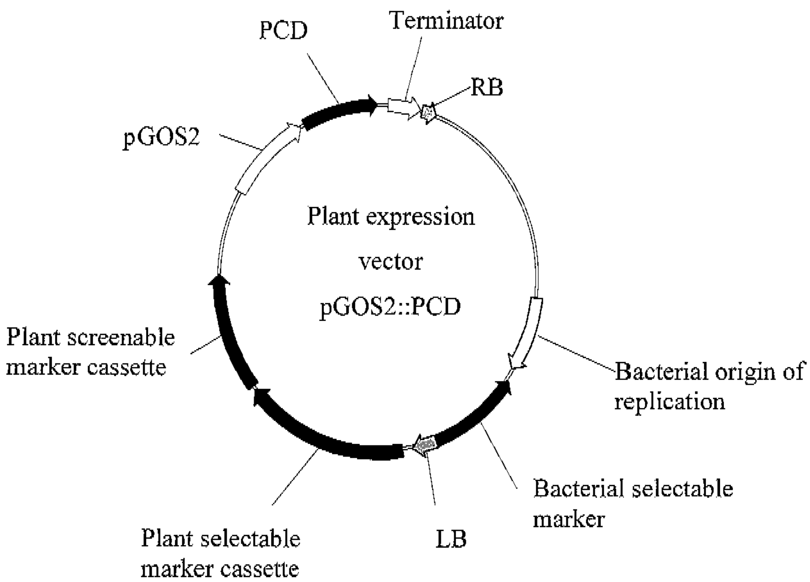


FIGURE 10

SEQUENCE LISTING

<110> BASF Plant Science GmbH

<120> Plants having enhanced yield-related traits and a method for making the same

<130> PF61130_PCT

<160> 480

<170> PatentIn version 3.3

<210> 1
<211> 1917
<212> DNA
<213> Oryza sativa

<400> 1
```
tcacctcctc cctacctctc gccattatta aatcctctcc tctcctctct tctccacatc     60
cacaagaaat ttcgtagcgg aatcacgagc cctcgtctcg atcgtagctt gagcttgagc    120
tgagttgctg gtttggtgtg tggagagatc agtggggtgg gtgggggtgg gtgatgtacg    180
gcgcgccggt gtccaaggac ctgagcctgc agccagccgg ggtgcggacg ccgccgcaga    240
tgagctcgcc gggcttgctg cggtacaggt cagcgccgag cacgctgctc ggggaggtct    300
gcggcgactt cgtcctcccc ggtggtggtg gtggtggtgg gcagctgcag ctgcagctgc    360
agcagcagcg gcctgggagc ccggaccacg ccgcggacac cgtcctcgcg cgcttcctcg    420
ccggccatgg cggccacgac aacaagcctc cccgccccgc cgcccacttc gccccaccgg    480
aagactccat ggcctcgcat cagcagcagc tcatgtacca gtcgcatcag cagcagcagc    540
agatggcttc cgccatggag ggactctacc gcaccgtcag ctccggcggc acggagtcca    600
ccgccgccgc cgccggcaac agcctcctcc ggcagagcag ctcccccgcc ggcttcctca    660
accatcttac catggacaac ggatacggga atatgctaag agcgggcatg ggcggcggcg    720
gcggtggcgg cgacccgcgg ctcaaggggc agctcagctt ctcgtcgcgg caggggtcgg    780
tgatgtccca gatctcggag atgggcagcg aggacgagga gctcgccggc ggcggcggca    840
gcccggaggc tggcagcaac ggcggtggcg ccgcacgcgg cggctacggc ggcgggtacg    900
cgatggggtc ctccgcctgg gaggagccct cgccgccggc gacgtcgctc ctcccggaca    960
gcagcctccc gtccaagcgc ccccgcgacg acctgccgcg gcagctcagc ctcccggcgg   1020
cgtccaagaa cagcagcaag ccccccctcct ccgcctccgc cgccgcgtcg ccggagatgg   1080
ccgccatcga gaagttcctc cagttccagg acgccgtccc ctgcaagatc cgcgccaagc   1140
gcggctgcgc cacccacccc cgcagcatcg ccgagcgggt gaggaggacg cggatcagcg   1200
agcggatacg gaagctgcag gagctcgtcc ccaacatgga aaagcaaacc aacactgctg   1260
acatgttgga tcttgccgtg gactacatca aggagctcca gaagcaggtc aaggtgttaa   1320
acgacagccg ttccagctgc acctgctcgg cgagcaagca gaagcacttc gccggctaaa   1380
tcaatcaagt caatcaatcg ccatgaccgg tgcagcagtg cgcaacacac gaacggccgc   1440
aaaaggcatg cagcagaagc atctgcaccg gcagtttttc taagcttcgt ttccttcttt   1500
gctgatgaat tcgctggtct atctggtaaa ttgaacatga gggcattgga ttttggagca   1560
gtggcatgca ttcttcagag ttcacaaaga tgatgggttg gggtcccaca tggtagtggt   1620
agactactgt agctgcaaag cttgattaat taattagcct gttcttttga taatgaagga   1680
gaggagagta actcacaagt gtatagatgg ttgtctcatc tctgtttgtc atagtacaca   1740
ccaagtgcaa gttgcagctg tgtctgcttg caagaatgca tgcccatgta cattgcatcc   1800
ccctttgctt gacaaatgac acatccagac agcctccata tcctgtttcc tctcgttgct   1860
acaccctgtt tttgtgaagt acatgcatcg caacaatgtt cacgaataat cctggtc       1917
```

<210> 2
<211> 387
<212> PRT
<213> Oryza sativa

<400> 2
```
Met Asn Arg Met Thr Ala Pro His Gly Gly Met Pro Pro Pro Pro Met
```

```
1               5                   10                  15
Pro Ala Ala Gly Gly Leu Ala Arg Tyr Gly Ser Ala Pro Gly Ser Leu
            20                  25                  30
Leu Ala Ser Ile Ala Asp Ser Val Ile Arg Gly Arg Gly Val Gly Val
        35                  40                  45
Val Asp Gln Leu His His His Gln His Gln His Gln Leu Pro Pro Pro
    50                  55                  60
Pro Pro Pro Gln Gln Gln Gln Met Val Gly Arg Tyr Phe Ser Ala Glu
65                  70                  75                  80
Ser Ser Gly Leu Thr Ser Cys Glu Ser Ser Cys Arg Thr Thr Thr Thr
                85                  90                  95
Thr Ser Thr Ala Ala Ala Ala Asp Val Gly Arg His Pro Leu Glu Arg
            100                 105                 110
Ala Tyr Gly Gly Ser Gly Glu Ile His Val Asp Ala Ser Ser Ala Ala
        115                 120                 125
Val Pro Leu Phe Arg His Ser Ser Ser Pro Ala Gly Leu Leu Ser Arg
    130                 135                 140
Leu Met Ala Asp Pro His Gly Asn Gly Met Ala Ala Thr Arg Gly Met
145                 150                 155                 160
Gly Gly Tyr Ser Gly Gly Gly Gly Asp Ala Gly Ala Met Ala His Arg
                165                 170                 175
Arg Leu Ser Ser Gln Trp Ser Phe Ser Arg Gln Asp Leu Pro Gln Ile
            180                 185                 190
Ser Glu Met Gly Gly Leu Ile Pro Asp Ile Gly Glu Ser Ile Val Thr
        195                 200                 205
Gly Gly Gly Gly Asn Ser Ser Ser Asp Gly Ala Gly His Gly Ala Gln
    210                 215                 220
Ser Ser Ser Phe Leu Ser Ser Arg Asn Phe Ser Met Ser Ser Trp Asp
225                 230                 235                 240
Asp Thr Asn Ser Ile Met Phe Ser Pro Pro Ser Ser Ser Lys Lys Ala
                245                 250                 255
Arg Val Ala Ala Ala Ala Ala Gly Asp His Gly Asp Asp Met Val Ser
            260                 265                 270
Ser Phe Ser Asn Ile Asp Ser Gln Phe Gly Leu Ser Lys Gln Ser Ser
        275                 280                 285
Leu Glu Met Ala Gly Met Asp Asp Phe Leu Gln Leu Gln Pro Asp Ser
    290                 295                 300
Val Ala Cys Arg Ala Arg Ala Lys Arg Gly Cys Ala Thr His Pro Arg
305                 310                 315                 320
Ser Ile Ala Glu Arg Glu Arg Arg Thr Arg Ile Ser Lys Arg Leu Lys
                325                 330                 335
Lys Leu Gln Asp Leu Val Pro Asn Met Asp Lys Gln Thr Asn Thr Ser
            340                 345                 350
Asp Met Leu Asp Ile Ala Val Thr Tyr Ile Lys Glu Leu Gln Gly Gln
        355                 360                 365
Val Glu Lys Leu Lys His Asp Gln Ala Asn Cys Thr Cys Ser Gly Lys
    370                 375                 380
His Asp Cys
385

<210>  3
<211>  1679
<212>  DNA
<213>  Arabidopsis thaliana

<400>  3
ttcatcatca ccttcatctc tctcttcctc tcatcagact tcaccgttct tctttctctc      60
ttcttcatct tcagacgaaa cctcctttac ttccccttca aggttttata tttcccaaat     120
cctacacctg ctcacatctc aagctcaagt tttgttttcc cgggaaaagt gacacaaaca     180
```

```
agaacttaac aagggagaag aaaaagctta tattcatatc aacggttgac gttggaaagc    240
tattaagatt tggttttcta caaatttgtt cttcctgaaa cgtcacgaga cagagcttac    300
aagaagagaa aacagaggaa atttcgttgc attttttta catattgatt cgattaatgg    360
attcaaataa tcatctctac gacccgaatc ccaccgggtc gggtcttctt cgttttagat    420
cagctccgag ctctgttctc gccgcttttg ttgacgacga caagattggt ttcgactccg    480
ataggttgct ttcaagattc gtgacctcta atggcgttaa cggagatctg ggttcaccta    540
aattcgagga taagtctccg gtttcgttaa cgaacacctc tgtttcatac gccgccactc    600
tgccgccacc gccgcagctt gagccgtcga gttttctggg tttgccgccg cattacccga    660
ggcagagtaa aggatatg aactcggttg gtttggatca gtttctcggt atcaataatc    720
atcacaccaa accagttgaa tctaatcttc tccgtcaaag cagctctcca gccggaatgt    780
ttactaatct ctctgaccaa aacggttatg gttcaatgag gaatttgatg aattacgaag    840
aagatgaaga gagtccatct aattccaatg gattaagacg ccattgcagt ctctcttcaa    900
ggccaccttc ttcacttgga atgctttctc aaatacctga aatcgcaccc gaaactaatt    960
ttccatatag ccattggaat gatccatcca gctttattga taacttatcc tcacttaaaa   1020
gagaagccga ggacgatgga aaattgtttc tcggagctca gaacggagag tccgggaatc   1080
gtatgcagtt actgtcgcat catttgagcc taccaaagtc atcatcgaca gcctcggaca   1140
tggtttcagt ggataagtat cttcagctac aagattctgt tccttgtaaa atcagagcca   1200
aacgtggttg cgctacacat cctcgaagca tcgctgaacg ggtaagaaga acgcggataa   1260
gcgagcgaat gaggaagtta caagagcttg ttcctaacat ggacaagcaa accaacactt   1320
cggatatgtt ggatttagct gtggattaca tcaaagattt acaaagacag tataagattt   1380
taaacgacaa cagagctaac tgtaagtgta tgaacaagga gaagaagtca atatagggcg   1440
caacaaagtg tgtagtagat aggactaaaa agcagggaga aggacaagaa agaaacaatg   1500
tcatgtctga atatttttta gccgaaacag accaaattgt ctatgtaagc tctcgagaaa   1560
agcatctgct tccaacaaaa ttctaagtaa taaaatagta ctcgatttgt tcttatttca   1620
ttattacaat gcagaatcta ctaatcaaat gttgtactac aaaaggtaag atcaactac    1679
```

<210> 4
<211> 359
<212> PRT
<213> Arabidopsis thaliana

<400> 4

```
Met Asp Ser Asn Asn His Leu Tyr Asp Pro Asn Pro Thr Gly Ser Gly
1               5                   10                  15
Leu Leu Arg Phe Arg Ser Ala Pro Ser Ser Val Leu Ala Ala Phe Val
            20                  25                  30
Asp Asp Asp Lys Ile Gly Phe Asp Ser Asp Arg Leu Leu Ser Arg Phe
        35                  40                  45
Val Thr Ser Asn Gly Val Asn Gly Asp Leu Gly Ser Pro Lys Phe Glu
    50                  55                  60
Asp Lys Ser Pro Val Ser Leu Thr Asn Thr Ser Val Ser Tyr Ala Ala
65                  70                  75                  80
Thr Leu Pro Pro Pro Pro Gln Leu Glu Pro Ser Ser Phe Leu Gly Leu
                85                  90                  95
Pro Pro His Tyr Pro Arg Gln Ser Lys Gly Ile Met Asn Ser Val Gly
            100                 105                 110
Leu Asp Gln Phe Leu Gly Ile Asn Asn His His Thr Lys Pro Val Glu
        115                 120                 125
Ser Asn Leu Leu Arg Gln Ser Ser Ser Pro Ala Gly Met Phe Thr Asn
    130                 135                 140
Leu Ser Asp Gln Asn Gly Tyr Gly Ser Met Arg Asn Leu Met Asn Tyr
145                 150                 155                 160
Glu Glu Asp Glu Glu Ser Pro Ser Asn Ser Asn Gly Leu Arg Arg His
                165                 170                 175
Cys Ser Leu Ser Ser Arg Pro Pro Ser Ser Leu Gly Met Leu Ser Gln
            180                 185                 190
Ile Pro Glu Ile Ala Pro Glu Thr Asn Phe Pro Tyr Ser His Trp Asn
        195                 200                 205
Asp Pro Ser Ser Phe Ile Asp Asn Leu Ser Ser Leu Lys Arg Glu Ala
```

```
    210                 215                 220
Glu Asp Asp Gly Lys Leu Phe Leu Gly Ala Gln Asn Gly Glu Ser Gly
225                 230                 235                 240
Asn Arg Met Gln Leu Leu Ser His His Leu Ser Leu Pro Lys Ser Ser
                245                 250                 255
Ser Thr Ala Ser Asp Met Val Ser Val Asp Lys Tyr Leu Gln Leu Gln
            260                 265                 270
Asp Ser Val Pro Cys Lys Ile Arg Ala Lys Arg Gly Cys Ala Thr His
        275                 280                 285
Pro Arg Ser Ile Ala Glu Arg Val Arg Arg Thr Arg Ile Ser Glu Arg
    290                 295                 300
Met Arg Lys Leu Gln Glu Leu Val Pro Asn Met Asp Lys Gln Thr Asn
305                 310                 315                 320
Thr Ser Asp Met Leu Asp Leu Ala Val Asp Tyr Ile Lys Asp Leu Gln
                325                 330                 335
Arg Gln Tyr Lys Ile Leu Asn Asp Asn Arg Ala Asn Cys Lys Cys Met
            340                 345                 350
Asn Lys Glu Lys Lys Ser Ile
        355
```

<210> 5
<211> 1625
<212> DNA
<213> Arabidopsis thaliana

<400> 5

```
aagtttctct cacgttctct tttttaattt taatttctcg ccggaaacaa tctcatctcc      60
cggcgaacga aacttccggt gtggtactgc aaacggagaa aaaaataacc aaagaagaga     120
gaaactcaaa agctactaag atggaatcag aattccagca acatcacttc cttctccacg     180
atcatcaaca ccagagacca agaaactcag gattgattcg ttaccaatca gcaccaagtt     240
cgtacttttc gagtttcggt gaatcaatcg aagagttttt agatcgaccc acaagtcctg     300
aaactgagcg aatcttatct ggctttttac aaaccaccga cacaagcgac aacgttgata     360
gtttccttca ccatactttt aacagtgatg gaactgagaa gaaacctccg gaagttaaaa     420
cagaggacga agatgctgaa attccggtga ctgcgacggc gacggcgatg gaggttgttg     480
tttccggtga tggtgaaatc tcagtgaatc ctgaagtatc gattgggtat gtggcttcgg     540
tttcgaggaa taagagacca agagagaaag atgatcggac tccggtgaat aatctagctc     600
gtcataatag ttcaccggcc ggattatttt catccattga tgttgaaaca gcttatgcag     660
ctgtaatgaa aagtatggga ggttttggag gaagtaatgt gatgagtaca agcaatactg     720
aagcttcgtc tcttactcct agaagcaagt tacttcctcc tacttctaga gcgatgagtc     780
cgatctctga ggttgatgtt aaacccggtt tctcgtctag attgcctcct cggacgcttt     840
ccggtgggtt taatcgttct tttgggaatg aagggtctgc ttcttccaag cttacagctc     900
ttgctaggac ccaatctgga ggtctagatc aatacaaaac caaggatgag gattcagcaa     960
gtagacgtcc tcctttggca catcacatga gtttgcccaa gtctttatca gatattgaac    1020
agttactgtc agattctatc ccatgtaaga tcagagccaa gcggggttgt gcaactcatc    1080
ctcgaagcat agccgagagg gtgagaagaa ccaagatcag tgaaagaatg aggaagctgc    1140
aagaccttgt tccaaacatg gacacgcaaa caaacacagc agacatgttg gatcttgcgg    1200
ttcaatacat caaggacctg caagaacaag tgaaggcgct cgaagagagt cgggcaagat    1260
gtagatgctc tagtgcgtga aactagaagt gggagtatgc gcgagtgcta gccagggagg    1320
gagttgtgca tagaagtatc ggttcggcct ttggagaaaa gtcgaagata gcaaagtaga    1380
gaagagatca tgaacaaagc taaatttggt ggtggtggtg aaaagggttt ttgtaaagtt    1440
ggaacctttt tttggtaggg aagaaagtag caaggttgtg taatggtccg aactccaatg    1500
ctattgtatg ttcttacatc caaaaaaaag aaaagcagaa gagaagtgat gtacaatgag    1560
aagcttattt tattgtaact caagaataat tcaggtaatt agtttatttt cttttgttag    1620
aaact                                                                1625
```

<210> 6
<211> 379
<212> PRT
<213> Arabidopsis thaliana

<400> 6

```
Met Glu Ser Glu Phe Gln Gln His His Phe Leu Leu His Asp His Gln
1               5                   10                  15
His Gln Arg Pro Arg Asn Ser Gly Leu Ile Arg Tyr Gln Ser Ala Pro
            20                  25                  30
Ser Ser Tyr Phe Ser Ser Phe Gly Glu Ser Ile Glu Glu Phe Leu Asp
        35                  40                  45
Arg Pro Thr Ser Pro Glu Thr Glu Arg Ile Leu Ser Gly Phe Leu Gln
    50                  55                  60
Thr Thr Asp Thr Ser Asp Asn Val Asp Ser Phe Leu His His Thr Phe
65                  70                  75                  80
Asn Ser Asp Gly Thr Glu Lys Lys Pro Pro Glu Val Lys Thr Glu Asp
                85                  90                  95
Glu Asp Ala Glu Ile Pro Val Thr Ala Thr Ala Thr Ala Met Glu Val
            100                 105                 110
Val Val Ser Gly Asp Gly Glu Ile Ser Val Asn Pro Glu Val Ser Ile
        115                 120                 125
Gly Tyr Val Ala Ser Val Ser Arg Asn Lys Arg Pro Arg Glu Lys Asp
    130                 135                 140
Asp Arg Thr Pro Val Asn Asn Leu Ala Arg His Asn Ser Ser Pro Ala
145                 150                 155                 160
Gly Leu Phe Ser Ser Ile Asp Val Glu Thr Ala Tyr Ala Ala Val Met
                165                 170                 175
Lys Ser Met Gly Gly Phe Gly Gly Ser Asn Val Met Ser Thr Ser Asn
            180                 185                 190
Thr Glu Ala Ser Ser Leu Thr Pro Arg Ser Lys Leu Leu Pro Pro Thr
        195                 200                 205
Ser Arg Ala Met Ser Pro Ile Ser Glu Val Asp Val Lys Pro Gly Phe
    210                 215                 220
Ser Ser Arg Leu Pro Pro Arg Thr Leu Ser Gly Gly Phe Asn Arg Ser
225                 230                 235                 240
Phe Gly Asn Glu Gly Ser Ala Ser Ser Lys Leu Thr Ala Leu Ala Arg
                245                 250                 255
Thr Gln Ser Gly Gly Leu Asp Gln Tyr Lys Thr Lys Asp Glu Asp Ser
            260                 265                 270
Ala Ser Arg Arg Pro Pro Leu Ala His His Met Ser Leu Pro Lys Ser
        275                 280                 285
Leu Ser Asp Ile Glu Gln Leu Leu Ser Asp Ser Ile Pro Cys Lys Ile
    290                 295                 300
Arg Ala Lys Arg Gly Cys Ala Thr His Pro Arg Ser Ile Ala Glu Arg
305                 310                 315                 320
Val Arg Arg Thr Lys Ile Ser Glu Arg Met Arg Lys Leu Gln Asp Leu
                325                 330                 335
Val Pro Asn Met Asp Thr Gln Thr Asn Thr Ala Asp Met Leu Asp Leu
            340                 345                 350
Ala Val Gln Tyr Ile Lys Asp Leu Gln Glu Gln Val Lys Ala Leu Glu
        355                 360                 365
Glu Ser Arg Ala Arg Cys Arg Cys Ser Ser Ala
    370                 375
```

<210> 7
<211> 1125
<212> DNA
<213> Arabidopsis thaliana

<400> 7

```
tgtattaatt aaagaaaaaa ataaaacaga gcaagaaaca aaaaaaaaaa tgtaaagaaa      60
gagaaaaaaa gctttcgtag tgtctattga aaccagagaa aagccaaagg ggatgcaacc     120
```

```
aacatccgtc ggtagtagcg gcggtggtga cgacggagga ggcagaggag gaggaggagg    180
gctaagtaga agtggactat ctcggatccg ttcagctcca gcgacttggc ttgaagcttt    240
acttgaggaa gatgaagaag agtctttgaa acctaatctt ggtctcaccg atttgcttac    300
cgggaactcg aacgatttac cgacaagtcg cggctcgttc gagttcccga ttcctgttga    360
gcaagggttg tatcaacaag gtgggtttca ccgacagaat agtactccgg cggattttct    420
tagtggttct gatggattta tccaaagctt tgggattcag gcgaattacg attacttatc    480
ggggaatatc gatgtttctc cgggaagtaa gcggtctaga gaaatggaag cactcttctc    540
ttctcctgag tttacttctc aaatgaaagg agagcaaagc agcggtcaag ttcctaccgg    600
agtatcaagc atgtcggata tgaacatgga gaaccttatg gaggactctg ttgcttttag    660
ggttcgggct aaacgtggtt gcgcaactca tccccgcagc attgccgaga gggtacgaag    720
gacgcggatt agtgatcgga taaggaagct acaagagctt gtacctaaca tggacaagca    780
aaccaacact gcagacatgt tagaagaagc agtagaatac gtgaaagttc ttcaaaggca    840
gatccaggag ttaacagaag aacagaagag gtgcacatgc atacctaagg aagaacaata    900
aggtttgctc ctgatttgtt ttatatttgc ttaacggcaa tgatctgatc gaaaaattcg    960
aaagatgatc ttagcttgaa tttagatgga tgtcatgttg aaaagtatat tatttgataa   1020
atggatgtag gtgtaatata aaatttttgt acaataatga agaaagttaa aaagaattaa   1080
tgaaaacata tattctttat gatattagtg aaatgacttt gattt                   1125
```

<210> 8
<211> 262
<212> PRT
<213> Arabidopsis thaliana

<400> 8

```
Met Gln Pro Thr Ser Val Gly Ser Ser Gly Gly Gly Asp Asp Gly Gly
1               5                   10                  15
Gly Arg Gly Gly Gly Gly Gly Leu Ser Arg Ser Gly Leu Ser Arg Ile
            20                  25                  30
Arg Ser Ala Pro Ala Thr Trp Leu Glu Ala Leu Leu Glu Glu Asp Glu
        35                  40                  45
Glu Glu Ser Leu Lys Pro Asn Leu Gly Leu Thr Asp Leu Leu Thr Gly
    50                  55                  60
Asn Ser Asn Asp Leu Pro Thr Ser Arg Gly Ser Phe Glu Phe Pro Ile
65                  70                  75                  80
Pro Val Glu Gln Gly Leu Tyr Gln Gln Gly Gly Phe His Arg Gln Asn
                85                  90                  95
Ser Thr Pro Ala Asp Phe Leu Ser Gly Ser Asp Gly Phe Ile Gln Ser
            100                 105                 110
Phe Gly Ile Gln Ala Asn Tyr Asp Tyr Leu Ser Gly Asn Ile Asp Val
        115                 120                 125
Ser Pro Gly Ser Lys Arg Ser Arg Glu Met Glu Ala Leu Phe Ser Ser
    130                 135                 140
Pro Glu Phe Thr Ser Gln Met Lys Gly Glu Gln Ser Ser Gly Gln Val
145                 150                 155                 160
Pro Thr Gly Val Ser Ser Met Ser Asp Met Asn Met Glu Asn Leu Met
                165                 170                 175
Glu Asp Ser Val Ala Phe Arg Val Arg Ala Lys Arg Gly Cys Ala Thr
            180                 185                 190
His Pro Arg Ser Ile Ala Glu Arg Val Arg Arg Thr Arg Ile Ser Asp
        195                 200                 205
Arg Ile Arg Lys Leu Gln Glu Leu Val Pro Asn Met Asp Lys Gln Thr
    210                 215                 220
Asn Thr Ala Asp Met Leu Glu Glu Ala Val Glu Tyr Val Lys Val Leu
225                 230                 235                 240
Gln Arg Gln Ile Gln Glu Leu Thr Glu Glu Gln Lys Arg Cys Thr Cys
                245                 250                 255
Ile Pro Lys Glu Glu Gln
            260
```

<210> 9
<211> 1151
<212> DNA
<213> Arabidopsis thaliana

<400> 9
```
ctgtttttgt atccgtgtaa attaatcaca cggtagtttt tgatgaaaag acaacaatcg     60
gagaacaatc tggtctgctg ctaaaattta ataaattgtt ttgtctaatt gtctccaccc    120
ataaaaaagc gcgaattcaa ttcaccgact aaagacattc tccggtggag accccgatgc    180
aatccactca tataagcggc ggaagtagcg gtggtggtgg tggaggagga ggagaggtga    240
gtcgaagtgg attatctcgg atccgttcag ctccagctac ttggattgaa accctactcg    300
aagaagatga agaagaaggt ttaaaaccta acctttgttt aacagagctg cttactggta    360
ataataactc tggaggagtg ataacgagtc gtgacgactc gttcgagttc ctgagttctg    420
ttgagcaagg attgtataat catcatcaag gtggtggctt tcaccgtcag aatagttctc    480
cggctgattt tcttagtggg tctggttctg ggactgatgg gtatttctct aattttggta    540
ttccggcgaa ttatgactat ttgtcgacca acgttgatat ttctccgact aaacggtcta    600
gagatatgga aacacagttt tcttctcagc tgaaagaaga gcaaatgagt ggtgggatat    660
caggaatgat ggatatgaac atggacaaga tttttgagga ttcagttcct tgtagggttc    720
gtgctaaacg tggttgtgct actcatcctc gtagcattgc tgaacgggtg agaagaacgc    780
gaataagtga tcggattagg aggctgcaag agcttgttcc taacatggat aagcaaacca    840
acactgcaga catgttggaa gaagctgtgg agtatgtgaa ggctcttcaa agccagatcc    900
aggaattgac agagcagcag aagagatgca aatgcaaacc taaagaagaa caataatgta    960
tcctttagga tttgatatat ctgtatttta tttttgtact atctaaaaat ggtgatgatc   1020
tgttcgaaaa ttcgaaacat gatcttatat attgaactag aaaaaataga tatatatgaa   1080
ttttagctgt aaaatttttg tacaataagg agaaaaagat ttagaagagt caataaaaag   1140
atgatgttta c                                                        1151
```

<210> 10
<211> 259
<212> PRT
<213> Arabidopsis thaliana

<400> 10
```
Met Gln Ser Thr His Ile Ser Gly Gly Ser Ser Gly Gly Gly Gly Gly
1               5                   10                  15
Gly Gly Gly Glu Val Ser Arg Ser Gly Leu Ser Arg Ile Arg Ser Ala
            20                  25                  30
Pro Ala Thr Trp Ile Glu Thr Leu Leu Glu Glu Asp Glu Glu Glu Gly
        35                  40                  45
Leu Lys Pro Asn Leu Cys Leu Thr Glu Leu Leu Thr Gly Asn Asn Asn
    50                  55                  60
Ser Gly Gly Val Ile Thr Ser Arg Asp Asp Ser Phe Glu Phe Leu Ser
65                  70                  75                  80
Ser Val Glu Gln Gly Leu Tyr Asn His His Gln Gly Gly Gly Phe His
                85                  90                  95
Arg Gln Asn Ser Ser Pro Ala Asp Phe Leu Ser Gly Ser Gly Ser Gly
            100                 105                 110
Thr Asp Gly Tyr Phe Ser Asn Phe Gly Ile Pro Ala Asn Tyr Asp Tyr
        115                 120                 125
Leu Ser Thr Asn Val Asp Ile Ser Pro Thr Lys Arg Ser Arg Asp Met
    130                 135                 140
Glu Thr Gln Phe Ser Ser Gln Leu Lys Glu Glu Gln Met Ser Gly Gly
145                 150                 155                 160
Ile Ser Gly Met Met Asp Met Asn Met Asp Lys Ile Phe Glu Asp Ser
                165                 170                 175
Val Pro Cys Arg Val Arg Ala Lys Arg Gly Cys Ala Thr His Pro Arg
            180                 185                 190
Ser Ile Ala Glu Arg Val Arg Arg Thr Arg Ile Ser Asp Arg Ile Arg
        195                 200                 205
```

```
Arg Leu Gln Glu Leu Val Pro Asn Met Asp Lys Gln Thr Asn Thr Ala
    210                 215                 220
Asp Met Leu Glu Glu Ala Val Glu Tyr Val Lys Ala Leu Gln Ser Gln
225                 230                 235                 240
Ile Gln Glu Leu Thr Glu Gln Gln Lys Arg Cys Lys Cys Lys Pro Lys
                245                 250                 255
Glu Glu Gln


<210>  11
<211>  1425
<212>  DNA
<213>  Arabidopsis thaliana

<400>  11
atcttttatt tttcttttag aaaccaaact tttcagattt ctctctctcc atttggcgat      60
agaagaagtc atgtaccaat catcatcctc cacgtcatca tcatcgcaga gatcatcgct     120
tcccggcggc ggaggactga tccgttacgg ctcagctccg ggatcgtttc taaactctgt     180
ggttgacgaa gtcatcggag gaggctcatc aaacgctcgt gacttcaccg gctatcaacc     240
gtcgtcggat aacttcatcg gtaacttttt caccggagct gctgactcat cctcgctgag     300
atccgattcg acgacttgtg gagtcaacaa ctcatccgac ggacagaaac agctaggcaa     360
taacaataat aataatagta ataaagatat cttcctcgac agatcctacg gtggattcaa     420
cgagatctcg caacaacaca agagcaacga catcggagga ggaaacagct caggatctta     480
ctctctcgct agacaacgta gctctcccgc cgatttcttc acctacctcg cctcagataa     540
aaacaatttc tcgttgaacc aaccaaccag tgattatagt ccgcaaggag ggtctaatgg     600
gggacgagga cattccagat tgaagtctca gctaagcttc acgaatcacg actctctggc     660
tcggatcaac gaggtcaatg agaccccagt ccacgacggt tcaggccatt cgttttctgc     720
ggctagcttt ggtgcagcca ctactgattc ttgggatgac ggttccggtt cgatagggtt     780
taccgtgact aggcccagta aacgatccaa ggacatggac tctggtctct tttcgcagta     840
tagtcttcct tcagacactt caatgaacta catggataac ttcatgcagc ttccagaaga     900
ttctgtaccc tgcaaaatcc gggccaaacg cggctgcgcc acccatccta gaagcatcgc     960
tgagcgggag aggagaacga gaataagtgg gaagctaaag aagctacaag atcttgtccc    1020
caacatggat aagcaaacaa gctattcaga catgctggat ttagctgtac aacacatcaa    1080
aggccttcag catcaacttc agaatttgaa aaaagatcaa gagaattgca cgtgtgggtg    1140
cagtgagaaa ccaagctagc tcaaacccaa aggtagggtc atcctaattt tatttgcatt    1200
gttccgattt tatcatatga gagaattaga gaagatgtaa ataaaataga ttatattaaa    1260
tgtctaatcc tcttataatt aagctcctca ttcgttatca ttataagata gaacagtgac    1320
aattgtccat tatgtttttg tttcaagttt atgttttgag tgtcgcctat tttgtaagga    1380
ttatgctatg aattctgaaa ctaattcgag ttatgatcat aagca                    1425

<210>  12
<211>  362
<212>  PRT
<213>  Arabidopsis thaliana

<400>  12
Met Tyr Gln Ser Ser Ser Ser Thr Ser Ser Ser Ser Gln Arg Ser Ser
1               5                   10                  15
Leu Pro Gly Gly Gly Gly Leu Ile Arg Tyr Gly Ser Ala Pro Gly Ser
            20                  25                  30
Phe Leu Asn Ser Val Val Asp Glu Val Ile Gly Gly Gly Ser Ser Asn
        35                  40                  45
Ala Arg Asp Phe Thr Gly Tyr Gln Pro Ser Ser Asp Asn Phe Ile Gly
    50                  55                  60
Asn Phe Phe Thr Gly Ala Ala Asp Ser Ser Ser Leu Arg Ser Asp Ser
65                  70                  75                  80
Thr Thr Cys Gly Val Asn Asn Ser Ser Asp Gly Gln Lys Gln Leu Gly
                85                  90                  95
Asn Asn Asn Asn Asn Asn Ser Asn Lys Asp Ile Phe Leu Asp Arg Ser
```

```
            100                 105                 110
Tyr Gly Gly Phe Asn Glu Ile Ser Gln Gln His Lys Ser Asn Asp Ile
        115                 120                 125
Gly Gly Gly Asn Ser Ser Gly Ser Tyr Ser Leu Ala Arg Gln Arg Ser
    130                 135                 140
Ser Pro Ala Asp Phe Phe Thr Tyr Leu Ala Ser Asp Lys Asn Asn Phe
145                 150                 155                 160
Ser Leu Asn Gln Pro Thr Ser Asp Tyr Ser Pro Gln Gly Gly Ser Asn
                165                 170                 175
Gly Gly Arg Gly His Ser Arg Leu Lys Ser Gln Leu Ser Phe Thr Asn
            180                 185                 190
His Asp Ser Leu Ala Arg Ile Asn Glu Val Asn Glu Thr Pro Val His
        195                 200                 205
Asp Gly Ser Gly His Ser Phe Ser Ala Ala Ser Phe Gly Ala Ala Thr
    210                 215                 220
Thr Asp Ser Trp Asp Asp Gly Ser Gly Ser Ile Gly Phe Thr Val Thr
225                 230                 235                 240
Arg Pro Ser Lys Arg Ser Lys Asp Met Asp Ser Gly Leu Phe Ser Gln
                245                 250                 255
Tyr Ser Leu Pro Ser Asp Thr Ser Met Asn Tyr Met Asp Asn Phe Met
            260                 265                 270
Gln Leu Pro Glu Asp Ser Val Pro Cys Lys Ile Arg Ala Lys Arg Gly
        275                 280                 285
Cys Ala Thr His Pro Arg Ser Ile Ala Glu Arg Glu Arg Arg Thr Arg
    290                 295                 300
Ile Ser Gly Lys Leu Lys Lys Leu Gln Asp Leu Val Pro Asn Met Asp
305                 310                 315                 320
Lys Gln Thr Ser Tyr Ser Asp Met Leu Asp Leu Ala Val Gln His Ile
                325                 330                 335
Lys Gly Leu Gln His Gln Leu Gln Asn Leu Lys Lys Asp Gln Glu Asn
            340                 345                 350
Cys Thr Cys Gly Cys Ser Glu Lys Pro Ser
        355                 360
```

<210> 13
<211> 937
<212> DNA
<213> Arabidopsis thaliana

<400> 13

```
aagcatcaga ttagcttttg tagggttttt cttttccggt ttcatgtacc ctcctaattc     60
ctctaagtcc accgctcatg acggcggcgg cgatgccgac accaatcaat atgactcagc    120
cgctggagct acccgtgatt tctcctctct tggccctcaa acccaccatc atccgccacc    180
gcagcggcag cagcagcatc agcagaatcc caacctcgtc ggccattatt taccgggcga    240
gccatcttcc atcggattcg attccaacgc ttcttcttcg tcttctttgt tccgacacag    300
aagctctccg gctggattct acgaccaaca tcttcccact gatcccaacg gaacaggttt    360
ttctctagga cggccaaacg gaggctacgg cggaggagga gagcaagggc cgtcgaggtt    420
gaagtcggag ctgagattct ctagtgggag tagtagccat caagaacata attctctacc    480
gcgaatctcg gaggttgaag cggctgcagc ggctagaaac ggtgtcgcat caagtagtat    540
gagttttgga aataatcgta ctaacaattg ggacaactcg tcttctcata tcagtttcac    600
cattgatcaa cccggaaaac ggtccaagaa ctccgacttt ttcaccttag aaactcagta    660
tagcatgccg caaacaactc tggaaatggc gacaatggag aacttgatga acatcccaga    720
ggactcggtg ccttgtaggg ctagagccaa gcgcggcttc gcgactcacc cacgcagcat    780
tgctgaaagg gagagaagaa cgaggataag cgggaagctg aagaagctac aagaacttgt    840
gcctaatatg gacaagcaaa cgagctacgc agatatgttg gatttggctg ttgagcatat    900
caaaggtctt cagcaccaag tagaggtgcg tccttag                            937
```

<210> 14
<211> 297

<212> PRT
<213> Arabidopsis thaliana

<400> 14

```
Met Tyr Pro Pro Asn Ser Ser Lys Ser Thr Ala His Asp Gly Gly Gly
1               5                   10                  15
Asp Ala Asp Thr Asn Gln Tyr Asp Ser Ala Ala Gly Ala Thr Arg Asp
            20                  25                  30
Phe Ser Ser Leu Gly Pro Gln Thr His His His Pro Pro Pro Gln Arg
        35                  40                  45
Gln Gln Gln His Gln Gln Asn Pro Asn Leu Val Gly His Tyr Leu Pro
    50                  55                  60
Gly Glu Pro Ser Ser Ile Gly Phe Asp Ser Asn Ala Ser Ser Ser Ser
65                  70                  75                  80
Ser Leu Phe Arg His Arg Ser Ser Pro Ala Gly Phe Tyr Asp Gln His
                85                  90                  95
Leu Pro Thr Asp Pro Asn Gly Thr Gly Phe Ser Leu Gly Arg Pro Asn
            100                 105                 110
Gly Gly Tyr Gly Gly Gly Gly Glu Gln Gly Pro Ser Arg Leu Lys Ser
        115                 120                 125
Glu Leu Arg Phe Ser Ser Gly Ser Ser Ser His Gln Glu His Asn Ser
    130                 135                 140
Leu Pro Arg Ile Ser Glu Val Glu Ala Ala Ala Ala Ala Arg Asn Gly
145                 150                 155                 160
Val Ala Ser Ser Ser Met Ser Phe Gly Asn Asn Arg Thr Asn Asn Trp
                165                 170                 175
Asp Asn Ser Ser Ser His Ile Ser Phe Thr Ile Asp Gln Pro Gly Lys
            180                 185                 190
Arg Ser Lys Asn Ser Asp Phe Phe Thr Leu Glu Thr Gln Tyr Ser Met
        195                 200                 205
Pro Gln Thr Thr Leu Glu Met Ala Thr Met Glu Asn Leu Met Asn Ile
    210                 215                 220
Pro Glu Asp Ser Val Pro Cys Arg Ala Arg Ala Lys Arg Gly Phe Ala
225                 230                 235                 240
Thr His Pro Arg Ser Ile Ala Glu Arg Glu Arg Arg Thr Arg Ile Ser
                245                 250                 255
Gly Lys Leu Lys Lys Leu Gln Glu Leu Val Pro Asn Met Asp Lys Gln
            260                 265                 270
Thr Ser Tyr Ala Asp Met Leu Asp Leu Ala Val Glu His Ile Lys Gly
        275                 280                 285
Leu Gln His Gln Val Glu Val Arg Pro
    290                 295
```

<210> 15
<211> 1242
<212> DNA
<213> Aquilegia formosa

<400> 15

```
gagttattga tgagcatgaa agtgaacaga tattgtttaa cactccacca ccactaccac     60
aaccacctaa acaacaagca cctatgtcca atcatgcttc aaatgttgct gagaatgttt    120
atagggttgc gagttcgatg ggtttggatc atcaatcatc acaaatgaag atggggaatt    180
ctaatcttat tagacacagt agttctccgg cagggctctt tgccaattta aacgttgaaa    240
acggttatgc tgtaatgcgg ggcataggaa atttcggagc tggaaatagt actaatgaag    300
ctgcatcaac taggaggttg aagaatcaga tgaactaccc atccgggcca ccttcttctt    360
cagggttaat gtctcctatc tctgaagttg gaagtgaaag cattggaaca tgtgctggag    420
atggtggaag tgtgggcaac agcaatgtta gcaatcaata ccctcccaac ttccctctca    480
actcttggga agactctgca ctggagaatt tcacaagcct taaaagattc agagatgcgg    540
acacaaagat gcattctggt ttaaactcgt cagagcctga gaatccagag gctggaaatc    600
```

```
aaactcgcaa tttgacccac cattttagtt tgccaaaaac gtcagtggag atggctgcca    660
tccttcaatt ccaggattct gttccttgta aaattcgagc aaagcggggt tgtgctactc    720
acccacgaag tatcgcggag agggttagac gaactcgaat cagtgaaaga atgagaaagc    780
tacaagaact tgttccaaac atggacaagc aaacaaacac cgcagacatg ttagatttgg    840
ctgtggagta cattaaagag ctccaaaaac aggtcaagaa acttaatgat aatcgagcga    900
cttgtacgtg ttccagtaag cagaaaccat actaaaatcc tagaggttga tatgtttctg    960
tacagaaaaa taaggaagaa aaaggatgga ataaagagaa agtgagagca aagctgactt   1020
ctctagcttt tagttagaag aatgtaagta ggggttactg atgtgggtat ggagatgatt   1080
aaaatccaaa accctatagg caatcagagg ttttctccta gtataaaaat gttgtatagc   1140
aaatcttcca aatgtacttc cttgttgtat gtaatttatt tgtttagtta gtacatagac   1200
tcaacaagct tgttgatatc ctcttcttta ttctgataat ta                       1242

<210>  16
<211>  283
<212>  PRT
<213>  Aquilegia formosa

<400>  16
Met Ser Asn His Ala Ser Asn Val Ala Glu Asn Val Tyr Arg Val Ala
1               5                   10                  15
Ser Ser Met Gly Leu Asp His Gln Ser Ser Gln Met Lys Met Gly Asn
            20                  25                  30
Ser Asn Leu Ile Arg His Ser Ser Ser Pro Ala Gly Leu Phe Ala Asn
        35                  40                  45
Leu Asn Val Glu Asn Gly Tyr Ala Val Met Arg Gly Ile Gly Asn Phe
    50                  55                  60
Gly Ala Gly Asn Ser Thr Asn Glu Ala Ala Ser Thr Arg Arg Leu Lys
65                  70                  75                  80
Asn Gln Met Asn Tyr Pro Ser Gly Pro Pro Ser Ser Ser Gly Leu Met
                85                  90                  95
Ser Pro Ile Ser Glu Val Gly Ser Glu Ser Ile Gly Thr Cys Ala Gly
            100                 105                 110
Asp Gly Gly Ser Val Gly Asn Ser Asn Val Ser Asn Gln Tyr Pro Pro
        115                 120                 125
Asn Phe Pro Leu Asn Ser Trp Glu Asp Ser Ala Leu Glu Asn Phe Thr
    130                 135                 140
Ser Leu Lys Arg Phe Arg Asp Ala Asp Thr Lys Met His Ser Gly Leu
145                 150                 155                 160
Asn Ser Ser Glu Pro Glu Asn Pro Glu Ala Gly Asn Gln Thr Arg Asn
                165                 170                 175
Leu Thr His His Phe Ser Leu Pro Lys Thr Ser Val Glu Met Ala Ala
            180                 185                 190
Ile Leu Gln Phe Gln Asp Ser Val Pro Cys Lys Ile Arg Ala Lys Arg
        195                 200                 205
Gly Cys Ala Thr His Pro Arg Ser Ile Ala Glu Arg Val Arg Arg Thr
    210                 215                 220
Arg Ile Ser Glu Arg Met Arg Lys Leu Gln Glu Leu Val Pro Asn Met
225                 230                 235                 240
Asp Lys Gln Thr Asn Thr Ala Asp Met Leu Asp Leu Ala Val Glu Tyr
                245                 250                 255
Ile Lys Glu Leu Gln Lys Gln Val Lys Lys Leu Asn Asp Asn Arg Ala
            260                 265                 270
Thr Cys Thr Cys Ser Ser Lys Gln Lys Pro Tyr
        275                 280

<210>  17
<211>  1329
<212>  DNA
<213>  Aquilegia formosa
```

```
<400>  17
taaagcatta tacatttata aagaatagag actgactaaa ttataatcac aagagaagat     60
aatacccgtt cttgagaaga cacctgagac ctaaatctca ctgatctgac ctagcagtag    120
tacactaaac agtgagtctg cacttttta tatcttcttc cacttattcc ctacctaggc    180
atttgcaatt cttttggtgc tcagtgagtt cctgccaagt ttgggatttt agatgtacta    240
aaatgctgct ctggttgcat caaaacaact atatctatta agatgacatt cttttacatt    300
ttcgggtttc tctaattcag tacctgtatt tgattctgta agaatttgac atattcaact    360
gcttcttcca acatgtctgc agtattagtt tgcttatcca tattaggtac aagttcctga    420
agttttctta tgcgatcact gattcgtgtc cttcgaacct tactactagt cgtcctgata    480
ttgcttatag tgttggcatg tgtactcact ttcaatccaa tcccaaggaa tctcactact    540
ctgatgttaa acgggttatc cgatatgtca atagcacggc taatcatggt ctctggtata    600
ctcgtgatac taatagtgtt gtcgcaggat actgtgatgc cgattatggc agtgacttat    660
ctgatcgtca cagtaccagt ggtgggtgct tttatctggg gaataatttg atttcttgga    720
acaataagaa acagtccact gttgccatgt ccagtgctga ggcagaatat attgctgcgg    780
ctggttgttg tactcaattg ttgtggatga aacagatgct atctgactat ggtctttccc    840
aaaacctcat aactctgttt ctctctgcaa tgctccgggg atgggttgca cagccacgct    900
ttgcacgaac gcgacaagga acagaatcct ggaaaagctt ctccatatcc atatcagaaa    960
aactatcact gtgctccttt ttcaattggg attcgtgtga agaatctgac tctctttcta   1020
atttaccagt agtggggatg tcaagaaatg aaagtatatc atagttggaa gctaccccat   1080
catcagaagc aagatttgga gaaggtgaag tgagattggc aagaagttca ggtggtgaac   1140
tgttgtttct ggtgaaggtg ctagaagtat caaataagtt agtggggtca taaatattgg   1200
agtttctaag gttgggtgct tgagaaacac attgggtttg tggtaattgt aaaggaagaa   1260
cttcatcatc ttcttgaagt aagctatcaa accatgatac tggagctgat ttaaaccttg   1320
caagtccac                                                           1329

<210>  18
<211>  91
<212>  PRT
<213>  Aquilegia formosa

<400>  18
Met Ile Ser Arg Ala Ile Asp Ile Ser Asp Asn Pro Phe Asn Ile Arg
1               5                   10                  15
Val Val Arg Phe Leu Gly Ile Gly Leu Lys Val Ser Thr His Ala Asn
            20                  25                  30
Thr Ile Ser Asn Ile Arg Thr Thr Ser Ser Lys Val Arg Arg Thr Arg
        35                  40                  45
Ile Ser Asp Arg Ile Arg Lys Leu Gln Glu Leu Val Pro Asn Met Asp
    50                  55                  60
Lys Gln Thr Asn Thr Ala Asp Met Leu Glu Glu Ala Val Glu Tyr Val
65                  70                  75                  80
Lys Phe Leu Gln Asn Gln Ile Gln Val Leu Asn
                85                  90

<210>  19
<211>  685
<212>  DNA
<213>  Brassica napus

<400>  19
acgaatttg ttttattggt taaatttgat tgatcgaact aatttaagtt gtcactatgg     60
tgacgacttc gagaatgtgt caaaaccaga cttcattaat agaccaccat cactttcttt    120
ggacctccaa aatcacaagt atataattaa aatctttttg ttgctgttga attgattcct    180
taagtctcct tttcttctct tctatacttc atgtttggaa acacatcata tcatttcatc    240
atcgcttctt gcatgcccca caagtgcatc tctccatccc cttctccagc gactctactt    300
ggtgttgaag acctttgata tgctcaacag ccaaatccaa catgtctgca tagctcgttt    360
gtttgtccat attgggcacc agttcttgta gcttcttcag cttcccgctt attctcgttc    420
ttctctccct ttcagcgata ctacgcgggt gagtcgcaca tccacgcttg gccctaaccc    480
```

```
tacagggcac cgaaccctct gggatgttca tcagactctc cattcttgcc atttccagag    540
acgtttgtgg catgctaaac tgagtttcta aggtgaaaaa gtcggtggtc ttggaccgtt    600
ttccaggttc atcaatggtg aaactgattt gagaagacga cctgtcccaa atgttactat    660
catttccaaa actcatactc gtcga                                          685

<210>  20
<211>  145
<212>  PRT
<213>  Brassica napus

<400>  20
Met Ser Phe Gly Asn Asp Ser Asn Ile Trp Asp Arg Ser Ser Ser Gln
1               5                   10                  15
Ile Ser Phe Thr Ile Asp Glu Pro Gly Lys Arg Ser Lys Thr Thr Asp
            20                  25                  30
Phe Phe Thr Leu Glu Thr Gln Phe Ser Met Pro Gln Thr Ser Leu Glu
        35                  40                  45
Met Ala Arg Met Glu Ser Leu Met Asn Ile Pro Glu Gly Ser Val Pro
    50                  55                  60
Cys Arg Val Arg Ala Lys Arg Gly Cys Ala Thr His Pro Arg Ser Ile
65                  70                  75                  80
Ala Glu Arg Glu Arg Arg Thr Arg Ile Ser Gly Lys Leu Lys Lys Leu
                85                  90                  95
Gln Glu Leu Val Pro Asn Met Asp Lys Gln Thr Ser Tyr Ala Asp Met
            100                 105                 110
Leu Asp Leu Ala Val Glu His Ile Lys Gly Leu Gln His Gln Val Glu
        115                 120                 125
Ser Leu Glu Lys Gly Met Glu Arg Cys Thr Cys Gly Ala Cys Lys Lys
    130                 135                 140
Arg
145

<210>  21
<211>  584
<212>  DNA
<213>  Brassica oleracea

<400>  21
cccacgcgtc cgttctcaga tgaaagaaga gcaaatgagt ggtggtgtat caggaatgat     60
ggatatcaac atggataagc tacttgagga ctcggttcct tgtagggttc gtgctaaacg    120
cggttgtgca actcatcctc gtagcattgc tgaacgggtg aggagaacgc gaataagtga    180
tcggatcagg aggctgcaag aacttgttcc taacatggat aagcaaacca acactgcaga    240
catgttagaa gaagctgtgg agtatgtgaa agctcttcaa agcaagatcc aggagttgac    300
agagcagcag aggaggtgca tatgcaaacc taaggaagaa caataaggtt tcctatagga    360
ttgatatata tatatatcaa taagtatttt tttttttga atctacttgt gctgatgatc     420
ggttcgaaaa tttgaaacat gatcctatac ggaactagaa aaaatagatt atttactact    480
gtctgtcaaa aacacatccg agaaccttct ctctaccaaa gtttctaaat caaagaatct    540
cgtcggtgac cggtggtctt gccgccggtt accatctttt ttct                     584

<210>  22
<211>  108
<212>  PRT
<213>  Brassica oleracea

<400>  22
Met Lys Glu Glu Gln Met Ser Gly Gly Val Ser Gly Met Met Asp Ile
1               5                   10                  15
Asn Met Asp Lys Leu Leu Glu Asp Ser Val Pro Cys Arg Val Arg Ala
            20                  25                  30
```

```
Lys Arg Gly Cys Ala Thr His Pro Arg Ser Ile Ala Glu Arg Val Arg
        35                  40                  45
Arg Thr Arg Ile Ser Asp Arg Ile Arg Arg Leu Gln Glu Leu Val Pro
    50                  55                  60
Asn Met Asp Lys Gln Thr Asn Thr Ala Asp Met Leu Glu Glu Ala Val
65                  70                  75                  80
Glu Tyr Val Lys Ala Leu Gln Ser Lys Ile Gln Glu Leu Thr Glu Gln
                85                  90                  95
Gln Arg Arg Cys Ile Cys Lys Pro Lys Glu Glu Gln
            100                 105

<210>  23
<211>  1410
<212>  DNA
<213>  Brassica oleracea

<400>  23
tccgctctct ctctctctct ctctcggaca ctcctcctcc tcctccggct caatttggcg     60
atagaacaag tcatgtacca atcatcttcc cccacgtcat catcgtcaca aagatcatcg    120
ctcccccagcg gtggcgcagg actgatccga tacggttcag ctcctggatc gctcctgaac    180
tctatggtgg atgaagtcat tggagggtca aacggtcgag acttcaactt tccaccgccg    240
gataacttcc tcggtgattt cttcgccgga gctgattctt cctcgctgag atccgagtcc    300
atgacttgcg gagtcaactc atccgacggc caaaaacatc ttggcagcaa caacaacaat    360
aataataata ataataaaga tttgctcctg gacagatcct acggtggatt caacgagatc    420
tcacagcaca agatcaacaa ccacgttgga ggaggaggaa gtagctctgg atcttactct    480
ctagctagac aacgtagctc tcctgctgac ttcttcagct acctctccgg tgaaaaaaac    540
aacttctcgt tgaaccaacc aaccagtgat tataatcagc aaggagggtc taacgcagga    600
cgtggaccat cgagactcaa gtctcagcta agcttcacta gtcacgaccc actgtctcgt    660
atctctgagg tcaatgagac atctgtccac gatggttcag gccattcgtt ttccgcggct    720
agcttcggtg ccgccactga ttcttgggat gacggttccg gttcaatagg gtttactgtg    780
acccgaccca ctaaacgacc caaggacatg aactcaggtc tcttttcaca gtatagtctt    840
ccttcagaca cttcaatgaa ctacatggat aactacatgc agcttcccga agattctgta    900
ccatgcaaag ttcgggccaa acgtggcttc gccacccacc cgagaagcat cgccgagcgg    960
gagaggagaa cgagaattag tgggagacta aagaagctac aagatcttgt ccccaacatg   1020
gataagcaaa caagctattc tgacatgctg gatttagcgg tacaacatat caaaggcctt   1080
caacaccaac ttcagacttt gaagaaagaa caagagaatt gcacgtgcga atgcagtgag   1140
agaccaaact agcctagcta cgcaacccta acggtaggga tgcagtgaga gaggtagctg   1200
caaccctaac ggtagggatc attccaaatt tatcatatgc tgtttgataa tgtaagagaa   1260
ttagagaaga tgtaaataaa tacattatca attgcctaat tctctcttat aagcttccct   1320
catcgaaaca gtgccaattg tccattacat ttttttcaag tcttatgttc tctgaatatt   1380
tggaataatt cgaattatga atttacaatt                                    1410

<210>  24
<211>  359
<212>  PRT
<213>  Brassica oleracea

<400>  24
Met Tyr Gln Ser Ser Ser Pro Thr Ser Ser Ser Ser Gln Arg Ser Ser
1               5                   10                  15
Leu Pro Ser Gly Gly Ala Gly Leu Ile Arg Tyr Gly Ser Ala Pro Gly
            20                  25                  30
Ser Leu Leu Asn Ser Met Val Asp Glu Val Ile Gly Gly Ser Asn Gly
        35                  40                  45
Arg Asp Phe Asn Phe Pro Pro Pro Asp Asn Phe Leu Gly Asp Phe Phe
    50                  55                  60
Ala Gly Ala Asp Ser Ser Ser Leu Arg Ser Glu Ser Met Thr Cys Gly
65                  70                  75                  80
Val Asn Ser Ser Asp Gly Gln Lys His Leu Gly Ser Asn Asn Asn Asn
```

```
                    85                  90                  95
Asn Asn Asn Asn Asn Lys Asp Leu Leu Leu Asp Arg Ser Tyr Gly Gly
                100                 105                 110
Phe Asn Glu Ile Ser Gln His Lys Ile Asn Asn His Val Gly Gly Gly
            115                 120                 125
Gly Ser Ser Ser Gly Ser Tyr Ser Leu Ala Arg Gln Arg Ser Ser Pro
    130                 135                 140
Ala Asp Phe Phe Ser Tyr Leu Ser Gly Glu Lys Asn Asn Phe Ser Leu
145                 150                 155                 160
Asn Gln Pro Thr Ser Asp Tyr Asn Gln Gln Gly Gly Ser Asn Ala Gly
                165                 170                 175
Arg Gly Pro Ser Arg Leu Lys Ser Gln Leu Ser Phe Thr Ser His Asp
            180                 185                 190
Pro Leu Ser Arg Ile Ser Glu Val Asn Glu Thr Ser Val His Asp Gly
        195                 200                 205
Ser Gly His Ser Phe Ser Ala Ala Ser Phe Gly Ala Ala Thr Asp Ser
    210                 215                 220
Trp Asp Asp Gly Ser Gly Ser Ile Gly Phe Thr Val Thr Arg Pro Thr
225                 230                 235                 240
Lys Arg Pro Lys Asp Met Asn Ser Gly Leu Phe Ser Gln Tyr Ser Leu
                245                 250                 255
Pro Ser Asp Thr Ser Met Asn Tyr Met Asp Asn Tyr Met Gln Leu Pro
            260                 265                 270
Glu Asp Ser Val Pro Cys Lys Val Arg Ala Lys Arg Gly Phe Ala Thr
        275                 280                 285
His Pro Arg Ser Ile Ala Glu Arg Glu Arg Arg Thr Arg Ile Ser Gly
    290                 295                 300
Arg Leu Lys Lys Leu Gln Asp Leu Val Pro Asn Met Asp Lys Gln Thr
305                 310                 315                 320
Ser Tyr Ser Asp Met Leu Asp Leu Ala Val Gln His Ile Lys Gly Leu
                325                 330                 335
Gln His Gln Leu Gln Thr Leu Lys Lys Glu Gln Glu Asn Cys Thr Cys
            340                 345                 350
Glu Cys Ser Glu Arg Pro Asn
        355
```

<210> 25
<211> 1182
<212> DNA
<213> Citrus clementina

<400> 25

```
tttgaatctc tctcattttt ttcttcttaa gaagtaacat agaagaagaa caataatgta      60
tcatcatcca tcatcatcct cttcttcaca acaaaatcca atgggcccca ctactgctgc     120
tggtgctggt ggcagtggtg ggctgactcg gtacgtctca gctccaggct ctcttttgag     180
gacagccgtt gattccatca tcggatcatc atcaccatca ccatcaccat cacaaccttc     240
catcgtctcc tcctctcaca gccaccaaca ccaccattac ttcgccggtg gctctacaac     300
cacagctggc ggtgactctt ctcctgtatc ttcgtttacc accactgagt caacttgcaa     360
agtcaactca tccaacgctc caggtaataa caccaaatct ggcttgcaga gatcctacgg     420
cctcaatgag attcctcaaa cacatactgc ctcctcctcc ttgctcagac agcgtagttc     480
tcccgccggc tttcttagtc atttcgcctc cgaaaacgta ggtttttcag ttacaaggga     540
aaccggaaac tataactcac aaggcagatc tagtggtggc ctcggagtgt caaggttaaa     600
gtctcaatta agtttcacag gacaagattc tctttcccag atctctgaag aaagtgagaa     660
tggtattgat ggagttagtt ctggtagtgg ccggcaaaat gccacacatt cttattcgac     720
tgctagtttt gggatggact cttgggagaa tggaacttcc attgtgtttt ctgcaccacc     780
aagtaaaaga actaaaacct tggatggaga catttttaat tgtctcaatg ctttggactc     840
tcagtttaca atgccgcaaa catctcttga aatggctaca gtggataatt tattaaatat     900
ccctgaagac tctgtccctt gcaaaattcg tgctaaacgt ggctgtgcta ctcatccccg     960
aacatccctg aaagggagaa aaaaccaaaa ttaacaagga aattgaaaaa attaacagac    1020
```

```
tttgttcaaa acatggaaag ccaaacaatt aattctaaat ttttaaactt tggaatgcaa   1080
catataaaag cccttcaaaa caaatggaaa aatccccaca aaaatcttta aaaattgcac   1140
ggtgggaaag caagcccaac ccttttgatt ttttgctttt at                      1182

<210>  26
<211>  261
<212>  PRT
<213>  Citrus clementina

<400>  26
Met Gln Pro Thr Ala Gly Gly Ser Ser Ser Ser Ser Gly Gly Gly Gly
1               5                   10                  15
Gly Gly Val Gly Gly Arg Gly Glu Leu Ser Arg Gly Gly Leu Ala Arg
            20                  25                  30
Leu Arg Ser Ala Pro Ala Ser Trp Ile Asp Ala Leu Leu Glu Glu Glu
        35                  40                  45
Leu Glu Asp Pro Leu Lys Pro Asn Gln Cys Leu Thr Gln Leu Leu Ser
    50                  55                  60
Ser Gly Asn Pro Val Ser Val Thr Ala Gly Leu Ser Leu Ser Gln Ser
65                  70                  75                  80
Gln Leu Asp Gln Val Gly Phe Gln Arg Gln Asn Ser Ser Pro Ala Asp
                85                  90                  95
Leu Phe Asp Gly Tyr Phe Ser Asn Tyr Ala Thr Pro Ser Ser Tyr Asp
            100                 105                 110
Tyr Val Asp Val Ser Pro Asn Ser Asn Lys Arg Ala Arg Glu Asp Asn
        115                 120                 125
Asn Thr Gln Phe Pro Ser Pro Thr Ala Lys Leu Asn Phe His Ser His
    130                 135                 140
Leu Lys Val Glu Gln Ser Gly Gln Val Pro Gly Gly Val Ser Asn Leu
145                 150                 155                 160
Val Asp Met Asp Met Glu Lys Leu Leu Glu Asp Ser Val Pro Cys Arg
                165                 170                 175
Val Arg Ala Lys Arg Gly Cys Ala Thr His Pro Arg Ser Ile Ala Glu
            180                 185                 190
Arg Val Arg Arg Thr Arg Ile Ser Asp Arg Ile Arg Lys Leu Gln Asp
        195                 200                 205
Leu Val Pro Asn Met Asp Lys Gln Thr Asn Thr Ala Asp Met Leu Glu
    210                 215                 220
Glu Ala Val Glu Tyr Val Lys Phe Leu Gln Lys Gln Ile Glu Glu Leu
225                 230                 235                 240
Thr Glu His Gln Arg Arg Cys Lys Cys Ser Ala Lys Asp Lys Ser Glu
                245                 250                 255
Lys Leu Pro Asn Leu
            260

<210>  27
<211>  776
<212>  DNA
<213>  Cichorium intybus

<400>  27
tgagttgctt aaccacagga aatcatctat acccacagag gaatagatta gagatgcaac     60
cgacaggcag cggcggagga ctgtccaggt tccggtcagc tccggcgaca tggctggaag    120
cactgctaga atcagaagaa gaagatgtaa tcattgaccc accaaaacca tccttaacta    180
cgactcaacc ttctatttca gccacttcct ccagacctac gtacgctgat cctaatatct    240
tcttacctag ccccgttagt ctccgacaga acagctctcc ggcggaattt ctttctcaga    300
tcaacaatcc tgatgcctac ctctccaact actcgacggc gaattacgac gactacctct    360
ccccttcctt tcacggcgtt aaatcatcca gagatgtggc tttagacgac caacaagtca    420
agttcaccac gcagctgagc ggagatcaga gcgcgttgtt ggatgttgag atggataagc    480
```

ttctggggga ttctgtgccg tgtcgagtac gagcaaagcg cggctgtgcc actcatcctc 540
ggagtatcgc cgagagggta agaaggactc ggattagtga cagaatcagg aagctccaag 600
aactggttcc gaatatggat aagcaaacaa acacagccga catgctagaa gaggcagttg 660
agtacgtcaa gtttctacaa agacagattc aggaacttag ggagcaccga gacaaatgca 720
gatgtgtggt taacgattga tctcagcaaa gaagctacca attttcaagg gattga 776

<210> 28
<211> 228
<212> PRT
<213> Cichorium intybus

<400> 28
Met Gln Pro Thr Gly Ser Gly Gly Gly Leu Ser Arg Phe Arg Ser Ala
1 5 10 15
Pro Ala Thr Trp Leu Glu Ala Leu Leu Glu Ser Glu Glu Glu Asp Val
20 25 30
Ile Ile Asp Pro Pro Lys Pro Ser Leu Thr Thr Thr Gln Pro Ser Ile
35 40 45
Ser Ala Thr Ser Ser Arg Pro Thr Tyr Ala Asp Pro Asn Ile Phe Leu
50 55 60
Pro Ser Pro Val Ser Leu Arg Gln Asn Ser Ser Pro Ala Glu Phe Leu
65 70 75 80
Ser Gln Ile Asn Asn Pro Asp Ala Tyr Leu Ser Asn Tyr Ser Thr Ala
85 90 95
Asn Tyr Asp Asp Tyr Leu Ser Pro Ser Phe His Gly Val Lys Ser Ser
100 105 110
Arg Asp Val Ala Leu Asp Asp Gln Gln Val Lys Phe Thr Thr Gln Leu
115 120 125
Ser Gly Asp Gln Ser Ala Leu Leu Asp Val Glu Met Asp Lys Leu Leu
130 135 140
Gly Asp Ser Val Pro Cys Arg Val Arg Ala Lys Arg Gly Cys Ala Thr
145 150 155 160
His Pro Arg Ser Ile Ala Glu Arg Val Arg Arg Thr Arg Ile Ser Asp
165 170 175
Arg Ile Arg Lys Leu Gln Glu Leu Val Pro Asn Met Asp Lys Gln Thr
180 185 190
Asn Thr Ala Asp Met Leu Glu Glu Ala Val Glu Tyr Val Lys Phe Leu
195 200 205
Gln Arg Gln Ile Gln Glu Leu Arg Glu His Arg Asp Lys Cys Arg Cys
210 215 220
Val Val Asn Asp
225

<210> 29
<211> 630
<212> DNA
<213> Chamaecyparis obtusa

<400> 29
gggatgatgc aacaaggcat tctggaaaac attctagctt gcaaaatggt acaaactatg 60
ctgcaagaaa gcgttcaaaa gaaatggatg ggaaagtgat gatgcaagat ctgcataacc 120
cagatgccca gaaggtagag acaggaagtc agggcacatc tacattagtc aatccatcac 180
acaatttgtc aaggtccata tctgcagaac ttgcattgga agagctcatg caagactctg 240
ttccctgcaa agttcgtgca aagcggggat ttgcaacaca cccacgaagc attgccgaga 300
gagtgcgaag aacacgaatc agtgaacgaa tgaggaaatt gcaggagctt gtgccaaaca 360
gtgacaagca aactacaaat attgctgaca tgttagatga ggctgttgaa tatgtgaaag 420
cccttcagaa acaggttcag gagttcaagg aaaagcaagc caactgcaca tgcatacacg 480
taccagattg taaattcaaa agataacctt tttgtgaaca tgcattttta ggctttcaaa 540
gcctagttgc aatattgtta ttaatattcc ttttggaata tggcatttcc ccttggaggg 600

tgttaatatt tgtgatgcca aacaatactg 630

<210> 30
<211> 140
<212> PRT
<213> Chamaecyparis obtusa

<400> 30
Met Asp Gly Lys Val Met Met Gln Asp Leu His Asn Pro Asp Ala Gln
1 5 10 15
Lys Val Glu Thr Gly Ser Gln Gly Thr Ser Thr Leu Val Asn Pro Ser
20 25 30
His Asn Leu Ser Arg Ser Ile Ser Ala Glu Leu Ala Leu Glu Glu Leu
35 40 45
Met Gln Asp Ser Val Pro Cys Lys Val Arg Ala Lys Arg Gly Phe Ala
50 55 60
Thr His Pro Arg Ser Ile Ala Glu Arg Val Arg Arg Thr Arg Ile Ser
65 70 75 80
Glu Arg Met Arg Lys Leu Gln Glu Leu Val Pro Asn Ser Asp Lys Gln
85 90 95
Thr Thr Asn Ile Ala Asp Met Leu Asp Glu Ala Val Glu Tyr Val Lys
100 105 110
Ala Leu Gln Lys Gln Val Gln Glu Phe Lys Glu Lys Gln Ala Asn Cys
115 120 125
Thr Cys Ile His Val Pro Asp Cys Lys Phe Lys Arg
130 135 140

<210> 31
<211> 973
<212> DNA
<213> Citrus sinensis

<400> 31
agcacatcgg ctagtaggct aatcaatcgt gttaacttgt cacctgggtc atcttcaaga 60
tttatgcctc agattgctga aactgggaac caaagcatgg ggaatagtag cccagagaaa 120
aactttgtgg gcaataatgg tgccagtaga caatacatgc ccaattttcc aagcgatcct 180
tgggatgatg cttcattcag tggtgttaag agggctagag acagtacttg caacatgtct 240
tttggtctag acgcatatga aactcagaat ggaaattctg gaaaccaatc tactcgcttg 300
gtccaccatt tgagcttacc aaaaacatct gctgaaatgg ctgctgtgga gaagtttttg 360
cattttcaag gttctgttcc ttgcaagatt cgagccaaga gaggttgtgc cactcatcca 420
agaagcattg ccgagagggt tagaagaact cggataagtg aacgaatgag gaaattgcaa 480
gaccttttcc caaacatgga caagcaaaca aacacagctg aaatgttaga tctggcagtt 540
gaacacatta aagatcttca gaaacaagtc aagttactca cggacaataa ggcaaaatgc 600
atgtgcccaa acaaacaaaa gctatgttga attacaaaaa cttccggtag acttgtatct 660
gtacagatag cttaaggaag ctgctacttg tgagatgaaa aagaaagatg actcctcatt 720
tccactgccc ttaaaaaata ataggatagc gagtgagatt tagcgggttg aaaagattgc 780
tgttgtaatt tattgtaaga tccagttgag tagagtgatt gtcttgcttt gattctcttc 840
acgtagatta attgtattaa gcttcttcat ccatgatttc ttcaatacaa agtacaatct 900
tttagctgtt ggccgaaagt ttgaaaagaa attgtatgag ttgggatgct gaaagaaatt 960
ggaaggcgag cta 973

<210> 32
<211> 253
<212> PRT
<213> Citrus sinensis

<400> 32
Met Gln Pro Thr Ala Gly Gly Ser Ser Ser Ser Ser Gly Gly Gly Gly
1 5 10 15

```
Gly Gly Val Gly Gly Arg Gly Glu Leu Ser Arg Gly Gly Leu Ala Arg
            20                  25                  30
Leu Arg Ser Ala Pro Ala Ser Trp Ile Asp Ala Leu Leu Glu Glu Glu
        35                  40                  45
Leu Glu Asp Pro Leu Lys Pro Asn Gln Cys Leu Thr Gln Leu Leu Ser
    50                  55                  60
Ser Gly Asp Pro Val Ser Val Thr Ala Gly Leu Ser Leu Ser Gln Ser
65                  70                  75                  80
Gln Leu Asp Gln Val Gly Phe Gln Arg Gln Asn Ser Ser Pro Ala Asp
                85                  90                  95
Leu Phe Asp Gly Tyr Phe Ser Asn Tyr Ala Thr Pro Ser Ser Tyr Asp
            100                 105                 110
Tyr Val Asp Val Ser Pro Asn Ser Asn Lys Arg Ala Arg Glu Asp Asn
        115                 120                 125
Asn Thr Gln Phe Pro Ser Pro Thr Ala Lys Leu Asn Phe His Ser His
    130                 135                 140
Leu Lys Val Glu Gln Ser Gly Gln Val Pro Gly Gly Val Ser Asn Leu
145                 150                 155                 160
Val Asp Met Asp Met Glu Lys Leu Leu Glu Asp Ser Val Pro Cys Arg
                165                 170                 175
Val Arg Ala Lys Arg Gly Cys Ala Thr His Pro Arg Ser Ile Ala Glu
            180                 185                 190
Arg Val Arg Arg Thr Arg Ile Ser Asp Arg Ile Arg Lys Leu His Asp
        195                 200                 205
Leu Val Pro Asn Met Asp Lys Gln Thr Asn Thr Ala Asp Met Leu Glu
    210                 215                 220
Glu Ala Val Glu Tyr Val Lys Phe Leu Gln Lys Gln Ile Glu Glu Leu
225                 230                 235                 240
Thr Glu His Gln Arg Arg Cys Lys Cys Ser Ala Lys Asp
                245                 250
```

<210> 33
<211> 1298
<212> DNA
<213> Citrus sinensis

<400> 33

```
ggctggtctt gtttccactt accagagaaa gatagagtat aaaataaatt ataaaaaaaa      60
aaaaaataga ggtagaggta gagggttcat ttcatttcat attcatttgt ttattctcta     120
tctaaatttc aaaaaaaaaa aaaaaattag agaaaaggaa gtgtaaacta aaagctagca     180
aatcagtggc gataaaacag atgcaaccga cagctggtgg tagcagtagc tcaagcggcg     240
gaggtggagg tggagttggc ggtcgtggag aactgagccg aggaggactc gctcggctcc     300
ggtcagctcc agcttcgtgg atagatgcac ttttagaaga agaattagag gacccattga     360
agccaaacca gtgcctgact cagctactta gtagcggcga tcctgttagt gttactgctg     420
ggctgagctt gagtcagagt cagctggacc aagttggttt ccagcggcag aacagctccc     480
ctgccgattt atttgatggg tacttttcaa actatgcaac tccatcgagt tacgattatg     540
tcgatgtttc tcctaattcc aataagagag ctagggaaga caacaataca caatttccat     600
ctccaactgc aaaactgaac tttcactctc acttgaaagt ggagcaaagt ggacaggtac     660
ctggtggtgt ttcaaatttg gtagatatgg atatggagaa actgttggag gattcagtgc     720
cttgtagggt tcgtgcaaag cgtggttgtg ccactcatcc tcgaagtatt gctgagaggg     780
ttcgtaggac gcggatcagt gaccgtatta ggaagcttca cgatcttgtg ccgaacatgg     840
acaagcaaac aaacaccgct gatatgttag aggaggcagt agaatatgtc aagtttcttc     900
aaaagcaaat tgaggaactc acagagcatc agaggagatg caaatgctcg gcaaaagatt     960
aatctgagaa gttaccaaat ttgtaactat atatacaagg ttaaagtcca tcatggtttg    1020
tttggcgata atatcctatg attcctaccc tccaagagat cagcctcgca acaacaggtg    1080
gcacaatctt tatcactaac tattcctcta tatttggaag attgccagtc atatggaact    1140
atagccatgg cgttcatgat caggactttt tcattttggc aaagaactga acttcatgct    1200
ctctgaagtt cagttggtat ggctgtaaaa gtgctgtaca cgataattta ttatcagtct    1260
cgttagcaag cttctttttt atgaattatt ttttcaat                            1298
```

<210> 34
<211> 188
<212> PRT
<213> Citrus sinensis

<400> 34
```
Met Pro Gln Ile Ala Glu Thr Gly Asn Gln Ser Met Gly Asn Ser Ser
1               5                   10                  15
Pro Glu Lys Asn Phe Val Gly Asn Asn Gly Ala Ser Arg Gln Tyr Met
            20                  25                  30
Pro Asn Phe Pro Ser Asp Pro Trp Asp Asp Ala Ser Phe Ser Gly Val
        35                  40                  45
Lys Arg Ala Arg Asp Ser Thr Cys Asn Met Ser Phe Gly Leu Asp Ala
    50                  55                  60
Tyr Glu Thr Gln Asn Gly Asn Ser Gly Asn Gln Ser Thr Arg Leu Val
65                  70                  75                  80
His His Leu Ser Leu Pro Lys Thr Ser Ala Glu Met Ala Ala Val Glu
                85                  90                  95
Lys Phe Leu His Phe Gln Gly Ser Val Pro Cys Lys Ile Arg Ala Lys
            100                 105                 110
Arg Gly Cys Ala Thr His Pro Arg Ser Ile Ala Glu Arg Val Arg Arg
        115                 120                 125
Thr Arg Ile Ser Glu Arg Met Arg Lys Leu Gln Asp Leu Phe Pro Asn
    130                 135                 140
Met Asp Lys Gln Thr Asn Thr Ala Glu Met Leu Asp Leu Ala Val Glu
145                 150                 155                 160
His Ile Lys Asp Leu Gln Lys Gln Val Lys Leu Leu Thr Asp Asn Lys
                165                 170                 175
Ala Lys Cys Met Cys Pro Asn Lys Gln Lys Leu Cys
            180                 185
```

<210> 35
<211> 750
<212> DNA
<213> Centaurea solstitialis

<400> 35
```
gcgggggaat tattattcac atttagaccg atgaattcga caagcacgaa tctgattcga      60
cagagtagct ctccggctgg atttttgtcc tccttgaacg ctgaaaccgg catgagagat     120
gcaaaccaag tcatttcgtc aaacgggtta aatagtaatc atatcggttt ctcatcggca     180
ccatcgtctt ctcctatgta cttgccccaa attgccgaaa acaggaatga gatgaacgat     240
tccacattca gaagcttgaa aaggatgaca gatggcgatt caaacatgtt ccatacctcg     300
ataaagatgg acacacagca taatggacca tctggacatt atacaccaag tttacttcac     360
catatgagct tgccgaacac ctcttcggaa atggcggttg cagaaaagtt cttgcgattt     420
gagcaagact cgattccttg caagacacgt gcaaaaagag gatttgccac acacccgcga     480
aacattgcgg agagggttag aagaaccaga ataagtgata gaatgaaaaa gctaccagag     540
cttttcccta ccatggacaa gcgaacaagc acagcagaca tgttagatat ggctgttcaa     600
tacattacag atctccaaaa gaatatgcag gctctcaacg atgctcgagc acggtgcacg     660
tgttcaagct aactattaca aatgggctcg cccacataac caaagccttt gcatcataga     720
acacgaaaca ggtccatggg ccattgtgta                                      750
```

<210> 36
<211> 213
<212> PRT
<213> Centaurea solstitialis

<400> 36
```
Met Asn Ser Thr Ser Thr Asn Leu Ile Arg Gln Ser Ser Ser Pro Ala
```

```
1               5                   10                  15
Gly Phe Leu Ser Ser Leu Asn Ala Glu Thr Gly Met Arg Asp Ala Asn
            20                  25                  30
Gln Val Ile Ser Ser Asn Gly Leu Asn Ser Asn His Ile Gly Phe Ser
        35                  40                  45
Ser Ala Pro Ser Ser Ser Pro Met Tyr Leu Pro Gln Ile Ala Glu Asn
    50                  55                  60
Arg Asn Glu Met Asn Asp Ser Thr Phe Arg Ser Leu Lys Arg Met Thr
65                  70                  75                  80
Asp Gly Asp Ser Asn Met Phe His Thr Ser Ile Lys Met Asp Thr Gln
            85                  90                  95
His Asn Gly Pro Ser Gly His Tyr Thr Pro Ser Leu Leu His His Met
            100                 105                 110
Ser Leu Pro Asn Thr Ser Ser Glu Met Ala Val Ala Glu Lys Phe Leu
        115                 120                 125
Arg Phe Glu Gln Asp Ser Ile Pro Cys Lys Thr Arg Ala Lys Arg Gly
    130                 135                 140
Phe Ala Thr His Pro Arg Asn Ile Ala Glu Arg Val Arg Arg Thr Arg
145                 150                 155                 160
Ile Ser Asp Arg Met Lys Lys Leu Pro Glu Leu Phe Pro Thr Met Asp
            165                 170                 175
Lys Arg Thr Ser Thr Ala Asp Met Leu Asp Met Ala Val Gln Tyr Ile
            180                 185                 190
Thr Asp Leu Gln Lys Asn Met Gln Ala Leu Asn Asp Ala Arg Ala Arg
        195                 200                 205
Cys Thr Cys Ser Ser
    210
```

<210> 37
<211> 1361
<212> DNA
<213> Carthamus tinctorius

<400> 37

```
cctccatatc aaaaaaagaa aaaagaaaga taaacacttt gacgtacaaa aaaggtcgtt     60
ttacatgaaa aaagagaggc aaaggcatat tggtttctga gaggacgtga gtttggcatg    120
taaaacgtag tactacaaac ctacctaatc ccccttattc actacaaagc caccttttcc    180
attcacacaa cctttttttc ttcatattca caaatgaatc aagaattcag cttgctccag    240
agacaacaag caagaaccaa atggcaacaa gaccatgagt ttttcaatgg tgatgatgat    300
caccaccacc ttcaaaatat gttcgctccg ttttcgggtt tgatcgataa ccagccgacg    360
agcttagacg cggttaagcc ggacccccggg atgcacgaag ggtggccgga tttctcgtat    420
ggggatcaat tgggttatgg gtatatgaat cagacatttc taaatgggaa ttcaggttca    480
gtttcagtgt cacctaagaa gaggaaaact gataagggtg gtaaaagcct agagctagtt    540
tctgaaaagg agaagaggat aaaagggtgt gctgaagaag gagattctaa gatcacacat    600
caatataaca ataataataa taatagcaat tgtgacaata agagcagtaa taacagcaaa    660
gaagcttcta ctacaaacac ttcatctaag gagaaatcga aggtctccga ggttcaaaaa    720
cccgattata ttcatgttcg agctcgccgt ggccaagcca ccgacagcca tagtttagcc    780
gaaagggtaa gaagggagaa gatcagtgag aggatgaaat acctgcagga tttggtacca    840
gggtgcaata aaatcacagg aaaagctgga atgcttgatg aaatcatcaa ctatgttcaa    900
tcccttcaaa aacaagtaga gtttctgtcc atgaagcttg ctactgttaa cccggagcta    960
gacttcaaca tcgataatgt gttcatgaaa gagatgttcc agtcctcgac aagcgaattt   1020
ccagcactag ggtgttctcc ggaagcagct aattctgcat attttcaatt aagttcatta   1080
gagcaagcgg tttcatgctg tggattagac atgggaatga actcgaccga aatggcatta   1140
agaagaagta ttagtgttcc aacttcggtc cctggaacat ttatggattc atcctgtttc   1200
aatcaaatcc agccaactgc tacctgggat gctgatttac agaatctgta caaaatggaa   1260
tttgaacaag gaacattgat accatttcaa tcccatcaat ttacaggttc aaatgaagga   1320
agcaatctga agatggagat gtgatcagta gaaggaattt t                        1361
```

<210> 38

```
<211>  215
<212>  PRT
<213>  Carthamus tinctorius

<400>  38
Met Gln Pro Pro Gly Gly Ala Gly Gly Leu Ser Arg Phe Gln Ser Ala
1               5                   10                  15
Pro Ala Thr Trp Leu Glu Thr Leu Leu Glu Ser Glu Glu Glu Glu Glu
            20                  25                  30
Gln Asp Val Ile Ile Asp Pro Pro Lys Pro Thr Ile Gly Gly Ile Tyr
        35                  40                  45
Leu Asp Pro Asn Leu Leu Leu Pro Ile Pro Pro Ala Ala Ala Val Val
    50                  55                  60
Gly Arg Arg Gln Asn Ser Ser Pro Ala Glu Phe Leu Ser Glu Thr Asn
65                  70                  75                  80
Asn Leu His Gly His Gly Tyr Leu Ser Asn Phe Asp Asp Tyr Leu Ser
                85                  90                  95
Ser Ser Phe His Gly Gly Ala Lys Pro Pro Thr Asp Pro Lys Phe Thr
            100                 105                 110
Thr Gln Leu Ser Gly Asp Gln Ser Ala Leu Leu Asp Val Asp Met Asp
        115                 120                 125
Lys Leu Leu Glu Asp Ser Val Pro Cys Arg Val Arg Ala Lys Arg Gly
    130                 135                 140
Cys Ala Thr His Pro Arg Ser Ile Ala Glu Arg Val Arg Arg Thr Arg
145                 150                 155                 160
Ile Ser Asp Arg Ile Arg Lys Leu Gln Glu Leu Val Pro Asn Met Asp
                165                 170                 175
Lys Gln Ser Asn Thr Ala Asp Met Leu Glu Glu Ala Val Asp Tyr Val
            180                 185                 190
Lys Phe Leu Gln His Gln Ile Gln Glu Leu Arg Ala His Arg Asp Lys
        195                 200                 205
Cys Thr Cys Ser Val Asn Asp
    210                 215

<210>  39
<211>  1276
<212>  DNA
<213>  Euphorbia esula

<220>
<221>  misc_feature
<222>  (1244)..(1246)
<223>  n is a, c, g, or t

<400>  39
atttttttcag aatcaattga aacctccatt accggatcag aattcagttc cgggaggaat      60
ggattaccgg atgggaaatt cacgggccgg agcaggaacc ggacggcttt ccggcgcaaa     120
aaatgggggga aatagctcga atcttttacg gcataatagc tcccccgctg gacttttctc     180
caatatcact gttgaaatgg aaaacggcta cgctgtaatg agaggtatgg gagattatgt     240
gacacccaaa agagaagcat cttattctcc ttctccggca acaagtaggc ccccgccggc     300
agccacctca ggcgggagaa tgagtccaat tacggaaatt gggaacacca ataacaacaa     360
cattggagaa aataataatt cagacagtaa ttgtgtttat gatgagagta gaaataatga     420
ttatgtgaca ggttttccaa tgggatcatg ggatcatgat aatgcatcat catcctctgt     480
catgtctcat ggtttaactg atgatgatcg tacactttct ggtggcttaa ttagttcaga     540
aactcagaat cgagattcgg ggattccagc acctccgaga ttgtcacatc acttgagttt     600
accaaaaaca acagctgaaa tgtcagccat agaaaagttt ctccaacttc aagattccgt     660
cccttgcaag attcgagcta aacgtggatt tgctactcat cctaggagca ttgctgagag     720
ggtaagaaga actcgaatta gtgaaagaat gaggaagctt caagacttgg taccaaacat     780
ggataagcaa acaaacacat cagacatgtt ggatttggct gttgactaca ttaaagacct     840
```

```
tcaaagacaa gtcaagacac tttccgacag tcgtgcaaat tgtacctgca atgcaagtca     900
acaaccgctc taggtttaca caagtatggc ttgattagag atgtatatac attattatta     960
ataagaaccc ctagatagca ggtgtaaaac gccatcgttt ggggtatcac gagagggcga    1020
gaaaagggta aagttctact agaacctaac cgcaagaatt ggacacattt atcaaaatag    1080
aatagttgtg tacattagca aagtcaagat cataaatttg aggggcaact gcaacacttg    1140
taatctcatt tgggacagag tatctcgtga aaccacccct ttaaaagggt ggattccggt    1200
gaccggagat aaaaacgatt tcaaagcacc cgtctctctg tcannnggat gtatatagtt    1260
caagtttgat gtactc                                                    1276

<210>  40
<211>  284
<212>  PRT
<213>  Euphorbia esula

<400>  40
Met Asp Tyr Arg Met Gly Asn Ser Arg Ala Gly Ala Gly Thr Gly Arg
1               5                   10                  15
Leu Ser Gly Ala Lys Asn Gly Gly Asn Ser Ser Asn Leu Leu Arg His
            20                  25                  30
Asn Ser Ser Pro Ala Gly Leu Phe Ser Asn Ile Thr Val Glu Met Glu
        35                  40                  45
Asn Gly Tyr Ala Val Met Arg Gly Met Gly Asp Tyr Val Thr Pro Lys
    50                  55                  60
Arg Glu Ala Ser Tyr Ser Pro Ser Pro Ala Thr Ser Arg Pro Pro Pro
65                  70                  75                  80
Ala Ala Thr Ser Gly Gly Arg Met Ser Pro Ile Thr Glu Ile Gly Asn
                85                  90                  95
Thr Asn Asn Asn Asn Ile Gly Glu Asn Asn Asn Ser Asp Ser Asn Cys
            100                 105                 110
Val Tyr Asp Glu Ser Arg Asn Asn Asp Tyr Val Thr Gly Phe Pro Met
        115                 120                 125
Gly Ser Trp Asp His Asp Asn Ala Ser Ser Ser Ser Val Met Ser His
    130                 135                 140
Gly Leu Thr Asp Asp Asp Arg Thr Leu Ser Gly Gly Leu Ile Ser Ser
145                 150                 155                 160
Glu Thr Gln Asn Arg Asp Ser Gly Ile Pro Ala Pro Pro Arg Leu Ser
                165                 170                 175
His His Leu Ser Leu Pro Lys Thr Thr Ala Glu Met Ser Ala Ile Glu
            180                 185                 190
Lys Phe Leu Gln Leu Gln Asp Ser Val Pro Cys Lys Ile Arg Ala Lys
        195                 200                 205
Arg Gly Phe Ala Thr His Pro Arg Ser Ile Ala Glu Arg Val Arg Arg
    210                 215                 220
Thr Arg Ile Ser Glu Arg Met Arg Lys Leu Gln Asp Leu Val Pro Asn
225                 230                 235                 240
Met Asp Lys Gln Thr Asn Thr Ser Asp Met Leu Asp Leu Ala Val Asp
                245                 250                 255
Tyr Ile Lys Asp Leu Gln Arg Gln Val Lys Thr Leu Ser Asp Ser Arg
            260                 265                 270
Ala Asn Cys Thr Cys Asn Ala Ser Gln Gln Pro Leu
        275                 280

<210>  41
<211>  1402
<212>  DNA
<213>  Gossypium hirsutum

<400>  41
ttctccttta acttatataa tttcccttcc tagttctccc atttctccac gctaccaaag      60
```

```
ccatgtatcc atcatcttcg tctcaaaagc ccatgggtca atccgggctc acccgttacg    120
gttctgctcc cggctccttt ctggccaacg ccgttgattc cgtcatcgga gcggacccta    180
acctcgtcgg ccactacttc tccgccgctg actcctcctc tttaacttca gagtcaactt    240
gtaaagtcag ctcatccaac gatccgcgag agcctaaatc agctgctgct gctgctcctg    300
cccctcctca tcctcctccg catcatcccc atggatctta ccctgcacct tcttcttcct    360
ctctgctcag acaacgcagc tcccccgccg gtttccttag tcacctcaca tcggaaaacg    420
gtttttctgt tacaatgggc aacagaaatt atagctctca aggcagtggt aatggtggcc    480
atggagtgtc aaggttgaag tcccagttga gtttcactag gcaagattct ctttctcaga    540
tctctgaagt gagtgagaat cttgtggatg gtgttagctc taacagtaac caccaaaatc    600
ctgcacattc gtttgctgct gctggctttg gaatggattc atgggataat acaaactcca    660
ttgtgttttc tgccccatca agcaagagag ctaaaaatct tgatggcgat atttataact    720
gtttcaatgc cttggaaact cagtttagct tgccccaaac gacactcgag atggccacag    780
ttgaaaagct gctacatatt cctgaagatt ccgtgccctg caaaatccga gccaaacgtg    840
gttgtgccac tcaccctcga agcattgctg aaagagagag aagaacaaga ataagtggga    900
agctgaagaa attacaagag cttgttccta acatggacaa gcaaacgagc tatgcagaca    960
tgctggattt ggctgtgcag catattaaag gccttcaaaa cgaagttcag aaactccaca   1020
aagaattgga aagttgtaca tgtgggtgca aacaaagctc atgatgatga aggctaacca   1080
ggtttaagga aacaagaagt ttgtctgatt tccaaatttt gaagccaaga ctaatttagt   1140
tggcttagtt tgacttaata atgaaaaagt agaggtaaac aactcgaaga tgtatataaa   1200
tggagtcaca ttgtttccct agtttgagaa gtgattgagt tggaaaatga aaaaaaagaa   1260
gcaaattgtt agttttacct attaaagttc agtgtctgta atgtaacata tgttttggct   1320
tttgtatttt aaatgggaat gaagaattca gtttacatgt ttgggtattt aatttaatat   1380
tttgagtttc ttatatgtaa aa                                            1402
```

```
<210>  42
<211>  333
<212>  PRT
<213>  Gossypium hirsutum

<400>  42
Met Tyr Pro Ser Ser Ser Ser Gln Lys Pro Met Gly Gln Ser Gly Leu
1               5                   10                  15
Thr Arg Tyr Gly Ser Ala Pro Gly Ser Phe Leu Ala Asn Ala Val Asp
            20                  25                  30
Ser Val Ile Gly Ala Asp Pro Asn Leu Val Gly His Tyr Phe Ser Ala
        35                  40                  45
Ala Asp Ser Ser Ser Leu Thr Ser Glu Ser Thr Cys Lys Val Ser Ser
    50                  55                  60
Ser Asn Asp Pro Arg Glu Pro Lys Ser Ala Ala Ala Ala Ala Pro Ala
65                  70                  75                  80
Pro Pro His Pro Pro Pro His His Pro His Gly Ser Tyr Pro Ala Pro
                85                  90                  95
Ser Ser Ser Ser Leu Leu Arg Gln Arg Ser Ser Pro Ala Gly Phe Leu
            100                 105                 110
Ser His Leu Thr Ser Glu Asn Gly Phe Ser Val Thr Met Gly Asn Arg
        115                 120                 125
Asn Tyr Ser Ser Gln Gly Ser Gly Asn Gly Gly His Gly Val Ser Arg
    130                 135                 140
Leu Lys Ser Gln Leu Ser Phe Thr Arg Gln Asp Ser Leu Ser Gln Ile
145                 150                 155                 160
Ser Glu Val Ser Glu Asn Leu Val Asp Gly Val Ser Ser Asn Ser Asn
                165                 170                 175
His Gln Asn Pro Ala His Ser Phe Ala Ala Ala Gly Phe Gly Met Asp
            180                 185                 190
Ser Trp Asp Asn Thr Asn Ser Ile Val Phe Ser Ala Pro Ser Ser Lys
        195                 200                 205
Arg Ala Lys Asn Leu Asp Gly Asp Ile Tyr Asn Cys Phe Asn Ala Leu
    210                 215                 220
Glu Thr Gln Phe Ser Leu Pro Gln Thr Thr Leu Glu Met Ala Thr Val
```

```
225                 230                 235                 240
Glu Lys Leu Leu His Ile Pro Glu Asp Ser Val Pro Cys Lys Ile Arg
                245                 250                 255
Ala Lys Arg Gly Cys Ala Thr His Pro Arg Ser Ile Ala Glu Arg Glu
            260                 265                 270
Arg Arg Thr Arg Ile Ser Gly Lys Leu Lys Lys Leu Gln Glu Leu Val
        275                 280                 285
Pro Asn Met Asp Lys Gln Thr Ser Tyr Ala Asp Met Leu Asp Leu Ala
    290                 295                 300
Val Gln His Ile Lys Gly Leu Gln Asn Glu Val Gln Lys Leu His Lys
305                 310                 315                 320
Glu Leu Glu Ser Cys Thr Cys Gly Cys Lys Gln Ser Ser
                325                 330
```

<210> 43
<211> 647
<212> DNA
<213> Gerbera hybrid cultivar

<400> 43

```
gtgccgaaaa tgagaatgaa atggatgata cttcgttcaa cagcttgaaa cggagcagaa     60
atggtgattt cttgcagttc caacaagact tagtaccttg caagatacgt gcaaaaagag    120
gattcgctac acatccacga agcatcgcag agagggagag acgaacccgg atcagcgaac    180
ggattaaaaa gttgcaagag cttttcccta acatggataa gcaaatgaac atagcagata    240
tgttggatat ggctttggac tacattaaag atctccaaaa agaagtgcag agtttaaatg    300
atgctcgagc aaggtgcatg tgttcaagta aacaattaca gtcaggttca accaaatagc    360
cccgtgtttt aatacacaac ttgtaatttc taaataaaaa tacaaattta tttatgtgat    420
actaagtttt taaagtatag tattcaaact tgtatcgtat aagatatttt tgtacaatat    480
aagttgtggt ataaaatgct tattttgata aaaacaaata tattttagtt gtaaacaaat    540
atcatcttta gtcctgacac tgggatgggt accctaggta taaaagtata ttgtataaaa    600
agtatccgtt atttttgcac actcaatatt aaacctgaaa atgataa                  647
```

<210> 44
<211> 112
<212> PRT
<213> Gerbera hybrid cultivar

<400> 44

```
Met Asp Asp Thr Ser Phe Asn Ser Leu Lys Arg Ser Arg Asn Gly Asp
1               5                   10                  15
Phe Leu Gln Phe Gln Gln Asp Leu Val Pro Cys Lys Ile Arg Ala Lys
            20                  25                  30
Arg Gly Phe Ala Thr His Pro Arg Ser Ile Ala Glu Arg Glu Arg Arg
        35                  40                  45
Thr Arg Ile Ser Glu Arg Ile Lys Lys Leu Gln Glu Leu Phe Pro Asn
    50                  55                  60
Met Asp Lys Gln Met Asn Ile Ala Asp Met Leu Asp Met Ala Leu Asp
65                  70                  75                  80
Tyr Ile Lys Asp Leu Gln Lys Glu Val Gln Ser Leu Asn Asp Ala Arg
                85                  90                  95
Ala Arg Cys Met Cys Ser Ser Lys Gln Leu Gln Ser Gly Ser Thr Lys
            100                 105                 110
```

<210> 45
<211> 967
<212> DNA
<213> Glycine max

<400> 45

```
ccctatccat gtatccctct tcctcttcct cctcctcctc ttcctctcag tccatgaccc      60
aagctggcct cactcgctac ggttccgccc ccggttctct tctcaccagc acggttgatt     120
ctcttattgg tggatcccgt cccagtccct acttttccgg agagtcttct gagtctccct     180
gcaaagagca aagatctttc cacaaccacc ccccctcctt cgcttcctcc ttgctccgac     240
agaaaaactc tctcgacggc ttcctttccc atctctccaa acaccatggg gtggggttca     300
accatcaccc ccgggtggtt atcaaatgca aattcaaatt caattgttct ctcttgaagt     360
ctcaactcag cttcacccac gaatctcttt ccaacactgt taatgttgat ccctccagca     420
ccaccttcgg aatggatccc tgggacaaca attccatcgc cttttctgca acttcaacca     480
agcgctccaa aaccaacacc aatgatcctg acattgtaca ttccctgaat tcagccttgg     540
gatcacagtt caatcgtcca catacaagtc tggaaatgtc aaccgtggac aagctattgc     600
acattcctga agattctgtc ccttgtaaaa tccgtgctaa gcgaggttgt gctactcatc     660
cccgtagcat tgccgagcgg gagagaagaa caaggatcag tggcaagctc aagaaattac     720
aggaccttgt acccaatatg gataagcaaa caagctatgc agacatgctc gatttggccg     780
ttcaacacat taaaggtctt caaactcagg ttcagaagct tcacaaagaa atggagaatt     840
gcacttgcgg atgcaaacaa agcaaataac ttcttttttt acgagattct atagcaacgt     900
gcatagaagt caaggttttg atggcaaaga ttgaaaactg atttgtacc gatctactta      960
aatgaat                                                               967
```

```
<210>  46
<211>  418
<212>  PRT
<213>  Glycine max

<400>  46
Met Glu Ser Asp Leu Gln Gln His Pro Pro Met Phe Leu Asp His His
1               5                   10                  15
Gln Gln Gln Met Asn Ser Gly Leu Thr Arg Tyr Arg Ser Ala Pro Ser
            20                  25                  30
Ser Tyr Phe Ser Ser Ile Ile Asp His Glu Phe Tyr Glu His Val Phe
        35                  40                  45
Asn Arg Pro Ser Ser Pro Glu Thr Glu Arg Met Leu Thr Arg Phe Val
    50                  55                  60
Asn Ser Leu Gly Gly Gly Asp Ala Asp Ala Glu Asp Ser Leu Ala Thr
65                  70                  75                  80
Thr Gln Asn Pro Pro Thr Val Val Ala Val Lys Glu Glu Val Asn Gln
                85                  90                  95
Gln Pro Pro Asp Val Thr Ser Met Asn Asn Glu Pro Leu Val Leu Gln
            100                 105                 110
Gln Gln Gln Gln Gln Gln Gln Gln Gln Gln Gln Ser Asn Asn Met Tyr
        115                 120                 125
Asn Tyr Gly Ser Ser Gly Thr Gln Asn Phe Tyr Gln Ser Thr Gly Arg
    130                 135                 140
Pro Pro Leu Pro Asn Gln Met Lys Thr Gly His Gly Ser Ser Ser Asn
145                 150                 155                 160
Leu Ile Arg His Gly Ser Ser Pro Ala Gly Leu Phe Ser Asn Ile Asn
                165                 170                 175
Ile Asp Ile Thr Gly Tyr Ala Ala Val Val Arg Gly Met Gly Thr Met
            180                 185                 190
Gly Ala Ala Ala Asn Asn Thr Ser Glu Glu Ala Asn Phe Ser Pro Ala
        195                 200                 205
Thr Arg Met Lys Asn Asn Ala Pro Asn Phe Ser Ser Gly Leu Met Ser
    210                 215                 220
Ser Arg Ala Glu Val Gly Asn Lys Ser Asn Thr Gln Asn Asn Asn Ala
225                 230                 235                 240
Glu Asn Glu Gly Phe Ala Glu Ser Gln Gly Asn Glu Phe Ile Pro Ala
                245                 250                 255
Gly Phe Pro Val Gly Pro Trp Asn Asp Ser Ala Ile Met Ser Asp Asn
            260                 265                 270
Val Thr Gly Leu Lys Arg Phe Arg Asp Glu Asp Val Lys Pro Phe Ser
```

```
        275                 280                 285
Gly Gly Leu Asn Ala Pro Glu Ser Gln Asn Glu Thr Gly Gly Gln Gln
    290                 295                 300
Pro Ser Ser Ser Ala Leu Ala His Gln Leu Ser Leu Pro Asn Thr Ser
305                 310                 315                 320
Ala Glu Met Ala Ala Ile Glu Lys Phe Leu Gln Leu Ser Asp Ser Val
                325                 330                 335
Pro Cys Lys Ile Arg Ala Lys Arg Gly Cys Ala Thr His Pro Arg Ser
            340                 345                 350
Ile Ala Glu Arg Val Arg Arg Thr Lys Ile Ser Glu Arg Met Arg Lys
        355                 360                 365
Leu Gln Asp Leu Val Pro Asn Met Asp Lys Gln Thr Asn Thr Ala Asp
    370                 375                 380
Met Leu Asp Leu Ala Val Glu Tyr Ile Lys Asp Leu Gln Asn Gln Val
385                 390                 395                 400
Glu Ala Leu Ser Asp Asn Arg Ala Lys Cys Thr Cys Leu His Lys Lys
                405                 410                 415
Gln Gln
```

<210> 47
<211> 2146
<212> DNA
<213> Glycine max

<400> 47

```
aaaattgagg gcattctagt aaaactcaaa acaccaaatc tgagagctgg atttatggct      60
ctgttcactt ctcattggct gcacctcttc acaaactaaa tggaatgcta ttttataatc     120
atttttcatc ttttctcttg aaccttcctt cgcccatctt ctccaccact ccacacaacc     180
ttaaatccaa acccaaacca aacgaaaaac acacacacac acaccccaag agaagaattt     240
tctttcttca ggattccgag aagttggtgt gatctgatgc gagtgttaag aagataatct     300
gaggagaaaa aagaaagaaa aaaaagtttc attttttttt ttcaatggag tcagatcttc     360
agcagcaccc tcccatgttt ttggatcacc accagcagca gatgaattca gggttgacac     420
gttaccgatc ggcgccaagt tcgtattttt cgagcatcat tgaccatgag ttctacgagc     480
acgttttcaa tcgaccctct agccccgaaa cagagcgaat gttgacacgc tttgtgaata     540
gtcttggtgg gggtgatgct gatgcagagg attcacttgc tactactcag aatcccccaa     600
ctgttgttgc ggttaaggaa gaggttaatc agcaacctcc ggatgttaca tccatgaata     660
atgaaccact tgttctccaa caacaacagc agcagcagca acaacaacaa caaagtaata     720
atatgtataa ttatggctct tctggtactc agaattttta ccaaagcact ggacgaccac     780
ctttgccaaa ccagatgaag actggtcatg gaagtagttc taatcttatc agacatggta     840
gctcacctgc tggattgttt tcaaacatta acattgatat tactggctat gctgctgttg     900
taaggggcat gggaactatg ggagctgctg ctaataacac cagcgaggaa gcaaactttt     960
caccagcaac aaggatgaag aataatgcac caaacttctc ttcaggacta atgtcatcaa    1020
gggctgaagt tggaaacaag agcaatacac agaacaataa tgcagaaaat gaagggtttg    1080
ctgaaagcca gggcaatgag tttatccctg caggtttccc cgttggtccg tggaatgatt    1140
ctgcaatcat gtctgacaac gtgacaggtc tgaagagatt tagagatgaa gatgtgaaac    1200
cattttctgg tggtttaaat gcacctgaaa gtcagaatga aactggaggc caacaacctt    1260
cttcttctgc tttggctcat caattgagtt tgccaaatac ttctgcagaa atggcagcca    1320
ttgagaagtt tctgcagctc tcagattctg ttccttgcaa aattcgtgcc aaacggggct    1380
gtgccactca cccaagaagc attgcagaga gggttagaag aactaaaatt agtgagcgaa    1440
tgaggaagct acaagatctg gtccccaaca tggataagca aacaaacaca gcagacatgt    1500
tggacttggc tgttgagtac attaaagacc ttcaaaacca agttgaggca ctttcggaca    1560
atcgggcaaa gtgtacgtgt ttacacaaga agcagcaata acggaaaagg aggagggtac    1620
ccgcgtcatt ccctgtacag cttagtaata atttggtcta gaaactagcg cacctttccg    1680
ataattatct agctacatca cttcttctgc gcttgtttgg gagtgtggtg gcaaaagttg    1740
agtttatata tgatcgatca atatttgatg cccacatatt caaggcatgg tgtacaaaat    1800
tttctgatat gtaagtctat ttgcctttac tcttaggggc ccccatttac ggggatgatg    1860
gaggatgggg tgagttggaa aatggaaatt ttcatttttg catcttcaag gttcagcaat    1920
aacgtttaaa aaaaagagag agagagagag agagaatgtt atgtatgggt ttggtttcac    1980
```

```
attcaaggac atgggaaaca tggggggacc gaagccgaca tagttttgtt ggttatatat   2040
atacttggat gcctattgag tagtgtgtgt ttgtactttt atttttttt cctttttta   2100
tgattttaat acgaagtagt tgtcattaaa agtatgacac atttga                  2146

<210>  48
<211>  286
<212>  PRT
<213>  Glycine max

<400>  48
Met Tyr Pro Ser Ser Ser Ser Ser Ser Ser Ser Ser Ser Gln Ser Met
1               5                   10                  15
Thr Gln Ala Gly Leu Thr Arg Tyr Gly Ser Ala Pro Gly Ser Leu Leu
            20                  25                  30
Thr Ser Thr Val Asp Ser Leu Ile Gly Gly Ser Arg Pro Ser Pro Tyr
        35                  40                  45
Phe Ser Gly Glu Ser Ser Glu Ser Pro Cys Lys Glu Gln Arg Ser Phe
    50                  55                  60
His Asn His Pro Pro Ser Phe Ala Ser Ser Leu Leu Arg Gln Lys Asn
65                  70                  75                  80
Ser Leu Asp Gly Phe Leu Ser His Leu Ser Lys His His Gly Val Gly
                85                  90                  95
Phe Asn His His Pro Arg Val Val Ile Lys Cys Lys Phe Lys Phe Asn
            100                 105                 110
Cys Ser Leu Leu Lys Ser Gln Leu Ser Phe Thr His Glu Ser Leu Ser
        115                 120                 125
Asn Thr Val Asn Val Asp Pro Ser Ser Thr Thr Phe Gly Met Asp Pro
    130                 135                 140
Trp Asp Asn Asn Ser Ile Ala Phe Ser Ala Thr Ser Thr Lys Arg Ser
145                 150                 155                 160
Lys Thr Asn Thr Asn Asp Pro Asp Ile Val His Ser Leu Asn Ser Ala
                165                 170                 175
Leu Gly Ser Gln Phe Asn Arg Pro His Thr Ser Leu Glu Met Ser Thr
            180                 185                 190
Val Asp Lys Leu Leu His Ile Pro Glu Asp Ser Val Pro Cys Lys Ile
        195                 200                 205
Arg Ala Lys Arg Gly Cys Ala Thr His Pro Arg Ser Ile Ala Glu Arg
    210                 215                 220
Glu Arg Arg Thr Arg Ile Ser Gly Lys Leu Lys Lys Leu Gln Asp Leu
225                 230                 235                 240
Val Pro Asn Met Asp Lys Gln Thr Ser Tyr Ala Asp Met Leu Asp Leu
                245                 250                 255
Ala Val Gln His Ile Lys Gly Leu Gln Thr Gln Val Gln Lys Leu His
            260                 265                 270
Lys Glu Met Glu Asn Cys Thr Cys Gly Cys Lys Gln Ser Lys
        275                 280                 285

<210>  49
<211>  1105
<212>  DNA
<213>  Glycine max

<220>
<221>  misc_feature
<222>  (1099)..(1100)
<223>  n is a, c, g, or t

<400>  49
taaatgttac atgcctagtt tcaccactga cttctgggat ggttctgcat tcagtgcctc     60
```

```
cagaacagct agtaacagag gtgaaatctc attttccact tcaaaggcta tggatattca    120
ggatgaagat tttggatacc aaaaagttgg tttgacccac catttgagtc tgcctggctc    180
ttccagtagg atggctacaa tggagaagct ttatcaaatt caaggatctg ttccatgtaa    240
aattcgtgcc aagagaggtt ttgccactca cccgagaagt attgctgaaa gggaaaggag    300
aacacgaatt agcgcaagaa tcaagaaatt gcaagacctt ttcccaaaat cagacaagca    360
aacaagcact gcagatatgt tggatttggc agttgagtac attaaagact tgcagaaaca    420
agttaagatt ctcagagata ctagggcaaa atgcacttgt acaagcaatc agaagcactg    480
atttatatat ccttgtagat aacaaaaaga aggagcattt ttttttcttt tcatgtattt    540
atcctttggt gtaccaaaat ttttggtcag tgtgattatg tagagtagcg ctgatatttg    600
ctagtatcta agaatttagg tccaggtccg tgtgagtctc ttcagttttg tttttcattt    660
tccctcattt gttttttctt tctttccttt gcttgattta agtatcacaa tgtaataaaa    720
agtttagaaa gagccaattg taactgttca ttttactcat aagaggtttg atggcatcac    780
aaaataaaat tgagtctcaa ctgtcacacg ctaaagattg agttctatat ggaagaaatt    840
accggtgcaa taagtagtta agatctttgt attcgtgttg caaatatagg gggtgtcctg    900
attaggagtt cagaaaagag gggtggaatt gccttgatgt tgaaccaatg ccaagctatg    960
cacactccag tgcttatcat catttcagaa gggtgatgaa gacattagat gtgagaggag   1020
aggtctagct tatattagat gtagcaaaga agagtgctgg gaatttattt tccaatacac   1080
cgaatttgtt agaacaacnn gatca                                         1105
```

<210> 50
<211> 157
<212> PRT
<213> Glycine max

<400> 50

```
Met Gln Pro Thr Ser Gly Gly Gly Gly Leu Ala Arg Phe Arg Ser Ala
1               5                   10                  15
Pro Ala Ser Trp Leu Glu Ser Val Leu Leu Lys Glu Glu Glu Glu Glu
            20                  25                  30
Glu Asp Pro Leu Ser Phe Thr Gln Leu Leu Ser Thr Ile Asp Asp Ala
        35                  40                  45
Pro Ser His Ser Gln His Gln Leu Tyr Gly Ala Ala Leu Ser Asn Lys
    50                  55                  60
Asp Lys Thr Pro Glu Ile Phe Met Leu Glu Asp Ser Val Pro Cys Arg
65                  70                  75                  80
Val Arg Ala Lys Arg Gly Cys Ala Thr His Pro Arg Ser Ile Ala Glu
                85                  90                  95
Arg Val Arg Arg Thr Arg Ile Ser Asp Arg Ile Arg Lys Leu Gln Glu
            100                 105                 110
Leu Val Pro Asn Met Asp Lys Gln Thr Asn Thr Ala Asp Met Leu Asp
        115                 120                 125
Glu Ala Val Ala Tyr Val Lys Phe Leu Gln Lys Gln Ile Glu Glu Leu
    130                 135                 140
Ser Glu His Gln Arg Arg Cys Lys Cys Val Val Gln Glu
145                 150                 155
```

<210> 51
<211> 599
<212> DNA
<213> Glycine max

<220>
<221> misc_feature
<222> (554)..(557)
<223> n is a, c, g, or t

<400> 51

```
agaatggaga gctagcaaac caagttaata tgttatcaca tcacttgagt ttaccaaaaa     60
cttcagcaga gatgattact atggagaagt tgcttcagtt ccctgattct gttccttgta    120
```

```
aaatcagagc aaagcgaggc tgtgctactc atcctcgaag cattgctgaa agggtgagaa    180
gaactcggat cagtgaaaga atgaggaaat tacaagagct tgtcccacac atggataagc    240
aaaccaacac agcagacatg ttggacttgg ctgttgaata cattaaagat cttcagaaac    300
aattcaagac tctaagtgaa aaaagggcaa actgcaagtg tataagcatg ccaaaggcag    360
atacaaatca aattgcttga tcacctttct ttatgagcaa tgcatagcaa agatggcttg    420
gatcggatgc ttttaacttt aatgctgagc aagtagaaag aaagatgcta atataggatg    480
ctactaccag aaataggagg gtgaaaaata gagtagaaat aaggtgagaa agatgtgtta    540
tttggattga tacnnnnagg agataaacag tagagaaagt tctccttaca tatttggat     599
```

<210> 52
<211> 357
<212> PRT
<213> Glycine max

<400> 52

```
Met Glu Thr Met Leu Ser Lys Leu Leu Pro Ser Asn Asn Gly Trp Ser
1               5                   10                  15
Asn Ser Glu Ala Leu Gln Glu Phe Gly Gly Lys Pro Val Lys Gln Glu
            20                  25                  30
Ile Gly Glu Ser Ile Pro Gln Glu Pro Pro Gln Gln Asn Gly Tyr Ser
        35                  40                  45
Tyr Gly Gly Ser Gln Leu Ile Tyr Gln Ser Gln Gln Ile Gln Gly Leu
    50                  55                  60
Pro Asn Gly Gly Ser Ser Ser Ala Ser Gly Ser Ala Phe Asp Gly Ser
65                  70                  75                  80
Phe Gly Val Val His Ser Met Ala Ser Glu Asp Ser Ile Gln Ser Lys
                85                  90                  95
Met Gly Ile Arg Asn Cys Ser Asn Leu Phe Arg Gln Lys Ser Ser Pro
            100                 105                 110
Ala Ala Phe Phe Ser Ile Glu Asn Asp Leu Ala Ala Leu Arg Glu Val
        115                 120                 125
Gly Ser Phe Lys Ala Asp Asp Val Ser Asn Gly Leu Val Thr Ala Ser
    130                 135                 140
Thr Gly Gly Leu His Ser Ser His Thr Phe Ser Ser Arg Pro Ser Ser
145                 150                 155                 160
Cys Leu Lys Arg Leu Pro Gln Ile Ala Glu Asn Gly Asn Glu Ser Leu
                165                 170                 175
Glu Glu Asn Cys Asp Gln Ser Arg Asn Leu Val Asn Asp Asn Gly Ser
            180                 185                 190
Ser Lys Cys Tyr Ile Pro Ser Phe Thr Asn Glu Leu Trp Glu Ser Ser
        195                 200                 205
Ala Phe Asn Ala Pro Lys Thr Glu Asn Glu Asp Glu Ile Met Phe Ser
    210                 215                 220
Thr Ser Asn Ile Leu Glu Ser Gln Glu Ala Asp Phe Ser Phe Gln Asn
225                 230                 235                 240
Leu Gly Leu Thr His His Leu Ser Leu Pro Ser Ser Ser Thr Lys Met
                245                 250                 255
Ser Ser Ile Glu Lys Phe Leu Gln Ile Gln Gly Ser Val Pro Cys Lys
            260                 265                 270
Ile Arg Ala Lys Arg Gly Phe Ala Thr His Pro Arg Ser Ile Ala Glu
        275                 280                 285
Arg Val Arg Arg Thr Arg Ile Ser Glu Arg Ile Lys Lys Leu Gln Asp
    290                 295                 300
Leu Phe Pro Lys Ser Glu Lys Gln Thr Ser Thr Ala Asp Met Leu Asp
305                 310                 315                 320
Leu Ala Val Glu Tyr Ile Lys Asp Leu Gln Gln Lys Val Lys Ile Leu
                325                 330                 335
Ser Asp Cys Lys Ala Lys Cys Lys Cys Thr Ser Asn Glu Lys His Tyr
            340                 345                 350
```

Thr Arg Thr Cys Ala
        355

<210> 53
<211> 1451
<212> DNA
<213> Glycine max

<400> 53
gctgtgtcaa cgaggaatcg ttcacaagtg agaatcatca tcatcagcag cagcagcagc 60
agcagcatta tcttccatct acaagttcag aaatggagac catgttatcc aaattgctac 120
catccaacaa tggatggagc aattcagaag ctttgcaaga atttggaggc aagcctgtga 180
agcaggaaat aggggaatct attccacaag agccaccaca acagaatggt tattcttatg 240
gtggctctca gttgatttac caatctcagc aaattcaagg cttgccaaat ggtggatctt 300
ccagtgcttc tggcagtgct tttgatggct cttttggtgt ggtgcactcc atggcttcag 360
aggactccat tcaatctaaa atgggtatca ggaattgctc caatctcttt aggcaaaaga 420
gttcccctgc tgcatttttc tccattgaaa atgatcttgc agcattgaga gaagtaggaa 480
gctttaaagc cgatgatgtt tcaaatggac tagttactgc atcaactggt gggttgcata 540
gttctcatac tttctcatct aggccatctt catgcttgaa acggttgcca caaatagccg 600
aaaatggaaa tgaaagccta gaagaaaatt gtgaccaaag tagaaaccta gtaaatgaca 660
atggtagctc aaaatgttac atacctagct tcaccaatga attatgggaa agttctgcat 720
tcaatgcccc aaaaacagag aatgaagatg aaatcatgtt ttccacttca aatattttgg 780
aatctcagga agcagatttc agctttcaaa atctgggttt gactcaccat ttgagtctac 840
ctagctcttc tactaagatg tcatccatag aaaagtttct tcagattcaa ggttctgttc 900
cttgtaaaat tcgagccaaa agaggctttg ccactcaccc aagaagtatt gcagagaggg 960
taagaagaac aagaattagt gaaagaatca agaaactgca agaccttttt ccaaaatcag 1020
aaaagcaaac aagcacagca gatatgttag atttggcagt tgagtatatt aaggacctgc 1080
agcaaaaagt taagatactc tcagattgta aggcaaagtg caaatgtaca agtaatgaga 1140
agcattacac tagaacttgt gcttgattta tttgcatgta gatagcaaaa ggatggaaga 1200
tttttagtg tgccaaagtt agcaattgta attagataaa ataatgctga catttgcctg 1260
gagtgagttt aggaccaggt gaatttttt ttttactttt ttttttcatt ttttccctta 1320
atttcttttt cttcatagtt tgatttaagg atcataaaga cctagcatgt ttagcatgag 1380
ttttgtagct cttcattatt acatataaga atttatgttg aaatttatat ggagactgct 1440
tttgctcgtg c 1451

<210> 54
<211> 156
<212> PRT
<213> Glycine max

<400> 54
Met Pro Ser Phe Thr Thr Asp Phe Trp Asp Gly Ser Ala Phe Ser Ala
1               5                   10                  15
Ser Arg Thr Ala Ser Asn Arg Gly Glu Ile Ser Phe Ser Thr Ser Lys
            20                  25                  30
Ala Met Asp Ile Gln Asp Glu Asp Phe Gly Tyr Gln Lys Val Gly Leu
        35                  40                  45
Thr His His Leu Ser Leu Pro Gly Ser Ser Ser Arg Met Ala Thr Met
    50                  55                  60
Glu Lys Leu Tyr Gln Ile Gln Gly Ser Val Pro Cys Lys Ile Arg Ala
65                  70                  75                  80
Lys Arg Gly Phe Ala Thr His Pro Arg Ser Ile Ala Glu Arg Glu Arg
                85                  90                  95
Arg Thr Arg Ile Ser Ala Arg Ile Lys Lys Leu Gln Asp Leu Phe Pro
            100                 105                 110
Lys Ser Asp Lys Gln Thr Ser Thr Ala Asp Met Leu Asp Leu Ala Val
        115                 120                 125
Glu Tyr Ile Lys Asp Leu Gln Lys Gln Val Lys Ile Leu Arg Asp Thr
    130                 135                 140

```
Arg Ala Lys Cys Thr Cys Thr Ser Asn Gln Lys His
145                 150                 155

<210>  55
<211>  1376
<212>  DNA
<213>  Gossypium raimondii

<400>  55
gatggaaacc aaaaagaaaa aaggagagtt tttcttagct tcacaaagct aactcctcaa      60
gctgaacaaa gaagcagatg aaaccgacgc ctggcggcag tagcagctct agtggcggtg     120
gcgctgaagg gagtcgagct ggactcgccc gtttccgctc agctccagcc acttggttag     180
aagcccttct cgaggaagaa gaagaggacc ctttgaagcc tagccagtgc ttgactcagc     240
tactaactgg aaattcaacc tcaacaccca ccattcgaaa ttcattgctt tttcccaact     300
cagcctatcc atctgggttg tttgagcctt cgggtttcca gaggcaaaac agttctccag     360
ctgattttcc tgggaataat cctgctgctt cctctgatgc ctacttctct aactttggag     420
ttgttgcaaa ttatgactat ttatcgccaa ctatggatgt ttcccccctcg agtaagaggg    480
ctagcgagct tgatacgcaa ttcccaccaa caaagttcca ttctcagttg aaaggagagc     540
caagtgatca gatttctggt gggatatcta atcttataga tgtagatatg gagaaacttt     600
tggaggattc ggtaccttgc agggtccgtg cgaagcgagg gtgtgctact catcctcgaa     660
gtattgcaga aagggttcgg agaacacgga ttagcgatcg cattagaaag cttcaggagc     720
ttgtccccaa catggataag caaaccaacc cggcggacat gctggaagaa gcggtggagt     780
atgttaaata tcttcaaagg cagattcagg aactcacaga gcatcaaaag agatgcaaat     840
gcaaagctaa agattgatcg actaccaaga gcttttagtg taaaccgtct cgcctacagt     900
gataaagttg ggtggtagat cccccctgta attactagat ttccccccta cgatgttgca     960
tttttcagca tgcaatacct agatgtggct gcttttgaag ctaactatag ccctcatatc    1020
aggacctctg ctttgctttg cctagtactg ctcctggaag atcaattggt ataattgtac    1080
ttgtccaata ttgacgtaga ctataaatat ttttttatta actatatagc tataatatat    1140
atatatatat atatatatcc catttttaat taagatattt gtttctccat aattcatatt    1200
gtatatttag ataattgtat tcagtgaagt tctttatttc cattttaaga atcggtgagg    1260
atctgtgttc gaggtttcag atatttgttg ttgacctggg aatggcatga tcccaggaaa    1320
agaaattgct tgctttgtat ggtgattttt ttacaaattc agatgataac aatggc         1376

<210>  56
<211>  259
<212>  PRT
<213>  Gossypium raimondii

<400>  56
Met Lys Pro Thr Pro Gly Gly Ser Ser Ser Ser Ser Gly Gly Gly Ala
1               5                   10                  15
Glu Gly Ser Arg Ala Gly Leu Ala Arg Phe Arg Ser Ala Pro Ala Thr
            20                  25                  30
Trp Leu Glu Ala Leu Leu Glu Glu Glu Glu Glu Asp Pro Leu Lys Pro
        35                  40                  45
Ser Gln Cys Leu Thr Gln Leu Leu Thr Gly Asn Ser Thr Ser Thr Pro
    50                  55                  60
Thr Ile Arg Asn Ser Leu Leu Phe Pro Asn Ser Ala Tyr Pro Ser Gly
65                  70                  75                  80
Leu Phe Glu Pro Ser Gly Phe Gln Arg Gln Asn Ser Ser Pro Ala Asp
                85                  90                  95
Phe Pro Gly Asn Asn Pro Ala Ala Ser Ser Asp Ala Tyr Phe Ser Asn
            100                 105                 110
Phe Gly Val Val Ala Asn Tyr Asp Tyr Leu Ser Pro Thr Met Asp Val
        115                 120                 125
Ser Pro Ser Ser Lys Arg Ala Ser Glu Leu Asp Thr Gln Phe Pro Pro
    130                 135                 140
Thr Lys Phe His Ser Gln Leu Lys Gly Glu Pro Ser Asp Gln Ile Ser
145                 150                 155                 160
```

```
Gly Gly Ile Ser Asn Leu Ile Asp Val Asp Met Glu Lys Leu Leu Glu
                165                 170                 175
Asp Ser Val Pro Cys Arg Val Arg Ala Lys Arg Gly Cys Ala Thr His
            180                 185                 190
Pro Arg Ser Ile Ala Glu Arg Val Arg Arg Thr Arg Ile Ser Asp Arg
        195                 200                 205
Ile Arg Lys Leu Gln Glu Leu Val Pro Asn Met Asp Lys Gln Thr Asn
    210                 215                 220
Pro Ala Asp Met Leu Glu Glu Ala Val Glu Tyr Val Lys Tyr Leu Gln
225                 230                 235                 240
Arg Gln Ile Gln Glu Leu Thr Glu His Gln Lys Arg Cys Lys Cys Lys
                245                 250                 255
Ala Lys Asp


<210>  57
<211>  680
<212>  DNA
<213>  Glycine soja

<400>  57
tggcaatgat ggtgaactta gtctatctat gaacagaatg aagaatcaca ttagcttctc      60
atcaataagt ccttcttctt ccttgggaat gctatcacca acctccaaaa tgggcacgga     120
gggtattagg gtaactagac ctgaggatgg gagacaggga agttgcaatg gtgatgctcg     180
atattatggt cctggattcc cttatgcttc ttggaatgag ccctcacacc ccaaaagaca     240
gcgaagtagt aatgatgagt tactttttga ttctcagaat ggagagccag gaaatcaagt     300
tcaaaggctt tcgcatcatt tgagtttgcc aagaacgtcg tcggagatgt ttgctatgga     360
taatttgctt cagttctcag attctgttcc ttgtaaaatc agagcaaaga gaggctttgc     420
tactcatcct cgaagcattg ccgaaagggt gagaagaaca aggatcagtg aacgaataag     480
aaaattgcaa gagcttgttc caaccatgga caagcaaact agcacagcag agatgttgga     540
cttggcttta gactacataa aagatcttca gaaacaattc aagactctga gcgataaacg     600
ggctaaatgc aaatgtataa acatgcagaa gtcagaagca gatcgagttg cttgagtttc     660
ttttgtacag ggctgccagg                                                 680

<210>  58
<211>  208
<212>  PRT
<213>  Glycine soja

<400>  58
Met Asn Arg Met Lys Asn His Ile Ser Phe Ser Ser Ile Ser Pro Ser
1               5                   10                  15
Ser Ser Leu Gly Met Leu Ser Pro Thr Ser Lys Met Gly Thr Glu Gly
            20                  25                  30
Ile Arg Val Thr Arg Pro Glu Asp Gly Arg Gln Gly Ser Cys Asn Gly
        35                  40                  45
Asp Ala Arg Tyr Tyr Gly Pro Gly Phe Pro Tyr Ala Ser Trp Asn Glu
    50                  55                  60
Pro Ser His Pro Lys Arg Gln Arg Ser Ser Asn Asp Glu Leu Leu Phe
65                  70                  75                  80
Asp Ser Gln Asn Gly Glu Pro Gly Asn Gln Val Gln Arg Leu Ser His
                85                  90                  95
His Leu Ser Leu Pro Arg Thr Ser Ser Glu Met Phe Ala Met Asp Asn
            100                 105                 110
Leu Leu Gln Phe Ser Asp Ser Val Pro Cys Lys Ile Arg Ala Lys Arg
        115                 120                 125
Gly Phe Ala Thr His Pro Arg Ser Ile Ala Glu Arg Val Arg Arg Thr
    130                 135                 140
Arg Ile Ser Glu Arg Ile Arg Lys Leu Gln Glu Leu Val Pro Thr Met
```

```
145                 150                 155                 160
Asp Lys Gln Thr Ser Thr Ala Glu Met Leu Asp Leu Ala Leu Asp Tyr
                165                 170                 175
Ile Lys Asp Leu Gln Lys Gln Phe Lys Thr Leu Ser Asp Lys Arg Ala
            180                 185                 190
Lys Cys Lys Cys Ile Asn Met Gln Lys Ser Glu Ala Asp Arg Val Ala
        195                 200                 205

<210>  59
<211>  700
<212>  DNA
<213>  Helianthus ciliaris

<400>  59
ggatttaga aacgttatac tactgacaat ggttacccta tgatgagatc aatcgaagaa     60
tttcgattat tgtcacgtat ccctgaacac gagggaaaaa acaactaccc tggtggttct    120
tgggacgatt cggcaatgtt gactgatggg tttcttaacg aatttgaaga aagtcaagaa    180
aaattgaatg atggagggag gataactcaa gcaactcatg ctcccactgg gttgactcat    240
cagctgagtt tgccaactga gttgtctgtg aaggagaaac taatgcattt tcaagataat    300
gtgccactaa ggagcagggc taaaaggggt tgtgccactc atccaagaag cattgctgaa    360
agggtcaggc ggacacgaat aagtgaaaga atgaggaagc tgcaagatct tgtcccaaat    420
atggataagc aaacgagcac ggcagacatg ttggatttgg ctgttgagta tatcaaagaa    480
cttcagaatc aagttgagac tctttcagac cgtcaacgaa agtgcacatg tccacataag    540
ctggagtcgt aacacaagtt taagcatagt tatttctcgc ttttgtatat aatacatata    600
aattcaagta atcgaagaaa tagaagattc ttgtttggta atttaactcc gtttgttatg    660
taacatttag catattctta attctctagc cattctaaat                          700

<210>  60
<211>  170
<212>  PRT
<213>  Helianthus ciliaris

<400>  60
Met Met Arg Ser Ile Glu Glu Phe Arg Leu Leu Ser Arg Ile Pro Glu
1               5                   10                  15
His Glu Gly Lys Asn Asn Tyr Pro Gly Gly Ser Trp Asp Asp Ser Ala
            20                  25                  30
Met Leu Thr Asp Gly Phe Leu Asn Glu Phe Glu Glu Ser Gln Glu Lys
        35                  40                  45
Leu Asn Asp Gly Gly Arg Ile Thr Gln Ala Thr His Ala Pro Thr Gly
    50                  55                  60
Leu Thr His Gln Leu Ser Leu Pro Thr Glu Leu Ser Val Lys Glu Lys
65                  70                  75                  80
Leu Met His Phe Gln Asp Asn Val Pro Leu Arg Ser Arg Ala Lys Arg
                85                  90                  95
Gly Cys Ala Thr His Pro Arg Ser Ile Ala Glu Arg Val Arg Arg Thr
            100                 105                 110
Arg Ile Ser Glu Arg Met Arg Lys Leu Gln Asp Leu Val Pro Asn Met
        115                 120                 125
Asp Lys Gln Thr Ser Thr Ala Asp Met Leu Asp Leu Ala Val Glu Tyr
    130                 135                 140
Ile Lys Glu Leu Gln Asn Gln Val Glu Thr Leu Ser Asp Arg Gln Arg
145                 150                 155                 160
Lys Cys Thr Cys Pro His Lys Leu Glu Ser
                165                 170

<210>  61
<211>  625
<212>  DNA
```

<213> Helianthus paradoxus

<220>
<221> misc_feature
<222> (496)..(496)
<223> n is a, c, g, or t

<400> 61
acgtgctgaa aagcgcttat gatttcttta gaccacactt acaaatgaga agaaaacact 60
cacgttactt aggtgtgatc tttgttgatt tcaaaatgga ttatcctcac cttaaacacc 120
cagtattgcc aaccctttac agcttgactc aaaacttcac tctgacatgg aatttgacga 180
caagatcctt gcgacttgaa ctacacttgc atcctacaac gagtcctaca ccacaatcaa 240
gcgccacaca cccgccaagc atagctgaga gggtgagaag aactcgaata agtgatagaa 300
tgaagaagct tcaagagctc ttccctaaca tggacaagca aacaagcaca gcagacatgt 360
tagatatggc tgttcagtac attaaagatc tccagaaaga attggagact ctcagagatg 420
cgcggtcaaa gtgcgagtgt tcaaggaaac aataacatat gttggatcat ataacttgct 480
ttctgatacg tcttanaatg gaaaaaagac gagtgtagtt taggtttatt cgatcaaaca 540
ttctacttcg agctgtattg cccgtggcga ctctcatcat caaagtgtgg atcattgtga 600
ttatcatttg taactcatga agtag 625

<210> 62
<211> 136
<212> PRT
<213> Helianthus paradoxus

<400> 62
Met Arg Arg Lys His Ser Arg Tyr Leu Gly Val Ile Phe Val Asp Phe
1 5 10 15
Lys Met Asp Tyr Pro His Leu Lys His Pro Val Leu Pro Thr Leu Tyr
20 25 30
Ser Leu Thr Gln Asn Phe Thr Leu Thr Trp Asn Leu Thr Thr Arg Ser
35 40 45
Leu Arg Leu Glu Leu His Leu His Pro Thr Thr Ser Pro Thr Pro Gln
50 55 60
Ser Ser Ala Thr His Pro Pro Ser Ile Ala Glu Arg Val Arg Arg Thr
65 70 75 80
Arg Ile Ser Asp Arg Met Lys Lys Leu Gln Glu Leu Phe Pro Asn Met
85 90 95
Asp Lys Gln Thr Ser Thr Ala Asp Met Leu Asp Met Ala Val Gln Tyr
100 105 110
Ile Lys Asp Leu Gln Lys Glu Leu Glu Thr Leu Arg Asp Ala Arg Ser
115 120 125
Lys Cys Glu Cys Ser Arg Lys Gln
130 135

<210> 63
<211> 883
<212> DNA
<213> Helianthus petiolaris

<400> 63
atagagatca aaccaaatgc aacccacaca cggtggcact agcggcggct ccggtggaac 60
cggtaccgga ctttccaggt tccggtcagc tccggcaaca tggctagaag ctctactgga 120
atctgaagaa gaagacgtca tcattgatcc acccaaacca accttaacac ctcctattgt 180
tcctcgtgtt catcaacaca cttccattgc aacaacaaca agtgacacaa aacctacgtt 240
tgttgatccg aatcttctca tacctaatcc catcggtctc cgacaaaaca gttctcctgc 300
tgagtttctt tctcagatca acaatcccga tgcgtttcta cctaactact caactgctgg 360
ttttgatgat tatctttcat cttctagaga catcggtggt gggtctaagt tcaccactca 420
gctgagtgga gatcaaagta tgttgttgga gggtgatatg gataagatgt tgggggattc 480

```
tgtgccgtgt agagtgcggg cgaagcgcgg gtgtgcgact catcctcgga gtatcgcgga    540
gcgggttagg aggactcgga ttagtgacag aataaggaag cttcaagaac tggttccaaa    600
tatggataag caaacgaata cagcagatat gttagacgag gcggtggagt atgtcaagtt    660
tctgcaacga cagattcagg aacttaggga gcatcaagac aaatgcacat gtcagtcaat    720
atcaacatga agaaatgact caatggtgtt tatgttgtaa acatcgctcc ttttgtgcta    780
tagtgcttct tccaacacct gagatcaaat tattattttt tttgtttcgt gagtcgtgta    840
atactattag aaacgagtag attagggtta tatatgcgat gct                      883
```

<210> 64
<211> 237
<212> PRT
<213> Helianthus petiolaris

<400> 64

```
Met Gln Pro Thr His Gly Gly Thr Ser Gly Gly Ser Gly Gly Thr Gly
1               5                   10                  15
Thr Gly Leu Ser Arg Phe Arg Ser Ala Pro Ala Thr Trp Leu Glu Ala
            20                  25                  30
Leu Leu Glu Ser Glu Glu Glu Asp Val Ile Ile Asp Pro Pro Lys Pro
        35                  40                  45
Thr Leu Thr Pro Pro Ile Val Pro Arg Val His Gln His Thr Ser Ile
    50                  55                  60
Ala Thr Thr Thr Ser Asp Thr Lys Pro Thr Phe Val Asp Pro Asn Leu
65                  70                  75                  80
Leu Ile Pro Asn Pro Ile Gly Leu Arg Gln Asn Ser Ser Pro Ala Glu
                85                  90                  95
Phe Leu Ser Gln Ile Asn Asn Pro Asp Ala Phe Leu Pro Asn Tyr Ser
            100                 105                 110
Thr Ala Gly Phe Asp Asp Tyr Leu Ser Ser Ser Arg Asp Ile Gly Gly
        115                 120                 125
Gly Ser Lys Phe Thr Thr Gln Leu Ser Gly Asp Gln Ser Met Leu Leu
    130                 135                 140
Glu Gly Asp Met Asp Lys Met Leu Gly Asp Ser Val Pro Cys Arg Val
145                 150                 155                 160
Arg Ala Lys Arg Gly Cys Ala Thr His Pro Arg Ser Ile Ala Glu Arg
                165                 170                 175
Val Arg Arg Thr Arg Ile Ser Asp Arg Ile Arg Lys Leu Gln Glu Leu
            180                 185                 190
Val Pro Asn Met Asp Lys Gln Thr Asn Thr Ala Asp Met Leu Asp Glu
        195                 200                 205
Ala Val Glu Tyr Val Lys Phe Leu Gln Arg Gln Ile Gln Glu Leu Arg
    210                 215                 220
Glu His Gln Asp Lys Cys Thr Cys Gln Ser Ile Ser Thr
225                 230                 235
```

<210> 65
<211> 1454
<212> DNA
<213> Hordeum vulgare

<400> 65

```
cccacttgca ccacccccgc cccgcatagg ataggccacg ccaatattcc acgccaaaac     60
acacgcgcag gccagcgata gccccggctc ggccgtcgac gacccacgcc accccgtcga    120
tcgatccacc agtacgtgtc cggccgctcg tcgacgcatg ctgatgcagc tgcgccggaa    180
gcgggcttgt ttttgctaac cgcacgatcg acatgaggag gttcttgccg gcgggagcag    240
gggagccctc ctcctcgtcg tcgtcggggc cgcacgggaa gcacgagggc agcgaggccg    300
ccgccgctgc tggtggtctg cggtatggag gcggagacat ctcgctaggg cgcggcaacg    360
acctcctcca cgcccagttt cgcggcggcg agggggagat gaaggacgac ggagcggaca    420
tgctggcccg gcacagtagc tcgccggcgg ggttcttctc caacctcatg gtggatgacg    480
```

```
gatatcatgg ctcgagaggc gccggagtag ctggtggcag cggcgaagcc caccgtaaca    540
ccagtagcag cacgaagatg aaatcccagc tgagcttcac gagcggcggg ccacagactg    600
ccgcgcacct ctcgcgtatc tccgagggcg cctccttatt ccccggcgcc gacgtcgtcc    660
gcgctgctgc gcactcgggc ggcgagcacc ccgtgtcgcg ttccttctcg gccagcggca    720
gtagcggggg cttctccatc gtggggccgt gggatgagtc gagggagatc atcggcaccc    780
tcgatctcgg tggttacgag tctcagttta gtggcatggc gagctcgtcg tcgctggagc    840
tggccgggat ggacaagtac atgcaggcgc agcagcagca ggaccaggtt gggttcaagg    900
tgcgggccaa gcgcggctgc gcgacgcacc cgaggagcat cgctgagagg gaacgcagga    960
cgaggatcag cgagaagctc aggaagctgc aggacctagt gcccaacatg gacaagcaaa   1020
cgagcacctc ggacatgttg gacctcgcgg ttgagcacat caggggcctc cagagccagc   1080
tgcaggtcat gaagcacgag caggacaaat gtacctgctg cagcaagcct tgagcaattt   1140
agcaggggac cagtaactga tcagcacaac gcaatggatg acaccacctt tagtcgagag   1200
aaatgcacat gacgcatgaa ttagtgcatg ccaatgctaa atgagtacat gcagctcaac   1260
ggttcatact ttctcaaggc ggcaaggaac tggcagccgt acgttctcat ggacccttca   1320
acttgttccc agtcattggc cataattatc atgaagcagg tagctggtag attaggtacc   1380
tcttccgttt ccacgaaaac gtgaattttg gttctaactg ctgagactga gaatttttat   1440
tctatattct tcat                                                     1454
```

<210> 66
<211> 196
<212> PRT
<213> Hordeum vulgare

<400> 66

```
Met Ser Gln Ile Ser Glu Met Val Gly Glu Glu Met Gly Gly Gly Gly
1               5                   10                  15
Ser Ser Gly Asp Asp Asp Glu Ala Gly Ser Tyr Gly Gly Ile Pro Gly
            20                  25                  30
Tyr Pro Val Val Ala Pro Ser Gly Thr Gly Trp Asp Glu Pro Ser Pro
        35                  40                  45
Ser Pro Pro Pro Ser Leu Leu Thr Ser Asp Gly Met Ser Gly Pro Ala
    50                  55                  60
Ala Lys Arg Arg Pro Arg Glu Ala Ala Asn Gly Arg Ser Gly Gln Leu
65                  70                  75                  80
Lys Pro Gln Phe Ser Leu Pro Ala Gly Ser Lys Pro Ser Pro Glu Ile
                85                  90                  95
Ala Ala Ile Glu Lys Phe Leu Gln Phe Gln Asp Ser Val Pro Cys Lys
            100                 105                 110
Ile Arg Ala Lys Arg Gly Cys Ala Thr His Pro Arg Ser Ile Ala Glu
        115                 120                 125
Arg Val Arg Arg Thr Lys Ile Ser Glu Arg Ile Arg Lys Leu Gln Glu
    130                 135                 140
Leu Val Pro Asn Met Glu Lys Gln Thr Asn Thr Ser Asp Met Leu Asp
145                 150                 155                 160
Leu Ala Val Asp Tyr Ile Lys Glu Leu Gln Met Gln Val Lys Val Met
                165                 170                 175
Asn Asp Gly Arg Ala Gly Cys Thr Cys Ser Ala Gly Arg Pro Gln Lys
            180                 185                 190
His Phe Ala Gly
        195
```

<210> 67
<211> 2160
<212> DNA
<213> Hordeum vulgare

<220>
<221> misc_feature
<222> (10)..(10)

<223>  n is a, c, g, or t

<400>  67
cggcacgagn gccccctttc cgcccaaaca aaccgccccc tccccctccc cctcccgtcg    60
ccctcacctc ctccttcgcc ggcagtatac tactaccact ccccgccacc agctccgacg   120
acccgcgcct tccctcccgc ccgcccgcgc accgtcgtct cccgtccccc cgccgcgggt   180
ttgttctaga gggtgtattt gtataaaagg aattcaggcc atcaaagact gtcttacaat   240
atcaagttaa attccctttt catggatgag tgataggatc ttcagttggc cactttaagt   300
atctagaagc cactcttttc ttaatatttc tgagcagagg ctgtgaagtc aaattgtaag   360
ggtgtaagat gaactactct gatggttctt tctttccttc atggcctggc aattccgctt   420
ctgagaacta tagcttcgtt gatggttcag tggaatcata tacagaagaa ggaagtatgc   480
caactacagg ctatttcaga gctagatcaa atcagaattt aacatttgat gagcatgaac   540
agaaccctgc tatgcttgca aatggatgct tgccatacaa cactcagact gatctattat   600
ctggcgagat tctatcagac gacaagcctt caaacagcct tgtggagctt tcacaacttc   660
agaacaatgt ctgtctgcaa aataatttaa ttcccccagg gactcttcag tgcaattcaa   720
cacctggaac ttttgacctg cagttggata cccctggcct tctggaacta cctcatgcct   780
tgtccagttc aattgaaagc aatggtagtg aagtttcagc ttttcttgct gatgtacaag   840
ctgtttcttc agcctcgact ctgtgttcca catttcaaaa tgttccttcc tacatggaac   900
cagtaagcct agaagctttc agttttcaag ggatgcaaaa tgctgctatg ttcaacaatg   960
caagtcattc aaatgggaac ctgtcagtat ttgatgaggc aaccatggca tcactacatg  1020
atagcaaaga gtttctcaat ggtagcatct catctcttgg taccggccag caatcacaac  1080
tagctggtgg tggtttgaag gctgaacaac aggagcaaaa tacaatgtgc aatattccgc  1140
tcccttcttt cgtttctgct agtcagatgg cagtgagtga agcacaaggg gcactgattc  1200
cttcaaagac aacatcaata acccataaca ataaaagtga gtaccctatc cctatcagcc  1260
attctgctga tgtgcagcat aaggcaaatt caggtaatgg aaatagtgcc agtgctaaac  1320
cacgagcaag agctcgacgt ggacaggcaa ctgaccctca tagtattgct gaacggcttc  1380
gcagagagaa gatctcagag aggatgaaaa atctccaaga ccttgtacca aactcaaata  1440
aggcagataa gtcatcaatg ctcgatgaaa taattgatta tgtgaaattt cttcagcttc  1500
aggtgaaggt cttaagcatg agtaggctag gagctcctgg ggcagttctt cccctcctcg  1560
cagaatctca gactgagggc cgtagcaatt cacctctatc atctccaacc acctcacaag  1620
ggctactgga cgtagcaggc ccagaagata gcttggtgtt tgagcaagaa gttataaagc  1680
tgatggaaac aagcatcacc aacgcaatgc agtaccttca gaacaagggc ctctgcctga  1740
tgcctatcgc tcttgcttca gccatatcca accagaaagg cacttctgca gctgcaatcc  1800
ctcctgaaag gtgaaaacag aaagcacatc atgctcctcc catcggcacg cggaaacaac  1860
tgtcgaaatg tgctagagtt aatagaagtt atgagaaaac taaaatccga gggtgaagga  1920
taggaagtta tgcgactaat agtacaagct tatggtataa tctagtagct tactccaaga  1980
aacctatact ttttctattc tctgttgcac atcagttgat gtgtattctt ttgttttttg  2040
gacggcattg ctaacttggt acaagtgtct ggcaccttgg gttgtagatg gatctatgag  2100
gtttattacc gactgttcat gcaggttaca gctttactaa atctgttttt ccttattgct  2160

<210>  68
<211>  306
<212>  PRT
<213>  Hordeum vulgare

<400>  68
Met Arg Arg Phe Leu Pro Ala Gly Ala Gly Glu Pro Ser Ser Ser Ser
1               5                   10                  15
Ser Ser Gly Pro His Gly Lys His Glu Gly Ser Glu Ala Ala Ala Ala
            20                  25                  30
Ala Gly Gly Leu Arg Tyr Gly Gly Gly Asp Ile Ser Leu Gly Arg Gly
        35                  40                  45
Asn Asp Leu Leu His Ala Gln Phe Arg Gly Gly Glu Gly Glu Met Lys
    50                  55                  60
Asp Asp Gly Ala Asp Met Leu Ala Arg His Ser Ser Ser Pro Ala Gly
65                  70                  75                  80
Phe Phe Ser Asn Leu Met Val Asp Asp Gly Tyr His Gly Ser Arg Gly
                85                  90                  95
Ala Gly Val Ala Gly Gly Ser Gly Glu Ala His Arg Asn Thr Ser Ser

```
            100                     105                     110
Ser Thr Lys Met Lys Ser Gln Leu Ser Phe Thr Ser Gly Gly Pro Gln
        115                     120                     125
Thr Ala Ala His Leu Ser Arg Ile Ser Glu Gly Ala Ser Leu Phe Pro
    130                     135                     140
Gly Ala Asp Val Val Arg Ala Ala Ala His Ser Gly Gly Glu His Pro
145                     150                     155                     160
Val Ser Arg Ser Phe Ser Ala Ser Gly Ser Ser Gly Gly Phe Ser Ile
                165                     170                     175
Val Gly Pro Trp Asp Glu Ser Arg Glu Ile Ile Gly Thr Leu Asp Leu
            180                     185                     190
Gly Gly Tyr Glu Ser Gln Phe Ser Gly Met Ala Ser Ser Ser Ser Leu
        195                     200                     205
Glu Leu Ala Gly Met Asp Lys Tyr Met Gln Ala Gln Gln Gln Gln Asp
    210                     215                     220
Gln Val Gly Phe Lys Val Arg Ala Lys Arg Gly Cys Ala Thr His Pro
225                     230                     235                     240
Arg Ser Ile Ala Glu Arg Glu Arg Arg Thr Arg Ile Ser Glu Lys Leu
                245                     250                     255
Arg Lys Leu Gln Asp Leu Val Pro Asn Met Asp Lys Gln Thr Ser Thr
            260                     265                     270
Ser Asp Met Leu Asp Leu Ala Val Glu His Ile Arg Gly Leu Gln Ser
        275                     280                     285
Gln Leu Gln Val Met Lys His Glu Gln Asp Lys Cys Thr Cys Cys Ser
    290                     295                     300
Lys Pro
305
```

<210> 69
<211> 786
<212> DNA
<213> Ipomoea nil

<220>
<221> misc_feature
<222> (775)..(775)
<223> n is a, c, g, or t

<400> 69

```
caggcgcaat ggcgtctata tctgaaattg gagaagaggg actgaaagaa gaaaccagtc     60
ccagggcatg gaggatttag tgaaactcag aaaaatgatg agttctccat gccatattgg    120
gaagattcag acattttgtc tgatcttttc ctaaatggat cagaagaaaa gccattttca    180
aatccaaatg tatcagatat tcagagcgaa gatcaaactc gccctcctgg cctgctgtct    240
catcacttaa gtttgcccaa agttcctgcc cagttatctg ccataatgca agattcagtg    300
ccctgtaagg tcagagcaaa gaggggttgt gcaactcacc ctcgcagtat cgctgagagg    360
gttagaagaa ccaaaataag tgagaggatg agaaagctgc aagagctcgt cccaaacatg    420
gacaagcaaa caaacacagc agatatgttg gacttagctg ttgactacat caaagattta    480
gagtcccagg tccaggtgtt atcggaaaac cgggctaaat gtacatgctc atcaagtaaa    540
tagaaagtca cagacataga ggaccaggta gggcacacat ctgcaaatta atggactgtc    600
aaaagtatat gagaatgggg tagcattttg gttggtaaaa tgagcatata gactaagctg    660
tatataaaac cattaatctg tattttttgg gttggtctct tttgttagcc ataaaaagga    720
ataccaagga ggaggaggag ggctttgata tccatatcct ccaccttacc tttcngaaaa    780
aaattc                                                                786
```

<210> 70
<211> 144
<212> PRT
<213> Ipomoea nil

<400> 70
Met Pro Tyr Trp Glu Asp Ser Asp Ile Leu Ser Asp Leu Phe Leu Asn
1 5 10 15
Gly Ser Glu Glu Lys Pro Phe Ser Asn Pro Asn Val Ser Asp Ile Gln
20 25 30
Ser Glu Asp Gln Thr Arg Pro Pro Gly Leu Leu Ser His His Leu Ser
35 40 45
Leu Pro Lys Val Pro Ala Gln Leu Ser Ala Ile Met Gln Asp Ser Val
50 55 60
Pro Cys Lys Val Arg Ala Lys Arg Gly Cys Ala Thr His Pro Arg Ser
65 70 75 80
Ile Ala Glu Arg Val Arg Arg Thr Lys Ile Ser Glu Arg Met Arg Lys
85 90 95
Leu Gln Glu Leu Val Pro Asn Met Asp Lys Gln Thr Asn Thr Ala Asp
100 105 110
Met Leu Asp Leu Ala Val Asp Tyr Ile Lys Asp Leu Glu Ser Gln Val
115 120 125
Gln Val Leu Ser Glu Asn Arg Ala Lys Cys Thr Cys Ser Ser Ser Lys
130 135 140

<210> 71
<211> 762
<212> DNA
<213> Ipomoea nil

<400> 71
tgttcggggg atggcgtcgg gggcttcaat gaagatcaga caatggatga aagttacttg 60
tcaagtttcc ctataccttc ttgggatgat ttagatctct tgtctgatga cttccttaaa 120
gtaacagatg aaaagccaag tttgaaagca aaagcaccag attttaagaa cacagaaggt 180
agtcgaagtc gccctcccgg ccgtttatct catcacctca gtttacccaa aagctccgag 240
gagttatcgt ccatattgca ggattctgtg ccttgcaggg tccgggcaaa gagaggttgc 300
gctactcacc ctcgcagcat tgccgagagg gttagaagaa ctcgaataag cgacagaatg 360
aggaagctgc aagagctcgt cccgaacatg gacaagcaaa caaacacagc agatatgttg 420
gacctggcag tggattacat taaagatcta gagcagaaag tgaagaggtt agctgataat 480
cgtgccaagt gtagatgctc gaataagtag tagggcaggg atgtaccgat gaatgtaacg 540
tggaaagttg atgaacattg tgggattcct cctggcacta aattaatcac agatcaaaag 600
aagaaaatgg gatgggagtg ccacaaaaac aatttcattt gatgtaacat atgaagcgct 660
gttgaacata aaagcattaa tgtttgtgtt gggtgtaccc ctctttttgt ccataaataa 720
tgaaaagaat ttggtaggct cgagtgtcct ttacacgcta tt 762

<210> 72
<211> 159
<212> PRT
<213> Ipomoea nil

<400> 72
Met Pro Ser Ile Ala Glu Asn Glu Ser Trp Asn Asp Ser Ser Phe Asn
1 5 10 15
Cys Leu Lys Arg Ser Arg Asp Gly Asp Phe Lys Met Leu Ser Ala Leu
20 25 30
Asn Gly Ile Glu Ser Gln Asn Gly Gly Glu Arg Arg Asn Cys Thr Pro
35 40 45
Gly Leu Thr His His Leu Ser Leu Pro Ser Ser Val Glu Ile Glu Lys
50 55 60
Tyr Leu His Phe Gln Gln Asp Ala Val Pro Cys Lys Phe Arg Ala Lys
65 70 75 80
Arg Gly Cys Ala Thr His Pro Arg Ser Ile Ala Glu Arg Asn Arg Arg
85 90 95
Thr Arg Ile Ser Glu Arg Met Lys Lys Leu Gln Glu Leu Phe Pro Lys

```
            100                 105                 110
Met Asp Lys Gln Thr Ser Thr Ala Asp Met Leu Asp Trp Ala Val Asp
        115                 120                 125
His Ile Lys Glu Leu Gln Lys Gln Val Gln Ile Leu Thr Asp Lys Lys
    130                 135                 140
Ala Lys Cys Thr Cys Ser Ser Glu Ala Gln Lys Gly Gly Met Leu
145                 150                 155

<210>  73
<211>  1337
<212>  DNA
<213>  Ipomoea nil

<220>
<221>  misc_feature
<222>  (1255)..(1255)
<223>  n is a, c, g, or t

<220>
<221>  misc_feature
<222>  (1302)..(1302)
<223>  n is a, c, g, or t

<220>
<221>  misc_feature
<222>  (1310)..(1310)
<223>  n is a, c, g, or t

<400>  73
gatgcatatt ctctctctct ctctctctct ctcagctctc agacaagttt tcctgacaag     60
acatattgcg ttaattacag tatattagaa aatatatagt acgagaaacc ggcgatcagc    120
ttatgggtag cagtatgctt ttcccgccgg ttcaattgca gggtttgatg aactatgacg    180
atcagaatct catccagatt ctggagaatt atggctggat tgaacggccc tcagacattg    240
gaatcaatta cgcactttcc agctgctcac cagccgccac ctctgcagct gggaaaaaga    300
aagatgacgc ttttccgccg gaaaatctgg gaagagagag cttcaagagg agaaaaaccg    360
acaaggcagt tgcagttgca gaagaggaaa gaaaggacag gtggaagcag aaaggatgcc    420
aagaagatgt cagaagttca gagcagactg acaacaatag taagaaagaa catagcactg    480
acacttccaa ggtaacacaa gacaaaaagt cagactacat tcatgtccgg gcacgacggg    540
gccaagccac tgacagtcat agtttagcag aaagagtgag gagggaaaaa atcagtgaga    600
gaatgagata cctgcaggat ctagtaccag ggtgcaataa gatcactggg aaagcgggaa    660
tgctagatga gataatcaac tatgtccagt cactgcaaaa acaagtagag ttcttgtcaa    720
tgaaactggc tgctgttaat ccaagactcg atttcaatat tgacagttta ttcaccaaag    780
agatgtttca atctagtcca tctaattttc ccccggttgg aataatatca gatgtgtcta    840
ctccttccaa tctgctgcaa ttagttgcat ctttgggatt ggaaataggc ataagtcctt    900
cagatatgcc tcttagaaca accattaatt ctcctgtctc actacccaaa acactcctca    960
attcgtccgc tataaatcag ggacagacct gtccaatttg ggatggagag ttacaaaacc   1020
tgtatgccat ggaggttcaa caaggaagat caccatcctt cacttcacag ccatttatgg   1080
gttttgtaga aggcagtaat ccaaggatgg agatctgagc cggaattcag attcttgaat   1140
cagttctttc agaagttacc aaatactagc tgattctttc ttccaagtac ataaacatat   1200
atgtctgtat gtatacataa ggtgtacaaa tgtcatactg gcagtatgct atcantattc   1260
aactctcata ttttataaaa agagaagctt ttgtatatac anatatatan tatatatata   1320
tatcgaacca agaaacc                                                   1337

<210>  74
<211>  277
<212>  PRT
<213>  Ipomoea nil

<400>  74
```

```
Met Gln Pro Glu Ser Gly Ala Gly Gly Gly Gly Ser Glu Pro Ser Arg
1               5                   10                  15
Gly Ala Gly Leu Thr Arg Phe Arg Ser Ala Pro Ala Thr Trp Leu Glu
            20                  25                  30
Ala Leu Leu Asp Ser Asp Thr Glu Asn Asp Val Val Leu Asp Pro Pro
        35                  40                  45
Pro Ile Leu Pro Ser Ser Ser Lys Pro Pro Pro His Pro His Ser Gln
    50                  55                  60
Pro Leu Pro Thr Pro Pro Ala Gln Leu Lys Gln Ala Ser Val Gly Gly
65                  70                  75                  80
Ser Gly Ser Arg Tyr Ala Ala Asp Leu Gly Leu Phe Glu Ser Gly Gly
                85                  90                  95
Gly Gly Gly Glu Ala Ala Ser Gly Leu Ser Asn Phe Val Arg Gln Asn
            100                 105                 110
Ser Ser Pro Ala Glu Phe Leu Ser Gln Ile Ser Ser Asp Ile Phe Phe
        115                 120                 125
Pro Ser Phe Gly Ala Pro Pro Ser Tyr Asp Tyr Leu Ser Ser Pro Met
    130                 135                 140
Asp Val Ala Gln Ser Ala Lys Arg Pro Arg Glu Ala Asp Ser Gln Ser
145                 150                 155                 160
Pro Ser Ala Lys Leu Ser Ser Pro Leu Lys Gly Glu Gln Ser Gly Arg
                165                 170                 175
Leu Arg Ser Ala Gly Gly Ser Leu Asp Ala Glu Met Glu Lys Met Met
            180                 185                 190
Glu Asp Leu Val Pro Cys Arg Val Arg Ala Lys Arg Gly Cys Ala Thr
        195                 200                 205
His Pro Arg Ser Ile Ala Glu Arg Val Arg Arg Thr Arg Ile Ser Asp
    210                 215                 220
Arg Ile Arg Lys Leu Gln Glu Leu Val Pro Asn Met Asp Lys Gln Thr
225                 230                 235                 240
Asn Thr Ala Asp Met Leu Glu Glu Ala Val Glu Tyr Val Lys Phe Leu
                245                 250                 255
Gln Lys Gln Ile Gln Glu Leu Thr Glu His Gln Lys Lys Cys Thr Cys
            260                 265                 270
Ser Thr Thr Glu Gln
        275
```

<210> 75
<211> 1294
<212> DNA
<213> Ipomoea nil

<400> 75

```
gctctcgggt tcttgcctct tgctcacagt aacaccacaa aattcacctg aaaacgggga     60
taacgatata tccggatata gaaagtaacc gatgcagccg gagagtggcg ccggaggcgg    120
cggaagtgag ccgagccgag gagcaggact gacgcgcttc cgctcagctc cggcgacctg    180
gttggaagcg ctcctggatt ccgacacgga aaacgacgtc gttctcgacc ctcctccgat    240
acttccttct tctagcaagc ctccaccgca cccgcactct caacccttgc ctacgccacc    300
agctcagctt aagcaggcgt ctgttggcgg aagtggctcc agatacgccg ccgatctggg    360
cctattcgag tccggaggcg gtggcgggga agcagcatct ggacttagta atttcgtcag    420
gcagaacagc tctccggcgg agtttctctc tcagatcagc tcagatatct tcttccccag    480
cttcggagct ccgcccagct acgactacct ttcttctccc atggatgtgg ctcagtcagc    540
taagcggccc agagaggccg attcccaaag cccctccgct aaattatcat ctccccttgaa   600
aggagaacaa agtgggcgat tacgcagtgc tggtgggtcg ttggatgcag aaatggagaa    660
gatgatggaa gatttagtcc catgcagggt tagagcaaag cgtggttgtg ctacccatcc    720
ccgaagtatt gcagagaggg ttagaagaac gcgaattagt gacaggataa ggaagcttca    780
ggagcttgtg cctaatatgg ataagcaaac taatacggct gacatgttgg aagaagcagt    840
agaatatgtc aagtttctac agaagcagat tcaggaactc accgagcatc agaagaaatg    900
cacttgctcc acgaccgagc aatgagagag agaaagagag agatcactga gggtttcctg    960
```

```
cagtaaatag gcaataatat ggggttttgg tatctcggat acaatcatcc gagatatggt   1020
attttctctt ttatgtcata ctattctgca tgtctagatg ttggcaagta gaacccataa   1080
tttttacagt agaattcacg ctcaatcagc tctagctact ccacgcataa taacctagat   1140
tattgtattc ctacttgtaa aatgctgtat aaagtagtat cgttcaataa gatagtcaca   1200
aatcttcacc tgaaaatcta tgttcatgac gatgtactta aaaaattcgt gcccatactc   1260
ctgcagcata gtgggtcaat atcagatcaa caag                                1294

<210>  76
<211>  155
<212>  PRT
<213>  Ipomoea nil

<400>  76
Met Asp Glu Ser Tyr Leu Ser Ser Phe Pro Ile Pro Ser Trp Asp Asp
1               5                   10                  15
Leu Asp Leu Leu Ser Asp Asp Phe Leu Lys Val Thr Asp Glu Lys Pro
            20                  25                  30
Ser Leu Lys Ala Lys Ala Pro Asp Phe Lys Asn Thr Glu Gly Ser Arg
        35                  40                  45
Ser Arg Pro Pro Gly Arg Leu Ser His His Leu Ser Leu Pro Lys Ser
    50                  55                  60
Ser Glu Glu Leu Ser Ser Ile Leu Gln Asp Ser Val Pro Cys Arg Val
65                  70                  75                  80
Arg Ala Lys Arg Gly Cys Ala Thr His Pro Arg Ser Ile Ala Glu Arg
                85                  90                  95
Val Arg Arg Thr Arg Ile Ser Asp Arg Met Arg Lys Leu Gln Glu Leu
            100                 105                 110
Val Pro Asn Met Asp Lys Gln Thr Asn Thr Ala Asp Met Leu Asp Leu
        115                 120                 125
Ala Val Asp Tyr Ile Lys Asp Leu Glu Gln Lys Val Lys Arg Leu Ala
    130                 135                 140
Asp Asn Arg Ala Lys Cys Arg Cys Ser Asn Lys
145                 150                 155

<210>  77
<211>  997
<212>  DNA
<213>  Lactuca sativa

<220>
<221>  misc_feature
<222>  (861)..(861)
<223>  n is a, c, g, or t

<400>  77
gcggggagct gcttcttcac gaccttcatc tctctccatc ttccccattt ctggcaaaga     60
aagattgata tgtatcccac ctccaattca tcttccgcat ctcagacttc aggaggagat    120
cctaacgcca tcaatggcag taacgttagc ggcggtgggg gaggagcaca gcaaggcctc    180
gctcgctacc gttctgctcc cgtctccttt ttaaccacca ccgtagactc cgtcatcaat    240
gggcaaaccc aacataccac cgtcggaaat cacaatatgg ttggcggagg cggaacccct    300
actcgctttt tctcacctcc agactcgaca tcttcgcatc tctccaccgg agatcggtta    360
caaaccacct cattccgttt aaacgagttc gcaaccgctt ttaatggttt gaaaggtaca    420
tctcagactc cgtctccctt attccggcac ggcagctccc ccgccggttt tctcaacacc    480
cttgtttctt ctaccccac tgacggaagg ggttcaagat taagctctca acttagtttc    540
actggaacaa gttctttttc tcaaatatcc gaagagaata acattgcaaa ttccctaatg    600
ttttcaaact catcgcataa caaaagagct aagcttgaca tcaatggtct taatgtcatg    660
gattctgagc ttaattttgg gctatcagaa tctgctctag aggcagccac gatggagaag    720
atgatggacc ttcctcatga ttctgtccca tgtaaaatcc gtgccaagcg tggttgcgct    780
actcatcccc ggagcattgc tgaaagggaa agaagaacaa gaataagcgg gaagctgaag    840
```

```
aagttgcaag atcttgttcc naatatggat aagcaaacaa gctattcaga catgttggac    900
ctggctgtac aacatataaa aagccttcaa actcacgttc agagccctta caatgaactc    960
caaaattgca agtgtggatg caaaccccaa tgaaaat                             997

<210>  78
<211>  307
<212>  PRT
<213>  Lactuca sativa

<400>  78
Met Tyr Pro Thr Ser Asn Ser Ser Ser Ala Ser Gln Thr Ser Gly Gly
1               5                   10                  15
Asp Pro Asn Ala Ile Asn Gly Ser Asn Val Ser Gly Gly Gly Gly Gly
            20                  25                  30
Ala Gln Gln Gly Leu Ala Arg Tyr Arg Ser Ala Pro Val Ser Phe Leu
        35                  40                  45
Thr Thr Thr Val Asp Ser Val Ile Asn Gly Gln Thr Gln His Thr Thr
    50                  55                  60
Val Gly Asn His Asn Met Val Gly Gly Gly Gly Thr Pro Thr Arg Phe
65                  70                  75                  80
Phe Ser Pro Pro Asp Ser Thr Ser Ser His Leu Ser Thr Gly Asp Arg
                85                  90                  95
Leu Gln Thr Thr Ser Phe Arg Leu Asn Glu Phe Ala Thr Ala Phe Asn
            100                 105                 110
Gly Leu Lys Gly Thr Ser Gln Thr Pro Ser Pro Leu Phe Arg His Gly
        115                 120                 125
Ser Ser Pro Ala Gly Phe Leu Asn Thr Leu Val Ser Ser Thr Pro Thr
    130                 135                 140
Asp Gly Arg Gly Ser Arg Leu Ser Ser Gln Leu Ser Phe Thr Gly Thr
145                 150                 155                 160
Ser Ser Phe Ser Gln Ile Ser Glu Glu Asn Asn Ile Ala Asn Ser Leu
                165                 170                 175
Met Phe Ser Asn Ser Ser His Asn Lys Arg Ala Lys Leu Asp Ile Asn
            180                 185                 190
Gly Leu Asn Val Met Asp Ser Glu Leu Asn Phe Gly Leu Ser Glu Ser
        195                 200                 205
Ala Leu Glu Ala Ala Thr Met Glu Lys Met Met Asp Leu Pro His Asp
    210                 215                 220
Ser Val Pro Cys Lys Ile Arg Ala Lys Arg Gly Cys Ala Thr His Pro
225                 230                 235                 240
Arg Ser Ile Ala Glu Arg Glu Arg Arg Thr Arg Ile Ser Gly Lys Leu
                245                 250                 255
Lys Lys Leu Gln Asp Leu Val Pro Asn Met Asp Lys Gln Thr Ser Tyr
            260                 265                 270
Ser Asp Met Leu Asp Leu Ala Val Gln His Ile Lys Ser Leu Gln Thr
        275                 280                 285
His Val Gln Ser Pro Tyr Asn Glu Leu Gln Asn Cys Lys Cys Gly Cys
    290                 295                 300
Lys Pro Gln
305

<210>  79
<211>  931
<212>  DNA
<213>  Lactuca sativa

<400>  79
cggcgggggt tgcagagcat ttactcctgt gaagcggtga cgtagcagtt gtttcctcca     60
atccacctct tttcactcat cttttttatc gtaaataaat taaaaatctg tagtagcaag    120
```

```
ataaacacga gttgcttaac caaaaggtaa tccaacagag gagtaaagat gcaaccgaca    180
ggcagtggcg gtggtggagg actatccaga ttccggtcag ctccggcgac atggctggaa    240
gcactgctag aatcagaaga agaagatgta atcattgacc caccaaaacc acctccacat    300
cagcaagctt ccattgcagg cacccccacc ccctccagac ctacttacgt tgatcccaat    360
atcttcttac ctagccgaca gaacagctct cccgccgaat tcttttctga gatcaacaat    420
cctgatgcct atctctccaa ttacgacgac tacctctcat cgtcctacca caacgttaaa    480
ccatccaggg ctgtcgatgt agacgaccaa caacccaagt tcaccacaca gctcagcgga    540
gatcagagcg cgttgttgga tgttgagatg gataagcttc tgggggattc ggtgccgtgt    600
cgagtacgag caaagcgcgg atgtgccact catcctcgga gtatcgccga gagggttaga    660
aggactcgga ttagtgacag aataaggaag ctccaagaac tggttccgaa tatggataag    720
caaaccaata cggcagacat gctagaagag gcggttgaat atgtcaagtt tctgcaaaga    780
cagattcagg aactaaagga gcaccgagac aaatgcacat gtgtggttga cgattgataa    840
cagaaaagaa gctaaccttt ttttgatcgt ggttcaaggg atcataatgg aggactatgg    900
tgcttatcca aaatctcaga tcaaatatat t                                   931
```

<210> 80
<211> 222
<212> PRT
<213> Lactuca sativa

<400> 80

```
Met Gln Pro Thr Gly Ser Gly Gly Gly Gly Gly Leu Ser Arg Phe Arg
1               5                   10                  15
Ser Ala Pro Ala Thr Trp Leu Glu Ala Leu Leu Glu Ser Glu Glu Glu
            20                  25                  30
Asp Val Ile Ile Asp Pro Pro Lys Pro Pro Pro His Gln Gln Ala Ser
        35                  40                  45
Ile Ala Gly Thr Pro Thr Pro Ser Arg Pro Thr Tyr Val Asp Pro Asn
    50                  55                  60
Ile Phe Leu Pro Ser Arg Gln Asn Ser Ser Pro Ala Glu Phe Phe Ser
65                  70                  75                  80
Glu Ile Asn Asn Pro Asp Ala Tyr Leu Ser Asn Tyr Asp Asp Tyr Leu
                85                  90                  95
Ser Ser Ser Tyr His Asn Val Lys Pro Ser Arg Ala Val Asp Val Asp
            100                 105                 110
Asp Gln Gln Pro Lys Phe Thr Thr Gln Leu Ser Gly Asp Gln Ser Ala
        115                 120                 125
Leu Leu Asp Val Glu Met Asp Lys Leu Leu Gly Asp Ser Val Pro Cys
    130                 135                 140
Arg Val Arg Ala Lys Arg Gly Cys Ala Thr His Pro Arg Ser Ile Ala
145                 150                 155                 160
Glu Arg Val Arg Arg Thr Arg Ile Ser Asp Arg Ile Arg Lys Leu Gln
                165                 170                 175
Glu Leu Val Pro Asn Met Asp Lys Gln Thr Asn Thr Ala Asp Met Leu
            180                 185                 190
Glu Glu Ala Val Glu Tyr Val Lys Phe Leu Gln Arg Gln Ile Gln Glu
        195                 200                 205
Leu Lys Glu His Arg Asp Lys Cys Thr Cys Val Val Asp Asp
    210                 215                 220
```

<210> 81
<211> 877
<212> DNA
<213> Lactuca serriola

<400> 81

```
ccccttccca ccctcctcct aagccctctc cgctactccc tttctaaccc agattacgat     60
tctccggcat ggctacagac ccgccggagg gctacggaga cgacttcctt gaacaaattc    120
tcgcgattcc ttcttacaac atcgccggat gcttgcctgg taatactacc gacgctaatg    180
```

cttctgaaac ggtgtctgtt catcgtcaac agcaacagca gcaaccggtg tttccgttag 240
gtttgagcct tgataatggc cgtgaaacaa tcggagcgtt tgcagggcag cagcagcagc 300
agcagaggga gagaggaggg agcatgaaca tgactggatt gtttccttca tttgaaaatt 360
tacagtcaca ttctctgctt cattctgttc ctcaggcttt ccaagggcaa tcaactacca 420
gtacagctgt cactgttccc catccacctt ctattcgccc tagggtgcgg gcccgcagag 480
gacaagctac agatcctcat agcatagctg agcgtctacg ccgagaaaga attgcagaaa 540
gaatgagggc tttgcaggaa cttgttccta gctgcaacaa gaccgataaa gctgcaatgc 600
tagatgaaat tcttgattat gtgaagttct tacggcttca ggtcaaggtt cttagcatga 660
gtaggctggg tggggccggt gcagtggcac aactcgtatc tgacgtcccc ttgcaatccg 720
ttgaggtaac tttcacaaac aaacatttta tcattaatag taaaaatatg aaacattgtt 780
gtatataggg ggacacgagt gaaaatggat ataatcaacc ggcatgggaa aactggtcaa 840
acgatgacac agaacgtgaa gtagcaaagc tcatgga 877

<210> 82
<211> 222
<212> PRT
<213> Lactuca serriola

<400> 82
Met Gln Pro Thr Gly Ser Gly Gly Gly Gly Gly Leu Ser Arg Phe Arg
1 5 10 15
Ser Ala Pro Ala Thr Trp Leu Glu Ala Leu Leu Glu Ser Glu Glu Glu
20 25 30
Asp Val Ile Ile Asp Pro Pro Lys Pro Pro Pro His Gln Gln Ala Ser
35 40 45
Ile Ala Gly Thr Pro Thr Pro Ser Arg Pro Thr Tyr Val Asp Pro Asn
50 55 60
Ile Phe Leu Pro Ser Arg Gln Asn Ser Ser Pro Ala Glu Phe Phe Ser
65 70 75 80
Glu Ile Asn Asn Pro Asp Ala Tyr Leu Ser Asn Tyr Asp Asp Tyr Leu
85 90 95
Ser Ser Ser Tyr His Asn Val Lys Pro Ser Arg Ala Val Asp Val Asp
100 105 110
Asp Gln Gln Pro Lys Phe Thr Thr Gln Leu Ser Gly Asp Gln Ser Ala
115 120 125
Leu Leu Asp Val Glu Met Asp Lys Leu Leu Gly Asp Ser Val Pro Cys
130 135 140
Arg Val Arg Ala Lys Arg Val Cys Ala Thr His Pro Arg Ser Ile Ala
145 150 155 160
Glu Arg Val Arg Arg Thr Arg Ile Ser Asp Arg Ile Arg Lys Leu Gln
165 170 175
Glu Leu Val Pro Asn Met Asp Lys Gln Thr Asn Thr Ala Asp Met Leu
180 185 190
Glu Glu Ala Val Glu Tyr Val Lys Phe Leu Gln Arg Gln Ile Gln Glu
195 200 205
Leu Lys Glu His Arg Asp Lys Cys Thr Cys Val Val Gly Asp
210 215 220

<210> 83
<211> 960
<212> DNA
<213> Medicago truncatula

<400> 83
atggatttga attctcatta tcagcaactc caacaaaacc aaccaaattc aaggttgctt 60
cgttttcgtt caagtttcaa acaacaaggt gaaggttgtg gtggtagtgc aactgctgct 120
acaaattctg gtgaaacttc ttcatctacc tttagagaat tcatggacca taacccttct 180
aatcataaag ttgacaacaa cgagtcttca ttatcacaaa gacacatgaa ttctcagcag 240
ggttatagaa tggatcaaca caaaggtttt tataccaatc ttttaagaca aagtagttct 300

```
catgatggtc atttctccaa caacaacatc atctcctttg gaaatgggta tgaacctatg    360
aaaggtgttg aaaactatga tggggtgaag gacagtgatg gtgaacttac tctatctatg    420
aacatattaa acaatcaaat tggcttctca ccaagaactc cttcttcctt cagaatgtta    480
tcacagaacc ctaaaactgg gagtgatggt attggaacaa ctagccacga tgatcgaaga    540
caagtaggta gcaatgacga cgctcaatat tatggtcata aactcgttta tgattctaat    600
gaccagaatg taggggtacg aaatcaagtt gatacactat cacatcactt gagtttgcca    660
agaaaatcat cagaaatgtt tgttgtggag aaattgcttc agtttccaga ttctgttcct    720
agcagtatca gagcaaagag aggctttgca acacatccta gaagccttgc cgaaagggta    780
agaagaactc ggataagtga acgaatgaga aaattgcaag agattgttcc aaacattgat    840
aagcaaactt gcacatcaga aatgttagac ttggctgtag agtatattaa agatcttcaa    900
aaacaattaa agactatgag tgcaaaaaga gctaagtgca gatgcagaaa caagaaatga    960
```

<210> 84
<211> 319
<212> PRT
<213> Medicago truncatula

<400> 84

```
Met Asp Leu Asn Ser His Tyr Gln Gln Leu Gln Gln Asn Gln Pro Asn
1               5                   10                  15
Ser Arg Leu Leu Arg Phe Arg Ser Ser Phe Lys Gln Gln Gly Glu Gly
            20                  25                  30
Cys Gly Gly Ser Ala Thr Ala Ala Thr Asn Ser Gly Glu Thr Ser Ser
        35                  40                  45
Ser Thr Phe Arg Glu Phe Met Asp His Asn Pro Ser Asn His Lys Val
    50                  55                  60
Asp Asn Asn Glu Ser Ser Leu Ser Gln Arg His Met Asn Ser Gln Gln
65                  70                  75                  80
Gly Tyr Arg Met Asp Gln His Lys Gly Phe Tyr Thr Asn Leu Leu Arg
                85                  90                  95
Gln Ser Ser Ser His Asp Gly His Phe Ser Asn Asn Asn Ile Ile Ser
            100                 105                 110
Phe Gly Asn Gly Tyr Glu Pro Met Lys Gly Val Glu Asn Tyr Asp Gly
        115                 120                 125
Val Lys Asp Ser Asp Gly Glu Leu Thr Leu Ser Met Asn Ile Leu Asn
    130                 135                 140
Asn Gln Ile Gly Phe Ser Pro Arg Thr Pro Ser Ser Phe Arg Met Leu
145                 150                 155                 160
Ser Gln Asn Pro Lys Thr Gly Ser Asp Gly Ile Gly Thr Thr Ser His
                165                 170                 175
Asp Asp Arg Arg Gln Val Gly Ser Asn Asp Asp Ala Gln Tyr Tyr Gly
            180                 185                 190
His Lys Leu Val Tyr Asp Ser Asn Asp Gln Asn Val Gly Val Arg Asn
        195                 200                 205
Gln Val Asp Thr Leu Ser His His Leu Ser Leu Pro Arg Lys Ser Ser
    210                 215                 220
Glu Met Phe Val Val Glu Lys Leu Leu Gln Phe Pro Asp Ser Val Pro
225                 230                 235                 240
Ser Ser Ile Arg Ala Lys Arg Gly Phe Ala Thr His Pro Arg Ser Leu
                245                 250                 255
Ala Glu Arg Val Arg Arg Thr Arg Ile Ser Glu Arg Met Arg Lys Leu
            260                 265                 270
Gln Glu Ile Val Pro Asn Ile Asp Lys Gln Thr Cys Thr Ser Glu Met
        275                 280                 285
Leu Asp Leu Ala Val Glu Tyr Ile Lys Asp Leu Gln Lys Gln Leu Lys
    290                 295                 300
Thr Met Ser Ala Lys Arg Ala Lys Cys Arg Cys Arg Asn Lys Lys
305                 310                 315
```

<210> 85
<211> 1224
<212> DNA
<213> Medicago truncatula

<400> 85
atgcagcctt gtagcagaga aatgcaatct cttaactctc tcttcaactc ttcttcttct 60
tcttcttctg aaattcccat tcctctccaa agtcagatcc aaattaacaa caacaacaac 120
aatcacaacg acacattcaa ccatcacgac gattttctca aacaaatcct ctccaccaat 180
cttcctcctt catggaacac tctcgaccca aaccataaca aacccttatt atgggacccc 240
aattccgacg aaaatgtcac tttccctaat tacgatgaac aacacaacaa ccttgcctct 300
aagtttcgta accaccagat cactgacaaa accgccgccg ctctcatgct tctcaccgcc 360
gattccggag gtctcctcca catgcctgct gactttgact cctcccaaaa cgacgtcgtt 420
aacacctcct ccgctgctgg tgatgcttcc gttcaagctc tcttcaacgg tttctccgga 480
tctctccacg gtgtcgctca acctcaccat tttcaacctc cacaggggca gagttttgga 540
agtggttcgg tgagtgcaac gaaccaagct ccggcaagtg gcgctccggc tcagccgaga 600
caaaaggtta gggcaaggag agggcaagcc accgacccac acagcatcgc cgaaaggcta 660
cggagggaaa gaattgctga gagaatgaaa gctttgcagg aacttgttcc taatgcaaac 720
aagacagaca aggcttccat gctggatgag atcatcgatt atgtgaagtt cctacaagtc 780
caagtcaaag ttttgagtat gagcagatta ggtggagcag gggctgtagc tcctcttgtg 840
gctgatatgt cctccgaggg tgtcagtgac tgcgtccaaa caaacggcaa cggaggggtc 900
cacccacgaa accctaaaac ggcgtcgtcc tccaacgaaa gcctcaccat gacggagcat 960
caagtggcca agctaatgga agaagacatg ggatctgcca tgcaatactt acaaggcaaa 1020
ggtctttgcc tcatgcctat ttctcttgct actgcaatct ctactgccac gtgtcacacc 1080
aggaaccctt tgatcaatgc tgctaataat aacatcaacg gtagttccaa ccctatcacc 1140
gcatctaacg gtgatgggcc atcttctccc ggaatgtccg ttaacagcat cgtcaaagat 1200
gctaactccg cttctaagtc gtag 1224

<210> 86
<211> 343
<212> PRT
<213> Medicago truncatula

<400> 86
Met Tyr Arg Pro Pro Ser Ser Ser Ala Ser Ser Ser Ser Ser Ser Ser
1 5 10 15
Gln Gln Gln Gln Ser Ser Ile Ser Gln Thr Gly Leu Thr Arg Tyr Gly
20 25 30
Ser Ala Pro Ser Ser Leu Leu Asn Thr Thr Val Asp Ala Ile Ile Gly
35 40 45
Gly Ser Arg Leu Leu Pro Gly Thr Gly Gln His Tyr Phe Ser Asp Asp
50 55 60
Ser Thr Gln Gln Gln Gln Gln His His His Gln Gln Gln Gln Gln Gln
65 70 75 80
Gln Gln Gln Arg Ser Ser Tyr Glu Gly Gln Gly Gly Gly Phe Asp Gly
85 90 95
Ser Ala Leu Leu Arg Gln Lys Ser Ser Pro Ala Gly Phe Leu Asn His
100 105 110
Leu Ala Thr Leu Asn His Asn Asn Asn Asn Asn Asn Asn Asn Asn Asn
115 120 125
Ala Gly Gly Gly Phe Thr Ile Thr Arg Gly Asn Ser Arg Leu Lys Ser
130 135 140
Glu Leu Ser Phe Thr Gly Gly Gly Gln Glu Cys Leu Ser Arg Ile Ser
145 150 155 160
Glu Asn Val Val Asp Tyr Ala Ser Ala Ala Ala Ala Ala Gly Asn Gly
165 170 175
Ser Leu His Thr Asn Ser Thr Asn Thr Trp Gly Gly Gly Gly Pro Asp
180 185 190
Asn Asn Asn Asn Ser Asn Ser Ile Val Phe Ser Ser Ser Gln Thr Gln

```
        195                 200                 205
Thr Asn Asn Asn Ser Lys Lys Arg Ser Ser Arg Thr Asp Pro Asn Asp
    210                 215                 220
Asp Pro Asp Leu Leu Leu His Cys Leu Asn Ala Leu Glu Thr Gln Tyr
225                 230                 235                 240
Ser Leu Pro Gln Thr Ser Leu Glu Met Asp Gln Leu Met His Asn Ile
                245                 250                 255
Pro Gln Asp Ser Val Pro Cys Lys Ile Arg Ala Lys Arg Gly Cys Ala
            260                 265                 270
Thr His Pro Arg Ser Ile Ala Glu Arg Glu Arg Arg Thr Arg Ile Ser
        275                 280                 285
Gly Lys Leu Lys Lys Leu Gln Asp Leu Val Pro Asn Met Asp Lys Gln
    290                 295                 300
Thr Ser Tyr Ser Asp Met Leu Asp Leu Ala Val Gln His Ile Lys Gly
305                 310                 315                 320
Leu Gln Thr Gln Val Gln Lys Leu His Glu Asp Leu Glu Asn Cys Thr
                325                 330                 335
Cys Gly Cys Lys Gln Ser Thr
            340

<210>  87
<211>  775
<212>  DNA
<213>  Medicago truncatula

<400>  87
ttatcccagg tttcccctct aggtactaca tgggaggata ctgcgatgat atctgacaat      60
attactggct tgaaaagata cagagatgat gatgatgtaa aaccatttcc tccaggttta     120
aatccagctg aaactaagaa tgaacaggga ggccagacta cttctgctcc tttggctcat     180
cagatgagta tgccaaatac tacggccgaa ttggcagcca ttgagaagtt tttgcagttc     240
tcagattctg ttccgtgcaa aattcgtgct aaacgtggct gtgccactca cccaagaagc     300
attgcagaaa gggttagaag aactaaaata agcgagcgaa tgaggaaact acaggatctt     360
gtgccaaaca tggataagca aacaaacaca tcagacatgt tggacttggc tgttgagtac     420
attaaagacc tacaaaacca agttgagact ctttcagaca atcgggctaa gtgcacgtgt     480
tcacacaagc aacaacaata agggaaaaga attaaaggat gatgataaga gagtcagagt     540
tttttgtaca gattttgttg taataatatg gtctagaaat tagagcagct taattttcag     600
atataataat atttatgatg ttgtcacttt ttctatggtt gtttgggaat ggtggggaat     660
gagatcataa aagttgactt ttattataag atcatataat ttgatgatga tgatgtatgt     720
cacatgtcac atatgcaaag cattgtgtac aaaaatatga tgaagcaagt ttatt          775

<210>  88
<211>  151
<212>  PRT
<213>  Medicago truncatula

<400>  88
Met Ile Ser Asp Asn Ile Thr Gly Leu Lys Arg Tyr Arg Asp Asp Asp
1               5                   10                  15
Asp Val Lys Pro Phe Pro Pro Gly Leu Asn Pro Ala Glu Thr Lys Asn
            20                  25                  30
Glu Gln Gly Gly Gln Thr Thr Ser Ala Pro Leu Ala His Gln Met Ser
        35                  40                  45
Met Pro Asn Thr Thr Ala Glu Leu Ala Ala Ile Glu Lys Phe Leu Gln
    50                  55                  60
Phe Ser Asp Ser Val Pro Cys Lys Ile Arg Ala Lys Arg Gly Cys Ala
65                  70                  75                  80
Thr His Pro Arg Ser Ile Ala Glu Arg Val Arg Arg Thr Lys Ile Ser
                85                  90                  95
Glu Arg Met Arg Lys Leu Gln Asp Leu Val Pro Asn Met Asp Lys Gln
```

```
            100                 105                 110
Thr Asn Thr Ser Asp Met Leu Asp Leu Ala Val Glu Tyr Ile Lys Asp
        115                 120                 125
Leu Gln Asn Gln Val Glu Thr Leu Ser Asp Asn Arg Ala Lys Cys Thr
    130                 135                 140
Cys Ser His Lys Gln Gln Gln
145                 150
```

<210> 89
<211> 1368
<212> DNA
<213> Nicotiana benthamiana

<400> 89

```
gattatgtat ccatcctcaa cttcttcttc acctcaaaat tcaatgagcc atagcaccac     60
cgctggcggc gatagccacg atggcggagg actgacccgt tatggttcag ctccgggctc    120
gtttttaacg actgcagttc aatccgtcac tggtgctact aatcacgatt ttaacctcca    180
cgggtcccac catcaacacc ttggaccttc gcgatattat tcgccaaatt tggcgtcatc    240
taattcatta aattcggaat ctactagtaa ggctaaggaa caatcgagtt tacagaggtc    300
aattggtttc aatgatttaa caattggcgg cggcagtgga agtgacggtg gcggtggatg    360
tggagttttg ccggcggcga attcgacgac gccgttgatt cgacatagta gctcaccagc    420
taggtttctt aatcaacttg ctgctgctgc tggtgatact gtatcaatgg gaagaggaag    480
ctacaactct aaaggcatcg cagacagtag ttgtggtata acaaggctga actctcagct    540
cagcttcact aggcaagaag ctctctctca aatagcagag gaaaatgagg atgttgaagg    600
gaccagtaca gacaatggcc acagaaagtc aacacattct tatgccactg caagtagttt    660
cgcaatgggt tcttgggaag ataacaattc tataatgttc tctgtcactc cgagcaaacg    720
agccaagcaa attagcaatg acattgtcaa taatgggctt gatgacgggg aaactcagtt    780
ccagtttggc ttgtctcaga cagcactaga aatggcatca atggatagat ttctgcacat    840
ccctgaggat tctgttcctt gcaaaattcg tgccaagggt ggttgtgcta ctcatcctcg    900
cagcattgca gaaagggaga gaagaaccag aataagcggg aagcttaaga agttacaaga    960
tcttgttcca aacatggaca agcaaacgag ctacgctgac atgctggatc tagcagtgca   1020
gcatattcga acccttcaag atcaggttca gcatctcaat actgaacttg aaaactgcaa   1080
atgtggatgt aagaaatcca gtcaataacc gtgcatataa aatgggagga accattagct   1140
gaagattgac ctaatggctt tctgtcacca ttattgcctc gtagaagaat tccaaatctg   1200
tacatatgga agcaaatttc ctactagctt tttaaagcta aaagtgaatt ctcttggtcg   1260
ttttttcctt ctcagttgga ccacatatat ggttgtttgg attatttcta gtttttttct   1320
cctctaacat ttgatttata attcagtttt gggttcattt tgctttct                1368
```

<210> 90
<211> 367
<212> PRT
<213> Nicotiana benthamiana

<400> 90

```
Met Tyr Pro Ser Ser Thr Ser Ser Ser Pro Gln Asn Ser Met Ser His
1               5                   10                  15
Ser Thr Thr Ala Gly Gly Asp Ser His Asp Gly Gly Gly Leu Thr Arg
            20                  25                  30
Tyr Gly Ser Ala Pro Gly Ser Phe Leu Thr Thr Ala Val Gln Ser Val
        35                  40                  45
Thr Gly Ala Thr Asn His Asp Phe Asn Leu His Gly Ser His His Gln
    50                  55                  60
His Leu Gly Pro Ser Arg Tyr Tyr Ser Pro Asn Leu Ala Ser Ser Asn
65                  70                  75                  80
Ser Leu Asn Ser Glu Ser Thr Ser Lys Ala Lys Glu Gln Ser Ser Leu
                85                  90                  95
Gln Arg Ser Ile Gly Phe Asn Asp Leu Thr Ile Gly Gly Gly Ser Gly
            100                 105                 110
Ser Asp Gly Gly Gly Gly Cys Gly Val Leu Pro Ala Ala Asn Ser Thr
```

```
        115                 120                 125
Thr Pro Leu Ile Arg His Ser Ser Ser Pro Ala Arg Phe Leu Asn Gln
    130                 135                 140
Leu Ala Ala Ala Ala Gly Asp Thr Val Ser Met Gly Arg Gly Ser Tyr
145                 150                 155                 160
Asn Ser Lys Gly Ile Ala Asp Ser Ser Cys Gly Ile Thr Arg Leu Asn
                165                 170                 175
Ser Gln Leu Ser Phe Thr Arg Gln Glu Ala Leu Ser Gln Ile Ala Glu
            180                 185                 190
Glu Asn Glu Asp Val Glu Gly Thr Ser Thr Asp Asn Gly His Arg Lys
        195                 200                 205
Ser Thr His Ser Tyr Ala Thr Ala Ser Ser Phe Ala Met Gly Ser Trp
    210                 215                 220
Glu Asp Asn Asn Ser Ile Met Phe Ser Val Thr Pro Ser Lys Arg Ala
225                 230                 235                 240
Lys Gln Ile Ser Asn Asp Ile Val Asn Asn Gly Leu Asp Asp Gly Glu
                245                 250                 255
Thr Gln Phe Gln Phe Gly Leu Ser Gln Thr Ala Leu Glu Met Ala Ser
            260                 265                 270
Met Asp Arg Phe Leu His Ile Pro Glu Asp Ser Val Pro Cys Lys Ile
        275                 280                 285
Arg Ala Lys Gly Gly Cys Ala Thr His Pro Arg Ser Ile Ala Glu Arg
    290                 295                 300
Glu Arg Arg Thr Arg Ile Ser Gly Lys Leu Lys Lys Leu Gln Asp Leu
305                 310                 315                 320
Val Pro Asn Met Asp Lys Gln Thr Ser Tyr Ala Asp Met Leu Asp Leu
                325                 330                 335
Ala Val Gln His Ile Arg Thr Leu Gln Asp Gln Val Gln His Leu Asn
            340                 345                 350
Thr Glu Leu Glu Asn Cys Lys Cys Gly Cys Lys Lys Ser Ser Gln
        355                 360                 365

<210>  91
<211>  1464
<212>  DNA
<213>  Nicotiana benthamiana

<400>  91
caattatgta tccatcctca acttcttctt catctcaaaa ttcaatgagc cacagcacca      60
ccgctggcgg cggtagcaac ggcggtggag gactgacccg ttacggttca gctccgggcg     120
catttttaac tactgcagtt gaatccgtca ttggtgctaa taatcacgat tttaacctcc     180
acgggtccca ccatcaacac cttggacctt cgcgatatta ttcgccaaat atggcgtcat     240
ctaattcgtt aaattcggaa tctactagta aggctaagga acaatcgagt ttacagagat     300
caattggatt caatgattta acaattggcg gcggcggtgg aagtggcggc agtggatgtg     360
gatttatgcc ggcggcgaat tcgacaacgc cgttggttcg acatagtagc tcaccggcta     420
agtttcttaa tcaacttgct gctgttgctg gtgatactgg cttttcagca tcaatgggga     480
gaggaagcta taactctaaa ggcgtcgcag atagtagccg ggtataaca aggctgaact     540
ctcagctcag cttcactagg caagaagttc tctctcaaat agcagaggaa aatgaggatg     600
ttgaagggac cagtacagag gatggctaca gaaagtcaac acattcttat gccactgcaa     660
gtagtttcgc aatgggttca tgggaagata acaattctat aatgttctct gtcacaccaa     720
gcaaacgagc caagcacatt agcgatgaca ttgtcaatgg gctcgatgat ggggaaactc     780
agtttcggtt tggcttgtct cagacagcac tagaaatggc gtcaatggat agattgctgc     840
acatccctga ggattctgtt ccttgcaaaa ttcgtgccaa gcgtggttgt gctactcatc     900
ctcgcagcat tgcagaaagg gagagaagaa ccagaataag tgggaagcta aagaagttac     960
aagatcttgt tccaaacatg gacaagcaaa cgagctacgc tgacatgctg gatctagcag    1020
tgcagcacat tcgaaccctt caagatcagg ttcagaatct caatactgaa cttgaaaact    1080
gcaaatgtgg atgtaagaaa tcgagtcaat aacagtgcaa caaaatggga ggaaccatta    1140
gctgaagatt gacctaaagg ctttttgtca ccattattgc ctctgtagaa gaattcaaaa    1200
tctgtacata tggaagcaaa tttcctacta gctttttttaa agctaaaagt gaattctctt    1260
```

```
ggtcgttttt ttccttttca gttggaccac gtatatgatt ttagataatt tctagttttt   1320
tccctctaac atttaatttg taattcagtt ttgggttcat tttgctttct agattatttt   1380
cttgagtatc ataaggctaa ttcatgctga tggactatag cttctctatt tcattcccaa   1440
tttaaagaga aaaattgatc ctcc                                          1464

<210>  92
<211>  368
<212>  PRT
<213>  Nicotiana benthamiana

<400>  92
Met Tyr Pro Ser Ser Thr Ser Ser Ser Ser Gln Asn Ser Met Ser His
1               5                   10                  15
Ser Thr Thr Ala Gly Gly Gly Ser Asn Gly Gly Gly Gly Leu Thr Arg
            20                  25                  30
Tyr Gly Ser Ala Pro Gly Ala Phe Leu Thr Thr Ala Val Glu Ser Val
        35                  40                  45
Ile Gly Ala Asn Asn His Asp Phe Asn Leu His Gly Ser His His Gln
    50                  55                  60
His Leu Gly Pro Ser Arg Tyr Tyr Ser Pro Asn Met Ala Ser Ser Asn
65                  70                  75                  80
Ser Leu Asn Ser Glu Ser Thr Ser Lys Ala Lys Glu Gln Ser Ser Leu
                85                  90                  95
Gln Arg Ser Ile Gly Phe Asn Asp Leu Thr Ile Gly Gly Gly Gly Gly
            100                 105                 110
Ser Gly Gly Ser Gly Cys Gly Phe Met Pro Ala Ala Asn Ser Thr Thr
        115                 120                 125
Pro Leu Val Arg His Ser Ser Ser Pro Ala Lys Phe Leu Asn Gln Leu
    130                 135                 140
Ala Ala Val Ala Gly Asp Thr Gly Phe Ser Ala Ser Met Gly Arg Gly
145                 150                 155                 160
Ser Tyr Asn Ser Lys Gly Val Ala Asp Ser Ser Arg Gly Ile Thr Arg
                165                 170                 175
Leu Asn Ser Gln Leu Ser Phe Thr Arg Gln Glu Val Leu Ser Gln Ile
            180                 185                 190
Ala Glu Glu Asn Glu Asp Val Glu Gly Thr Ser Thr Glu Asp Gly Tyr
        195                 200                 205
Arg Lys Ser Thr His Ser Tyr Ala Thr Ala Ser Ser Phe Ala Met Gly
    210                 215                 220
Ser Trp Glu Asp Asn Asn Ser Ile Met Phe Ser Val Thr Pro Ser Lys
225                 230                 235                 240
Arg Ala Lys His Ile Ser Asp Asp Ile Val Asn Gly Leu Asp Asp Gly
                245                 250                 255
Glu Thr Gln Phe Arg Phe Gly Leu Ser Gln Thr Ala Leu Glu Met Ala
            260                 265                 270
Ser Met Asp Arg Leu Leu His Ile Pro Glu Asp Ser Val Pro Cys Lys
        275                 280                 285
Ile Arg Ala Lys Arg Gly Cys Ala Thr His Pro Arg Ser Ile Ala Glu
    290                 295                 300
Arg Glu Arg Arg Thr Arg Ile Ser Gly Lys Leu Lys Lys Leu Gln Asp
305                 310                 315                 320
Leu Val Pro Asn Met Asp Lys Gln Thr Ser Tyr Ala Asp Met Leu Asp
                325                 330                 335
Leu Ala Val Gln His Ile Arg Thr Leu Gln Asp Gln Val Gln Asn Leu
            340                 345                 350
Asn Thr Glu Leu Glu Asn Cys Lys Cys Gly Cys Lys Lys Ser Ser Gln
        355                 360                 365

<210>  93
```

<211>  1555
<212>  DNA
<213>  Oryza sativa

<400>  93
tagccccacc acctccacca cctcacctcc cccttccctt cccttcccct cttctctcct     60
ctactagcta tagtataggc cgcccccgtc gttttgttaa ttttcacgcc gaaatttcca    120
cccacaccac gcgccgcctg cctacctgcg cctgcgcgag ctgcagatcg atcgacgaga    180
ggccgcgtct cggtcgcggg gggtggagtt gatcgatcga tccaaggagg ggtatgatga    240
ggaggtttct gccggtgggg ggaggaggag gaggaggggt ggagccgtcg tcgtcgtcga    300
cgacgccgca gcgaggggag gcggaggcgg cggggctgcg gttcggtggg ggggacatct    360
cgctgggccc gcacggcggc ggcggcggcg gcggcggagg gggcggaggg catgggcatc    420
agctgcagga cgggagcgtg gacttgctgg cgcggcacag cagctcgccg gccggattct    480
tctccaacct catggccagc aacggctttc caggctcaaa aggcggggga ggcagcggag    540
ccgaagccca ccaccaccct tccatggccg gcagcggcag cggcagcagc agcggcggga    600
ggaagatgaa gtcccagctg agcttcacgg ccgggccgcc gcacctctcg cacatcgcgg    660
aggacggcgc cttccccgac cgcgccggcg ccgaggcctc cgtgccccgc accttctccg    720
ccggcggcag cagcggcggc ggcgggttct ccatcgtcgg gccgtgggag gagtccaggg    780
acatcatctc caccctcggc gggtacgagt cccagttcgg cggcatggcg agcacgtcgg    840
cgctggagat ggccggcatg gacaggtacc tgcagctgca gcatgaccag gtgccgttca    900
aagtgcgggc caagcgcggc tgcgcgacgc accccaggag catcgcggag agggaacgga    960
ggacgagaat tagcgagaag ctcaggaagc tgcaggagct ggtgcccaac atggacaagc   1020
aaacaagcac agcggacatg ttggaccttg cagttgagca catcaagggt ctgcagagcc   1080
agctgcaggc tctgaagcat gagcaggaga agtgcacttg ctgcagcagg ccttgattga   1140
tgaagcagtg agacggcgaa actgaaaggt atctatcctt agtttggaac cctgattacg   1200
gatgatactt tcatatttcc ttgagagtaa cgcatgcgtg gatgcatgaa ccaattaatg   1260
tcagtgacac aatccaaatc gaccgtgagc atttgtcgaa aatgtgtaaa ttgttagata   1320
ggacatggca aggacatgt atatgcatgc ctgcatgcat gagtgagtgc atggagctag   1380
ctactccgaa atagcggggt gcagccattg tgatccctta attaattctc gatccactga   1440
tgattaatta gcttgtaatt ctgtctttaa accttttttt taatgacgag gggagagaaa   1500
aagaaaatga aattttggtc agcatgcaga aagaactaca ttattaattc gtgcc        1555

<210>  94
<211>  300
<212>  PRT
<213>  Oryza sativa

<400>  94
Met Met Arg Arg Phe Leu Pro Val Gly Gly Gly Gly Gly Gly Gly Val
1               5                   10                  15
Glu Pro Ser Ser Ser Ser Thr Thr Pro Gln Arg Gly Glu Ala Glu Ala
            20                  25                  30
Ala Gly Leu Arg Phe Gly Gly Gly Asp Ile Ser Leu Gly Pro His Gly
        35                  40                  45
Gly Gly Gly Gly Gly Gly Gly Gly Gly Gly Gly His Gly His Gln Leu
    50                  55                  60
Gln Asp Gly Ser Val Asp Leu Leu Ala Arg His Ser Ser Ser Pro Ala
65                  70                  75                  80
Gly Phe Phe Ser Asn Leu Met Ala Ser Asn Gly Phe Pro Gly Ser Lys
                85                  90                  95
Gly Gly Gly Gly Ser Gly Ala Glu Ala His His His Pro Ser Met Ala
            100                 105                 110
Gly Ser Gly Ser Gly Ser Ser Ser Gly Gly Arg Lys Met Lys Ser Gln
        115                 120                 125
Leu Ser Phe Thr Ala Gly Pro Pro His Leu Ser His Ile Ala Glu Asp
    130                 135                 140
Gly Ala Phe Pro Asp Arg Ala Gly Ala Glu Ala Ser Val Pro Arg Thr
145                 150                 155                 160
Phe Ser Ala Gly Gly Ser Ser Gly Gly Gly Gly Phe Ser Ile Val Gly

```
                165                 170                 175
Pro Trp Glu Glu Ser Arg Asp Ile Ile Ser Thr Leu Gly Gly Tyr Glu
            180                 185                 190
Ser Gln Phe Gly Gly Met Ala Ser Thr Ser Ala Leu Glu Met Ala Gly
        195                 200                 205
Met Asp Arg Tyr Leu Gln Leu Gln His Asp Gln Val Pro Phe Lys Val
    210                 215                 220
Arg Ala Lys Arg Gly Cys Ala Thr His Pro Arg Ser Ile Ala Glu Arg
225                 230                 235                 240
Glu Arg Arg Thr Arg Ile Ser Glu Lys Leu Arg Lys Leu Gln Glu Leu
                245                 250                 255
Val Pro Asn Met Asp Lys Gln Thr Ser Thr Ala Asp Met Leu Asp Leu
            260                 265                 270
Ala Val Glu His Ile Lys Gly Leu Gln Ser Gln Leu Gln Ala Leu Lys
        275                 280                 285
His Glu Gln Glu Lys Cys Thr Cys Cys Ser Arg Pro
    290                 295                 300
```

<210> 95
<211> 1670
<212> DNA
<213> Oryza sativa

<400> 95

```
agccgaagcc gcaacctgca aaaccaagtc cacctccgcc tccgcctccg cctccgcctc     60
cacccaccac caccaccgcc gccgccgcgc cgccggagat gaacaggatg acggcgccgc    120
acggggggat gccgccgccg ccgatgccgg cggcgggggg gctggcgagg tacggctcgg    180
cgccggggtc gctgctggcg tcgatcgccg actcggtgat ccggggccgc ggggttgggg    240
ttgtcgatca gctgcatcat catcagcatc agcatcagct tccgccgccg ccgccgccgc    300
agcagcagca gatggtgggg aggtacttct ccgcggagtc gtcggggctc acctcctgcg    360
agtccagctg ccggacgacg acgacgacgt ccacagcggc ggcggccgat gtcgggcggc    420
acccgctcga gcgcgcgtac ggcggctccg gcgagatcca cgtcgacgcc tcctccgccg    480
ccgtcccgct cttccgccac agcagctccc ccgccggcct cctctcccgc ctcatggctg    540
acccgcacgg caatggcatg gccgcgacga gagggatggg cggctactcc ggcggcggcg    600
gcgacgccgg cgcgatggcg cacaggaggc tgagctcgca gtggagcttc tcgcggcagg    660
acctgccgca gatatcggag atgggcggcc tcatccccga catcggggag agcatcgtca    720
ccggcggcgg cggcaacagc tccagcgatg gcgccggcca cggcgcgcag tcctcctcct    780
tcctctcctc caggaacttc tccatgagct cctgggacga caccaactcc atcatgttct    840
cgcccccgag cagcagcaag aaggcccgcg tcgccgccgc cgccgccggc gaccacggcg    900
acgacatggt ctccagcttc agcaacatcg actcccagtt tggcttgtcg aagcagtcgt    960
cgctggagat ggccggcatg gatgacttct tgcagctcca gccagactcc gtcgcctgca   1020
gggcccgggc aaagcgcggc tgcgcgacgc acccgcggag catcgctgag agggagagga   1080
gaacgaggat tagcaagagg ctgaagaagc tgcaggatct cgtgccgaac atggacaagc   1140
aaacgaatac atcagacatg ttggatattg ctgtaaccta catcaaggag cttcagggcc   1200
aagttgagaa gctaaaacat gaccaagcaa actgcacttg ctcaggcaag cacgattgct   1260
gaggctggag aaccagatct ccgagctaat gttgtaatga tgtgattggt gaacccaaag   1320
ttttgcttca gaagattagg gtgtaaaagc acttcacatg tcaagtggcc tccatgcctc   1380
gccctcccct ttatcggttg tgttatggac agcgtcaatg acttgaagag taggcggtga   1440
caaagtagat taattgttgt attttaattg atgtaaggca gtggcttgta tgtaaaatca   1500
tccatttcaa cgccaatggg attgcttctt attatcattg ctcttattaa aagtatcctc   1560
ctctttggca ccatttttgt gaattaccga ctcattactg ttgtttttgc tgtaaatcac   1620
cttgtatgtc tccttttcgt ttttccaaca aacttattag ttgttgtgtc              1670
```

<210> 96
<211> 369
<212> PRT
<213> Oryza sativa

<400> 96

```
Met Asn Pro Ala Pro Ser Arg Ala Pro Gln Arg Gln Gln Arg Gly Gly
1               5                   10                  15
Glu Met Ser Ala Arg Tyr Gly Gly Gly Leu Gln Phe Phe Ala Asp Ala
            20                  25                  30
Pro Pro Ala Gly Val Glu Gly Gly Ala Ala Thr Ala Arg Thr Phe Phe
        35                  40                  45
Pro Val Pro Gly Gly Gly Gly Glu Gln Gln Pro Pro Glu Arg Ala Met
    50                  55                  60
Arg Gln Gln His Tyr Gly Gly Gly Gly Ser Gly Ala Ala Glu Ile Ser
65                  70                  75                  80
Leu Gly His Gly His Gly His Gly Gly Lys His His Phe His Gln Phe
                85                  90                  95
Gly Val Glu Ala Lys Asp Gly Gly Gly Gly Gly Asp Gln Ser Gly Phe
            100                 105                 110
Leu Thr Arg His Asn Ser Ser Pro Pro Gly Phe Phe Ser Ser Pro Val
        115                 120                 125
Met Asp Asn Gly Phe Ser Ser Ser Ala Arg Pro Ala Gly Ser Ser Leu
    130                 135                 140
Gly Glu Val Arg His Gly Ala Met Ser Ser Ser Ser Asn Asn Asn Lys
145                 150                 155                 160
Lys Met Lys Ala Pro Leu Ser Phe Ala Ser Ser Arg Gln Gly Ser Gly
                165                 170                 175
Gly Leu Ser Gln Ile Ser Glu Asp Gly Ile Pro Asp Leu Thr Asp Ser
            180                 185                 190
Ile His Gly Ala Ala His His His Gly Arg Ser Glu Glu Asn Val Ser
        195                 200                 205
Thr His Asp His Val Val Arg Ser Phe Ser Ser Gly Gly Phe Ser Ile
    210                 215                 220
Gly Ser Trp Glu Asp Ser Asn Ser Ile Val Phe Ser Thr Ser Thr Gly
225                 230                 235                 240
Lys Ser Gly Ala His Gly Asn Asp Asp Ile Ile Ala Thr Leu Ser Asn
                245                 250                 255
Tyr Glu Ser Gln Leu Val Ala Pro Arg Glu Met Ala Gly Val Glu Lys
            260                 265                 270
Tyr Leu Gln Met Gln His Asp Gln Val Pro Phe Arg Val Arg Ala Lys
        275                 280                 285
Arg Gly Cys Ala Thr His Pro Arg Ser Ile Ala Glu Arg Glu Arg Arg
    290                 295                 300
Thr Arg Ile Ser Glu Lys Leu Arg Lys Leu Gln Ala Leu Val Pro Asn
305                 310                 315                 320
Met Asp Lys Gln Thr Ser Thr Ser Asp Met Leu Asp Leu Ala Val Asp
                325                 330                 335
His Ile Lys Gly Leu Gln Ser Gln Leu Gln Thr Leu Lys Glu Asp Lys
            340                 345                 350
Glu Lys Cys Thr Cys Ser Cys Lys Gln Ala Ser Arg Asn Arg Pro Ala
        355                 360                 365
Asp
```

<210> 97
<211> 1500
<212> DNA
<213> Oryza sativa

<400> 97

```
atggctgcgg cggcggcggc gggccagatc tccctggacg acctccgcaa cggcggcggg      60
gtcgccgcca atgccggcgg cggcggcggc gtccacgacg acttcctcga ccagatgctc     120
agcagcctgc cgccctcggc gtggcccgac ctcgccgccg ggaaggcggc cgaggacgac     180
gccgaggga tgcatcacca ccaccaccag cagcagcagc agtttggtgg gccgtacgac     240
```

```
gagtcggcga tgctggcgtc gcggctccgg cagcaccaga tcagcggcgg cggaggagga    300
ggcggcggtg gcgcggccgc cgtgaagcag atggtgctgc agcagctcgc cgatctgagg    360
caggggcacc acatgatgct ccagggcttg gggggacggt cgccggcggg gggcggcggc    420
ggcggcggcg acggggggcct tctcctcccg ctcaccctcg gcagcggcgg atcgggcggc    480
gacgtgcagg cgttgctcaa ggccgccgcg gccaattccg ccggaggagg agacgccggc    540
ggcgtctacg gcggcggatt cgccggctcg ctccatcaac agcagcaaca tttccagcca    600
catccacaga cggcgccgac gattccgacg cagagcttcg gcggcggcgg cggcggagga    660
ggaggaggaa ccgcgtcagg cggcggggcg gcgcagcctc aggccggcgc ggctggagga    720
ggcgcgccgg cgccgccgcg gcagcgggtg cgggcacggc gaggccaggc caccgacccc    780
cacagcatcg ccgagcggct tcggagggag aggatcgccg agaggatgaa ggcactgcaa    840
gaactggttc ccaacgccaa caagttgatg cagacggaca aggcttccat gctggacgag    900
atcatcgact acgtcaagtt cctacaactc caagtcaagg caagcacgta cactaaatta    960
ctaatacatg ttttgagcat gagccggtta ggtggcgccg cagccgtcgc gccgctcgtg   1020
gccgacatgt cctcggaggg gcgaggcggc ggcgcggcga acggcggagc accggcggcg   1080
gcggggagcg atagcctgac ggtgacggag cagcaggtgg ccaagctgat ggaggaggac   1140
atgggcaccg ccatgcagta cctgcagggg aagggcctct gcctcatgcc gatctccctc   1200
gcgtccgcca tctcctcggc gacgtgccat ctccggccgc cggtggtcgc cgccgcgcag   1260
cagttcccgg ccgggctcgg cgccgccgct gccgccgcgc accaccacca actgagcgcg   1320
gcggcggcgg cggcggccat gcgcggacac ctgcccggcc tgaacgccga cggctccgtg   1380
ccggcctcgc cgagcatgtc ggtgctcacg gcgcagtcgg ccatggccaa cggcggcggg   1440
ggcgccgccg acggcgaggg gtcgcagctc aaggacgccg cctccgtctc caagccgtga   1500
```

<210> 98
<211> 399
<212> PRT
<213> Oryza sativa

<400> 98

```
Met Tyr Gly Ser Pro Val Ser Lys Asp Leu Asn Leu Pro Val Gln Pro
1               5                   10                  15
Pro Ala Met Ser Ser Ser Gly Leu Leu Arg Tyr Arg Ser Ala Pro Ser
            20                  25                  30
Thr Leu Leu Ala Glu Phe Cys Asp Asp Phe Leu Pro Pro Ala Ala Ala
        35                  40                  45
Pro Arg Ala Ala Ser Pro Asp Ala Asp Asn Val Phe Ser Arg Phe Leu
    50                  55                  60
Ala Asp His Gln Ile Arg Asp Lys Ser Pro Pro Ala Thr Ala Ala Ala
65                  70                  75                  80
Ala Ala Ala Ala Ala His Phe Pro Asp Asp Pro Thr Met Ala Thr Gln
                85                  90                  95
His His His Gln Gln Gln Met Met Phe Gln His His Pro Gln Gln Met
            100                 105                 110
Ala Ser Val Glu Gly Leu Tyr Arg Thr Val Ser Ser Thr Gly Ile Asp
        115                 120                 125
Ala Ala Thr Ala Ala Ala Asn Ala Ala Gly Gly Gly Gly Gly Gly Leu
    130                 135                 140
Leu Arg Gln Ser Ser Ser Pro Ala Gly Phe Leu Asn His Leu Asn Met
145                 150                 155                 160
Asp Asn Gly Tyr Gly Ser Met Leu Arg Ala Gly Met Ala Ala Ala Gly
                165                 170                 175
Gly Gly Val Gly Phe Arg Asn Gly Ala Asn Ala Ala Ala Ala Ala Asp
            180                 185                 190
Ser Pro Gly Gly Ser Gly Gly Arg Leu Lys Gly Gln Leu Ser Phe Ser
        195                 200                 205
Ser Arg Gln Gly Ser Leu Met Ser Gln Ile Ser Glu Met Asp Ser Glu
    210                 215                 220
Glu Leu Gly Gly Ser Ser Pro Glu Gly Ala Gly Gly Gly Gly Gly Gly
225                 230                 235                 240
Gly Gly Arg Gly Tyr Leu Ser Gly Tyr Pro Met Ser Ser Gly Trp Glu
```

```
                245                 250                 255
Glu Ser Ser Leu Met Ser Asp Thr Asn Ile Ser Gly Val Lys Arg Gln
            260                 265                 270
Arg Asp Ser Ser Glu Pro Ser Gln Asn Gly Gly Gly Gly Gly Gly Leu
        275                 280                 285
Ala His Gln Phe Ser Leu Pro Lys Thr Ser Ser Glu Met Ala Ala Ile
    290                 295                 300
Glu Lys Phe Leu Gln Phe Gln Asp Ala Val Pro Cys Lys Ile Arg Ala
305                 310                 315                 320
Lys Arg Gly Cys Ala Thr His Pro Arg Ser Ile Ala Glu Arg Val Arg
                325                 330                 335
Arg Thr Arg Ile Ser Glu Arg Ile Arg Lys Leu Gln Glu Leu Val Pro
            340                 345                 350
Asn Met Asp Lys Gln Thr Asn Thr Ala Asp Met Leu Asp Leu Ala Val
        355                 360                 365
Asp Tyr Ile Lys Asp Leu Gln Lys Gln Val Lys Gly Leu Asn Asp Ser
    370                 375                 380
Arg Ala Asn Cys Thr Cys Ser Ala Lys His Gln Gln Tyr Ser Gly
385                 390                 395

<210>  99
<211>  2031
<212>  DNA
<213>  Oryza sativa

<400>  99
gcatcccccc gcaaaagcaa tccgccgatt aaactaaccc aaaaaaaaaa gaaaaaaaac      60
tactcctcct cctcgcccaa ccccgcaaaa gcagcggcga cctcgcgaag ccaagccacc     120
accacttctt ccttcgctcg ctcgcgtatt ttcttttgct tttttgtgtg ctcatttcct     180
cgccgtaaag aagcgaggag gaggaagcgt agcttgagag gctggagaga tcgatcgaga     240
gagcttagca acagcagcaa agtgattgga gttgagcttg tccgtcttga aaagtcgctg     300
atcttttctt gtttactttc ttctttttat ttctttcttg tgttgctgaa ttgaggttgt     360
gtgggcggcc atggcgagga ggcggagata ggaggtgggt ggttgttgtt gttgtttgat     420
ttggtgggtt gaggtgggag aattggagtt ttgttggaga agagaggaga gaaggggatg     480
tacggctcgc cggtttccaa ggatctgaac ctcccggtgc agccgccggc gatgagctcg     540
tcgggcttgc tgcggtacag atcggcgccg agcacgctgc tcgccgagtt ctgcgacgac     600
ttcctcccgc ccgccgccgc gccccgcgcc gccagccccg acgccgacaa cgtcttctcc     660
cgcttcctcg ccgaccacca gatccgtgac aagtctcccc ccgccaccgc cgccgccgcc     720
gccgctgccg ctcacttccc cgacgacccc accatggcca cccagcacca ccaccagcag     780
cagatgatgt tccagcacca cccgcagcag atggcctctg tcgaggggct ctaccgcacc     840
gtcagctcca ccgggattga cgccgccacc gccgccgcca acgccgccgg tggcggcggc     900
ggcgggctgc tccggcagag tagctcgccc gccggattcc tcaatcattt gaacatggac     960
aacgggtacg ggagcatgct gcgggcgggc atggcggcag ccggcggtgg ggtggggttc    1020
aggaacggcg cgaacgccgc cgccgcggcc gactcccccg gcggcagcgg gggcaggctg    1080
aaggggcagc tcagcttctc gtcgcggcag ggctcgctca tgtcgcagat ctcggagatg    1140
gacagcgagg agctcggtgg gagcagcccc gagggcgccg gtggcggcgg cggcggcggt    1200
ggccggggtt acctctccgg gtacccgatg agctccgggt gggaggagtc gtcgctcatg    1260
tcggacacga acatctccgg cgtgaagcgc cagcgcgact cgtcggagcc gtcccagaac    1320
ggcggcggcg gcggcggcct ggcgcaccag ttcagcctgc cgaagacctc gtcggagatg    1380
gcggccatcg agaagttcct ccagttccag gacgcggtgc cctgcaagat ccgcgccaag    1440
cgcggctgcg ccacgcaccc gcgcagcatc gccgagcggg tgaggaggac aaggatcagc    1500
gagcgaatca ggaagctgca agaactcgtg ccaaacatgg acaagcaaac caacactgct    1560
gacatgctgg atttggctgt tgactacatc aaggatcttc agaagcaggt caagggatta    1620
aacgacagcc gagctaactg cacctgctca gcgaagcatc agcagtactc tggctagaaa    1680
gctttgtaca agtcatggag acttcagaaa agaacacaag attgttttgg agattaggca    1740
ggactgagac gatctctgaa gatgtgtaat ggttaatttc atttgtttgg gggggtttaa    1800
ttagagaggc acatggaaga aaaggctctt gtgtgccaaa gcactgtata agcaaagggc    1860
aatggagcag tggcatcatc aggattcaag tgataaaaag aaaaaggaaa aacaaggagg    1920
aaaaagggtg ggagatgggt gaaacaattg tatagttttc ttgtaacttt tgccatatat    1980
```

```
acccctgcat gccccatgtc cagtgcattt tcgatagaaa tttattactg c          2031

<210>  100
<211>  401
<212>  PRT
<213>  Oryza sativa

<400>  100
Met Tyr Gly Ala Pro Val Ser Lys Asp Leu Ser Leu Gln Pro Ala Gly
1               5                   10                  15
Val Arg Thr Pro Pro Gln Met Ser Ser Pro Gly Leu Leu Arg Tyr Arg
            20                  25                  30
Ser Ala Pro Ser Thr Leu Leu Gly Glu Val Cys Gly Asp Phe Val Leu
        35                  40                  45
Pro Gly Gly Gly Gly Gly Gly Gly Gln Leu Gln Leu Gln Leu Gln Gln
    50                  55                  60
Gln Arg Pro Gly Ser Pro Asp His Ala Ala Asp Thr Val Leu Ala Arg
65                  70                  75                  80
Phe Leu Ala Gly His Gly Gly His Asp Asn Lys Pro Pro Arg Pro Ala
                85                  90                  95
Ala His Phe Ala Pro Pro Glu Asp Ser Met Ala Ser His Gln Gln Gln
            100                 105                 110
Leu Met Tyr Gln Ser His Gln Gln Gln Gln Gln Met Ala Ser Ala Met
        115                 120                 125
Glu Gly Leu Tyr Arg Thr Val Ser Ser Gly Gly Thr Glu Ser Thr Ala
    130                 135                 140
Ala Ala Ala Gly Asn Ser Leu Leu Arg Gln Ser Ser Ser Pro Ala Gly
145                 150                 155                 160
Phe Leu Asn His Leu Thr Met Asp Asn Gly Tyr Gly Asn Met Leu Arg
                165                 170                 175
Ala Gly Met Gly Gly Gly Gly Gly Gly Gly Asp Pro Arg Leu Lys Gly
            180                 185                 190
Gln Leu Ser Phe Ser Ser Arg Gln Gly Ser Val Met Ser Gln Ile Ser
        195                 200                 205
Glu Met Gly Ser Glu Asp Glu Glu Leu Ala Gly Gly Gly Gly Ser Pro
    210                 215                 220
Glu Ala Gly Ser Asn Gly Gly Gly Ala Ala Arg Gly Gly Tyr Gly Gly
225                 230                 235                 240
Gly Tyr Ala Met Gly Ser Ser Ala Trp Glu Glu Pro Ser Pro Pro Ala
                245                 250                 255
Thr Ser Leu Leu Pro Asp Ser Ser Leu Pro Ser Lys Arg Pro Arg Asp
            260                 265                 270
Asp Leu Pro Arg Gln Leu Ser Leu Pro Ala Ala Ser Lys Asn Ser Ser
        275                 280                 285
Lys Pro Pro Ser Ser Ala Ser Ala Ala Ala Ser Pro Glu Met Ala Ala
    290                 295                 300
Ile Glu Lys Phe Leu Gln Phe Gln Asp Ala Val Pro Cys Lys Ile Arg
305                 310                 315                 320
Ala Lys Arg Gly Cys Ala Thr His Pro Arg Ser Ile Ala Glu Arg Val
                325                 330                 335
Arg Arg Thr Arg Ile Ser Glu Arg Ile Arg Lys Leu Gln Glu Leu Val
            340                 345                 350
Pro Asn Met Glu Lys Gln Thr Asn Thr Ala Asp Met Leu Asp Leu Ala
        355                 360                 365
Val Asp Tyr Ile Lys Glu Leu Gln Lys Gln Val Lys Val Leu Asn Asp
    370                 375                 380
Ser Arg Ser Ser Cys Thr Cys Ser Ala Ser Lys Gln Lys His Phe Ala
385                 390                 395                 400
Gly
```

<210> 101
<211> 1875
<212> DNA
<213> Physcomitrella patents

<400> 101
actcggtctt cgagatgatc cgagcggtgt ggtgagaagt acttcgaaat gggttccaag 60
tagttggaaa gcagtgtgca cgagtgaaag ctatacctgt agtgttccca agtgttgaac 120
agtgacggtt gctgatgtgg gaggtgcgag cttcagttgg ttggtcggtc ggtcggtcgg 180
tcggtcggtc agtcgatcag ctgcactagg acttgtagga aacctgaagg tgaatttatg 240
gctgggtaac gcagcacaga tagtcgtcct agttggcgcg cgttttcaaa aaagtgacta 300
aaagttcaag gtttgttgag aatgtgtggt aagagcgtgg ctgtcatgga tggagccgcg 360
agcaccatga tacagagcca ccgaggcact tcccaagcgt cttattcttc aggaaggagt 420
gctgtggatg aagagatctc ggcgatgttg cacagtggaa attactcgca cattagtcaa 480
gcaccgcttg ctgtgtccgt aggactagaa cattttcctt attctgtgta ccaaggttac 540
ccaggtaatc aaactcgacc tgttccgacg acgcaacgga atacaaacat gcctcgggga 600
gaaataggcg acgggaatgc acatgttggg cctctactgc acagaggatc tcctcctcgg 660
cattcaactt tcaacttgga agagaaatac gtgttggaag ctaaaagcga agcgttggac 720
gcaaacgtac tgcggaatgg tgctacttat ggcacaatct atgagaattc gagaccatcg 780
gcgatgagca cgactgtgcc cttgctgcac cgctaccatt cagctcctgc aagcacgtat 840
ttgacccaga tgaatgagga gcagatcggt aacaccgtta tgtcttcgat ttttacggat 900
ggagggctca cccccataac agaaaacatg gacgttgagc gattgggctg cgggaactca 960
aactcgaacg aattcgagca ttacttagcc ccagagaacg atttcagtcg ccgggctgcg 1020
ttttcggctc ttcacaggga agaggcaacg cccgattcct ttgcgaccac gttccagaat 1080
tccaagaatg cggtgttatg tcaatcaagc ttatcgtctg ctgggttgtc gcaacccaat 1140
atatccatgc aagataggat ggctgagaat ggctcggaaa agaacagctc tcgcaactcg 1200
gacgataata atcacctgat tagcggttac gcggcatcga tatattctca agaggactgt 1260
cactggaaat cgcctacaag tccagccaag aggcaacggg gactggacgg cgagagtgaa 1320
acctcgagcg gctcagtcga aggctcgacg tatcggaaaa gccaacaagg tggcttaatc 1380
cgtcacatga gcttaccaca ctcaaccaac ggagattcta gtagcccagg agtggaggac 1440
aacaccttcc acacggtccc tatgcgcacc cgtgcgaaac gaggatgcgc cacacacccc 1500
cgcagcatcg cagagcgcgt gcgcaggacg aagatcagcg agcggatgaa gaagctccag 1560
gacttggttc ccagcatgga caagcagacc aacacctccg acatgctaga cgagaccgtg 1620
gagtacgtga aatcgctgca gcgacaggtg caagagttga gcgacacggt ggtgcggctt 1680
gaagccgccg cagcacaaaa aatcttttca gattttcaga tccgtcaaaa gtatcttgca 1740
actcctaacc cttacagtgt agatgtgacg caagctgatg caactgccca tcgtctttct 1800
ggcaagcaac agattcaagt tggtacagtg atgagtggca ttcttgacca tgacaccata 1860
tcacatgatg tttaa 1875

<210> 102
<211> 95
<212> PRT
<213> Physcomitrella patents

<400> 102
Met Gly Tyr Asp Asp Leu Leu Asp Leu Tyr Ser Pro Cys Lys Thr Arg
1 5 10 15
Ala Arg Arg Gly Tyr Ala Thr His Pro Arg Ser Ile Ala Glu Arg Asn
20 25 30
Arg Arg Ser Arg Ile Ser Glu Arg Met Lys Lys Leu Gln Asp Leu Val
35 40 45
Pro Asn Met Asp Lys Gln Thr Asn Thr Ala Asp Met Leu Asp Glu Ala
50 55 60
Val Glu Tyr Val Lys His Leu Gln Thr Gln Val Lys Asp Leu Ser Glu
65 70 75 80
Thr Ile Val Arg Leu Lys Asn Ser Ile Arg Ala Gln Thr Ala Ser
85 90 95

<210> 103
<211> 246
<212> DNA
<213> Physcomitrella patents

<400> 103
```
atgcgagctc gtgcgaaacg tggatgcgct acccacccca gaagcattgc cgaacgtgtg     60
cgtcggacac gaattagtga gcgcatgaag aaacttcaag accttgtccc gaacatggag    120
aagacgacca acacatcaga catgcttgac gaaaccgtgg agtatgtgaa atccctgcaa    180
atgaaagtga aggaattaac ggagactatt gcacagctga aagctgccac acagatgagc    240
ccataa                                                               246
```

<210> 104
<211> 144
<212> PRT
<213> Physcomitrella patents

<400> 104
```
Met Glu Pro Pro Leu Asp Trp Ser Arg Thr Asn Ser Phe Arg Thr Ala
1               5                   10                  15
Ala Arg Val Gly Glu Ala Gly Pro Lys Leu Gly Gly Leu Ile Arg His
            20                  25                  30
Ser Ser Leu Pro Ala Pro Ser Arg Pro Phe Ser Gly Asn Val Asp Phe
        35                  40                  45
Asp Asp Leu Phe Ala Asp Pro Ser Ala Val Pro Leu Lys Thr Ile Arg
    50                  55                  60
Ala Asn Arg Gly His Ala Thr His Pro Arg Ser Ile Ala Glu Arg Val
65                  70                  75                  80
Arg Arg Gly Lys Ile Ser Glu Arg Met Lys Lys Leu Gln Asp Leu Val
                85                  90                  95
Pro Ser Met Asp Arg Gln Thr Asn Thr Ala Asp Met Leu Asp Asp Ala
            100                 105                 110
Val Glu Tyr Val Lys Gln Leu Gln Gln Gln Val Gln Glu Leu Ser Lys
        115                 120                 125
Thr Val Ala Glu Leu Gln Gln Leu Gln Glu Arg Leu Gly Arg Arg Asp
    130                 135                 140
```

<210> 105
<211> 715
<212> DNA
<213> Physcomitrella patents

<400> 105
```
gcaagagcag ctctcgtgat tcagacgata acaaccacct catcaacggt tacacagcat     60
tgatatacac tcaagaagat tttacctgga agtcgcctac aagtccagcc aagaggcaac    120
ggggtctaga cggcgatagc gaaacatcaa gtggctcagc agagggatca acctaccgca    180
actgccaaca cagcggccta gttcgtcaca tgagcctgcc ctcaacaaac ggcggtccca    240
acagccccgg cctggaggat aaccactacg gcgccgtccc catgcgcact cgtgcgaaga    300
gaggctgcgc cacgcatccc cgcagcattg cagagcgcgt ccgcagaacc aagatcagcg    360
aacgcatgaa gcggctccag gacctagtgc ctaacatgga caagcagact aacacctccg    420
acatgcttga cgagaccgtg gaatacgtga aatcgctcca gcgaaaggtg caggagctca    480
gcgatactgt ggcgcggctt aaggctgacg cctcgcagag agcaaaaaat tcgaataata    540
acaactcaag taactagttc gacgatattc agtctagtct ggatagatag ccgtagctta    600
caaagatttc accacaacga ttcgacggtg tgattcaccg cgctcgaatc aatctcggag    660
aagttcgagt gtaacatact gcagcaagga agatacgtta aaccggacac gggtt         715
```

<210> 106
<211> 216

<212> PRT
<213> Physcomitrella patents

<400> 106
Met Arg Cys Tyr Val Asn Gln Ala Tyr Cys Leu Leu Gly Cys Arg Asn
1 5 10 15
Pro Asn Ile Ser Met Gln Asp Arg Met Ala Glu Asn Gly Ser Glu Lys
20 25 30
Asn Ser Ser Arg Asn Ser Asp Asp Asn Asn His Leu Ile Ser Gly Tyr
35 40 45
Ala Ala Ser Ile Tyr Ser Gln Glu Asp Cys His Trp Lys Ser Pro Thr
50 55 60
Ser Pro Ala Lys Arg Gln Arg Gly Leu Asp Gly Glu Ser Glu Thr Ser
65 70 75 80
Ser Gly Ser Val Glu Gly Ser Thr Tyr Arg Lys Ser Gln Gln Gly Gly
85 90 95
Leu Ile Arg His Met Ser Leu Pro His Ser Thr Asn Gly Asp Ser Ser
100 105 110
Ser Pro Gly Val Glu Asp Asn Thr Phe His Thr Val Pro Met Arg Thr
115 120 125
Arg Ala Lys Arg Gly Cys Ala Thr His Pro Arg Ser Ile Ala Glu Arg
130 135 140
Val Arg Arg Thr Lys Ile Ser Glu Arg Met Lys Lys Leu Gln Asp Leu
145 150 155 160
Val Pro Ser Met Asp Lys Gln Thr Asn Thr Ser Asp Met Leu Asp Glu
165 170 175
Thr Val Glu Tyr Val Lys Ser Leu Gln Arg Gln Val Gln Glu Leu Ser
180 185 190
Asp Thr Val Val Arg Leu Glu Ala Ala Ala Ala Gln Lys Val Ser Asn
195 200 205
Thr Ser His Ser Pro Glu Ile Ala
210 215

<210> 107
<211> 708
<212> DNA
<213> Physcomitrella patents

<220>
<221> misc_feature
<222> (699)..(699)
<223> n is a, c, g, or t

<400> 107
gtggactcaa agcggctatg tattcacagg acgatttgtt gaacaacgat actgtgactt 60
ctatgccaac aagcccaggc actccagggg gaaagaggca acgagggttt gtgggagaaa 120
acgagatatc gagtggctcg gagttgaact ccaaatcata ccggctgaac aataaccagt 180
ccactggtct tatccgccac atgagcttgc caacatctgg agatgtgcca aacatgagcc 240
ttaacactga tgaccattac gtccagatgc gagctcgggc caagcgtgga tgtgccaccc 300
accccaggag catagcggag cgcgtgaggc ggactcgcat cagtgagcga atgaagaaat 360
tgcaggacct ggtgcccaat atggaaaaga cgaccaacac ggctgatatg ctggacgaaa 420
ctgtggagta tgtgaagtcg ctgcaagtga aagtttccga gctccaagag acgattgcga 480
agctcaaagc tgctagcgcc actcaaatca cgagctcatg agtgggacga ctgatttctg 540
cgtatttctc ggcgttttgt aacatatcat gaattcgacg ttaccattga aatctgactc 600
acattccagg tttgcgaaac ttgtacagtc gatcggtatt aagtttggag ttcgttggtg 660
ccgtcccctc cggttgtaaa tgcacggatt gttgggtgna aagcaaac 708

<210> 108
<211> 167

```
<212>  PRT
<213>  Physcomitrella patents

<400>  108
Met Tyr Ser Gln Asp Asp Leu Leu Asn Asn Asp Thr Val Thr Ser Met
1               5                   10                  15
Pro Thr Ser Pro Gly Thr Pro Gly Gly Lys Arg Gln Arg Gly Phe Val
            20                  25                  30
Gly Glu Asn Glu Ile Ser Ser Gly Ser Glu Leu Asn Ser Lys Ser Tyr
        35                  40                  45
Arg Leu Asn Asn Asn Gln Ser Thr Gly Leu Ile Arg His Met Ser Leu
    50                  55                  60
Pro Thr Ser Gly Asp Val Pro Asn Met Ser Leu Asn Thr Asp Asp His
65                  70                  75                  80
Tyr Val Gln Met Arg Ala Arg Ala Lys Arg Gly Cys Ala Thr His Pro
                85                  90                  95
Arg Ser Ile Ala Glu Arg Val Arg Arg Thr Arg Ile Ser Glu Arg Met
            100                 105                 110
Lys Lys Leu Gln Asp Leu Val Pro Asn Met Glu Lys Thr Thr Asn Thr
        115                 120                 125
Ala Asp Met Leu Asp Glu Thr Val Glu Tyr Val Lys Ser Leu Gln Val
    130                 135                 140
Lys Val Ser Glu Leu Gln Glu Thr Ile Ala Lys Leu Lys Ala Ala Ser
145                 150                 155                 160
Ala Thr Gln Ile Thr Ser Ser
                165

<210>  109
<211>  1574
<212>  DNA
<213>  Pinus taeda

<400>  109
cggccgggaa gtcaaatgaa ttttatgagg cagggtagtt ctacttcagg gctttcctct      60
cagctgagtg acatgggttt gccacactgt ggataatctt aatatgcccg tgggctcttc     120
cgctgatagc ttgggtggga gcagttcaga tgatggtagt ttaagcaatg gcaatggagt     180
ccaacgttac atgtcaagct tccgggttgg atcatgggat gatacagtga tagtttctga     240
gggttttgca ggcatgcaag atggggcagc cttttctgcg aggaagcgtg taagagatct     300
gaatgggaaa atgatgcctg ctttgaatct agcagagtca cagaagggag agccaagtag     360
tcatgttccc atgggattaa atcatcagtt cagcctaacc aaatcacctt ccgaaattgc     420
aatggagaag ctactccagg actcggttcc ttgcaagatt cgagcaaagc gaggctgtgc     480
aacacatcca cgaagtatcg cagaaagggt ccgaagaaca cgcatcagtg aaaagatgag     540
aaatttgcag caacttgtgc caaacatgga caagcaaacc aacactgcag acatgttaga     600
tgaagcagtt gattatatca agttccttca gaaacaagtt caggaacttt ctgaaaatga     660
gcccaaatgc aattgctctt gtaaaatgaa tgcatcagaa actacttaaa aatctagcag     720
acttggggag attaatggaa tgtatgtatc ttgaagccat gctttcaagg aattgaagga     780
aacaaccagc aaccctgttc taaatatgtc ctctcaaaga catacacgac cccgttttcc     840
cttgtggaaa gtaagagtct atccaatctt tctcctgatc ctgtaaaggt cagacaattg     900
tgctgcaatg ttggggtgtg cttgggttct tctatgtttt tgttgagtac ctaaaaaaaa     960
ctggcccttc ttttattttc ctttgccagt ttctttttca gaattcagac ttcccaatta    1020
ttatggaata tcattcagtc ttgtatggtt tttcgcaacc atgccagaac tttagtgatt    1080
tggttgacgt tggtttctct caaccaaaga agcccgtttt tgatgttttc cagcatcatt    1140
atgtaagagt gacatcaagt gtaagactta ggatgctgat tgtagtcaac ttgtcaggct    1200
gatttttctt tttttggtgg aactgatgtt gtacatgcat tcagtgtaat taagacatag    1260
gggtggattt tatgcagact tgagcaacga ctatgctact tttcaaattt ccaaggatcc    1320
tcatccatct gaaaagcatg gcagattgtt cttgtaaact gtgaagtcac attcctgcca    1380
aagattggct catgttagcg tctatgtgct gttaactcga cgtgtaaaat tctggccatt    1440
tgggcttctc gtgaatatgg aaaaatcttt tgaatcaaga agctcatgct ttaccaagca    1500
atatcatgct ttgaacttac tataatagga tttatgattt tatcttggtt cagtaatgaa    1560
```

acaaactttc ctgg 1574

<210> 110
<211> 201
<212> PRT
<213> Pinus taeda

<400> 110
Met Pro Val Gly Ser Ser Ala Asp Ser Leu Gly Gly Ser Ser Ser Asp
1               5                   10                  15
Asp Gly Ser Leu Ser Asn Gly Asn Gly Val Gln Arg Tyr Met Ser Ser
            20                  25                  30
Phe Arg Val Gly Ser Trp Asp Asp Thr Val Ile Val Ser Glu Gly Phe
        35                  40                  45
Ala Gly Met Gln Asp Gly Ala Ala Phe Ser Ala Arg Lys Arg Val Arg
    50                  55                  60
Asp Leu Asn Gly Lys Met Met Pro Ala Leu Asn Leu Ala Glu Ser Gln
65                  70                  75                  80
Lys Gly Glu Pro Ser Ser His Val Pro Met Gly Leu Asn His Gln Phe
                85                  90                  95
Ser Leu Thr Lys Ser Pro Ser Glu Ile Ala Met Glu Lys Leu Leu Gln
            100                 105                 110
Asp Ser Val Pro Cys Lys Ile Arg Ala Lys Arg Gly Cys Ala Thr His
        115                 120                 125
Pro Arg Ser Ile Ala Glu Arg Val Arg Arg Thr Arg Ile Ser Glu Lys
    130                 135                 140
Met Arg Asn Leu Gln Gln Leu Val Pro Asn Met Asp Lys Gln Thr Asn
145                 150                 155                 160
Thr Ala Asp Met Leu Asp Glu Ala Val Asp Tyr Ile Lys Phe Leu Gln
                165                 170                 175
Lys Gln Val Gln Glu Leu Ser Glu Asn Glu Pro Lys Cys Asn Cys Ser
            180                 185                 190
Cys Lys Met Asn Ala Ser Glu Thr Thr
        195                 200

<210> 111
<211> 717
<212> DNA
<213> Populus tremula

<400> 111
ccaggcaagg tacatgtcta ggttctctag tgattcttgg gacggtgttt ctctgagtgg 60
tctcaagaga cagagggatc acgacgggaa catgttttct ggcctaaaca cactggacaa 120
tcaggatgga aattctggaa accgagcgac tggtttgact caccacttga gcttgcccaa 180
gactgtttca gaaatggcta ccatcgagaa gtttttggat tttcaaggca attctgtccc 240
gtgtaagatt cgagccaaaa gaggtttcgc tacccaccca cgaagcatag cagagagggt 300
aagaagaact cggattagtg aaagaatgag aaaattgcaa gaacttttcc caaacatgga 360
caagcaaaca aacacagcag atatgttaga tttggcagtt gagcacatta aagatctaca 420
gaaacaagct aagaccctca cggatacgaa agcgaagtgc acgtgttcta gtaaacagaa 480
gcaatattca agtccctcag cttgattcta gtttctgtac agatagcact aggacaatgg 540
caagacaaaa aacaagcaca tagctaccta gttttgatct tctgtaaaat aataggtatc 600
aggagacagt gagctttaag ggtaatttga tatacacttt tctctgtaat atttgtagt 660
gctggtctcg gcattctatc gacagcaatg ccctttcaaa agatagatct acagctc 717

<210> 112
<211> 163
<212> PRT
<213> Populus tremula

<400> 112
Met Ser Arg Phe Ser Ser Asp Ser Trp Asp Gly Val Ser Leu Ser Gly
1 5 10 15
Leu Lys Arg Gln Arg Asp His Asp Gly Asn Met Phe Ser Gly Leu Asn
20 25 30
Thr Leu Asp Asn Gln Asp Gly Asn Ser Gly Asn Arg Ala Thr Gly Leu
35 40 45
Thr His His Leu Ser Leu Pro Lys Thr Val Ser Glu Met Ala Thr Ile
50 55 60
Glu Lys Phe Leu Asp Phe Gln Gly Asn Ser Val Pro Cys Lys Ile Arg
65 70 75 80
Ala Lys Arg Gly Phe Ala Thr His Pro Arg Ser Ile Ala Glu Arg Val
85 90 95
Arg Arg Thr Arg Ile Ser Glu Arg Met Arg Lys Leu Gln Glu Leu Phe
100 105 110
Pro Asn Met Asp Lys Gln Thr Asn Thr Ala Asp Met Leu Asp Leu Ala
115 120 125
Val Glu His Ile Lys Asp Leu Gln Lys Gln Ala Lys Thr Leu Thr Asp
130 135 140
Thr Lys Ala Lys Cys Thr Cys Ser Ser Lys Gln Lys Gln Tyr Ser Ser
145 150 155 160
Pro Ser Ala

<210> 113
<211> 803
<212> DNA
<213> Populus tremula

<400> 113
gaaatcggag aagaatgtat tggtgggagc agtccagaag gcgattttag caagagaaag 60
tacatgtata acttcaatag cgatacttgg ggtgatgctt cgagattgaa ggataatgat 120
gggaacatgt ttctgccct gaacacacgg gaaagtcagg ttgggaattc tggaaaccgc 180
atgactggtt tgactcatca cttgagcttg cctaagactt ttgcagaaat ggctatggcg 240
gagaagtttt tggatttcca aggcaatttt gttccgttta agatccgagc caagagaggt 300
ttcgctactc acccacgaag catagcagag agggtaagaa gaactcggat tagtgaaaga 360
atgagaaaat tgcaagaact tttcccagac atggataagc aaacaagcac cgcagataag 420
ctagatttgt caattgagct catcaaagac ctccagaaac aagtcaagac tctcacggat 480
actaaagcca agtgcacgtg ttcaagtaaa cagaaataat attcaagtac ccctgcttga 540
ttttgtttct gtacagatag ccctaggaaa atgaaaaaaa gagaaaaaaa gacatagctc 600
cttagttatc ttctgtaaaa taatagggta tcggagattg tgaactcaag gataagtaga 660
tacacttttc actgtaatat tctgtaccac tgctgatctc attacacggt accggaagca 720
gtgccctttc atagctcatc ttcggattgt attttcctgt ttgtagcttt tgatgtgtta 780
agcaaggttg ttattgcatt gcc 803

<210> 114
<211> 151
<212> PRT
<213> Populus tremula

<400> 114
Met Tyr Asn Phe Asn Ser Asp Thr Trp Gly Asp Ala Ser Arg Leu Lys
1 5 10 15
Asp Asn Asp Gly Asn Met Phe Ser Ala Leu Asn Thr Arg Glu Ser Gln
20 25 30
Val Gly Asn Ser Gly Asn Arg Met Thr Gly Leu Thr His His Leu Ser
35 40 45
Leu Pro Lys Thr Phe Ala Glu Met Ala Met Ala Glu Lys Phe Leu Asp
50 55 60

```
Phe Gln Gly Asn Phe Val Pro Phe Lys Ile Arg Ala Lys Arg Gly Phe
65                  70                  75                  80
Ala Thr His Pro Arg Ser Ile Ala Glu Arg Val Arg Arg Thr Arg Ile
                85                  90                  95
Ser Glu Arg Met Arg Lys Leu Gln Glu Leu Phe Pro Asp Met Asp Lys
            100                 105                 110
Gln Thr Ser Thr Ala Asp Lys Leu Asp Leu Ser Ile Glu Leu Ile Lys
        115                 120                 125
Asp Leu Gln Lys Gln Val Lys Thr Leu Thr Asp Thr Lys Ala Lys Cys
    130                 135                 140
Thr Cys Ser Ser Lys Gln Lys
145                 150

<210>  115
<211>  845
<212>  DNA
<213>  Populus trichocarpa

<400>  115
gatttagaga attctaagca agcaacaaag atgagtacta gaaatggatc taatcttgct      60
aggcaaaaca gctctccagc tggaattttg tccaaccatg gcgttgataa tggctttgct     120
gtaatgagaa atgcgggtag cttcagagct ggcaatggca cgaatggaga agctactcca     180
tcaactagta ggttaagaag tcacgtgaat ttctcgtcag ggcataggac tttgcctcag     240
attgctgaaa ttggagaaga atgtattggt gggagcagtc cagaaggcga ttttagcaag     300
agaaagtaca tgtataactt caatagtgat acttggggtg atgcttcgag attgaaggat     360
aatgatggga acatgttttc tggcctgaac agacgggaaa gtcaggttgg gaattctgga     420
aaccgcatga ctggtttgac tcatcacttg agcttgccta agactgttgc agaaatggct     480
acggccgaga agtttttgga tttccaaggc aattttgttc cgtgtaagat tcgagccaag     540
agaggtttcg ctactcaccc acgaagcata gcagagaggg taagaagaac tcggattagt     600
gaaagaatga gaaaattgca agaacttttc ccagacatgg ataagcaaac aagcaccgca     660
gataagctag atttgtcaat tgagctcatc aaagacctcc agaaacaagt taagagtctc     720
gcggatacta aagctaagtg cacgtgttca agtaaacaga aataatattc aagtacctca     780
gcttgatttt gtttctgtac agattgcact aggaaaaaga aaaaaagaag aaaaagcata     840
gctcc                                                                 845

<210>  116
<211>  244
<212>  PRT
<213>  Populus trichocarpa

<400>  116
Met Ser Thr Arg Asn Gly Ser Asn Leu Ala Arg Gln Asn Ser Ser Pro
1               5                   10                  15
Ala Gly Ile Leu Ser Asn His Gly Val Asp Asn Gly Phe Ala Val Met
            20                  25                  30
Arg Asn Ala Gly Ser Phe Arg Ala Gly Asn Gly Thr Asn Gly Glu Ala
        35                  40                  45
Thr Pro Ser Thr Ser Arg Leu Arg Ser His Val Asn Phe Ser Ser Gly
    50                  55                  60
His Arg Thr Leu Pro Gln Ile Ala Glu Ile Gly Glu Glu Cys Ile Gly
65                  70                  75                  80
Gly Ser Ser Pro Glu Gly Asp Phe Ser Lys Arg Lys Tyr Met Tyr Asn
                85                  90                  95
Phe Asn Ser Asp Thr Trp Gly Asp Ala Ser Arg Leu Lys Asp Asn Asp
            100                 105                 110
Gly Asn Met Phe Ser Gly Leu Asn Arg Arg Glu Ser Gln Val Gly Asn
        115                 120                 125
Ser Gly Asn Arg Met Thr Gly Leu Thr His His Leu Ser Leu Pro Lys
    130                 135                 140
```

```
Thr Val Ala Glu Met Ala Thr Ala Glu Lys Phe Leu Asp Phe Gln Gly
145                 150                 155                 160
Asn Phe Val Pro Cys Lys Ile Arg Ala Lys Arg Gly Phe Ala Thr His
                165                 170                 175
Pro Arg Ser Ile Ala Glu Arg Val Arg Arg Thr Arg Ile Ser Glu Arg
            180                 185                 190
Met Arg Lys Leu Gln Glu Leu Phe Pro Asp Met Asp Lys Gln Thr Ser
        195                 200                 205
Thr Ala Asp Lys Leu Asp Leu Ser Ile Glu Leu Ile Lys Asp Leu Gln
    210                 215                 220
Lys Gln Val Lys Ser Leu Ala Asp Thr Lys Ala Lys Cys Thr Cys Ser
225                 230                 235                 240
Ser Lys Gln Lys


<210>  117
<211>  1379
<212>  DNA
<213>  Populus trichocarpa

<400>  117
tagaaactta atcaagaaac aggggattca atggagtcag atcttcaaca ccagcatcat      60
tttcttcatg gtcataacca gcaccaacaa attcatcaga aacaaatgaa ttctgggttg     120
acaagatatc agtctgcacc aagttcatat tttcaagta atttagacag agacttctgt     180
gaagagtttc tcaataggcc aacaagccct gaaacagaac gaattttcgc gagattttta     240
gccaattccg gtggcaatac agagaatata ccaggttcaa atctttgtga gattaagcaa     300
gattctccag taaaagaatc agtctcacaa attaaccagc aaccccagat gatggcttca     360
atgaataatc atagtagtga tacaaggctg catcaacatc aacatcagca gcaccaacat     420
ggcaattact ctgcctcaca gggtttttat caaagtcgat caaaacctcc attaccagat     480
cataatccag gttctggtat gaatcataga tcaacgaatt caacggggtt ggagaggatg     540
ccttccatga agcctagcag tggaaacaat ccgaatctag ttcgacacag tagctcacca     600
gcagggcttt tctccaacat aaatattgaa tttgaaaatg gctatgctgt attgagagat     660
gtgggagatc ttggagcagg taatagagac acaacttatt ctgcagctgg taggccacct     720
tcatcttcag ggataaggag tacaattgct gaaatgggaa acaaaaacat gggagaaaat     780
agcccagaca gtggtggttt tggtgaaact cctggcaata attatgatta ccctattgga     840
tcatgggatg attcggctgt gatgtctact gggtcaaaaa gataccttac agatgatgac     900
agaacacttt ctggtctaaa ttcatcggaa actcagcaga atgaagaggc aggaaatcgc     960
cctccaatgt tggctcatca cttgagcttg ccaaaaacat cagcagagat gtctaccatt    1020
gaaaactttt tgcaatttca agattctgtt ccttgtaaga tcagagccaa gcgtggttgt    1080
gccacccatc caagaagcat tgctgagagg gtgagaagaa ctagaattag tgaacgaatg    1140
aggaaactcc aggaccttgt accaaacatg gacaagcaaa caaacacatc agacatgctg    1200
gatttggctg ttgactacat taaagacctt caaagacaat tcaaggcact ttcagagaat    1260
cgtgcaagat gtacatgctt aaagaagcag cagccctagc tgaaaaaacc aaagtaagaa    1320
ttggtttgta tgtatagaac taataagaat ttgcaagacg agagataaag gcgatgcta     1379

<210>  118
<211>  422
<212>  PRT
<213>  Populus trichocarpa

<400>  118
Met Glu Ser Asp Leu Gln His Gln His His Phe Leu His Gly His Asn
1               5                   10                  15
Gln His Gln Gln Ile His Gln Lys Gln Met Asn Ser Gly Leu Thr Arg
            20                  25                  30
Tyr Gln Ser Ala Pro Ser Ser Tyr Phe Ser Ser Asn Leu Asp Arg Asp
        35                  40                  45
Phe Cys Glu Glu Phe Leu Asn Arg Pro Thr Ser Pro Glu Thr Glu Arg
    50                  55                  60
```

```
Ile Phe Ala Arg Phe Leu Ala Asn Ser Gly Gly Asn Thr Glu Asn Ile
65                  70                  75                  80
Pro Gly Ser Asn Leu Cys Glu Ile Lys Gln Asp Ser Pro Val Lys Glu
                85                  90                  95
Ser Val Ser Gln Ile Asn Gln Gln Pro Gln Met Met Ala Ser Met Asn
            100                 105                 110
Asn His Ser Ser Asp Thr Arg Leu His Gln His Gln His Gln Gln His
        115                 120                 125
Gln His Gly Asn Tyr Ser Ala Ser Gln Gly Phe Tyr Gln Ser Arg Ser
    130                 135                 140
Lys Pro Pro Leu Pro Asp His Asn Pro Gly Ser Gly Met Asn His Arg
145                 150                 155                 160
Ser Thr Asn Ser Thr Gly Leu Glu Arg Met Pro Ser Met Lys Pro Ser
                165                 170                 175
Ser Gly Asn Asn Pro Asn Leu Val Arg His Ser Ser Ser Pro Ala Gly
            180                 185                 190
Leu Phe Ser Asn Ile Asn Ile Glu Phe Glu Asn Gly Tyr Ala Val Leu
        195                 200                 205
Arg Asp Val Gly Asp Leu Gly Ala Gly Asn Arg Asp Thr Thr Tyr Ser
    210                 215                 220
Ala Ala Gly Arg Pro Pro Ser Ser Ser Gly Ile Arg Ser Thr Ile Ala
225                 230                 235                 240
Glu Met Gly Asn Lys Asn Met Gly Glu Asn Ser Pro Asp Ser Gly Gly
                245                 250                 255
Phe Gly Glu Thr Pro Gly Asn Asn Tyr Asp Tyr Pro Ile Gly Ser Trp
            260                 265                 270
Asp Asp Ser Ala Val Met Ser Thr Gly Ser Lys Arg Tyr Leu Thr Asp
        275                 280                 285
Asp Asp Arg Thr Leu Ser Gly Leu Asn Ser Ser Glu Thr Gln Gln Asn
    290                 295                 300
Glu Glu Ala Gly Asn Arg Pro Pro Met Leu Ala His His Leu Ser Leu
305                 310                 315                 320
Pro Lys Thr Ser Ala Glu Met Ser Thr Ile Glu Asn Phe Leu Gln Phe
                325                 330                 335
Gln Asp Ser Val Pro Cys Lys Ile Arg Ala Lys Arg Gly Cys Ala Thr
            340                 345                 350
His Pro Arg Ser Ile Ala Glu Arg Val Arg Arg Thr Arg Ile Ser Glu
        355                 360                 365
Arg Met Arg Lys Leu Gln Asp Leu Val Pro Asn Met Asp Lys Gln Thr
    370                 375                 380
Asn Thr Ser Asp Met Leu Asp Leu Ala Val Asp Tyr Ile Lys Asp Leu
385                 390                 395                 400
Gln Arg Gln Phe Lys Ala Leu Ser Glu Asn Arg Ala Arg Cys Thr Cys
                405                 410                 415
Leu Lys Lys Gln Gln Pro
            420
```

```
<210>  119
<211>  1139
<212>  DNA
<213>  Populus trichocarpa

<400>  119
tcgtgggaag aagcctgcgc agcacgcatc atgtatcaac aaccgtcgtc gtcttcttct      60
tcaccgcaaa aatccaatct gccaagtggg ctaacaaggt atggttcggc cccgggttct     120
tttttaaccc gagcagttga ttcagtcatc ggggctgacc gtgaactctc tggtcttgga     180
tcaactcccc tcgtgggccg tcaacaacac ttctctgggg actcaccctc aatcacctcg     240
gagtcgacct gcaaagtcaa ctcatccagc tgcgatcgaa aagcacccaa aagtggcggc     300
ggcgggggt tgcagaggtc gtacggtttg aatgagatag cacatggtgc tggatctttg     360
```

```
gtgcggcaga ggagctcgcc tgctggattt ctaagccatc tcgccactga aaatggaggt    420
ttctcaatta caaggggaac tggaggctat aactctcgca atggctctgg tggtggcccc    480
tcaaggttga agtctcaatt gagcttcact agacaagact ctctttctca gatatctgaa    540
gttagtgaga atgttgttga aggcatcggc tctgacaatg gcagtcagaa ctccactcat    600
tcttattctg cggctagctt tggtatggaa tcttgggaca ctccaaattc cattgtattt    660
tctggccatc cctcgaaaca agctaggact ggagatggag acatttatag ctgtttcaat    720
gcgttagaaa ctcagtttag cctgccccag acaagtcttg agatggcaac agtggagaag    780
ctgctacaaa ttcctgaaga ctctgttcct tgcaaaatcc gtgctaaacg tggttgtgcc    840
actcatccta gaagcattgc tgaaagggag agaagaacca gaataagtgg gaagctgaag    900
acactacaag acctcgttcc taacatggat aagcaaacaa gttatgcaga catgttggag    960
ttggcagtga agcatataaa gggccttcaa aatgaagtgg agaaactcca caaagaattg   1020
gaaggttgta cttgtggatg caaacaatca accccatgat attgccaaca aaggagatct   1080
aaatcgaccg gcaaaatcta tgctttttga atgaaaattt ttcacttcac ggtaaggtt    1139

<210>  120
<211>  421
<212>  PRT
<213>  Populus trichocarpa

<400>  120
Met Asn Leu Leu Tyr Ser Ser Ser Phe Lys Tyr Ser Asp Gly Glu Leu
1               5                   10                  15
Arg Lys Ser Gln Glu Phe Met Asp Leu Asn Pro Tyr His Tyr His Gln
            20                  25                  30
Gln Gln Gln Gln Ile Gln Gln Asn Ser Gly Leu Met Arg Tyr Arg Ser
        35                  40                  45
Ala Pro Ser Ser Ile Leu Glu Ser Leu Val Asn Gly Thr Ser Gly His
    50                  55                  60
Asp Gly Gly Gly Ile Glu Ser Gly Asp Tyr Arg Tyr Leu Arg Ser Ser
65                  70                  75                  80
Ser Pro Glu Met Asp Thr Met Leu Ala Arg Phe Met Ser Ser Cys Asn
                85                  90                  95
Gly Ser Gly Asp Ser Ser Ser Gln Asn Leu Gln Glu Phe Gly Glu Arg
            100                 105                 110
Pro Ala Ile Lys Gln Glu Gly Gly Asp Ser Glu Met Val Tyr Gln Ser
        115                 120                 125
Leu Pro Gly His Asn Leu Val Thr Asp Asn Ser Val Ser Val Gly Asn
    130                 135                 140
Ser Met Asp Ser Ala Phe Asn Val Met Ser Ser Met Ala Leu Glu Asn
145                 150                 155                 160
Ser Met Gln Ala Thr Lys Met Ser Thr Ala Asn Gly Ser Asn Leu Ala
                165                 170                 175
Arg Gln Asn Ser Ser Pro Ala Gly Leu Phe Ser Asp Leu Gly Val Asp
            180                 185                 190
Asn Gly Phe Val Val Met Arg Glu Gly Gly Ser Phe Arg Ala Gly Asn
        195                 200                 205
Gly Thr Asn Gly Glu Ala Ser Pro Thr Asn Lys Leu Arg Arg His Val
    210                 215                 220
Asn Phe Ser Ser Gly Gln Arg Met Leu Pro Gln Ile Ala Glu Ile Gly
225                 230                 235                 240
Glu Glu Cys Ile Gly Gly Arg Ser Pro Glu Gly Asp Val Ser Glu Ala
                245                 250                 255
Arg Tyr Met Ser Arg Phe Thr Ser Asp Ser Trp Asp Gly Ala Ser Leu
            260                 265                 270
Ser Gly Leu Lys Arg Gln Arg Asp Asn Asp Gly Asn Met Phe Ser Gly
        275                 280                 285
Leu Asn Thr Leu Asp Asn Gln Asp Gly Asn Ser Gly Asn Arg Val Thr
    290                 295                 300
Gly Leu Thr His His Leu Ser Leu Pro Lys Thr Leu Ser Glu Thr Ala
```

```
305                 310                 315                 320
Thr Ile Glu Lys Phe Leu Asp Phe Gln Gly Asn Ser Val Pro Cys Lys
                325                 330                 335
Ile Arg Ala Lys Arg Gly Phe Ala Thr His Pro Arg Ser Ile Ala Glu
            340                 345                 350
Arg Val Arg Arg Thr Arg Ile Ser Glu Arg Met Arg Lys Leu Gln Glu
        355                 360                 365
Leu Phe Pro Asn Met Asp Lys Gln Thr Asn Thr Ala Asp Met Leu Asp
    370                 375                 380
Leu Ala Val Glu His Ile Lys Asp Leu Gln Lys Gln Val Lys Thr Leu
385                 390                 395                 400
Thr Asp Thr Lys Ala Lys Cys Thr Cys Ser Ser Lys Gln Lys His Tyr
                405                 410                 415
Ser Ser Pro Ser Ala
            420
```

<210> 121
<211> 1376
<212> DNA
<213> Populus trichocarpa

<400> 121

```
ggtggcttgc agggatcctc aaaagaagac atgaatcttt tgtacagttc tagtttcaag     60
tattcagatg gagaattgag aaagagtcaa gaattcatgg atttaaatcc ctatcactac    120
catcaacaac aacaacaaat ccagcaaaat tctggtctga tgcgttatcg ctctgcgcca    180
agctcaatcc tggagagcct tgtgaatggt acaagtggcc atgatggtgg tggcatcgaa    240
agcggggatt atcggtatct tcgatcttca agccctgaaa tggataccat gttggcaagg    300
tttatgtcat catgtaatgg ttctggtgat tccagttctc aaaatctgca agaatttgga    360
gagagaccag cgataaagca agaaggagga gattcggaga tggtttatca aagtttacca    420
gggcataatt tggttactga caattcagtg agtgttggga attctatgga tagcgcattc    480
aatgttatga gttcaatggc tttagagaat tctatgcaag caactaagat gagtaccgcg    540
aacggatcta atcttgctag gcaaaacagc tctccagctg gacttttctc cgaccttggc    600
gttgacaatg gctttgttgt aatgagagaa ggtggcagct tcagagctgg aaatggtacg    660
aatggagaag ctagtccaac taataagtta agaaggcatg tgaacttctc atcagggcaa    720
aggatgttgc ctcagattgc tgaaattgga gaagaatgta ttggtgggag gagtccagaa    780
ggcgatgtta gcgaggcaag gtacatgtct aggttcacta gtgattcttg ggacggtgct    840
tctctgagtg gtcttaagag acagagggat aatgatggga acatgttttc tggcctaaac    900
acactggaca atcaggatgg aaattctgga aaccgtgtga ctggtttgac tcaccacttg    960
agcttgccca agactctttc agaaacggct accatcgaga agtttttgga ttttcaaggc   1020
aattctgtcc catgtaagat tcgagccaaa agaggtttcg ctacccaccc acgaagcata   1080
gcagagaggg taagaagaac tcggattagt gaaagaatga gaaaattgca agaacttttc   1140
ccgaacatgg acaagcaaac aaacacggca gatatgttag atttggcagt tgagcacatt   1200
aaagatctac agaaacaagt aaagaccctc acggatacta aagcgaagtg cacgtgttct   1260
agtaaacaga agcactattc aagtccctca gcttgatttt agtttctgta cagatagcac   1320
taggacaatg gcaagacaaa aacaagcac atagctacct agttttgatc tactgt        1376
```

<210> 122
<211> 342
<212> PRT
<213> Populus trichocarpa

<400> 122

```
Met Tyr Gln Gln Pro Ser Ser Ser Ser Ser Ser Pro Gln Lys Ser Asn
1               5                   10                  15
Leu Pro Ser Gly Leu Thr Arg Tyr Gly Ser Ala Pro Gly Ser Phe Leu
            20                  25                  30
Thr Arg Ala Val Asp Ser Val Ile Gly Ala Asp Arg Glu Leu Ser Gly
        35                  40                  45
Leu Gly Ser Thr Pro Leu Val Gly Arg Gln Gln His Phe Ser Gly Asp
```

```
    50                  55                  60
Ser Pro Ser Ile Thr Ser Glu Ser Thr Cys Lys Val Asn Ser Ser Ser
65                  70                  75                  80
Cys Asp Arg Lys Ala Pro Lys Ser Gly Gly Gly Gly Gly Leu Gln Arg
                85                  90                  95
Ser Tyr Gly Leu Asn Glu Ile Ala His Gly Ala Gly Ser Leu Val Arg
            100                 105                 110
Gln Arg Ser Ser Pro Ala Gly Phe Leu Ser His Leu Ala Thr Glu Asn
        115                 120                 125
Gly Gly Phe Ser Ile Thr Arg Gly Thr Gly Gly Tyr Asn Ser Arg Asn
    130                 135                 140
Gly Ser Gly Gly Gly Pro Ser Arg Leu Lys Ser Gln Leu Ser Phe Thr
145                 150                 155                 160
Arg Gln Asp Ser Leu Ser Gln Ile Ser Glu Val Ser Glu Asn Val Val
                165                 170                 175
Glu Gly Ile Gly Ser Asp Asn Gly Ser Gln Asn Ser Thr His Ser Tyr
            180                 185                 190
Ser Ala Ala Ser Phe Gly Met Glu Ser Trp Asp Thr Pro Asn Ser Ile
        195                 200                 205
Val Phe Ser Gly His Pro Ser Lys Gln Ala Arg Thr Gly Asp Gly Asp
    210                 215                 220
Ile Tyr Ser Cys Phe Asn Ala Leu Glu Thr Gln Phe Ser Leu Pro Gln
225                 230                 235                 240
Thr Ser Leu Glu Met Ala Thr Val Glu Lys Leu Leu Gln Ile Pro Glu
                245                 250                 255
Asp Ser Val Pro Cys Lys Ile Arg Ala Lys Arg Gly Cys Ala Thr His
            260                 265                 270
Pro Arg Ser Ile Ala Glu Arg Glu Arg Arg Thr Arg Ile Ser Gly Lys
        275                 280                 285
Leu Lys Thr Leu Gln Asp Leu Val Pro Asn Met Asp Lys Gln Thr Ser
    290                 295                 300
Tyr Ala Asp Met Leu Glu Leu Ala Val Lys His Ile Lys Gly Leu Gln
305                 310                 315                 320
Asn Glu Val Glu Lys Leu His Lys Glu Leu Glu Gly Cys Thr Cys Gly
                325                 330                 335
Cys Lys Gln Ser Thr Pro
            340
```

```
<210>  123
<211>  896
<212>  DNA
<213>  Populus trichocarpa

<400>  123
aaagcctcta atacccgagt gaagaagcag atgcaaccaa cacctgccgg aagcagcaac      60
actagtgctg gtggcggtag cgctggtggc ggtggaggtg gagggattgc taggtttcgt     120
tcagcaccag ccacatggat agaagcactt cttggagaag aagaagagga cccattaaag     180
caaagtcaga acttgactga gttacttact tcaaacacac cttcaagtag agattcagtg     240
ccttttaatg cttctagtgc tgctgttgaa ccgggtttgt ttgaaccggt tggtggtttt     300
caaaggcaaa atagctctcc aactgatttt cttcggagtt ctgggatcgg aagtgatcaa     360
gggtattttt ctagttacgg aaatgcttca aattatgagt atatgacgcc aaatatggat     420
gtttcgcctt ctgataaaag agctagagag gttgagttgc aaaacccttc tgcgagatat     480
ccacctcctc cgtcgaaagg agagcaaact gggcctttaa gggcatctag tttgatagaa     540
atggagatgg ataagctttt ggaggattca gtgccttgca gggttcgtgc gaagcgtggc     600
tgtgctacgc atcctcggag tattgcagag agggttcgga gaactcgaat cagtgaccgc     660
ataaggaaac ttcaggaact tgttccaaat atggataagc aaacaaacac tgctgatatg     720
ttagacgagg ctttagcgta tgttaagttt cttcaaaggc agattcagga actcacggaa     780
cagcaaagaa aatgcaaatg catagctaaa gaataacaga ggtaatatgc ctgcccactg     840
ttttgtgtaa ttatcagatt aatgagattt catttaaggt ccatcatagt atttct          896
```

<210> 124
<211> 261
<212> PRT
<213> Populus trichocarpa

<400> 124
Met Gln Pro Thr Pro Ala Gly Ser Ser Asn Thr Ser Ala Gly Gly Gly
1 5 10 15
Ser Ala Gly Gly Gly Gly Gly Gly Gly Ile Ala Arg Phe Arg Ser Ala
20 25 30
Pro Ala Thr Trp Ile Glu Ala Leu Leu Gly Glu Glu Glu Glu Asp Pro
35 40 45
Leu Lys Gln Ser Gln Asn Leu Thr Glu Leu Leu Thr Ser Asn Thr Pro
50 55 60
Ser Ser Arg Asp Ser Val Pro Phe Asn Ala Ser Ser Ala Ala Val Glu
65 70 75 80
Pro Gly Leu Phe Glu Pro Val Gly Gly Phe Gln Arg Gln Asn Ser Ser
85 90 95
Pro Thr Asp Phe Leu Arg Ser Ser Gly Ile Gly Ser Asp Gln Gly Tyr
100 105 110
Phe Ser Ser Tyr Gly Asn Ala Ser Asn Tyr Glu Tyr Met Thr Pro Asn
115 120 125
Met Asp Val Ser Pro Ser Asp Lys Arg Ala Arg Glu Val Glu Leu Gln
130 135 140
Asn Pro Ser Ala Arg Tyr Pro Pro Pro Pro Ser Lys Gly Glu Gln Thr
145 150 155 160
Gly Pro Leu Arg Ala Ser Ser Leu Ile Glu Met Glu Met Asp Lys Leu
165 170 175
Leu Glu Asp Ser Val Pro Cys Arg Val Arg Ala Lys Arg Gly Cys Ala
180 185 190
Thr His Pro Arg Ser Ile Ala Glu Arg Val Arg Arg Thr Arg Ile Ser
195 200 205
Asp Arg Ile Arg Lys Leu Gln Glu Leu Val Pro Asn Met Asp Lys Gln
210 215 220
Thr Asn Thr Ala Asp Met Leu Asp Glu Ala Leu Ala Tyr Val Lys Phe
225 230 235 240
Leu Gln Arg Gln Ile Gln Glu Leu Thr Glu Gln Gln Arg Lys Cys Lys
245 250 255
Cys Ile Ala Lys Glu
260

<210> 125
<211> 1166
<212> DNA
<213> Populus trichocarpa

<400> 125
gagaaagaga gtaaaacaga gcatatgcca atggattcaa gtagtaataa caactatcat 60
caacaaaatc aacccagttc agggttattg agatttcgct cagctccaag ttcccttctt 120
gcaagtttta atgacagcgg agttgacaat gattctgttt tgaattacca agagtttgag 180
gataaatctg cagcaagagt gagagaggaa gctgttaact attcaaactt tccgcggagt 240
tattcaggtt tgcctcctca ttatccaaga cagggctcgg ccacaaattc ttcagctatg 300
gatagttcat atggtttgat tggttcaatc tcaatgggtc atcatgagca acttaaaagg 360
gttgatccaa gtctagctag gcagaatagc tctcctgctg gactttttgg gaacgtttct 420
gttcaaaatg gctatcctgg gtggaatggt actaatggag aagcaaattc aagattgaag 480
agtcaactta gcctctcatc aagagcacct tcttccttag gctttcggtc acagatttct 540
gaaattggga gcgaaagcat tgaggcaggc agccctagtg ctgacagtcg atttcatagc 600
agtcacgggt ttccatatgg ttcttggaac aattcgcatt tgtcagagaa ttttagcagc 660

```
atgaaaagag atcaagaaaa tggaaacctg ttttctaata atgctcagaa cggagagctt    720
ggaaatcgca ctcatgtatt cgcccatcac ttgagtttac caaagacttc agttgaaatg    780
gttgccatgg aaaagttcct tcatttgcaa gattctgttc cttgcaaaat tagagcaaag    840
cgtggttgtg ccactcaccc tcgaagtatt gcagaaagag taagaagaac tcggatcagc    900
gaaagaatga ggaaactaca agagcttgtc ccaaacatgg acaagcaaac aaacacagca    960
gacatgctag atttggctgt agattacatc aaagaccttc agaagcagta caagacactc   1020
agcgacaaca gagccaactg caagtgttta agcaagcaga aaccagtgca aaacaaaatt   1080
gtttgaggtg cttctgtaca gaaatccaag ggctttagg acctgttctt attgatcttc   1140
agtatcaaaa gcaaaatatt tttgtt                                         1166

<210>  126
<211>  353
<212>  PRT
<213>  Populus trichocarpa

<400>  126
Met Pro Met Asp Ser Ser Ser Asn Asn Asn Tyr His Gln Gln Asn Gln
1               5                   10                  15
Pro Ser Ser Gly Leu Leu Arg Phe Arg Ser Ala Pro Ser Ser Leu Leu
            20                  25                  30
Ala Ser Phe Asn Asp Ser Gly Val Asp Asn Asp Ser Val Leu Asn Tyr
        35                  40                  45
Gln Glu Phe Glu Asp Lys Ser Ala Ala Arg Val Arg Glu Glu Ala Val
    50                  55                  60
Asn Tyr Ser Asn Phe Pro Arg Ser Tyr Ser Gly Leu Pro Pro His Tyr
65                  70                  75                  80
Pro Arg Gln Gly Ser Ala Thr Asn Ser Ser Ala Met Asp Ser Ser Tyr
                85                  90                  95
Gly Leu Ile Gly Ser Ile Ser Met Gly His His Glu Gln Leu Lys Arg
            100                 105                 110
Val Asp Pro Ser Leu Ala Arg Gln Asn Ser Ser Pro Ala Gly Leu Phe
        115                 120                 125
Gly Asn Val Ser Val Gln Asn Gly Tyr Pro Gly Trp Asn Gly Thr Asn
    130                 135                 140
Gly Glu Ala Asn Ser Arg Leu Lys Ser Gln Leu Ser Leu Ser Ser Arg
145                 150                 155                 160
Ala Pro Ser Ser Leu Gly Phe Arg Ser Gln Ile Ser Glu Ile Gly Ser
                165                 170                 175
Glu Ser Ile Glu Ala Gly Ser Pro Ser Ala Asp Ser Arg Phe His Ser
            180                 185                 190
Ser His Gly Phe Pro Tyr Gly Ser Trp Asn Asn Ser His Leu Ser Glu
        195                 200                 205
Asn Phe Ser Ser Met Lys Arg Asp Gln Glu Asn Gly Asn Leu Phe Ser
    210                 215                 220
Asn Asn Ala Gln Asn Gly Glu Leu Gly Asn Arg Thr His Val Phe Ala
225                 230                 235                 240
His His Leu Ser Leu Pro Lys Thr Ser Val Glu Met Val Ala Met Glu
                245                 250                 255
Lys Phe Leu His Leu Gln Asp Ser Val Pro Cys Lys Ile Arg Ala Lys
            260                 265                 270
Arg Gly Cys Ala Thr His Pro Arg Ser Ile Ala Glu Arg Val Arg Arg
        275                 280                 285
Thr Arg Ile Ser Glu Arg Met Arg Lys Leu Gln Glu Leu Val Pro Asn
    290                 295                 300
Met Asp Lys Gln Thr Asn Thr Ala Asp Met Leu Asp Leu Ala Val Asp
305                 310                 315                 320
Tyr Ile Lys Asp Leu Gln Lys Gln Tyr Lys Thr Leu Ser Asp Asn Arg
                325                 330                 335
Ala Asn Cys Lys Cys Leu Ser Lys Gln Lys Pro Val Gln Asn Lys Ile
```

```
            340                 345                 350
Val


<210>  127
<211>  789
<212>  DNA
<213>  Ricinus communis

<400>  127
gtgaaactcg cagcaataat tatgtcacag gtttccccat tggatcttgg gatgatacag     60
ctgtgatgtc tgctggttta aaaagactga cagatgatga tagaacactt tctggcctga    120
atgcatctga aaatgagagt ggagaagtgg gaaatcatcc tcctatgttg gctcatcact    180
tgagtttgcc aaaaacttca gctgaattgt ctgcaatcga gaagtatctg caattgcaag    240
attctgtgcc ttgcaagatt cgagctaagc gtggatgtgc cactcatcca aggagcattg    300
ccgagagggt aagaagaact cgaattagcg aacgcatgag gaaactacaa gatcttgtac    360
cgaacatgga taagcaaaca aacacatcag acatgttgga tttggctgtt gactacatta    420
aagaccttca gagacaggtc gagacactat cagagaatcg ttcgaagtgt acgtgcgcaa    480
gcaagcagca acagtcttag cagagaatat gcccaagtag gagtagatag gtgtgtgggg    540
gtgagtggtg gtcatagctg gatccaatga atgagtgact atctagttgg gccccaatcc    600
attaagaagg tgaatgccgt gtcattgctg acttcactgg gtcaccccac cgggtcgggc    660
ggagtctcat aacactgcct cactccgcgg ctctgcctgc ccctttatcg cactccagag    720
gcaatttgct tttttgtcca gtttacgtgc tcaggagggt accgatggtt ttacgatttt    780
gaccgataa                                                            789

<210>  128
<211>  144
<212>  PRT
<213>  Ricinus communis

<400>  128
Met Ser Ala Gly Leu Lys Arg Leu Thr Asp Asp Asp Arg Thr Leu Ser
1               5                   10                  15
Gly Leu Asn Ala Ser Glu Asn Glu Ser Gly Glu Val Gly Asn His Pro
            20                  25                  30
Pro Met Leu Ala His His Leu Ser Leu Pro Lys Thr Ser Ala Glu Leu
        35                  40                  45
Ser Ala Ile Glu Lys Tyr Leu Gln Leu Gln Asp Ser Val Pro Cys Lys
    50                  55                  60
Ile Arg Ala Lys Arg Gly Cys Ala Thr His Pro Arg Ser Ile Ala Glu
65                  70                  75                  80
Arg Val Arg Arg Thr Arg Ile Ser Glu Arg Met Arg Lys Leu Gln Asp
                85                  90                  95
Leu Val Pro Asn Met Asp Lys Gln Thr Asn Thr Ser Asp Met Leu Asp
            100                 105                 110
Leu Ala Val Asp Tyr Ile Lys Asp Leu Gln Arg Gln Val Glu Thr Leu
        115                 120                 125
Ser Glu Asn Arg Ser Lys Cys Thr Cys Ala Ser Lys Gln Gln Gln Ser
    130                 135                 140

<210>  129
<211>  1504
<212>  DNA
<213>  Sorghum bicolor

<400>  129
gtccctcccg gccaccgcgc gcgtgcctcg tcgtcggcac gccggcgcgt gcaatcgatc     60
tactcccagc cgcccaagtt cgtggactgg gtagtagtag cagctagggg ttgtcggcag    120
gcatccttat gaggcgcttc ctgccggccg gaggcggcgg cggcggcggc ggggagccgt    180
```

```
cgtcttcctc ctcctcgggc gggcaacacc agcacggcgg cgggctcgcg ggcgagaggg    240
cggcggcggg cctgcggtac cgaggaggcg acatctcgct ggggcacggg cacgacgacg    300
gacatcgtcg tcatcagtac cacctcggcg ccggcgacgc ggcggatagg cagaacgacg    360
ggtccatgga catgctggcg cggcacagca gctcgccggt cggcttcttc tccaacctcg    420
tcatggacaa cgggtaccca cgctcaaata aagccggagt cagtggtggc aggcgaaggt    480
caccgtgatc cttcaacggc caacaacaat agtggaagta gcagcagcgg ccggaagatg    540
aagccgtcgt cgtccgggtt caacttcgcc ggcgggacgc agcagcaggg ccagcagcag    600
ggcgccgccg gggcccacct ctcgcggatc tccgaggacg gctgcgccag cttcccggcc    660
ggcggcctgg tgggcgaccg cgctgcaggt ggccgcggct ctggcgagag cagcagcggt    720
ggtgctgccg cggcggcgcg ctcgtactcc ggcgggttct ccattgttgg gccgtgggaa    780
gagtccaggg acatcattac caccctcggc gcatacgacc ctcagtttag cggcgccatg    840
gcgggtacgg cgctggagat ggcgggcatg gacaggtaca tgcagctgca gcaggaccag    900
gtgccgttca aagtgcgcgc caagcgcggg tgcgccacgc accccaggag catcgccgag    960
agggaaaggc ggacgaggat cagcgagaag ctcaggaagc cccaggacct cgtgcccaac   1020
atggacaagc aaacgagcac cgcggacatg ttggaccttg cagttgagca catcaagggc   1080
ctgcagagcg aactgcaggc tctgaaacac gagcgggaga agtgcacctg ctgccgaaag   1140
cgatagcgat tcacaacagg gagggcatag aacacagaaa cgcgtccatc tttaacggtt   1200
actgctacct tcaatttcct tgatgtgagg aattgtacat ggatgaagcc atctacatat   1260
cttgattgtc gagtccttgt tgagagtcct aattttagtg attattagca cacaatggga   1320
gtgcattcgg cgcggcccat ggatggagcg tgcatatgca attttgcgtg catagctacc   1380
atttccaaag cagtgagatc cacatatagt catgcatgga catctagcta ggcaggcagt   1440
gccaaaatag gcaacccttc tgttctgata ctttggtttc ttcggtttgt tgatgttcaa   1500
cttg                                                                1504
```

```
<210>  130
<211>  202
<212>  PRT
<213>  Sorghum bicolor

<400>  130
Met Lys Pro Ser Ser Ser Gly Phe Asn Phe Ala Gly Gly Thr Gln Gln
1               5                   10                  15
Gln Gly Gln Gln Gln Gly Ala Ala Gly Gly His Leu Ser Arg Ile Ser
            20                  25                  30
Glu Asp Gly Cys Ala Ser Phe Pro Ala Gly Gly Leu Val Gly Asp Arg
        35                  40                  45
Ala Ala Gly Gly Arg Gly Ser Gly Glu Ser Ser Ser Gly Gly Ala Ala
    50                  55                  60
Ala Ala Ala Arg Ser Tyr Ser Gly Gly Phe Ser Ile Val Gly Pro Trp
65                  70                  75                  80
Glu Glu Ser Arg Asp Ile Ile Thr Thr Leu Gly Ala Tyr Asp Pro Gln
                85                  90                  95
Phe Ser Gly Ala Met Ala Gly Thr Ala Leu Glu Met Ala Gly Met Asp
            100                 105                 110
Arg Tyr Met Gln Leu Gln Gln Asp Gln Val Pro Phe Lys Val Arg Ala
        115                 120                 125
Lys Arg Gly Cys Ala Thr His Pro Arg Ser Ile Ala Glu Arg Glu Arg
    130                 135                 140
Arg Thr Arg Ile Ser Glu Lys Leu Arg Lys Pro Gln Asp Leu Val Pro
145                 150                 155                 160
Asn Met Asp Lys Gln Thr Ser Thr Ala Asp Met Leu Asp Leu Ala Val
                165                 170                 175
Glu His Ile Lys Gly Leu Gln Ser Glu Leu Gln Ala Leu Lys His Glu
            180                 185                 190
Arg Glu Lys Cys Thr Cys Cys Arg Lys Arg
        195                 200

<210>  131
<211>  1630
```

<212>  DNA
<213>  Solanum lycopersicum

<400>  131
gcttgctagc tcatacgcca tttttccttc ttcttcttct tcgataaaat tcgaaaaaat    60
tgaagctata attccaaaaa agatattatt agaatcttga ttatgtttag ctcagagcca   120
atatctagag aaatggataa gttgaatagt ttcttttttt ccggtggtgg tgggggttct   180
tcaagcttca aaaatggtga aggaatggag tctgagttct ttaggagcaa agaaatgatg   240
ggttctgatt tttttcagca acagtcacag tttcagcaga gtaattccgg tgggttaacg   300
aggtacaggt cagctccaag ttcgtttttc gccggaattc ttgacggaga cgggaataat   360
tccggtgaaa atttcattac cggtgatggg tcttcgagtt ctgattcgga ttccatgttc   420
accgccttat tgaacaataa cgacaccacc aacaacaatg gtactcgaga tatgaatgat   480
caaaaccaga agaatcagct tcagtttggt acttctttga aacaggagat tggtgaggag   540
attgaatttg ggaatgaaaa tggtgtacaa aatagatatg agaacggtgg tgtttcttat   600
agtgtagggg tgcaaatgca aactagagca aatttgagta atggaaatgg tgactctgat   660
ctcatcagac aaaacagttc acctgctgga ttcttcaatg gatttatgag agaagttggg   720
aatttcggag caagtgttgg gactaacagg gaagcaagta catcaactaa tggtttcaac   780
aatcatataa gttactcaac taatcaatca tctacctcaa acttcatgcc tagcattgct   840
gagaatgaat cttggaacga cgcgtcgttt aattccttga agagaaatag agatggtgat   900
ctgaaaatgt tctctactaa tttcaatgga atgactaatc agaatgacga atcaagaaac   960
tacacttctt ctggtttatc tcatcattta agcttgccta agacatcttc tgagatggct  1020
gccatagaga aatacctaca gttccaacaa gattcagtgc cttgcaaaat acgtgctaaa  1080
cgaggctgtg ctacgcaccc acgaagtatt gcagaaagga tgagacgtac tcggattagt  1140
gaaagaatga aaaaactgca agatcttttc ccaaatatgg acaagcaaac aaacacagct  1200
gatatgttag atttggcagt tgattacatt aaagaccttc agaaacaagt ccagacactg  1260
accgataaaa aggcgaaatg ctcgtgtaca agtaagcagc tgcaatattc aaatggaaca  1320
acatgaaata gtcttgtgta cagatagtaa taggtgaagt tagatggaaa aggagagtaa  1380
ataaactctc tttctaccct aatatgctgg aaatatggtt ctactttgtt tttattttgc  1440
aacaaggcaa gtgtagtttt caatgtatta ttgagtttga ttagatctaa taacagtcta  1500
gtgagtacaa tctagatgta taggagcaca tgattattgt attatgctgt tgtttattgg  1560
aatttaacta tgttattagt tgattcaaaa ctttataaga caaataatat cgattggaaa  1620
gagtaattcc                                                          1630

<210>  132
<211>  407
<212>  PRT
<213>  Solanum lycopersicum

<400>  132
Met Phe Ser Ser Glu Pro Ile Ser Arg Glu Met Asp Lys Leu Asn Ser
1               5                   10                  15
Phe Phe Phe Ser Gly Gly Gly Gly Gly Ser Ser Ser Phe Lys Asn Gly
            20                  25                  30
Glu Gly Met Glu Ser Glu Phe Phe Arg Ser Lys Glu Met Met Gly Ser
        35                  40                  45
Asp Phe Phe Gln Gln Gln Ser Gln Phe Gln Gln Ser Asn Ser Gly Gly
    50                  55                  60
Leu Thr Arg Tyr Arg Ser Ala Pro Ser Ser Phe Phe Ala Gly Ile Leu
65                  70                  75                  80
Asp Gly Asp Gly Asn Asn Ser Gly Glu Asn Phe Ile Thr Gly Asp Gly
                85                  90                  95
Ser Ser Ser Ser Asp Ser Asp Ser Met Phe Thr Ala Leu Leu Asn Asn
            100                 105                 110
Asn Asp Thr Thr Asn Asn Asn Gly Thr Arg Asp Met Asn Asp Gln Asn
        115                 120                 125
Gln Lys Asn Gln Leu Gln Phe Gly Thr Ser Leu Lys Gln Glu Ile Gly
    130                 135                 140
Glu Glu Ile Glu Phe Gly Asn Glu Asn Gly Val Gln Asn Arg Tyr Glu
145                 150                 155                 160

```
Asn Gly Gly Val Ser Tyr Ser Val Gly Val Gln Met Gln Thr Arg Ala
                165                 170                 175
Asn Leu Ser Asn Gly Asn Gly Asp Ser Asp Leu Ile Arg Gln Asn Ser
            180                 185                 190
Ser Pro Ala Gly Phe Phe Asn Gly Phe Met Arg Glu Val Gly Asn Phe
        195                 200                 205
Gly Ala Ser Val Gly Thr Asn Arg Glu Ala Ser Thr Ser Thr Asn Gly
    210                 215                 220
Phe Asn Asn His Ile Ser Tyr Ser Thr Asn Gln Ser Ser Thr Ser Asn
225                 230                 235                 240
Phe Met Pro Ser Ile Ala Glu Asn Glu Ser Trp Asn Asp Ala Ser Phe
                245                 250                 255
Asn Ser Leu Lys Arg Asn Arg Asp Gly Asp Leu Lys Met Phe Ser Thr
            260                 265                 270
Asn Phe Asn Gly Met Thr Asn Gln Asn Asp Glu Ser Arg Asn Tyr Thr
        275                 280                 285
Ser Ser Gly Leu Ser His His Leu Ser Leu Pro Lys Thr Ser Ser Glu
    290                 295                 300
Met Ala Ala Ile Glu Lys Tyr Leu Gln Phe Gln Gln Asp Ser Val Pro
305                 310                 315                 320
Cys Lys Ile Arg Ala Lys Arg Gly Cys Ala Thr His Pro Arg Ser Ile
                325                 330                 335
Ala Glu Arg Met Arg Arg Thr Arg Ile Ser Glu Arg Met Lys Lys Leu
            340                 345                 350
Gln Asp Leu Phe Pro Asn Met Asp Lys Gln Thr Asn Thr Ala Asp Met
        355                 360                 365
Leu Asp Leu Ala Val Asp Tyr Ile Lys Asp Leu Gln Lys Gln Val Gln
    370                 375                 380
Thr Leu Thr Asp Lys Lys Ala Lys Cys Ser Cys Thr Ser Lys Gln Leu
385                 390                 395                 400
Gln Tyr Ser Asn Gly Thr Thr
                405

<210>  133
<211>  1191
<212>  DNA
<213>  Solanum lycopersicum

<400>  133
aaacataaaa acaacaaaaa attcacctga agagaaaaat taaagttaac ttttgctaga      60
aaaaatttca ccgatgcaac gtggaactac cggtgacggg ggtggtggac tttcccgttt     120
ccggtcagct ccggcgacat ggttagaagc tctacttgaa tccgacactg agaacgaagt     180
tattcttaac ccttcttctc ccattttaca cactcccaat aaaccaccac ctcacccatc     240
aactccgaag cttaagctgg aaaccggcgg agctactagg ttcactggcg atccgggtct     300
gtttgaatcc ggtggtagta gcaattttct caggcagaat agttctccgg cggagtttct     360
ttcacatatc agctctgatg gttacttctc aaactatgga attccgtcca gtttggacta     420
tctttctcca tccgttgatg tttcgcagtc cgctaagcga acccgagacg atgattccga     480
aagttccccc agaaaattag tatcccagtt gaaaggagag tcaagtgggc agttacatgg     540
ttctggtggg tcactggatg ctgaaatgga gaatctcatg gatgatttgg tgccttgtaa     600
ggttcgagca aagcgtggct gtgctaccca tcctcgcagc atagcggagc gggttcgtcg     660
aacgcgtata agtgacagaa ttaggaagct tcaggaactg gtgccaaata tggacaagca     720
aaccaacaca gccgacatgt tggaagaagc agtcgaatac gtcaagtttc tgcagaggca     780
gattcaggaa cttacggagc atcagaagaa atgcacgtgc tcaatgaaag atcaataatg     840
agaagaagag agctccacta gagtatatag gcaatagttt ggttagttgc atgcagttta     900
gaaatggtaa ttcactccta tatatcctat tcttcttcat gtgtagatat taatggaaat     960
ttgcaagagc tttgctactt tagaatccag agtcagcttt ggcttttaat tattaaattt    1020
aggttgtaca cttttctggt gcagttgtat catgttgtat atgtagtgtc aatttgctta    1080
atagtcaaat ttttgcagca gaaacacttc cctttctgaa gatgtaatca tgatttgcag    1140
atccgtcatg ttaaaatcaa tataaatcgt tgcatcagtg tgaagtttaa t             1191
```

<210> 134
<211> 254
<212> PRT
<213> Solanum lycopersicum

<400> 134
Met Gln Arg Gly Thr Thr Gly Asp Gly Gly Gly Gly Leu Ser Arg Phe
1 5 10 15
Arg Ser Ala Pro Ala Thr Trp Leu Glu Ala Leu Leu Glu Ser Asp Thr
20 25 30
Glu Asn Glu Val Ile Leu Asn Pro Ser Ser Pro Ile Leu His Thr Pro
35 40 45
Asn Lys Pro Pro Pro His Pro Ser Thr Pro Lys Leu Lys Leu Glu Thr
50 55 60
Gly Gly Ala Thr Arg Phe Thr Gly Asp Pro Gly Leu Phe Glu Ser Gly
65 70 75 80
Gly Ser Ser Asn Phe Leu Arg Gln Asn Ser Ser Pro Ala Glu Phe Leu
85 90 95
Ser His Ile Ser Ser Asp Gly Tyr Phe Ser Asn Tyr Gly Ile Pro Ser
100 105 110
Ser Leu Asp Tyr Leu Ser Pro Ser Val Asp Val Ser Gln Ser Ala Lys
115 120 125
Arg Thr Arg Asp Asp Asp Ser Glu Ser Ser Pro Arg Lys Leu Val Ser
130 135 140
Gln Leu Lys Gly Glu Ser Ser Gly Gln Leu His Gly Ser Gly Gly Ser
145 150 155 160
Leu Asp Ala Glu Met Glu Asn Leu Met Asp Asp Leu Val Pro Cys Lys
165 170 175
Val Arg Ala Lys Arg Gly Cys Ala Thr His Pro Arg Ser Ile Ala Glu
180 185 190
Arg Val Arg Arg Thr Arg Ile Ser Asp Arg Ile Arg Lys Leu Gln Glu
195 200 205
Leu Val Pro Asn Met Asp Lys Gln Thr Asn Thr Ala Asp Met Leu Glu
210 215 220
Glu Ala Val Glu Tyr Val Lys Phe Leu Gln Arg Gln Ile Gln Glu Leu
225 230 235 240
Thr Glu His Gln Lys Lys Cys Thr Cys Ser Met Lys Asp Gln
245 250

<210> 135
<211> 838
<212> DNA
<213> Saccharum officinarum

<400> 135
ggccaagggt ttccatggtt cagggagatt cattctattg tgtttccacc cgccaccaag 60
caggattccc ccaatggggc atcatagccg gaatagtact cttattaatt acagtttggt 120
ttccaaagaa aatgacgggc ttattcaggt ccagcaagac caagttccat tcagagtaca 180
agccaagcgt ggatgtgccc cgcatccaag gagcatccca gagcgggagc gaagaacgag 240
gatcagcgag aagctcaaaa agttgcaggc ccttgtgcct aacatggaca agcaaactag 300
cccagcggac atgctggatt tagcagttga tcacatcaga ggactccaga gcgagatgca 360
ggctctcaag gaagacaagg agaagtgcac ctgccgcagc aattgtccac cagggagata 420
acatatgcta aaaatgggtt ctcggcaaca tacagaacgg cattgcacat tgtcagacag 480
catgcaaaaa atggctactg tagcctattg gcttcagcag attggccgag atatttggta 540
ttcatcaaat ctagtgttta gcttcaatgc aagctagcta gctagaagta gctgttgtgt 600
tcatattgaa aagcagaagc gtattttatt tcaagcagga gtaagggggg aatgaaaggc 660
ttaggctact ctaagtttta agggcctatt gcagtttcat gaatttccct ggttctaata 720
agttcttttt tgagaagtac tgtactaata agttcttact acctgctagc cagtactagc 780

gcctcatgag cataccgtag atgctagata tgatattgaa ggatttttc ccctccgt 838

<210> 136
<211> 115
<212> PRT
<213> Saccharum officinarum

<400> 136
Met Gly His His Ser Arg Asn Ser Thr Leu Ile Asn Tyr Ser Leu Val
1 5 10 15
Ser Lys Glu Asn Asp Gly Leu Ile Gln Val Gln Gln Asp Gln Val Pro
20 25 30
Phe Arg Val Gln Ala Lys Arg Gly Cys Ala Pro His Pro Arg Ser Ile
35 40 45
Pro Glu Arg Glu Arg Arg Thr Arg Ile Ser Glu Lys Leu Lys Lys Leu
50 55 60
Gln Ala Leu Val Pro Asn Met Asp Lys Gln Thr Ser Pro Ala Asp Met
65 70 75 80
Leu Asp Leu Ala Val Asp His Ile Arg Gly Leu Gln Ser Glu Met Gln
85 90 95
Ala Leu Lys Glu Asp Lys Glu Lys Cys Thr Cys Arg Ser Asn Cys Pro
100 105 110
Pro Gly Arg
115

<210> 137
<211> 638
<212> DNA
<213> Saccharum officinarum

<400> 137
gccagtatta gtaactctta tgaataccag ttttggtgtc gcgaagaaaa tggcgggcct 60
actgcaggtg cagcaagacc aagttccatt cagagtacga gccaagcgtg gatgtgccac 120
gcatccaagg agcatcgcag agcgggagcg aagaacgagg atcagcgaga agctcagaaa 180
gttgcaggcc cttgtgccta acatggacaa gcaaactagc acagcggaca tgctggattt 240
agcagttgat cacatcagag gactccagag cgagctgcag gctctcaagg aagacaagga 300
gaagtgcccc tgccgcagca atcgtccccc agggagataa catatgctaa aaatgggttt 360
tcggcaacat acagaacggc attgcacatt gtcagacagc atgcaaaaaa tggctactgt 420
agcatattgg cttcagcaga ttgggcgaga tatttggtat tcatcaaatc tagtgtttag 480
cttcaatgca aactagctag ctagaagtag ctgttgtgtt catattgaaa agcagaagcg 540
tattttattt caagcaggag taagggggga atgaaaggct ttaagggcct attgcagttt 600
catgaatttc cctggttcta ataagttctt ttttgggg 638

<210> 138
<211> 106
<212> PRT
<213> Saccharum officinarum

<400> 138
Met Asn Thr Ser Phe Gly Val Ala Lys Lys Met Ala Gly Leu Leu Gln
1 5 10 15
Val Gln Gln Asp Gln Val Pro Phe Arg Val Arg Ala Lys Arg Gly Cys
20 25 30
Ala Thr His Pro Arg Ser Ile Ala Glu Arg Glu Arg Arg Thr Arg Ile
35 40 45
Ser Glu Lys Leu Arg Lys Leu Gln Ala Leu Val Pro Asn Met Asp Lys
50 55 60
Gln Thr Ser Thr Ala Asp Met Leu Asp Leu Ala Val Asp His Ile Arg
65 70 75 80

```
Gly Leu Gln Ser Glu Leu Gln Ala Leu Lys Glu Asp Lys Glu Lys Cys
                85                  90                  95
Pro Cys Arg Ser Asn Arg Pro Pro Gly Arg
            100                 105
```

<210> 139
<211> 1047
<212> DNA
<213> Solanum tuberosum

<400> 139

```
attgggcctt aaaccattga aggggccaaa ccaagccgga ataaaatttt gtggttaaag      60
gcccattata aaaagtgatt gaacagttat catggcaagg tttttcgaca aggctttctt     120
gggaattgaa tatccacgaa attgtgtgaa ctgaatggat tttccctaac caagttccca     180
ggacggattg atgaaatcgc cgggttccac ttcctcccaa taatgaaagt cccatctgaa     240
gaattcagag tactgaaggt cgagcttcgc cctcccactc tattgtctca tttgagttta     300
cctcaaacat caccggagtt atccgccatg gagaagctct tgcaagattc agtaccatgt     360
aaagtcagag caaagagagg ctgtgccact catcctcgta gcatcgccga aagggtaaga     420
agaacacgaa taagcgaaag aatgaggaag ctgcaagagc ttgtccccaa tatggacaag     480
caaacagacc cggcggatat gttggacttt gcagctgact acatcaaaga actagagaca     540
caagtcaagg cactatcgga aacgcgttca aagtgtacgt gctcgcatga atagaagacc     600
atattcaaaa tgataaggta gggcaatgct gtgcacattg tagtgggata aaaaggattg     660
acaatggaaa tagcaaagca gataggtgga ttctttttgg cactggaaaa agaagagaat     720
aggttgccat attttggtgt gtaaaaaaga tagattcaga ttcatctgat gtaacattat     780
aacgtaaact gtacataaaa tttccattgt ccacttatgt tctgtgaaag gaatataatg     840
ttaggacttt atcagctgct tttgtggact actttagtat gagcctaagt ggttaggtcc     900
ttttattatg ttgtgtaaat gtgtgtgttg ccattcaaaa gtccaaataa ctcttttctt     960
tttttagata aaaggaagta cttgggctaa aacagttgca aagatacttt taacatgatg    1020
tgatattgta ctatatgcac aatttt                                         1047
```

<210> 140
<211> 123
<212> PRT
<213> Solanum tuberosum

<400> 140

```
Met Lys Val Pro Ser Glu Glu Phe Arg Val Leu Lys Val Glu Leu Arg
1               5                   10                  15
Pro Pro Thr Leu Leu Ser His Leu Ser Leu Pro Gln Thr Ser Pro Glu
            20                  25                  30
Leu Ser Ala Met Glu Lys Leu Leu Gln Asp Ser Val Pro Cys Lys Val
        35                  40                  45
Arg Ala Lys Arg Gly Cys Ala Thr His Pro Arg Ser Ile Ala Glu Arg
    50                  55                  60
Val Arg Arg Thr Arg Ile Ser Glu Arg Met Arg Lys Leu Gln Glu Leu
65                  70                  75                  80
Val Pro Asn Met Asp Lys Gln Thr Asp Pro Ala Asp Met Leu Asp Phe
                85                  90                  95
Ala Ala Asp Tyr Ile Lys Glu Leu Glu Thr Gln Val Lys Ala Leu Ser
            100                 105                 110
Glu Thr Arg Ser Lys Cys Thr Cys Ser His Glu
        115                 120
```

<210> 141
<211> 1386
<212> DNA
<213> Solanum tuberosum

<400> 141

```
gcctcttacc ccatcacccc acccacccac ttctctctct attaagttct caacctccaa     60
atcttaacaa tccaattcaa aacaattctc aaatgtatcc atcatcaaca tcttcttcat    120
cacaaggttc aatgagccac accagcacca cagctggcgg cggcggtggt ctcactcgct    180
acggttcagc tccgggatca tttttaacta cagcagttga atccgtcgtt aacgcgaagg    240
aacaatcgaa tttacagagg tcaattggtt tcaacgactt aacaatcggc ggcggcggcg    300
gcggaggagg aggtttgtcg acgacttcaa cgacgccgtt ggttcgacat agtagctcac    360
cggcgaggtt tcttaatcaa cttgctactg ctgcaggtga tactggattt tcagtttcaa    420
tggggagagg aagctataac tcaaaaggcg gtggagatag tggccgggga ataacaaggc    480
tgaactctca gctcagcttc actaggcaag aagctctctc tcaaatagca gaggaaaatg    540
aggatattga agggaccagt atagccaatg gccacagaaa gtcaacacat tcttatgcca    600
gtgcaagtag tttcgcaatg ggttcttggg aagataacaa ctctataatg ttctctgtca    660
cacctagcaa acgagccaag cagattagca atgacatggt caatgggctc gatgatgggg    720
aaactcagtt tcagtttggc ttgtctcaga cagcactaga aatggcatct atggatagat    780
tgctgcacat ccccgaggat tctgttcctt gcaaaattcg tgccaagcgt ggttgtgcta    840
ctcatcctcg cagcattgca gaaagggaaa gaagaaccag aattagtggg aaactaaaga    900
agttacaaga tcttgttcca aacatggaca agcaaacgag ctacgctgac atgctggatc    960
tagcagtgca gcacattcga acccttcaag atcaggttca gaatctgaat acagaacttg   1020
aaaactgcaa atgtggatgt aagaaatcaa gtcaataaca aaatggaagg aagcacttta   1080
gctgaagatt gacttaaaag gatttttgtc accattattt tgcctcttag aaactcaaat   1140
ttgtacataa ggaagtaaat ttcatactag ttttttaaag ctaaaaagta aactctcttt   1200
tttgtttttt ttttcctttt ccagttagaa gactacgtat atgattgttt tggattattt   1260
ctagtttttt tttccatcta acatttacat ttttaattta gttttgagtt tattttggtt   1320
gaagatcata aagctaattg atgatgttgt acaacaactt tctctatttc atttttatgt   1380
aaaatg                                                               1386

<210>  142
<211>  321
<212>  PRT
<213>  Solanum tuberosum

<400>  142
Met Tyr Pro Ser Ser Thr Ser Ser Ser Ser Gln Gly Ser Met Ser His
1               5                   10                  15
Thr Ser Thr Thr Ala Gly Gly Gly Gly Gly Leu Thr Arg Tyr Gly Ser
            20                  25                  30
Ala Pro Gly Ser Phe Leu Thr Thr Ala Val Glu Ser Val Val Asn Ala
        35                  40                  45
Lys Glu Gln Ser Asn Leu Gln Arg Ser Ile Gly Phe Asn Asp Leu Thr
    50                  55                  60
Ile Gly Gly Gly Gly Gly Gly Gly Gly Gly Leu Ser Thr Thr Ser Thr
65                  70                  75                  80
Thr Pro Leu Val Arg His Ser Ser Ser Pro Ala Arg Phe Leu Asn Gln
                85                  90                  95
Leu Ala Thr Ala Ala Gly Asp Thr Gly Phe Ser Val Ser Met Gly Arg
            100                 105                 110
Gly Ser Tyr Asn Ser Lys Gly Gly Gly Asp Ser Gly Arg Gly Ile Thr
        115                 120                 125
Arg Leu Asn Ser Gln Leu Ser Phe Thr Arg Gln Glu Ala Leu Ser Gln
    130                 135                 140
Ile Ala Glu Glu Asn Glu Asp Ile Glu Gly Thr Ser Ile Ala Asn Gly
145                 150                 155                 160
His Arg Lys Ser Thr His Ser Tyr Ala Ser Ala Ser Ser Phe Ala Met
                165                 170                 175
Gly Ser Trp Glu Asp Asn Asn Ser Ile Met Phe Ser Val Thr Pro Ser
            180                 185                 190
Lys Arg Ala Lys Gln Ile Ser Asn Asp Met Val Asn Gly Leu Asp Asp
        195                 200                 205
Gly Glu Thr Gln Phe Gln Phe Gly Leu Ser Gln Thr Ala Leu Glu Met
    210                 215                 220
```

```
Ala Ser Met Asp Arg Leu Leu His Ile Pro Glu Asp Ser Val Pro Cys
225                 230                 235                 240
Lys Ile Arg Ala Lys Arg Gly Cys Ala Thr His Pro Arg Ser Ile Ala
                245                 250                 255
Glu Arg Glu Arg Arg Thr Arg Ile Ser Gly Lys Leu Lys Lys Leu Gln
            260                 265                 270
Asp Leu Val Pro Asn Met Asp Lys Gln Thr Ser Tyr Ala Asp Met Leu
        275                 280                 285
Asp Leu Ala Val Gln His Ile Arg Thr Leu Gln Asp Gln Val Gln Asn
    290                 295                 300
Leu Asn Thr Glu Leu Glu Asn Cys Lys Cys Gly Cys Lys Lys Ser Ser
305                 310                 315                 320
Gln


<210>  143
<211>  1331
<212>  DNA
<213>  Solanum tuberosum

<400>  143
cgtttttcgc cggaatcttg acggggacgg gaataattcc ggtgaagatg gtcattaccg      60
gtgatgggtc ttcgagttct gattcggatt ccatgttcac cgccttattg aacagtaacg     120
acaccaccaa caacaatggt actcgagatc tgaatgatca aaaccagaag aatcagctgc     180
agtttggttc ttctttgaag caggagattg gtgaggagat tgaatttggg aatgaaaatg     240
gtgtacaaaa tagatatgag aatggtggtg tttcatatgg tgtaggggtg caaatgcaaa     300
ctagagcaaa ttcgagtaat ggaaatggtg gctctaatct catcagacaa aacagttcac     360
cagctggatt cttcaatgga tttatgagag aagttgggaa ttttggagca agtgtaggga     420
ctaacaagga agcaagtaca tcaactaatg gtttcagcaa tcatataagt tattcaacta     480
atcaatcatc tacctcaaac ttcatgccta gcattgctga gaatgaatct tggaacgact     540
cgtcgttcaa ttctttgaag agaagtagag atggtgatct gaaaatgttc tctaatagtt     600
tcagtggaat gactaatcag aatgacgaat caagaaacta cacttcttct ggtttaactc     660
atcatttaag cttgcctaag acatcttctg agatggctgc catagaaaaa tacctacagt     720
ttcaacaaga ttcagtgcct tgcaaaatac gtgctaaacg aggctgtgct acgcacccac     780
gaagtattgc agaaaggatg agacgtactc ggattagtga aagaatgaaa aaactgcaag     840
atcttttccc aaatatggac aagcaaacaa acacagctga tatgttagat ttggcagttg     900
attacattaa agaccttcag aaacaagtcc agacactgaa cgataaaaag gcgaaatgct     960
cgtgtacaag taagcagctg caatattcaa atggaacaac atgaaatttt cgtctgtaca    1020
gatagtaata ggtgaagtta gatgggaaga tggaaaagga gagtaaatga actctctttc    1080
tgccctaata tgctggaaat atggtttact tgtttttatt tagcaataag acaagtgtag    1140
ttttcaatgt attattgagt ttgagtagat ctaataacag tctagtgggt acaatctaga    1200
tgtataggag cacatgatta ttgtattatg ctgttgttta ttggaattta actatgttgt    1260
tagttgattc aaaactttaa aagacagaaa atatcgattg aaaagagtac ataattggca    1320
attactctgt t                                                          1331

<210>  144
<211>  318
<212>  PRT
<213>  Solanum tuberosum

<400>  144
Met Val Ile Thr Gly Asp Gly Ser Ser Ser Ser Asp Ser Asp Ser Met
1               5                   10                  15
Phe Thr Ala Leu Leu Asn Ser Asn Asp Thr Thr Asn Asn Asn Gly Thr
            20                  25                  30
Arg Asp Leu Asn Asp Gln Asn Gln Lys Asn Gln Leu Gln Phe Gly Ser
        35                  40                  45
Ser Leu Lys Gln Glu Ile Gly Glu Glu Ile Glu Phe Gly Asn Glu Asn
    50                  55                  60
```

```
Gly Val Gln Asn Arg Tyr Glu Asn Gly Gly Val Ser Tyr Gly Val Gly
65                  70                  75                  80
Val Gln Met Gln Thr Arg Ala Asn Ser Ser Asn Gly Asn Gly Gly Ser
                85                  90                  95
Asn Leu Ile Arg Gln Asn Ser Ser Pro Ala Gly Phe Phe Asn Gly Phe
            100                 105                 110
Met Arg Glu Val Gly Asn Phe Gly Ala Ser Val Gly Thr Asn Lys Glu
        115                 120                 125
Ala Ser Thr Ser Thr Asn Gly Phe Ser Asn His Ile Ser Tyr Ser Thr
    130                 135                 140
Asn Gln Ser Ser Thr Ser Asn Phe Met Pro Ser Ile Ala Glu Asn Glu
145                 150                 155                 160
Ser Trp Asn Asp Ser Ser Phe Asn Ser Leu Lys Arg Ser Arg Asp Gly
                165                 170                 175
Asp Leu Lys Met Phe Ser Asn Ser Phe Ser Gly Met Thr Asn Gln Asn
            180                 185                 190
Asp Glu Ser Arg Asn Tyr Thr Ser Ser Gly Leu Thr His His Leu Ser
        195                 200                 205
Leu Pro Lys Thr Ser Ser Glu Met Ala Ala Ile Glu Lys Tyr Leu Gln
    210                 215                 220
Phe Gln Gln Asp Ser Val Pro Cys Lys Ile Arg Ala Lys Arg Gly Cys
225                 230                 235                 240
Ala Thr His Pro Arg Ser Ile Ala Glu Arg Met Arg Arg Thr Arg Ile
                245                 250                 255
Ser Glu Arg Met Lys Lys Leu Gln Asp Leu Phe Pro Asn Met Asp Lys
            260                 265                 270
Gln Thr Asn Thr Ala Asp Met Leu Asp Leu Ala Val Asp Tyr Ile Lys
        275                 280                 285
Asp Leu Gln Lys Gln Val Gln Thr Leu Asn Asp Lys Lys Ala Lys Cys
    290                 295                 300
Ser Cys Thr Ser Lys Gln Leu Gln Tyr Ser Asn Gly Thr Thr
305                 310                 315
```

<210> 145
<211> 1155
<212> DNA
<213> Solanum tuberosum

<400> 145

```
tagaaaaaaa aaaagttaac ttttgctaga aaaaattcac cgatgcaacg tggaactgcc      60
ggtgccggag gaggtggact ttcccgtttc cgttcagctc cggcgacatg gttagaagca     120
ctacttgagt ccgacactga gaacgaagtt attcttaacc cttcttctcc cattttacac     180
actcccaaaa aaccaccacc tcacccatca actccgaagc ttccggagct taaactggaa     240
actggcggag ctactaggtt cactggcgat ccgggtctgt ttgaatccgg tggtagtagc     300
aattttctca ggcagaacag ttctccggcg gagtttcttt cacatataag ctctgatggt     360
tacttctcaa actatggaat tccgtccagt ttggactatc tttctccatc cgttgatgtt     420
tcgcagtccg ctaagcgaac ccgagacggt gattccgaaa gttcgcccag aaaattagca     480
tcccagttga agggagagtc aagtgggaag ttacatggtt ctggtggatc gctggatgct     540
gaaatggaga atctcatgga tgatttggtg ccttgtaagg ttcgagcaaa gcgtggctgt     600
gctacccatc ctcgcagcat agcggagcgg gttcgtcgaa cgcgtataag tgacagaatt     660
aggaagcttc aggaactggt gccaaatatg gacaaacaaa ccaacacagc cgacatgttg     720
gaagaagcag tcgaatacgt gaagtttctg cagaggcaga ttcaggaact tacggagcat     780
cagaagaaat gcacgtgctc aatgaaagat caataatgag aagaagagag ctccactaga     840
gtatataggc aatagttttg ttagttgcat gcagtttaga aatggtaatt cactcctcta     900
tatcctattc ttcttcatgt gtagatatta atggaaattt gcaagagctt tgctacttta     960
gaatccagaa tcagcttcgg cttttaatta ttaaatttag gttgtacact tttctggtgc    1020
agttgtatca tgttgtatat gtagtgtcaa tttgcttaat agtcatattt ctgcagcaga    1080
aacacttcac tttctgaaga tgtaatcatg atttgcagac ctgtcatgtt gaaactcaat    1140
aaatatcgtt gcatc                                                     1155
```

<210> 146
<211> 257
<212> PRT
<213> Solanum tuberosum

<400> 146

```
Met Gln Arg Gly Thr Ala Gly Ala Gly Gly Gly Gly Leu Ser Arg Phe
1               5                   10                  15
Arg Ser Ala Pro Ala Thr Trp Leu Glu Ala Leu Leu Glu Ser Asp Thr
            20                  25                  30
Glu Asn Glu Val Ile Leu Asn Pro Ser Ser Pro Ile Leu His Thr Pro
        35                  40                  45
Lys Lys Pro Pro Pro His Pro Ser Thr Pro Lys Leu Pro Glu Leu Lys
    50                  55                  60
Leu Glu Thr Gly Gly Ala Thr Arg Phe Thr Gly Asp Pro Gly Leu Phe
65                  70                  75                  80
Glu Ser Gly Gly Ser Ser Asn Phe Leu Arg Gln Asn Ser Ser Pro Ala
                85                  90                  95
Glu Phe Leu Ser His Ile Ser Ser Asp Gly Tyr Phe Ser Asn Tyr Gly
            100                 105                 110
Ile Pro Ser Ser Leu Asp Tyr Leu Ser Pro Ser Val Asp Val Ser Gln
        115                 120                 125
Ser Ala Lys Arg Thr Arg Asp Gly Asp Ser Glu Ser Ser Pro Arg Lys
    130                 135                 140
Leu Ala Ser Gln Leu Lys Gly Glu Ser Ser Gly Lys Leu His Gly Ser
145                 150                 155                 160
Gly Gly Ser Leu Asp Ala Glu Met Glu Asn Leu Met Asp Asp Leu Val
                165                 170                 175
Pro Cys Lys Val Arg Ala Lys Arg Gly Cys Ala Thr His Pro Arg Ser
            180                 185                 190
Ile Ala Glu Arg Val Arg Arg Thr Arg Ile Ser Asp Arg Ile Arg Lys
        195                 200                 205
Leu Gln Glu Leu Val Pro Asn Met Asp Lys Gln Thr Asn Thr Ala Asp
    210                 215                 220
Met Leu Glu Glu Ala Val Glu Tyr Val Lys Phe Leu Gln Arg Gln Ile
225                 230                 235                 240
Gln Glu Leu Thr Glu His Gln Lys Lys Cys Thr Cys Ser Met Lys Asp
                245                 250                 255
Gln
```

<210> 147
<211> 877
<212> DNA
<213> Solanum tuberosum

<400> 147

```
ggtcgactcc gaaaagtttc aagactcaag ttggattctc atccgggaaa cctcctgctt      60
ctcccaggct aatggctcca atctctgaat ttagagataa agtcttggaa gaaaagagca     120
cggggaatga acataaaaac gatgagaact gcattacaga tttccccatg ccttcttggg     180
aagattcaca cattttgtcc gacgatttcc tcaaagctga agacattgag attgagccat     240
actctaatga agatgcatcc cataatcaga atagtgaagg gctggctcgc ccgccaattc     300
ccttatctca tcatttgagt ttgcccacaa gtatcatgga gaagctcttg caagattcag     360
tacacttgaa tgtcagagca aagaggggtt gtgcaactca ccctcgtagc attgcagaaa     420
gggtgagaag aacacgaata agcgacagaa tgaggaagct gcaagagctt gttcccaaca     480
tggacaagca aacaaacaca gcagatatgt tggattttgc agtagattat attaaagagc     540
tggagaaaca agtcaagata ttagcagaaa tgcgctccaa atgtacgtgc ataaataagt     600
aaaaagctac agatcaaaag gtgaaagtag gatgaaaggg aaatgataaa ttgaaatcag     660
```

```
ctagcagaag tggattccct ttgatacttg aagaagacaa tggggatggc attttggtgg    720
gtagaatata tagaagcaaa ttcatttgat ataacataat atactttttt ataaaaatga    780
tgttcgaatc aaacttgtgc ccacctcaat tactagtgat ttgcttgtat gaataattat    840
gtccactaca acctcaaatg tacatatact tttgtct                             877

<210>  148
<211>  176
<212>  PRT
<213>  Solanum tuberosum

<400>  148
Met Ala Pro Ile Ser Glu Phe Arg Asp Lys Val Leu Glu Glu Lys Ser
1               5                   10                  15
Thr Gly Asn Glu His Lys Asn Asp Glu Asn Cys Ile Thr Asp Phe Pro
            20                  25                  30
Met Pro Ser Trp Glu Asp Ser His Ile Leu Ser Asp Asp Phe Leu Lys
        35                  40                  45
Ala Glu Asp Ile Glu Ile Glu Pro Tyr Ser Asn Glu Asp Ala Ser His
    50                  55                  60
Asn Gln Asn Ser Glu Gly Leu Ala Arg Pro Pro Ile Pro Leu Ser His
65                  70                  75                  80
His Leu Ser Leu Pro Thr Ser Ile Met Glu Lys Leu Leu Gln Asp Ser
                85                  90                  95
Val His Leu Asn Val Arg Ala Lys Arg Gly Cys Ala Thr His Pro Arg
            100                 105                 110
Ser Ile Ala Glu Arg Val Arg Arg Thr Arg Ile Ser Asp Arg Met Arg
        115                 120                 125
Lys Leu Gln Glu Leu Val Pro Asn Met Asp Lys Gln Thr Asn Thr Ala
    130                 135                 140
Asp Met Leu Asp Phe Ala Val Asp Tyr Ile Lys Glu Leu Glu Lys Gln
145                 150                 155                 160
Val Lys Ile Leu Ala Glu Met Arg Ser Lys Cys Thr Cys Ile Asn Lys
                165                 170                 175

<210>  149
<211>  1056
<212>  DNA
<213>  Triticum aestivum

<220>
<221>  misc_feature
<222>  (1053)..(1053)
<223>  n is a, c, g, or t

<400>  149
gaggggacga cggagcggaa tgctggcccg gcacagcagc tcgccggcgg ggttcttctc     60
caacctcatg gtggatgacg gatatcatgg ctcgagaggc gccggagtag ctggtggcag    120
cagcggcgga gaagcccacc gtcacgccag tagcagcacg aagatgaagt cccagctgag    180
cttcacggcc ggcggacaac agaccactgc ccacctctcg cgtatctccg agggcgcctc    240
cttattcccc agcgccgacg ccgtccgcgc tgccgcgcac tcgggcagcg agcaccccgt    300
gtcgcgttcc ttctcggcca gcggcagtag cgggggcttc tccatcgtag ggccgtggga    360
tgagtcgagg gagatcatcg gcaccctcga tctcggcggt tacgagtctc agttcagtgg    420
catggcgagc tcgtcgtcgc tggagctggc ggggatggac aagtacatgc aggcgcagca    480
gcagcaggac caggtggcgt tcaaggtgcg ggccaagcgc ggctgcgcga cgcacccgag    540
gagcatcgcc gagagggagc ggaggacgag gatcagctac aagctcagga agctgcagga    600
cctagtgccc aacatggaca agcaaacgag cacctcggac atgttggacc tcgcggtgga    660
gcacatcaag ggcctccaga gccagctgca ggccatgaag cacgagcagg acaaatgcac    720
ctgctgcaac aagccttgag caattaagca gaggaccaag gactgatcag cacaacgcaa    780
agaatgacac cacccttact cgagagaaat gtacatgcat gcatgggtaa tactattgat    840
```

ccaacatgat gcatgaatta gtgcatgcca atgctaaatg agtacatgga gctcaacggt 900
tcatacttta ttcaaggcgg caaggaactg gcagccgttc tgatggaccc ttcaacttgt 960
tcccagtctt tggccataat tatcttgaag caggtggcta gtagatcagg tacctctccc 1020
gtttccacga gaacatgaat tttggtacta atnatg 1056

<210> 150
<211> 239
<212> PRT
<213> Triticum aestivum

<400> 150
Met Leu Ala Arg His Ser Ser Ser Pro Ala Gly Phe Phe Ser Asn Leu
1 5 10 15
Met Val Asp Asp Gly Tyr His Gly Ser Arg Gly Ala Gly Val Ala Gly
20 25 30
Gly Ser Ser Gly Gly Glu Ala His Arg His Ala Ser Ser Ser Thr Lys
35 40 45
Met Lys Ser Gln Leu Ser Phe Thr Ala Gly Gly Gln Gln Thr Thr Ala
50 55 60
His Leu Ser Arg Ile Ser Glu Gly Ala Ser Leu Phe Pro Ser Ala Asp
65 70 75 80
Ala Val Arg Ala Ala Ala His Ser Gly Ser Glu His Pro Val Ser Arg
85 90 95
Ser Phe Ser Ala Ser Gly Ser Ser Gly Gly Phe Ser Ile Val Gly Pro
100 105 110
Trp Asp Glu Ser Arg Glu Ile Ile Gly Thr Leu Asp Leu Gly Gly Tyr
115 120 125
Glu Ser Gln Phe Ser Gly Met Ala Ser Ser Ser Ser Leu Glu Leu Ala
130 135 140
Gly Met Asp Lys Tyr Met Gln Ala Gln Gln Gln Gln Asp Gln Val Ala
145 150 155 160
Phe Lys Val Arg Ala Lys Arg Gly Cys Ala Thr His Pro Arg Ser Ile
165 170 175
Ala Glu Arg Glu Arg Arg Thr Arg Ile Ser Tyr Lys Leu Arg Lys Leu
180 185 190
Gln Asp Leu Val Pro Asn Met Asp Lys Gln Thr Ser Thr Ser Asp Met
195 200 205
Leu Asp Leu Ala Val Glu His Ile Lys Gly Leu Gln Ser Gln Leu Gln
210 215 220
Ala Met Lys His Glu Gln Asp Lys Cys Thr Cys Cys Asn Lys Pro
225 230 235

<210> 151
<211> 873
<212> DNA
<213> Taraxacum officinale

<400> 151
gcgggcacag cacagatgat ttatcaaaac caccagcaat ccgatcataa tcataactcg 60
actgtctctg gctctacctc cgcattggtc aacccgctag cgacgatgaa tcctccaatg 120
aatcttcttc gtcagagtag ctcaccagct ggattcttcg aacacattaa tatggacaat 180
ggttactcta cgatgagatc catggacaaa tttcgaactg ctaacggcag tgtcccggat 240
tcagcaaaga gatttgaaaa ccaaatagct ttcttatcag gtttacattc atcttctaga 300
ccactgtctc ttatccccga acacgagccc aaaaccatga gaataagcgg tgcacataac 360
aacgcaggtt tcaatcaaaa tcttccgaaa actaccggtt acgttcccac ctttccccat 420
ggtacttctt gggatgaacg agcaatgtta actgacgatt tcatgaaaga actaggagaa 480
accgatcaaa taaattcatc tgaaaaccag aacgatgaag ggagaattca tgtttccact 540
ggtttgactc atcagatgag tttgccaacg ggttcctctg agttatccgt catggaaaaa 600
ctaatgcagt tacaagacag tgtcccttta agaagcaggg ctaaaagggg ctgtgctacc 660

```
catccacgaa gcatagcaga aagggtaaga agaacacgaa taagtgaaag aatgaggaag    720
ctacaagaac ttgtccccaa tatggacaag caaacaaaca cagcagacat gttggatttg    780
gctgtcgact acatcaaaga acttcaaaaa caagttgaga agctttcaga tcatcatgca    840
aagtgtacat gtctacataa gggggaaatg tga                                 873

<210>  152
<211>  285
<212>  PRT
<213>  Taraxacum officinale

<400>  152
Met Ile Tyr Gln Asn His Gln Gln Ser Asp His Asn His Asn Ser Thr
1               5                   10                  15
Val Ser Gly Ser Thr Ser Ala Leu Val Asn Pro Leu Ala Thr Met Asn
            20                  25                  30
Pro Pro Met Asn Leu Leu Arg Gln Ser Ser Ser Pro Ala Gly Phe Phe
        35                  40                  45
Glu His Ile Asn Met Asp Asn Gly Tyr Ser Thr Met Arg Ser Met Asp
    50                  55                  60
Lys Phe Arg Thr Ala Asn Gly Ser Val Pro Asp Ser Ala Lys Arg Phe
65                  70                  75                  80
Glu Asn Gln Ile Ala Phe Leu Ser Gly Leu His Ser Ser Ser Arg Pro
                85                  90                  95
Leu Ser Leu Ile Pro Glu His Glu Pro Lys Thr Met Arg Ile Ser Gly
            100                 105                 110
Ala His Asn Asn Ala Gly Phe Asn Gln Asn Leu Pro Lys Thr Thr Gly
        115                 120                 125
Tyr Val Pro Thr Phe Pro His Gly Thr Ser Trp Asp Glu Arg Ala Met
    130                 135                 140
Leu Thr Asp Asp Phe Met Lys Glu Leu Gly Glu Thr Asp Gln Ile Asn
145                 150                 155                 160
Ser Ser Glu Asn Gln Asn Asp Glu Gly Arg Ile His Val Ser Thr Gly
                165                 170                 175
Leu Thr His Gln Met Ser Leu Pro Thr Gly Ser Ser Glu Leu Ser Val
            180                 185                 190
Met Glu Lys Leu Met Gln Leu Gln Asp Ser Val Pro Leu Arg Ser Arg
        195                 200                 205
Ala Lys Arg Gly Cys Ala Thr His Pro Arg Ser Ile Ala Glu Arg Val
    210                 215                 220
Arg Arg Thr Arg Ile Ser Glu Arg Met Arg Lys Leu Gln Glu Leu Val
225                 230                 235                 240
Pro Asn Met Asp Lys Gln Thr Asn Thr Ala Asp Met Leu Asp Leu Ala
                245                 250                 255
Val Asp Tyr Ile Lys Glu Leu Gln Lys Gln Val Glu Lys Leu Ser Asp
            260                 265                 270
His His Ala Lys Cys Thr Cys Leu His Lys Gly Glu Met
        275                 280                 285

<210>  153
<211>  546
<212>  DNA
<213>  Vitis vinifera

<400>  153
atgttcgacg gcgccggtgc gcaagggttt ctccggcaga gcagcttgcc gacggagttc     60
ctctcccaga tcaattcgtc ggagggctat ttttcgagct ttgggattcc agcagggttc    120
gactacgcgg cgtcgccggc agtggatggc tctccgacag ggaagcgggc gagggagttg    180
gaatcgcgaa gttcgtctag gaaattttca tctcagtcga aaggggagca gagttctcga    240
ttaaccggta gcgtggcaag cttactggat gtggacatgg aaaagctttt agaggattca    300
```

gtaccctgca gggttcgggc caagcggggt tgtgctacac atcctcgcag cattgcagaa 360
agagttcgga ggacccgaat cagtgacaga ataaggaaac ttcaggaact tgtgcccaat 420
atggataagc aaacaaacac tgcagatatg ctagaagagg cagtagagta tgtcaagttt 480
ctacaacaaa aaattcagga actgtccgag catcaaaaaa aatgcacatg cttagctaga 540
gaataa 546

<210> 154
<211> 181
<212> PRT
<213> Vitis vinifera

<400> 154
Met Phe Asp Gly Ala Gly Ala Gln Gly Phe Leu Arg Gln Ser Ser Leu
1 5 10 15
Pro Thr Glu Phe Leu Ser Gln Ile Asn Ser Ser Glu Gly Tyr Phe Ser
20 25 30
Ser Phe Gly Ile Pro Ala Gly Phe Asp Tyr Ala Ala Ser Pro Ala Val
35 40 45
Asp Gly Ser Pro Thr Gly Lys Arg Ala Arg Glu Leu Glu Ser Arg Ser
50 55 60
Ser Ser Arg Lys Phe Ser Ser Gln Ser Lys Gly Glu Gln Ser Ser Arg
65 70 75 80
Leu Thr Gly Ser Val Ala Ser Leu Leu Asp Val Asp Met Glu Lys Leu
85 90 95
Leu Glu Asp Ser Val Pro Cys Arg Val Arg Ala Lys Arg Gly Cys Ala
100 105 110
Thr His Pro Arg Ser Ile Ala Glu Arg Val Arg Arg Thr Arg Ile Ser
115 120 125
Asp Arg Ile Arg Lys Leu Gln Glu Leu Val Pro Asn Met Asp Lys Gln
130 135 140
Thr Asn Thr Ala Asp Met Leu Glu Glu Ala Val Glu Tyr Val Lys Phe
145 150 155 160
Leu Gln Gln Lys Ile Gln Glu Leu Ser Glu His Gln Lys Lys Cys Thr
165 170 175
Cys Leu Ala Arg Glu
180

<210> 155
<211> 1062
<212> DNA
<213> Vitis vinifera

<400> 155
atgtatcctt cttctgcttc ctcctcttct caccgttcac tgcattcctc cggccttatt 60
cgctacggtt cagctccggg ctccctcttg acctccgccg tcgattccct cgtctcaact 120
gaccgtgaat tctcccccct cggatctcac catatcatgc ctcaccagta cttttccggt 180
gactcttcct ccgagtccac ctgtaaggtg gctgcgccgc cggatcacaa ggaggccgga 240
gccggaggcc ccgctgctat gttgcagagg tcctacgggt ttagtgagat gcctgtcgcc 300
ggcttctcta cagctagtag tttgaaaggc ggtggtgaag gcggcggtgg cggtggatct 360
gcgccgtcgt tgattcgcca tagcagctcc cctgctggct tcctcaacca gctcactgcc 420
gataacttga caaggggggac tggaaactat agctctcaag gtggttgtaa tggtggtcat 480
ggaatatcaa ggttgaagtc ccagttaagc ttcaagaggc aagattctct ttcccagatc 540
tcagaagtaa gtgagaatat ggttgatggc atcagctctg acaatggcca cagaaatgcc 600
actcattctt atgccactgc tagttttcct atggatgcct gggacaatac caacaccatt 660
gtgttttcca caacaccaaa caaacgagct aagaacatca atggtgacat tctcaacagt 720
ctcagttcct tagagccaca gtttagtctg ccacagacaa gtttagagat ggcagcagta 780
gagaagctgc tgcaagtacc tgaagactct gttccatgca aagttcgagc taagcgtggc 840
tgtgccactc atcctagaag tattgctgaa agggagagaa gaactagaat aagcgggaaa 900
ctgaagaaac tgcaagatct tgttcctaac atggataagc aaactagcta tgctgacatg 960

```
ttggacttgg ctgtgcaaca tatcaaaggt cttcaaaatg aagtgcagaa gctcaataaa   1020
gaactggaga actgtacttg tggttgcaag caagccctct aa                      1062

<210>  156
<211>  353
<212>  PRT
<213>  Vitis vinifera

<400>  156
Met Tyr Pro Ser Ser Ala Ser Ser Ser Ser His Arg Ser Leu His Ser
1               5                   10                  15
Ser Gly Leu Ile Arg Tyr Gly Ser Ala Pro Gly Ser Leu Leu Thr Ser
            20                  25                  30
Ala Val Asp Ser Leu Val Ser Thr Asp Arg Glu Phe Ser Pro Leu Gly
        35                  40                  45
Ser His His Ile Met Pro His Gln Tyr Phe Ser Gly Asp Ser Ser Ser
    50                  55                  60
Glu Ser Thr Cys Lys Val Ala Ala Pro Pro Asp His Lys Glu Ala Gly
65                  70                  75                  80
Ala Gly Gly Pro Ala Ala Met Leu Gln Arg Ser Tyr Gly Phe Ser Glu
                85                  90                  95
Met Pro Val Ala Gly Phe Ser Thr Ala Ser Ser Leu Lys Gly Gly Gly
            100                 105                 110
Glu Gly Gly Gly Gly Gly Gly Ser Ala Pro Ser Leu Ile Arg His Ser
        115                 120                 125
Ser Ser Pro Ala Gly Phe Leu Asn Gln Leu Thr Ala Asp Asn Leu Thr
    130                 135                 140
Arg Gly Thr Gly Asn Tyr Ser Ser Gln Gly Gly Cys Asn Gly Gly His
145                 150                 155                 160
Gly Ile Ser Arg Leu Lys Ser Gln Leu Ser Phe Lys Arg Gln Asp Ser
                165                 170                 175
Leu Ser Gln Ile Ser Glu Val Ser Glu Asn Met Val Asp Gly Ile Ser
            180                 185                 190
Ser Asp Asn Gly His Arg Asn Ala Thr His Ser Tyr Ala Thr Ala Ser
        195                 200                 205
Phe Pro Met Asp Ala Trp Asp Asn Thr Asn Thr Ile Val Phe Ser Thr
    210                 215                 220
Thr Pro Asn Lys Arg Ala Lys Asn Ile Asn Gly Asp Ile Leu Asn Ser
225                 230                 235                 240
Leu Ser Ser Leu Glu Pro Gln Phe Ser Leu Pro Gln Thr Ser Leu Glu
                245                 250                 255
Met Ala Ala Val Glu Lys Leu Leu Gln Val Pro Glu Asp Ser Val Pro
            260                 265                 270
Cys Lys Val Arg Ala Lys Arg Gly Cys Ala Thr His Pro Arg Ser Ile
        275                 280                 285
Ala Glu Arg Glu Arg Arg Thr Arg Ile Ser Gly Lys Leu Lys Lys Leu
    290                 295                 300
Gln Asp Leu Val Pro Asn Met Asp Lys Gln Thr Ser Tyr Ala Asp Met
305                 310                 315                 320
Leu Asp Leu Ala Val Gln His Ile Lys Gly Leu Gln Asn Glu Val Gln
                325                 330                 335
Lys Leu Asn Lys Glu Leu Glu Asn Cys Thr Cys Gly Cys Lys Gln Ala
            340                 345                 350
Leu


<210>  157
<211>  1185
<212>  DNA
```

<213>  Vitis vinifera

<400>  157
atggccccag atgtctataa tcatcagcac cacccccacc aaaactcagg cctaatgcgg     60
taccagtctg caccaagctc acttctgggg agttttgttg atggcagctc tgtccatctt    120
cagtcttcaa gccatgaaac cgaaaccatg ttcgcgaggc tcatgtcggg tagttcggat    180
tctcaaggcc tgcagggtgt tggagctatg aagcatgaag aagaggtgat ggtggagggt    240
gttccacagc agaatggata ctctaatggc tcccagatga tctacaacag ccagccaatg    300
caaactatat ccgttcataa ttcagcgagt ccccggacta atatggaaag ctcctttatg    360
acttccatgg ccgctgagaa ttctatgaag attaggaatg aaaattgttc cagtcttgtt    420
aggcagagca gctcccctcc tggacttttc cccaacttaa cttctgaaaa tggtatttgt    480
ttttcttcat ttcttcattc atttttgttt caaacttggt tggtaatggg aaaccttaga    540
gcaggcaaca gtactaatgt cgaagccaat ccgtcggtta gcagactgaa caatcaaata    600
agcttctcat ctgggttatc ttcttgcaat ggactactac ctaaaatctc cgaaatcaga    660
aatgaaaaca tagggctgca tagccccaag gatggcaata atagtaacag cagatgctac    720
atttctaact tcacaacaga ttcctggagt gattctccct tcagtggtct gacaagaatg    780
gctgctgata atgatttgaa gatgttttcg ggtttaaaca gtttggaagc tcagaatgtg    840
gattcgcgat atcggtccct tggcttgact caccatctga gcttgcctaa aaacttccct    900
cagatcacta ctatagagaa gtttctgcac ttccaagact cggttccttg taagattcga    960
gcgaaaagag gatgtgccac ccatcccaga agcatagcag agagagtgag aagaacccga   1020
atcagcgaaa gaatgaggaa actgcaggag ctttttccca acatggacaa gcaaacaaac   1080
acagcagata tgctagattt ggcagtagaa tacatcaaag accttcagaa acaggtcaag   1140
acacttaatg atactaaagt caagtgcaca tgttccagca tatga                   1185

<210>  158
<211>  394
<212>  PRT
<213>  Vitis vinifera

<400>  158
Met Ala Pro Asp Val Tyr Asn His Gln His His Pro His Gln Asn Ser
1               5                   10                  15
Gly Leu Met Arg Tyr Gln Ser Ala Pro Ser Ser Leu Leu Gly Ser Phe
            20                  25                  30
Val Asp Gly Ser Ser Val His Leu Gln Ser Ser Ser His Glu Thr Glu
        35                  40                  45
Thr Met Phe Ala Arg Leu Met Ser Gly Ser Ser Asp Ser Gln Gly Leu
    50                  55                  60
Gln Gly Val Gly Ala Met Lys His Glu Glu Glu Val Met Val Glu Gly
65                  70                  75                  80
Val Pro Gln Gln Asn Gly Tyr Ser Asn Gly Ser Gln Met Ile Tyr Asn
                85                  90                  95
Ser Gln Pro Met Gln Thr Ile Ser Val His Asn Ser Ala Ser Pro Arg
            100                 105                 110
Thr Asn Met Glu Ser Ser Phe Met Thr Ser Met Ala Ala Glu Asn Ser
        115                 120                 125
Met Lys Ile Arg Asn Glu Asn Cys Ser Ser Leu Val Arg Gln Ser Ser
    130                 135                 140
Ser Pro Pro Gly Leu Phe Pro Asn Leu Thr Ser Glu Asn Gly Ile Cys
145                 150                 155                 160
Phe Ser Ser Phe Leu His Ser Phe Leu Phe Gln Thr Trp Leu Val Met
                165                 170                 175
Gly Asn Leu Arg Ala Gly Asn Ser Thr Asn Val Glu Ala Asn Pro Ser
            180                 185                 190
Val Ser Arg Leu Asn Asn Gln Ile Ser Phe Ser Ser Gly Leu Ser Ser
        195                 200                 205
Cys Asn Gly Leu Leu Pro Lys Ile Ser Glu Ile Arg Asn Glu Asn Ile
    210                 215                 220
Gly Leu His Ser Pro Lys Asp Gly Asn Asn Ser Asn Ser Arg Cys Tyr

```
225                 230                 235                 240
Ile Ser Asn Phe Thr Thr Asp Ser Trp Ser Asp Ser Pro Phe Ser Gly
                245                 250                 255
Leu Thr Arg Met Ala Ala Asp Asn Asp Leu Lys Met Phe Ser Gly Leu
            260                 265                 270
Asn Ser Leu Glu Ala Gln Asn Val Asp Ser Arg Tyr Arg Ser Leu Gly
        275                 280                 285
Leu Thr His His Leu Ser Leu Pro Lys Asn Phe Pro Gln Ile Thr Thr
    290                 295                 300
Ile Glu Lys Phe Leu His Phe Gln Asp Ser Val Pro Cys Lys Ile Arg
305                 310                 315                 320
Ala Lys Arg Gly Cys Ala Thr His Pro Arg Ser Ile Ala Glu Arg Val
                325                 330                 335
Arg Arg Thr Arg Ile Ser Glu Arg Met Arg Lys Leu Gln Glu Leu Phe
            340                 345                 350
Pro Asn Met Asp Lys Gln Thr Asn Thr Ala Asp Met Leu Asp Leu Ala
        355                 360                 365
Val Glu Tyr Ile Lys Asp Leu Gln Lys Gln Val Lys Thr Leu Asn Asp
    370                 375                 380
Thr Lys Val Lys Cys Thr Cys Ser Ser Ile
385                 390
```

<210>  159
<211>  1101
<212>  DNA
<213>  Vitis vinifera

<400>  159

```
atggccagtg gcgggacaga agattcatcg tctcatactg ttatgaatat gaggcagaat     60
tctcaggcaa tggcgccaga atcagtggta gttgtgagcc agcagaatca gtttatggcg    120
tcgatgaagc acggagcgga ggttcttcag cagcagcaga atggttatgc ttctggttcg    180
cagatgatgt atcagacttc atcacctatg ccacatcata attcagcagc tcctggtaca    240
gtggagaatt cttacagtgc ggtttcttca atggggatgg atcagtcgca gcagataaag    300
attggtggag gcaacaattc gaatcttatt cgacatagta gctcacctgc aggactgttc    360
tcccacctaa atgttgaaaa tggctatgct ataatgagag gcatgggaaa tttcgggtct    420
ggaagtggga ctaatggaga accatctttt tcttctgcaa gcagattgaa gggtcaaata    480
aacttctcgt cagggccacc ttcttcctca ggcctagtga ctcccatctc tgaaatgggg    540
aacaaaagca tgggaacagg tagtccagac aatggaagtt ttggtgaagg tcatagtaac    600
agtggaggtt tcatcacagg cttcccaata ggctcttggg atgattctgc aatcatgtct    660
gaaagtttta gtagcttgaa aagtgttaga gatgatgagg caaagacttt ttctggtttg    720
aatgcatctg aagctcagaa gggcgagcct gcaaaccgcc ctcctgtttt ggctcatcac    780
ttgagtttgc caacaaagac ttctgcagat ttgacgacca ttgaaaagta tttgcagttc    840
caagattctg taccttgtaa gattcgagcc aaacggggat gtgccactca tccacgaagc    900
atcgctgaga gggtgagaag aacccgaatc agcgaaagga tgaggaaact acaagagctt    960
gtccctaaca tggacaagca aacaaacaca tcagacatgt tagatttggc tgttgactat   1020
attaaagacc ttcagaaaca ggtcaagacc ctctcagata atcgggcaaa gtgtacgtgt   1080
tcaaacaagc agaagccata g                                              1101
```

<210>  160
<211>  366
<212>  PRT
<213>  Vitis vinifera

<400>  160

```
Met Ala Ser Gly Gly Thr Glu Asp Ser Ser Ser His Thr Val Met Asn
1               5                   10                  15
Met Arg Gln Asn Ser Gln Ala Met Ala Pro Glu Ser Val Val Val Val
            20                  25                  30
Ser Gln Gln Asn Gln Phe Met Ala Ser Met Lys His Gly Ala Glu Val
```

```
        35                  40                  45
Leu Gln Gln Gln Gln Asn Gly Tyr Ala Ser Gly Ser Gln Met Met Tyr
    50                  55                  60
Gln Thr Ser Ser Pro Met Pro His His Asn Ser Ala Ala Pro Gly Thr
65                  70                  75                  80
Val Glu Asn Ser Tyr Ser Ala Val Ser Ser Met Gly Met Asp Gln Ser
                85                  90                  95
Gln Gln Ile Lys Ile Gly Gly Gly Asn Asn Ser Asn Leu Ile Arg His
            100                 105                 110
Ser Ser Ser Pro Ala Gly Leu Phe Ser His Leu Asn Val Glu Asn Gly
        115                 120                 125
Tyr Ala Ile Met Arg Gly Met Gly Asn Phe Gly Ser Gly Ser Gly Thr
    130                 135                 140
Asn Gly Glu Pro Ser Phe Ser Ser Ala Ser Arg Leu Lys Gly Gln Ile
145                 150                 155                 160
Asn Phe Ser Ser Gly Pro Pro Ser Ser Ser Gly Leu Val Thr Pro Ile
                165                 170                 175
Ser Glu Met Gly Asn Lys Ser Met Gly Thr Gly Ser Pro Asp Asn Gly
            180                 185                 190
Ser Phe Gly Glu Gly His Ser Asn Ser Gly Gly Phe Ile Thr Gly Phe
        195                 200                 205
Pro Ile Gly Ser Trp Asp Asp Ser Ala Ile Met Ser Glu Ser Phe Ser
    210                 215                 220
Ser Leu Lys Ser Val Arg Asp Asp Glu Ala Lys Thr Phe Ser Gly Leu
225                 230                 235                 240
Asn Ala Ser Glu Ala Gln Lys Gly Glu Pro Ala Asn Arg Pro Pro Val
                245                 250                 255
Leu Ala His His Leu Ser Leu Pro Thr Lys Thr Ser Ala Asp Leu Thr
            260                 265                 270
Thr Ile Glu Lys Tyr Leu Gln Phe Gln Asp Ser Val Pro Cys Lys Ile
        275                 280                 285
Arg Ala Lys Arg Gly Cys Ala Thr His Pro Arg Ser Ile Ala Glu Arg
    290                 295                 300
Val Arg Arg Thr Arg Ile Ser Glu Arg Met Arg Lys Leu Gln Glu Leu
305                 310                 315                 320
Val Pro Asn Met Asp Lys Gln Thr Asn Thr Ser Asp Met Leu Asp Leu
                325                 330                 335
Ala Val Asp Tyr Ile Lys Asp Leu Gln Lys Gln Val Lys Thr Leu Ser
            340                 345                 350
Asp Asn Arg Ala Lys Cys Thr Cys Ser Asn Lys Gln Lys Pro
        355                 360                 365
```

<210> 161
<211> 834
<212> DNA
<213> Vitis vinifera

<400> 161

```
atgagttgtc agagtcagtt gccgccgcag tatccgaggc aaagctcaag tacgagttcg     60
tctgccatgg atggctcata tggggttgtg aattccaaca acatggaagc caaacagggt    120
tcgagtcttg tcaggcaaag tagctctcct gctgggcttt tctctcacct ttcgggtcag    180
aatggctatg ccattatgag aggtgtggga aactttagac ctgggaatgt tggtaatgga    240
gaaatcagcc catcaactag caggttgaag cctcaagtta gcttctcttc tggattacct    300
tcttccttgg gactattgcc tcagatctct gaaattggaa gtgaccctgg attcccatat    360
ggctcttgga atgattctgc ccattttgag aacttcagtg gcatgaaaag ggaccaagac    420
aatgatggga agttgtattc tggttcaaat acatctggta ttcgggtatc attttgcata    480
gtagatgtat tttggactag tactattctc catacttcag ctgagatggc tgccatggag    540
aagttccttc agttccaaga ctctgttcct tgtaagattc gtgcaaagcg gggttgtgct    600
actcatccaa gaagcatcgc tgaaagggta aggagaaccc ggatcagcga aagaatgagg    660
```

aaactacaag agcttgtccc aaacatggac aagcaaacca acactgcaga catgttagat 720
ttggccgtgg aatacattaa ggaccttcag aaacagtaca atacacttac tgataatcgt 780
gcacactgca agtgtttagg taagcagaaa ccggtcccaa atcaaactgt ttga 834

<210> 162
<211> 277
<212> PRT
<213> Vitis vinifera

<400> 162
Met Ser Cys Gln Ser Gln Leu Pro Pro Gln Tyr Pro Arg Gln Ser Ser
1 5 10 15
Ser Thr Ser Ser Ser Ala Met Asp Gly Ser Tyr Gly Val Val Asn Ser
20 25 30
Asn Asn Met Glu Ala Lys Gln Gly Ser Ser Leu Val Arg Gln Ser Ser
35 40 45
Ser Pro Ala Gly Leu Phe Ser His Leu Ser Gly Gln Asn Gly Tyr Ala
50 55 60
Ile Met Arg Gly Val Gly Asn Phe Arg Pro Gly Asn Val Gly Asn Gly
65 70 75 80
Glu Ile Ser Pro Ser Thr Ser Arg Leu Lys Pro Gln Val Ser Phe Ser
85 90 95
Ser Gly Leu Pro Ser Ser Leu Gly Leu Leu Pro Gln Ile Ser Glu Ile
100 105 110
Gly Ser Asp Pro Gly Phe Pro Tyr Gly Ser Trp Asn Asp Ser Ala His
115 120 125
Phe Glu Asn Phe Ser Gly Met Lys Arg Asp Gln Asp Asn Asp Gly Lys
130 135 140
Leu Tyr Ser Gly Ser Asn Thr Ser Gly Ile Arg Val Ser Phe Cys Ile
145 150 155 160
Val Asp Val Phe Trp Thr Ser Thr Ile Leu His Thr Ser Ala Glu Met
165 170 175
Ala Ala Met Glu Lys Phe Leu Gln Phe Gln Asp Ser Val Pro Cys Lys
180 185 190
Ile Arg Ala Lys Arg Gly Cys Ala Thr His Pro Arg Ser Ile Ala Glu
195 200 205
Arg Val Arg Arg Thr Arg Ile Ser Glu Arg Met Arg Lys Leu Gln Glu
210 215 220
Leu Val Pro Asn Met Asp Lys Gln Thr Asn Thr Ala Asp Met Leu Asp
225 230 235 240
Leu Ala Val Glu Tyr Ile Lys Asp Leu Gln Lys Gln Tyr Asn Thr Leu
245 250 255
Thr Asp Asn Arg Ala His Cys Lys Cys Leu Gly Lys Gln Lys Pro Val
260 265 270
Pro Asn Gln Thr Val
275

<210> 163
<211> 951
<212> DNA
<213> Vitis vinifera

<400> 163
tattcctttt accttccggg gttttctgta gtggttgttt tattttggaa cagagtggaa 60
attttatcgg gaaaatttgt tggagggttg tgagggatgt ataatgtttc tcaagcttct 120
ccccatgaca tgagtcttat gtactcctcc aatttcaagc actctggaga agaagaaacg 180
gagaaaacca gagctgtatt catggattca aatacgaatt accatcacca gcaaaaccag 240
aatcaaccac ccaactccgg ccttctgcgg tttcgctcgg cgccgagctc gcttctcgcg 300
aatttcacgg acagtggaga tggtgttcac aagggagcta atctttgtga cgattttgaa 360

```
tcggagaggt tcatgccgtg tggggtttt caagattcgg ctatgagttg tgctactcat    420
ccaagaagca tcgctgaaag ggtaaggaga acccggatca gcgaaagaat gaggaaacta    480
caagagcttg tcccaaacat ggacaagcaa accaacactg cagacatgtt agatttggcc    540
gtggaataca ttaaggacct tcagaaacag tacaatacac ttactgataa tcgtgcacac    600
tgcaagtgtt taggtaagca gaaaccggtc ccaaatcaaa ctgtttgact tgcttctgta    660
caggaaacga ggaaaaagat gaaaagtgga tagaaaacag agctagcttc tctttttgc     720
actaacaata aagagagagg acattttttt tttcaagtgt gtgaagtaga aaggcagcta    780
gaatctctcc acgggaaaat ccagattctg taagcaaagg aagcttccaa ctacatcata    840
aagaagtaaa agtgataaat tgtatactgt ataggagacg tagcaataat aatgtaagaa    900
cacgttcctt gtgttgtatg taatttatcc tgttgtcttc aataaaatct a            951
```

<210> 164
<211> 183
<212> PRT
<213> Vitis vinifera

<400> 164

```
Met Tyr Asn Val Ser Gln Ala Ser Pro His Asp Met Ser Leu Met Tyr
1               5                   10                  15
Ser Ser Asn Phe Lys His Ser Gly Glu Glu Glu Thr Glu Lys Thr Arg
            20                  25                  30
Ala Val Phe Met Asp Ser Asn Thr Asn Tyr His His Gln Gln Asn Gln
        35                  40                  45
Asn Gln Pro Pro Asn Ser Gly Leu Leu Arg Phe Arg Ser Ala Pro Ser
    50                  55                  60
Ser Leu Leu Ala Asn Phe Thr Asp Ser Gly Asp Gly Val His Lys Gly
65                  70                  75                  80
Ala Asn Leu Cys Asp Asp Phe Glu Ser Glu Arg Phe Met Pro Cys Gly
                85                  90                  95
Gly Phe Gln Asp Ser Ala Met Ser Cys Ala Thr His Pro Arg Ser Ile
            100                 105                 110
Ala Glu Arg Val Arg Arg Thr Arg Ile Ser Glu Arg Met Arg Lys Leu
        115                 120                 125
Gln Glu Leu Val Pro Asn Met Asp Lys Gln Thr Asn Thr Ala Asp Met
    130                 135                 140
Leu Asp Leu Ala Val Glu Tyr Ile Lys Asp Leu Gln Lys Gln Tyr Asn
145                 150                 155                 160
Thr Leu Thr Asp Asn Arg Ala His Cys Lys Cys Leu Gly Lys Gln Lys
                165                 170                 175
Pro Val Pro Asn Gln Thr Val
            180
```

<210> 165
<211> 1139
<212> DNA
<213> Zea mays

<400> 165

```
gggccacctc tcgcggatct ccgaggacgg cgccttcccg ggaggcatga tgggcgaccg     60
cgcgggccgc ggctctgctg gcgagagcag cggtgccgcc gccgcggcgc gctcgtactc    120
ggccggcggg ttctccatcg ttgggccgtg ggaggagtcc agggacatca ttaccaccct    180
cggcgcatac gaccctcagt ttagcggcgc catggcgggt acggcgctag agatggcggg    240
catggacagg tacgtgcagc tgcagcagga ccaggtgccg ttcaaagtgc gcgccaagcg    300
cgggtgcgct acgcacccca ggagcatcgc cgagagggag aggaggacga ggatcagcga    360
gaagctcagg aagctccagg acctcgtgcc caacatggac aagcaaacga gcaccgcgga    420
catgtgggac cttgcagttg agcacatcag gggcctgcag agcgagctgc aggctctgaa    480
acacgaacag gagaagtgta cctgctgccg aaagcgatag cgattcacga cggccggggg    540
atcggaccaa gagagcagaa acacgtacgt cgtctatcat atctgtagcg gttcatagtt    600
gatacgtgcc cttagcttca gtgtccttga tgagaggaat gtacatggat gaagacatga    660
```

```
cagacagcca tctaatttta gttacaacaa catgtcggag tacctactgc tccacctttt    720
ccgaagaccg aagcaacgaa gacccacgac ccacgtgagc agtacctagc cagtagccag    780
tgcagtgcag tgctaaaatg ggcggccctc ctggtctgat ccgatgatcc ttggatttct    840
tctgttcctg cttgatcttc aacttcaatt ttagatctct tttaggatct gaaaacagtt    900
ttgctattta tttaaacaag ctgcttaatt ttattttgat atttagtata attaatattt    960
gttgactgac gtcagaagaa gaggctccta cttttttttt tcttttgtat aaggtaaaag   1020
aaagggcttt taggctttta tacagatata aaggctttgg tgatgttttt gtatatactt   1080
cttaaactta taattgtttc aataggcatt ataatagatc tgtattattt cagaaaaaa    1139

<210>  166
<211>  157
<212>  PRT
<213>  Zea mays

<400>  166
Met Met Gly Asp Arg Ala Gly Arg Gly Ser Ala Gly Glu Ser Ser Gly
1               5                   10                  15
Ala Ala Ala Ala Ala Arg Ser Tyr Ser Ala Gly Gly Phe Ser Ile Val
            20                  25                  30
Gly Pro Trp Glu Glu Ser Arg Asp Ile Ile Thr Thr Leu Gly Ala Tyr
        35                  40                  45
Asp Pro Gln Phe Ser Gly Ala Met Ala Gly Thr Ala Leu Glu Met Ala
    50                  55                  60
Gly Met Asp Arg Tyr Val Gln Leu Gln Gln Asp Gln Val Pro Phe Lys
65                  70                  75                  80
Val Arg Ala Lys Arg Gly Cys Ala Thr His Pro Arg Ser Ile Ala Glu
                85                  90                  95
Arg Glu Arg Arg Thr Arg Ile Ser Glu Lys Leu Arg Lys Leu Gln Asp
            100                 105                 110
Leu Val Pro Asn Met Asp Lys Gln Thr Ser Thr Ala Asp Met Trp Asp
        115                 120                 125
Leu Ala Val Glu His Ile Arg Gly Leu Gln Ser Glu Leu Gln Ala Leu
    130                 135                 140
Lys His Glu Gln Glu Lys Cys Thr Cys Cys Arg Lys Arg
145                 150                 155

<210>  167
<211>  1736
<212>  DNA
<213>  Zea mays

<400>  167
ctacaacacg cctccccagt caacacagct cagctcagct cggctagcga gctcctcgct     60
cggctctact agctagtgtg ttgggacttg ggagctaggc ggggttgatt gcgattgggg    120
gaggcgggag taggtgatgt acggcgcgcc gttgcccaag gacctgaacc tgccagcagc    180
cctgccgcag ccgaccagga cgccaccgca gcagcagatg ggctccccgg gactgctgcg    240
gtacaggtca gcgccgagca cgctgctcgg cgaggtgatg tgtggtggtg atcaggactt    300
ccccgcggct ggcggcgcag ggcacggacc accggaccac gccgccgcgg acaacgtcct    360
cgcgcgcttt ctcgccgggc accactccga gacccgcgac tgcaagcctc cccgccccgc    420
cgccgccgct cacttcatgg acgaagctgc cgtcgcgtcc atggccgcct cacagcagca    480
gcagctcatg taccagtcgc agcaggagca gcagatggcc gccatggagg ggctgtaccg    540
caacgtcagc tccggcggca cggagcacgg tgcagccgtt ggcgctggta acaacagcct    600
gatccgccag agcagctccc ccgccggctt cctcaaccac ttgaacatgg acaacgggta    660
cgggagcatg ctccgggtgg gcatgggcgg cggcgggttc aggaacggcg tcagcgacgc    720
gcggctcaag ggcagctgag cttctcgtc ccggcagggg tcggtgatgt cgcagatctc     780
ggaggtgggc agcgaggagc tcgacggagg cggcggcagc ggcagccccg aggcggccgg    840
cagcaacgcc agcggcgcgg cgcgtgggta cagcggcatc ccggggtacc cgatggcgg     900
cctcgcgtcc ggcgcctggc cggacgaggc gtcgccgtcg ccgacgtccg gcgcgaagcg    960
cccccgcgac tccggccccg cgctgcagca gccgctggcg ccgcagctca gcctgcccag   1020
```

```
cggaaagaac aagggcggca gggcggcctc ggcggagatg gcggcgatcg agaagttcct   1080
ccagttccag gacgccgtcc cctgcaagat ccgcgccaag cgcgggtgcg ccacccaccc   1140
gcgcagcatc gccgagcggg tgaggaggac gaagatcagc gagcggatac ggaagctgca   1200
ggagctcgtg cccaacatgg aaaagcaaac caacactgcc gacatgctgg atctggccgt   1260
ggactacatc aaggatcttc agaagcaggt caaggtactg aacgacggcc gcgccagctg   1320
cacctgctcc gccggcgagc tgcagggcca gttcacccgt taaaaaggag ccgcgccacc   1380
tttttctcag gggggccagt tcagctcgca tagttgcgtt acgttacatg gtgaggactg   1440
atcgaggagg tatatattgg aattgagacg atagtagcta gtctgtacat gcaacaagtt   1500
cattagcagg cagagtaggc ctcgcatgtt agtgatagct gcgtcagtta atgtacgttt   1560
agattgcaca tgtattcatc ttgatttaca tgtgttagag tagtttagtg tggattttag   1620
tttaaatttc acattaatcc acttcaacat acattaaccg atgtgggtat atatgaccat   1680
ctaattagaa tctttatata cacatgtatt tactcaatcc acatgattag cctgtc       1736

<210>  168
<211>  408
<212>  PRT
<213>  Zea mays

<400>  168
Met Tyr Gly Ala Pro Leu Pro Lys Asp Leu Asn Leu Pro Ala Ala Leu
1               5                   10                  15
Pro Gln Pro Thr Arg Thr Pro Pro Gln Gln Gln Met Gly Ser Pro Gly
            20                  25                  30
Leu Leu Arg Tyr Arg Ser Ala Pro Ser Thr Leu Leu Gly Glu Val Met
        35                  40                  45
Cys Gly Gly Asp Gln Asp Phe Pro Ala Ala Gly Gly Ala Gly His Gly
    50                  55                  60
Pro Pro Asp His Ala Ala Ala Asp Asn Val Leu Ala Arg Phe Leu Ala
65                  70                  75                  80
Gly His His Ser Glu Thr Arg Asp Cys Lys Pro Pro Arg Pro Ala Ala
                85                  90                  95
Ala Ala His Phe Met Asp Glu Ala Ala Val Ala Ser Met Ala Ala Ser
            100                 105                 110
Gln Gln Gln Gln Leu Met Tyr Gln Ser Gln Gln Glu Gln Gln Met Ala
        115                 120                 125
Ala Met Glu Gly Leu Tyr Arg Asn Val Ser Ser Gly Gly Thr Glu His
    130                 135                 140
Gly Ala Ala Val Gly Ala Gly Asn Asn Ser Leu Ile Arg Gln Ser Ser
145                 150                 155                 160
Ser Pro Ala Gly Phe Leu Asn His Leu Asn Met Asp Asn Gly Tyr Gly
                165                 170                 175
Ser Met Leu Arg Val Gly Met Gly Gly Gly Gly Phe Arg Asn Gly Val
            180                 185                 190
Ser Asp Ala Arg Leu Lys Gly Gln Leu Ser Phe Ser Ser Arg Gln Gly
        195                 200                 205
Ser Val Met Ser Gln Ile Ser Glu Val Gly Ser Glu Glu Leu Asp Gly
    210                 215                 220
Gly Gly Gly Ser Gly Ser Pro Glu Ala Ala Gly Ser Asn Ala Ser Gly
225                 230                 235                 240
Ala Ala Arg Gly Tyr Ser Gly Ile Pro Gly Tyr Pro Met Gly Gly Leu
                245                 250                 255
Ala Ser Gly Ala Trp Pro Asp Glu Ala Ser Pro Ser Pro Thr Ser Gly
            260                 265                 270
Ala Lys Arg Pro Arg Asp Ser Gly Pro Ala Leu Gln Gln Pro Leu Ala
        275                 280                 285
Pro Gln Leu Ser Leu Pro Ser Gly Lys Asn Lys Gly Gly Arg Ala Ala
    290                 295                 300
Ser Ala Glu Met Ala Ala Ile Glu Lys Phe Leu Gln Phe Gln Asp Ala
305                 310                 315                 320
```

```
Val Pro Cys Lys Ile Arg Ala Lys Arg Gly Cys Ala Thr His Pro Arg
                325                 330                 335
Ser Ile Ala Glu Arg Val Arg Arg Thr Lys Ile Ser Glu Arg Ile Arg
            340                 345                 350
Lys Leu Gln Glu Leu Val Pro Asn Met Glu Lys Gln Thr Asn Thr Ala
        355                 360                 365
Asp Met Leu Asp Leu Ala Val Asp Tyr Ile Lys Asp Leu Gln Lys Gln
    370                 375                 380
Val Lys Val Leu Asn Asp Gly Arg Ala Ser Cys Thr Cys Ser Ala Gly
385                 390                 395                 400
Glu Leu Gln Gly Gln Phe Thr Arg
                405

<210>  169
<211>  905
<212>  DNA
<213>  Zea mays

<400>  169
cttctccagc cccgtcatgg ataacggttt tccaagtgat agggttgaag ttggtggtga     60
ggttcaccat gcaatgaggg actatcacaa gaagatgaag tccccccctga acttgtccaa    120
acaaggagcc ctctctcaat tatgtgagca tggattatcg gatggtctca ccaataatgt    180
gcatggaact ggacactcca aagagaacat caccaccaac aacgtggcac gtcccttcac    240
aagtgggttc tcaattggat catgggagga ttcaaattct attgcgtttt ccaacccagc    300
aaacaaatcg ggaatacaca ataatgatga catcatagac aatattagta actcttatga    360
actacagttt ggtgtggcga aggaaatggc gggcctacta cagatacaac aagaccgggt    420
tccattcaga gtacgagcaa agcgtggatg tgccacacat cctaggagca tcgcagagcg    480
agagcgaaga acgaggatca gcgagaagct caggaagttg caggcccttg tgcctaacat    540
ggacaagcaa actagcacag cggacatgct ggatttagca gttgaccaca tcagaggact    600
ccagaacgag ctgcaggtat atgctctcaa ggaagacaag gagaagtgca gctgccgcgg    660
caatcgtcca gcggggagat aacgcacgta cgtaggctaa aaacaatggg ttctcggcaa    720
cacattatag aacggcattg cagacattgt tgtcagacag cagcagcatc agaaggtggc    780
tgctgtggca tatatattgg cttcagcaga ccggtcgaga tatttcatat tcgttaaaaa    840
cctagtgttt tagcttgttc gtttcacttt aatttatatc ggtctctact aaaaaaaaaa    900
aaaaa                                                                 905

<210>  170
<211>  221
<212>  PRT
<213>  Zea mays

<400>  170
Met Asp Asn Gly Phe Pro Ser Asp Arg Val Glu Val Gly Gly Glu Val
1               5                   10                  15
His His Ala Met Arg Asp Tyr His Lys Lys Met Lys Ser Pro Leu Asn
            20                  25                  30
Leu Ser Lys Gln Gly Ala Leu Ser Gln Leu Cys Glu His Gly Leu Ser
        35                  40                  45
Asp Gly Leu Thr Asn Asn Val His Gly Thr Gly His Ser Lys Glu Asn
    50                  55                  60
Ile Thr Thr Asn Asn Val Ala Arg Pro Phe Thr Ser Gly Phe Ser Ile
65                  70                  75                  80
Gly Ser Trp Glu Asp Ser Asn Ser Ile Ala Phe Ser Asn Pro Ala Asn
                85                  90                  95
Lys Ser Gly Ile His Asn Asn Asp Asp Ile Ile Asp Asn Ile Ser Asn
            100                 105                 110
Ser Tyr Glu Leu Gln Phe Gly Val Ala Lys Glu Met Ala Gly Leu Leu
        115                 120                 125
Gln Ile Gln Gln Asp Arg Val Pro Phe Arg Val Arg Ala Lys Arg Gly
```

```
    130                 135                 140
Cys Ala Thr His Pro Arg Ser Ile Ala Glu Arg Glu Arg Arg Thr Arg
145                 150                 155                 160
Ile Ser Glu Lys Leu Arg Lys Leu Gln Ala Leu Val Pro Asn Met Asp
                165                 170                 175
Lys Gln Thr Ser Thr Ala Asp Met Leu Asp Leu Ala Val Asp His Ile
            180                 185                 190
Arg Gly Leu Gln Asn Glu Leu Gln Val Tyr Ala Leu Lys Glu Asp Lys
        195                 200                 205
Glu Lys Cys Ser Cys Arg Gly Asn Arg Pro Ala Gly Arg
    210                 215                 220
```

<210> 171
<211> 1958
<212> DNA
<213> Zea mays

<400> 171

```
gagggaaagt gaagcaggag ggtggaccac tgcgcgcgtg cggcgccttc ctcctcgaat      60
caccgccgac agcttctgca gctagcgtta tcacccccac caccccgggg ctcggattag     120
gtacgctgtt catgccgtct ccacggccaa ggaccgggga ggagcttctt tctctagctg     180
ggggatgagc aacaccaagt gatgttccat catgtttgct taaaaagaca cgcactgcct     240
ctgcctgtgc ctgcctgttc atggtcctca tcctcacaga gctggttacc gaaaaattgg     300
tatacttttt cttggcgcag gatctgagct gaagctgcca ggccaggcga tcgagccgat     360
gtacggctcg ccggtgtcca aggacctcaa cctcccggcg cagccgccgc cgatggcctc     420
gtccgggctg ctacgctaca gatcggcgcc cagcgcggtg ctcggcggcc tctgcgagga     480
ccagctccag ctccctgccg ccgctccgag cgccgccgac aacgtcttct caaggttcct     540
gcccgaccac cacatccggg acgacaagcc gtcgcctgcc cacttcccga gtgcggccga     600
catggcctcc caccaccagc aggagcaaat gatgttccac tcccagtccc agtcccagca     660
ccagcaggag acgggtaggt ccgggggcgg cctctaccgc accgtcagct ctggcatgga     720
agcgggcggc actggcgtcg gcgccgccag cagcctgatt cggcagagca gctcccccgc     780
cgggttcctg gaccatttcg gcatggacaa cgggtacggg gcgatgctga gggcgagcat     840
gggcatgggg ttccaggacg gtggtgccag tgactccctc gcgggcggcg gcggcggtag     900
cggcaggctc gggggccagc tgagcttctc gtcgcggcag ggtcgctca tgtcccagat      960
ctcggagatg gacagccagg aggatgtcgt cggagcgagc agccccgacg ctggcggcgg    1020
cggggacgcc gcctacatgc cggggtaccc gatgagctcc ggcgggtggg acgactcgtc    1080
gtcggcgctc ttgccggaca gcctgcccgc gacgaataaa cgcccgcggg actcgttgga    1140
gcacggcggc gggctggcgc accagttcag cctccccaag acgtcgtcgt cggaggtggc    1200
cgccatcgag aagttcctcc agttccagga cgccgtgccc tgcaaggtcc gcgccaagcg    1260
cgggtgcgcc acgcacccgc gcagcatcgc cgagcgggtg aggaggacga agatcagcga    1320
gcggatcagg aagctgcagg agctcgtgcc cgacatggac aagcaaacca acacctccga    1380
catgctggac ttggccgtcg actacatcaa ggatctccag aagcaggtca aggcgttgaa    1440
cgagagccgc gccagctgca cctgcccggc gagcaagcac cagcagcggt tctccggctg    1500
aagcaccggc cgggccggca gtccccatcg gtccccggcg gctgcgtagc acgctccagt    1560
acacagctag tgcagcaaag atggacccac cacaaggcct ggagacagac ctgactgaga    1620
ccgagataga gaggaacacc gccatcgtct gcagtgcatg cgcgcgagca gcatgtacag    1680
cgccggagcg atcgaggctc gctggactgc tggatcgagc agtgtggcat catcaagtat    1740
tcaagcgact gggaggaagg aagaaagaat cgagtcatgg gaccgaggag gaagaacgaa    1800
tgaattgtac agtgctgttt cttttttttt ttttgctgta aacacttttg ctacatctgt    1860
gctgctgctg ctctccatat gccgttgccc attgttacta cgttaagaat cacagtgaga    1920
tatagatcga ggaaaacttc actactgtta ccaccacc                            1958
```

<210> 172
<211> 380
<212> PRT
<213> Zea mays

<400> 172

```
Met Tyr Gly Ser Pro Val Ser Lys Asp Leu Asn Leu Pro Ala Gln Pro
```

```
1               5                   10                  15
Pro Pro Met Ala Ser Ser Gly Leu Leu Arg Tyr Arg Ser Ala Pro Ser
            20                  25                  30
Ala Val Leu Gly Gly Leu Cys Glu Asp Gln Leu Gln Leu Pro Ala Ala
        35                  40                  45
Ala Pro Ser Ala Ala Asp Asn Val Phe Ser Arg Phe Leu Pro Asp His
    50                  55                  60
His Ile Arg Asp Asp Lys Pro Ser Pro Ala His Phe Pro Ser Ala Ala
65                  70                  75                  80
Asp Met Ala Ser His His Gln Gln Glu Gln Met Met Phe His Ser Gln
                85                  90                  95
Ser Gln Ser Gln His Gln Gln Glu Thr Gly Arg Ser Gly Gly Gly Leu
            100                 105                 110
Tyr Arg Thr Val Ser Ser Gly Met Glu Ala Gly Gly Thr Gly Val Gly
        115                 120                 125
Ala Ala Ser Ser Leu Ile Arg Gln Ser Ser Ser Pro Ala Gly Phe Leu
    130                 135                 140
Asp His Phe Gly Met Asp Asn Gly Tyr Gly Ala Met Leu Arg Ala Ser
145                 150                 155                 160
Met Gly Met Gly Phe Gln Asp Gly Gly Ala Ser Asp Ser Leu Ala Gly
                165                 170                 175
Gly Gly Gly Gly Ser Gly Arg Leu Gly Gly Gln Leu Ser Phe Ser Ser
            180                 185                 190
Arg Gln Gly Ser Leu Met Ser Gln Ile Ser Glu Met Asp Ser Gln Glu
        195                 200                 205
Asp Val Val Gly Ala Ser Ser Pro Asp Ala Gly Gly Gly Gly Asp Ala
    210                 215                 220
Ala Tyr Met Pro Gly Tyr Pro Met Ser Ser Gly Gly Trp Asp Asp Ser
225                 230                 235                 240
Ser Ser Ala Leu Leu Pro Asp Ser Leu Pro Ala Thr Asn Lys Arg Pro
                245                 250                 255
Arg Asp Ser Leu Glu His Gly Gly Gly Leu Ala His Gln Phe Ser Leu
            260                 265                 270
Pro Lys Thr Ser Ser Ser Glu Val Ala Ala Ile Glu Lys Phe Leu Gln
        275                 280                 285
Phe Gln Asp Ala Val Pro Cys Lys Val Arg Ala Lys Arg Gly Cys Ala
    290                 295                 300
Thr His Pro Arg Ser Ile Ala Glu Arg Val Arg Arg Thr Lys Ile Ser
305                 310                 315                 320
Glu Arg Ile Arg Lys Leu Gln Glu Leu Val Pro Asp Met Asp Lys Gln
                325                 330                 335
Thr Asn Thr Ser Asp Met Leu Asp Leu Ala Val Asp Tyr Ile Lys Asp
            340                 345                 350
Leu Gln Lys Gln Val Lys Ala Leu Asn Glu Ser Arg Ala Ser Cys Thr
        355                 360                 365
Cys Pro Ala Ser Lys His Gln Gln Arg Phe Ser Gly
    370                 375                 380
```

<210> 173
<211> 1392
<212> DNA
<213> Zingiber officinale

<400> 173

```
ttctcctccc gccgcgagtc ggcccctctc tgtctctctc taccgcgcgc acggaggcga     60
tgaaccattc gccgtctggc ggagaccgtc cggccgcgat ggggccccccg ccggggctcg    120
ctcgctacgg ctcggccccc gggtcgttcc tcggcgggat cgccgactcg gtcatcgccg    180
ccggcggatc ggagaggatg atgacccggt tctacgccgg cgactcgccg tgcctgacct    240
ccgaatcgag ctgctgggcc ccggacggcg acgccgccgc agccgccgcc gctggaggtt    300
```

```
gctcgcgggg gtcgtacgga tccggggagc tccaactctc ttccgccacc ggatccccgt    360
tggtccggca cagcagcttg cctacggggt tcttctccca tctactggcc gatcacgcag    420
gtaattctag tacaaggatg attggaaact actctcaagc aggcacagaa agcatccatg    480
tattggcaca tagaaggcag catgccccgt ggggcttctc gcggcaagac tccttgtcgc    540
aaatttctga attgagcatc ccagaaatgg aagagagtga cacgcaccat gattcagacg    600
aggtagctgg gaatgccggc cagtcctaca tttctagcaa ttttcaatta ggctcatggg    660
atgataaaaa ttcaattgct ttttctgctc cacaaagcaa aaggattaag tgcgataacg    720
gggatgcagt tgcaagtctc ggtaacattg attcacagtt tagttttcca aggacatcat    780
ctgaaatgtc tgagttagag aaataccttc agatccaaca agattcagtc ccttgcagag    840
cacgagctaa gcgtggctgc gcaactcatc cccggagcat tgctgagagg gaaaggagaa    900
cacgaattag caaaaggctg cagaaattgc aagatcttgt gcctaacatg gacaagcaaa    960
cgagcacctc agatatgctg gaattggcaa ttcaacacat caaagagctg cagggccaag   1020
tagagaagct caaacaagaa caaacaaatt gtacctgcac tgcaaagcag gagaaggctt   1080
gagggcggcc agtcactgtc attatagcta ctttgtcttt ttgtttttta aaacaaaaat   1140
tgctataaac gatcatattg agttcattga actagaatgg atccatggca agataaggag   1200
ttgtacattg ccatttccgt tcatctgtat ttgagttggc agccgtattc cctccctgct   1260
aggttcatca agccggccga cctaccggtc agctgtgccg gagctgcttc atttctttcc   1320
aaacattagt aaacattcaa atacttatca tgttttacat ttcaaaatgc aaataattat   1380
aataaagaaa tg                                                       1392
```

<210> 174
<211> 340
<212> PRT
<213> Zingiber officinale

<400> 174

```
Met Asn His Ser Pro Ser Gly Gly Asp Arg Pro Ala Ala Met Gly Pro
1               5                   10                  15
Pro Pro Gly Leu Ala Arg Tyr Gly Ser Ala Pro Gly Ser Phe Leu Gly
            20                  25                  30
Gly Ile Ala Asp Ser Val Ile Ala Ala Gly Gly Ser Glu Arg Met Met
        35                  40                  45
Thr Arg Phe Tyr Ala Gly Asp Ser Pro Cys Leu Thr Ser Glu Ser Ser
    50                  55                  60
Cys Trp Ala Pro Asp Gly Asp Ala Ala Ala Ala Ala Ala Ala Gly Gly
65                  70                  75                  80
Cys Ser Arg Gly Ser Tyr Gly Ser Gly Glu Leu Gln Leu Ser Ser Ala
                85                  90                  95
Thr Gly Ser Pro Leu Val Arg His Ser Ser Leu Pro Thr Gly Phe Phe
            100                 105                 110
Ser His Leu Leu Ala Asp His Ala Gly Asn Ser Ser Thr Arg Met Ile
        115                 120                 125
Gly Asn Tyr Ser Gln Ala Gly Thr Glu Ser Ile His Val Leu Ala His
    130                 135                 140
Arg Arg Gln His Ala Pro Trp Gly Phe Ser Arg Gln Asp Ser Leu Ser
145                 150                 155                 160
Gln Ile Ser Glu Leu Ser Ile Pro Glu Met Glu Glu Ser Asp Thr His
                165                 170                 175
His Asp Ser Asp Glu Val Ala Gly Asn Ala Gly Gln Ser Tyr Ile Ser
            180                 185                 190
Ser Asn Phe Gln Leu Gly Ser Trp Asp Asp Lys Asn Ser Ile Ala Phe
        195                 200                 205
Ser Ala Pro Gln Ser Lys Arg Ile Lys Cys Asp Asn Gly Asp Ala Val
    210                 215                 220
Ala Ser Leu Gly Asn Ile Asp Ser Gln Phe Ser Phe Pro Arg Thr Ser
225                 230                 235                 240
Ser Glu Met Ser Glu Leu Glu Lys Tyr Leu Gln Ile Gln Gln Asp Ser
                245                 250                 255
Val Pro Cys Arg Ala Arg Ala Lys Arg Gly Cys Ala Thr His Pro Arg
```

```
            260                 265                 270
Ser Ile Ala Glu Arg Glu Arg Arg Thr Arg Ile Ser Lys Arg Leu Gln
        275                 280                 285
Lys Leu Gln Asp Leu Val Pro Asn Met Asp Lys Gln Thr Ser Thr Ser
    290                 295                 300
Asp Met Leu Glu Leu Ala Ile Gln His Ile Lys Glu Leu Gln Gly Gln
305                 310                 315                 320
Val Glu Lys Leu Lys Gln Glu Gln Thr Asn Cys Thr Cys Thr Ala Lys
                325                 330                 335
Gln Glu Lys Ala
            340
```

```
<210>  175
<211>  2228
<212>  DNA
<213>  Zingiber officinale

<400>  175
gcacccttt aaaattgaag ttcatggaag aaaattgaaa catagcctaa aaaaaaaaca      60
gttattagag aggacgagaa agagagatgt caacatattt tatacaaact tggaatgcac    120
cccactatgg agtcatgaat tttgtttcag tagaaatcaa tgaggaaggc atctcactcg    180
agttctcaca tgcaattatt ttcaggggga gcagcatcca taatccatta ggcgtgtttg    240
tttggcatgg aatttggggg ggtgtgttgg ctagaagttg aatggtctct ctcgttgtca    300
ttgtccaggc taccattagc ttcaccagcg tcttcttcgt catcctgtgc gaaagatcct    360
gctctcaaga gttctttgac cttatggaat tcgtcatagt gagctttccg gtggtcgttg    420
aaacttagcc ttggagcatc tgcttcagaa tcatcatctt cttccatggc atcagtttct    480
tcttcagaag aggtccaatc cccatttgag cttctgcttg aagaagctac atcattcaaa    540
gcagtagtga gcctcgtgcc gaattcggca cgaggattta tttgttcaca tcaaacctgg    600
atatgcagca atgtaaatca acactaaggc catgaaactt tcattgtggt attaaaatcc    660
atttacaatg aaatctactt cttcccccctc tgattcttcc acctctcatt ttccattcac    720
atttctaacc gtacgcatac aacacaatct ctacaaaagc agcttcacct cttgtgaaga    780
gtattcacaa gttcatggac gaaccgcggc agaaataagt cttaggaagt ggaggtggag    840
gaggagaccg gccgtgaaag aaaaaggtgg gcgttgtttt gtggttttgg atcgaggatt    900
tgggataaag attggatttt tggagtgtat agtgatgtat ggatcgccgt cgatgccggc    960
gtcgaaggat ctaagctcca ttctgttcgc cggaaaccac cctcaggagc acccgatgag   1020
ctccggcggc ctcctccgct accgatcggc tccgagctcc ctgctcggag aaatccgcga   1080
ggatttcctc ccggtgagct cccccccgtg tcccgagaca gcaacaagcg ccgccatgtt   1140
ctgtagattt ttagccgagg atatccagat ccgcgacgac gtgccccccg gacgaggtgg   1200
cgcggccgca gcccagccgc tcctccctca gttcactacg ctggcgtcgg cggagcggag   1260
tggagcactt ttctcccctg cttcgcagca aaatccacca gcagcagcag cagcagcagc   1320
agcagctcgc gagggtgatg gagcaaacta agaacggcgg cgccgccgcc tccggctcat   1380
caaatctcgt aaggcacaga agttcccccg ccggagttct ctcgcatctc aacgcgaatg   1440
ctggatatcc aaacatatca aagaatcaaa tcagcttctc ttcaatccaa aacatctccg   1500
aggtcgtcat cgccgggagc agctccgacg acagcagcgg atacataccg cgattcccgt   1560
cgagcaataa ttgggagaac ttttcgcgga gaaagagcac gatcgacgat actctcaatc   1620
caacagagct cgaggcgagg aatcacgcgc cgactctgcc ccatcagttc agcttgccgc   1680
cgaaaccctc gccggaaatg gccgccgtgg agaactactt gcacttccag gactcaggcc   1740
catttaagat ccgagccaag cgaggttgcg cgactcatcc ccgaagcata gccgagagag   1800
tgaggagaac aagaatcagt gaaagaataa agaagctgca ggaactagtc ccaaacatgg   1860
atacgcaaac caacactgca gacatgttag gtctggccat cagctacatc aaagatctcc   1920
aagaacaagt tcagacactt tccgagagcc ggtcgagctg cacctgttca tctggcaagc   1980
agaagcaaga tcatcatcat catcatccat cagcttgaga agtagaaaaa tagaaaagat   2040
ccatgtagat atatctgtgt ttttatgaag aatctgtaag tctttagatg tactgaagag   2100
tgttcttcct aaaccacatt gtcaattcca tgaactcgat gatgattaca agctaatatt   2160
cgaccggcaa gctgtaaaat aataatggag aaaggaactt gtccccctcg tgccgaattc   2220
ggcacgag                                                            2228

<210>  176
<211>  226
```

<212> PRT
<213> Zingiber officinale

<400> 176
```
Met Glu Gln Thr Lys Asn Gly Gly Ala Ala Ala Ser Gly Ser Ser Asn
1               5                   10                  15
Leu Val Arg His Arg Ser Ser Pro Ala Gly Val Leu Ser His Leu Asn
            20                  25                  30
Ala Asn Ala Gly Tyr Pro Asn Ile Ser Lys Asn Gln Ile Ser Phe Ser
        35                  40                  45
Ser Ile Gln Asn Ile Ser Glu Val Val Ile Ala Gly Ser Ser Ser Asp
    50                  55                  60
Asp Ser Ser Gly Tyr Ile Pro Arg Phe Pro Ser Ser Asn Asn Trp Glu
65                  70                  75                  80
Asn Phe Ser Arg Arg Lys Ser Thr Ile Asp Asp Thr Leu Asn Pro Thr
                85                  90                  95
Glu Leu Glu Ala Arg Asn His Ala Pro Thr Leu Pro His Gln Phe Ser
            100                 105                 110
Leu Pro Pro Lys Pro Ser Pro Glu Met Ala Ala Val Glu Asn Tyr Leu
        115                 120                 125
His Phe Gln Asp Ser Gly Pro Phe Lys Ile Arg Ala Lys Arg Gly Cys
    130                 135                 140
Ala Thr His Pro Arg Ser Ile Ala Glu Arg Val Arg Arg Thr Arg Ile
145                 150                 155                 160
Ser Glu Arg Ile Lys Lys Leu Gln Glu Leu Val Pro Asn Met Asp Thr
                165                 170                 175
Gln Thr Asn Thr Ala Asp Met Leu Gly Leu Ala Ile Ser Tyr Ile Lys
            180                 185                 190
Asp Leu Gln Glu Gln Val Gln Thr Leu Ser Glu Ser Arg Ser Ser Cys
        195                 200                 205
Thr Cys Ser Ser Gly Lys Gln Lys Gln Asp His His His His His Pro
    210                 215                 220
Ser Ala
225
```

<210> 177
<211> 924
<212> DNA
<213> Zingiber officinale

<400> 177
```
gcaaagctcg ctgatgtcgc agatatcgga gatgggggac gaggaactgg gcggaagcag     60
tcccgacgag agtagccagc gttacgccgc cggagttccg atgacatctt gggccgaagc    120
ctcgttgctc gccggcaaca atccgtcagg accaggaaac agagaggagg aagggaaggt    180
agtggaactg aggaactatg ttgcaggtct cacacaccaa ctcagcttgc caaagcccgc    240
cgcagaaatg gccgccatgg aaaaactcat ccagttccag gacgcatccc cctgccggat    300
cagagccaag cggggttgcg caacgcaccc ccgaagcatc gcagagaggg tgaggaggac    360
caaaatcagc gaaaggatgc ggaaattgca ggaactggtt cccaacatgg acaagcaaac    420
caacacagca gacatgctgg acttggccgt cgagtacatt aaggatctcc agagacaagt    480
cgatgtctta agggaaggcc aagcaagctg cccctgctcg tctagcaagc tgcagaagcc    540
ttaccacaac tcatgattgg gggatattta cttttaagtt tcaagtagtt cttaataagg    600
aatgcggagc agctgtatcc aacgaagaag atggtggatg cagagccacc caccgtcgcc    660
gtcgtcatcg tcgtggtggt caattggtca tcatcagcct caccactcac ggcggtcgat    720
ttgccctcgg gatgccagcg agtttccttc gccagacggc agaagtgaat gggcactgcg    780
gcgacagcga ggagatcgat tgaggcgtga aagtgtcctg tcacatgaga ttagtttgca    840
gaactgtgga ttgccgacaa ctacgtgcag cagcatcagc gatgctgctg ctgatgatga    900
tgatcaggaa tataattcga ttaa                                           924
```

<210> 178

<211> 180
<212> PRT
<213> Zingiber officinale

<400> 178
Met Ser Gln Ile Ser Glu Met Gly Asp Glu Glu Leu Gly Gly Ser Ser
1 5 10 15
Pro Asp Glu Ser Ser Gln Arg Tyr Ala Ala Gly Val Pro Met Thr Ser
20 25 30
Trp Ala Glu Ala Ser Leu Leu Ala Gly Asn Asn Pro Ser Gly Pro Gly
35 40 45
Asn Arg Glu Glu Glu Gly Lys Val Val Glu Leu Arg Asn Tyr Val Ala
50 55 60
Gly Leu Thr His Gln Leu Ser Leu Pro Lys Pro Ala Ala Glu Met Ala
65 70 75 80
Ala Met Glu Lys Leu Ile Gln Phe Gln Asp Ala Ser Pro Cys Arg Ile
85 90 95
Arg Ala Lys Arg Gly Cys Ala Thr His Pro Arg Ser Ile Ala Glu Arg
100 105 110
Val Arg Arg Thr Lys Ile Ser Glu Arg Met Arg Lys Leu Gln Glu Leu
115 120 125
Val Pro Asn Met Asp Lys Gln Thr Asn Thr Ala Asp Met Leu Asp Leu
130 135 140
Ala Val Glu Tyr Ile Lys Asp Leu Gln Arg Gln Val Asp Val Leu Arg
145 150 155 160
Glu Gly Gln Ala Ser Cys Pro Cys Ser Ser Ser Lys Leu Gln Lys Pro
165 170 175
Tyr His Asn Ser
180

<210> 179
<211> 856
<212> DNA
<213> Zingiber officinale

<400> 179
ggcacgaggg aagaaggaag agtagttgat gcaccgaact cctcgaagga acctcaaaaa 60
agaacgtaac tcctccgcgg acaaccacag atgagatccg gcctcgagcg tttccagtcc 120
gttccgagct cgtttcttgg agaaatctgc ggcgagttcc tcgccgtccg tcccgagata 180
gataccatgt tccctcgatt ctctgcccca aatttcccgg aaaacttctc ttctggcggc 240
ggtcactcgc ggccgccgac tttgtcggag atcctcatcc cccatcataa ggttgcattc 300
ccctcttctc cgctgacgat gtttcactcg aatgcagata atggctgcgc tgcgataagt 360
tcgagcagtt tcagaaacaa ggagcttcct cccattagct gcccaccgag gcaaaactcc 420
caaatgtctc agatctctga gactcagatg agcttgccga agaacttcaa acagttccag 480
gatgcagtac catgcagggt ccgagcaaaa cggggtttcg ctactcaccc tcgaagtgtt 540
gctgaaaggg tgaggaggag taaaattagt gaaagaatga aaaagctaca ggaacttgtg 600
cctaacatgg ataagcaaac caacacggcc gctatgctcg atttagcagt catttatatc 660
aaggatcttc agaaacaagt caaggtgcta tcggaggggc aggcgaagtg tacctgctta 720
tctagcgaga agagcaagta cctgaagaat ggagcgggtg aaggctaaag aattggagat 780
gctggtaatt gatgagcaga gcaagaaaca ttgtctgtgg ctgtgtagct cctacccaat 840
tactggaatc agtaaa 856

<210> 180
<211> 225
<212> PRT
<213> Zingiber officinale

<400> 180
Met Arg Ser Gly Leu Glu Arg Phe Gln Ser Val Pro Ser Ser Phe Leu

```
1               5                   10                  15
Gly Glu Ile Cys Gly Glu Phe Leu Ala Val Arg Pro Glu Ile Asp Thr
            20                  25                  30
Met Phe Pro Arg Phe Ser Ala Pro Asn Phe Pro Glu Asn Phe Ser Ser
        35                  40                  45
Gly Gly Gly His Ser Arg Pro Pro Thr Leu Ser Glu Ile Leu Ile Pro
    50                  55                  60
His His Lys Val Ala Phe Pro Ser Ser Pro Leu Thr Met Phe His Ser
65                  70                  75                  80
Asn Ala Asp Asn Gly Cys Ala Ala Ile Ser Ser Ser Ser Phe Arg Asn
                85                  90                  95
Lys Glu Leu Pro Pro Ile Ser Cys Pro Pro Arg Gln Asn Ser Gln Met
            100                 105                 110
Ser Gln Ile Ser Glu Thr Gln Met Ser Leu Pro Lys Asn Phe Lys Gln
        115                 120                 125
Phe Gln Asp Ala Val Pro Cys Arg Val Arg Ala Lys Arg Gly Phe Ala
    130                 135                 140
Thr His Pro Arg Ser Val Ala Glu Arg Val Arg Arg Ser Lys Ile Ser
145                 150                 155                 160
Glu Arg Met Lys Lys Leu Gln Glu Leu Val Pro Asn Met Asp Lys Gln
                165                 170                 175
Thr Asn Thr Ala Ala Met Leu Asp Leu Ala Val Ile Tyr Ile Lys Asp
            180                 185                 190
Leu Gln Lys Gln Val Lys Val Leu Ser Glu Gly Gln Ala Lys Cys Thr
        195                 200                 205
Cys Leu Ser Ser Glu Lys Ser Lys Tyr Leu Lys Asn Gly Ala Gly Glu
    210                 215                 220
Gly
225

<210>  181
<211>  50
<212>  PRT
<213>  Artificial sequence

<220>
<223>  HLH domain of SEQ IS NO: 2

<220>
<221>  UNSURE
<222>  (34)..(34)
<223>  Unknown amino acid

<400>  181
His Pro Arg Ser Ile Ala Glu Arg Val Arg Arg Thr Arg Ile Ser Asp
1               5                   10                  15
Arg Ile Arg Arg Leu Gln Glu Leu Val Pro Asn Met Asp Lys Gln Thr
            20                  25                  30
Asn Xaa Thr Ala Asp Met Leu Glu Glu Ala Val Glu Tyr Val Lys Ala
        35                  40                  45
Leu Gln
    50

<210>  182
<211>  50
<212>  PRT
<213>  Artificial sequence

<220>
```

<223> HLH domain of SEQ IS NO: 4

<220>
<221> UNSURE
<222> (34)..(34)
<223> Unknown amino acid

<400> 182
His Pro Arg Ser Ile Ala Glu Arg Val Arg Arg Thr Arg Ile Ser Asp
1 5 10 15
Arg Ile Arg Lys Leu Gln Glu Leu Val Pro Asn Met Asp Lys Gln Thr
20 25 30
Asn Xaa Thr Ala Asp Met Leu Glu Glu Ala Val Glu Tyr Val Lys Val
35 40 45
Leu Gln
50

<210> 183
<211> 50
<212> PRT
<213> Artificial sequence

<220>
<223> HLH domain of SEQ IS NO: 6

<220>
<221> UNSURE
<222> (34)..(34)
<223> Unknown amino acid

<400> 183
His Pro Arg Ser Ile Ala Glu Arg Val Arg Arg Thr Lys Ile Ser Glu
1 5 10 15
Arg Met Arg Lys Leu Gln Asp Leu Val Pro Asn Met Asp Thr Gln Thr
20 25 30
Asn Xaa Thr Ala Asp Met Leu Asp Leu Ala Val Gln Tyr Ile Lys Asp
35 40 45
Leu Gln
50

<210> 184
<211> 50
<212> PRT
<213> Artificial sequence

<220>
<223> HLH domain of SEQ IS NO: 8

<220>
<221> UNSURE
<222> (34)..(34)
<223> Unknown amino acid

<400> 184
His Pro Arg Ser Ile Ala Glu Arg Glu Arg Arg Thr Arg Ile Ser Gly
1 5 10 15
Lys Leu Lys Lys Leu Gln Asp Leu Val Pro Asn Met Asp Lys Gln Thr
20 25 30
Ser Xaa Tyr Ser Asp Met Leu Asp Leu Ala Val Gln His Ile Lys Gly

```
        35                  40                  45
Leu Gln
    50

<210>  185
<211>  50
<212>  PRT
<213>  Artificial sequence

<220>
<223>  HLH domain of SEQ IS NO: 10

<220>
<221>  UNSURE
<222>  (34)..(34)
<223>  Unknown amino acid

<400>  185
His Pro Arg Ser Ile Ala Glu Arg Glu Arg Arg Thr Arg Ile Ser Gly
1               5                   10                  15
Lys Leu Lys Lys Leu Gln Glu Leu Val Pro Asn Met Asp Lys Gln Thr
            20                  25                  30
Ser Xaa Tyr Ala Asp Met Leu Asp Leu Ala Val Glu His Ile Lys Gly
        35                  40                  45
Leu Gln
    50

<210>  186
<211>  50
<212>  PRT
<213>  Artificial sequence

<220>
<223>   HLH domain of SEQ IS NO: 12

<220>
<221>  UNSURE
<222>  (34)..(34)
<223>  Unknown amino acid

<400>  186
His Pro Arg Ser Ile Ala Glu Arg Val Arg Arg Thr Arg Ile Ser Glu
1               5                   10                  15
Arg Met Arg Lys Leu Gln Glu Leu Val Pro Asn Met Asp Lys Gln Thr
            20                  25                  30
Asn Xaa Thr Ser Asp Met Leu Asp Leu Ala Val Asp Tyr Ile Lys Asp
        35                  40                  45
Leu Gln
    50

<210>  187
<211>  50
<212>  PRT
<213>  Artificial sequence

<220>
<223>  HLH domain of SEQ IS NO: 14

<220>
```

```
<221>  UNSURE
<222>  (34)..(34)
<223>  Unknown amino acid

<400>  187
His Pro Arg Ser Ile Ala Glu Arg Glu Arg Arg Thr Arg Ile Ser Gly
1               5                   10                  15
Lys Leu Lys Lys Leu Gln Glu Leu Val Pro Asn Met Asp Lys Gln Thr
            20                  25                  30
Ser Xaa Tyr Ala Asp Met Leu Asp Leu Ala Val Glu His Ile Lys Gly
        35                  40                  45
Leu Gln
    50

<210>  188
<211>  50
<212>  PRT
<213>  Artificial sequence

<220>
<223>  HLH domain of SEQ IS NO: 20

<220>
<221>  UNSURE
<222>  (34)..(34)
<223>  Unknown amino acid

<400>  188
His Pro Arg Ser Ile Ala Glu Arg Val Arg Arg Thr Arg Ile Ser Asp
1               5                   10                  15
Arg Ile Arg Arg Leu Gln Glu Leu Val Pro Asn Met Asp Lys Gln Thr
            20                  25                  30
Asn Xaa Thr Ala Asp Met Leu Glu Glu Ala Val Glu Tyr Val Lys Ala
        35                  40                  45
Leu Gln
    50

<210>  189
<211>  50
<212>  PRT
<213>  Artificial sequence

<220>
<223>  HLH domain of SEQ IS NO: 22

<220>
<221>  UNSURE
<222>  (34)..(34)
<223>  Unknown amino acid

<400>  189
His Pro Arg Ser Ile Ala Glu Arg Glu Arg Arg Thr Arg Ile Ser Gly
1               5                   10                  15
Arg Leu Lys Lys Leu Gln Asp Leu Val Pro Asn Met Asp Lys Gln Thr
            20                  25                  30
Ser Xaa Tyr Ser Asp Met Leu Asp Leu Ala Val Gln His Ile Lys Gly
        35                  40                  45
Leu Gln
    50
```

```
<210>  190
<211>  50
<212>  PRT
<213>  Artificial sequence

<220>
<223>  HLH domain of SEQ IS NO: 24

<220>
<221>  UNSURE
<222>  (34)..(34)
<223>  Unknown amino acid

<400>  190
His Pro Arg Ser Ile Ala Glu Arg Val Arg Arg Thr Arg Ile Ser Asp
1               5                   10                  15
Arg Ile Arg Lys Leu Gln Asp Leu Val Pro Asn Met Asp Lys Gln Thr
            20                  25                  30
Asn Xaa Thr Ala Asp Met Leu Glu Glu Ala Val Glu Tyr Val Lys Phe
        35                  40                  45
Leu Gln
    50

<210>  191
<211>  50
<212>  PRT
<213>  Artificial sequence

<220>
<223>  HLH domain of SEQ IS NO: 26

<220>
<221>  UNSURE
<222>  (34)..(34)
<223>  Unknown amino acid

<400>  191
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Glu Glu Ala Val Glu Tyr Val Lys Phe
        35                  40                  45
Leu Gln
    50

<210>  192
<211>  50
<212>  PRT
<213>  Artificial sequence

<220>
<223>  HLH domain of SEQ IS NO: 28

<400>  192
His Pro Arg Ser Ile Ala Glu Arg Val Arg Arg Thr Arg Ile Ser Glu
1               5                   10                  15
Arg Met Arg Lys Leu Gln Glu Leu Val Pro Asn Ser Asp Lys Gln Thr
```

```
                20                  25                  30
Thr Asn Ile Ala Asp Met Leu Asp Glu Ala Val Glu Tyr Val Lys Ala
            35                  40                  45
Leu Gln
    50

<210>  193
<211>  50
<212>  PRT
<213>  Artificial sequence

<220>
<223>  HLH domain of SEQ IS NO: 30

<220>
<221>  UNSURE
<222>  (34)..(34)
<223>  Unknown amino acid

<400>  193
His Pro Arg Ser Ile Ala Glu Arg Val Arg Arg Thr Arg Ile Ser Asp
1               5                   10                  15
Arg Ile Arg Lys Leu His Asp Leu Val Pro Asn Met Asp Lys Gln Thr
                20                  25                  30
Asn Xaa Thr Ala Asp Met Leu Glu Glu Ala Val Glu Tyr Val Lys Phe
            35                  40                  45
Leu Gln
    50

<210>  194
<211>  50
<212>  PRT
<213>  Artificial sequence

<220>
<223>  HLH domain of SEQ IS NO: 32

<220>
<221>  UNSURE
<222>  (34)..(34)
<223>  Unknown amino acid

<400>  194
His Pro Arg Ser Ile Ala Glu Arg Val Arg Arg Thr Arg Ile Ser Glu
1               5                   10                  15
Arg Met Arg Lys Leu Gln Asp Leu Phe Pro Asn Met Asp Lys Gln Thr
                20                  25                  30
Asn Xaa Thr Ala Glu Met Leu Asp Leu Ala Val Glu His Ile Lys Asp
            35                  40                  45
Leu Gln
    50

<210>  195
<211>  50
<212>  PRT
<213>  Artificial sequence

<220>
<223>  HLH domain of SEQ IS NO: 34
```

```
<220>
<221>  UNSURE
<222>  (34)..(34)
<223>  Unknown amino acid

<400>  195
His Pro Arg Asn Ile Ala Glu Arg Val Arg Arg Thr Arg Ile Ser Asp
1               5                   10                  15
Arg Met Lys Lys Leu Pro Glu Leu Phe Pro Thr Met Asp Lys Arg Thr
            20                  25                  30
Ser Xaa Thr Ala Asp Met Leu Asp Met Ala Val Gln Tyr Ile Thr Asp
        35                  40                  45
Leu Gln
    50

<210>  196
<211>  50
<212>  PRT
<213>  Artificial sequence

<220>
<223>  HLH domain of SEQ IS NO: 36

<220>
<221>  UNSURE
<222>  (34)..(34)
<223>  Unknown amino acid

<400>  196
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Ser
            20                  25                  30
Asn Xaa Thr Ala Asp Met Leu Glu Glu Ala Val Asp Tyr Val Lys Phe
        35                  40                  45
Leu Gln
    50

<210>  197
<211>  50
<212>  PRT
<213>  Artificial sequence

<220>
<223>  HLH domain of SEQ IS NO: 38

<220>
<221>  UNSURE
<222>  (34)..(34)
<223>  Unknown amino acid

<400>  197
His Pro Arg Ser Ile Ala Glu Arg Val Arg Arg Thr Arg Ile Ser Glu
1               5                   10                  15
Arg Met Arg Lys Leu Gln Asp Leu Val Pro Asn Met Asp Lys Gln Thr
            20                  25                  30
Asn Xaa Thr Ser Asp Met Leu Asp Leu Ala Val Asp Tyr Ile Lys Asp
        35                  40                  45
```

```
Leu Gln
    50

<210>  198
<211>  50
<212>  PRT
<213>  Artificial sequence

<220>
<223>  HLH domain of SEQ IS NO: 40

<220>
<221>  UNSURE
<222>  (34)..(34)
<223>  Unknown amino acid

<400>  198
His Pro Arg Ser Ile Ala Glu Arg Glu Arg Arg Thr Arg Ile Ser Gly
1               5                   10                  15
Lys Leu Lys Lys Leu Gln Glu Leu Val Pro Asn Met Asp Lys Gln Thr
            20                  25                  30
Ser Xaa Tyr Ala Asp Met Leu Asp Leu Ala Val Gln His Ile Lys Gly
        35                  40                  45
Leu Gln
    50

<210>  199
<211>  50
<212>  PRT
<213>  Artificial sequence

<220>
<223>  HLH domain of SEQ IS NO: 42

<220>
<221>  UNSURE
<222>  (34)..(34)
<223>  Unknown amino acid

<400>  199
His Pro Arg Ser Ile Ala Glu Arg Glu Arg Arg Thr Arg Ile Ser Glu
1               5                   10                  15
Arg Ile Lys Lys Leu Gln Glu Leu Phe Pro Asn Met Asp Lys Gln Met
            20                  25                  30
Asn Xaa Ile Ala Asp Met Leu Asp Met Ala Leu Asp Tyr Ile Lys Asp
        35                  40                  45
Leu Gln
    50

<210>  200
<211>  50
<212>  PRT
<213>  Artificial sequence

<220>
<223>  HLH domain of SEQ IS NO: 44

<220>
<221>  UNSURE
```

```
<222>  (34)..(34)
<223>  Unknown amino acid

<400>  200
His Pro Arg Ser Ile Ala Glu Arg Val Arg Arg Thr Lys Ile Ser Glu
1               5                   10                  15
Arg Met Arg Lys Leu Gln Asp Leu Val Pro Asn Met Asp Lys Gln Thr
            20                  25                  30
Asn Xaa Thr Ala Asp Met Leu Asp Leu Ala Val Glu Tyr Ile Lys Asp
        35                  40                  45
Leu Gln
    50

<210>  201
<211>  50
<212>  PRT
<213>  Artificial sequence

<220>
<223>  HLH domain of SEQ IS NO: 46

<220>
<221>  UNSURE
<222>  (34)..(34)
<223>  Unknown amino acid

<400>  201
His Pro Arg Ser Ile Ala Glu Arg Glu Arg Arg Thr Arg Ile Ser Gly
1               5                   10                  15
Lys Leu Lys Lys Leu Gln Asp Leu Val Pro Asn Met Asp Lys Gln Thr
            20                  25                  30
Ser Xaa Tyr Ala Asp Met Leu Asp Leu Ala Val Gln His Ile Lys Gly
        35                  40                  45
Leu Gln
    50

<210>  202
<211>  50
<212>  PRT
<213>  Artificial sequence

<220>
<223>  HLH domain of SEQ IS NO: 48

<220>
<221>  UNSURE
<222>  (34)..(34)
<223>  Unknown amino acid

<400>  202
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Asp Glu Ala Val Ala Tyr Val Lys Phe
        35                  40                  45
Leu Gln
    50
```

```
<210>  203
<211>  50
<212>  PRT
<213>  Artificial sequence

<220>
<223>  HLH domain of SEQ IS NO: 50

<220>
<221>  UNSURE
<222>  (34)..(34)
<223>  Unknown amino acid

<400>  203
His Pro Arg Ser Ile Ala Glu Arg Val Arg Arg Thr Arg Ile Ser Glu
1               5                   10                  15
Arg Ile Lys Lys Leu Gln Asp Leu Phe Pro Lys Ser Glu Lys Gln Thr
            20                  25                  30
Ser Xaa Thr Ala Asp Met Leu Asp Leu Ala Val Glu Tyr Ile Lys Asp
        35                  40                  45
Leu Gln
    50

<210>  204
<211>  50
<212>  PRT
<213>  Artificial sequence

<220>
<223>  HLH domain of SEQ IS NO: 52

<220>
<221>  UNSURE
<222>  (34)..(34)
<223>  Unknown amino acid

<400>  204
His Pro Arg Ser Ile Ala Glu Arg Glu Arg Arg Thr Arg Ile Ser Ala
1               5                   10                  15
Arg Ile Lys Lys Leu Gln Asp Leu Phe Pro Lys Ser Asp Lys Gln Thr
            20                  25                  30
Ser Xaa Thr Ala Asp Met Leu Asp Leu Ala Val Glu Tyr Ile Lys Asp
        35                  40                  45
Leu Gln
    50

<210>  205
<211>  50
<212>  PRT
<213>  Artificial sequence

<220>
<223>  HLH domain of SEQ IS NO: 54

<220>
<221>  UNSURE
<222>  (34)..(34)
<223>  Unknown amino acid
```

```
<400>  205
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Pro Ala Asp Met Leu Glu Glu Ala Val Glu Tyr Val Lys Tyr
        35                  40                  45
Leu Gln
    50

<210>  206
<211>  50
<212>  PRT
<213>  Artificial sequence

<220>
<223>  HLH domain of SEQ IS NO: 56

<220>
<221>  UNSURE
<222>  (34)..(34)
<223>  Unknown amino acid

<400>  206
His Pro Arg Ser Ile Ala Glu Arg Val Arg Arg Thr Arg Ile Ser Glu
1               5                   10                  15
Arg Ile Arg Lys Leu Gln Glu Leu Val Pro Thr Met Asp Lys Gln Thr
            20                  25                  30
Ser Xaa Thr Ala Glu Met Leu Asp Leu Ala Leu Asp Tyr Ile Lys Asp
        35                  40                  45
Leu Gln
    50

<210>  207
<211>  50
<212>  PRT
<213>  Artificial sequence

<220>
<223>  HLH domain of SEQ IS NO: 58

<220>
<221>  UNSURE
<222>  (34)..(34)
<223>  Unknown amino acid

<400>  207
His Pro Arg Ser Ile Ala Glu Arg Val Arg Arg Thr Arg Ile Ser Glu
1               5                   10                  15
Arg Met Arg Lys Leu Gln Asp Leu Val Pro Asn Met Asp Lys Gln Thr
            20                  25                  30
Ser Xaa Thr Ala Asp Met Leu Asp Leu Ala Val Glu Tyr Ile Lys Glu
        35                  40                  45
Leu Gln
    50

<210>  208
<211>  50
<212>  PRT
```

```
<213>  Artificial sequence

<220>
<223>  HLH domain of SEQ IS NO: 60

<220>
<221>  UNSURE
<222>  (34)..(34)
<223>  Unknown amino acid

<400>  208
His Pro Pro Ser Ile Ala Glu Arg Val Arg Arg Thr Arg Ile Ser Asp
1               5                   10                  15
Arg Met Lys Lys Leu Gln Glu Leu Phe Pro Asn Met Asp Lys Gln Thr
            20                  25                  30
Ser Xaa Thr Ala Asp Met Leu Asp Met Ala Val Gln Tyr Ile Lys Asp
        35                  40                  45
Leu Gln
    50

<210>  209
<211>  50
<212>  PRT
<213>  Artificial sequence

<220>
<223>  HLH domain of SEQ IS NO: 62

<220>
<221>  UNSURE
<222>  (34)..(34)
<223>  Unknown amino acid

<400>  209
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Asp Glu Ala Val Glu Tyr Val Lys Phe
        35                  40                  45
Leu Gln
    50

<210>  210
<211>  50
<212>  PRT
<213>  Artificial sequence

<220>
<223>  HLH domain of SEQ IS NO: 64

<220>
<221>  UNSURE
<222>  (34)..(34)
<223>  Unknown amino acid

<400>  210
His Pro Arg Ser Ile Ala Glu Arg Val Arg Arg Thr Lys Ile Ser Glu
1               5                   10                  15
```

```
Arg Ile Arg Lys Leu Gln Glu Leu Val Pro Asn Met Glu Lys Gln Thr
            20                  25                  30
Asn Xaa Thr Ser Asp Met Leu Asp Leu Ala Val Asp Tyr Ile Lys Glu
        35                  40                  45
Leu Gln
    50

<210>  211
<211>  50
<212>  PRT
<213>  Artificial sequence

<220>
<223>  HLH domain of SEQ IS NO: 66

<220>
<221>  UNSURE
<222>  (34)..(34)
<223>  Unknown amino acid

<400>  211
His Pro Arg Ser Ile Ala Glu Arg Glu Arg Arg Thr Arg Ile Ser Glu
1               5                   10                  15
Lys Leu Arg Lys Leu Gln Asp Leu Val Pro Asn Met Asp Lys Gln Thr
            20                  25                  30
Ser Xaa Thr Ser Asp Met Leu Asp Leu Ala Val Glu His Ile Arg Gly
        35                  40                  45
Leu Gln
    50

<210>  212
<211>  50
<212>  PRT
<213>  Artificial sequence

<220>
<223>  HLH domain of SEQ IS NO: 68

<220>
<221>  UNSURE
<222>  (34)..(34)
<223>  Unknown amino acid

<400>  212
His Pro Arg Ser Ile Ala Glu Arg Val Arg Arg Thr Lys Ile Ser Glu
1               5                   10                  15
Arg Met Arg Lys Leu Gln Glu Leu Val Pro Asn Met Asp Lys Gln Thr
            20                  25                  30
Asn Xaa Thr Ala Asp Met Leu Asp Leu Ala Val Asp Tyr Ile Lys Asp
        35                  40                  45
Leu Glu
    50

<210>  213
<211>  50
<212>  PRT
<213>  Artificial sequence

<220>
```

<223> HLH domain of SEQ IS NO: 70

<220>
<221> UNSURE
<222> (34)..(34)
<223> Unknown amino acid

<400> 213
His Pro Arg Ser Ile Ala Glu Arg Asn Arg Arg Thr Arg Ile Ser Glu
1 5 10 15
Arg Met Lys Lys Leu Gln Glu Leu Phe Pro Lys Met Asp Lys Gln Thr
20 25 30
Ser Xaa Thr Ala Asp Met Leu Asp Trp Ala Val Asp His Ile Lys Glu
35 40 45
Leu Gln
50

<210> 214
<211> 50
<212> PRT
<213> Artificial sequence

<220>
<223> HLH domain of SEQ IS NO: 72

<220>
<221> UNSURE
<222> (34)..(34)
<223> Unknown amino acid

<400> 214
His Pro Arg Ser Ile Ala Glu Arg Val Arg Arg Thr Arg Ile Ser Asp
1 5 10 15
Arg Ile Arg Lys Leu Gln Glu Leu Val Pro Asn Met Asp Lys Gln Thr
20 25 30
Asn Xaa Thr Ala Asp Met Leu Glu Glu Ala Val Glu Tyr Val Lys Phe
35 40 45
Leu Gln
50

<210> 215
<211> 50
<212> PRT
<213> Artificial sequence

<220>
<223> HLH domain of SEQ IS NO: 74

<220>
<221> UNSURE
<222> (34)..(34)
<223> Unknown amino acid

<400> 215
His Pro Arg Ser Ile Ala Glu Arg Val Arg Arg Thr Arg Ile Ser Asp
1 5 10 15
Arg Met Arg Lys Leu Gln Glu Leu Val Pro Asn Met Asp Lys Gln Thr
20 25 30
Asn Xaa Thr Ala Asp Met Leu Asp Leu Ala Val Asp Tyr Ile Lys Asp

```
        35                  40                  45
Leu Glu
    50

<210>  216
<211>  50
<212>  PRT
<213>  Artificial sequence

<220>
<223>  HLH domain of SEQ IS NO: 76

<220>
<221>  UNSURE
<222>  (34)..(34)
<223>  Unknown amino acid

<400>  216
His Pro Arg Ser Ile Ala Glu Arg Glu Arg Arg Thr Arg Ile Ser Gly
1               5                   10                  15
Lys Leu Lys Lys Leu Gln Asp Leu Val Pro Asn Met Asp Lys Gln Thr
            20                  25                  30
Ser Xaa Tyr Ser Asp Met Leu Asp Leu Ala Val Gln His Ile Lys Ser
        35                  40                  45
Leu Gln
    50

<210>  217
<211>  50
<212>  PRT
<213>  Artificial sequence

<220>
<223>  HLH domain of SEQ IS NO: 78

<220>
<221>  UNSURE
<222>  (34)..(34)
<223>  Unknown amino acid

<400>  217
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Glu Glu Ala Val Glu Tyr Val Lys Phe
        35                  40                  45
Leu Gln
    50

<210>  218
<211>  50
<212>  PRT
<213>  Artificial sequence

<220>
<223>  HLH domain of SEQ IS NO: 80

<220>
```

<221>  UNSURE
<222>  (34)..(34)
<223>  Unknown amino acid

<400>  218
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Glu Glu Ala Val Glu Tyr Val Lys Phe
        35                  40                  45
Leu Gln
    50

<210>  219
<211>  50
<212>  PRT
<213>  Artificial sequence

<220>
<223>  HLH domain of SEQ IS NO: 82

<220>
<221>  UNSURE
<222>  (34)..(34)
<223>  Unknown amino acid

<400>  219
His Pro Arg Ser Leu Ala Glu Arg Val Arg Arg Thr Arg Ile Ser Glu
1               5                   10                  15
Arg Met Arg Lys Leu Gln Glu Ile Val Pro Asn Ile Asp Lys Gln Thr
            20                  25                  30
Cys Xaa Thr Ser Glu Met Leu Asp Leu Ala Val Glu Tyr Ile Lys Asp
        35                  40                  45
Leu Gln
    50

<210>  220
<211>  50
<212>  PRT
<213>  Artificial sequence

<220>
<223>  HLH domain of SEQ IS NO: 84

<220>
<221>  UNSURE
<222>  (34)..(34)
<223>  Unknown amino acid

<400>  220
His Pro Arg Ser Ile Ala Glu Arg Glu Arg Arg Thr Arg Ile Ser Gly
1               5                   10                  15
Lys Leu Lys Lys Leu Gln Asp Leu Val Pro Asn Met Asp Lys Gln Thr
            20                  25                  30
Ser Xaa Tyr Ser Asp Met Leu Asp Leu Ala Val Gln His Ile Lys Gly
        35                  40                  45
Leu Gln
    50

<210> 221
<211> 50
<212> PRT
<213> Artificial sequence

<220>
<223> HLH domain of SEQ IS NO: 86

<220>
<221> UNSURE
<222> (34)..(34)
<223> Unknown amino acid

<400> 221
```
His Pro Arg Ser Ile Ala Glu Arg Val Arg Arg Thr Lys Ile Ser Glu
1               5                   10                  15
Arg Met Arg Lys Leu Gln Asp Leu Val Pro Asn Met Asp Lys Gln Thr
            20                  25                  30
Asn Xaa Thr Ser Asp Met Leu Asp Leu Ala Val Glu Tyr Ile Lys Asp
        35                  40                  45
Leu Gln
    50
```

<210> 222
<211> 50
<212> PRT
<213> Artificial sequence

<220>
<223> HLH domain of SEQ IS NO: 88

<220>
<221> UNSURE
<222> (34)..(34)
<223> Unknown amino acid

<400> 222
```
His Pro Arg Ser Ile Ala Glu Arg Glu Arg Arg Thr Arg Ile Ser Gly
1               5                   10                  15
Lys Leu Lys Lys Leu Gln Asp Leu Val Pro Asn Met Asp Lys Gln Thr
            20                  25                  30
Ser Xaa Tyr Ala Asp Met Leu Asp Leu Ala Val Gln His Ile Arg Thr
        35                  40                  45
Leu Gln
    50
```

<210> 223
<211> 50
<212> PRT
<213> Artificial sequence

<220>
<223> HLH domain of SEQ IS NO: 90

<220>
<221> UNSURE
<222> (34)..(34)
<223> Unknown amino acid

<400> 223
His Pro Arg Ser Ile Ala Glu Arg Glu Arg Arg Thr Arg Ile Ser Gly
1 5 10 15
Lys Leu Lys Lys Leu Gln Asp Leu Val Pro Asn Met Asp Lys Gln Thr
20 25 30
Ser Xaa Tyr Ala Asp Met Leu Asp Leu Ala Val Gln His Ile Arg Thr
35 40 45
Leu Gln
50

<210> 224
<211> 50
<212> PRT
<213> Artificial sequence

<220>
<223> HLH domain of SEQ IS NO: 92

<220>
<221> UNSURE
<222> (34)..(34)
<223> Unknown amino acid

<400> 224
His Pro Arg Ser Ile Ala Glu Arg Glu Arg Arg Thr Arg Ile Ser Glu
1 5 10 15
Lys Leu Arg Lys Leu Gln Glu Leu Val Pro Asn Met Asp Lys Gln Thr
20 25 30
Ser Xaa Thr Ala Asp Met Leu Asp Leu Ala Val Glu His Ile Lys Gly
35 40 45
Leu Gln
50

<210> 225
<211> 50
<212> PRT
<213> Artificial sequence

<220>
<223> HLH domain of SEQ IS NO: 94

<220>
<221> UNSURE
<222> (34)..(34)
<223> Unknown amino acid

<400> 225
His Pro Arg Ser Ile Ala Glu Arg Glu Arg Arg Thr Arg Ile Ser Lys
1 5 10 15
Arg Leu Lys Lys Leu Gln Asp Leu Val Pro Asn Met Asp Lys Gln Thr
20 25 30
Asn Xaa Thr Ser Asp Met Leu Asp Ile Ala Val Thr Tyr Ile Lys Glu
35 40 45
Leu Gln
50

<210> 226
<211> 50

```
<212>  PRT
<213>  Artificial sequence

<220>
<223>  HLH domain of SEQ IS NO: 96

<220>
<221>  UNSURE
<222>  (34)..(34)
<223>  Unknown amino acid

<400>  226
His Pro Arg Ser Ile Ala Glu Arg Glu Arg Arg Thr Arg Ile Ser Glu
1               5                   10                  15
Lys Leu Arg Lys Leu Gln Ala Leu Val Pro Asn Met Asp Lys Gln Thr
            20                  25                  30
Ser Xaa Thr Ser Asp Met Leu Asp Leu Ala Val Asp His Ile Lys Gly
        35                  40                  45
Leu Gln
    50

<210>  227
<211>  50
<212>  PRT
<213>  Artificial sequence

<220>
<223>  HLH domain of SEQ IS NO: 98

<220>
<221>  UNSURE
<222>  (34)..(34)
<223>  Unknown amino acid

<400>  227
His Pro Arg Ser Ile Ala Glu Arg Val Arg Arg Thr Arg Ile Ser Glu
1               5                   10                  15
Arg Ile Arg Lys Leu Gln Glu Leu Val Pro Asn Met Asp Lys Gln Thr
            20                  25                  30
Asn Xaa Thr Ala Asp Met Leu Asp Leu Ala Val Asp Tyr Ile Lys Asp
        35                  40                  45
Leu Gln
    50

<210>  228
<211>  50
<212>  PRT
<213>  Artificial sequence

<220>
<223>  HLH domain of SEQ IS NO: 100

<220>
<221>  UNSURE
<222>  (34)..(34)
<223>  Unknown amino acid

<400>  228
His Pro Arg Ser Ile Ala Glu Arg Val Arg Arg Thr Arg Ile Ser Glu
```

```
1               5                   10                  15
Arg Ile Arg Lys Leu Gln Glu Leu Val Pro Asn Met Glu Lys Gln Thr
            20                  25                  30
Asn Xaa Thr Ala Asp Met Leu Asp Leu Ala Val Asp Tyr Ile Lys Glu
        35                  40                  45
Leu Gln
    50

<210>  229
<211>  50
<212>  PRT
<213>  Artificial sequence

<220>
<223>  HLH domain of SEQ IS NO: 102

<220>
<221>  UNSURE
<222>  (34)..(34)
<223>  Unknown amino acid

<400>  229
His Pro Arg Ser Ile Ala Glu Arg Asn Arg Arg Ser Arg Ile Ser Glu
1               5                   10                  15
Arg Met Lys Lys Leu Gln Asp Leu Val Pro Asn Met Asp Lys Gln Thr
            20                  25                  30
Asn Xaa Thr Ala Asp Met Leu Asp Glu Ala Val Glu Tyr Val Lys His
        35                  40                  45
Leu Gln
    50

<210>  230
<211>  50
<212>  PRT
<213>  Artificial sequence

<220>
<223>  HLH domain of SEQ IS NO: 104

<220>
<221>  UNSURE
<222>  (34)..(34)
<223>  Unknown amino acid

<400>  230
His Pro Arg Ser Ile Ala Glu Arg Val Arg Arg Gly Lys Ile Ser Glu
1               5                   10                  15
Arg Met Lys Lys Leu Gln Asp Leu Val Pro Ser Met Asp Arg Gln Thr
            20                  25                  30
Asn Xaa Thr Ala Asp Met Leu Asp Asp Ala Val Glu Tyr Val Lys Gln
        35                  40                  45
Leu Gln
    50

<210>  231
<211>  50
<212>  PRT
<213>  Artificial sequence
```

<220>
<223> HLH domain of SEQ IS NO: 106

<220>
<221> UNSURE
<222> (34)..(34)
<223> Unknown amino acid

<400> 231
His Pro Arg Ser Ile Ala Glu Arg Val Arg Arg Thr Lys Ile Ser Glu
1 5 10 15
Arg Met Lys Lys Leu Gln Asp Leu Val Pro Ser Met Asp Lys Gln Thr
20 25 30
Asn Xaa Thr Ser Asp Met Leu Asp Glu Thr Val Glu Tyr Val Lys Ser
35 40 45
Leu Gln
50

<210> 232
<211> 50
<212> PRT
<213> Artificial sequence

<220>
<223> HLH domain of SEQ IS NO: 108

<220>
<221> UNSURE
<222> (34)..(34)
<223> Unknown amino acid

<400> 232
His Pro Arg Ser Ile Ala Glu Arg Val Arg Arg Thr Arg Ile Ser Glu
1 5 10 15
Arg Met Lys Lys Leu Gln Asp Leu Val Pro Asn Met Glu Lys Thr Thr
20 25 30
Asn Xaa Thr Ala Asp Met Leu Asp Glu Thr Val Glu Tyr Val Lys Ser
35 40 45
Leu Gln
50

<210> 233
<211> 50
<212> PRT
<213> Artificial sequence

<220>
<223> HLH domain of SEQ IS NO: 110

<220>
<221> UNSURE
<222> (34)..(34)
<223> Unknown amino acid

<400> 233
His Pro Arg Ser Ile Ala Glu Arg Val Arg Arg Thr Arg Ile Ser Glu
1 5 10 15
Lys Met Arg Asn Leu Gln Gln Leu Val Pro Asn Met Asp Lys Gln Thr
20 25 30

```
Asn Xaa Thr Ala Asp Met Leu Asp Glu Ala Val Asp Tyr Ile Lys Phe
        35                  40                  45
Leu Gln
    50

<210>  234
<211>  50
<212>  PRT
<213>  Artificial sequence

<220>
<223>  HLH domain of SEQ IS NO: 112

<220>
<221>  UNSURE
<222>  (34)..(34)
<223>  Unknown amino acid

<400>  234
His Pro Arg Ser Ile Ala Glu Arg Val Arg Arg Thr Arg Ile Ser Glu
1               5                   10                  15
Arg Met Arg Lys Leu Gln Glu Leu Phe Pro Asn Met Asp Lys Gln Thr
            20                  25                  30
Asn Xaa Thr Ala Asp Met Leu Asp Leu Ala Val Glu His Ile Lys Asp
        35                  40                  45
Leu Gln
    50

<210>  235
<211>  50
<212>  PRT
<213>  Artificial sequence

<220>
<223>  HLH domain of SEQ IS NO: 114

<220>
<221>  UNSURE
<222>  (34)..(34)
<223>  Unknown amino acid

<400>  235
His Pro Arg Ser Ile Ala Glu Arg Val Arg Arg Thr Arg Ile Ser Glu
1               5                   10                  15
Arg Met Arg Lys Leu Gln Glu Leu Phe Pro Asp Met Asp Lys Gln Thr
            20                  25                  30
Ser Xaa Thr Ala Asp Lys Leu Asp Leu Ser Ile Glu Leu Ile Lys Asp
        35                  40                  45
Leu Gln
    50

<210>  236
<211>  50
<212>  PRT
<213>  Artificial sequence

<220>
<223>  HLH domain of SEQ IS NO: 116
```

<220>
<221> UNSURE
<222> (34)..(34)
<223> Unknown amino acid

<400> 236
His Pro Arg Ser Ile Ala Glu Arg Val Arg Arg Thr Arg Ile Ser Glu
1 5 10 15
Arg Met Arg Lys Leu Gln Glu Leu Phe Pro Asp Met Asp Lys Gln Thr
20 25 30
Ser Xaa Thr Ala Asp Lys Leu Asp Leu Ser Ile Glu Leu Ile Lys Asp
35 40 45
Leu Gln
50

<210> 237
<211> 50
<212> PRT
<213> Artificial sequence

<220>
<223> HLH domain of SEQ IS NO: 118

<220>
<221> UNSURE
<222> (34)..(34)
<223> Unknown amino acid

<400> 237
His Pro Arg Ser Ile Ala Glu Arg Val Arg Arg Thr Arg Ile Ser Glu
1 5 10 15
Arg Met Arg Lys Leu Gln Asp Leu Val Pro Asn Met Asp Lys Gln Thr
20 25 30
Asn Xaa Thr Ser Asp Met Leu Asp Leu Ala Val Asp Tyr Ile Lys Asp
35 40 45
Leu Gln
50

<210> 238
<211> 50
<212> PRT
<213> Artificial sequence

<220>
<223> HLH domain of SEQ IS NO: 120

<220>
<221> UNSURE
<222> (34)..(34)
<223> Unknown amino acid

<400> 238
His Pro Arg Ser Ile Ala Glu Arg Val Arg Arg Thr Arg Ile Ser Glu
1 5 10 15
Arg Met Arg Lys Leu Gln Glu Leu Phe Pro Asn Met Asp Lys Gln Thr
20 25 30
Asn Xaa Thr Ala Asp Met Leu Asp Leu Ala Val Glu His Ile Lys Asp
35 40 45
Leu Gln

```
    50

<210>  239
<211>  50
<212>  PRT
<213>  Artificial sequence

<220>
<223>  HLH domain of SEQ IS NO: 122

<220>
<221>  UNSURE
<222>  (34)..(34)
<223>  Unknown amino acid

<400>  239
His Pro Arg Ser Ile Ala Glu Arg Glu Arg Arg Thr Arg Ile Ser Gly
1               5                   10                  15
Lys Leu Lys Thr Leu Gln Asp Leu Val Pro Asn Met Asp Lys Gln Thr
            20                  25                  30
Ser Xaa Tyr Ala Asp Met Leu Glu Leu Ala Val Lys His Ile Lys Gly
        35                  40                  45
Leu Gln
    50

<210>  240
<211>  50
<212>  PRT
<213>  Artificial sequence

<220>
<223>  HLH domain of SEQ IS NO: 124

<220>
<221>  UNSURE
<222>  (34)..(34)
<223>  Unknown amino acid

<400>  240
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Asp Glu Ala Leu Ala Tyr Val Lys Phe
        35                  40                  45
Leu Gln
    50

<210>  241
<211>  50
<212>  PRT
<213>  Artificial sequence

<220>
<223>  HLH domain of SEQ IS NO: 126

<220>
<221>  UNSURE
<222>  (34)..(34)
```

<223> Unknown amino acid

<400> 241
His Pro Arg Ser Ile Ala Glu Arg Val Arg Arg Thr Arg Ile Ser Glu
1 5 10 15
Arg Met Arg Lys Leu Gln Glu Leu Val Pro Asn Met Asp Lys Gln Thr
20 25 30
Asn Xaa Thr Ala Asp Met Leu Asp Leu Ala Val Asp Tyr Ile Lys Asp
35 40 45
Leu Gln
50

<210> 242
<211> 50
<212> PRT
<213> Artificial sequence

<220>
<223> HLH domain of SEQ IS NO: 128

<220>
<221> UNSURE
<222> (34)..(34)
<223> Unknown amino acid

<400> 242
His Pro Arg Ser Ile Ala Glu Arg Val Arg Arg Thr Arg Ile Ser Glu
1 5 10 15
Arg Met Arg Lys Leu Gln Asp Leu Val Pro Asn Met Asp Lys Gln Thr
20 25 30
Asn Xaa Thr Ser Asp Met Leu Asp Leu Ala Val Asp Tyr Ile Lys Asp
35 40 45
Leu Gln
50

<210> 243
<211> 50
<212> PRT
<213> Artificial sequence

<220>
<223> HLH domain of SEQ IS NO: 130

<220>
<221> UNSURE
<222> (34)..(34)
<223> Unknown amino acid

<400> 243
His Pro Arg Ser Ile Ala Glu Arg Glu Arg Arg Thr Arg Ile Ser Glu
1 5 10 15
Lys Leu Arg Lys Pro Gln Asp Leu Val Pro Asn Met Asp Lys Gln Thr
20 25 30
Ser Xaa Thr Ala Asp Met Leu Asp Leu Ala Val Glu His Ile Lys Gly
35 40 45
Leu Gln
50

<210> 244

<211>  50
<212>  PRT
<213>  Artificial sequence

<220>
<223>  HLH domain of SEQ IS NO: 132

<220>
<221>  UNSURE
<222>  (34)..(34)
<223>  Unknown amino acid

<400>  244

```
His Pro Arg Ser Ile Ala Glu Arg Met Arg Arg Thr Arg Ile Ser Glu
1               5                   10                  15
Arg Met Lys Lys Leu Gln Asp Leu Phe Pro Asn Met Asp Lys Gln Thr
            20                  25                  30
Asn Xaa Thr Ala Asp Met Leu Asp Leu Ala Val Asp Tyr Ile Lys Asp
        35                  40                  45
Leu Gln
    50
```

<210>  245
<211>  50
<212>  PRT
<213>  Artificial sequence

<220>
<223>  HLH domain of SEQ IS NO: 134

<220>
<221>  UNSURE
<222>  (34)..(34)
<223>  Unknown amino acid

<400>  245

```
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Glu Glu Ala Val Glu Tyr Val Lys Phe
        35                  40                  45
Leu Gln
    50
```

<210>  246
<211>  50
<212>  PRT
<213>  Artificial sequence

<220>
<223>  HLH domain of SEQ IS NO: 136

<220>
<221>  UNSURE
<222>  (34)..(34)
<223>  Unknown amino acid

<400>  246

```
His Pro Arg Ser Ile Pro Glu Arg Glu Arg Arg Thr Arg Ile Ser Glu
1               5                   10                  15
Lys Leu Lys Lys Leu Gln Ala Leu Val Pro Asn Met Asp Lys Gln Thr
            20                  25                  30
Ser Xaa Pro Ala Asp Met Leu Asp Leu Ala Val Asp His Ile Arg Gly
        35                  40                  45
Leu Gln
    50

<210>  247
<211>  50
<212>  PRT
<213>  Artificial sequence

<220>
<223>  HLH domain of SEQ IS NO: 138

<220>
<221>  UNSURE
<222>  (34)..(34)
<223>  Unknown amino acid

<400>  247
His Pro Arg Ser Ile Ala Glu Arg Glu Arg Arg Thr Arg Ile Ser Glu
1               5                   10                  15
Lys Leu Arg Lys Leu Gln Ala Leu Val Pro Asn Met Asp Lys Gln Thr
            20                  25                  30
Ser Xaa Thr Ala Asp Met Leu Asp Leu Ala Val Asp His Ile Arg Gly
        35                  40                  45
Leu Gln
    50

<210>  248
<211>  50
<212>  PRT
<213>  Artificial sequence

<220>
<223>  HLH domain of SEQ IS NO: 140

<220>
<221>  UNSURE
<222>  (34)..(34)
<223>  Unknown amino acid

<400>  248
His Pro Arg Ser Ile Ala Glu Arg Val Arg Arg Thr Arg Ile Ser Glu
1               5                   10                  15
Arg Met Arg Lys Leu Gln Glu Leu Val Pro Asn Met Asp Lys Gln Thr
            20                  25                  30
Asp Xaa Pro Ala Asp Met Leu Asp Phe Ala Ala Asp Tyr Ile Lys Glu
        35                  40                  45
Leu Glu
    50

<210>  249
<211>  50
<212>  PRT
<213>  Artificial sequence
```

```
<220>
<223>  HLH domain of SEQ IS NO: 142

<220>
<221>  UNSURE
<222>  (34)..(34)
<223>  Unknown amino acid

<400>  249
His Pro Arg Ser Ile Ala Glu Arg Glu Arg Arg Thr Arg Ile Ser Gly
1               5                   10                  15
Lys Leu Lys Lys Leu Gln Asp Leu Val Pro Asn Met Asp Lys Gln Thr
            20                  25                  30
Ser Xaa Tyr Ala Asp Met Leu Asp Leu Ala Val Gln His Ile Arg Thr
        35                  40                  45
Leu Gln
    50

<210>  250
<211>  50
<212>  PRT
<213>  Artificial sequence

<220>
<223>  HLH domain of SEQ IS NO: 144

<220>
<221>  UNSURE
<222>  (34)..(34)
<223>  Unknown amino acid

<400>  250
His Pro Arg Ser Ile Ala Glu Arg Met Arg Arg Thr Arg Ile Ser Glu
1               5                   10                  15
Arg Met Lys Lys Leu Gln Asp Leu Phe Pro Asn Met Asp Lys Gln Thr
            20                  25                  30
Asn Xaa Thr Ala Asp Met Leu Asp Leu Ala Val Asp Tyr Ile Lys Asp
        35                  40                  45
Leu Gln
    50

<210>  251
<211>  50
<212>  PRT
<213>  Artificial sequence

<220>
<223>  HLH domain of SEQ IS NO: 146

<220>
<221>  UNSURE
<222>  (34)..(34)
<223>  Unknown amino acid

<400>  251
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
```

```
                20                  25                  30
Asn Xaa Thr Ala Asp Met Leu Glu Glu Ala Val Glu Tyr Val Lys Phe
        35                  40                  45
Leu Gln
    50
```

<210> 252
<211> 50
<212> PRT
<213> Artificial sequence

<220>
<223> HLH domain of SEQ IS NO: 148

<220>
<221> UNSURE
<222> (34)..(34)
<223> Unknown amino acid

<400> 252

```
His Pro Arg Ser Ile Ala Glu Arg Val Arg Arg Thr Arg Ile Ser Asp
1               5                   10                  15
Arg Met Arg Lys Leu Gln Glu Leu Val Pro Asn Met Asp Lys Gln Thr
            20                  25                  30
Asn Xaa Thr Ala Asp Met Leu Asp Phe Ala Val Asp Tyr Ile Lys Glu
        35                  40                  45
Leu Glu
    50
```

<210> 253
<211> 50
<212> PRT
<213> Artificial sequence

<220>
<223> HLH domain of SEQ IS NO: 150

<220>
<221> UNSURE
<222> (34)..(34)
<223> Unknown amino acid

<400> 253

```
His Pro Arg Ser Ile Ala Glu Arg Glu Arg Arg Thr Arg Ile Ser Tyr
1               5                   10                  15
Lys Leu Arg Lys Leu Gln Asp Leu Val Pro Asn Met Asp Lys Gln Thr
            20                  25                  30
Ser Xaa Thr Ser Asp Met Leu Asp Leu Ala Val Glu His Ile Lys Gly
        35                  40                  45
Leu Gln
    50
```

<210> 254
<211> 50
<212> PRT
<213> Artificial sequence

<220>
<223> HLH domain of SEQ IS NO: 152

```
<220>
<221>  UNSURE
<222>  (34)..(34)
<223>  Unknown amino acid

<400>  254
His Pro Arg Ser Ile Ala Glu Arg Val Arg Arg Thr Arg Ile Ser Glu
1               5                   10                  15
Arg Met Arg Lys Leu Gln Glu Leu Val Pro Asn Met Asp Lys Gln Thr
            20                  25                  30
Asn Xaa Thr Ala Asp Met Leu Asp Leu Ala Val Asp Tyr Ile Lys Glu
        35                  40                  45
Leu Gln
    50

<210>  255
<211>  50
<212>  PRT
<213>  Artificial sequence

<220>
<223>  HLH domain of SEQ IS NO: 154

<220>
<221>  UNSURE
<222>  (34)..(34)
<223>  Unknown amino acid

<400>  255
His Pro Arg Ser Ile Ala Glu Arg Val Arg Arg Thr Arg Ile Ser Asp
1               5                   10                  15
Arg Ile Arg Lys Leu Gln Glu Leu Val Pro Asn Met Asp Lys Gln Thr
            20                  25                  30
Asn Xaa Thr Ala Asp Met Leu Glu Glu Ala Val Glu Tyr Val Lys Phe
        35                  40                  45
Leu Gln
    50

<210>  256
<211>  50
<212>  PRT
<213>  Artificial sequence

<220>
<223>  HLH domain of SEQ IS NO: 156

<220>
<221>  UNSURE
<222>  (34)..(34)
<223>  Unknown amino acid

<400>  256
His Pro Arg Ser Ile Ala Glu Arg Glu Arg Arg Thr Arg Ile Ser Gly
1               5                   10                  15
Lys Leu Lys Lys Leu Gln Asp Leu Val Pro Asn Met Asp Lys Gln Thr
            20                  25                  30
Ser Xaa Tyr Ala Asp Met Leu Asp Leu Ala Val Gln His Ile Lys Gly
        35                  40                  45
```

Leu Gln
    50

<210>  257
<211>  50
<212>  PRT
<213>  Artificial sequence

<220>
<223>  HLH domain of SEQ IS NO: 158

<220>
<221>  UNSURE
<222>  (34)..(34)
<223>  Unknown amino acid

<400>  257
His Pro Arg Ser Ile Ala Glu Arg Val Arg Arg Thr Arg Ile Ser Glu
1               5                   10                  15
Arg Met Arg Lys Leu Gln Glu Leu Phe Pro Asn Met Asp Lys Gln Thr
            20                  25                  30
Asn Xaa Thr Ala Asp Met Leu Asp Leu Ala Val Glu Tyr Ile Lys Asp
        35                  40                  45
Leu Gln
    50

<210>  258
<211>  50
<212>  PRT
<213>  Artificial sequence

<220>
<223>  HLH domain of SEQ IS NO: 160

<220>
<221>  UNSURE
<222>  (34)..(34)
<223>  Unknown amino acid

<400>  258
His Pro Arg Ser Ile Ala Glu Arg Val Arg Arg Thr Arg Ile Ser Glu
1               5                   10                  15
Arg Met Arg Lys Leu Gln Glu Leu Val Pro Asn Met Asp Lys Gln Thr
            20                  25                  30
Asn Xaa Thr Ser Asp Met Leu Asp Leu Ala Val Asp Tyr Ile Lys Asp
        35                  40                  45
Leu Gln
    50

<210>  259
<211>  50
<212>  PRT
<213>  Artificial sequence

<220>
<223>  HLH domain of SEQ IS NO: 162

<220>
<221>  UNSURE

<222> (34)..(34)
<223> Unknown amino acid

<400> 259
His Pro Arg Ser Ile Ala Glu Arg Val Arg Arg Thr Arg Ile Ser Glu
1 5 10 15
Arg Met Arg Lys Leu Gln Glu Leu Val Pro Asn Met Asp Lys Gln Thr
20 25 30
Asn Xaa Thr Ala Asp Met Leu Asp Leu Ala Val Glu Tyr Ile Lys Asp
35 40 45
Leu Gln
50

<210> 260
<211> 50
<212> PRT
<213> Artificial sequence

<220>
<223> HLH domain of SEQ IS NO: 164

<220>
<221> VARIANT
<222> (34)..(34)
<223> Unknown amino acid

<400> 260
His Pro Arg Ser Ile Ala Glu Arg Val Arg Arg Thr Arg Ile Ser Glu
1 5 10 15
Arg Met Arg Lys Leu Gln Glu Leu Val Pro Asn Met Asp Lys Gln Thr
20 25 30
Asn Xaa Thr Ala Asp Met Leu Asp Leu Ala Val Glu Tyr Ile Lys Asp
35 40 45
Leu Gln
50

<210> 261
<211> 50
<212> PRT
<213> Artificial sequence

<220>
<223> HLH domain of SEQ IS NO: 166

<220>
<221> UNSURE
<222> (34)..(34)
<223> Unknown amino acid

<400> 261
His Pro Arg Ser Ile Ala Glu Arg Val Arg Arg Thr Lys Ile Ser Glu
1 5 10 15
Arg Ile Arg Lys Leu Gln Glu Leu Val Pro Asn Met Glu Lys Gln Thr
20 25 30
Asn Xaa Thr Ala Asp Met Leu Asp Leu Ala Val Asp Tyr Ile Lys Asp
35 40 45
Leu Gln
50

```
<210>  262
<211>  50
<212>  PRT
<213>  Artificial sequence

<220>
<223>  HLH domain of SEQ IS NO: 168

<220>
<221>  UNSURE
<222>  (34)..(34)
<223>  Unknown amino acid

<400>  262
His Pro Arg Ser Ile Ala Glu Arg Glu Arg Arg Thr Arg Ile Ser Glu
1               5                   10                  15
Lys Leu Arg Lys Leu Gln Asp Leu Val Pro Asn Met Asp Lys Gln Thr
            20                  25                  30
Ser Xaa Thr Ala Asp Met Trp Asp Leu Ala Val Glu His Ile Arg Gly
        35                  40                  45
Leu Gln
    50

<210>  263
<211>  50
<212>  PRT
<213>  Artificial sequence

<220>
<223>  HLH domain of SEQ IS NO: 170

<220>
<221>  UNSURE
<222>  (34)..(34)
<223>  Unknown amino acid

<400>  263
His Pro Arg Ser Ile Ala Glu Arg Glu Arg Arg Thr Arg Ile Ser Glu
1               5                   10                  15
Lys Leu Arg Lys Leu Gln Ala Leu Val Pro Asn Met Asp Lys Gln Thr
            20                  25                  30
Ser Xaa Thr Ala Asp Met Leu Asp Leu Ala Val Asp His Ile Arg Gly
        35                  40                  45
Leu Gln
    50

<210>  264
<211>  50
<212>  PRT
<213>  Artificial sequence

<220>
<223>  HLH domain of SEQ IS NO: 172

<220>
<221>  UNSURE
<222>  (34)..(34)
<223>  Unknown amino acid
```

<400> 264
His Pro Arg Ser Ile Ala Glu Arg Val Arg Arg Thr Lys Ile Ser Glu
1 5 10 15
Arg Ile Arg Lys Leu Gln Glu Leu Val Pro Asp Met Asp Lys Gln Thr
20 25 30
Asn Xaa Thr Ser Asp Met Leu Asp Leu Ala Val Asp Tyr Ile Lys Asp
35 40 45
Leu Gln
50

<210> 265
<211> 50
<212> PRT
<213> Artificial sequence

<220>
<223> HLH domain of SEQ IS NO: 174

<220>
<221> UNSURE
<222> (34)..(34)
<223> Unknown amino acid

<400> 265
His Pro Arg Ser Ile Ala Glu Arg Glu Arg Arg Thr Arg Ile Ser Lys
1 5 10 15
Arg Leu Gln Lys Leu Gln Asp Leu Val Pro Asn Met Asp Lys Gln Thr
20 25 30
Ser Xaa Thr Ser Asp Met Leu Glu Leu Ala Ile Gln His Ile Lys Glu
35 40 45
Leu Gln
50

<210> 266
<211> 50
<212> PRT
<213> Artificial sequence

<220>
<223> HLH domain of SEQ IS NO: 176

<220>
<221> UNSURE
<222> (34)..(34)
<223> Unknown amino acid

<400> 266
His Pro Arg Ser Ile Ala Glu Arg Val Arg Arg Thr Arg Ile Ser Glu
1 5 10 15
Arg Ile Lys Lys Leu Gln Glu Leu Val Pro Asn Met Asp Thr Gln Thr
20 25 30
Asn Xaa Thr Ala Asp Met Leu Gly Leu Ala Ile Ser Tyr Ile Lys Asp
35 40 45
Leu Gln
50

<210> 267
<211> 50
<212> PRT

<213>  Artificial sequence

<220>
<223>  HLH domain of SEQ IS NO: 178

<220>
<221>  UNSURE
<222>  (34)..(34)
<223>  Unknown amino acid

<400>  267

```
His Pro Arg Ser Ile Ala Glu Arg Val Arg Arg Thr Lys Ile Ser Glu
1               5                   10                  15
Arg Met Arg Lys Leu Gln Glu Leu Val Pro Asn Met Asp Lys Gln Thr
            20                  25                  30
Asn Xaa Thr Ala Asp Met Leu Asp Leu Ala Val Glu Tyr Ile Lys Asp
        35                  40                  45
Leu Gln
    50
```

<210>  268
<211>  50
<212>  PRT
<213>  Artificial sequence

<220>
<223>  HLH domain of SEQ IS NO: 180

<220>
<221>  UNSURE
<222>  (34)..(34)
<223>  Unknown amino acid

<400>  268

```
His Pro Arg Ser Val Ala Glu Arg Val Arg Arg Ser Lys Ile Ser Glu
1               5                   10                  15
Arg Met Lys Lys Leu Gln Glu Leu Val Pro Asn Met Asp Lys Gln Thr
            20                  25                  30
Asn Xaa Thr Ala Ala Met Leu Asp Leu Ala Val Ile Tyr Ile Lys Asp
        35                  40                  45
Leu Gln
    50
```

<210>  269
<211>  59
<212>  PRT
<213>  Artificial sequence

<220>
<223>  consensus motif 1

<220>
<221>  UNSURE
<222>  (11)..(18)
<223>  Unknown amino acid or a gap

<220>
<221>  UNSURE
<222>  (55)..(55)

<223> Unknown amino acid

<400> 269
Val Pro Cys Lys Ile Arg Ala Lys Arg Gly Xaa Xaa Xaa Xaa Xaa Xaa
1 5 10 15
Xaa Xaa Cys Ala Thr His Pro Arg Ser Ile Ala Glu Arg Val Arg Arg
20 25 30
Thr Arg Ile Ser Glu Arg Met Arg Lys Leu Gln Glu Leu Val Pro Asn
35 40 45
Met Asp Lys Gln Thr Asn Xaa Thr Ala Asp Met
50 55

<210> 270
<211> 31
<212> PRT
<213> Artificial sequence

<220>
<223> consensus motif 2

<220>
<221> UNSURE
<222> (10)..(10)
<223> Unknown amino acid

<220>
<221> UNSURE
<222> (17)..(19)
<223> Unknown amino acid

<220>
<221> UNSURE
<222> (21)..(21)
<223> Unknown amino acid

<220>
<221> UNSURE
<222> (23)..(23)
<223> Unknown amino acid

<220>
<221> UNSURE
<222> (25)..(25)
<223> Unknown amino acid

<220>
<221> UNSURE
<222> (30)..(31)
<223> Unknown amino acid

<400> 270
Leu Asp Leu Ala Val Glu Tyr Ile Lys Xaa Leu Gln Lys Gln Val Gln
1 5 10 15
Xaa Xaa Xaa Leu Xaa Glu Xaa Arg Xaa Lys Cys Thr Cys Xaa Xaa
20 25 30

<210> 271
<211> 52
<212> DNA

<213> Artificial sequence

<220>
<223> primer 1

<400> 271
ggggacaagt ttgtacaaaa aagcaggctt aaacaatgaa caggatgacg gc 52

<210> 272
<211> 49
<212> DNA
<213> Artificial sequence

<220>
<223> primer 2

<400> 272
ggggaccact ttgtacaaga aagctgggtt tacaacatta gctcggaga 49

<210> 273
<211> 6
<212> DNA
<213> Artificial sequence

<220>
<223> E-box

<220>
<221> misc_feature
<222> (3)..(4)
<223> n is a, c, g, or t

<400> 273
canntg 6

<210> 274
<211> 6
<212> DNA
<213> Artificial sequence

<220>
<223> G-box

<400> 274
cacgtg 6

<210> 275
<211> 2194
<212> DNA
<213> Oryza sativa

<400> 275
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420

```
ttgtgcattc gtcatatcgc acatcattaa ggacatgtct tactccatcc caatttttat     480
ttagtaatta aagacaattg acttattttt attatttatc tttttcgat tagatgcaag      540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct    2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttc                                2194
```

<210> 276
<211> 623
<212> PRT
<213> Arabidopsis thaliana

<400> 276

```
Met Thr Asp Tyr Arg Leu Gln Pro Thr Met Asn Leu Trp Thr Thr Asp
1               5                   10                  15
Asp Asn Ala Ser Met Met Glu Ala Phe Met Ser Ser Ser Asp Ile Ser
            20                  25                  30
Thr Leu Trp Pro Pro Ala Ser Thr Thr Thr Thr Thr Ala Thr Thr Glu
        35                  40                  45
Thr Thr Pro Thr Pro Ala Met Glu Ile Pro Ala Gln Ala Gly Phe Asn
    50                  55                  60
Gln Glu Thr Leu Gln Gln Arg Leu Gln Ala Leu Ile Glu Gly Thr His
65                  70                  75                  80
Glu Gly Trp Thr Tyr Ala Ile Phe Trp Gln Pro Ser Tyr Asp Phe Ser
                85                  90                  95
Gly Ala Ser Val Leu Gly Trp Gly Asp Gly Tyr Tyr Lys Gly Glu Glu
            100                 105                 110
Asp Lys Ala Asn Pro Arg Arg Arg Ser Ser Ser Pro Pro Phe Ser Thr
        115                 120                 125
Pro Ala Asp Gln Glu Tyr Arg Lys Lys Val Leu Arg Glu Leu Asn Ser
    130                 135                 140
Leu Ile Ser Gly Gly Val Ala Pro Ser Asp Asp Ala Val Asp Glu Glu
145                 150                 155                 160
Val Thr Asp Thr Glu Trp Phe Phe Leu Val Ser Met Thr Gln Ser Phe
                165                 170                 175
```

Ala Cys Gly Ala Gly Leu Ala Gly Lys Ala Phe Ala Thr Gly Asn Ala
180 185 190
Val Trp Val Ser Gly Ser Asp Gln Leu Ser Gly Ser Gly Cys Glu Arg
195 200 205
Ala Lys Gln Gly Gly Val Phe Gly Met His Thr Ile Ala Cys Ile Pro
210 215 220
Ser Ala Asn Gly Val Val Glu Val Gly Ser Thr Glu Pro Ile Arg Gln
225 230 235 240
Ser Ser Asp Leu Ile Asn Lys Val Arg Ile Leu Phe Asn Phe Asp Gly
245 250 255
Gly Ala Gly Asp Leu Ser Gly Leu Asn Trp Asn Leu Asp Pro Asp Gln
260 265 270
Gly Glu Asn Asp Pro Ser Met Trp Ile Asn Asp Pro Ile Gly Thr Pro
275 280 285
Gly Ser Asn Glu Pro Gly Asn Gly Ala Pro Ser Ser Ser Ser Gln Leu
290 295 300
Phe Ser Lys Ser Ile Gln Phe Glu Asn Gly Ser Ser Ser Thr Ile Thr
305 310 315 320
Glu Asn Pro Asn Leu Asp Pro Thr Pro Ser Pro Val His Ser Gln Thr
325 330 335
Gln Asn Pro Lys Phe Asn Asn Thr Phe Ser Arg Glu Leu Asn Phe Ser
340 345 350
Thr Ser Ser Ser Thr Leu Val Lys Pro Arg Ser Gly Glu Ile Leu Asn
355 360 365
Phe Gly Asp Glu Gly Lys Arg Ser Ser Gly Asn Pro Asp Pro Ser Ser
370 375 380
Tyr Ser Gly Gln Thr Gln Phe Glu Asn Lys Arg Lys Arg Ser Met Val
385 390 395 400
Leu Asn Glu Asp Lys Val Leu Ser Phe Gly Asp Lys Thr Ala Gly Glu
405 410 415
Ser Asp His Ser Asp Leu Glu Ala Ser Val Val Lys Glu Val Ala Val
420 425 430
Glu Lys Arg Pro Lys Lys Arg Gly Arg Lys Pro Ala Asn Gly Arg Glu
435 440 445
Glu Pro Leu Asn His Val Glu Ala Glu Arg Gln Arg Arg Glu Lys Leu
450 455 460
Asn Gln Arg Phe Tyr Ala Leu Arg Ala Val Val Pro Asn Val Ser Lys
465 470 475 480
Met Asp Lys Ala Ser Leu Leu Gly Asp Ala Ile Ala Tyr Ile Asn Glu
485 490 495
Leu Lys Ser Lys Val Val Lys Thr Glu Ser Glu Lys Leu Gln Ile Lys
500 505 510
Asn Gln Leu Glu Glu Val Lys Leu Glu Leu Ala Gly Arg Lys Ala Ser
515 520 525
Ala Ser Gly Gly Asp Met Ser Ser Ser Cys Ser Ser Ile Lys Pro Val
530 535 540
Gly Met Glu Ile Glu Val Lys Ile Ile Gly Trp Asp Ala Met Ile Arg
545 550 555 560
Val Glu Ser Ser Lys Arg Asn His Pro Ala Ala Arg Leu Met Ser Ala
565 570 575
Leu Met Asp Leu Glu Leu Glu Val Asn His Ala Ser Met Ser Val Val
580 585 590
Asn Asp Leu Met Ile Gln Gln Ala Thr Val Lys Met Gly Phe Arg Ile
595 600 605
Tyr Thr Gln Glu Gln Leu Arg Ala Ser Leu Ile Ser Lys Ile Gly
610 615 620

<210> 277
<211> 302

<212> PRT
<213> Arabidopsis thaliana

<400> 277

```
Met Ala Asn Asn Asn Asn Ile Pro His Asp Ser Ile Ser Asp Pro Ser
1               5                   10                  15
Pro Thr Asp Asp Phe Phe Glu Gln Ile Leu Gly Leu Ser Asn Phe Ser
            20                  25                  30
Gly Ser Ser Gly Ser Gly Leu Ser Gly Ile Gly Gly Val Gly Pro Pro
        35                  40                  45
Pro Met Met Leu Gln Leu Gly Ser Gly Asn Glu Gly Asn His Asn His
    50                  55                  60
Met Gly Ala Ile Gly Gly Gly Gly Pro Val Gly Phe His Asn Gln Met
65                  70                  75                  80
Phe Pro Leu Gly Leu Ser Leu Asp Gln Gly Lys Gly His Gly Phe Leu
                85                  90                  95
Lys Pro Asp Glu Thr Gly Lys Arg Phe Gln Asp Asp Val Leu Asp Asn
            100                 105                 110
Arg Cys Ser Ser Met Lys Pro Ile Phe His Gly Gln Pro Met Ser Gln
        115                 120                 125
Pro Ala Pro Pro Met Pro His Gln Gln Ser Thr Ile Arg Pro Arg Val
    130                 135                 140
Arg Ala Arg Arg Gly Gln Ala Thr Asp Pro His Ser Ile Ala Glu Arg
145                 150                 155                 160
Leu Arg Arg Glu Arg Ile Ala Glu Arg Ile Arg Ser Leu Gln Glu Leu
                165                 170                 175
Val Pro Thr Val Asn Lys Thr Asp Arg Ala Ala Met Ile Asp Glu Ile
            180                 185                 190
Val Asp Tyr Val Lys Phe Leu Arg Leu Gln Val Lys Val Leu Ser Met
        195                 200                 205
Ser Arg Leu Gly Gly Ala Gly Ala Val Ala Pro Leu Val Thr Glu Met
    210                 215                 220
Pro Leu Ser Ser Ser Val Glu Asp Glu Thr Gln Ala Val Trp Glu Lys
225                 230                 235                 240
Trp Ser Asn Asp Gly Thr Glu Arg Gln Val Ala Lys Leu Met Glu Glu
                245                 250                 255
Asn Val Gly Ala Ala Met Gln Leu Leu Gln Ser Lys Ala Leu Cys Ile
            260                 265                 270
Met Pro Ile Ser Leu Ala Met Ala Ile Tyr His Ser Gln Pro Pro Asp
        275                 280                 285
Thr Ser Ser Ser Ile Val Lys Pro Glu Met Asn Pro Pro Pro
    290                 295                 300
```

<210> 278
<211> 430
<212> PRT
<213> Arabidopsis thaliana

<400> 278

```
Met Glu His Gln Gly Trp Ser Phe Glu Glu Asn Tyr Ser Leu Ser Thr
1               5                   10                  15
Asn Arg Arg Ser Ile Arg Pro Gln Asp Glu Leu Val Glu Leu Leu Trp
            20                  25                  30
Arg Asp Gly Gln Val Val Leu Gln Ser Gln Thr His Arg Glu Gln Thr
        35                  40                  45
Gln Thr Gln Lys Gln Asp His His Glu Glu Ala Leu Arg Ser Ser Thr
    50                  55                  60
Phe Leu Glu Asp Gln Glu Thr Val Ser Trp Ile Gln Tyr Pro Pro Asp
65                  70                  75                  80
```

```
Glu Asp Pro Phe Glu Pro Asp Asp Phe Ser Ser His Phe Phe Ser Thr
                85                  90                  95
Met Asp Pro Leu Gln Arg Pro Thr Ser Glu Thr Val Lys Pro Lys Ser
            100                 105                 110
Ser Pro Glu Pro Pro Gln Val Met Val Lys Pro Lys Ala Cys Pro Asp
        115                 120                 125
Pro Pro Pro Gln Val Met Pro Pro Pro Lys Phe Arg Leu Thr Asn Ser
    130                 135                 140
Ser Ser Gly Ile Arg Glu Thr Glu Met Glu Gln Tyr Ser Val Thr Thr
145                 150                 155                 160
Val Gly Pro Ser His Cys Gly Ser Asn Pro Ser Gln Asn Asp Leu Asp
                165                 170                 175
Val Ser Met Ser His Asp Arg Ser Lys Asn Ile Glu Glu Lys Leu Asn
            180                 185                 190
Pro Asn Ala Ser Ser Ser Ser Gly Gly Ser Ser Gly Cys Ser Phe Gly
        195                 200                 205
Lys Asp Ile Lys Glu Met Ala Ser Gly Arg Cys Ile Thr Thr Asp Arg
    210                 215                 220
Lys Arg Lys Arg Ile Asn His Thr Asp Glu Ser Val Ser Leu Ser Asp
225                 230                 235                 240
Ala Ile Gly Asn Lys Ser Asn Gln Arg Ser Gly Ser Asn Arg Arg Ser
                245                 250                 255
Arg Ala Ala Glu Val His Asn Leu Ser Glu Arg Arg Arg Arg Asp Arg
            260                 265                 270
Ile Asn Glu Arg Met Lys Ala Leu Gln Glu Leu Ile Pro His Cys Ser
        275                 280                 285
Lys Thr Asp Lys Ala Ser Ile Leu Asp Glu Ala Ile Asp Tyr Leu Lys
    290                 295                 300
Ser Leu Gln Leu Gln Leu Gln Val Met Trp Met Gly Ser Gly Met Ala
305                 310                 315                 320
Ala Ala Ala Ala Ser Ala Pro Met Met Phe Pro Gly Val Gln Pro Gln
                325                 330                 335
Gln Phe Ile Arg Gln Ile Gln Ser Pro Val Gln Leu Pro Arg Phe Pro
            340                 345                 350
Val Met Asp Gln Ser Ala Ile Gln Asn Asn Pro Gly Leu Val Cys Gln
        355                 360                 365
Asn Pro Val Gln Asn Gln Ile Ile Ser Asp Arg Phe Ala Arg Tyr Ile
    370                 375                 380
Gly Gly Phe Pro His Met Gln Ala Ala Thr Gln Met Gln Pro Met Glu
385                 390                 395                 400
Met Leu Arg Phe Ser Ser Pro Ala Gly Gln Gln Ser Gln Gln Pro Ser
                405                 410                 415
Ser Val Pro Thr Lys Thr Thr Asp Gly Ser Arg Leu Asp His
            420                 425                 430

<210>  279
<211>  458
<212>  PRT
<213>  Arabidopsis thaliana

<400>  279
Met Glu Glu Glu Arg Glu Ser Leu Tyr Glu Glu Met Gly Cys Phe Asp
1               5                   10                  15
Pro Asn Thr Pro Ala Glu Val Thr Val Glu Ser Ser Phe Ser Gln Ala
            20                  25                  30
Glu Pro Pro Pro Pro Pro Pro Gln Val Leu Val Ala Gly Ser Thr Ser
        35                  40                  45
Asn Ser Asn Cys Ser Val Glu Val Glu Glu Leu Ser Glu Phe His Leu
    50                  55                  60
```

```
Ser Pro Gln Asp Cys Pro Gln Ala Ser Ser Thr Pro Leu Gln Phe His
65                  70                  75                  80
Ile Asn Pro Pro Pro Pro Pro Pro Pro Pro Cys Asp Gln Leu His Asn
                85                  90                  95
Asn Leu Ile His Gln Met Ala Ser His Gln Gln Gln His Ser Asn Trp
            100                 105                 110
Asp Asn Gly Tyr Gln Asp Phe Val Asn Leu Gly Pro Asn Ser Ala Thr
        115                 120                 125
Thr Pro Asp Leu Leu Ser Leu Leu His Leu Pro Arg Cys Ser Leu Pro
    130                 135                 140
Pro Asn His His Pro Ser Ser Met Leu Pro Thr Ser Phe Ser Asp Ile
145                 150                 155                 160
Met Ser Ser Ser Ser Ala Ala Ala Val Met Tyr Asp Pro Leu Phe His
                165                 170                 175
Leu Asn Phe Pro Met Gln Pro Arg Asp Gln Asn Gln Leu Arg Asn Gly
            180                 185                 190
Ser Cys Leu Leu Gly Val Glu Asp Gln Ile Gln Met Asp Ala Asn Gly
        195                 200                 205
Gly Met Asn Val Leu Tyr Phe Glu Gly Ala Asn Asn Asn Asn Gly Gly
    210                 215                 220
Phe Glu Asn Glu Ile Leu Glu Phe Asn Asn Gly Val Thr Arg Lys Gly
225                 230                 235                 240
Arg Gly Ser Arg Lys Ser Arg Thr Ser Pro Thr Glu Arg Glu Arg Arg
                245                 250                 255
Val His Phe Asn Asp Arg Phe Phe Asp Leu Lys Asn Leu Ile Pro Asn
            260                 265                 270
Pro Thr Lys Ile Asp Arg Ala Ser Ile Val Gly Glu Ala Ile Asp Tyr
        275                 280                 285
Ile Lys Glu Leu Leu Arg Thr Ile Glu Glu Phe Lys Met Leu Val Glu
    290                 295                 300
Lys Lys Arg Cys Gly Arg Phe Arg Ser Lys Lys Arg Ala Arg Val Gly
305                 310                 315                 320
Glu Gly Gly Gly Gly Glu Asp Gln Glu Glu Glu Glu Asp Thr Val Asn
                325                 330                 335
Tyr Lys Pro Gln Ser Glu Val Asp Gln Ser Cys Phe Asn Lys Asn Asn
            340                 345                 350
Asn Asn Ser Leu Arg Cys Ser Trp Leu Lys Arg Lys Ser Lys Val Thr
        355                 360                 365
Glu Val Asp Val Arg Ile Ile Asp Asp Glu Val Thr Ile Lys Leu Val
    370                 375                 380
Gln Lys Lys Lys Ile Asn Cys Leu Leu Phe Thr Thr Lys Val Leu Asp
385                 390                 395                 400
Gln Leu Gln Leu Asp Leu His His Val Ala Gly Gly Gln Ile Gly Glu
                405                 410                 415
His Tyr Ser Phe Leu Phe Asn Thr Lys Ile Cys Glu Gly Ser Cys Val
            420                 425                 430
Tyr Ala Ser Gly Ile Ala Asp Thr Leu Met Glu Val Val Glu Lys Gln
        435                 440                 445
Tyr Met Glu Ala Val Pro Ser Asn Gly Tyr
    450                 455

<210>  280
<211>  286
<212>  PRT
<213>  Arabidopsis thaliana

<400>  280
Met Asp Gln Pro Met Lys Pro Lys Thr Cys Ser Glu Ser Asp Phe Ala
1               5                   10                  15
```

Asp Asp Ser Ser Ala Ser Ser Ser Ser Ser Ser Gly Gln Asn Leu Arg
20 25 30
Gly Ala Glu Met Val Val Glu Val Lys Lys Glu Ala Val Cys Ser Gln
35 40 45
Lys Ala Glu Arg Glu Lys Leu Arg Arg Asp Lys Leu Lys Glu Gln Phe
50 55 60
Leu Glu Leu Gly Asn Ala Leu Asp Pro Asn Arg Pro Lys Ser Asp Lys
65 70 75 80
Ala Ser Val Leu Thr Asp Thr Ile Gln Met Leu Lys Asp Val Met Asn
85 90 95
Gln Val Asp Arg Leu Lys Ala Glu Tyr Glu Thr Leu Ser Gln Glu Ser
100 105 110
Arg Glu Leu Ile Gln Glu Lys Ser Glu Leu Arg Glu Glu Lys Ala Thr
115 120 125
Leu Lys Ser Asp Ile Glu Ile Leu Asn Ala Gln Tyr Gln His Arg Ile
130 135 140
Lys Thr Met Val Pro Trp Val Pro His Tyr Ser Tyr His Ile Pro Phe
145 150 155 160
Val Ala Ile Thr Gln Gly Gln Ser Ser Phe Ile Pro Tyr Ser Ala Ser
165 170 175
Val Asn Pro Leu Thr Glu Gln Gln Ala Ser Val Gln Gln His Ser Ser
180 185 190
Ser Ser Ala Asp Ala Ser Met Lys Gln Asp Ser Lys Ile Lys Pro Leu
195 200 205
Asp Leu Asp Leu Met Met Asn Ser Asn His Ser Gly Gln Gly Asn Asp
210 215 220
Gln Lys Asp Asp Val Arg Leu Lys Leu Glu Leu Lys Ile His Ala Ser
225 230 235 240
Ser Leu Ala Gln Gln Asp Val Ser Gly Lys Glu Lys Lys Val Ser Leu
245 250 255
Thr Thr Thr Ala Ser Ser Ser Asn Ser Tyr Ser Leu Ser Gln Ala Val
260 265 270
Gln Asp Ser Ser Pro Gly Thr Val Asn Asp Met Leu Lys Pro
275 280 285

<210> 281
<211> 590
<212> PRT
<213> Arabidopsis thaliana

<400> 281
Met Asn Ile Gly Arg Leu Val Trp Asn Glu Asp Asp Lys Ala Ile Val
1 5 10 15
Ala Ser Leu Leu Gly Lys Arg Ala Leu Asp Tyr Leu Leu Ser Asn Ser
20 25 30
Val Ser Asn Ala Asn Leu Leu Met Thr Leu Gly Ser Asp Glu Asn Leu
35 40 45
Gln Asn Lys Leu Ser Asp Leu Val Glu Arg Pro Asn Ala Ser Asn Phe
50 55 60
Ser Trp Asn Tyr Ala Ile Phe Trp Gln Ile Ser Arg Ser Lys Ala Gly
65 70 75 80
Asp Leu Val Leu Cys Trp Gly Asp Gly Tyr Cys Arg Glu Pro Lys Glu
85 90 95
Gly Glu Lys Ser Glu Ile Val Arg Ile Leu Ser Met Gly Arg Glu Glu
100 105 110
Glu Thr His Gln Thr Met Arg Lys Arg Val Leu Gln Lys Leu His Asp
115 120 125
Leu Phe Gly Gly Ser Glu Glu Glu Asn Cys Ala Leu Gly Leu Asp Arg
130 135 140

```
Val Thr Asp Thr Glu Met Phe Leu Leu Ser Ser Met Tyr Phe Ser Phe
145                 150                 155                 160
Pro Arg Gly Glu Gly Gly Pro Gly Lys Cys Phe Ala Ser Ala Lys Pro
                165                 170                 175
Val Trp Leu Ser Asp Val Val Asn Ser Gly Ser Asp Tyr Cys Val Arg
            180                 185                 190
Ser Phe Leu Ala Lys Ser Ala Gly Ile Gln Thr Val Val Leu Val Pro
        195                 200                 205
Thr Asp Leu Gly Val Val Glu Leu Gly Ser Thr Ser Cys Leu Pro Glu
    210                 215                 220
Ser Glu Asp Ser Ile Leu Ser Ile Arg Ser Leu Phe Thr Ser Ser Leu
225                 230                 235                 240
Pro Pro Val Arg Ala Val Ala Leu Pro Val Thr Val Ala Glu Lys Ile
                245                 250                 255
Asp Asp Asn Arg Thr Lys Ile Phe Gly Lys Asp Leu His Asn Ser Gly
            260                 265                 270
Phe Leu Gln His His Gln His His Gln Gln Gln Gln Gln Gln Pro Pro
        275                 280                 285
Gln Gln Gln Gln His Arg Gln Phe Arg Glu Lys Leu Thr Val Arg Lys
    290                 295                 300
Met Asp Asp Arg Ala Pro Lys Arg Leu Asp Ala Tyr Pro Asn Asn Gly
305                 310                 315                 320
Asn Arg Phe Met Phe Ser Asn Pro Gly Thr Asn Asn Asn Thr Leu Leu
                325                 330                 335
Ser Pro Thr Trp Val Gln Pro Glu Asn Tyr Thr Arg Pro Ile Asn Val
            340                 345                 350
Lys Glu Val Pro Ser Thr Asp Glu Phe Lys Phe Leu Pro Leu Gln Gln
        355                 360                 365
Ser Ser Gln Arg Leu Leu Pro Pro Ala Gln Met Gln Ile Asp Phe Ser
    370                 375                 380
Ala Ala Ser Ser Arg Ala Ser Glu Asn Asn Ser Asp Gly Glu Gly Gly
385                 390                 395                 400
Gly Glu Trp Ala Asp Ala Val Gly Ala Asp Glu Ser Gly Asn Asn Arg
                405                 410                 415
Pro Arg Lys Arg Gly Arg Arg Pro Ala Asn Gly Arg Ala Glu Ala Leu
            420                 425                 430
Asn His Val Glu Ala Glu Arg Gln Arg Arg Glu Lys Leu Asn Gln Arg
        435                 440                 445
Phe Tyr Ala Leu Arg Ser Val Val Pro Asn Ile Ser Lys Met Asp Lys
    450                 455                 460
Ala Ser Leu Leu Gly Asp Ala Val Ser Tyr Ile Asn Glu Leu His Ala
465                 470                 475                 480
Lys Leu Lys Val Met Glu Ala Glu Arg Glu Arg Leu Gly Tyr Ser Ser
                485                 490                 495
Asn Pro Pro Ile Ser Leu Asp Ser Asp Ile Asn Val Gln Thr Ser Gly
            500                 505                 510
Glu Asp Val Thr Val Arg Ile Asn Cys Pro Leu Glu Ser His Pro Ala
        515                 520                 525
Ser Arg Ile Phe His Ala Phe Glu Glu Ser Lys Val Glu Val Ile Asn
    530                 535                 540
Ser Asn Leu Glu Val Ser Gln Asp Thr Val Leu His Thr Phe Val Val
545                 550                 555                 560
Lys Ser Glu Glu Leu Thr Lys Glu Lys Leu Ile Ser Ala Leu Ser Arg
                565                 570                 575
Glu Gln Thr Asn Ser Val Gln Ser Arg Thr Ser Ser Gly Arg
            580                 585                 590
```

<210> 282
<211> 314

```
<212>  PRT
<213>  Arabidopsis thaliana

<400>  282
Met Asp Leu Met Ile Asp Thr Asp Glu Tyr Leu Ile Asp Asp Trp Glu
1               5                   10                  15
Ser Asp Phe Pro Ile Cys Gly Glu Thr Asn Thr Asn Pro Gly Ser Glu
            20                  25                  30
Ser Gly Ser Gly Thr Gly Phe Glu Leu Leu Ala Glu Arg Pro Thr Lys
        35                  40                  45
Gln Met Lys Thr Asn Asn Asn Met Asn Ser Thr Ser Ser Ser Pro Ser
    50                  55                  60
Ser Ser Ser Ser Ser Gly Ser Arg Thr Ser Gln Val Ile Ser Phe Gly
65                  70                  75                  80
Ser Pro Asp Thr Lys Thr Asn Pro Val Glu Thr Ser Leu Asn Phe Ser
                85                  90                  95
Asn Gln Val Ser Met Asp Gln Lys Val Gly Ser Lys Arg Lys Asp Cys
            100                 105                 110
Val Asn Asn Gly Gly Arg Arg Glu Pro His Leu Leu Lys Glu His Val
        115                 120                 125
Leu Ala Glu Arg Lys Arg Arg Gln Lys Leu Asn Glu Arg Leu Ile Ala
    130                 135                 140
Leu Ser Ala Leu Leu Pro Gly Leu Lys Lys Thr Asp Lys Ala Thr Val
145                 150                 155                 160
Leu Glu Asp Ala Ile Lys His Leu Lys Gln Leu Gln Glu Arg Val Lys
                165                 170                 175
Lys Leu Glu Glu Glu Arg Val Val Thr Lys Lys Met Asp Gln Ser Ile
            180                 185                 190
Ile Leu Val Lys Arg Ser Gln Val Tyr Leu Asp Asp Asp Ser Ser Ser
        195                 200                 205
Tyr Ser Ser Thr Cys Ser Ala Ala Ser Pro Leu Ser Ser Ser Ser Asp
    210                 215                 220
Glu Val Ser Ile Phe Lys Gln Thr Met Pro Met Ile Glu Ala Arg Val
225                 230                 235                 240
Ser Asp Arg Asp Leu Leu Ile Arg Val His Cys Glu Lys Asn Lys Gly
                245                 250                 255
Cys Met Ile Lys Ile Leu Ser Ser Leu Glu Lys Phe Arg Leu Glu Val
            260                 265                 270
Val Asn Ser Phe Thr Leu Pro Phe Gly Asn Ser Thr Leu Val Ile Thr
        275                 280                 285
Ile Leu Thr Lys Met Asp Asn Lys Phe Ser Arg Pro Val Glu Glu Val
    290                 295                 300
Val Lys Asn Ile Arg Val Ala Leu Ala Glu
305                 310

<210>  283
<211>  292
<212>  PRT
<213>  Arabidopsis thaliana

<400>  283
Met Ser Asn Asn Gln Ala Phe Met Glu Leu Gly Trp Arg Asn Asp Val
1               5                   10                  15
Gly Ser Leu Ala Val Lys Asp Gln Gly Met Met Ser Glu Arg Ala Arg
            20                  25                  30
Ser Asp Glu Asp Arg Leu Ile Asn Gly Leu Lys Trp Gly Tyr Gly Tyr
        35                  40                  45
Phe Asp His Asp Gln Thr Asp Asn Tyr Leu Gln Ile Val Pro Glu Ile
    50                  55                  60
```

His Lys Glu Val Glu Asn Ala Lys Glu Asp Leu Leu Val Val Val Pro
65 70 75 80
Asp Glu His Ser Glu Thr Asp Asp His His His Ile Lys Asp Phe Ser
85 90 95
Glu Arg Ser Asp His Arg Phe Tyr Leu Arg Asn Lys His Glu Asn Pro
100 105 110
Lys Lys Arg Arg Ile Gln Val Leu Ser Ser Asp Asp Glu Ser Glu Glu
115 120 125
Phe Thr Arg Glu Val Pro Ser Val Thr Arg Lys Gly Ser Lys Arg Arg
130 135 140
Arg Arg Asp Glu Lys Met Ser Asn Lys Met Arg Lys Leu Gln Gln Leu
145 150 155 160
Val Pro Asn Cys His Lys Thr Asp Lys Val Ser Val Leu Asp Lys Thr
165 170 175
Ile Glu Tyr Met Lys Asn Leu Gln Leu Gln Leu Gln Met Met Ser Thr
180 185 190
Val Gly Val Asn Pro Tyr Phe Leu Pro Ala Thr Leu Gly Phe Gly Met
195 200 205
His Asn His Met Leu Thr Ala Met Ala Ser Ala His Gly Leu Asn Pro
210 215 220
Ala Asn His Met Met Pro Ser Pro Leu Ile Pro Ala Leu Asn Trp Pro
225 230 235 240
Leu Pro Pro Phe Thr Asn Ile Ser Phe Pro His Ser Ser Ser Gln Ser
245 250 255
Leu Phe Leu Thr Thr Ser Ser Pro Ala Ser Ser Pro Gln Ser Leu His
260 265 270
Gly Leu Val Pro Tyr Phe Pro Ser Phe Leu Asp Phe Ser Ser His Ala
275 280 285
Met Arg Arg Leu
290

<210> 284
<211> 318
<212> PRT
<213> Arabidopsis thaliana

<400> 284
Met Glu Gly Arg Val Asn Ala Leu Ser Asn Ile Asn Asp Leu Glu Leu
1 5 10 15
His Asn Phe Leu Val Asp Pro Asn Phe Asp Gln Phe Ile Asn Leu Ile
20 25 30
Arg Gly Asp His Gln Thr Ile Asp Glu Asn Pro Val Leu Asp Phe Asp
35 40 45
Leu Gly Pro Leu Gln Asn Ser Pro Cys Phe Ile Asp Glu Asn Gln Phe
50 55 60
Ile Pro Thr Pro Val Asp Asp Leu Phe Asp Glu Leu Pro Asp Leu Asp
65 70 75 80
Ser Asn Val Ala Glu Ser Phe Arg Ser Phe Asp Gly Asp Ser Val Arg
85 90 95
Ala Gly Gly Glu Glu Asp Glu Glu Asp Tyr Asn Asp Gly Asp Asp Ser
100 105 110
Ser Ala Thr Thr Thr Asn Asn Asp Gly Thr Arg Lys Thr Lys Thr Asp
115 120 125
Arg Ser Arg Thr Leu Ile Ser Glu Arg Arg Arg Arg Gly Arg Met Lys
130 135 140
Asp Lys Leu Tyr Ala Leu Arg Ser Leu Val Pro Asn Ile Thr Lys Met
145 150 155 160
Asp Lys Ala Ser Ile Val Gly Asp Ala Val Leu Tyr Val Gln Glu Leu
165 170 175

```
Gln Ser Gln Ala Lys Lys Leu Lys Ser Asp Ile Ala Gly Leu Glu Ala
            180                 185                 190
Ser Leu Asn Ser Thr Gly Gly Tyr Gln Glu His Ala Pro Asp Ala Gln
        195                 200                 205
Lys Thr Gln Pro Phe Arg Gly Ile Asn Pro Pro Ala Ser Lys Lys Ile
    210                 215                 220
Ile Gln Met Asp Val Ile Gln Val Glu Glu Lys Gly Phe Tyr Val Arg
225                 230                 235                 240
Leu Val Cys Asn Lys Gly Glu Gly Val Ala Pro Ser Leu Tyr Lys Ser
                245                 250                 255
Leu Glu Ser Leu Thr Ser Phe Gln Val Gln Asn Ser Asn Leu Ser Ser
            260                 265                 270
Pro Ser Pro Asp Thr Tyr Leu Leu Thr Tyr Thr Leu Asp Gly Thr Cys
        275                 280                 285
Phe Glu Gln Ser Leu Asn Leu Pro Asn Leu Lys Leu Trp Ile Thr Gly
    290                 295                 300
Ser Leu Leu Asn Gln Gly Phe Glu Phe Ile Lys Ser Phe Thr
305                 310                 315

<210>  285
<211>  368
<212>  PRT
<213>  Arabidopsis thaliana

<400>  285
Met Cys Ala Lys Lys Glu Glu Glu Glu Glu Glu Glu Glu Asp Ser Ser
1               5                   10                  15
Glu Ala Met Asn Asn Ile Gln Asn Tyr Gln Asn Asp Leu Phe Phe His
            20                  25                  30
Gln Leu Ile Ser His His His His His His His Asp Pro Ser Gln Ser
        35                  40                  45
Glu Thr Leu Gly Ala Ser Gly Asn Val Gly Ser Gly Phe Thr Ile Phe
    50                  55                  60
Ser Gln Asp Ser Val Ser Pro Ile Trp Ser Leu Pro Pro Pro Thr Ser
65                  70                  75                  80
Ile Gln Pro Pro Phe Asp Gln Phe Pro Pro Pro Ser Ser Ser Pro Ala
                85                  90                  95
Ser Phe Tyr Gly Ser Phe Phe Asn Arg Ser Arg Ala His His Gln Gly
            100                 105                 110
Leu Gln Phe Gly Tyr Glu Gly Phe Gly Gly Ala Thr Ser Ala Ala His
        115                 120                 125
His His His Glu Gln Leu Arg Ile Leu Ser Glu Ala Leu Gly Pro Val
    130                 135                 140
Val Gln Ala Gly Ser Gly Pro Phe Gly Leu Gln Ala Glu Leu Gly Lys
145                 150                 155                 160
Met Thr Ala Gln Glu Ile Met Asp Ala Lys Ala Leu Ala Ala Ser Lys
                165                 170                 175
Ser His Ser Glu Ala Glu Arg Arg Arg Arg Glu Arg Ile Asn Asn His
            180                 185                 190
Leu Ala Lys Leu Arg Ser Ile Leu Pro Asn Thr Thr Lys Thr Asp Lys
        195                 200                 205
Ala Ser Leu Leu Ala Glu Val Ile Gln His Val Lys Glu Leu Lys Arg
    210                 215                 220
Glu Thr Ser Val Ile Ser Glu Thr Asn Leu Val Pro Thr Glu Ser Asp
225                 230                 235                 240
Glu Leu Thr Val Ala Phe Thr Glu Glu Glu Glu Thr Gly Asp Gly Arg
                245                 250                 255
Phe Val Ile Lys Ala Ser Leu Cys Cys Glu Asp Arg Ser Asp Leu Leu
            260                 265                 270
```

```
Pro Asp Met Ile Lys Thr Leu Lys Ala Met Arg Leu Lys Thr Leu Lys
        275                 280                 285
Ala Glu Ile Thr Thr Val Gly Gly Arg Val Lys Asn Val Leu Phe Val
    290                 295                 300
Thr Gly Glu Glu Ser Ser Gly Glu Glu Val Glu Glu Glu Tyr Cys Ile
305                 310                 315                 320
Gly Thr Ile Glu Glu Ala Leu Lys Ala Val Met Glu Lys Ser Asn Val
                325                 330                 335
Glu Glu Ser Ser Ser Ser Gly Asn Ala Lys Arg Gln Arg Met Ser Ser
            340                 345                 350
His Asn Thr Ile Thr Ile Val Glu Gln Gln Gln Gln Tyr Asn Gln Arg
        355                 360                 365

<210>  286
<211>  828
<212>  PRT
<213>  Arabidopsis thaliana

<400>  286
Met Glu Ser Arg Glu Asp Ser Phe Ile Ser Lys Glu Lys Lys Ser Thr
1               5                   10                  15
Met Lys Lys Glu Lys Gln Ala Ile Ala Ser Gln Arg Asn Arg Arg Arg
            20                  25                  30
Val Ile Lys Asn Arg Gly Asn Gly Lys Arg Leu Ile Ala Ser Leu Ser
        35                  40                  45
Gln Arg Lys Arg Arg Arg Ile Pro Arg Gly Arg Gly Asn Glu Lys Ala
    50                  55                  60
Val Phe Ala Pro Ser Ser Leu Pro Asn Asp Val Val Glu Glu Ile Phe
65                  70                  75                  80
Leu Arg Leu Pro Val Lys Ala Ile Ile Gln Leu Lys Ser Leu Ser Lys
                85                  90                  95
Gln Trp Arg Ser Thr Ile Glu Ser Arg Ser Phe Glu Glu Arg His Leu
            100                 105                 110
Lys Ile Val Glu Arg Ser Arg Val Asp Phe Pro Gln Val Met Val Met
        115                 120                 125
Ser Glu Glu Tyr Ser Leu Lys Gly Ser Lys Gly Asn Gln Pro Arg Pro
    130                 135                 140
Asp Thr Asp Ile Gly Phe Ser Thr Ile Cys Leu Glu Ser Ala Ser Ile
145                 150                 155                 160
Leu Ser Ser Thr Leu Ile Thr Phe Pro Gln Gly Phe Gln His Arg Ile
                165                 170                 175
Tyr Ala Ser Glu Ser Cys Asp Gly Leu Phe Cys Ile His Ser Leu Lys
            180                 185                 190
Thr Gln Ala Ile Tyr Val Val Asn Pro Ala Thr Arg Trp Phe Arg Gln
        195                 200                 205
Leu Pro Pro Ala Arg Phe Gln Ile Leu Met Gln Lys Leu Tyr Pro Thr
    210                 215                 220
Gln Asp Thr Trp Ile Asp Ile Lys Pro Val Val Cys Tyr Thr Ala Phe
225                 230                 235                 240
Val Lys Ala Asn Asp Tyr Lys Leu Val Trp Leu Tyr Asn Ser Asp Ala
                245                 250                 255
Ser Asn Pro Asn Leu Gly Val Thr Lys Cys Glu Val Phe Asp Phe Arg
            260                 265                 270
Ala Asn Ala Trp Arg Tyr Leu Thr Cys Thr Pro Ser Tyr Arg Ile Phe
        275                 280                 285
Pro Asp Gln Val Pro Ala Ala Thr Asn Gly Ser Ile Tyr Trp Phe Thr
    290                 295                 300
Glu Pro Tyr Asn Gly Glu Ile Lys Val Val Ala Leu Asp Ile His Thr
305                 310                 315                 320
```

```
Glu Thr Phe Arg Val Leu Pro Lys Ile Asn Pro Ala Ile Ala Ser Ser
                325                 330                 335
Asp Pro Asp His Ile Asp Met Cys Thr Leu Asp Asn Gly Leu Cys Met
            340                 345                 350
Ser Lys Arg Glu Ser Asp Thr Leu Val Gln Glu Ile Trp Arg Leu Lys
        355                 360                 365
Ser Ser Glu Asp Ser Trp Glu Lys Phe Asp Met Asn Ser Asp Gly Val
    370                 375                 380
Trp Leu Asp Gly Ser Gly Glu Ser Pro Glu Val Asn Asn Gly Glu Ala
385                 390                 395                 400
Ala Ser Trp Val Arg Asn Pro Asp Glu Asp Trp Phe Asn Asn Pro Pro
                405                 410                 415
Pro Pro Gln His Thr Asn Gln Asn Asp Phe Arg Phe Asn Gly Gly Phe
            420                 425                 430
Pro Leu Asn Pro Ser Glu Asn Leu Leu Leu Leu Leu Gln Gln Ser Ile
        435                 440                 445
Asp Ser Ser Ser Ser Ser Ser Pro Leu Leu His Pro Phe Thr Leu Asp
    450                 455                 460
Ala Ala Ser Gln Gln Gln Gln Gln Gln Gln Gln Gln Gln Glu Gln Ser
465                 470                 475                 480
Phe Leu Ala Thr Lys Ala Cys Ile Val Ser Leu Leu Asn Val Pro Thr
                485                 490                 495
Ile Asn Asn Asn Thr Phe Asp Asp Phe Gly Phe Asp Ser Gly Phe Leu
            500                 505                 510
Gly Gln Gln Phe His Gly Asn His Gln Ser Pro Asn Ser Met Asn Phe
        515                 520                 525
Thr Gly Leu Asn His Ser Val Pro Asp Phe Leu Pro Ala Pro Glu Asn
    530                 535                 540
Ser Ser Gly Ser Cys Gly Leu Ser Pro Leu Phe Ser Asn Arg Ala Lys
545                 550                 555                 560
Val Leu Lys Pro Leu Gln Val Met Ala Ser Ser Gly Ser Gln Pro Thr
                565                 570                 575
Leu Phe Gln Lys Arg Ala Ala Met Arg Gln Ser Ser Ser Ser Lys Met
            580                 585                 590
Cys Asn Ser Glu Ser Ser Ser Glu Met Arg Lys Ser Ser Tyr Glu Arg
        595                 600                 605
Glu Ile Asp Asp Thr Ser Thr Gly Ile Ile Asp Ile Ser Gly Leu Asn
    610                 615                 620
Tyr Glu Ser Asp Asp His Asn Thr Asn Asn Asn Lys Gly Lys Lys Lys
625                 630                 635                 640
Gly Met Pro Ala Lys Asn Leu Met Ala Glu Arg Arg Arg Arg Lys Lys
                645                 650                 655
Leu Asn Asp Arg Leu Tyr Met Leu Arg Ser Val Val Pro Lys Ile Ser
            660                 665                 670
Lys Met Asp Arg Ala Ser Ile Leu Gly Asp Ala Ile Asp Tyr Leu Lys
        675                 680                 685
Glu Leu Leu Gln Arg Ile Asn Asp Leu His Thr Glu Leu Glu Ser Thr
    690                 695                 700
Pro Pro Ser Ser Ser Ser Leu His Pro Leu Thr Pro Thr Pro Gln Thr
705                 710                 715                 720
Leu Ser Tyr Arg Val Lys Glu Glu Leu Cys Pro Ser Ser Ser Leu Pro
                725                 730                 735
Ser Pro Lys Gly Gln Gln Pro Arg Val Glu Val Arg Leu Arg Glu Gly
            740                 745                 750
Lys Ala Val Asn Ile His Met Phe Cys Gly Arg Arg Pro Gly Leu Leu
        755                 760                 765
Leu Ser Thr Met Arg Ala Leu Asp Asn Leu Gly Leu Asp Val Gln Gln
    770                 775                 780
Ala Val Ile Ser Cys Phe Asn Gly Phe Ala Leu Asp Val Phe Arg Ala
```

```
785                 790                 795                 800
Glu Gln Cys Gln Glu Asp His Asp Val Leu Pro Glu Gln Ile Lys Ala
                805                 810                 815
Val Leu Leu Asp Thr Ala Gly Tyr Ala Gly Leu Val
            820                 825

<210>  287
<211>  247
<212>  PRT
<213>  Arabidopsis thaliana

<400>  287
Met Glu Asp Ile Val Asp Gln Glu Leu Ser Asn Tyr Trp Glu Pro Ser
1               5                   10                  15
Ser Phe Leu Gln Asn Glu Asp Phe Glu Tyr Asp Ser Trp Pro Leu Glu
            20                  25                  30
Glu Ala Ile Ser Gly Ser Tyr Asp Ser Ser Ser Pro Asp Gly Ala Ala
        35                  40                  45
Ser Ser Pro Ala Ser Lys Asn Ile Val Ser Glu Arg Asn Arg Arg Gln
    50                  55                  60
Lys Leu Asn Gln Arg Leu Phe Ala Leu Arg Ser Val Val Pro Asn Ile
65                  70                  75                  80
Thr Lys Met Asp Lys Ala Ser Ile Ile Lys Asp Ala Ile Ser Tyr Ile
                85                  90                  95
Glu Gly Leu Gln Tyr Glu Glu Lys Lys Leu Glu Ala Glu Ile Arg Glu
            100                 105                 110
Leu Glu Ser Thr Pro Lys Ser Ser Leu Ser Phe Ser Lys Asp Phe Asp
        115                 120                 125
Arg Asp Leu Leu Val Pro Val Thr Ser Lys Lys Met Lys Gln Leu Asp
    130                 135                 140
Ser Gly Ser Ser Thr Ser Leu Ile Glu Val Leu Glu Leu Lys Val Thr
145                 150                 155                 160
Phe Met Gly Glu Arg Thr Met Val Val Ser Val Thr Cys Asn Lys Arg
                165                 170                 175
Thr Asp Thr Met Val Lys Leu Cys Glu Val Phe Glu Ser Leu Asn Leu
            180                 185                 190
Lys Ile Leu Thr Ser Asn Leu Thr Ser Phe Ser Gly Met Ile Phe His
        195                 200                 205
Thr Val Phe Ile Glu Ala Asp Glu Glu Glu Gln Glu Val Leu Arg Leu
    210                 215                 220
Lys Ile Glu Thr Gly Ile Gly Ala Tyr Asn Glu Thr Gln Ser Pro Thr
225                 230                 235                 240
Leu Ser Ile Asp Ser Leu Tyr
                245

<210>  288
<211>  379
<212>  PRT
<213>  Arabidopsis thaliana

<400>  288
Met Asp Glu Ser Ser Ile Ile Pro Ala Glu Lys Val Ala Gly Ala Glu
1               5                   10                  15
Lys Lys Glu Leu Gln Gly Leu Leu Lys Thr Ala Val Gln Ser Val Asp
            20                  25                  30
Trp Thr Tyr Ser Val Phe Trp Gln Phe Cys Pro Gln Gln Arg Val Leu
        35                  40                  45
Val Trp Gly Asn Gly Tyr Tyr Asn Gly Ala Ile Lys Thr Arg Lys Thr
    50                  55                  60
```

```
Thr Gln Pro Ala Glu Val Thr Ala Glu Glu Ala Ala Leu Glu Arg Ser
65                  70                  75                  80
Gln Gln Leu Arg Glu Leu Tyr Glu Thr Leu Leu Ala Gly Glu Ser Thr
                85                  90                  95
Ser Glu Ala Arg Ala Cys Thr Ala Leu Ser Pro Glu Asp Leu Thr Glu
            100                 105                 110
Thr Glu Trp Phe Tyr Leu Met Cys Val Ser Phe Ser Phe Pro Pro Pro
        115                 120                 125
Ser Gly Met Pro Gly Lys Ala Tyr Ala Arg Arg Lys His Val Trp Leu
    130                 135                 140
Ser Gly Ala Asn Glu Val Asp Ser Lys Thr Phe Ser Arg Ala Ile Leu
145                 150                 155                 160
Ala Lys Thr Val Val Cys Ile Pro Met Leu Asp Gly Val Val Glu Leu
                165                 170                 175
Gly Thr Thr Lys Lys Asn Gly Lys Glu His Gln Gln Val Lys Thr Ala
            180                 185                 190
Pro Ser Ser Gln Trp Val Leu Lys Gln Met Ile Phe Arg Val Pro Phe
        195                 200                 205
Leu His Asp Asn Thr Lys Asp Lys Arg Leu Pro Arg Glu Asp Leu Ser
    210                 215                 220
His Val Val Ala Glu Arg Arg Arg Arg Glu Lys Leu Asn Glu Lys Phe
225                 230                 235                 240
Ile Thr Leu Arg Ser Met Val Pro Phe Val Thr Lys Met Asp Lys Val
                245                 250                 255
Ser Ile Leu Gly Asp Thr Ile Ala Tyr Val Asn His Leu Arg Lys Arg
            260                 265                 270
Val His Glu Leu Glu Asn Thr His His Glu Gln Gln His Lys Arg Thr
        275                 280                 285
Arg Thr Cys Lys Arg Lys Thr Ser Glu Glu Val Glu Val Ser Ile Ile
    290                 295                 300
Glu Asn Asp Val Leu Leu Glu Met Arg Cys Glu Tyr Arg Asp Gly Leu
305                 310                 315                 320
Leu Leu Asp Ile Leu Gln Val Leu His Glu Leu Gly Ile Glu Thr Thr
                325                 330                 335
Ala Val His Thr Ser Val Asn Asp His Asp Phe Glu Ala Glu Ile Arg
            340                 345                 350
Ala Lys Val Arg Gly Lys Lys Ala Ser Ile Ala Glu Val Lys Arg Ala
        355                 360                 365
Ile His Gln Val Ile Ile His Asp Thr Asn Leu
    370                 375

<210>  289
<211>  261
<212>  PRT
<213>  Arabidopsis thaliana

<400>  289
Met Ala Asn Leu Ser Ser Asp Phe Gln Thr Phe Thr Met Asp Asp Pro
1               5                   10                  15
Ile Arg Gln Leu Ala Glu Leu Ser Asn Thr Leu His His Phe Gln Thr
            20                  25                  30
Phe Pro Pro Pro Phe Ser Ser Ser Leu Asp Ser Leu Phe Phe His Asn
        35                  40                  45
Gln Phe Pro Asp His Phe Pro Gly Lys Ser Leu Glu Asn Asn Phe His
    50                  55                  60
Gln Gly Ile Phe Phe Pro Ser Asn Ile Gln Asn Asn Glu Glu Ser Ser
65                  70                  75                  80
Ser Gln Phe Asp Thr Lys Lys Arg Lys Ser Leu Met Glu Ala Val Ser
                85                  90                  95
```

```
Thr Ser Glu Asn Ser Val Ser Asp Gln Thr Leu Ser Thr Ser Ser Ala
            100                 105                 110
Gln Val Ser Ile Asn Gly Asn Ile Ser Thr Lys Asn Asn Ser Ser Arg
        115                 120                 125
Arg Gly Lys Arg Ser Lys Asn Arg Glu Glu Glu Lys Glu Arg Glu Val
    130                 135                 140
Val His Val Arg Ala Arg Arg Gly Gln Ala Thr Asp Ser His Ser Ile
145                 150                 155                 160
Ala Glu Arg Val Arg Arg Gly Lys Ile Asn Glu Arg Leu Lys Cys Leu
                165                 170                 175
Gln Asp Ile Val Pro Gly Cys Tyr Lys Thr Met Gly Met Ala Thr Met
            180                 185                 190
Leu Asp Glu Ile Ile Asn Tyr Val Gln Ser Leu Gln Asn Gln Val Glu
        195                 200                 205
Phe Leu Ser Met Lys Leu Thr Ala Ala Ser Ser Tyr Tyr Asp Phe Asn
    210                 215                 220
Ser Glu Thr Asp Ala Val Glu Ser Met Gln Lys Ala Lys Ala Arg Glu
225                 230                 235                 240
Ala Val Glu Met Gly Gln Gly Arg Asp Gly Ser Ser Val Phe His Ser
                245                 250                 255
Ser Ser Trp Thr Leu
            260

<210>  290
<211>  358
<212>  PRT
<213>  Arabidopsis thaliana

<400>  290
Met Glu Arg Phe Gln Gly His Ile Asn Pro Cys Phe Phe Asp Arg Lys
1               5                   10                  15
Pro Asp Val Arg Ser Leu Glu Val Gln Gly Phe Ala Glu Ala Gln Ser
            20                  25                  30
Phe Ala Phe Lys Glu Lys Glu Glu Glu Ser Leu Gln Asp Thr Val Pro
        35                  40                  45
Phe Leu Gln Met Leu Gln Ser Glu Asp Pro Ser Ser Phe Phe Ser Ile
    50                  55                  60
Lys Glu Pro Asn Phe Leu Thr Leu Leu Ser Leu Gln Thr Leu Lys Glu
65                  70                  75                  80
Pro Trp Glu Leu Glu Arg Tyr Leu Ser Leu Glu Asp Ser Gln Phe His
                85                  90                  95
Ser Pro Val Gln Ser Glu Thr Asn Arg Phe Met Glu Gly Ala Asn Gln
            100                 105                 110
Ala Val Ser Ser Gln Glu Ile Pro Phe Ser Gln Ala Asn Met Thr Leu
        115                 120                 125
Pro Ser Ser Thr Ser Ser Pro Leu Ser Ala His Ser Arg Arg Lys Arg
    130                 135                 140
Lys Ile Asn His Leu Leu Pro Gln Glu Met Thr Arg Glu Lys Arg Lys
145                 150                 155                 160
Arg Arg Lys Thr Lys Pro Ser Lys Asn Asn Glu Glu Ile Glu Asn Gln
                165                 170                 175
Arg Ile Asn His Ile Ala Val Glu Arg Asn Arg Arg Arg Gln Met Asn
            180                 185                 190
Glu His Ile Asn Ser Leu Arg Ala Leu Leu Pro Pro Ser Tyr Ile Gln
        195                 200                 205
Arg Gly Asp Gln Ala Ser Ile Val Gly Gly Ala Ile Asn Tyr Val Lys
    210                 215                 220
Val Leu Glu Gln Ile Ile Gln Ser Leu Glu Ser Gln Lys Arg Thr Gln
225                 230                 235                 240
```

Gln Gln Ser Asn Ser Glu Val Val Glu Asn Ala Leu Asn His Leu Ser
                245                 250                 255
Gly Ile Ser Ser Asn Asp Leu Trp Thr Thr Leu Glu Asp Gln Thr Cys
            260                 265                 270
Ile Pro Lys Ile Glu Ala Thr Val Ile Gln Asn His Val Ser Leu Lys
        275                 280                 285
Val Gln Cys Glu Lys Lys Gln Gly Gln Leu Leu Lys Gly Ile Ile Ser
    290                 295                 300
Leu Glu Lys Leu Lys Leu Thr Val Leu His Leu Asn Ile Thr Thr Ser
305                 310                 315                 320
Ser His Ser Ser Val Ser Tyr Ser Phe Asn Leu Lys Met Glu Asp Glu
                325                 330                 335
Cys Asp Leu Glu Ser Ala Asp Glu Ile Thr Ala Ala Val His Arg Ile
            340                 345                 350
Phe Asp Ile Pro Thr Ile
        355

<210> 291
<211> 328
<212> PRT
<213> Arabidopsis thaliana

<400> 291
Met Glu Ala Met Gly Glu Trp Ser Thr Gly Leu Gly Gly Ile Tyr Thr
1               5                   10                  15
Glu Glu Ala Asp Phe Met Asn Gln Leu Leu Ala Ser Tyr Glu Gln Pro
            20                  25                  30
Cys Gly Gly Ser Ser Ser Glu Thr Thr Ala Thr Leu Thr Ala Tyr His
        35                  40                  45
His Gln Gly Ser Gln Trp Asn Gly Gly Phe Cys Phe Ser Gln Glu Ser
    50                  55                  60
Ser Ser Tyr Ser Gly Tyr Cys Ala Ala Met Pro Arg Gln Glu Glu Asp
65                  70                  75                  80
Asn Asn Gly Met Glu Asp Ala Thr Ile Asn Thr Asn Leu Tyr Leu Val
                85                  90                  95
Gly Glu Glu Thr Ser Glu Cys Asp Ala Thr Glu Tyr Ser Gly Lys Ser
            100                 105                 110
Leu Leu Pro Leu Glu Thr Val Ala Glu Asn His Asp His Ser Met Leu
        115                 120                 125
Gln Pro Glu Asn Ser Leu Thr Thr Thr Thr Asp Glu Lys Met Phe Asn
    130                 135                 140
Gln Cys Glu Ser Ser Lys Lys Arg Thr Arg Ala Thr Thr Thr Asp Lys
145                 150                 155                 160
Asn Lys Arg Ala Asn Lys Ala Arg Arg Ser Gln Lys Cys Val Glu Met
                165                 170                 175
Ser Gly Glu Asn Glu Asn Ser Gly Glu Glu Glu Tyr Thr Glu Lys Ala
            180                 185                 190
Ala Gly Lys Arg Lys Thr Lys Pro Leu Lys Pro Gln Lys Thr Cys Cys
        195                 200                 205
Ser Asp Asp Glu Ser Asn Gly Gly Asp Thr Phe Leu Ser Lys Glu Asp
    210                 215                 220
Gly Glu Asp Ser Lys Ala Leu Asn Leu Asn Gly Lys Thr Arg Ala Ser
225                 230                 235                 240
Arg Gly Ala Ala Thr Asp Pro Gln Ser Leu Tyr Ala Arg Lys Arg Arg
                245                 250                 255
Glu Arg Ile Asn Glu Arg Leu Arg Ile Leu Gln His Leu Val Pro Asn
            260                 265                 270
Gly Thr Lys Val Asp Ile Ser Thr Met Leu Glu Glu Ala Val Gln Tyr
        275                 280                 285

```
Val Lys Phe Leu Gln Leu Gln Ile Lys Leu Leu Ser Ser Asp Asp Leu
    290                 295                 300
Trp Met Tyr Ala Pro Ile Ala Tyr Asn Gly Met Asp Ile Gly Leu Asp
305                 310                 315                 320
Leu Lys Leu Asn Ala Leu Thr Arg
                325
```

<210> 292
<211> 373
<212> PRT
<213> Arabidopsis thaliana

<400> 292

```
Met Glu Gly Leu Glu Ser Val Tyr Ala Gln Ala Met Tyr Gly Met Thr
1               5                   10                  15
Arg Glu Ser Lys Ile Met Glu His Gln Gly Ser Asp Leu Ile Trp Gly
            20                  25                  30
Gly Asn Glu Leu Met Ala Arg Glu Leu Cys Ser Ser Ser Ser Tyr His
        35                  40                  45
His Gln Leu Ile Asn Pro Asn Leu Ser Ser Cys Phe Met Ser Asp Leu
    50                  55                  60
Gly Val Leu Gly Glu Ile Gln Gln Gln Gln His Val Gly Asn Arg Ala
65                  70                  75                  80
Ser Ser Ile Asp Pro Ser Ser Leu Asp Cys Leu Leu Ser Ala Thr Ser
                85                  90                  95
Asn Ser Asn Asn Thr Ser Thr Glu Asp Asp Glu Gly Ile Ser Val Leu
            100                 105                 110
Phe Ser Asp Cys Gln Thr Leu Trp Ser Phe Gly Gly Val Ser Ser Ala
        115                 120                 125
Glu Ser Glu Asn Arg Glu Ile Thr Thr Glu Thr Thr Thr Thr Ile Lys
    130                 135                 140
Pro Lys Pro Leu Lys Arg Asn Arg Gly Gly Asp Gly Gly Thr Thr Glu
145                 150                 155                 160
Thr Thr Thr Thr Thr Thr Lys Pro Lys Ser Leu Lys Arg Asn Arg Gly
                165                 170                 175
Asp Glu Thr Gly Ser His Phe Ser Leu Val His Pro Gln Asp Asp Ser
            180                 185                 190
Glu Lys Gly Gly Phe Lys Leu Ile Tyr Asp Glu Asn Gln Ser Lys Ser
        195                 200                 205
Lys Lys Pro Arg Thr Glu Lys Glu Arg Gly Gly Ser Ser Asn Ile Ser
    210                 215                 220
Phe Gln His Ser Thr Cys Leu Ser Asp Asn Val Glu Pro Asp Ala Glu
225                 230                 235                 240
Ala Ile Ala Gln Met Lys Glu Met Ile Tyr Arg Ala Ala Ala Phe Arg
                245                 250                 255
Pro Val Asn Phe Gly Leu Glu Ile Val Glu Lys Pro Lys Arg Lys Asn
            260                 265                 270
Val Lys Ile Ser Thr Asp Pro Gln Thr Val Ala Ala Arg Gln Arg Arg
        275                 280                 285
Glu Arg Ile Ser Glu Lys Ile Arg Val Leu Gln Thr Leu Val Pro Gly
    290                 295                 300
Gly Thr Lys Met Asp Thr Ala Ser Met Leu Asp Glu Ala Ala Asn Tyr
305                 310                 315                 320
Leu Lys Phe Leu Arg Ala Gln Val Lys Ala Leu Glu Asn Leu Arg Pro
                325                 330                 335
Lys Leu Asp Gln Thr Asn Leu Ser Phe Ser Ser Ala Pro Thr Ser Phe
            340                 345                 350
Pro Leu Phe His Pro Ser Phe Leu Pro Leu Gln Asn Pro Asn Gln Ile
        355                 360                 365
```

His His Pro Glu Cys
    370

<210> 293
<211> 247
<212> PRT
<213> Arabidopsis thaliana

<400> 293
```
Met Asp Asn Phe Phe Leu Gly Leu Ser Cys Gln Glu Glu Asn Asn Phe
1               5                   10                  15
Trp Asp Leu Ile Val Ala Asp Ile Ser Gly Asp Arg Ser Val Ser Val
            20                  25                  30
Pro Ile Arg Ser Ala Phe Arg Ser Tyr Met Lys Asp Thr Glu Leu Arg
        35                  40                  45
Met Met Ser Pro Lys Ile Ser Ser Ser Lys Val Asn Val Lys Lys Arg
    50                  55                  60
Met Val Asn Leu Leu Arg Lys Asn Trp Glu Glu Lys Lys Asn Thr Val
65                  70                  75                  80
Ala Pro Glu Lys Glu Arg Ser Arg Arg His Met Leu Lys Glu Arg Thr
                85                  90                  95
Arg Arg Glu Lys Gln Lys Gln Ser Tyr Leu Ala Leu His Ser Leu Leu
            100                 105                 110
Pro Phe Ala Thr Lys Asn Asp Lys Asn Ser Ile Val Glu Lys Ala Val
        115                 120                 125
Asp Glu Ile Ala Lys Leu Gln Arg Leu Lys Lys Glu Leu Val Arg Arg
    130                 135                 140
Ile Lys Val Ile Glu Glu Lys Ser Ala Lys Asp Gly His Asp Glu Met
145                 150                 155                 160
Ser Glu Thr Lys Val Arg Val Asn Leu Lys Glu Pro Leu Ser Gly Leu
                165                 170                 175
Asp Ser Met Leu Glu Ala Leu His Tyr Leu Lys Ser Met Gly Thr Lys
            180                 185                 190
Leu Lys Thr Val His Ala Asn Phe Ser Pro Gln Glu Phe Ser Ala Thr
        195                 200                 205
Met Thr Ile Glu Thr Gln Ile Arg Gly Glu Glu Val Glu Lys Arg Val
    210                 215                 220
Glu Arg Arg Leu Gln Glu Thr Glu Trp Lys Leu Leu Phe Leu Pro Glu
225                 230                 235                 240
Ala Ser Phe Tyr Lys Asp Tyr
                245
```

<210> 294
<211> 311
<212> PRT
<213> Arabidopsis thaliana

<400> 294
```
Met Arg Thr Gly Lys Gly Asn Gln Glu Glu Glu Asp Tyr Gly Glu Glu
1               5                   10                  15
Asp Phe Asn Ser Lys Arg Glu Gly Pro Ser Ser Asn Thr Thr Val His
            20                  25                  30
Ser Asn Arg Asp Ser Lys Glu Asn Asp Lys Ala Ser Ala Ile Arg Ser
        35                  40                  45
Lys His Ser Val Thr Glu Gln Arg Arg Arg Ser Lys Ile Asn Glu Arg
    50                  55                  60
Phe Gln Ile Leu Arg Glu Leu Ile Pro Asn Ser Glu Gln Lys Arg Asp
65                  70                  75                  80
Thr Ala Ser Phe Leu Leu Glu Val Ile Asp Tyr Val Gln Tyr Leu Gln
```

```
                85                  90                  95
Glu Lys Val Gln Lys Tyr Glu Gly Ser Tyr Pro Gly Trp Ser Gln Glu
            100                 105                 110
Pro Thr Lys Leu Thr Pro Trp Arg Asn Asn His Trp Arg Val Gln Ser
        115                 120                 125
Leu Gly Asn His Pro Val Ala Ile Asn Asn Gly Ser Gly Pro Gly Ile
    130                 135                 140
Pro Phe Pro Gly Lys Phe Glu Asp Asn Thr Val Thr Ser Thr Pro Ala
145                 150                 155                 160
Ile Ile Ala Glu Pro Gln Ile Pro Ile Glu Ser Asp Lys Ala Arg Ala
                165                 170                 175
Ile Thr Gly Ile Ser Ile Glu Ser Gln Pro Glu Leu Asp Asp Lys Gly
            180                 185                 190
Leu Pro Pro Leu Gln Pro Ile Leu Pro Met Val Gln Gly Glu Gln Ala
        195                 200                 205
Asn Glu Cys Pro Ala Thr Ser Asp Gly Leu Gly Gln Ser Asn Asp Leu
    210                 215                 220
Val Ile Glu Gly Gly Thr Ile Ser Ile Ser Ser Ala Tyr Ser His Glu
225                 230                 235                 240
Leu Leu Ser Ser Leu Thr Gln Ala Leu Gln Asn Ala Gly Ile Asp Leu
                245                 250                 255
Ser Gln Ala Lys Leu Ser Val Gln Ile Asp Leu Gly Lys Arg Ala Asn
            260                 265                 270
Gln Gly Leu Thr His Glu Glu Pro Ser Ser Lys Asn Pro Leu Ser Tyr
        275                 280                 285
Asp Thr Gln Gly Arg Asp Ser Ser Val Glu Glu Glu Ser Glu His Ser
    290                 295                 300
His Lys Arg Met Lys Thr Leu
305                 310
```

<210> 295
<211> 162
<212> PRT
<213> Arabidopsis thaliana

<400> 295

```
Met Glu Arg Asn Asn Arg Asn Glu Gly Thr His Glu Glu Glu Gln Cys
1               5                   10                  15
Ser Leu Ser Asp Ile Ile Tyr Ser Phe Cys Ser Glu Asn His Ser Glu
            20                  25                  30
Leu Asn Pro Leu Gln Glu Ile Phe Gly Val Thr Lys Asn Asn Asp His
        35                  40                  45
Glu Lys His Asp Glu Glu Pro Asp Glu Glu Ser Tyr Arg Met Ala Lys
    50                  55                  60
Arg Gln Arg Ser Met Glu Tyr Arg Met Met Met Glu Lys Pro Asp Leu
65                  70                  75                  80
Ala Ser Lys Leu Glu Asn Ile Ile Glu Tyr Ile Lys Ser Leu Lys Tyr
                85                  90                  95
Gln Val Asp Val Met Ser Met Ala Tyr Thr Thr Thr Pro Val Tyr Thr
            100                 105                 110
Pro Pro Phe Tyr Ala Ala Ala Gln Ala Pro Cys Met Ser Pro Trp Gly
        115                 120                 125
Tyr Tyr Thr Pro Gly Val Pro Met Met Pro Gln Gln Asn Met Thr Tyr
    130                 135                 140
Ile Pro Gln Tyr Pro Gln Val Tyr Gly Thr Val Pro Pro Asn Gln Thr
145                 150                 155                 160
Gln Pro
```

<210> 296
<211> 226
<212> PRT
<213> Arabidopsis thaliana

<400> 296
Met Val Ser Pro Glu Asn Thr Asn Trp Leu Ser Asp Tyr Pro Leu Ile
1 5 10 15
Glu Gly Ala Phe Ser Asp Gln Asn Pro Thr Phe Pro Trp Gln Ile Asp
20 25 30
Gly Ser Ala Thr Val Ser Val Glu Val Asp Gly Phe Leu Cys Asp Ala
35 40 45
Asp Val Ile Lys Glu Pro Ser Ser Arg Lys Arg Ile Lys Thr Glu Ser
50 55 60
Cys Thr Gly Ser Asn Ser Lys Ala Cys Arg Glu Lys Gln Arg Arg Asp
65 70 75 80
Arg Leu Asn Asp Lys Phe Thr Glu Leu Ser Ser Val Leu Glu Pro Gly
85 90 95
Arg Thr Pro Lys Thr Asp Lys Val Ala Ile Ile Asn Asp Ala Ile Arg
100 105 110
Met Val Asn Gln Ala Arg Asp Glu Ala Gln Lys Leu Lys Asp Leu Asn
115 120 125
Ser Ser Leu Gln Glu Lys Ile Lys Glu Leu Lys Asp Glu Lys Asn Glu
130 135 140
Leu Arg Asp Glu Lys Gln Lys Leu Lys Val Glu Lys Glu Arg Ile Asp
145 150 155 160
Gln Gln Leu Lys Ala Ile Lys Thr Gln Pro Gln Pro Gln Pro Cys Phe
165 170 175
Leu Pro Asn Pro Gln Thr Leu Ser Gln Ala Gln Ala Pro Gly Ser Lys
180 185 190
Leu Val Pro Phe Thr Thr Tyr Pro Gly Phe Ala Met Trp Gln Phe Met
195 200 205
Pro Pro Ala Ala Val Asp Thr Ser Gln Asp His Val Leu Arg Pro Pro
210 215 220
Val Ala
225

<210> 297
<211> 252
<212> PRT
<213> Arabidopsis thaliana

<400> 297
Met Glu Thr Pro Ala Tyr Asp Phe Asp Ser Leu Thr Asp Leu Pro Pro
1 5 10 15
Leu Pro Pro Ser Asp Phe Thr Pro Ser Asn Ala Phe Thr Phe Pro Asp
20 25 30
His Asn Leu Asp Phe Ser Phe Leu Asp Ser Thr Leu Ser Leu Leu Asn
35 40 45
Arg His His Leu Ser Glu Ser Thr Arg Leu Glu Gln Ile Phe Tyr Asp
50 55 60
Ser Thr His Thr Gln Leu Phe His Asn Asp Asp Thr Thr Thr Thr Thr
65 70 75 80
Thr Pro Phe Leu His Leu Pro Asp Leu Lys Ser Ile Asp Ala Val Glu
85 90 95
Glu Pro Thr Thr Met Lys Leu Phe Pro Ser Leu Ser Pro Pro Leu Pro
100 105 110
Ala Ala Lys Arg Gln Lys Leu Asn Ser Thr Ser Ser Ser Thr Thr Ser
115 120 125

```
Gly Ser Pro Thr Ala Ser Asn Asp Gly Gly Ile Ile Thr Lys Arg Arg
    130                 135                 140
Lys Ile Ser Asp Lys Ile Arg Ser Leu Glu Lys Leu Met Pro Trp Glu
145                 150                 155                 160
Arg Lys Met Asn Leu Ala Met Thr Leu Glu Glu Ser His Lys Tyr Ile
                165                 170                 175
Lys Phe Leu Gln Ser Gln Ile Ala Ser Leu Arg Trp Met Pro Leu Glu
            180                 185                 190
Ser Val Tyr Asn Thr Ala Gly Glu Val Gly Glu Thr Asp Leu Leu Lys
        195                 200                 205
Ser Leu Thr Arg Gln Gln Ile Leu Gln Val Leu Ala Asn Ser Pro Gly
    210                 215                 220
Ser Arg Asn Val Leu Ser Ser Arg Gly Val Cys Val Phe Ser Tyr Glu
225                 230                 235                 240
Gln Leu Leu Ser Leu Lys Thr Met Ser Arg Asn Leu
                245                 250

<210>  298
<211>  454
<212>  PRT
<213>  Arabidopsis thaliana

<400>  298
Met Gly Asp His His Asp Phe Ile Asn Ser Gly Ser Trp Trp Lys Val
1               5                   10                  15
Ser Ser Ser Ser Ser Pro Ser Ser Ser Ser Ser Met Arg Ala Ser Ser
            20                  25                  30
Ile Glu Ser Gly Gly Ser Ala Val Phe His Asp Lys Leu His His His
        35                  40                  45
Ser Leu Ala Thr Asp His His Leu Gln Met Ile Gly Leu Gly Leu Ser
    50                  55                  60
Ser Gln Ser Pro Val Asp Gln Trp Asn Gln Ser Leu Leu Arg Gly Asp
65                  70                  75                  80
Ser Lys Ala Glu Thr Ser Phe Gly Val Met Leu Gln Glu Asn Leu Asn
                85                  90                  95
Leu Asp Ala Thr Ser Asn Ala Asn Ala Asn Thr Thr Ser Ser Thr Ser
            100                 105                 110
Ser Tyr Gln Leu Gln Glu Ser Asp Ser Ser His His His Gln Ala Leu
        115                 120                 125
Trp Arg Asp Pro Gln Ser Asp Phe Lys Pro Gln Ile Leu Thr Ser Gly
    130                 135                 140
Gly Asn Arg Gly Phe Phe Leu Asp His Gln Phe Ser Pro His Gly Ser
145                 150                 155                 160
Ser Ser Thr Asp Ser Ser Thr Val Thr Cys Gln Gly Phe Ala Val Asp
                165                 170                 175
Asn Ser Ser Asn Ala Met Tyr Ala Ala Thr Thr Thr Thr Pro Asn Ser
            180                 185                 190
Ser Ser Gly Met Phe His His Gln Gln Ala Gly Gly Phe Gly Ser Ser
        195                 200                 205
Asp Gln Gln Pro Ser Arg Asn His Gln Gln Ser Ser Leu Gly Tyr Ser
    210                 215                 220
Gln Phe Gly Ser Ser Thr Gly Asn Tyr Asp Gln Met Ala Ser Ala Leu
225                 230                 235                 240
Pro Ser Thr Trp Phe Leu Arg Ser Ser Pro Pro Pro Lys Pro His Ser
                245                 250                 255
Pro Leu Arg Phe Ser Asn Asn Ala Thr Phe Trp Asn Pro Ala Ala Ala
            260                 265                 270
Gly Asn Ala Gly Ala Pro Pro Pro His Asp Ala Ser Ser Asn Phe Phe
        275                 280                 285
```

```
Pro Ala Leu Gln Pro Pro Gln Ile His Pro Gln Ser Phe Asp Glu Gln
    290                 295                 300
Pro Lys Asn Ile Ser Glu Ile Arg Asp Ser Ser Ser Asn Glu Val Lys
305                 310                 315                 320
Arg Gly Gly Asn Asp His Gln Pro Ala Ala Lys Arg Ala Lys Ser Glu
                325                 330                 335
Ala Ala Ser Pro Ser Pro Ala Phe Lys Arg Lys Glu Lys Met Gly Asp
            340                 345                 350
Arg Ile Ala Ala Leu Gln Gln Leu Val Ser Pro Phe Gly Lys Thr Asp
        355                 360                 365
Ala Ala Ser Val Leu Ser Glu Ala Ile Glu Tyr Ile Lys Phe Leu His
    370                 375                 380
Gln Gln Val Ser Ala Leu Ser Asn Pro Tyr Met Lys Ser Gly Ala Ser
385                 390                 395                 400
Leu Gln His Gln Gln Ser Asp His Ser Thr Glu Leu Glu Val Ser Glu
                405                 410                 415
Glu Pro Asp Leu Arg Ser Arg Gly Leu Cys Leu Val Pro Val Ser Ser
            420                 425                 430
Thr Phe Pro Val Thr His Asp Thr Thr Val Asp Phe Trp Thr Pro Thr
        435                 440                 445
Phe Gly Gly Thr Phe Arg
    450

<210>  299
<211>  259
<212>  PRT
<213>  Arabidopsis thaliana

<400>  299
Met Asp Cys Val Pro Ser Leu Phe Met Pro Asp Ser Thr Tyr Glu Asp
1               5                   10                  15
Gly Leu Leu Phe Ser Asp Ser Phe Leu Leu Ser Pro Phe Ile Ser Tyr
            20                  25                  30
Gln Asn Asn Asp Val Phe His Ser Ile Thr Asn Lys Ile Gly Gly Ser
        35                  40                  45
Asn Lys Lys Arg Ser Leu Cys Asp Ile Thr Tyr Gly Ala Asn Glu Ala
    50                  55                  60
Asn Lys Asn Asp Asp Asp Arg Glu Ser Lys Lys Met Lys His Arg Asp
65                  70                  75                  80
Ile Glu Arg Gln Arg Arg Gln Glu Val Ser Ser Leu Phe Lys Arg Leu
                85                  90                  95
Arg Thr Leu Leu Pro Phe Gln Tyr Ile Gln Gly Lys Arg Ser Thr Ser
            100                 105                 110
Asp His Ile Val Gln Ala Val Asn Tyr Ile Lys Asp Leu Gln Ile Lys
        115                 120                 125
Ile Lys Glu Leu Asn Glu Lys Arg Asn Arg Val Lys Lys Val Ile Ser
    130                 135                 140
Ala Thr Thr Thr Thr His Ser Ala Ile Glu Glu Cys Thr Ser Ser Leu
145                 150                 155                 160
Ser Ser Ser Ala Ala Ser Thr Leu Ser Ser Ser Cys Ser Cys Val Gly
                165                 170                 175
Asp Lys His Ile Thr Val Val Val Thr Pro Cys Leu Val Gly Val Glu
            180                 185                 190
Ile Ile Ile Ser Cys Cys Leu Gly Arg Asn Lys Ser Cys Leu Ser Ser
        195                 200                 205
Val Leu Gln Met Leu Ala Gln Glu Gln Arg Phe Ser Val Val Ser Cys
    210                 215                 220
Leu Ser Ala Arg Arg Gln Gln Arg Phe Met His Thr Ile Val Ser Gln
225                 230                 235                 240
```

```
Val Glu Asp Gly Lys Gln Ile Asn Ile Leu Glu Leu Lys Asp Lys Ile
                245                 250                 255
Met Thr Met
```

<210> 300
<211> 848
<212> DNA
<213> Solanum lycopersicum

<400> 300

```
atggagaatc taaacggctt agttcctgac atccctatac aagagcagaa agacaatgct     60
actgcttctg aaaatttcgg tcgaagtgtt gatgaaatgg ttgggaaggt tgatcagatt    120
gagcagagac tgaatgaggt tgagcatttc tattccaata ctagtaaaaa acaatcaaac    180
acgccaagag gtggctctat tctgaaagac aaagagaaac agatgtctag ctttagaagg    240
cggcagcagg atgcatcacg tagagaagca gctggttcca ggagaatgca agaacttatg    300
cgccaatttg gtactatatt acgtcagatc actcagcaca agtgggcaga acctttttatg    360
gaacctgtgg atgtcaaggg tcttggatta cacgattatt ttgaggttat tgagaagcct    420
atggacttca gtactatcaa aaacaagatg gaagcaaagg atggttctgg ctataagcat    480
gtccgagaga tatgtgctga cgtgaggcta atatttaaga atgcgatgaa atacaacgag    540
gaaagggatg acgttcatgt aatggcgaaa accttgttgg gaaaattcga ggagaaatgg    600
ttgcagcttt tgcccaaagt tgatgaagag gaaaagcggc gaaaggatga ggaagcagaa    660
gcccaacagg atatgcagct cgctcaagag gctgctcatg caaaaatggc aaaagattta    720
actattgagc ttgatgaggt ggacatgcaa ctggaagaac tcagggactt ggtgcttcag    780
aaatgcagaa agatttcaac ggaagacagg aaacaacttg ggaatgcgct cactaagctg    840
tctcctga                                                             848
```

<210> 301
<211> 378
<212> PRT
<213> Solanum lycopersicum

<400> 301

```
Met Glu Asn Leu Asn Gly Leu Val Pro Asp Ile Pro Ile Gln Glu Gln
1               5                   10                  15
Lys Asp Asn Ala Thr Ala Ser Glu Asn Phe Gly Arg Ser Val Asp Glu
            20                  25                  30
Met Val Gly Lys Val Asp Gln Ile Glu Gln Arg Leu Asn Glu Val Glu
        35                  40                  45
His Phe Tyr Ser Asn Thr Ser Lys Lys Gln Ser Asn Thr Pro Arg Gly
    50                  55                  60
Gly Ser Ile Leu Lys Asp Lys Glu Lys Gln Met Ser Ser Phe Arg Arg
65                  70                  75                  80
Arg Gln Gln Asp Ala Ser Arg Arg Glu Ala Ala Gly Ser Arg Arg Met
                85                  90                  95
Gln Glu Leu Met Arg Gln Phe Gly Thr Ile Leu Arg Gln Ile Thr Gln
            100                 105                 110
His Lys Trp Ala Glu Pro Phe Met Glu Pro Val Asp Val Lys Gly Leu
        115                 120                 125
Gly Leu His Asp Tyr Phe Glu Val Ile Glu Lys Pro Met Asp Phe Ser
    130                 135                 140
Thr Ile Lys Asn Lys Met Glu Ala Lys Asp Gly Ser Gly Tyr Lys His
145                 150                 155                 160
Val Arg Glu Ile Cys Ala Asp Val Arg Leu Ile Phe Lys Asn Ala Met
                165                 170                 175
Lys Tyr Asn Glu Glu Arg Asp Asp Val His Val Met Ala Lys Thr Leu
            180                 185                 190
Leu Gly Lys Phe Glu Glu Lys Trp Leu Gln Leu Leu Pro Lys Val Asp
        195                 200                 205
```

```
Glu Glu Glu Lys Arg Arg Lys Asp Glu Glu Ala Glu Ala Gln Gln Asp
    210                 215                 220
Met Gln Leu Ala Gln Glu Ala Ala His Ala Lys Met Ala Lys Asp Leu
225                 230                 235                 240
Thr Ile Glu Leu Asp Glu Val Asp Met Gln Leu Glu Glu Leu Arg Asp
                245                 250                 255
Leu Val Leu Gln Lys Cys Arg Lys Ile Ser Thr Glu Asp Arg Lys Gln
            260                 265                 270
Leu Gly Asn Ala Leu Thr Lys Leu Ser Pro Asp Asp Leu Asn Lys Ala
        275                 280                 285
Leu Leu Ile Val Ala Gln Asn Asp Pro Thr Phe Gln Pro Thr Ala Thr
    290                 295                 300
Glu Val Glu Leu Asp Met Asn Ala Arg Ser Glu Ser Thr Leu Trp Lys
305                 310                 315                 320
Leu Lys Phe Phe Val Gln Asp Val Leu His Thr Gln Gly Lys Ser Pro
                325                 330                 335
Val Ser Val Lys Thr Asn Asn Ile Asn Thr Ser Asn Leu Leu Asn Asn
            340                 345                 350
Asn Asn Asn Asn Lys Arg Arg Arg Glu Phe Tyr Asp Ala Leu Ala Lys
        355                 360                 365
Ser Ser Gln Lys Lys Ser Lys Lys Pro Ser
    370                 375
```

<210> 302
<211> 56
<212> DNA
<213> Artificial sequence

<220>
<223> primer: prm10548

<400> 302
ggggacaagt ttgtacaaaa aagcaggctt aaacaatgga gaatctaaac ggctta 56

<210> 303
<211> 50
<212> DNA
<213> Artificial sequence

<220>
<223> primer: prm10549

<400> 303
ggggaccact ttgtacaaga aagctgggtg taaaatcagg atggcttttt 50

<210> 304
<211> 31
<212> PRT
<213> Artificial sequence

<220>
<223> motif 3 (part of bromodomain)

<220>
<221> VARIANT
<222> (2)..(2)
<223> / replace = "Gln" / replace = "Glu" / replace = "His" / replace = "Gly"

```
<220>
<221>  VARIANT
<222>  (5)..(5)
<223>  / replace = "Met"

<220>
<221>  VARIANT
<222>  (6)..(6)
<223>  / replace = "Gln" / replace = "Lys" / replace = "Glu" / replace =
       "His"

<220>
<221>  VARIANT
<222>  (9)..(9)
<223>  / replace = "Asn"

<220>
<221>  VARIANT
<222>  (11)..(11)
<223>  / replace = "Val" / replace = "Lys"

<220>
<221>  VARIANT
<222>  (12)..(12)
<223>  / replace = "Thr"

<220>
<221>  VARIANT
<222>  (14)..(14)
<223>  / replace = "Gln" / replace = "His" / replace = "Cys" / replace =
       "Arg"

<220>
<221>  VARIANT
<222>  (15)..(15)
<223>  / replace = "Ile"

<220>
<221>  VARIANT
<222>  (16)..(16)
<223>  / replace = "Pro" / replace = "His" / replace = "Phe" / replace =
       "Asn"

<220>
<221>  VARIANT
<222>  (19)..(19)
<223>  / replace = "His" / replace = "Phe" / replace = "Asn"

<220>
<221>  VARIANT
<222>  (20)..(20)
<223>  / replace = "Lys" / replace = "Asn" / replace = "Glu" / replace =
       "Asp"

<220>
<221>  VARIANT
<222>  (21)..(21)
<223>  / replace = "Val"
```

<220>
<221> VARIANT
<222> (22)..(22)
<223> / replace = "Val"

<220>
<221> VARIANT
<222> (23)..(23)
<223> / replace = "Thr" / replace = "Asp" / replace = "Gln"

<220>
<221> VARIANT
<222> (24)..(24)
<223> / replace = "Gln" / replace = "Arg"

<220>
<221> VARIANT
<222> (28)..(28)
<223> / replace = "Leu"

<220>
<221> VARIANT
<222> (29)..(29)
<223> / replace = "Gly" / replace = "Arg"

<400> 304
```
Ala Trp Pro Phe Leu Asp Pro Val Asp Val Glu Gly Leu Gly Leu Tyr
1               5                   10                  15
Asp Tyr Tyr Gln Ile Ile Glu Lys Pro Met Asp Phe Ser Thr Ile
            20                  25                  30
```

<210> 305
<211> 13
<212> PRT
<213> Artificial sequence

<220>
<223> motif 4

<220>
<221> VARIANT
<222> (2)..(2)
<223> / replace = "Met"

<220>
<221> VARIANT
<222> (5)..(5)
<223> / replace = "Val"

<220>
<221> UNSURE
<222> (7)..(7)
<223> Xaa can be any nautrally occurring amino acid, preferably one of Lys, Thr, Ala, Glu, Ser, Gln or Asn

<220>
<221> VARIANT
<222> (11)..(11)
<223> / replace = "Arg" / replace = "Asn" / replace = "Thr"

<400> 305
Asp Val Arg Leu Ile Phe Xaa Asn Ala Met Lys Tyr Asn
1 5 10

<210> 306
<211> 12
<212> PRT
<213> Artificial sequence

<220>
<223> motif 5

<220>
<221> VARIANT
<222> (2)..(2)
<223> / replace = "Ser"

<220>
<221> VARIANT
<222> (3)..(3)
<223> / replace = "Glu" / replace = "Arg"

<220>
<221> VARIANT
<222> (4)..(4)
<223> / replace = "Phe" / replace = "Ser" / replace = "Lys"

<220>
<221> VARIANT
<222> (6)..(6)
<223> / replace = "Met" / replace = "Ser"

<220>
<221> VARIANT
<222> (7)..(7)
<223> / replace = "Asp" / replace = "Gly" / replace = "Ala"

<220>
<221> VARIANT
<222> (8)..(8)
<223> / replace = "Arg"

<220>
<221> VARIANT
<222> (11)..(11)
<223> / replace = "Glu" / replace = "Asp"

<220>
<221> VARIANT
<222> (12)..(12)
<223> / replace = "Lys"

<400> 306
Met Ala Lys Thr Leu Leu Glu Lys Phe Glu Gly Cys
1 5 10

<210> 307
<211> 5

<212> PRT
<213> Artificial sequence

<220>
<223> motif 6

<220>
<221> VARIANT
<222> (3)..(3)
<223> / replace = "Trp"

<220>
<221> VARIANT
<222> (4)..(4)
<223> / replace = "Lys"

<400> 307
Thr Leu Phe Arg Leu
1 5

<210> 308
<211> 5
<212> PRT
<213> Artificial sequence

<220>
<223> motif 7

<400> 308
Glu Ala Lys Asp Gly
1 5

<210> 309
<211> 5
<212> PRT
<213> Artificial sequence

<220>
<223> motif 8

<220>
<221> VARIANT
<222> (1)..(1)
<223> / replace = "His" / replace = "Lys"

<400> 309
Gln Leu Glu Glu Leu
1 5

<210> 310
<211> 5
<212> PRT
<213> Artificial sequence

<220>
<223> motif 9

<400> 310
Leu Ser Asn Glu Leu

1 5

<210> 311
<211> 6
<212> PRT
<213> Artificial sequence

<220>
<223> motif 10

<220>
<221> VARIANT
<222> (4)..(4)
<223> / replace = "Leu"

<220>
<221> VARIANT
<222> (5)..(5)
<223> / replace = "Leu" / replace = "Met"

<400> 311
Lys Ala Leu Glu Ile Val
1 5

<210> 312
<211> 5
<212> PRT
<213> Artificial sequence

<220>
<223> motif 11

<400> 312
Val Ile Gln Ile Ile
1 5

<210> 313
<211> 2194
<212> DNA
<213> Oryza sativa

<400> 313
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020

```
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                                2194

<210>  314
<211>  1564
<212>  DNA
<213>  Arabidopsis thaliana

<400>  314
agctgctgtc tcttccgtct tcgtcctccg acctctgtct ttcttctgat ccttcgaaaa     60
agctctcgtg agtcactact cttctctaat gtctgtacat gtcaaggaag aacctgtgct    120
tgttccaaac tgcgatgtcg agaatacaga gcttgcagtt ttcaatggaa atggagaaag    180
tgagcttgag aattttggga cttgcgtaga tgagataaca gatagagtga atcagcttga    240
gcaaaaagtt gtggaggtcg aacactttta ctctacaaaa gatggagctg ctcaaactaa    300
cacttctaag agtaactcgg gcgggaagaa gatcgctatt tctcaaccaa acaactcgaa    360
gggtaactcg gccgggaaag aaaaatcaaa gggaaaacat gtttccagcc ctgaccttat    420
gcgtcaattt gcaacaatgt ttcgtcagat tgctcaacat aaatgggcat ggccgttctt    480
ggagcctgtt gatgttaaag gacttggact acatgattat tacaaggtta tagagaagcc    540
aatggacttg ggaacaatta aaaaaaaaat ggagagttcg gagtatagca atgtcaggga    600
gatatatgcc gatgttagac tagttttcaa gaacgcgatg agatacaatg aagaaaaaga    660
agatgtttat gtgatggctg aatctcttct ggagaaattt gaggagaaat ggctcctgat    720
aatgcctaaa cttgtggagg aggaaaagaa acaagtagac gaagaagcgg agaagcatgc    780
caataagcag cttacaatgg aggctgcaca agcggaaatg gctagggatt taagcaacga    840
actatatgag attgatctgc agctggagaa actccgggaa tcagtagttc agagatgcag    900
aaaactgtcc actcaagaaa agaaaggact ttctgcagct ctaggtcgtc tctcacctga    960
ggatctgtct aaggcgttaa aaatggtttc agagagcaac ccgagtttcc cggctggagc   1020
accagaagtc gagcttgaca ttgatgttca gactgacgtt acattgtgga gactgaaggt   1080
gtttgtgcaa gaagcattga aagcagctaa caaaagctct ggaggaacaa acgctcaaaa   1140
caataacaat actggaactg gagagatcaa caagaacaat gcaaaaagac ggagagagat   1200
ctctgatgca atcaataaag cttcaattaa aagagctaag aaagcttgaa tctctctcta   1260
tctcttgatg tgtttattga tacagaagga acaagagttt ggtgaacacc aatctaaatt   1320
agaaactttc cagttccatt gcgcatgttg cttcaaatgt tgagcacaat gtaaaaaaga   1380
atgaatggga aaaaaatctg agctatagag gagatataaa gaagactgct tgagggagaa   1440
attctgacaa atcttctaat aaaatcttca gtttgagaga tctttggtgt gttgaacgtt   1500
tcttcgattt cattacctta aacctcggca cttataattt aaaatcaatt tttaaaaatg   1560
ataa                                                                 1564

<210>  315
<211>  1110
<212>  DNA
<213>  Arabidopsis thaliana
```

<400> 315
atggctgact cagtgccagg tcatgtcgcc ggcggaggtt tacagggttt ctctgtcgac 60
gccgagtgca tcaagcagcg tgtcgacgag gttttacagt gggttgattc gcttgagcat 120
aaactgaaag aagtggagga gttttactcg agcattggtg tctcaaactc tggctctatt 180
ggtaaagata cagaaaaagg gagacatgtt gtggggatta ggaagattca acaagaggct 240
gcgcgtagag aagctgttgc tgctaaaaga atgcaggacc ttatgcgtca atttggaaca 300
atctttcgtc agataactca gcataagtgc gcttggccgt ttatgcatcc tgtcaatgtt 360
gaaggtcttg gtttgcatga ctactttgag gttattgaca agcctatgga cttcagcacg 420
ataaaaaatc aaatggaggc taaggatggt accgggtaca aacatgtcat gcaaatttat 480
gctgatatgc ggctagtgtt tgagaacgca atgaattata atgaagaaac aagtgatgtt 540
tactctatgg cgaaaaagtt actggaaaag tttgaggaga agtgggcaca ctttcttcca 600
aaggttcaag aagaggagaa aatacgagag gaagaggaga agcaagcagc aaaggaggcg 660
ctgctagcaa aagaagcatc tcatatcaaa acaactagag aattggggaa tgagatttgc 720
catgctaatg acgagctgga gaagctaatg cgtaaagttg tggaaagatg caggaaaatc 780
acaattgagg agaagcgtaa tattggattg gcattgttaa aactgtcacc ggatgatcta 840
cagaaagtgt tgggtatagt tgctcaggct aacccgagct ttcaacctag agcagaggaa 900
gttagtattg agatggacat actggacgaa ccaacactat ggaggctaaa gttctttgtg 960
aaggatgcgt tggataatgc aatgaagaag aagaaagaag aggagacaaa gactagagaa 1020
ttgagtggag cacaaaagaa agaggtttcg aagaaacgaa atgcgactac aaagttagct 1080
gagaggaaaa cgaagcggtc acgcatatga 1110

<210> 316
<211> 1415
<212> DNA
<213> Centaurea solstitialis

<400> 316
acattggtct tggatattgg agatgatgga attggaatag agaagatgga ggaaaataat 60
gctgtgattg tggatgatat tgggcaacaa gtagatgatt tgttcactaa ggttgagaag 120
cttgagcaaa gagtaaatga gattgagcag ttctacttga attcaagcaa taaacaatcc 180
agttcttctc agaaaagctt gcttgtgaag gataaagaca aggtaaagca tattccagga 240
tataagaagc accaacagga agcagcacgt agagaggcaa ctgctgctaa gagaatgcag 300
gagattatgc gacaatttgg ctcaattctg cggcagatca cacagcacaa gtgggcctgg 360
ccatttatgc aacctgtaga tgttgaaggt cttggattgc gtgactatta cgaggtgatt 420
gacaggccaa tggacttcag tacgataaaa aatctaatgg aggccaagga tggtactgaa 480
tataaacatg ttcgggatat atgttcagat gtgcggttgg tttttcagaa tgccatgaaa 540
tataatgacg ataaaagtga tgttcatgtg atggccaaaa ctttgttaga gaagtttgaa 600
gagaaatggc ttcagttttt accaagggtt actgaagagg aaaaaagaag ggaggaggag 660
gaagcagaag cacagttgag catacagcgt gcacaggagg cggctcatgc taaaatggct 720
agggatctaa gtaatgagct ttacgaagcg gatatgcact tggaacacct aagagaaact 780
gtggttcaga aatgcaggaa aatgtcgatt gaagataaaa gaaaccttgg gatagctctt 840
actcagttgt ctcccgaaga tcttagcaag gcactcgata ttgtagctca gcataaccca 900
agttttcatt caacagcaat agaggtcgac cttgacatcg atgctcagag tgaatctact 960
ctatggaggt taaagtattt tgtgaaagat gctctccaag gttatgggaa ggatgatgcc 1020
atcattggaa atgaagctaa caacggccac gtgagcccca ccaaaccgac aaacacaacc 1080
cgaaccatcg cctccaaacg gaaaaaacag atatgtaatg ccctggccag gactgcaaag 1140
aaaaggaatc agaagctctc ttcttagaaa tggtgcaccc ataacaaggt tagccatcat 1200
tcttttgggc cgtaattttg tgcaatcctg ggaaacatga gaacatgact gagtaattcg 1260
atgacaaaaa atggtcggtg gctcgagtca cgtttaattt gggcatgaaa aatgggtggt 1320
ggttaaggtt ttgtaagaac atgaggttcc ccactttgat ggtaatcttt ttgttgcctt 1380
gtatggattg tagaagatgt taatgtcatc tttct 1415

<210> 317
<211> 667
<212> DNA
<213> Gossypium hirsutum

<400> 317

```
ctgtcgaggc agtggagtaa gagaagcagt gtagagataa accctagcaa attagaatcg      60
ttctatcgat agatctgctg gtcaaagttt tttccggtgc ttgtttgggt tgattttcgt     120
gtctgcggaa gtggaaacct aaaagaggct atttgaaggt ttcagcttaa cagaatgatt     180
gccgcgggaa gtgttgtgcc aaagctgaag ttgaagatca agttcccttc tcagaagata     240
gaagatcaat cttttgagtg gggccagcaa ctgagtagtt cttttgatgg aaagaagaca     300
tcacctgctg gaaattcaga agagacttca attgctcaca agcgtgaacc agagggggtg     360
aatgtgggtg gtccaccaga gaagaggcgg aagatggacc gttgcacgac tcagcagtgc     420
tctgccctac tgaaagcact gatgaaacat ccagctggct gggtttttaa tcaacctgtg     480
gatcccaaag cattacagat tcctgattat ttttctataa ttaaaaaccc catggatttg     540
ggtacaatta agtccaaact aggaaagaat atttatcttg gcactgatga gtttgtggct     600
gatattagac taacgttttc taatgccatg ctgtataatc cccatttgt ctcgtcctct      660
gcctgat                                                               667
```

<210> 318
<211> 740
<212> DNA
<213> Gossypium raimondii

<400> 318

```
tccaaaaggg catgaggttt atgcgttggc tgaggcagct tcttgcgaga ttcgaggaat      60
tgttccgccc tctgggtttg aaattggaag agcaagaaga tccacaagaa atgaattatt     120
acgaggaaga gttacaagca agttcttggg atcacgggga agctgagaga atgaagaaag     180
attgggaaat tgataaggaa gattccatta acattgcatc aagatctgat aaaattggag     240
gggtttcggg ctctgtgtcg aaccagaatg caccaccatc gcagatgcag ttgcagccgc     300
cagaaaggat ggcttctccg gtgaggcctc caccggttaa gccagtgaag ctgccaaagc     360
cgaaagctaa ggatccaaac aagagggaaa tgacaatgga ggagaagcaa aagttgggga     420
ttggattgca gagcttacca caggagaaga tggataatgt tgttcatatt ataaggaaaa     480
ggaacgggca tttgaggcaa gatggggatg agattgagct cgatatcgag gcaatggata     540
cggagacact gtgggagttg gatcggttcg tcactaatta taagaagatg gtcagcaaga     600
tcaagcgaca agctttgatg gcaaacaatg ttgcatctaa tgacagcaat agggtaagcg     660
ctaaccatat tgtgattgtt ttatatgaat ttgatgattt gtatttgtaa attaattaga     720
gaatgatgga aattacagag                                                 740
```

<210> 319
<211> 784
<212> DNA
<213> Gossypium raimondii

<400> 319

```
ccgactagtc ctttgaaact tgccatttaa ggtctccctt caatcatttt tcatttccca      60
tttcctttat aagcggtttt tcatttcctc aattgaactc ccccctcttc tctgatggaa     120
caggtaatgg catcaattcc tgattttgaa attgctgaaa ctggaatttc gaaagataat     180
agtgcggaag cggagcagtt caaaatccgg gttgatgaaa tctttcaaaa ggttgacgag     240
cttgagcaaa aggtgaatga agtggaacaa ttcaacttga attcaagtaa aaagcaacag     300
agcagttcta aaggaagctc catgggaaag gaacgagata agggaagaca agttccaagc     360
attaaaaagc agcaacaaga tgcgtcgcga cgggaagctg ctgctgcaaa gagaatgcaa     420
gagcttatgc gccaatttgg cacaatagtg cgtcagatta cacagcacaa gtgggcatgg     480
ccgtttatgc aacctgtaga cgtcaaaggt cttggtttac atgattacta tgagattatt     540
gaaaagccaa tggacttcag tacaataaaa aaccaaatgg aggccaagga tggtaccgga     600
tataagaatg ttcgagatat atgtgctgat gtaagattgg ttttcaataa tgcaatgaaa     660
tataatgatg aaggaagtga cgttcatcta atggccaaaa ctttgctgga aaagtttgag     720
gagaaatggc agcttcttct tcctaaagtc actgaaagag gagagaagac tagatgagga     780
ggag                                                                  784
```

<210> 320
<211> 844
<212> DNA
<213> Lactuca saligna

<400> 320
ttccaaccct tcttctctct ccaacgacgg acatcaccgg cgacacccta cggtcagtca 60
ccggagctta gtttgcgtcg gaaaatatat ctactgttcc aaaagtattt caatcgtgga 120
ttgaagtctg aagaatagat ggaaacaatg gatggacccg ttccggaagt tcgaaccgaa 180
ggcgaacccg ctgttgcgga gcaattaggg caaaatgttg atcagatcat cgtgaaggtt 240
gatgagcttg agcaaagatt aaacgaagtt gagcaattct attcaaagcc aggaaaaaaa 300
cagtcaaaca cctctaaagc aagttcagtg atgaaagaaa aagataaaga taaacaaata 360
accagcttta aaagacgcca gctggacgca tcacgcagag aagcagctgc tacaaaaaga 420
atgcaagatc ttatgcgaca gttttctgta ttattacgcc agcacatcat tggccacaag 480
tgggctggac catttatgca acctgtggat gtagtagggc ttggtttgca tgattactat 540
gaggttattg aaaagccaat ggacttcagt acaattaagg ccaaaatgga ggtgaaagat 600
gggacagggt ataataatgt gagagaaata tgtgcagatg tgagattaat ttttaaaaat 660
gctatgaaat ataatgatga aagaaacgat gttcatgtca tggccaaaac tttactcgca 720
aaatttgagg aaaaatggct ccaacttttg ccaaaagttg acgaagagga tgaaagacga 780
aaaaaggaga agcagaacca cagttagata tccaacttgc tcaagaggtt tctcatgcca 840
aaat 844

<210> 321
<211> 1110
<212> DNA
<213> Medicago truncatula

<400> 321
atggaaccat acccacaacg accatctgcc aatgctgatc ggttggaggg tttcaggaat 60
tctgttgacg aatttcgtac ccaagttgac aatcttcaga aacaagtgat tgaagttgag 120
cactactacg aatcctctgg tatttttcaa ggcaacagtt ctagaggtgg ttcagttgta 180
aaagagaagg gtcgggagaa aactcttgct ggcactaaaa caccgctgca agatgcattg 240
cgtacggaaa ccgctgctgg aaagaggatg caggagctga tgcgtcagtt ttccacaata 300
ttacgtcagg caacttttca gatcactcag cacaagtggg cctggccctt tctggaacct 360
gtagatgtag aaggtctcgg gcttcacgac tattatgaga ttattgataa gcccatggac 420
ttcggtacaa taaaaaataa aatggaagct aaggatggca ctggatacaa gaatgtccgg 480
gagatttatg cggatgtgag attgatattt aagaatgcca tgaagtacaa caatgaaaaa 540
catgatgtcc atgtaatggc aaagaccttg atggaaaaat ttgaagataa atggctgcta 600
ctgttaccca aagttgctga agaggaaaag agacaaatag aggaagaagc tcaagtgcag 660
atggacattc atcttgctca ggagacgacc tatgccgata tggctaagga tttaagcaat 720
gagcttaatg aggttggtat acgcttgatg gaattcagag aaaaggtcat tcagaattgc 780
aggaaactat ctaccggaga gaagaaagca cttgggaagg ctatcgccaa attgtctcct 840
gaaaatctcc agagggcatt ggatttagtt gctgagatca acccaagctt tgaatctact 900
gcagacgagg tggtgcttga cattaatgct cagagtgact acacagtctg gaggttgtac 960
cattttgtga aggtgctctc agaaggtcaa caaggcacag ctgttaataa tgataccgag 1020
gagaagagat acagctctag aaaaaggcga gaattttctg atgatcatgc caaaaaccca 1080
tcaaagagta gaaaactatc gacttcctga 1110

<210> 322
<211> 1077
<212> DNA
<213> Medicago truncatula

<400> 322
atgtcacttg ggaagcaagt aaatgatgtt gagcagtttt accaatccac tgatgttcaa 60
cagaatgatt gcaaatacaa aggcagggag aagcctccta ccggatctaa gaaggcgttg 120
aaacgtgctt cagaagatat gcaagcggag atgaggcaca attttaacaa aatattcaat 180
gaggcaagtg cagtgaagct gcttgccatg accaattata tattacttca gcctctatca 240
ttcagctata tttatccggt gttgttaaat agtagctata gtggagtgga atttgaacat 300
atcaccatca tagctaagga taaatgggcg tggccatttt tggatcctgt agatgttgaa 360
ggtcttgggc tgtatgacta ttatcagatc attgagaagc ctatggattt tagtactata 420
aaaataagaa tggaggctaa agatggctct ggttataaga acgtgaggga gatatatgct 480
gatgtaaggc tgatttttaa gaatgcaatg aagtacaatg atgaaaagaa tgatgtccat 540
gtgatggcta agaccttgct ggaaaaattt gagggttgca cttatagaaa taacagtggt 600

```
ttcagccatc ctatgcaata tttttctgag agtgacctat caaaggaaga agcacatgag    660
gaattgaata aaaggcttgc tcaagaagca acttatgcca atatgactag ggaattaagc    720
actgagctgt ctaaggttga tatggctttg agaagtctca aaacaacagc gatttcacag    780
tgcaggtctg taaagagtca ctcgatgaat gatagtttag tgctacatat tatctgtcac    840
gctgtcatcg atttgaattg tctagctaag atcaggaaac tgtcacatcc ggagaaatta    900
atacttgcga atgccttcac caaattgtct cctgataaca tcgtcaaggc attggagata    960
gttaaagaga gcaatccaaa tttcaaagat cgtattgata tggtgaccct tgaccttgat   1020
tctcagagcg actacacatt gtttagattg catatgtttg ttaaaaacac actagaa      1077
```

<210> 323
<211> 1083
<212> DNA
<213> Oryza sativa

<400> 323

```
atggtgccgg gaggtggagt cccacagggg gcggagggag ggagggctat ggcggcggcg     60
gagacggccg ccactgccgc cgccgccggg gagagggccc cggggacgga ggtggacgcg    120
ttccggcgcc aggtcgagga cctcgtctcc aagaccgacc agctggagag gagggtgaat    180
gaagtggtgg gattctacga cgggaagaaa catggcagtg gtggccggaa ggctggtcgc    240
aaggatagca gcctttccaa agggatgcct gatcttatgc gccagtttgg cacgattgtg    300
cgccagatca catctcatga atgggccgag ccatttctca agccggttga tgtggtaggc    360
cttcagcttg atgactatta caagattatc accaaaccta tggatttttc gaccatacaa    420
aagaaaatgg aagggaaaga tgacaacaag tataacaatg tccgagaaat atattcggat    480
gttagattaa tttttgccaa tgcaatgaag tacaatgatg aacgacatga tgttcatata    540
atggcgaaat cattgcttga gaaatttgaa gagaaatggc tccagcttct tcctaaagtt    600
gaaaatgagg aaaggaaaca aaaggatgag gaatccaacg gtgttccaaa agtgaacatt    660
tctccagaag aagctattgc aaaactagca aaagatactg acaatgagct gattgaaatc    720
aacaagcagt tggaggagct tcggcaaatg gtggtccaga aatgccggaa aatgactaca    780
tatgagaaaa gaaaactggg tgcaggtctc tgccacctgt ctccagaaga gcttactaag    840
gcattggaga tggttgcaca agacaaccct agcttcgaag ctaaaggaga cgaactggaa    900
cttgatatgg atgctcagag tgagacaacc ctctggaggt tgaaattctt tgtgagggaa    960
gcattggaac gacaggccaa cgtcgcttct ggcaggactg atgaaaatgc gaaaagaaag   1020
agggagatat gcaatgctct ggccagaact gcttcaaaaa gagtcaagca gcaacctaat   1080
tag                                                                 1083
```

<210> 324
<211> 1522
<212> DNA
<213> Oryza sativa

<400> 324

```
ccgtcgagtc gaggcgccgg tggtttcgcc tttttcgctc gcagacggcc gccggcgacg     60
cgcgtggcgg cgagccctag atatggtgcc gggaggtgga gtcccacagg gggcggaggg    120
agggagggct atggcggcgg cggagacggc cgccactgcc gccgccgccg gggagagggc    180
cccggggacg gaggtggacg cgttccggcg ccaggtcgag gacctcgtct ccaagaccga    240
ccagctggag aggagggtga atgaagtggt gggattctac gacgggaaga aacatggcag    300
tggtggccgg aaggctggtc gcaaggatag cagcctttcc aaagggatgc ctgatcttat    360
gcgccagttt ggcacgattg tgcgccagat cacatctcat gaatgggccg agccatttct    420
caagccggtt gatgtggtag gccttcagct tgatgactat tacaagatta tcaccaaacc    480
tatggatttt tcgaccatac aaaagaaaat ggaagggaaa gatgacaaca agtataacaa    540
tgtccgagaa atatattcgg atgttagatt aatttttgcc aatgcaatga agtacaatga    600
tgaacgacat gatgttcata taatggcgaa atcattgctt gagaaatttg aagagaaatg    660
gctccagctt cttcctaaag ttgaaaatga ggaaaggaaa caaaaggatg aggaatccaa    720
cggtgttcca aaagtgaaca tttctccaga agaagctatt gcaaaactag caaaagatac    780
tgacaatgag ctgattgaaa tcaacaagca gttggaggag cttcggcaaa tggtggtcca    840
gaaatgccgg aaaatgacta catatgagaa aagaaaactg ggtgcaggtc tctgccacct    900
gtctccagaa gagcttacta aggcattgga gatggttgca caagacaacc ctagcttcga    960
agctaaagga gacgaactgg aacttgatat ggatgctcag agtgagacaa ccctctggag   1020
gttgaaattc tttgtgaggg aagcattgga acgacaggcc aacgtcgctt ctggcaggac   1080
```

tgatgaaaat gcgaaaagaa agagggagat atgcaatgct ctggccagaa ctgcttcaaa 1140
aagagtcaag cagcaaccta attagtggcc tctgaccatt gtgcatttgt gtcttcactt 1200
gttgtttaga atgtttctga gaaatagcct tgcaaaataa tctcttcctt ctatggagca 1260
tgtaaataac tagtctctgc ttagctgtgt gaggttgcaa caatagcgaa atgtttccca 1320
aaaaaagggt cttatgttcc acctgtagat tgtgtgttcc atgcaattgt accatcaaga 1380
aaaatctagt atagcgttgc tggcgccatt tcactgtggc cattagccca ttactcattc 1440
cgaagagcaa actgtgaaaa aaaatccata agaacaaaat gtgatttttg ttttcaaaag 1500
aatgaacaat tcttagcatt cc 1522

<210> 325
<211> 1554
<212> DNA
<213> Physcomitrella patens

<400> 325
atggtcaatg gaaaatgtat gcttaaagac atggtagctg tagtcgtgtc gctctcgttt 60
cccgccttca gtcttgaagg tttcctactt gtgctactag atgcgaggcc cctttctgag 120
cgccgatcag attgcatttc taccgtcacc aagcaaggtt tcgacgtcgt tcagagaact 180
cgagatggac acatcaccaa tttaccttgc tctgaaccca tttctgagaa gaaggcttta 240
gtaacccagc aaatgaaaat gaaaccacga attggagtgg atggtgacat ggctgcagaa 300
cctgctcaat gtggaccagt acaggaaggg cgaggagcaa gagataggga cgacagagtc 360
ccatcaccag ctgcagttgg gcagaatagg gacagcacgg tagaagccaa tgaggttgaa 420
acggaggtct cagaggccgc gagaaatctt ctgaagcaac aagttcagac cctcacagca 480
aaagttgaag agattgagag aaagattgca ctggtaaccc aagaaaagaa tgctgaaagt 540
aaaagcaaag gagagtcagg aactggtttg aaagatcgcg acaaaggttg tggtaccctc 600
aacaaaaagc agcagtatct gctagataat aacagagggg atgtagctcg aagcaagcgt 660
aatcaagaat taatgaatca aattagaggc atctggagac agatatctca gcacaagtgg 720
gcttggcctt ttttaaaacc tgtggatgtc gaaggcctgg gtctgcacga ctacaacgat 780
gtaattgaga aaccaatgga cttgggcaca ataaagaaca aaatggatgc gaaagacact 840
tcaggctacc agcatgttca ggaggtctgt gatgatatgc gtttggtctt ctccaatgcc 900
atgacttata atccagaggg ctctgacgta catgtgatgt cgaaaaccct ctctgacaag 960
tttgaggaaa aatggaaggc tcttatagaa ccaaagttgc actttgagga atcgaagact 1020
cagcaggaag acaatgaagt acagctgaaa gaagcaggaa tgcaggtagt tgaagagata 1080
gacacaaaga aactgacaga acaatatttg cttcagttgg aggaactcga taaacagcta 1140
gaagacctaa agcgacaagc agctcctacg tgcagcaggg cgatgtcaat cgaggagaaa 1200
aggcatctgg gacagaacct tgggaaattg cctcctgaaa atctgagtca cgtcatacaa 1260
atcattgccc agagaaaccc atcgttcaat ataaattccg acgaagttga agttgatatt 1320
gacgcccagg accccgcaac actttggagg ttgcagcgct atgtgcaggc agttctaagt 1380
ggctccggag cacggcagac cacggcgagg aaccagccca ccaagcgcag ctgcggttat 1440
gtacagaaca aaaattcgag caagcgtggc aagaagacca ccacgccttg ccatggacaa 1500
ctctctcacg tcatgaagta cagctcaata ccaggattct tatcccacca gtaa 1554

<210> 326
<211> 1212
<212> DNA
<213> Physcomitrella patens

<400> 326
atgaaacctc gaattagcgc cgaaagtgac gaggccacac aacccgcaca ggaaggtcga 60
ggtgcaagag ctaaggatga cagaccgcaa ttgccagctg cagttgcaca gactagagac 120
agcattccag gagctaacga ggacgaaacg gaaacctcag aggctgcaaa agcccttcta 180
aaacaacagg ttcaggccct gacagccgaa gttgaagaga tggagaggaa aatcgccgaa 240
gtaactcaga agcggaatgc tgaacgcaaa agcaagggta aaatagggac ttctgtgaaa 300
gaccgtgata aaggtgccgg tgcttttacc aaaaagcagc agcaattgct tgatattaac 360
agacgggata cgtctcgaag caagcggatg caggagctaa tgcgtcaggt tcaaagcatt 420
tggaaacaga tatctcagca taaatgggct tggcctttca tgaagcccgt ggatgtcaag 480
ggtttgggtc tccatgacta ctacgatgtt attgaaaagc caatggactt gggcacaata 540
aagaacaaat tggatgtaaa agatggtttg ggtaccagc atgttcagga ggtctgtgat 600
gacgtgcggc tggtttttc caatgctatg acttataatc cagaggggtc tgatgtctac 660

```
gtgatgtcta aaaccctctc tgacaaattt gaggagaaat ggaagacgct tattgaaccg     720
aaattgcaag aggaattgaa aagatcacat gatgacagcg aagtgcaagc taacgaagga     780
ggagttccgg tagtagaaga gatagataca gaaaaagtga ttgagcaata tgcacttcag     840
ttggaggaac ttgataagca gctagaagag ctgaagcaac aagcaactcc taagtttagc     900
agagcaatgt ctgtagagga aaaaaggcat ctggggcaga gcttggggag gttaccacct     960
gataatttga gccacgtcat tcaaattatt gcacagaaaa acccctcctt taacataaac    1020
tccgacgagg ttgaagttga cattgacgcc caggacccgg caaccctctg gagattgcag    1080
cgatacgtgc aggcagtttt aagcggttcg gcagctcgcc aagctacacc gagaagccag    1140
gctaccaagc gcaatggcag ttctctgctt aaaaaaaact ccagcaagcg tagcaaggcc    1200
actacgtctt ga                                                        1212
```

<210> 327
<211> 807
<212> DNA
<213> Physcomitrella patens

<400> 327

```
atgacttcgt acattcctca gatatcctcg cataagtggg cttggccttt catgaagccc      60
gtggatgtca agggcttggg tctacatgac tattatgagg tcatcgaaaa gccaatggac     120
ctgggcacga taaagaataa aatggatgca aaagatgctt cgggatacca gcatgttcag     180
gaagtctatc aagacgtgcg gctggtcttc tcaaatgcta tgaaatacaa tccagagggt     240
tctgacgtat atgtgatgtc taaaacactg tcagaaaaat ttgaggagaa atggaagact     300
cttgtagaac cgaaattgca cgaggaggag tcgaaaaaat cgcaggaaga caatgaagta     360
aggacgaaag aaccaggagt ccaggccgtg gaagagatag atacagaaga attgactgaa     420
caatatgcac ttcagttgga ggagcttgat aagcagctag aagatttgaa gcaacaagca     480
actcccaaca gcagggcaat gtctgtagag gaaagaaggc atctggggca gagcttaggg     540
agattgccgc ctgataattt gagtcacgtc attcaaatca ttgcacagaa aaacccctcg     600
ttcaacatga actccgacga agtcgaagta gatatcgacg cccaagaccc tgcaacactc     660
tggaggttgc agcgatacgt gcaggcggta ttaagcgggt cggtggtcg ccaggctacg     720
ccgaggaacc aggctgccaa acgcaatggt agctctctac atagcaaaag ctccagcaag     780
cgtagcaaga aggtcaccgt tccttga                                         807
```

<210> 328
<211> 1155
<212> DNA
<213> Populus trichocarpa

<400> 328

```
atggaaccgt cggtttttta ttttggtaat cttccggtaa gaaatcccga cggcaacgat      60
gccgacacgg agggtttcaa gcatagcgtg gatgaaattt tccaaaaagt tgataaattg     120
gagcaaagaa tgaatggggt tgagcagttt tacttggata taagtaaaaa gcagcagagt     180
ggttcttcga aggtggtgg tagctcgatt gtgaaagata aagataaaga gaggcatgtt     240
actagtatta ggaaacagca gcaagatgcg tcgaaacgag aggctgctgc tgctaagaga     300
atgcaagagc ttatgcgcca attcggtacc atattgcgtc aaatcacgca gcacaaatgg     360
gcttggcctt ttatgcaacc tgtagatgtt aaaggtctta ggttgcatga ctattatgag     420
gttatcgaca agcccatgga cttcagtaca ataaaaaatc aaatggaggc taaggatggc     480
acaggatata agaatgtcag agagataagt gctgatgtga ggttagtgtt taagaatgca     540
atgaaatata atgatgaaag aagcgatgtt catgttatgg ccaaaacatt gctgggaaaa     600
tttgaggaga agtggctaca acttctgcct aaagtcactg aagaggaaaa aagaagagaa     660
gacgaggaag tggaggctaa attggatatg cagcttgctc aggaggctgc tcatgctaaa     720
atggctcgtg atttgagtaa tgagctttat gaggttgata tgcatctgga agagcttcgg     780
gatattgttg ttcaaaagtg cagaaagatg tccactgaag agaaaagaaa gctcggggtg     840
gctcttacga gactttctcc tgaagatcta actaaagcat tggagattgt tgctcggagt     900
aatccaggct tccaagcaac tgctgaagag gtggatctcg acattgatgc tcagactgaa     960
tccaccctgt ggaggctaaa gtttttggtg aaagatgtgc tggaagtcca gggaaaaagt    1020
gcggcaagca caggtggcaa taacaacaac aacaacaaca acaagaacac cagcaataac    1080
aacaaacgaa aaagagagat ttgcgatgct attgccaaaa ctgccaagaa aaggagtaaa    1140
aagccctctt cttga                                                     1155
```

<210> 329
<211> 1113
<212> DNA
<213> Populus trichocarpa

<400> 329
atggaagcga taagcccgtc gattgtggat tccggtaatc tcccgataag aaattccgat 60
gccgaagcag agggtttcaa gcatagcgtg gatgaaattc ttcaaaaagt tgataaattg 120
gagcaaagag tgaacgaggt cgagcagttt tactcgaaga atacaagtaa aaagcagcag 180
agtggttctt cgaaaggtgg aagctccact gtgaaagata aagataaaga gaggcatatt 240
cctactgctg ctaagagaat gcaagagctt atgcgccaat tcggtacaat attgcgtcaa 300
attacacagc acaaatgggc gtggcctttt atgcaacctg tggatgtcaa aggtcttggt 360
ttgcatgact attatgaggt tatcgacaag cccatggatt tcagtacaat taaaaatcaa 420
atggaggcca aggatggcac aggatacaag agtgtaaggg agatatgtgc tgatgtgagg 480
ttagtgttta agaatgcaat gaaatataat gatgaaagaa gtgatgttca tgttatggcc 540
aaaacattgc tgggaaaatt tgaggagaaa tggctacagt ttctgcctaa agtcactgaa 600
gaggagaaaa gaagagagga ggaggaagcg gaggctcaat tggatatgca gcttgctcag 660
gaggctgctc atgctaaaat ggctcgtgat ttgggtaatg agctttatga ggttgatatg 720
catctggaag agcttcgaga aatggttgtt caaaagtgca gaaagatgtc caccgaagag 780
aaaagaaagc ttggggcagc tcttacaagg ctatctcctg aagacctgac caaagcattg 840
gagattgttg ctcagaataa tccaggcttt caagcaactg ctgaagaggt ggaccttgac 900
atcgatgcac agagcgaaac caccctatgg aggctaaagt tttttgtgaa ggatgcgctg 960
gaagtccagg gaaaaagtgc agccagcgca ggtggccgaa acaacaccac taccccctagc 1020
aataataaca ataacaacaa caaacgtaaa agagagattt gcgatgctat tgccaaaact 1080
gccaagaaaa ggagtaagaa gccctcctct tga 1113

<210> 330
<211> 1134
<212> DNA
<213> Populus trichocarpa

<400> 330
atggaacata tgatcagatc tattcgaggt tccagaaatg ttggaactgg gaacattgaa 60
gttgataact cagaaatgga gtttgagcaa cgagtagatg cagttgaaag atactacgca 120
gataataaag aactaaatac tgctaaatgt agctcaattc tgaaggataa aatcaagaag 180
aaacatttga tgaccgttga gaagcaacaa caagattctg aaaagataac gcaagacctc 240
atgcgccagt ttgcagtaat atttcgccag atcgctcagc acaagtgggc atggcctttt 300
ctggaaccag tggatgttga aggtctttgt ttgcatgatt attatgaggt tattgaaaag 360
ccaatggact ttcgtacaat aaagaataga atggaggcta aggatggcac tgggtacaag 420
aatgttcggg agatatatgc tgatgttaga cttgttttta agaatgcaat gaaatataat 480
gatgaaagag atgatgtcca tgtgatggcc agaactttgt tagaaaaatt tgaagaaaaa 540
tggctgcaac ttctgcctaa agttgccgaa gaggaaaaaa gacgagaaaa ggagcaaaca 600
gcaactcagg tggctacgaa actagctgag gagtcttctt atgctaacat ggctcaggat 660
ttaagcaatg aactgcatgg agttgatatg caattggaga gaatcagaga aatggtggtt 720
cgcaacagca gaaagatatc cactgaagag aagaagaaac ttgggacagc gctcactcaa 780
ttatctcatc aagatctgat tagggcactg gaaatagttg ctgagcataa tccgagcttc 840
caagcaacag ctcaagaggt gaaccttgac atggatactc agagtgatgt gacgttatgg 900
aggttaaagg tttttgtgca agatgcacta aaagtttctg gcaggaattc tggggggcacg 960
ggtatggggt gcaacagcaa tattaacaat gatgataaca aggccaagat caacatcaac 1020
aacaagaaca ggaataccac tgcttccaaa aggaaaggag agaggtgtga tgccgtgacc 1080
aaggcatctg ccaagaggac taagaagatc tcactcaatt cccttaatcc atag 1134

<210> 331
<211> 1393
<212> DNA
<213> Sorghum bicolor

<400> 331
aaaaagggaa aaggaaacaa aaaaaaaccg tcagagcggc ggtcggctgt agtgcagttg 60

```
caggtgttcg ccggcacacg cgcgcggccg cgaaccctag atgacgccgg cgaacggcgc    120
cccagcgccg gcagcggagt ctgtcccccc cgaggtggag tcggaggccg atgcgttccg    180
gcgccaggtt gacgacctcg tctcgaagac cgacgtgctg gagaagcggg tgaaagaggt    240
ggtggacttc tatgatggca agaagcacgg aagcggcggg cggaagggcg gcggtggcgg    300
caggcacggg gcatactcga gggggatgcc cgaccttatg cgccagttcg gcgtgctttt    360
gaaagagatc acctctcata aagatgcatg gccatttctg aagccggtgg atgttgtaac    420
cctgcacatt cctgactatc acaaaattat tacccaacct atggacttct ctaccatcca    480
aaagaaaatg gaaaggaaag atggtacctg ttacaccaat gttcgagaaa tatgttctga    540
tgttcgatta atttttgcca atgcgatgaa gtacaatgat gatcaaaatg tagtccactt    600
gatggctaaa tcattgcttg agaaatttga agagaagtgg ctccattttc ttcctaaagt    660
tgagagtgag gaaaaaagac aaaaggagga ggaatcaaag ggtgttgcag ccacaaatac    720
ttctcgagaa gttgcaatcg caaaattagc aaaagatact gatgatgagc taaatcagat    780
caataggaag ctggaggaac tccggaaaat ggtggttcac agatgcagga aaatgaccac    840
agatgagaag aggaagctgg gtgcagggat ctgccacttg tctccagatg atcttaacaa    900
ggcgctagaa attgttgcac aagacaatcc tagcttccaa actaaagcag aagaagtgga    960
tcttgatatg gatgcccaga gtgagacaac cctatggagg ctgaaattct ttgtgaggga   1020
agcactggaa cgacaggcca acgttgcctc tgggaagatg gacgaaaacg ccaaaagaaa   1080
gagggagata tgcaatgctc tggccaaaac agcatcgaaa cggatcaaga agcaacccta   1140
gccatcttcc agatctttga ctcgcgtcct tctgtgattg ttaagaagtt tttttggtag   1200
gagtagccat tagctttgtg aaaggatttc ctgcattttt tgtggagctg tgtatatagt   1260
acactgtctg tccagtaaca agtgatttca tgtgtaacca tgagctgatc gcatcgactc   1320
tgaccaaaac aacaaacatt gtctctactt gcaattgcta gaatgaaagc cacaaccaca   1380
atgtgattga tgt                                                      1393

<210>  332
<211>  1261
<212>  DNA
<213>  Triticum aestivum

<400>  332
cgaggggcgg caatggaggg agcgtggccc catccggcgc cgatggagcc gaccaccgcc     60
gcggatgggc ctcaagtgtc ggaggtgaac tcgttccggc gccaggtcga cgacctcctc    120
tccaagaccg acgtgctgga gaagagggtg aacgaggtgg tagggttcta caattccaag    180
aagcacagca gtggaggccg caaggctggc ggcaggtacg cggaaaacgg tgccagggac    240
agccacggca atgggatgcc cgacctcatg cgccagtttg ccggtatcat tcgccagatt    300
acatctcatg aatgggcaca gccgtttttg caaccggtag acgtcgtagg tcttcaactt    360
gatgactatc accagattat aacaaaacct atggatttct cgaccatccg aaacaaaatg    420
gaagggaagg aaagtaccac atataacagt gtccgagaaa tatattctga tgttagatta    480
gtttttacca atgcaatgaa gtacaatgtt caaggtcacc ctgttcacat aatggccaag    540
ttcctactcg agagatttga ggagaaatgg cttcaccttc tccctaaagt tgagaacgag    600
gaaagggaac gggaggaacc aaatgatgct ccaaccataa gcatttctcc ggaagctgcc    660
attgcaatat tagctgaaga tactggtaat gagctgaatg agattaataa gcagctggag    720
gagctccaga aaatggtggt tcagagatgc aggaaaatga ccacggacga gaagagaaaa    780
ctcggcgcag gtctttgcca attgtctcca gaagatctta acaaggcgct agagttggtc    840
gcgcaagaca atcctagctt ccaaactaca gctgaagaag tggaccttga catggatgct    900
cagagcgaga cgaccctctg gaggctgaag ttctttgtga gggaagcatt ggaacaacag    960
gcgaatgtag gcgtagtggc ctgtggcaag atcgatgaaa acacgaagag gagccgcgac   1020
atgtacaatg ctctagccaa gaccgtttcg aaacggatta agaaataacc ttagcggctc   1080
ggcctttctg atggttcatg gtatttctgt agaaaatagg gccatgttct cccttgtgtg   1140
aagacatgtt gtatatatac gaagatgaat ctgcactaag atatgcttgg tcgcacccgg   1200
tctcatgtgg catgggtagc atgctattgg tagctatgta aaataaagag caatgttggc   1260
c                                                                   1261

<210>  333
<211>  1317
<212>  DNA
<213>  Vitis vinifera

<400>  333
```

```
atggctttcc gcattgcttt atccccatca gtaataacag aaagaggctt cttgttattc      60
attgcatcca gaaaagtctc taagacccat aaaactgaac ggttaacaaa cagaccccag     120
tgttttcata aggaagatcg agagacccgg ccgcctctgg aatgtcttgt atcagatgaa     180
ccaatggatg catcaattac aaatgttaga aatgtggaaa ttggaaagca caaaggtaat     240
actgaccaag ttgagggttt caaatcttgt gttgatgaca tattcacaaa ggttgataag     300
cttgagcaaa gagtgaatga agttgagcta ttctacttga ccgctagcaa aaggcaactg     360
aatggttata aaggtagctc agtccttaaa gataaggaga ggcatgttgc cagtgcaaag     420
aagcagcaac aagatgcatc acgcagagaa gcagctgctg tgaagagaat gcaagagctt     480
atgcgtcagt tcggcacaat attgcgtcag atcatgcaac acaagtgggc tgggcccttt     540
ttgcatcctg tagatgttga aggccttggt ttgcatgact actatgaggt aattgataag     600
cccatggact tcagtacaat aaagaatcaa atggaagcta aggatggtac tggatataag     660
aatgtcaggg agatatgtgc ggatgtgagg ttggttttta agaatgcaat gaaatacaat     720
gatgagagac atgatgttca tgttatggcc aaaactttat tgggaaagtt tgaggagaag     780
tggctgcaac ttctgcctaa agttgcagaa gaggaaaaaa gacgagaaga ggaagaagca     840
gaggcgcagt tagacatgca gcttgctcag gaggctgctc atgccaaaat ggcaagagag     900
ataagtaatg agctttacga tattgatatg catctggaag aagtcagaga aatggttatt     960
cggaagtgca gaaaaatgtc aactgaagag aaaaggaaac ttggagcagc cctctctaga    1020
ttgtccgctg aagatctgag taaggctctg gagattgttg cacagaataa cccgagcttt    1080
caagccacag ctgaagaggt ggatcttgac attgatgcac agacggagtc gaccttatgg    1140
aggttaaagt tttttgtgaa ggatgcattg gaagttcagg gaaagagttc agcaagcaag    1200
gggacaaca  ccaacaccac caccactgcc accaacaaca acaaacggaa aaaagagatt    1260
tgtgatgcta ttgccaagac tgcaaagaag aagagtaaaa agctccctct cgattaa       1317

<210>  334
<211>  855
<212>  DNA
<213>  Vitis vinifera

<400>  334
atggctcttt ggcatatttc cctgatgtct ctgaggtttt actggcagat catgcaacac      60
aagtgggctg ggcccttttt gcatcctgta gatgttgaag gccttggttt gcatgactac     120
tatgaggtaa ttgataagcc catggacttc agtacaataa agaatcaaat ggaagctaag     180
gatggtactg gatataagaa tgtcagggag atatgtgcgg atgtgaggtt ggtttttaag     240
aatgcaatga aatacaatga tgagagacat gatgttcatg ttatggccaa aactttattg     300
ggaaagtttg aggagaagtg gctgcaactt ctgcctaaag ttgcagaaga ggaaaaaaga     360
cgagaagagg aagaagcaga ggcgcagtta gacatgcagc ttgctcagga ggctgctcat     420
gccaaaatgg caagagagat aagtaatgag ctttacgata ttgatatgca tctggaagaa     480
gtcagagaaa tggttattcg gaagtgcaga aaaatgtcaa ctgaagagaa aaggaaactt     540
ggagcagccc tctctagatt gtccgctgaa gatctgagta aggctctgga gattgttgca     600
cagaataacc cgagctttca agccacagct gaagaggtgg atcttgacat tgatgcacag     660
acggagtcga ccttatggag gttaaagttt tttgtgaagg atgcattgga agttcaggga     720
aagagttcag caagcaaggg ggacaacacc aacaccacca ccactgccac caacaacaac     780
aaacggaaaa aagagatttg tgatgctatt gccaagactg caaagaagaa gagtaaaaag     840
ctccctctcg attaa                                                      855

<210>  335
<211>  1494
<212>  DNA
<213>  Zea mays

<400>  335
gaaaaaaccg tcagagcgac gttagtcggc tcagctgtag tgtagttgca ggtgttcgcc      60
ggcacacgcg cgtggccgcg aaccctagat gacaccggcg aacggcgccc cagcgcgggc     120
agcggaggcg ggcccccacg aggtggagtc ggaggccgac gcgttccggc gccaggtcga     180
cgacctcgtc tcgaagaccg acgtgctgga gcggcgggtg aaagaggtgg cggacttcta     240
cgatggcaaa aagcacggaa gcggcgggag gaagggtggt ggtggtggca ggtatggggc     300
atcctcgagg ggaatgccgg accttatgcg ccagttcggc gtgcttttga aagagatcac     360
ctctcataaa gatgcatggc catttctgga gccggtggat gttgtaactc tacagcttcc     420
tgactatcac aacattatta cccaacctat ggacttctct accatccaaa agaaaatgga     480
```

```
aaggaaggat ggtacttgtt acaccaatgt tagagaaata tgttctgatg ttcgattaat    540
ttttgccaat gcgatgaagt acaatgatga tcaaaatgtc atccacttga tggctaaatc    600
attgcttgag aaatttgaag agaagtggct ccattttctt cctaaagttg agagtgagga    660
aaaaagacaa aaggaggagg aatcaaaggg tgttgcagcc acaaatactt ctcgagaagt    720
tgcaatcgta aaattagcaa aagacactga tgatgagcta aatcagatca acaagaagct    780
ggaggagctc cggaaaatgg tggttcacag atgcaggaaa atgacgacag acgagaagag    840
gaagctgggt gcaggtatct gtcacttgtc tccagatgat cttagcaagg cgctagagat    900
tgtcgcacaa gacaatccta gcttccagac taaagcagaa gaagtggatc ttgatatgga    960
tgcccagagc gagacaactc tatggaggct gaaattcttt gtgagggaag cgctggaacg   1020
acagggccac gttgcctctg ggaagatgga ctataacgcc cagggaaaag agggagatat   1080
gcaatgctct ggccaaaacc acctcgagac ggatcaagaa gcagccctag ccatctttcc   1140
atatcatcac tcgcgtcctc tgattgttta gaagttcttt ttttgatact agccccccag   1200
ctttgtggaa cgttttcctg catttttttt gtgatgtgta tatagtagta tagtgaactg   1260
tccagtccag taacaagtga tttcgtgtgt aaccatgagc tggtcgcatt cgcgtcccct   1320
ctgatccaac aacaaacatt gtctcacaat gtgattgatt gtagaagcaa gcttttgtca   1380
cagggcgatg ttgctcactt gtggaatcag atgatgcata catttcgtaa ctgttcattc   1440
attctccacc ttggcttgaa aattaaggtg agaaggcctg cggatttaca tgtt         1494
```

<210> 336
<211> 386
<212> PRT
<213> Arabidopsis thaliana

<400> 336

```
Met Ser Val His Val Lys Glu Glu Pro Val Leu Val Pro Asn Cys Asp
1               5                   10                  15
Val Glu Asn Thr Glu Leu Ala Val Phe Asn Gly Asn Gly Glu Ser Glu
            20                  25                  30
Leu Glu Asn Phe Gly Thr Cys Val Asp Glu Ile Thr Asp Arg Val Asn
        35                  40                  45
Gln Leu Glu Gln Lys Val Val Glu Val Glu His Phe Tyr Ser Thr Lys
    50                  55                  60
Asp Gly Ala Ala Gln Thr Asn Thr Ser Lys Ser Asn Ser Gly Gly Lys
65                  70                  75                  80
Lys Ile Ala Ile Ser Gln Pro Asn Asn Ser Lys Gly Asn Ser Ala Gly
                85                  90                  95
Lys Glu Lys Ser Lys Gly Lys His Val Ser Ser Pro Asp Leu Met Arg
            100                 105                 110
Gln Phe Ala Thr Met Phe Arg Gln Ile Ala Gln His Lys Trp Ala Trp
        115                 120                 125
Pro Phe Leu Glu Pro Val Asp Val Lys Gly Leu Gly Leu His Asp Tyr
    130                 135                 140
Tyr Lys Val Ile Glu Lys Pro Met Asp Leu Gly Thr Ile Lys Lys Lys
145                 150                 155                 160
Met Glu Ser Ser Glu Tyr Ser Asn Val Arg Glu Ile Tyr Ala Asp Val
                165                 170                 175
Arg Leu Val Phe Lys Asn Ala Met Arg Tyr Asn Glu Glu Lys Glu Asp
            180                 185                 190
Val Tyr Val Met Ala Glu Ser Leu Leu Glu Lys Phe Glu Glu Lys Trp
        195                 200                 205
Leu Leu Ile Met Pro Lys Leu Val Glu Glu Glu Lys Lys Gln Val Asp
    210                 215                 220
Glu Glu Ala Glu Lys His Ala Asn Lys Gln Leu Thr Met Glu Ala Ala
225                 230                 235                 240
Gln Ala Glu Met Ala Arg Asp Leu Ser Asn Glu Leu Tyr Glu Ile Asp
                245                 250                 255
Leu Gln Leu Glu Lys Leu Arg Glu Ser Val Val Gln Arg Cys Arg Lys
            260                 265                 270
Leu Ser Thr Gln Glu Lys Lys Gly Leu Ser Ala Ala Leu Gly Arg Leu
```

```
        275                 280                 285
Ser Pro Glu Asp Leu Ser Lys Ala Leu Lys Met Val Ser Glu Ser Asn
    290                 295                 300
Pro Ser Phe Pro Ala Gly Ala Pro Glu Val Glu Leu Asp Ile Asp Val
305                 310                 315                 320
Gln Thr Asp Val Thr Leu Trp Arg Leu Lys Val Phe Val Gln Glu Ala
                325                 330                 335
Leu Lys Ala Ala Asn Lys Ser Ser Gly Gly Thr Asn Ala Gln Asn Asn
            340                 345                 350
Asn Asn Thr Gly Thr Gly Glu Ile Asn Lys Asn Asn Ala Lys Arg Arg
        355                 360                 365
Arg Glu Ile Ser Asp Ala Ile Asn Lys Ala Ser Ile Lys Arg Ala Lys
    370                 375                 380
Lys Ala
385
```

<210> 337
<211> 369
<212> PRT
<213> Arabidopsis thaliana

<400> 337

```
Met Ala Asp Ser Val Pro Gly His Val Ala Gly Gly Gly Leu Gln Gly
1               5                   10                  15
Phe Ser Val Asp Ala Glu Cys Ile Lys Gln Arg Val Asp Glu Val Leu
            20                  25                  30
Gln Trp Val Asp Ser Leu Glu His Lys Leu Lys Glu Val Glu Glu Phe
        35                  40                  45
Tyr Ser Ser Ile Gly Val Ser Asn Ser Gly Ser Ile Gly Lys Asp Thr
    50                  55                  60
Glu Lys Gly Arg His Val Val Gly Ile Arg Lys Ile Gln Gln Glu Ala
65                  70                  75                  80
Ala Arg Arg Glu Ala Val Ala Ala Lys Arg Met Gln Asp Leu Met Arg
                85                  90                  95
Gln Phe Gly Thr Ile Phe Arg Gln Ile Thr Gln His Lys Cys Ala Trp
            100                 105                 110
Pro Phe Met His Pro Val Asn Val Glu Gly Leu Gly Leu His Asp Tyr
        115                 120                 125
Phe Glu Val Ile Asp Lys Pro Met Asp Phe Ser Thr Ile Lys Asn Gln
    130                 135                 140
Met Glu Ala Lys Asp Gly Thr Gly Tyr Lys His Val Met Gln Ile Tyr
145                 150                 155                 160
Ala Asp Met Arg Leu Val Phe Glu Asn Ala Met Asn Tyr Asn Glu Glu
                165                 170                 175
Thr Ser Asp Val Tyr Ser Met Ala Lys Lys Leu Leu Glu Lys Phe Glu
            180                 185                 190
Glu Lys Trp Ala His Phe Leu Pro Lys Val Gln Glu Glu Glu Lys Ile
        195                 200                 205
Arg Glu Glu Glu Glu Lys Gln Ala Ala Lys Glu Ala Leu Leu Ala Lys
    210                 215                 220
Glu Ala Ser His Ile Lys Thr Thr Arg Glu Leu Gly Asn Glu Ile Cys
225                 230                 235                 240
His Ala Asn Asp Glu Leu Glu Lys Leu Met Arg Lys Val Val Glu Arg
                245                 250                 255
Cys Arg Lys Ile Thr Ile Glu Glu Lys Arg Asn Ile Gly Leu Ala Leu
            260                 265                 270
Leu Lys Leu Ser Pro Asp Asp Leu Gln Lys Val Leu Gly Ile Val Ala
        275                 280                 285
Gln Ala Asn Pro Ser Phe Gln Pro Arg Ala Glu Glu Val Ser Ile Glu
```

290 295 300
Met Asp Ile Leu Asp Glu Pro Thr Leu Trp Arg Leu Lys Phe Phe Val
305 310 315 320
Lys Asp Ala Leu Asp Asn Ala Met Lys Lys Lys Lys Glu Glu Glu Thr
325 330 335
Lys Thr Arg Glu Leu Ser Gly Ala Gln Lys Lys Glu Val Ser Lys Lys
340 345 350
Arg Asn Ala Thr Thr Lys Leu Ala Glu Arg Lys Thr Lys Arg Ser Arg
355 360 365
Ile

<210> 338
<211> 373
<212> PRT
<213> Centaurea solstitialis

<400> 338
Met Glu Glu Asn Asn Ala Val Ile Val Asp Asp Ile Gly Gln Gln Val
1 5 10 15
Asp Asp Leu Phe Thr Lys Val Glu Lys Leu Glu Gln Arg Val Asn Glu
20 25 30
Ile Glu Gln Phe Tyr Leu Asn Ser Ser Asn Lys Gln Ser Ser Ser Ser
35 40 45
Gln Lys Ser Leu Leu Val Lys Asp Lys Asp Lys Val Lys His Ile Pro
50 55 60
Gly Tyr Lys Lys His Gln Gln Glu Ala Ala Arg Arg Glu Ala Thr Ala
65 70 75 80
Ala Lys Arg Met Gln Glu Ile Met Arg Gln Phe Gly Ser Ile Leu Arg
85 90 95
Gln Ile Thr Gln His Lys Trp Ala Trp Pro Phe Met Gln Pro Val Asp
100 105 110
Val Glu Gly Leu Gly Leu Arg Asp Tyr Tyr Glu Val Ile Asp Arg Pro
115 120 125
Met Asp Phe Ser Thr Ile Lys Asn Leu Met Glu Ala Lys Asp Gly Thr
130 135 140
Glu Tyr Lys His Val Arg Asp Ile Cys Ser Asp Val Arg Leu Val Phe
145 150 155 160
Gln Asn Ala Met Lys Tyr Asn Asp Asp Lys Ser Asp Val His Val Met
165 170 175
Ala Lys Thr Leu Leu Glu Lys Phe Glu Glu Lys Trp Leu Gln Phe Leu
180 185 190
Pro Arg Val Thr Glu Glu Glu Lys Arg Arg Glu Glu Glu Glu Ala Glu
195 200 205
Ala Gln Leu Ser Ile Gln Arg Ala Gln Glu Ala Ala His Ala Lys Met
210 215 220
Ala Arg Asp Leu Ser Asn Glu Leu Tyr Glu Ala Asp Met His Leu Glu
225 230 235 240
His Leu Arg Glu Thr Val Val Gln Lys Cys Arg Lys Met Ser Ile Glu
245 250 255
Asp Lys Arg Asn Leu Gly Ile Ala Leu Thr Gln Leu Ser Pro Glu Asp
260 265 270
Leu Ser Lys Ala Leu Asp Ile Val Ala Gln His Asn Pro Ser Phe His
275 280 285
Ser Thr Ala Ile Glu Val Asp Leu Asp Ile Asp Ala Gln Ser Glu Ser
290 295 300
Thr Leu Trp Arg Leu Lys Tyr Phe Val Lys Asp Ala Leu Gln Gly Tyr
305 310 315 320
Gly Lys Asp Asp Ala Ile Ile Gly Asn Glu Ala Asn Asn Gly His Val

```
                325                 330                 335
Ser Pro Thr Lys Pro Thr Asn Thr Thr Arg Thr Ile Ala Ser Lys Arg
            340                 345                 350
Lys Lys Gln Ile Cys Asn Ala Leu Ala Arg Thr Ala Lys Lys Arg Asn
        355                 360                 365
Gln Lys Leu Ser Ser
    370
```

<210> 339
<211> 374
<212> PRT
<213> Gossypium hirsutum

<400> 339

```
Met Glu Ala Val Lys Val Glu Gly Phe Gly Pro Asp Ser Gly Val Glu
1               5                   10                  15
Asp Leu Gly Arg Cys Val Asp Glu Ile Ser Thr Thr Val Ser Arg Leu
            20                  25                  30
Glu Gln Arg Val Asn Asp Val Glu Gln Phe Tyr Leu Thr Thr Asp Asn
        35                  40                  45
Met Glu Leu Thr Ile Thr Lys Ser Ala Ser Ala Phe Lys Glu Lys Val
    50                  55                  60
Lys Glu Lys Gln Pro Thr Gly Leu Glu Lys Gln Gln Gln Glu Ala Ser
65                  70                  75                  80
Gln Arg Glu Ala Ala Ala Leu Lys Arg Met Gln Glu Leu Met Arg Gln
                85                  90                  95
Phe Ala Thr Ile Leu Arg Gln Thr Thr Gln His Lys Trp Ala His Pro
            100                 105                 110
Phe Met His Pro Val Asp Val Glu Gly Leu Gly Leu His Asp Tyr Tyr
        115                 120                 125
Glu Ile Val Gln Lys Pro Met Asp Phe Gly Thr Ile Lys Ser Lys Met
    130                 135                 140
Glu Ala Lys Asp Gly Thr Gly Tyr Lys Asn Val Arg Glu Ile Tyr Ser
145                 150                 155                 160
Asp Val Arg Leu Val Phe Lys Asn Ala Met Lys Tyr Asn Asp Glu Arg
                165                 170                 175
His Asp Val His Ile Met Ala Lys Thr Leu Leu Glu Lys Phe Glu Glu
            180                 185                 190
Lys Trp Leu Gln Leu Leu Pro Lys Val Ala Glu Glu Glu Lys Arg Gln
        195                 200                 205
Ala Glu Glu Glu Ala Lys Ala Glu Leu Asp Val Lys Leu Ala Gln Glu
    210                 215                 220
Ala Val His Ala Asn Met Ala Lys Glu Leu Ser Asn Glu Leu Cys Asp
225                 230                 235                 240
Val Asp Leu Gln Leu Glu Lys Leu Arg Gln Ile Val Ile Gln Lys Cys
                245                 250                 255
Arg Lys Met Ser Thr Glu Glu Lys Lys Lys Leu Gly Thr Ala Leu Thr
            260                 265                 270
Arg Leu Ser Pro Glu Asp Leu Gly Lys Ala Leu Glu Ile Val Ala Glu
        275                 280                 285
Asn Asn Pro Gly Phe Gln Pro Thr Ala Gln Glu Val Asp Leu Asp Ile
    290                 295                 300
Asp Ala Gln Ser Glu Leu Thr Leu Trp Arg Leu Lys Val Phe Val Gln
305                 310                 315                 320
Asp Lys Leu Lys Leu Ala Gly Lys Cys Ser Glu Ala Val Val Cys Asn
                325                 330                 335
Asn Ile Asn Asn Asn Glu Asn Thr Ile Lys Ser Asn Ser Lys Arg Arg
            340                 345                 350
Arg Glu Ile Ser Asp Ala Leu Thr Lys Asn Ala Ile Lys Arg Asn Arg
```

```
        355                 360                 365
Lys Leu Ser Pro Asn Ser
    370

<210>  340
<211>  220
<212>  PRT
<213>  Gossypium raimondii

<400>  340
Met Glu Gln Val Met Ala Ser Ile Pro Asp Phe Glu Ile Ala Glu Thr
1               5                   10                  15
Gly Ile Ser Lys Asp Asn Ser Ala Glu Ala Glu Gln Phe Lys Ile Arg
            20                  25                  30
Val Asp Glu Ile Phe Gln Lys Val Asp Glu Leu Glu Gln Lys Val Asn
        35                  40                  45
Glu Val Glu Gln Phe Asn Leu Asn Ser Ser Lys Lys Gln Gln Ser Ser
    50                  55                  60
Ser Lys Gly Ser Ser Met Gly Lys Glu Arg Asp Lys Gly Arg Gln Val
65                  70                  75                  80
Pro Ser Ile Lys Lys Gln Gln Gln Asp Ala Ser Arg Arg Glu Ala Ala
                85                  90                  95
Ala Ala Lys Arg Met Gln Glu Leu Met Arg Gln Phe Gly Thr Ile Val
            100                 105                 110
Arg Gln Ile Thr Gln His Lys Trp Ala Trp Pro Phe Met Gln Pro Val
        115                 120                 125
Asp Val Lys Gly Leu Gly Leu His Asp Tyr Tyr Glu Ile Ile Glu Lys
    130                 135                 140
Pro Met Asp Phe Ser Thr Ile Lys Asn Gln Met Glu Ala Lys Asp Gly
145                 150                 155                 160
Thr Gly Tyr Lys Asn Val Arg Asp Ile Cys Ala Asp Val Arg Leu Val
                165                 170                 175
Phe Asn Asn Ala Met Lys Tyr Asn Asp Glu Gly Ser Asp Val His Leu
            180                 185                 190
Met Ala Lys Thr Leu Leu Glu Lys Phe Glu Glu Lys Trp Gln Leu Leu
        195                 200                 205
Leu Pro Lys Val Thr Glu Arg Gly Glu Lys Thr Arg
    210                 215                 220

<210>  341
<211>  374
<212>  PRT
<213>  Gossypium raimondii

<400>  341
Met Glu Ala Val Gln Phe Glu Gly Phe Gly Pro Asp Ser Glu Val Glu
1               5                   10                  15
Asp Phe Gly Arg Cys Val Asp Glu Ile Ser Thr Thr Val Ser Arg Leu
            20                  25                  30
Glu Gln Arg Val Asn Asp Val Glu Gln Phe Tyr Leu Thr Thr Asp Asn
        35                  40                  45
Met Glu Leu Thr Ile Thr Lys Ser Ala Ser Ala Phe Lys Glu Lys Val
    50                  55                  60
Lys Glu Lys Gln Leu Thr Gly Leu Glu Lys Gln Gln Gln Glu Ala Ser
65                  70                  75                  80
Gln Arg Glu Ala Ala Ala Leu Lys Arg Met Gln Glu Leu Met Arg Gln
                85                  90                  95
Phe Ala Thr Ile Leu Arg Gln Ile Thr Gln His Lys Trp Ala His Pro
            100                 105                 110
```

```
Phe Met His Pro Val Asp Val Glu Gly Leu Gly Leu His Asp Tyr Tyr
        115                 120                 125
Glu Ile Val Gln Lys Pro Met Asp Phe Gly Thr Ile Lys Ser Lys Met
    130                 135                 140
Glu Ala Lys Asp Gly Thr Gly Tyr Lys Asn Val Arg Glu Ile Tyr Ser
145                 150                 155                 160
Asp Val Arg Leu Val Phe Lys Asn Ala Met Lys Tyr Asn Asp Glu Arg
                165                 170                 175
His Asp Val His Ile Met Ala Lys Thr Leu Leu Glu Lys Phe Glu Glu
            180                 185                 190
Lys Trp Leu Gln Leu Leu Pro Lys Val Ala Glu Glu Glu Lys Arg Gln
        195                 200                 205
Val Glu Glu Glu Ala Lys Ala Glu Leu Asp Val Lys Leu Ala Gln Glu
    210                 215                 220
Ala Val His Ala Asn Met Ala Lys Glu Leu Ser Asn Glu Leu Phe Asp
225                 230                 235                 240
Val Asp Leu Gln Leu Glu Lys Leu Arg Gln Ile Val Ile Gln Lys Cys
                245                 250                 255
Arg Lys Met Ser Thr Glu Glu Lys Lys Lys Leu Gly Thr Ala Leu Thr
            260                 265                 270
Arg Leu Ser Pro Glu Asp Leu Gly Lys Ala Leu Glu Ile Val Ala Glu
        275                 280                 285
Asn Asn Pro Gly Phe Gln Pro Thr Ala Gln Glu Val Asp Leu Asp Ile
    290                 295                 300
Asp Ala Gln Ser Glu Leu Thr Leu Trp Arg Leu Lys Val Phe Val Gln
305                 310                 315                 320
Asp Lys Leu Lys Leu Ala Gly Lys Cys Phe Glu Ala Val Val Cys Thr
                325                 330                 335
Asn Ile Asn Asn Asn Glu Asn Pro Ile Lys Ser Asn Ser Lys Arg Arg
            340                 345                 350
Arg Glu Ile Phe Asp Ala Leu Thr Lys Asn Ala Ile Lys Arg Asn Arg
        355                 360                 365
Lys Leu Phe Pro Asn Ser
    370
```

```
<210>  342
<211>  222
<212>  PRT
<213>  Lactuca saligna

<400>  342
Met Glu Thr Met Asp Gly Pro Val Pro Glu Val Arg Thr Glu Gly Glu
1               5                   10                  15
Pro Ala Val Ala Glu Gln Leu Gly Gln Asn Val Asp Gln Ile Ile Val
            20                  25                  30
Lys Val Asp Glu Leu Glu Gln Arg Leu Asn Glu Val Glu Gln Phe Tyr
        35                  40                  45
Ser Lys Pro Gly Lys Lys Gln Ser Asn Thr Ser Lys Ala Ser Ser Val
    50                  55                  60
Met Lys Glu Lys Asp Lys Asp Lys Gln Ile Thr Ser Phe Lys Arg Arg
65                  70                  75                  80
Gln Leu Asp Ala Ser Arg Arg Glu Ala Ala Ala Thr Lys Arg Met Gln
                85                  90                  95
Asp Leu Met Arg Gln Phe Ser Val Leu Leu Arg Gln His Ile Ile Gly
            100                 105                 110
His Lys Trp Ala Gly Pro Phe Met Gln Pro Val Asp Val Val Gly Leu
        115                 120                 125
Gly Leu His Asp Tyr Tyr Glu Val Ile Glu Lys Pro Met Asp Phe Ser
    130                 135                 140
```

```
Thr Ile Lys Ala Lys Met Glu Val Lys Asp Gly Thr Gly Tyr Asn Asn
145                 150                 155                 160
Val Arg Glu Ile Cys Ala Asp Val Arg Leu Ile Phe Lys Asn Ala Met
                165                 170                 175
Lys Tyr Asn Asp Glu Arg Asn Asp Val His Val Met Ala Lys Thr Leu
            180                 185                 190
Leu Ala Lys Phe Glu Glu Lys Trp Leu Gln Leu Leu Pro Lys Val Asp
        195                 200                 205
Glu Glu Asp Glu Arg Arg Lys Lys Glu Lys Gln Asn His Ser
    210                 215                 220

<210>  343
<211>  369
<212>  PRT
<213>  Medicago truncatula

<400>  343
Met Glu Pro Tyr Pro Gln Arg Pro Ser Ala Asn Ala Asp Arg Leu Glu
1               5                   10                  15
Gly Phe Arg Asn Ser Val Asp Glu Phe Arg Thr Gln Val Asp Asn Leu
            20                  25                  30
Gln Lys Gln Val Ile Glu Val Glu His Tyr Tyr Glu Ser Ser Gly Ile
        35                  40                  45
Phe Gln Gly Asn Ser Ser Arg Gly Gly Ser Val Val Lys Glu Lys Gly
    50                  55                  60
Arg Glu Lys Thr Leu Ala Gly Thr Lys Thr Pro Leu Gln Asp Ala Leu
65                  70                  75                  80
Arg Thr Glu Thr Ala Ala Gly Lys Arg Met Gln Glu Leu Met Arg Gln
                85                  90                  95
Phe Ser Thr Ile Leu Arg Gln Ala Thr Phe Gln Ile Thr Gln His Lys
            100                 105                 110
Trp Ala Trp Pro Phe Leu Glu Pro Val Asp Val Glu Gly Leu Gly Leu
        115                 120                 125
His Asp Tyr Tyr Glu Ile Ile Asp Lys Pro Met Asp Phe Gly Thr Ile
    130                 135                 140
Lys Asn Lys Met Glu Ala Lys Asp Gly Thr Gly Tyr Lys Asn Val Arg
145                 150                 155                 160
Glu Ile Tyr Ala Asp Val Arg Leu Ile Phe Lys Asn Ala Met Lys Tyr
                165                 170                 175
Asn Asn Glu Lys His Asp Val His Val Met Ala Lys Thr Leu Met Glu
            180                 185                 190
Lys Phe Glu Asp Lys Trp Leu Leu Leu Leu Pro Lys Val Ala Glu Glu
        195                 200                 205
Glu Lys Arg Gln Ile Glu Glu Glu Ala Gln Val Gln Met Asp Ile His
    210                 215                 220
Leu Ala Gln Glu Thr Thr Tyr Ala Asp Met Ala Lys Asp Leu Ser Asn
225                 230                 235                 240
Glu Leu Asn Glu Val Gly Ile Arg Leu Met Glu Phe Arg Glu Lys Val
                245                 250                 255
Ile Gln Asn Cys Arg Lys Leu Ser Thr Gly Glu Lys Lys Ala Leu Gly
            260                 265                 270
Lys Ala Ile Ala Lys Leu Ser Pro Glu Asn Leu Gln Arg Ala Leu Asp
        275                 280                 285
Leu Val Ala Glu Ile Asn Pro Ser Phe Glu Ser Thr Ala Asp Glu Val
    290                 295                 300
Val Leu Asp Ile Asn Ala Gln Ser Asp Tyr Thr Val Trp Arg Leu Tyr
305                 310                 315                 320
His Phe Val Lys Gly Ala Leu Glu Gly Gln Gln Gly Thr Ala Val Asn
                325                 330                 335
```

```
Asn Asp Thr Glu Glu Lys Arg Tyr Ser Ser Arg Lys Arg Arg Glu Phe
            340                 345                 350
Ser Asp Asp His Ala Lys Asn Pro Ser Lys Ser Arg Lys Leu Ser Thr
        355                 360                 365
Ser


<210>  344
<211>  359
<212>  PRT
<213>  Medicago truncatula

<400>  344
Met Ser Leu Gly Lys Gln Val Asn Asp Val Glu Gln Phe Tyr Gln Ser
1               5                   10                  15
Thr Asp Val Gln Gln Asn Asp Cys Lys Tyr Lys Gly Arg Glu Lys Pro
            20                  25                  30
Pro Thr Gly Ser Lys Lys Ala Leu Lys Arg Ala Ser Glu Asp Met Gln
        35                  40                  45
Ala Glu Met Arg His Asn Phe Asn Lys Ile Phe Asn Glu Ala Ser Ala
    50                  55                  60
Val Lys Leu Leu Ala Met Thr Asn Tyr Ile Leu Leu Gln Pro Leu Ser
65                  70                  75                  80
Phe Ser Tyr Ile Tyr Pro Val Leu Leu Asn Ser Ser Tyr Ser Gly Val
                85                  90                  95
Glu Phe Glu His Ile Thr Ile Ile Ala Lys Asp Lys Trp Ala Trp Pro
            100                 105                 110
Phe Leu Asp Pro Val Asp Val Glu Gly Leu Gly Leu Tyr Asp Tyr Tyr
        115                 120                 125
Gln Ile Ile Glu Lys Pro Met Asp Phe Ser Thr Ile Lys Ile Arg Met
    130                 135                 140
Glu Ala Lys Asp Gly Ser Gly Tyr Lys Asn Val Arg Glu Ile Tyr Ala
145                 150                 155                 160
Asp Val Arg Leu Ile Phe Lys Asn Ala Met Lys Tyr Asn Asp Glu Lys
                165                 170                 175
Asn Asp Val His Val Met Ala Lys Thr Leu Leu Glu Lys Phe Glu Gly
            180                 185                 190
Cys Thr Tyr Arg Asn Asn Ser Gly Phe Ser His Pro Met Gln Tyr Phe
        195                 200                 205
Ser Glu Ser Asp Leu Ser Lys Glu Glu Ala His Glu Glu Leu Asn Lys
    210                 215                 220
Arg Leu Ala Gln Glu Ala Thr Tyr Ala Asn Met Thr Arg Glu Leu Ser
225                 230                 235                 240
Thr Glu Leu Ser Lys Val Asp Met Ala Leu Arg Ser Leu Lys Thr Thr
                245                 250                 255
Ala Ile Ser Gln Cys Arg Ser Val Lys Ser His Ser Met Asn Asp Ser
            260                 265                 270
Leu Val Leu His Ile Ile Cys His Ala Val Ile Asp Leu Asn Cys Leu
        275                 280                 285
Ala Lys Ile Arg Lys Leu Ser His Pro Glu Lys Leu Ile Leu Ala Asn
    290                 295                 300
Ala Phe Thr Lys Leu Ser Pro Asp Asn Ile Val Lys Ala Leu Glu Ile
305                 310                 315                 320
Val Lys Glu Ser Asn Pro Asn Phe Lys Asp Arg Ile Asp Met Val Thr
                325                 330                 335
Leu Asp Leu Asp Ser Gln Ser Asp Tyr Thr Leu Phe Arg Leu His Met
            340                 345                 350
Phe Val Lys Asn Thr Leu Glu
        355
```

<210> 345
<211> 360
<212> PRT
<213> Oryza sativa

<400> 345

```
Met Val Pro Gly Gly Gly Val Pro Gln Gly Ala Glu Gly Gly Arg Ala
1               5                   10                  15
Met Ala Ala Ala Glu Thr Ala Ala Thr Ala Ala Ala Ala Gly Glu Arg
            20                  25                  30
Ala Pro Gly Thr Glu Val Asp Ala Phe Arg Arg Gln Val Glu Asp Leu
        35                  40                  45
Val Ser Lys Thr Asp Gln Leu Glu Arg Arg Val Asn Glu Val Val Gly
    50                  55                  60
Phe Tyr Asp Gly Lys Lys His Gly Ser Gly Gly Arg Lys Ala Gly Arg
65                  70                  75                  80
Lys Asp Ser Ser Leu Ser Lys Gly Met Pro Asp Leu Met Arg Gln Phe
                85                  90                  95
Gly Thr Ile Val Arg Gln Ile Thr Ser His Glu Trp Ala Glu Pro Phe
            100                 105                 110
Leu Lys Pro Val Asp Val Val Gly Leu Gln Leu Asp Asp Tyr Tyr Lys
        115                 120                 125
Ile Ile Thr Lys Pro Met Asp Phe Ser Thr Ile Gln Lys Lys Met Glu
    130                 135                 140
Gly Lys Asp Asp Asn Lys Tyr Asn Asn Val Arg Glu Ile Tyr Ser Asp
145                 150                 155                 160
Val Arg Leu Ile Phe Ala Asn Ala Met Lys Tyr Asn Asp Glu Arg His
                165                 170                 175
Asp Val His Ile Met Ala Lys Ser Leu Leu Glu Lys Phe Glu Glu Lys
            180                 185                 190
Trp Leu Gln Leu Leu Pro Lys Val Glu Asn Glu Glu Arg Lys Gln Lys
        195                 200                 205
Asp Glu Glu Ser Asn Gly Val Pro Lys Val Asn Ile Ser Pro Glu Glu
    210                 215                 220
Ala Ile Ala Lys Leu Ala Lys Asp Thr Asp Asn Glu Leu Ile Glu Ile
225                 230                 235                 240
Asn Lys Gln Leu Glu Glu Leu Arg Gln Met Val Val Gln Lys Cys Arg
                245                 250                 255
Lys Met Thr Thr Tyr Glu Lys Arg Lys Leu Gly Ala Gly Leu Cys His
            260                 265                 270
Leu Ser Pro Glu Glu Leu Thr Lys Ala Leu Glu Met Val Ala Gln Asp
        275                 280                 285
Asn Pro Ser Phe Glu Ala Lys Gly Asp Glu Leu Glu Leu Asp Met Asp
    290                 295                 300
Ala Gln Ser Glu Thr Thr Leu Trp Arg Leu Lys Phe Phe Val Arg Glu
305                 310                 315                 320
Ala Leu Glu Arg Gln Ala Asn Val Ala Ser Gly Arg Thr Asp Glu Asn
                325                 330                 335
Ala Lys Arg Lys Arg Glu Ile Cys Asn Ala Leu Ala Arg Thr Ala Ser
            340                 345                 350
Lys Arg Val Lys Gln Gln Pro Asn
        355                 360
```

<210> 346
<211> 360
<212> PRT
<213> Oryza sativa

<400> 346
Met Val Pro Gly Gly Gly Val Pro Gln Gly Ala Glu Gly Gly Arg Ala
1 5 10 15
Met Ala Ala Ala Glu Thr Ala Ala Thr Ala Ala Ala Ala Gly Glu Arg
20 25 30
Ala Pro Gly Thr Glu Val Asp Ala Phe Arg Arg Gln Val Glu Asp Leu
35 40 45
Val Ser Lys Thr Asp Gln Leu Glu Arg Arg Val Asn Glu Val Val Gly
50 55 60
Phe Tyr Asp Gly Lys Lys His Gly Ser Gly Gly Arg Lys Ala Gly Arg
65 70 75 80
Lys Asp Ser Ser Leu Ser Lys Gly Met Pro Asp Leu Met Arg Gln Phe
85 90 95
Gly Thr Ile Val Arg Gln Ile Thr Ser His Glu Trp Ala Glu Pro Phe
100 105 110
Leu Lys Pro Val Asp Val Val Gly Leu Gln Leu Asp Asp Tyr Tyr Lys
115 120 125
Ile Ile Thr Lys Pro Met Asp Phe Ser Thr Ile Gln Lys Lys Met Glu
130 135 140
Gly Lys Asp Asp Asn Lys Tyr Asn Asn Val Arg Glu Ile Tyr Ser Asp
145 150 155 160
Val Arg Leu Ile Phe Ala Asn Ala Met Lys Tyr Asn Asp Glu Arg His
165 170 175
Asp Val His Ile Met Ala Lys Ser Leu Leu Glu Lys Phe Glu Glu Lys
180 185 190
Trp Leu Gln Leu Leu Pro Lys Val Glu Asn Glu Glu Arg Lys Gln Lys
195 200 205
Asp Glu Glu Ser Asn Gly Val Pro Lys Val Asn Ile Ser Pro Glu Glu
210 215 220
Ala Ile Ala Lys Leu Ala Lys Asp Thr Asp Asn Glu Leu Ile Glu Ile
225 230 235 240
Asn Lys Gln Leu Glu Glu Leu Arg Gln Met Val Val Gln Lys Cys Arg
245 250 255
Lys Met Thr Thr Tyr Glu Lys Arg Lys Leu Gly Ala Gly Leu Cys His
260 265 270
Leu Ser Pro Glu Glu Leu Thr Lys Ala Leu Glu Met Val Ala Gln Asp
275 280 285
Asn Pro Ser Phe Glu Ala Lys Gly Asp Glu Leu Glu Leu Asp Met Asp
290 295 300
Ala Gln Ser Glu Thr Thr Leu Trp Arg Leu Lys Phe Phe Val Arg Glu
305 310 315 320
Ala Leu Glu Arg Gln Ala Asn Val Ala Ser Gly Arg Thr Asp Glu Asn
325 330 335
Ala Lys Arg Lys Arg Glu Ile Cys Asn Ala Leu Ala Arg Thr Ala Ser
340 345 350
Lys Arg Val Lys Gln Gln Pro Asn
355 360

<210> 347
<211> 517
<212> PRT
<213> Physomitrella patents

<400> 347
Met Val Asn Gly Lys Cys Met Leu Lys Asp Met Val Ala Val Val Val
1 5 10 15
Ser Leu Ser Phe Pro Ala Phe Ser Leu Glu Gly Phe Leu Leu Val Leu
20 25 30
Leu Asp Ala Arg Pro Leu Ser Glu Arg Arg Ser Asp Cys Ile Ser Thr

```
        35                  40                  45
Val Thr Lys Gln Gly Phe Asp Val Val Gln Arg Thr Arg Asp Gly His
    50                  55                  60
Ile Thr Asn Leu Pro Cys Ser Glu Pro Ile Ser Glu Lys Lys Ala Leu
65                  70                  75                  80
Val Thr Gln Gln Met Lys Met Lys Pro Arg Ile Gly Val Asp Gly Asp
                85                  90                  95
Met Ala Ala Glu Pro Ala Gln Cys Gly Pro Val Gln Glu Gly Arg Gly
            100                 105                 110
Ala Arg Asp Arg Asp Asp Arg Val Pro Ser Pro Ala Ala Val Gly Gln
        115                 120                 125
Asn Arg Asp Ser Thr Val Glu Ala Asn Glu Val Glu Thr Glu Val Ser
    130                 135                 140
Glu Ala Ala Arg Asn Leu Leu Lys Gln Gln Val Gln Thr Leu Thr Ala
145                 150                 155                 160
Lys Val Glu Glu Ile Glu Arg Lys Ile Ala Leu Val Thr Gln Glu Lys
                165                 170                 175
Asn Ala Glu Ser Lys Ser Lys Gly Glu Ser Gly Thr Gly Leu Lys Asp
            180                 185                 190
Arg Asp Lys Gly Cys Gly Thr Leu Asn Lys Lys Gln Gln Tyr Leu Leu
        195                 200                 205
Asp Asn Asn Arg Gly Asp Val Ala Arg Ser Lys Arg Asn Gln Glu Leu
    210                 215                 220
Met Asn Gln Ile Arg Gly Ile Trp Arg Gln Ile Ser Gln His Lys Trp
225                 230                 235                 240
Ala Trp Pro Phe Leu Lys Pro Val Asp Val Glu Gly Leu Gly Leu His
                245                 250                 255
Asp Tyr Asn Asp Val Ile Glu Lys Pro Met Asp Leu Gly Thr Ile Lys
            260                 265                 270
Asn Lys Met Asp Ala Lys Asp Thr Ser Gly Tyr Gln His Val Gln Glu
        275                 280                 285
Val Cys Asp Asp Met Arg Leu Val Phe Ser Asn Ala Met Thr Tyr Asn
    290                 295                 300
Pro Glu Gly Ser Asp Val His Val Met Ser Lys Thr Leu Ser Asp Lys
305                 310                 315                 320
Phe Glu Glu Lys Trp Lys Ala Leu Ile Glu Pro Lys Leu His Phe Glu
                325                 330                 335
Glu Ser Lys Thr Gln Gln Glu Asp Asn Glu Val Gln Leu Lys Glu Ala
            340                 345                 350
Gly Met Gln Val Val Glu Glu Ile Asp Thr Lys Lys Leu Thr Glu Gln
        355                 360                 365
Tyr Leu Leu Gln Leu Glu Glu Leu Asp Lys Gln Leu Glu Asp Leu Lys
    370                 375                 380
Arg Gln Ala Ala Pro Thr Cys Ser Arg Ala Met Ser Ile Glu Glu Lys
385                 390                 395                 400
Arg His Leu Gly Gln Asn Leu Gly Lys Leu Pro Pro Glu Asn Leu Ser
                405                 410                 415
His Val Ile Gln Ile Ile Ala Gln Arg Asn Pro Ser Phe Asn Ile Asn
            420                 425                 430
Ser Asp Glu Val Glu Val Asp Ile Asp Ala Gln Asp Pro Ala Thr Leu
        435                 440                 445
Trp Arg Leu Gln Arg Tyr Val Gln Ala Val Leu Ser Gly Ser Gly Ala
    450                 455                 460
Arg Gln Thr Thr Ala Arg Asn Gln Pro Thr Lys Arg Ser Cys Gly Tyr
465                 470                 475                 480
Val Gln Asn Lys Asn Ser Ser Lys Arg Gly Lys Lys Thr Thr Thr Pro
                485                 490                 495
Cys His Gly Gln Leu Ser His Val Met Lys Tyr Ser Ser Ile Pro Gly
            500                 505                 510
```

```
Phe Leu Ser His Gln
        515

<210>  348
<211>  403
<212>  PRT
<213>  Physcomitrella patens

<400>  348
Met Lys Pro Arg Ile Ser Ala Glu Ser Asp Glu Ala Thr Gln Pro Ala
1               5                   10                  15
Gln Glu Gly Arg Gly Ala Arg Ala Lys Asp Asp Arg Pro Gln Leu Pro
            20                  25                  30
Ala Ala Val Ala Gln Thr Arg Asp Ser Ile Pro Gly Ala Asn Glu Asp
        35                  40                  45
Glu Thr Glu Thr Ser Glu Ala Ala Lys Ala Leu Leu Lys Gln Gln Val
    50                  55                  60
Gln Ala Leu Thr Ala Glu Val Glu Glu Met Glu Arg Lys Ile Ala Glu
65                  70                  75                  80
Val Thr Gln Lys Arg Asn Ala Glu Arg Lys Ser Lys Gly Lys Ile Gly
                85                  90                  95
Thr Ser Val Lys Asp Arg Asp Lys Gly Ala Gly Ala Phe Thr Lys Lys
            100                 105                 110
Gln Gln Gln Leu Leu Asp Ile Asn Arg Arg Asp Thr Ser Arg Ser Lys
        115                 120                 125
Arg Met Gln Glu Leu Met Arg Gln Val Gln Ser Ile Trp Lys Gln Ile
    130                 135                 140
Ser Gln His Lys Trp Ala Trp Pro Phe Met Lys Pro Val Asp Val Lys
145                 150                 155                 160
Gly Leu Gly Leu His Asp Tyr Tyr Asp Val Ile Glu Lys Pro Met Asp
                165                 170                 175
Leu Gly Thr Ile Lys Asn Lys Leu Asp Val Lys Asp Gly Leu Gly Tyr
            180                 185                 190
Gln His Val Gln Glu Val Cys Asp Asp Val Arg Leu Val Phe Ser Asn
        195                 200                 205
Ala Met Thr Tyr Asn Pro Glu Gly Ser Asp Val Tyr Val Met Ser Lys
    210                 215                 220
Thr Leu Ser Asp Lys Phe Glu Glu Lys Trp Lys Thr Leu Ile Glu Pro
225                 230                 235                 240
Lys Leu Gln Glu Glu Leu Lys Arg Ser His Asp Asp Ser Glu Val Gln
                245                 250                 255
Ala Asn Glu Gly Gly Val Pro Val Val Glu Glu Ile Asp Thr Glu Lys
            260                 265                 270
Val Ile Glu Gln Tyr Ala Leu Gln Leu Glu Glu Leu Asp Lys Gln Leu
        275                 280                 285
Glu Glu Leu Lys Gln Gln Ala Thr Pro Lys Phe Ser Arg Ala Met Ser
    290                 295                 300
Val Glu Glu Lys Arg His Leu Gly Gln Ser Leu Gly Arg Leu Pro Pro
305                 310                 315                 320
Asp Asn Leu Ser His Val Ile Gln Ile Ile Ala Gln Lys Asn Pro Ser
                325                 330                 335
Phe Asn Ile Asn Ser Asp Glu Val Glu Val Asp Ile Asp Ala Gln Asp
            340                 345                 350
Pro Ala Thr Leu Trp Arg Leu Gln Arg Tyr Val Gln Ala Val Leu Ser
        355                 360                 365
Gly Ser Ala Ala Arg Gln Ala Thr Pro Arg Ser Gln Ala Thr Lys Arg
    370                 375                 380
Asn Gly Ser Ser Leu Leu Lys Lys Asn Ser Ser Lys Arg Ser Lys Ala
385                 390                 395                 400
```

Thr Thr Ser

<210> 349
<211> 268
<212> PRT
<213> Physcomitrella patens

<400> 349
Met Thr Ser Tyr Ile Pro Gln Ile Ser Ser His Lys Trp Ala Trp Pro
1 5 10 15
Phe Met Lys Pro Val Asp Val Lys Gly Leu Gly Leu His Asp Tyr Tyr
20 25 30
Glu Val Ile Glu Lys Pro Met Asp Leu Gly Thr Ile Lys Asn Lys Met
35 40 45
Asp Ala Lys Asp Ala Ser Gly Tyr Gln His Val Gln Glu Val Tyr Gln
50 55 60
Asp Val Arg Leu Val Phe Ser Asn Ala Met Lys Tyr Asn Pro Glu Gly
65 70 75 80
Ser Asp Val Tyr Val Met Ser Lys Thr Leu Ser Glu Lys Phe Glu Glu
85 90 95
Lys Trp Lys Thr Leu Val Glu Pro Lys Leu His Glu Glu Glu Ser Lys
100 105 110
Lys Ser Gln Glu Asp Asn Glu Val Arg Thr Lys Glu Pro Gly Val Gln
115 120 125
Ala Val Glu Glu Ile Asp Thr Glu Glu Leu Thr Glu Gln Tyr Ala Leu
130 135 140
Gln Leu Glu Glu Leu Asp Lys Gln Leu Glu Asp Leu Lys Gln Gln Ala
145 150 155 160
Thr Pro Asn Ser Arg Ala Met Ser Val Glu Glu Arg Arg His Leu Gly
165 170 175
Gln Ser Leu Gly Arg Leu Pro Pro Asp Asn Leu Ser His Val Ile Gln
180 185 190
Ile Ile Ala Gln Lys Asn Pro Ser Phe Asn Met Asn Ser Asp Glu Val
195 200 205
Glu Val Asp Ile Asp Ala Gln Asp Pro Ala Thr Leu Trp Arg Leu Gln
210 215 220
Arg Tyr Val Gln Ala Val Leu Ser Gly Ser Gly Gly Arg Gln Ala Thr
225 230 235 240
Pro Arg Asn Gln Ala Ala Lys Arg Asn Gly Ser Ser Leu His Ser Lys
245 250 255
Ser Ser Ser Lys Arg Ser Lys Lys Val Thr Val Pro
260 265

<210> 350
<211> 384
<212> PRT
<213> Populus trichocarpa

<400> 350
Met Glu Pro Ser Val Phe Tyr Phe Gly Asn Leu Pro Val Arg Asn Pro
1 5 10 15
Asp Gly Asn Asp Ala Asp Thr Glu Gly Phe Lys His Ser Val Asp Glu
20 25 30
Ile Phe Gln Lys Val Asp Lys Leu Glu Gln Arg Met Asn Gly Val Glu
35 40 45
Gln Phe Tyr Leu Asp Ile Ser Lys Lys Gln Gln Ser Gly Ser Ser Lys
50 55 60
Gly Gly Gly Ser Ser Ile Val Lys Asp Lys Asp Lys Glu Arg His Val

```
65                  70                  75                  80
Thr Ser Ile Arg Lys Gln Gln Gln Asp Ala Ser Lys Arg Glu Ala Ala
                85                  90                  95
Ala Ala Lys Arg Met Gln Glu Leu Met Arg Gln Phe Gly Thr Ile Leu
            100                 105                 110
Arg Gln Ile Thr Gln His Lys Trp Ala Trp Pro Phe Met Gln Pro Val
        115                 120                 125
Asp Val Lys Gly Leu Arg Leu His Asp Tyr Tyr Glu Val Ile Asp Lys
    130                 135                 140
Pro Met Asp Phe Ser Thr Ile Lys Asn Gln Met Glu Ala Lys Asp Gly
145                 150                 155                 160
Thr Gly Tyr Lys Asn Val Arg Glu Ile Ser Ala Asp Val Arg Leu Val
                165                 170                 175
Phe Lys Asn Ala Met Lys Tyr Asn Asp Glu Arg Ser Asp Val His Val
            180                 185                 190
Met Ala Lys Thr Leu Leu Gly Lys Phe Glu Glu Lys Trp Leu Gln Leu
        195                 200                 205
Leu Pro Lys Val Thr Glu Glu Glu Lys Arg Arg Glu Asp Glu Glu Val
    210                 215                 220
Glu Ala Lys Leu Asp Met Gln Leu Ala Gln Glu Ala Ala His Ala Lys
225                 230                 235                 240
Met Ala Arg Asp Leu Ser Asn Glu Leu Tyr Glu Val Asp Met His Leu
                245                 250                 255
Glu Glu Leu Arg Asp Ile Val Val Gln Lys Cys Arg Lys Met Ser Thr
            260                 265                 270
Glu Glu Lys Arg Lys Leu Gly Val Ala Leu Thr Arg Leu Ser Pro Glu
        275                 280                 285
Asp Leu Thr Lys Ala Leu Glu Ile Val Ala Arg Ser Asn Pro Gly Phe
    290                 295                 300
Gln Ala Thr Ala Glu Glu Val Asp Leu Asp Ile Asp Ala Gln Thr Glu
305                 310                 315                 320
Ser Thr Leu Trp Arg Leu Lys Phe Leu Val Lys Asp Val Leu Glu Val
                325                 330                 335
Gln Gly Lys Ser Ala Ala Ser Thr Gly Gly Asn Asn Asn Asn Asn Asn
            340                 345                 350
Asn Asn Lys Asn Thr Ser Asn Asn Asn Lys Arg Lys Arg Glu Ile Cys
        355                 360                 365
Asp Ala Ile Ala Lys Thr Ala Lys Lys Arg Ser Lys Lys Pro Ser Ser
    370                 375                 380

<210>  351
<211>  370
<212>  PRT
<213>  Populus trichocarpa

<400>  351
Met Glu Ala Ile Ser Pro Ser Ile Val Asp Ser Gly Asn Leu Pro Ile
1               5                   10                  15
Arg Asn Ser Asp Ala Glu Ala Glu Gly Phe Lys His Ser Val Asp Glu
            20                  25                  30
Ile Leu Gln Lys Val Asp Lys Leu Glu Gln Arg Val Asn Glu Val Glu
        35                  40                  45
Gln Phe Tyr Ser Lys Asn Thr Ser Lys Lys Gln Gln Ser Gly Ser Ser
    50                  55                  60
Lys Gly Gly Ser Ser Thr Val Lys Asp Lys Asp Lys Glu Arg His Ile
65                  70                  75                  80
Pro Thr Ala Ala Lys Arg Met Gln Glu Leu Met Arg Gln Phe Gly Thr
                85                  90                  95
Ile Leu Arg Gln Ile Thr Gln His Lys Trp Ala Trp Pro Phe Met Gln
```

```
                100                 105                 110
Pro Val Asp Val Lys Gly Leu Gly Leu His Asp Tyr Tyr Glu Val Ile
        115                 120                 125
Asp Lys Pro Met Asp Phe Ser Thr Ile Lys Asn Gln Met Glu Ala Lys
    130                 135                 140
Asp Gly Thr Gly Tyr Lys Ser Val Arg Glu Ile Cys Ala Asp Val Arg
145                 150                 155                 160
Leu Val Phe Lys Asn Ala Met Lys Tyr Asn Asp Glu Arg Ser Asp Val
                165                 170                 175
His Val Met Ala Lys Thr Leu Leu Gly Lys Phe Glu Glu Lys Trp Leu
            180                 185                 190
Gln Phe Leu Pro Lys Val Thr Glu Glu Glu Lys Arg Arg Glu Glu Glu
        195                 200                 205
Glu Ala Glu Ala Gln Leu Asp Met Gln Leu Ala Gln Glu Ala Ala His
    210                 215                 220
Ala Lys Met Ala Arg Asp Leu Gly Asn Glu Leu Tyr Glu Val Asp Met
225                 230                 235                 240
His Leu Glu Glu Leu Arg Glu Met Val Val Gln Lys Cys Arg Lys Met
                245                 250                 255
Ser Thr Glu Glu Lys Arg Lys Leu Gly Ala Ala Leu Thr Arg Leu Ser
            260                 265                 270
Pro Glu Asp Leu Thr Lys Ala Leu Glu Ile Val Ala Gln Asn Asn Pro
        275                 280                 285
Gly Phe Gln Ala Thr Ala Glu Glu Val Asp Leu Asp Ile Asp Ala Gln
    290                 295                 300
Ser Glu Thr Thr Leu Trp Arg Leu Lys Phe Phe Val Lys Asp Ala Leu
305                 310                 315                 320
Glu Val Gln Gly Lys Ser Ala Ala Ser Ala Gly Gly Arg Asn Asn Thr
                325                 330                 335
Thr Thr Pro Ser Asn Asn Asn Asn Asn Asn Asn Lys Arg Lys Arg Glu
            340                 345                 350
Ile Cys Asp Ala Ile Ala Lys Thr Ala Lys Lys Arg Ser Lys Lys Pro
        355                 360                 365
Ser Ser
    370
```

<210> 352
<211> 377
<212> PRT
<213> Populus trichocarpa

<400> 352

```
Met Glu His Met Ile Arg Ser Ile Arg Gly Ser Arg Asn Val Gly Thr
1               5                   10                  15
Gly Asn Ile Glu Val Asp Asn Ser Glu Met Glu Phe Glu Gln Arg Val
            20                  25                  30
Asp Ala Val Glu Arg Tyr Tyr Ala Asp Asn Lys Glu Leu Asn Thr Ala
        35                  40                  45
Lys Cys Ser Ser Ile Leu Lys Asp Lys Ile Lys Lys Lys His Leu Met
    50                  55                  60
Thr Val Glu Lys Gln Gln Gln Asp Ser Glu Lys Ile Thr Gln Asp Leu
65                  70                  75                  80
Met Arg Gln Phe Ala Val Ile Phe Arg Gln Ile Ala Gln His Lys Trp
                85                  90                  95
Ala Trp Pro Phe Leu Glu Pro Val Asp Val Glu Gly Leu Cys Leu His
            100                 105                 110
Asp Tyr Tyr Glu Val Ile Glu Lys Pro Met Asp Phe Arg Thr Ile Lys
        115                 120                 125
Asn Arg Met Glu Ala Lys Asp Gly Thr Gly Tyr Lys Asn Val Arg Glu
```

```
    130                 135                 140
Ile Tyr Ala Asp Val Arg Leu Val Phe Lys Asn Ala Met Lys Tyr Asn
145                 150                 155                 160
Asp Glu Arg Asp Asp Val His Val Met Ala Arg Thr Leu Leu Glu Lys
                165                 170                 175
Phe Glu Glu Lys Trp Leu Gln Leu Leu Pro Lys Val Ala Glu Glu Glu
            180                 185                 190
Lys Arg Arg Glu Lys Glu Gln Thr Ala Thr Gln Val Ala Thr Lys Leu
        195                 200                 205
Ala Glu Glu Ser Ser Tyr Ala Asn Met Ala Gln Asp Leu Ser Asn Glu
    210                 215                 220
Leu His Gly Val Asp Met Gln Leu Glu Arg Ile Arg Glu Met Val Val
225                 230                 235                 240
Arg Asn Ser Arg Lys Ile Ser Thr Glu Glu Lys Lys Lys Leu Gly Thr
                245                 250                 255
Ala Leu Thr Gln Leu Ser His Gln Asp Leu Ile Arg Ala Leu Glu Ile
            260                 265                 270
Val Ala Glu His Asn Pro Ser Phe Gln Ala Thr Ala Gln Glu Val Asn
        275                 280                 285
Leu Asp Met Asp Thr Gln Ser Asp Val Thr Leu Trp Arg Leu Lys Val
    290                 295                 300
Phe Val Gln Asp Ala Leu Lys Val Ser Gly Arg Asn Ser Gly Gly Thr
305                 310                 315                 320
Gly Met Gly Cys Asn Ser Asn Ile Asn Asn Asp Asp Asn Lys Ala Lys
                325                 330                 335
Ile Asn Ile Asn Asn Lys Asn Arg Asn Thr Thr Ala Ser Lys Arg Lys
            340                 345                 350
Gly Glu Arg Cys Asp Ala Val Thr Lys Ala Ser Ala Lys Arg Thr Lys
        355                 360                 365
Lys Ile Ser Leu Asn Ser Leu Asn Pro
    370                 375

<210>  353
<211>  346
<212>  PRT
<213>  Sorghum bicolor

<400>  353
Met Thr Pro Ala Asn Gly Ala Pro Ala Pro Ala Ala Glu Ser Val Pro
1               5                   10                  15
Pro Glu Val Glu Ser Glu Ala Asp Ala Phe Arg Arg Gln Val Asp Asp
            20                  25                  30
Leu Val Ser Lys Thr Asp Val Leu Glu Lys Arg Val Lys Glu Val Val
        35                  40                  45
Asp Phe Tyr Asp Gly Lys Lys His Gly Ser Gly Gly Arg Lys Gly Gly
    50                  55                  60
Gly Gly Gly Arg His Gly Ala Tyr Ser Arg Gly Met Pro Asp Leu Met
65                  70                  75                  80
Arg Gln Phe Gly Val Leu Leu Lys Glu Ile Thr Ser His Lys Asp Ala
                85                  90                  95
Trp Pro Phe Leu Lys Pro Val Asp Val Val Thr Leu His Ile Pro Asp
            100                 105                 110
Tyr His Lys Ile Ile Thr Gln Pro Met Asp Phe Ser Thr Ile Gln Lys
        115                 120                 125
Lys Met Glu Arg Lys Asp Gly Thr Cys Tyr Thr Asn Val Arg Glu Ile
    130                 135                 140
Cys Ser Asp Val Arg Leu Ile Phe Ala Asn Ala Met Lys Tyr Asn Asp
145                 150                 155                 160
Asp Gln Asn Val Val His Leu Met Ala Lys Ser Leu Leu Glu Lys Phe
```

```
                165                 170                 175
Glu Glu Lys Trp Leu His Phe Leu Pro Lys Val Glu Ser Glu Glu Lys
            180                 185                 190
Arg Gln Lys Glu Glu Glu Ser Lys Gly Val Ala Ala Thr Asn Thr Ser
        195                 200                 205
Arg Glu Val Ala Ile Ala Lys Leu Ala Lys Asp Thr Asp Asp Glu Leu
    210                 215                 220
Asn Gln Ile Asn Arg Lys Leu Glu Glu Leu Arg Lys Met Val Val His
225                 230                 235                 240
Arg Cys Arg Lys Met Thr Thr Asp Glu Lys Arg Lys Leu Gly Ala Gly
                245                 250                 255
Ile Cys His Leu Ser Pro Asp Asp Leu Asn Lys Ala Leu Glu Ile Val
            260                 265                 270
Ala Gln Asp Asn Pro Ser Phe Gln Thr Lys Ala Glu Glu Val Asp Leu
        275                 280                 285
Asp Met Asp Ala Gln Ser Glu Thr Thr Leu Trp Arg Leu Lys Phe Phe
    290                 295                 300
Val Arg Glu Ala Leu Glu Arg Gln Ala Asn Val Ala Ser Gly Lys Met
305                 310                 315                 320
Asp Glu Asn Ala Lys Arg Lys Arg Glu Ile Cys Asn Ala Leu Ala Lys
                325                 330                 335
Thr Ala Ser Lys Arg Ile Lys Lys Gln Pro
            340                 345

<210>  354
<211>  351
<212>  PRT
<213>  Triticum aestivum

<400>  354
Met Glu Gly Ala Trp Pro His Pro Ala Pro Met Glu Pro Thr Thr Ala
1               5                   10                  15
Ala Asp Gly Pro Gln Val Ser Glu Val Asn Ser Phe Arg Arg Gln Val
            20                  25                  30
Asp Asp Leu Leu Ser Lys Thr Asp Val Leu Glu Lys Arg Val Asn Glu
        35                  40                  45
Val Val Gly Phe Tyr Asn Ser Lys Lys His Ser Ser Gly Gly Arg Lys
    50                  55                  60
Ala Gly Gly Arg Tyr Ala Glu Asn Gly Ala Arg Asp Ser His Gly Asn
65                  70                  75                  80
Gly Met Pro Asp Leu Met Arg Gln Phe Ala Gly Ile Ile Arg Gln Ile
                85                  90                  95
Thr Ser His Glu Trp Ala Gln Pro Phe Leu Gln Pro Val Asp Val Val
            100                 105                 110
Gly Leu Gln Leu Asp Asp Tyr His Gln Ile Ile Thr Lys Pro Met Asp
        115                 120                 125
Phe Ser Thr Ile Arg Asn Lys Met Glu Gly Lys Glu Ser Thr Thr Tyr
    130                 135                 140
Asn Ser Val Arg Glu Ile Tyr Ser Asp Val Arg Leu Val Phe Thr Asn
145                 150                 155                 160
Ala Met Lys Tyr Asn Val Gln Gly His Pro Val His Ile Met Ala Lys
                165                 170                 175
Phe Leu Leu Glu Arg Phe Glu Glu Lys Trp Leu His Leu Leu Pro Lys
            180                 185                 190
Val Glu Asn Glu Glu Arg Glu Arg Glu Glu Pro Asn Asp Ala Pro Thr
        195                 200                 205
Ile Ser Ile Ser Pro Glu Ala Ala Ile Ala Ile Leu Ala Glu Asp Thr
    210                 215                 220
Gly Asn Glu Leu Asn Glu Ile Asn Lys Gln Leu Glu Glu Leu Gln Lys
```

```
225                 230                 235                 240
Met Val Val Gln Arg Cys Arg Lys Met Thr Thr Asp Glu Lys Arg Lys
                245                 250                 255
Leu Gly Ala Gly Leu Cys Gln Leu Ser Pro Glu Asp Leu Asn Lys Ala
            260                 265                 270
Leu Glu Leu Val Ala Gln Asp Asn Pro Ser Phe Gln Thr Thr Ala Glu
        275                 280                 285
Glu Val Asp Leu Asp Met Asp Ala Gln Ser Glu Thr Thr Leu Trp Arg
    290                 295                 300
Leu Lys Phe Phe Val Arg Glu Ala Leu Glu Gln Gln Ala Asn Val Gly
305                 310                 315                 320
Val Val Ala Cys Gly Lys Ile Asp Glu Asn Thr Lys Arg Ser Arg Asp
                325                 330                 335
Met Tyr Asn Ala Leu Ala Lys Thr Val Ser Lys Arg Ile Lys Lys
            340                 345                 350
```

<210> 355
<211> 438
<212> PRT
<213> Vitis vinifera

<400> 355

```
Met Ala Phe Arg Ile Ala Leu Ser Pro Ser Val Ile Thr Glu Arg Gly
1               5                   10                  15
Phe Leu Leu Phe Ile Ala Ser Arg Lys Val Ser Lys Thr His Lys Thr
            20                  25                  30
Glu Arg Leu Thr Asn Arg Pro Gln Cys Phe His Lys Glu Asp Arg Glu
        35                  40                  45
Thr Arg Pro Pro Leu Glu Cys Leu Val Ser Asp Glu Pro Met Asp Ala
    50                  55                  60
Ser Ile Thr Asn Val Arg Asn Val Glu Ile Gly Lys His Lys Gly Asn
65                  70                  75                  80
Thr Asp Gln Val Glu Gly Phe Lys Ser Cys Val Asp Asp Ile Phe Thr
                85                  90                  95
Lys Val Asp Lys Leu Glu Gln Arg Val Asn Glu Val Glu Leu Phe Tyr
            100                 105                 110
Leu Thr Ala Ser Lys Arg Gln Leu Asn Gly Tyr Lys Gly Ser Ser Val
        115                 120                 125
Leu Lys Asp Lys Glu Arg His Val Ala Ser Ala Lys Lys Gln Gln Gln
    130                 135                 140
Asp Ala Ser Arg Arg Glu Ala Ala Ala Val Lys Arg Met Gln Glu Leu
145                 150                 155                 160
Met Arg Gln Phe Gly Thr Ile Leu Arg Gln Ile Met Gln His Lys Trp
                165                 170                 175
Ala Gly Pro Phe Leu His Pro Val Asp Val Glu Gly Leu Gly Leu His
            180                 185                 190
Asp Tyr Tyr Glu Val Ile Asp Lys Pro Met Asp Phe Ser Thr Ile Lys
        195                 200                 205
Asn Gln Met Glu Ala Lys Asp Gly Thr Gly Tyr Lys Asn Val Arg Glu
    210                 215                 220
Ile Cys Ala Asp Val Arg Leu Val Phe Lys Asn Ala Met Lys Tyr Asn
225                 230                 235                 240
Asp Glu Arg His Asp Val His Val Met Ala Lys Thr Leu Leu Gly Lys
                245                 250                 255
Phe Glu Glu Lys Trp Leu Gln Leu Leu Pro Lys Val Ala Glu Glu Glu
            260                 265                 270
Lys Arg Arg Glu Glu Glu Glu Ala Glu Ala Gln Leu Asp Met Gln Leu
        275                 280                 285
Ala Gln Glu Ala Ala His Ala Lys Met Ala Arg Glu Ile Ser Asn Glu
```

```
    290                 295                 300
Leu Tyr Asp Ile Asp Met His Leu Glu Glu Val Arg Glu Met Val Ile
305                 310                 315                 320
Arg Lys Cys Arg Lys Met Ser Thr Glu Glu Lys Arg Lys Leu Gly Ala
                325                 330                 335
Ala Leu Ser Arg Leu Ser Ala Glu Asp Leu Ser Lys Ala Leu Glu Ile
            340                 345                 350
Val Ala Gln Asn Asn Pro Ser Phe Gln Ala Thr Ala Glu Glu Val Asp
        355                 360                 365
Leu Asp Ile Asp Ala Gln Thr Glu Ser Thr Leu Trp Arg Leu Lys Phe
    370                 375                 380
Phe Val Lys Asp Ala Leu Glu Val Gln Gly Lys Ser Ser Ala Ser Lys
385                 390                 395                 400
Gly Asp Asn Thr Asn Thr Thr Thr Thr Ala Thr Asn Asn Asn Lys Arg
                405                 410                 415
Lys Lys Glu Ile Cys Asp Ala Ile Ala Lys Thr Ala Lys Lys Lys Ser
            420                 425                 430
Lys Lys Leu Pro Leu Asp
        435

<210>  356
<211>  284
<212>  PRT
<213>  Vitis vinifera

<400>  356
Met Ala Leu Trp His Ile Ser Leu Met Ser Leu Arg Phe Tyr Trp Gln
1               5                   10                  15
Ile Met Gln His Lys Trp Ala Gly Pro Phe Leu His Pro Val Asp Val
            20                  25                  30
Glu Gly Leu Gly Leu His Asp Tyr Tyr Glu Val Ile Asp Lys Pro Met
        35                  40                  45
Asp Phe Ser Thr Ile Lys Asn Gln Met Glu Ala Lys Asp Gly Thr Gly
    50                  55                  60
Tyr Lys Asn Val Arg Glu Ile Cys Ala Asp Val Arg Leu Val Phe Lys
65                  70                  75                  80
Asn Ala Met Lys Tyr Asn Asp Glu Arg His Asp Val His Val Met Ala
                85                  90                  95
Lys Thr Leu Leu Gly Lys Phe Glu Glu Lys Trp Leu Gln Leu Leu Pro
            100                 105                 110
Lys Val Ala Glu Glu Glu Lys Arg Arg Glu Glu Glu Glu Ala Glu Ala
        115                 120                 125
Gln Leu Asp Met Gln Leu Ala Gln Glu Ala Ala His Ala Lys Met Ala
    130                 135                 140
Arg Glu Ile Ser Asn Glu Leu Tyr Asp Ile Asp Met His Leu Glu Glu
145                 150                 155                 160
Val Arg Glu Met Val Ile Arg Lys Cys Arg Lys Met Ser Thr Glu Glu
                165                 170                 175
Lys Arg Lys Leu Gly Ala Ala Leu Ser Arg Leu Ser Ala Glu Asp Leu
            180                 185                 190
Ser Lys Ala Leu Glu Ile Val Ala Gln Asn Asn Pro Ser Phe Gln Ala
        195                 200                 205
Thr Ala Glu Glu Val Asp Leu Asp Ile Asp Ala Gln Thr Glu Ser Thr
    210                 215                 220
Leu Trp Arg Leu Lys Phe Phe Val Lys Asp Ala Leu Glu Val Gln Gly
225                 230                 235                 240
Lys Ser Ser Ala Ser Lys Gly Asp Asn Thr Asn Thr Thr Thr Thr Ala
                245                 250                 255
Thr Asn Asn Asn Lys Arg Lys Lys Glu Ile Cys Asp Ala Ile Ala Lys
```

```
        260                 265                 270
Thr Ala Lys Lys Lys Ser Lys Lys Leu Pro Leu Asp
        275                 280

<210>  357
<211>  360
<212>  PRT
<213>  Zea mays

<400>  357
Met Thr Pro Ala Asn Gly Ala Pro Ala Arg Ala Ala Glu Ala Gly Pro
1               5                   10                  15
His Glu Val Glu Ser Glu Ala Asp Ala Phe Arg Arg Gln Val Asp Asp
            20                  25                  30
Leu Val Ser Lys Thr Asp Val Leu Glu Arg Arg Val Lys Glu Val Ala
        35                  40                  45
Asp Phe Tyr Asp Gly Lys Lys His Gly Ser Gly Gly Arg Lys Gly Gly
    50                  55                  60
Gly Gly Gly Arg Tyr Gly Ala Ser Ser Arg Gly Met Pro Asp Leu Met
65                  70                  75                  80
Arg Gln Phe Gly Val Leu Leu Lys Glu Ile Thr Ser His Lys Asp Ala
                85                  90                  95
Trp Pro Phe Leu Glu Pro Val Asp Val Val Thr Leu Gln Leu Pro Asp
            100                 105                 110
Tyr His Asn Ile Ile Thr Gln Pro Met Asp Phe Ser Thr Ile Gln Lys
        115                 120                 125
Lys Met Glu Arg Lys Asp Gly Thr Cys Tyr Thr Asn Val Arg Glu Ile
    130                 135                 140
Cys Ser Asp Val Arg Leu Ile Phe Ala Asn Ala Met Lys Tyr Asn Asp
145                 150                 155                 160
Asp Gln Asn Val Ile His Leu Met Ala Lys Ser Leu Leu Glu Lys Phe
                165                 170                 175
Glu Glu Lys Trp Leu His Phe Leu Pro Lys Val Glu Ser Glu Glu Lys
            180                 185                 190
Arg Gln Lys Glu Glu Glu Ser Lys Gly Val Ala Ala Thr Asn Thr Ser
        195                 200                 205
Arg Glu Val Ala Ile Val Lys Leu Ala Lys Asp Thr Asp Asp Glu Leu
    210                 215                 220
Asn Gln Ile Asn Lys Lys Leu Glu Glu Leu Arg Lys Met Val Val His
225                 230                 235                 240
Arg Cys Arg Lys Met Thr Thr Asp Glu Lys Arg Lys Leu Gly Ala Gly
                245                 250                 255
Ile Cys His Leu Ser Pro Asp Asp Leu Ser Lys Ala Leu Glu Ile Val
            260                 265                 270
Ala Gln Asp Asn Pro Ser Phe Gln Thr Lys Ala Glu Glu Val Asp Leu
        275                 280                 285
Asp Met Asp Ala Gln Ser Glu Thr Thr Leu Trp Arg Leu Lys Phe Phe
    290                 295                 300
Val Arg Glu Ala Leu Glu Arg Gln Gly His Val Ala Ser Gly Lys Met
305                 310                 315                 320
Asp Tyr Asn Ala Gln Gly Lys Glu Gly Asp Met Gln Cys Ser Gly Gln
                325                 330                 335
Asn His Leu Glu Thr Asp Gln Glu Ala Ala Leu Ala Ile Phe Pro Tyr
            340                 345                 350
His His Ser Arg Pro Leu Ile Val
        355                 360

<210>  358
<211>  677
```

```
<212>  DNA
<213>  Oryza sativa

<400>  358
ttgtacaaaa aagcaggctt aaacaatggc gctcaccaac cacatcctcg cgccggcagc     60
cgcagcagcc tgctgcttcg gacggcgggt tcctctgccg ccgcctcatc agctcgccgt    120
gcggaggaag caaaagagcg tggtggtggc catggccgac ctgctgggcg actttggcgc    180
gcgcgacccc ttcccggagg agatcgagag caacttcggc gagagggtgc tgggcaacgt    240
cgacaccctc cacaacatcc tcatccccac gctctccgtg ctctccatcg cgcgcctccc    300
cctcgagcct aaccccgcgc ccgtcgacgc tgccgacgcc cgccgcctcc tccacaaggt    360
ggttggctgg cgcctcctcg acgacgccga cggcatgcgt ctgcagtgcg tctggaaggt    420
cagggacgaa gcctgcggcc acgagctcgt cgccaggatc aacgccgcgg ttgacggcgc    480
cccggccacc gtcgtcttcg aggcccccaa ccaggtcagg gccgagctgc agacgccctc    540
cgccggcggc ctcaccgtca atgacttcat cgtcgcggct cgcatagaca aggtcaagac    600
ggtcgacctt atccccaaga agagagtctg ggcctgattc ttcattcaca tatatcccct    660
tacccagctt tcttgta                                                   677

<210>  359
<211>  203
<212>  PRT
<213>  Oryza sativa

<400>  359
Met Ala Leu Thr Asn His Ile Leu Ala Pro Ala Ala Ala Ala Ala Cys
1               5                   10                  15
Cys Phe Gly Arg Arg Val Pro Leu Pro Pro Pro His Gln Leu Ala Val
            20                  25                  30
Arg Arg Lys Gln Lys Ser Val Val Val Ala Met Ala Asp Leu Leu Gly
        35                  40                  45
Asp Phe Gly Ala Arg Asp Pro Phe Pro Glu Glu Ile Glu Ser Asn Phe
    50                  55                  60
Gly Glu Arg Val Leu Gly Asn Val Asp Thr Leu His Asn Ile Leu Ile
65                  70                  75                  80
Pro Thr Leu Ser Val Leu Ser Ile Ala Arg Leu Pro Leu Glu Pro Asn
                85                  90                  95
Pro Ala Pro Val Asp Ala Ala Asp Ala Arg Arg Leu Leu His Lys Val
            100                 105                 110
Val Gly Trp Arg Leu Leu Asp Asp Ala Asp Gly Met Arg Leu Gln Cys
        115                 120                 125
Val Trp Lys Val Arg Asp Glu Ala Cys Gly His Glu Leu Val Ala Arg
    130                 135                 140
Ile Asn Ala Ala Val Asp Gly Ala Pro Ala Thr Val Val Phe Glu Ala
145                 150                 155                 160
Pro Asn Gln Val Arg Ala Glu Leu Gln Thr Pro Ser Ala Gly Gly Leu
                165                 170                 175
Thr Val Asn Asp Phe Ile Val Ala Ala Arg Ile Asp Lys Val Lys Thr
            180                 185                 190
Val Asp Leu Ile Pro Lys Lys Arg Val Trp Ala
        195                 200

<210>  360
<211>  753
<212>  DNA
<213>  Allium cepa

<400>  360
cacagcggct gctcgtcttt tgtcattctc cgaagcgaag ccgtctactc aattaaccgc     60
atcgtttctc gttagtaact atggacgttt gtgttcacgt atgttcaaga ttaatcgcaa    120
aagcaatgat gtgcattcac acatgaggtt acgctgtgca tttagttccc tttcttcatc    180
```

```
ccaagatttg acaagcaaaa aatgtgtgcc atgcaactca aaagatattc agccaatatc    240
tgaacaatca gcaaatgaac tactctcaaa ggtgcaaggt tgggaaatgt caaatgatgt    300
tggggtaatg aagctgcatc gttcttggaa agtcaagagt ttcatgaaag gccttgattt    360
tttcaagctt gttgctgatg tggctgaggc agaaggtcac catccagatc ttcatcttgt    420
tggatggaat aacgtgaaaa ttgatatatg gacccattca gttggaggtc tgactgaaaa    480
cgattttatc ctagctgcaa agatcaactc tcttgaaata gagcatctac taagcaagaa    540
ggggagaaaa tgactgctct tctgcagttt gaaatcaatc tgaagcaaat cttcttcttt    600
ttttggtgtt tgtgctgttt gccttctcgt tatatgctat gctgcatgat caaaagtagt    660
tgtgtttaaa agttaggtca ttttgttttg atagttaaat caaaatttat atttgagcat    720
aaagatgaac tactagctgt ggtacatgct tgg                                 753

<210>  361
<211>  150
<212>  PRT
<213>  Allium cepa

<400>  361
Met Phe Lys Ile Asn Arg Lys Ser Asn Asp Val His Ser His Met Arg
1               5                   10                  15
Leu Arg Cys Ala Phe Ser Ser Leu Ser Ser Ser Gln Asp Leu Thr Ser
            20                  25                  30
Lys Lys Cys Val Pro Cys Asn Ser Lys Asp Ile Gln Pro Ile Ser Glu
        35                  40                  45
Gln Ser Ala Asn Glu Leu Leu Ser Lys Val Gln Gly Trp Glu Met Ser
    50                  55                  60
Asn Asp Val Gly Val Met Lys Leu His Arg Ser Trp Lys Val Lys Ser
65                  70                  75                  80
Phe Met Lys Gly Leu Asp Phe Phe Lys Leu Val Ala Asp Val Ala Glu
                85                  90                  95
Ala Glu Gly His His Pro Asp Leu His Leu Val Gly Trp Asn Asn Val
            100                 105                 110
Lys Ile Asp Ile Trp Thr His Ser Val Gly Gly Leu Thr Glu Asn Asp
        115                 120                 125
Phe Ile Leu Ala Ala Lys Ile Asn Ser Leu Glu Ile Glu His Leu Leu
    130                 135                 140
Ser Lys Lys Gly Arg Lys
145                 150

<210>  362
<211>  896
<212>  DNA
<213>  Arabidopsis thaliana

<400>  362
aaacttaaga aattttgtca tttaacccct cactttcacg attagtaagt attacaaaac     60
tctccccgga aatttctgct ccgcaacggc ttcggcacca atgagtcggc tcttgctacc    120
taaacttttc tcaatctcta gaacacaggt tccagctgca tcattgttca ataatctgta    180
cagacgtcac aaacgttttg ttcattggac gagtaagatg tcaacagata gtgttagatc    240
atccacaact ggtggttctg cttctggagc tagaacattt tgctccctcg cagatttatc    300
aaccaaaaag tgtgtgccat gcaatgcgaa ggatctgcgc gccatgaccg aacaaagtgc    360
ccaagaccta cttcaaaagg ttgctggatg ggatttggcc aatgacaatg atacattaaa    420
gctgcatcgg tcatggaggg tgaaaagttt tacaaagggg ctagatttct tccaacgtgt    480
agctgatatc gctgaatcag aaggtcatca cccagatttg catctggtcg gctggaataa    540
tgtgaaaatt gagatatgga cacatgcgat aggtggtttg acagaaaacg acttcattct    600
tgctgctaag atcaacgagc tccaagtgga agatcttctg aggaagaaga aagttgctaa    660
gtgaaatgga atcaagatga attctgcaag ctaaagaaac atctttaaag actggctcag    720
aggtcaatat gaataagcat cttgaagcca taactgggat gtgttggtct taaaactctt    780
tgaagcatgc cacacaagct ttagatctat gtaagatgag aacaaaatga cagagagagt    840
aatgtttctt ttgtgtctta tggttcttgt aaagctgttt ctagtcttac aatctc        896
```

<210> 363
<211> 187
<212> PRT
<213> Arabidopsis thaliana

<400> 363
Met Ser Arg Leu Leu Leu Pro Lys Leu Phe Ser Ile Ser Arg Thr Gln
1 5 10 15
Val Pro Ala Ala Ser Leu Phe Asn Asn Leu Tyr Arg Arg His Lys Arg
20 25 30
Phe Val His Trp Thr Ser Lys Met Ser Thr Asp Ser Val Arg Ser Ser
35 40 45
Thr Thr Gly Gly Ser Ala Ser Gly Ala Arg Thr Phe Cys Ser Leu Ala
50 55 60
Asp Leu Ser Thr Lys Lys Cys Val Pro Cys Asn Ala Lys Asp Leu Arg
65 70 75 80
Ala Met Thr Glu Gln Ser Ala Gln Asp Leu Leu Gln Lys Val Ala Gly
85 90 95
Trp Asp Leu Ala Asn Asp Asn Asp Thr Leu Lys Leu His Arg Ser Trp
100 105 110
Arg Val Lys Ser Phe Thr Lys Gly Leu Asp Phe Phe Gln Arg Val Ala
115 120 125
Asp Ile Ala Glu Ser Glu Gly His His Pro Asp Leu His Leu Val Gly
130 135 140
Trp Asn Asn Val Lys Ile Glu Ile Trp Thr His Ala Ile Gly Gly Leu
145 150 155 160
Thr Glu Asn Asp Phe Ile Leu Ala Ala Lys Ile Asn Glu Leu Gln Val
165 170 175
Glu Asp Leu Leu Arg Lys Lys Lys Val Ala Lys
180 185

<210> 364
<211> 931
<212> DNA
<213> Arabidopsis thaliana

<400> 364
aactcagtcc agtggtctga aaatcgtttc gctctctaaa aaccaaaact cttatcttcg 60
gtacagagca aatcacagct cccgatggcc gccacgtcat catcaccgcc gtgtaacatc 120
tccgcgtcct ccctcctcct ccgtcaacct tctcgctcaa tccttaaagt gttcggttta 180
ttaccacctg taagtaggaa taaccgtaaa ctcggccggt taacggtgac ccggtctaat 240
ctggcgcagg attttcttgg tgacttcgga gctcgtgacc cttacccgga ggagatagcg 300
agtcaattcg gagataaagt gttgggatgc caaagcactg agcacaagat tttgatacca 360
aacgcatctg ttttgtctct ctcccagctt cagtgttccc ctgtttcgtc ttcacagcct 420
cctttgtccg gcgatgatgc cagaactctc ctccacaagg ttttgggatg gagtatagtg 480
gataatgaag cgggtggtct gaaaataagg tgtatgtgga aagtgaggga ttttgggtgc 540
ggtgttgaac tcataaacag gatccataag gttgctgaag cttctggtca ttacccttct 600
cttcacttgg aaagtcctac ccaagttcga gctgaactat ttacctcttc tatcggaggg 660
ttgagcatga atgatttcat aatggcggct aaaatagatg atatcaagac ttctgatctt 720
tcccctagga aaagagcttg ggcgtgatct tcttttcatt attttggaac atttttgttt 780
gatagtatgt aatgtatgtg atgtattcta aattgtgtgt gatcggtcta catgtcgagt 840
tatatagaaa gcattgtcat aaccgcataa gagatatcac gcaacatgtc tttgagattc 900
attcatcaat caaaagaaga aagaacatca a 931

<210> 365
<211> 220
<212> PRT
<213> Arabidopsis thaliana

<400> 365

```
Met Ala Ala Thr Ser Ser Ser Pro Pro Cys Asn Ile Ser Ala Ser Ser
1               5                   10                  15
Leu Leu Leu Arg Gln Pro Ser Arg Ser Ile Leu Lys Val Phe Gly Leu
            20                  25                  30
Leu Pro Pro Val Ser Arg Asn Asn Arg Lys Leu Gly Arg Leu Thr Val
        35                  40                  45
Thr Arg Ser Asn Leu Ala Gln Asp Phe Leu Gly Asp Phe Gly Ala Arg
    50                  55                  60
Asp Pro Tyr Pro Glu Glu Ile Ala Ser Gln Phe Gly Asp Lys Val Leu
65                  70                  75                  80
Gly Cys Gln Ser Thr Glu His Lys Ile Leu Ile Pro Asn Ala Ser Val
                85                  90                  95
Leu Ser Leu Ser Gln Leu Gln Cys Ser Pro Val Ser Ser Ser Gln Pro
            100                 105                 110
Pro Leu Ser Gly Asp Asp Ala Arg Thr Leu Leu His Lys Val Leu Gly
        115                 120                 125
Trp Ser Ile Val Asp Asn Glu Ala Gly Gly Leu Lys Ile Arg Cys Met
    130                 135                 140
Trp Lys Val Arg Asp Phe Gly Cys Gly Val Glu Leu Ile Asn Arg Ile
145                 150                 155                 160
His Lys Val Ala Glu Ala Ser Gly His Tyr Pro Ser Leu His Leu Glu
                165                 170                 175
Ser Pro Thr Gln Val Arg Ala Glu Leu Phe Thr Ser Ser Ile Gly Gly
            180                 185                 190
Leu Ser Met Asn Asp Phe Ile Met Ala Ala Lys Ile Asp Asp Ile Lys
        195                 200                 205
Thr Ser Asp Leu Ser Pro Arg Lys Arg Ala Trp Ala
    210                 215                 220
```

<210> 366
<211> 896
<212> DNA
<213> Arabidopsis thaliana

<400> 366

```
aaacttaaga aattttgtca tttaacccct cactttcacg attagtaagt attacaaaac     60
tctccccgga aatttctgct ccgcaacggc ttcggcacca atgagtcggc tcttgctacc    120
taaacttttc tcaatctcta gaacacaggt tccagctgca tcattgttca ataatctgta    180
cagacgtcac aaacgttttg ttcattggac gagtaagatg tcaacagata gtgttagatc    240
atccacaact ggtggttctg cttctggagc tagaacattt tgctccctcg cagatttatc    300
aaccaaaaag tgtgtgccat gcaatgcgaa ggatctgcgc gccatgaccg aacaaagtgc    360
ccaagaccta cttcaaaagg ttgctggatg ggatttggcc aatgacaatg atacattaaa    420
gctgcatcgg tcatggaggg tgaaaagttt tacaaagggg ctagatttct tccaacgtgt    480
agctgatatc gctgaatcag aaggtcatca cccagatttg catctggtcg gctggaataa    540
tgtgaaaatt gagatatgga cacatgcgat aggtggtttg acagaaaacg acttcattct    600
tgctgctaag atcaacgagc tccaagtgga agatcttctg aggaagaaga aagttgctaa    660
gtgaaatgga atcaagatga attctgcaag ctaaagaaac atctttaaag actggctcag    720
aggtcaatat gaataagcat cttgaagcca taactgggat gtgttggtct taaaactctt    780
tgaagcatgc cacacaagct ttagatctat gtaagatgag aacaaaatga cagagagagt    840
aatgtttctt ttgtgtctta tggttcttgt aaagctgttt ctagtcttac aatctc        896
```

<210> 367
<211> 187
<212> PRT
<213> Arabidopsis thaliana

<400> 367

```
Met Ser Arg Leu Leu Leu Pro Lys Leu Phe Ser Ile Ser Arg Thr Gln
1               5                   10                  15
Val Pro Ala Ala Ser Leu Phe Asn Asn Leu Tyr Arg Arg His Lys Arg
            20                  25                  30
Phe Val His Trp Thr Ser Lys Met Ser Thr Asp Ser Val Arg Ser Ser
        35                  40                  45
Thr Thr Gly Gly Ser Ala Ser Gly Ala Arg Thr Phe Cys Ser Leu Ala
    50                  55                  60
Asp Leu Ser Thr Lys Lys Cys Val Pro Cys Asn Ala Lys Asp Leu Arg
65                  70                  75                  80
Ala Met Thr Glu Gln Ser Ala Gln Asp Leu Leu Gln Lys Val Ala Gly
                85                  90                  95
Trp Asp Leu Ala Asn Asp Asn Asp Thr Leu Lys Leu His Arg Ser Trp
            100                 105                 110
Arg Val Lys Ser Phe Thr Lys Gly Leu Asp Phe Phe Gln Arg Val Ala
        115                 120                 125
Asp Ile Ala Glu Ser Glu Gly His His Pro Asp Leu His Leu Val Gly
    130                 135                 140
Trp Asn Asn Val Lys Ile Glu Ile Trp Thr His Ala Ile Gly Gly Leu
145                 150                 155                 160
Thr Glu Asn Asp Phe Ile Leu Ala Ala Lys Ile Asn Glu Leu Gln Val
                165                 170                 175
Glu Asp Leu Leu Arg Lys Lys Lys Val Ala Lys
            180                 185

<210>  368
<211>  931
<212>  DNA
<213>  Arabidopsis thaliana

<400>  368
aactcagtcc agtggtctga aaatcgtttc gctctctaaa aaccaaaact cttatcttcg      60
gtacagagca aatcacagct cccgatggcc gccacgtcat catcaccgcc gtgtaacatc     120
tccgcgtcct ccctcctcct ccgtcaacct tctcgctcaa tccttaaagt gttcggttta     180
ttaccacctg taagtaggaa taaccgtaaa ctcggccggt taacggtgac ccggtctaat     240
ctggcgcagg attttcttgg tgacttcgga gctcgtgacc cttacccgga ggagatagcg     300
agtcaattcg gagataaagt gttgggatgc caaagcactg agcacaagat tttgatacca     360
aacgcatctg ttttgtctct ctcccagctt cagtgttccc ctgtttcgtc ttcacagcct     420
cctttgtccg gcgatgatgc cagaactctc ctccacaagg ttttgggatg gagtatagtg     480
gataatgaag cgggtggtct gaaaataagg tgtatgtgga aagtgaggga ttttgggtgc     540
ggtgttgaac tcataaacag gatccataag gttgctgaag cttctggtca ttacccttct     600
cttcacttgg aaagtcctac ccaagttcga gctgaactat ttacctcttc tatcggaggg     660
ttgagcatga atgatttcat aatggcggct aaaatagatg atatcaagac ttctgatctt     720
tcccctagga aaagagcttg ggcgtgatct tcttttcatt attttggaac atttttgttt     780
gatagtatgt aatgtatgtg atgtattcta aattgtgtgt gatcggtcta catgtcgagt     840
tatatagaaa gcattgtcat aaccgcataa gagatatcac gcaacatgtc tttgagattc     900
attcatcaat caaaagaaga aagaacatca a                                    931

<210>  369
<211>  220
<212>  PRT
<213>  Arabidopsis thaliana

<400>  369
Met Ala Ala Thr Ser Ser Ser Pro Pro Cys Asn Ile Ser Ala Ser Ser
1               5                   10                  15
Leu Leu Leu Arg Gln Pro Ser Arg Ser Ile Leu Lys Val Phe Gly Leu
            20                  25                  30
Leu Pro Pro Val Ser Arg Asn Asn Arg Lys Leu Gly Arg Leu Thr Val
```

```
        35                  40                  45
Thr Arg Ser Asn Leu Ala Gln Asp Phe Leu Gly Asp Phe Gly Ala Arg
    50                  55                  60
Asp Pro Tyr Pro Glu Glu Ile Ala Ser Gln Phe Gly Asp Lys Val Leu
65                  70                  75                  80
Gly Cys Gln Ser Thr Glu His Lys Ile Leu Ile Pro Asn Ala Ser Val
                85                  90                  95
Leu Ser Leu Ser Gln Leu Gln Cys Ser Pro Val Ser Ser Ser Gln Pro
            100                 105                 110
Pro Leu Ser Gly Asp Asp Ala Arg Thr Leu Leu His Lys Val Leu Gly
        115                 120                 125
Trp Ser Ile Val Asp Asn Glu Ala Gly Gly Leu Lys Ile Arg Cys Met
    130                 135                 140
Trp Lys Val Arg Asp Phe Gly Cys Gly Val Glu Leu Ile Asn Arg Ile
145                 150                 155                 160
His Lys Val Ala Glu Ala Ser Gly His Tyr Pro Ser Leu His Leu Glu
                165                 170                 175
Ser Pro Thr Gln Val Arg Ala Glu Leu Phe Thr Ser Ser Ile Gly Gly
            180                 185                 190
Leu Ser Met Asn Asp Phe Ile Met Ala Ala Lys Ile Asp Asp Ile Lys
        195                 200                 205
Thr Ser Asp Leu Ser Pro Arg Lys Arg Ala Trp Ala
    210                 215                 220
```

```
<210>  370
<211>  576
<212>  DNA
<213>  Hordeum vulgare

<400>  370
cggcacgagg ggccctaaag gcctaacatg gcgggtctgc tactgcgtag gttcatgcgg      60
atccagcccc gtgcgccgat gaccgccgcc ggtggagccg cgttcttcaa cgcctcgtgc     120
ccctcgtccc cccgcaccgt cgcgcacctg gaggtgagcg gggagcaggc ctctcctaaa     180
gttgaattat cagagaagag ttgtatccca tgcaattcaa tggatctaca tgctatgtca     240
gaagattctg ctaaaaagtc acttgaacag gtgactggtt gggaactgaa aaatgaaggt     300
gacattttga aattacacag agcatggaag gtgaagaatt ttgtaaaagg gcttgagttc     360
tttcagcttg ttgctgctgt tgctgaggaa gaaggtcacc acccagatct tcatctcgtg     420
agttggaaca atgtgaaaat tgatgtctgg actcattcag tcagaggttt aacagataat     480
gacttcatcc ttgctgcaaa gatcaatgaa ctcaagctag aaggtctttt gagcaagaaa     540
aaagctacca cccatgagta gctttgggag atcaag                               576

<210>  371
<211>  177
<212>  PRT
<213>  Hordeum vulgare

<400>  371
Met Ala Gly Leu Leu Leu Arg Arg Phe Met Arg Ile Gln Pro Arg Ala
1               5                   10                  15
Pro Met Thr Ala Ala Gly Gly Ala Ala Phe Phe Asn Ala Ser Cys Pro
            20                  25                  30
Ser Ser Pro Arg Thr Val Ala His Leu Glu Val Ser Gly Glu Gln Ala
        35                  40                  45
Ser Pro Lys Val Glu Leu Ser Glu Lys Ser Cys Ile Pro Cys Asn Ser
    50                  55                  60
Met Asp Leu His Ala Met Ser Glu Asp Ser Ala Lys Lys Ser Leu Glu
65                  70                  75                  80
Gln Val Thr Gly Trp Glu Leu Lys Asn Glu Gly Asp Ile Leu Lys Leu
                85                  90                  95
```

```
His Arg Ala Trp Lys Val Lys Asn Phe Val Lys Gly Leu Glu Phe Phe
            100                 105                 110
Gln Leu Val Ala Ala Val Ala Glu Glu Glu Gly His His Pro Asp Leu
        115                 120                 125
His Leu Val Ser Trp Asn Asn Val Lys Ile Asp Val Trp Thr His Ser
    130                 135                 140
Val Arg Gly Leu Thr Asp Asn Asp Phe Ile Leu Ala Ala Lys Ile Asn
145                 150                 155                 160
Glu Leu Lys Leu Glu Gly Leu Leu Ser Lys Lys Lys Ala Thr Thr His
                165                 170                 175
Glu
```

```
<210>  372
<211>  932
<212>  DNA
<213>  Hordeum vulgare

<220>
<221>  misc_feature
<222>  (873)..(873)
<223>  n is a, c, g, or t

<220>
<221>  misc_feature
<222>  (917)..(917)
<223>  n is a, c, g, or t

<400>  372
cggcacgagg gtccgtccgt ccagctgcgc atcatccatc cgtcccactg caccaccatg      60
gcgctcatgc tcagcccggc cgcgaccttc ttccccgtcg ccggcaagcc ctccgccgcc     120
gccacgagga gcctcctctt caccaccagc agcagcagca gcagcaaggg aagaggcggt     180
cgcgggaagg tggtggcgat ggcggacatc ctgggggact tcggggcgcg ggacccgttc     240
ccggaggaga tcgcgagcaa cttcggggag aagacgctgg gcaacgtgga cacgctgcac     300
cgcatcctca tccccacgct ctccgtgctc tcgctctccc gcgtgccgct cgacgcccac     360
ccggcgccgc tctctgagga ggacgcccgc aggctgctct tcaaggtggt cggctggcgc     420
ctcctcttcc ccaccaaggg cgactccgac gtgctcaagc tcgagtgcgt ctggaaggtc     480
cgggaccagg cctgcggcga cgagctcgtc gccaggatca acaaggccct cgacggcgcc     540
ggccacgccc ccgccgcgct cagcttcgag ccgcccaacc aagtcagggc ccagctctac     600
accccgtccg taggtgggct gagcgccaac gacttcatca tcgccgccag gatcgaccag     660
atcaacacca aagatcttct ccccaagaag agggtctggg catgctgatg actgcccatg     720
ccgtcttgtt cctcttcctt tgcatgcata cagggacagt tttgtttcat ggaaatggaa     780
tggaagccat cctatccatg cttgtagctc tacccatagt gtttaagacg atgatgccat     840
caaatcaaat caaatgattt cctcaacttg ttncttcccg gttcttccca atacgaggca     900
cgattacgcc aacactncta taattacaaa at                                  932

<210>  373
<211>  216
<212>  PRT
<213>  Hordeum vulgare

<400>  373
Met Ala Leu Met Leu Ser Pro Ala Ala Thr Phe Phe Pro Val Ala Gly
1               5                   10                  15
Lys Pro Ser Ala Ala Ala Thr Arg Ser Leu Leu Phe Thr Thr Ser Ser
            20                  25                  30
Ser Ser Ser Ser Lys Gly Arg Gly Gly Arg Gly Lys Val Val Ala Met
        35                  40                  45
Ala Asp Ile Leu Gly Asp Phe Gly Ala Arg Asp Pro Phe Pro Glu Glu
```

```
    50                  55                  60
Ile Ala Ser Asn Phe Gly Glu Lys Thr Leu Gly Asn Val Asp Thr Leu
65                  70                  75                  80
His Arg Ile Leu Ile Pro Thr Leu Ser Val Leu Ser Leu Ser Arg Val
                85                  90                  95
Pro Leu Asp Ala His Pro Ala Pro Leu Ser Glu Glu Asp Ala Arg Arg
            100                 105                 110
Leu Leu Phe Lys Val Val Gly Trp Arg Leu Leu Phe Pro Thr Lys Gly
        115                 120                 125
Asp Ser Asp Val Leu Lys Leu Glu Cys Val Trp Lys Val Arg Asp Gln
    130                 135                 140
Ala Cys Gly Asp Glu Leu Val Ala Arg Ile Asn Lys Ala Leu Asp Gly
145                 150                 155                 160
Ala Gly His Ala Pro Ala Ala Leu Ser Phe Glu Pro Pro Asn Gln Val
                165                 170                 175
Arg Ala Gln Leu Tyr Thr Pro Ser Val Gly Gly Leu Ser Ala Asn Asp
            180                 185                 190
Phe Ile Ile Ala Ala Arg Ile Asp Gln Ile Asn Thr Lys Asp Leu Leu
        195                 200                 205
Pro Lys Lys Arg Val Trp Ala Cys
    210                 215
```

```
<210>  374
<211>  851
<212>  DNA
<213>  Hordeum vulgare

<400>  374
tcggcacgag gcctcgtgcc gaattcggca cgaggcaaag gcctcaacat ggcgggtctg      60
ctactgcgta ggttcatgcg gatccagccc cgtgcgccga tgaccgccgc cggtggagcc     120
gcgttcttca acgcctcgtg cccctcgtcc ccccgcaccg tcgcgcacct ggaggtgagc     180
ggggagcagg cctctcctaa agttgaatta tcagagaaga gttgtatccc atgcaattca     240
atggatctac atgctatgtc agaagattct gctaaaaagt cacttgaaca ggtgactggt     300
tgggaactga aaaatgaagg tgacattttg aaattacaca gagcatggaa ggtgaagaat     360
tttgtaaaag ggcttgagtt ctttcagctt gttgctgctg ttgctgagga agaaggtcac     420
cacccagatc ttcatctcgt gagttggaac aatgtgaaaa ttgatgtctg gactcattca     480
gtcagaggtt taacagataa tgacttcatc cttgctgcaa agatcaatga actcaagcta     540
gaaggccttt tgagcaagaa aaaggctacc acccatgagt agctttggga gatcaagcaa     600
ataacccatg tttgcatgtt ctctatttga acttttaatg tgtagacccc aagaaatgtc     660
cttttgtatt ttctgtgatg attgtacatg cctgtgagtt ctacttgacc tgtgttgaga     720
tctatccgtc ctgggtgaag cgtttctccc atcatttgaa tctgggtcac cctaatactt     780
gtctgtattt ctgaatatat tcagtagttt gaaggtgcta tgtatgcaat ggcacagata     840
tatgatcatc t                                                          851

<210>  375
<211>  177
<212>  PRT
<213>  Hordeum vulgare

<400>  375
Met Ala Gly Leu Leu Leu Arg Arg Phe Met Arg Ile Gln Pro Arg Ala
1               5                   10                  15
Pro Met Thr Ala Ala Gly Gly Ala Ala Phe Phe Asn Ala Ser Cys Pro
            20                  25                  30
Ser Ser Pro Arg Thr Val Ala His Leu Glu Val Ser Gly Glu Gln Ala
        35                  40                  45
Ser Pro Lys Val Glu Leu Ser Glu Lys Ser Cys Ile Pro Cys Asn Ser
    50                  55                  60
Met Asp Leu His Ala Met Ser Glu Asp Ser Ala Lys Lys Ser Leu Glu
```

```
65                  70                  75                  80
Gln Val Thr Gly Trp Glu Leu Lys Asn Glu Gly Asp Ile Leu Lys Leu
                85                  90                  95
His Arg Ala Trp Lys Val Lys Asn Phe Val Lys Gly Leu Glu Phe Phe
            100                 105                 110
Gln Leu Val Ala Ala Val Ala Glu Glu Glu Gly His His Pro Asp Leu
        115                 120                 125
His Leu Val Ser Trp Asn Asn Val Lys Ile Asp Val Trp Thr His Ser
    130                 135                 140
Val Arg Gly Leu Thr Asp Asn Asp Phe Ile Leu Ala Ala Lys Ile Asn
145                 150                 155                 160
Glu Leu Lys Leu Glu Gly Leu Leu Ser Lys Lys Lys Ala Thr Thr His
                165                 170                 175
Glu
```

<210> 376
<211> 902
<212> DNA
<213> Medicago truncatula

<220>
<221> misc_feature
<222> (902)..(902)
<223> n is a, c, g, or t

<400> 376

```
cggccgggga catccatttc cattgaactt cacaatccac acaacccaat aaacaaacac     60
caagcaaaca aagaggaaga agtatacact atccagtagt attagtgtgt ataataaatc    120
aatggctaca accaagcttg tattattatc cttccctgca gtgcatgtaa actcaaccac    180
taccaaaatc aacccttcat tcaatttcca acatcaccgt acaagtactc caaatgggtt    240
ctctccactt cggtccttag ccaacgattt cttgggggat tttggagcaa gagacccttt    300
cccagctgag ttggaaagtc aatttggaga gaaagttatt ggtagttata acactgaaca    360
caaaatcctt atcccaaata tttcagctct ttctctttct caacaggagt gtacccccat    420
ttcaccttta cagaaccccc tttctcaaga tgatgctaac cagcttataa gaaaggtttt    480
gggttggaga cttgtgaatg atgaaggaat acttaaactt cgatgcttgt ggaaattgaa    540
agattttaaa tgcggagttg aactcattaa cagaatctct acagttgtag agactgcagg    600
acatttcccc aatatccaca tagaacaacc aaatctagtc agagcagaac tatggacaac    660
atccattggt ggattaagta tgaatgattt cattgtagct gcaaagatag atgcaataaa    720
gacatcagat cttatcccaa ggaaaagagc ttgggcttaa tgctccatga gctccaatcc    780
ataaagtatt ttgttatgaa tttttgttca acaaagagtt accactaatt tgtgttattt    840
gatgaaatgt attattcttt gttctcattt taagtgttct tgcttctacc tgaagtctgt    900
tn                                                                   902
```

<210> 377
<211> 212
<212> PRT
<213> Medicago truncatula

<400> 377

```
Met Ala Thr Thr Lys Leu Val Leu Leu Ser Phe Pro Ala Val His Val
1               5                   10                  15
Asn Ser Thr Thr Thr Lys Ile Asn Pro Ser Phe Asn Phe Gln His His
            20                  25                  30
Arg Thr Ser Thr Pro Asn Gly Phe Ser Pro Leu Arg Ser Leu Ala Asn
        35                  40                  45
Asp Phe Leu Gly Asp Phe Gly Ala Arg Asp Pro Phe Pro Ala Glu Leu
    50                  55                  60
Glu Ser Gln Phe Gly Glu Lys Val Ile Gly Ser Tyr Asn Thr Glu His
```

```
65                  70                  75                  80
Lys Ile Leu Ile Pro Asn Ile Ser Ala Leu Ser Leu Ser Gln Gln Glu
                85                  90                  95
Cys Thr Pro Ile Ser Pro Leu Gln Asn Pro Leu Ser Gln Asp Asp Ala
            100                 105                 110
Asn Gln Leu Ile Arg Lys Val Leu Gly Trp Arg Leu Val Asn Asp Glu
        115                 120                 125
Gly Ile Leu Lys Leu Arg Cys Leu Trp Lys Leu Lys Asp Phe Lys Cys
    130                 135                 140
Gly Val Glu Leu Ile Asn Arg Ile Ser Thr Val Val Glu Thr Ala Gly
145                 150                 155                 160
His Phe Pro Asn Ile His Ile Glu Gln Pro Asn Leu Val Arg Ala Glu
                165                 170                 175
Leu Trp Thr Thr Ser Ile Gly Gly Leu Ser Met Asn Asp Phe Ile Val
            180                 185                 190
Ala Ala Lys Ile Asp Ala Ile Lys Thr Ser Asp Leu Ile Pro Arg Lys
        195                 200                 205
Arg Ala Trp Ala
    210
```

<210>  378
<211>  2745
<212>  DNA
<213>  Oryza sativa

<400>  378

```
aaatgtaact agtctcttct taccggaatc cccacacctt ctcgccacgg tccgacgcgc      60
aagcgcggcg gcgctcgctc tcgcggttct cggtgacggc ggcctcgtcc cactcccgcc     120
cggggatgaa cggcaccccg ttgcgttgcc atctctagtc gcgcatgacc acccgcgccg     180
gcgcacctct cgccgtcttc ctcgtctcgt ccctcaagtc ggccgccgca cgcctcagcc     240
acggcgagca gctccacgcg ctcgctgcca agtcgggcct gctcacctcc aatcttttcg     300
tccgcaactc cctcctcgcc ttctactccc gcgtcgcccc cagcctcgcc taccacctgt     360
tcgatgaaat acccccgctc ctccgcgacg ccaccgccca caacattctc ctctccgcgc     420
tcgcccgcgc gggccgcctc gaacgcgcgc agtgcctgct cgcggagatg cctcagaggg     480
acgcggtctc gttcaccact gtcatttccg ccctctcgcg ctccggtcat cctgagcgcg     540
ccctggccgt cttccgcgat atgctcaccg aggccgtgca gcccaacgag gttacacttg     600
cggaagtgct cacggcgatg gcctgtgacc acggggcacc agcaccagtt ggtgctgccc     660
atggcgttgc cgtgcggcgt gggcttgatg gatttgtcat cgtggccacc aacctggtcc     720
atgcatatgg agcagtgtca caggttcctt ctgcccgttc catattcgag ctaatgccag     780
atagaaacac tgtcacttgg aatacaatgc ttaattgcta tgtcaaggca ggaatgatta     840
acatggctgc tgaggtgttc ggtgtgatcc cagagaggga tgaggtctct tggttgacaa     900
tgatagatgg atatatgtgt gcggatttcc tactgcaggc tctcaggaca tatgttgcga     960
tggtgggtac ggtgggcata agggccaatg aggtgatact tgttggtttg gtcaaggcat    1020
gttctcgaca ttccgctgtc tcagaagggc aacaactcca cacagtcatt ctaaagaatg    1080
gttttgacgc tcatgcattt gtacaggcta ccctgatcca ttattacggg tcttgtgatt    1140
ttattgacca tgcccagatg caattcaaat tgtcagacaa gtcacacgta gcttcctgga    1200
atgcccttat ggctagccta ctaagaagga atctagtgca tgaagcaagg cagctgttcg    1260
atgacatgcc tgagagggac accatttctt ggagtacatt gatatctggg tatgtccaga    1320
gtgggaattc aaacatggct ttgcagatat tctgttcgat gctagatgct ggtgttgaac    1380
ctaatgagat cactttagca agtgctctgt ctgcagtagc caattctggc accttggggc    1440
aagcaagatg gatccatgac tacataatca gcagatcaat ccagcttact gataaactga    1500
gcgctggact gattaacgtg tatgcaaagt gtggtagcat tgctgaagca gttcagctat    1560
ttaaccatgt taaggataag tcaatttcag tgtctccttg gaattctatc atctgtaatt    1620
tagctatcca tggctatgtg aacatgtcgc tggaattgtt ttcacagttg caaagcacca    1680
cccatattaa acccaactca atcacatacc ttggtgtgtt aaatgcatgt tgtcatgctg    1740
ggatggtagc tgaggggaag cgccaatttg agtccatgag gcaacaatat ggaatccaac    1800
cagaaattaa gcactatggt tgcatggtcg atcttctttg tcgagctggg tatttggaag    1860
aagcagaact gcttattaag acgatgccaa tgaaagctga tgtggtagcc tggggttgta    1920
tccttgcagc tgcaaggact caaggaaatg tagccttagg agaaaaggct gcagaagaat    1980
```

```
tgtcaaagct tgacccaagc catggagcat ccaaaatagc cttgtcaaac ctctatgctg   2040
atgctggtca ttggagcaat gtgtcagtgg tgaggaagga actccagaat gagaatttgg   2100
aaagacttac aggaagcagt ggaattctac aattataggt tttagaaata tagctccctc   2160
aaggaacaat aattcttata gactttgaca cagtagggca tatttcggtc ttcagcttca   2220
catatgaaca ttaggttaca agaaactcaa ttgtcagcaa ataaaatatc caaactattg   2280
tcaacaagac agagatgata ttttcaatta ttatggaatt ttttgcaatt tgaaatcttg   2340
gccggttatt atcagagaat atatgggcaa gtgcttctcc tgaatgtgat gcatgcatgt   2400
atggtttaat tccagtttta ctttgcctcc tggctcctgg cgtagcagca gtacattttc   2460
tatggaagat aaagcttgag aacaaaatta atatgctgtg atgctggatg gaagatcaca   2520
tgatcaagtt ggcagcatag aggtgacgta cagaggcaag gaagtcggag gcgctgtgca   2580
tatgcaacgt tttcatttct atgttaattt tcattgtttt ggttaatacc atgtgatctc   2640
taatctaatt agtgtgggac caagccaaag accaggcacc ctatatatgg gtctaggccg   2700
attggaaaca gacaatacac aatcaatcga tacaaaattc ctgtc                   2745

<210>  379
<211>  657
<212>  PRT
<213>  Oryza sativa

<400>  379
Met Thr Thr Arg Ala Gly Ala Pro Leu Ala Val Phe Leu Val Ser Ser
1               5                   10                  15
Leu Lys Ser Ala Ala Ala Arg Leu Ser His Gly Glu Gln Leu His Ala
            20                  25                  30
Leu Ala Ala Lys Ser Gly Leu Leu Thr Ser Asn Leu Phe Val Arg Asn
        35                  40                  45
Ser Leu Leu Ala Phe Tyr Ser Arg Val Ala Pro Ser Leu Ala Tyr His
    50                  55                  60
Leu Phe Asp Glu Ile Pro Pro Leu Leu Arg Asp Ala Thr Ala His Asn
65                  70                  75                  80
Ile Leu Leu Ser Ala Leu Ala Arg Ala Gly Arg Leu Glu Arg Ala Gln
                85                  90                  95
Cys Leu Leu Ala Glu Met Pro Gln Arg Asp Ala Val Ser Phe Thr Thr
            100                 105                 110
Val Ile Ser Ala Leu Ser Arg Ser Gly His Pro Glu Arg Ala Leu Ala
        115                 120                 125
Val Phe Arg Asp Met Leu Thr Glu Ala Val Gln Pro Asn Glu Val Thr
    130                 135                 140
Leu Ala Glu Val Leu Thr Ala Met Ala Cys Asp His Gly Ala Pro Ala
145                 150                 155                 160
Pro Val Gly Ala Ala His Gly Val Ala Val Arg Arg Gly Leu Asp Gly
                165                 170                 175
Phe Val Ile Val Ala Thr Asn Leu Val His Ala Tyr Gly Ala Val Ser
            180                 185                 190
Gln Val Pro Ser Ala Arg Ser Ile Phe Glu Leu Met Pro Asp Arg Asn
        195                 200                 205
Thr Val Thr Trp Asn Thr Met Leu Asn Cys Tyr Val Lys Ala Gly Met
    210                 215                 220
Ile Asn Met Ala Ala Glu Val Phe Gly Val Ile Pro Glu Arg Asp Glu
225                 230                 235                 240
Val Ser Trp Leu Thr Met Ile Asp Gly Tyr Met Cys Ala Asp Phe Leu
                245                 250                 255
Leu Gln Ala Leu Arg Thr Tyr Val Ala Met Val Gly Thr Val Gly Ile
            260                 265                 270
Arg Ala Asn Glu Val Ile Leu Val Gly Leu Val Lys Ala Cys Ser Arg
        275                 280                 285
His Ser Ala Val Ser Glu Gly Gln Gln Leu His Thr Val Ile Leu Lys
    290                 295                 300
Asn Gly Phe Asp Ala His Ala Phe Val Gln Ala Thr Leu Ile His Tyr
```

```
305                 310                 315                 320
Tyr Gly Ser Cys Asp Phe Ile Asp His Ala Gln Met Gln Phe Lys Leu
                325                 330                 335
Ser Asp Lys Ser His Val Ala Ser Trp Asn Ala Leu Met Ala Ser Leu
            340                 345                 350
Leu Arg Arg Asn Leu Val His Glu Ala Arg Gln Leu Phe Asp Asp Met
        355                 360                 365
Pro Glu Arg Asp Thr Ile Ser Trp Ser Thr Leu Ile Ser Gly Tyr Val
    370                 375                 380
Gln Ser Gly Asn Ser Asn Met Ala Leu Gln Ile Phe Cys Ser Met Leu
385                 390                 395                 400
Asp Ala Gly Val Glu Pro Asn Glu Ile Thr Leu Ala Ser Ala Leu Ser
                405                 410                 415
Ala Val Ala Asn Ser Gly Thr Leu Gly Gln Ala Arg Trp Ile His Asp
            420                 425                 430
Tyr Ile Ile Ser Arg Ser Ile Gln Leu Thr Asp Lys Leu Ser Ala Gly
        435                 440                 445
Leu Ile Asn Val Tyr Ala Lys Cys Gly Ser Ile Ala Glu Ala Val Gln
    450                 455                 460
Leu Phe Asn His Val Lys Asp Lys Ser Ile Ser Val Ser Pro Trp Asn
465                 470                 475                 480
Ser Ile Ile Cys Asn Leu Ala Ile His Gly Tyr Val Asn Met Ser Leu
                485                 490                 495
Glu Leu Phe Ser Gln Leu Gln Ser Thr Thr His Ile Lys Pro Asn Ser
            500                 505                 510
Ile Thr Tyr Leu Gly Val Leu Asn Ala Cys Cys His Ala Gly Met Val
        515                 520                 525
Ala Glu Gly Lys Arg Gln Phe Glu Ser Met Arg Gln Gln Tyr Gly Ile
    530                 535                 540
Gln Pro Glu Ile Lys His Tyr Gly Cys Met Val Asp Leu Leu Cys Arg
545                 550                 555                 560
Ala Gly Tyr Leu Glu Glu Ala Glu Leu Leu Ile Lys Thr Met Pro Met
                565                 570                 575
Lys Ala Asp Val Val Ala Trp Gly Cys Ile Leu Ala Ala Ala Arg Thr
            580                 585                 590
Gln Gly Asn Val Ala Leu Gly Glu Lys Ala Ala Glu Glu Leu Ser Lys
        595                 600                 605
Leu Asp Pro Ser His Gly Ala Ser Lys Ile Ala Leu Ser Asn Leu Tyr
    610                 615                 620
Ala Asp Ala Gly His Trp Ser Asn Val Ser Val Val Arg Lys Glu Leu
625                 630                 635                 640
Gln Asn Glu Asn Leu Glu Arg Leu Thr Gly Ser Ser Gly Ile Leu Gln
                645                 650                 655
Leu
```

<210> 380
<211> 956
<212> DNA
<213> Oryza sativa

<400> 380

```
acgccaggca aacccacacg ccgccttccc gccgcctccg ctcttcttcc cccaaccgcg      60
tcgcaagccc acggcgagcg ccgccgccgt cccaccgtcg ccgatgaccc gcggcgtcgc     120
tatggcgcac gcgaggctcc tcctggcgcg ctactacgcg atggcggccc ccagttggcc     180
taccgtatcc aagaaccttc cgctgctggg ccatggacgt tctcatcatc cgatgtatgc     240
aagtcaagac gagattaaaa tgtcaagtag aagatggtgc catggttctc ctgacaatca     300
agagctggca aagaagattt gtgttccatg caattctaag gatatacatg ccatgccaga     360
agattctgct aagaagatgc tagaacaggt gggtggttgg gaattagcaa ccgaaggtga     420
```

```
cattctgaaa ttgcacagag catggaaggt gaagaatttt gtaaaagggc ttgaattcct    480
tcagcttgtt gctgctgttg ctgaggaaga aggccaccac cctgatcttc atcttgtcgg    540
ttggaacaat gtgaaaattg atgtttggac tcactctgtc agaggcttaa ctgacaatga    600
tttcatcctt gctgcgaaga tcaataacct caacttagaa ggcctcttaa gcaagaaagc    660
tacagtccaa aagtagttcg cgcagatgaa gcgattgatt tctgacgaga tttatgtact    720
tgcttcggtt agaatgcgtg agttgaggta tctttaactt gataagcttt tcttgaattt    780
gtaccgggag tttctctgtt ctttctaaat tttaccaatg ctgttggacc gggtctactt    840
ctgttacaga atgtcgagaa ccaccataat ctagatttga tcaaggagaa cctgtttctc    900
ttacatgtgg gccaagagat gtagggtcca cgaacatttt agagtccacc acacct        956
```

```
<210>  381
<211>  190
<212>  PRT
<213>  Oryza sativa

<400>  381
Met Thr Arg Gly Val Ala Met Ala His Ala Arg Leu Leu Leu Ala Arg
1               5                   10                  15
Tyr Tyr Ala Met Ala Ala Pro Ser Trp Pro Thr Val Ser Lys Asn Leu
            20                  25                  30
Pro Leu Leu Gly His Gly Arg Ser His His Pro Met Tyr Ala Ser Gln
        35                  40                  45
Asp Glu Ile Lys Met Ser Ser Arg Arg Trp Cys His Gly Ser Pro Asp
    50                  55                  60
Asn Gln Glu Leu Ala Lys Lys Ile Cys Val Pro Cys Asn Ser Lys Asp
65                  70                  75                  80
Ile His Ala Met Pro Glu Asp Ser Ala Lys Lys Met Leu Glu Gln Val
                85                  90                  95
Gly Gly Trp Glu Leu Ala Thr Glu Gly Asp Ile Leu Lys Leu His Arg
            100                 105                 110
Ala Trp Lys Val Lys Asn Phe Val Lys Gly Leu Glu Phe Leu Gln Leu
        115                 120                 125
Val Ala Ala Val Ala Glu Glu Glu Gly His His Pro Asp Leu His Leu
    130                 135                 140
Val Gly Trp Asn Asn Val Lys Ile Asp Val Trp Thr His Ser Val Arg
145                 150                 155                 160
Gly Leu Thr Asp Asn Asp Phe Ile Leu Ala Ala Lys Ile Asn Asn Leu
                165                 170                 175
Asn Leu Glu Gly Leu Leu Ser Lys Lys Ala Thr Val Gln Lys
            180                 185                 190
```

```
<210>  382
<211>  2009
<212>  DNA
<213>  Populus trichocarpa

<220>
<221>  misc_feature
<222>  (610)..(610)
<223>  n is a, c, g, or t

<400>  382
atggcaaata agctcaaagt agacgagctt cgagccgaac tcgctaaacg tggactcgat     60
accaccggaa ccaagccttc cctggtgcgg agactggaat cggcactcga gcaagaaaac    120
aagcagtcag aagtggactc ggtcgatggt ggaggtagta gtaacaacaa aagagaaagg    180
gagtctgaag atggaggagg agattctaat aacgggacag agaaaatcaa ggctattgaa    240
aagtttcggg acatgaatgt taagcaatta cgcgaacagg ccagtctcct tggagtttcc    300
actgccggaa ccaagagaca attaattgac aggctctgca acgctgaacc agacaatcac    360
cattcacttc aaccaaaaga agaagagcag gaacagggtg aaaaggaaaa ggataaaatt    420
```

```
gtaactgcta cgaaaaaggg cgcggcggtg ctagatcagt ttctttctga tcagataaag    480
tcagagtatc atgttttgca aataggtgat gatgtctatg atgccatgct gaatcagaca    540
aatgttgggg acaacaataa caagttctat gtgattcaac ttcttggcaa gctttcactt    600
ccgcccatgn gatatatggt ctataacaga tggggtaggg ttggggtgaa tggtcaagtt    660
aagctatttg gtccctacac ttcacgagat cctgctgttt ctgagtttga acggaagttc    720
tacgccaaga caaagaacga ttggtccaag cgaaaggagt tcatatgtta cccaaagtgc    780
tatgctttgt tggaaatgga ctacagtgag caggaaaagg agccagttgt taaagaaaag    840
cctgactctg ctatagcaat tcaacctcgg gagacacaac ttgagtcacg tattgccaaa    900
tttatctctc ttatatgtaa tgttagtatg atgaagcagc aaatgatgga aataggatac    960
aatgcaaata aattgccact tggtaaatta agcaagtcaa ctatattaaa gggttatgat   1020
gttttgaaga ggatttctga tgtaattgga acagcagata tgttaaaact tgaacaacta   1080
agcggagaat tttacactgt cattcctcat gattttggat ttaaaaaaat gcgagagttt   1140
gttattgata ctcctcagaa gttaaaatgc aagctggaaa tggttgaagc cctaggcgag   1200
attcaagttg caacaaagtt gttggaggat gaacctggaa tgcaggaaga tcccctgtat   1260
tctcattaca accggctacg ttgtgaattg actccagttg aagttgattc tgaggacttt   1320
gcaatgattg ccatgtattt gcagaatacg catgcaaaaa cacattcaca gtatactgtt   1380
gatattgctc aaatattcaa ggtctcaaga gaggatgaaa atgaaagctt cagaaagttc   1440
tctaacacaa aaaatagaat gcttttatgg catggctcgc gcctcacaaa ctggactggc   1500
attctatccc aaggcttgcg tattgctccc cctgaagcac cagtaactgg ttacatgttt   1560
ggaaagggag tttactttgc tgacatgttc tccaaaagtg caaattattg ctatgcaaat   1620
catgctgctc cagctggtgt gctgcttctg tgtgaggttg ctcttggaga catggctgaa   1680
ctattatatg caaactatga tgctgataag ttgccatcgg ggaagctaag cacgaaagga   1740
gttggtagaa ctgctccaga tctttcagat gccagggcac tggaggatgg tgttgttgtt   1800
ccccttggga agcccaaaga gcagcgaggc tcaaagggtg ctttgctgta caatgagtac   1860
atagtctaca acgtgggtca gattaggatg cgatatgtag ttcaagtgaa ttttaattac   1920
aagcattaat agcgtcagtt tgatcttgtg tttttgttct cagatttgtt cggctatgta   1980
tcatccttta caggtcttct agttgcaaa                                     2009
```

<210> 383
<211> 642
<212> PRT
<213> Populus trichocarpa

<220>
<221> misc_feature
<222> (204)..(204)
<223> Xaa can be any naturally occurring amino acid

<400> 383

```
Met Ala Asn Lys Leu Lys Val Asp Glu Leu Arg Ala Glu Leu Ala Lys
1               5                   10                  15
Arg Gly Leu Asp Thr Thr Gly Thr Lys Pro Ser Leu Val Arg Arg Leu
            20                  25                  30
Glu Ser Ala Leu Glu Gln Glu Asn Lys Gln Ser Glu Val Asp Ser Val
        35                  40                  45
Asp Gly Gly Gly Ser Ser Asn Asn Lys Arg Glu Arg Glu Ser Glu Asp
    50                  55                  60
Gly Gly Gly Asp Ser Asn Asn Gly Thr Glu Lys Ile Lys Ala Ile Glu
65                  70                  75                  80
Lys Phe Arg Asp Met Asn Val Lys Gln Leu Arg Glu Gln Ala Ser Leu
                85                  90                  95
Leu Gly Val Ser Thr Ala Gly Thr Lys Arg Gln Leu Ile Asp Arg Leu
            100                 105                 110
Cys Asn Ala Glu Pro Asp Asn His His Ser Leu Gln Pro Lys Glu Glu
        115                 120                 125
Glu Gln Glu Gln Gly Glu Lys Glu Lys Asp Lys Ile Val Thr Ala Thr
    130                 135                 140
Lys Lys Gly Ala Ala Val Leu Asp Gln Phe Leu Ser Asp Gln Ile Lys
145                 150                 155                 160
```

```
Ser Glu Tyr His Val Leu Gln Ile Gly Asp Asp Val Tyr Asp Ala Met
                165                 170                 175
Leu Asn Gln Thr Asn Val Gly Asp Asn Asn Asn Lys Phe Tyr Val Ile
            180                 185                 190
Gln Leu Leu Gly Lys Leu Ser Leu Pro Pro Met Xaa Tyr Met Val Tyr
        195                 200                 205
Asn Arg Trp Gly Arg Val Gly Val Asn Gly Gln Val Lys Leu Phe Gly
    210                 215                 220
Pro Tyr Thr Ser Arg Asp Pro Ala Val Ser Glu Phe Glu Arg Lys Phe
225                 230                 235                 240
Tyr Ala Lys Thr Lys Asn Asp Trp Ser Lys Arg Lys Glu Phe Ile Cys
                245                 250                 255
Tyr Pro Lys Cys Tyr Ala Leu Leu Glu Met Asp Tyr Ser Glu Gln Glu
            260                 265                 270
Lys Glu Pro Val Val Lys Glu Lys Pro Asp Ser Ala Ile Ala Ile Gln
        275                 280                 285
Pro Arg Glu Thr Gln Leu Glu Ser Arg Ile Ala Lys Phe Ile Ser Leu
    290                 295                 300
Ile Cys Asn Val Ser Met Met Lys Gln Gln Met Met Glu Ile Gly Tyr
305                 310                 315                 320
Asn Ala Asn Lys Leu Pro Leu Gly Lys Leu Ser Lys Ser Thr Ile Leu
                325                 330                 335
Lys Gly Tyr Asp Val Leu Lys Arg Ile Ser Asp Val Ile Gly Thr Ala
            340                 345                 350
Asp Met Leu Lys Leu Glu Gln Leu Ser Gly Glu Phe Tyr Thr Val Ile
        355                 360                 365
Pro His Asp Phe Gly Phe Lys Lys Met Arg Glu Phe Val Ile Asp Thr
    370                 375                 380
Pro Gln Lys Leu Lys Cys Lys Leu Glu Met Val Glu Ala Leu Gly Glu
385                 390                 395                 400
Ile Gln Val Ala Thr Lys Leu Leu Glu Asp Glu Pro Gly Met Gln Glu
                405                 410                 415
Asp Pro Leu Tyr Ser His Tyr Asn Arg Leu Arg Cys Glu Leu Thr Pro
            420                 425                 430
Val Glu Val Asp Ser Glu Asp Phe Ala Met Ile Ala Met Tyr Leu Gln
        435                 440                 445
Asn Thr His Ala Lys Thr His Ser Gln Tyr Thr Val Asp Ile Ala Gln
    450                 455                 460
Ile Phe Lys Val Ser Arg Glu Asp Glu Asn Glu Ser Phe Arg Lys Phe
465                 470                 475                 480
Ser Asn Thr Lys Asn Arg Met Leu Leu Trp His Gly Ser Arg Leu Thr
                485                 490                 495
Asn Trp Thr Gly Ile Leu Ser Gln Gly Leu Arg Ile Ala Pro Pro Glu
            500                 505                 510
Ala Pro Val Thr Gly Tyr Met Phe Gly Lys Gly Val Tyr Phe Ala Asp
        515                 520                 525
Met Phe Ser Lys Ser Ala Asn Tyr Cys Tyr Ala Asn His Ala Ala Pro
    530                 535                 540
Ala Gly Val Leu Leu Leu Cys Glu Val Ala Leu Gly Asp Met Ala Glu
545                 550                 555                 560
Leu Leu Tyr Ala Asn Tyr Asp Ala Asp Lys Leu Pro Ser Gly Lys Leu
                565                 570                 575
Ser Thr Lys Gly Val Gly Arg Thr Ala Pro Asp Leu Ser Asp Ala Arg
            580                 585                 590
Ala Leu Glu Asp Gly Val Val Val Pro Leu Gly Lys Pro Lys Glu Gln
        595                 600                 605
Arg Gly Ser Lys Gly Ala Leu Leu Tyr Asn Glu Tyr Ile Val Tyr Asn
    610                 615                 620
Val Gly Gln Ile Arg Met Arg Tyr Val Val Gln Val Asn Phe Asn Tyr
```

```
625                 630                 635                 640
Lys His


<210>  384
<211>  755
<212>  DNA
<213>  Populus trichocarpa

<400>  384
cgttatcacc accagtcaca gtaaaccacc atgggtacca ccgccacaac caccactcgc     60
ttcccattct ccctcccacc ccctaaatcc tatcctccac acaaccacca ccactccaaa    120
gtctccatct tgactcccac tataaccccc actttgagac tccaagctat gggagctggc    180
gacaagttag gtgaatttgg tgctagagac ccttttcctg ctgaaataga aagtgggttt    240
gctgagaaag tgttaggaaa tggtaatact gaacataaga ttctcattcc tactgtttct    300
gctctttctc tttctcagca agagtgtact cctatatctc ctttgcaaga tcccatgtct    360
aaagatgatg ctcaaaaact gttaaagaag gttctaggct ggagactcct ggatgaagaa    420
ggtggactga aactgcaatg cttgtggaag ttgagagatt ttaaatgtgg ggttgagctt    480
gtcaatagaa tttacaaagc tacagaatca tgtggccatt tcccaaatgt gcacttggaa    540
cagcccaatc aagttagagc tgaactatgg actgcatccc ttggtggctt gagtttgaat    600
gatttcattg tcgcggccaa gattgatgag attaagacat ctgaccttgt ccctaaaaaa    660
agagtctggg catagttgga tttttcttgt aagctttcct gaaggaacct gagagatcgg    720
tctcttaata agttggtgtt tcagaatcca acaac                               755

<210>  385
<211>  214
<212>  PRT
<213>  Populus trichocarpa

<400>  385
Met Gly Thr Thr Ala Thr Thr Thr Thr Arg Phe Pro Phe Ser Leu Pro
1               5                   10                  15
Pro Pro Lys Ser Tyr Pro Pro His Asn His His His Ser Lys Val Ser
            20                  25                  30
Ile Leu Thr Pro Thr Ile Thr Pro Thr Leu Arg Leu Gln Ala Met Gly
        35                  40                  45
Ala Gly Asp Lys Leu Gly Glu Phe Gly Ala Arg Asp Pro Phe Pro Ala
    50                  55                  60
Glu Ile Glu Ser Gly Phe Ala Glu Lys Val Leu Gly Asn Gly Asn Thr
65                  70                  75                  80
Glu His Lys Ile Leu Ile Pro Thr Val Ser Ala Leu Ser Leu Ser Gln
                85                  90                  95
Gln Glu Cys Thr Pro Ile Ser Pro Leu Gln Asp Pro Met Ser Lys Asp
            100                 105                 110
Asp Ala Gln Lys Leu Leu Lys Lys Val Leu Gly Trp Arg Leu Leu Asp
        115                 120                 125
Glu Glu Gly Gly Leu Lys Leu Gln Cys Leu Trp Lys Leu Arg Asp Phe
    130                 135                 140
Lys Cys Gly Val Glu Leu Val Asn Arg Ile Tyr Lys Ala Thr Glu Ser
145                 150                 155                 160
Cys Gly His Phe Pro Asn Val His Leu Glu Gln Pro Asn Gln Val Arg
                165                 170                 175
Ala Glu Leu Trp Thr Ala Ser Leu Gly Gly Leu Ser Leu Asn Asp Phe
            180                 185                 190
Ile Val Ala Ala Lys Ile Asp Glu Ile Lys Thr Ser Asp Leu Val Pro
        195                 200                 205
Lys Lys Arg Val Trp Ala
    210
```

<210> 386
<211> 524
<212> DNA
<213> Populus trichocarpa

<400> 386
ctctgtctga tatcctatct ggaggtcatc atggaagttt tgcttcctat gatagatttg 60
gctgcaaaga agtgtgtgcc atgcaactcg aaggatctac aagccatgac tgaagaaagt 120
gcaaatgacc ttctatcgaa ggttgctggg tggaatttgg tgaatgaaaa tggtacacta 180
aagctgaatc gttcatggaa agtaaagagt ttcactaaag ggctggaact attcaagctt 240
gttgggaatg ttgctgaagc agaagtaaca gtatatgcaa atgcaggtca ccatccggat 300
cttcatctgg ttggatggaa caatataaca atcgagatat ggactcatgc agtgggtgga 360
ctaacggaaa acgacttcat acttgctgct aagataaatg ggcttaacct gcaccaccta 420
ctgaggaaga aggctgctgc ttgacttgta aatacaatta gacttcagtt tttggttaat 480
agtcttatcc aaaggtatga ggaaagactt tttttttct ctcc 524

<210> 387
<211> 137
<212> PRT
<213> Populus trichocarpa

<400> 387
Met Glu Val Leu Leu Pro Met Ile Asp Leu Ala Ala Lys Lys Cys Val
1 5 10 15
Pro Cys Asn Ser Lys Asp Leu Gln Ala Met Thr Glu Glu Ser Ala Asn
20 25 30
Asp Leu Leu Ser Lys Val Ala Gly Trp Asn Leu Val Asn Glu Asn Gly
35 40 45
Thr Leu Lys Leu Asn Arg Ser Trp Lys Val Lys Ser Phe Thr Lys Gly
50 55 60
Leu Glu Leu Phe Lys Leu Val Gly Asn Val Ala Glu Ala Glu Val Thr
65 70 75 80
Val Tyr Ala Asn Ala Gly His His Pro Asp Leu His Leu Val Gly Trp
85 90 95
Asn Asn Ile Thr Ile Glu Ile Trp Thr His Ala Val Gly Gly Leu Thr
100 105 110
Glu Asn Asp Phe Ile Leu Ala Ala Lys Ile Asn Gly Leu Asn Leu His
115 120 125
His Leu Leu Arg Lys Lys Ala Ala Ala
130 135

<210> 388
<211> 483
<212> DNA
<213> Solanum lycopersicum

<400> 388
attcgaacgc ttcaagctca tatcgtttac atcactctcc caaaccttgt acagaaacaa 60
tggaagctca aatacacatg tagctggaac tggggtaatg tgcagtaatc ttgcagtgtc 120
agcaaaaact aacactcatt acgggattaa gactttgtct tcagcagcag ttttgtcaac 180
taagaagtgt gtaccatgca acacaaagga tttgaggcct atgactgaag aagctgccta 240
tcagttgatg ccacaggtgt cggggtggga tttggtgaat gatggtggca cactgaagct 300
gcataagtcc tggaaggtca agactttcac caaaggattg gagttctttc aagaggtggc 360
gagtgttgca gaagctgaag gtcatcatcc agatcttcat cttgttgggt ggaataatgt 420
gaaaattgat atatggactc attctgtggg tggactcaca gaaaatgatt tcatactagc 480
tgc 483

<210> 389
<211> 129

<212> PRT
<213> Solanum lycopersicum

<400> 389
```
Met Cys Ser Asn Leu Ala Val Ser Ala Lys Thr Asn Thr His Tyr Gly
1               5                   10                  15
Ile Lys Thr Leu Ser Ser Ala Ala Val Leu Ser Thr Lys Lys Cys Val
            20                  25                  30
Pro Cys Asn Thr Lys Asp Leu Arg Pro Met Thr Glu Glu Ala Ala Tyr
        35                  40                  45
Gln Leu Met Pro Gln Val Ser Gly Trp Asp Leu Val Asn Asp Gly Gly
    50                  55                  60
Thr Leu Lys Leu His Lys Ser Trp Lys Val Lys Thr Phe Thr Lys Gly
65                  70                  75                  80
Leu Glu Phe Phe Gln Glu Val Ala Ser Val Ala Glu Ala Glu Gly His
                85                  90                  95
His Pro Asp Leu His Leu Val Gly Trp Asn Asn Val Lys Ile Asp Ile
            100                 105                 110
Trp Thr His Ser Val Gly Gly Leu Thr Glu Asn Asp Phe Ile Leu Ala
        115                 120                 125
Ala
```

<210> 390
<211> 761
<212> DNA
<213> Solanum lycopersicum

<220>
<221> misc_feature
<222> (17)..(17)
<223> n is a, c, g, or t

<220>
<221> misc_feature
<222> (299)..(299)
<223> n is a, c, g, or t

<220>
<221> misc_feature
<222> (302)..(302)
<223> n is a, c, g, or t

<220>
<221> misc_feature
<222> (313)..(313)
<223> n is a, c, g, or t

<220>
<221> misc_feature
<222> (440)..(440)
<223> n is a, c, g, or t

<220>
<221> misc_feature
<222> (600)..(600)
<223> n is a, c, g, or t

<220>

<221> misc_feature
<222> (671)..(671)
<223> n is a, c, g, or t

<220>
<221> misc_feature
<222> (673)..(673)
<223> n is a, c, g, or t

<220>
<221> misc_feature
<222> (680)..(680)
<223> n is a, c, g, or t

<220>
<221> misc_feature
<222> (708)..(708)
<223> n is a, c, g, or t

<220>
<221> misc_feature
<222> (714)..(716)
<223> n is a, c, g, or t

<220>
<221> misc_feature
<222> (725)..(726)
<223> n is a, c, g, or t

<220>
<221> misc_feature
<222> (738)..(738)
<223> n is a, c, g, or t

<220>
<221> misc_feature
<222> (750)..(752)
<223> n is a, c, g, or t

<220>
<221> misc_feature
<222> (760)..(760)
<223> n is a, c, g, or t

<400> 390
agccttttct ttttccnaga agaggagctg gtcttttttt ttaagaaggt gattggaaga 60
gttatcaaac aggtggcagg aatcatcgat gattcgaacg cttcaagctc gtctggcaaa 120
tctcttggac actttttttt cctcctctcc ggtgttgtac ttatgtattg acattctttt 180
caatttcttc agatatcgtt tacatcactc tcccaaacct tgtacagaaa caatggaagt 240
tttgtcaact aaaaagtgtg taccatgcaa cacaaaggat ttgaggccta tgactgaana 300
anctgcctat canttgatgc cacaggtgtc ggggtgggat ttggtgaatg atggtggcac 360
actgaagctg cataagtcct ggaaggtcaa gactttcacc aaaggattgg agttctttca 420
agaggtggcg agtgttgcan aagctgaagg tcatcatcca gatcttcatc ttgttgggtg 480
gaataatgtg aaaattgata tatggactca ttctgtgggt ggactcacag aaaatgattt 540
catactagct gccaaagtta atgggcttga tgtgcaggac cttctcagca agaaacttan 600
caaaccagca caaagcagtg gttaaccttc caagtttaga gttgtattat gatgttagtt 660
tcttatatac nanatatcan gggcatttga gtactgttgt tatttaanaa atgnnnctag 720
aggannctgt cttttcangg ggcattttgn nnactatgan a 761

<210> 391
<211> 178
<212> PRT
<213> Solanum lycopersicum

<220>
<221> misc_feature
<222> (71)..(72)
<223> Xaa can be any naturally occurring amino acid

<220>
<221> misc_feature
<222> (75)..(75)
<223> Xaa can be any naturally occurring amino acid

<220>
<221> misc_feature
<222> (118)..(118)
<223> Xaa can be any naturally occurring amino acid

<220>
<221> misc_feature
<222> (171)..(171)
<223> Xaa can be any naturally occurring amino acid

<400> 391
```
Met Ile Arg Thr Leu Gln Ala Arg Leu Ala Asn Leu Leu Asp Thr Phe
1               5                   10                  15
Phe Ser Ser Ser Pro Val Leu Tyr Leu Cys Ile Asp Ile Leu Phe Asn
            20                  25                  30
Phe Phe Arg Tyr Arg Leu His His Ser Pro Lys Pro Cys Thr Glu Thr
        35                  40                  45
Met Glu Val Leu Ser Thr Lys Lys Cys Val Pro Cys Asn Thr Lys Asp
    50                  55                  60
Leu Arg Pro Met Thr Glu Xaa Xaa Ala Tyr Xaa Leu Met Pro Gln Val
65                  70                  75                  80
Ser Gly Trp Asp Leu Val Asn Asp Gly Gly Thr Leu Lys Leu His Lys
                85                  90                  95
Ser Trp Lys Val Lys Thr Phe Thr Lys Gly Leu Glu Phe Phe Gln Glu
            100                 105                 110
Val Ala Ser Val Ala Xaa Ala Glu Gly His His Pro Asp Leu His Leu
        115                 120                 125
Val Gly Trp Asn Asn Val Lys Ile Asp Ile Trp Thr His Ser Val Gly
    130                 135                 140
Gly Leu Thr Glu Asn Asp Phe Ile Leu Ala Ala Lys Val Asn Gly Leu
145                 150                 155                 160
Asp Val Gln Asp Leu Leu Ser Lys Lys Leu Xaa Lys Pro Ala Gln Ser
                165                 170                 175
Ser Gly
```

<210> 392
<211> 986
<212> DNA
<213> Solanum lycopersicum

<400> 392
```
ttctagataa aatcaccaca catatcctct ctaataagag cagagcagag cagagcagtg      60
aactcctctt ctctttctca gatggctgct gcaactcatc tttctttttc tccaccaatt     120
```

```
tcagtttctt ctctcccttt acgcaaacct aactttgtta ctcccaatct ccagaccagt    180
caatctcgaa tttgcacaag aatacgttgc aatactaact tattaggtga ttttggggcc    240
agagacccat ttccagctga agtggagagt aaatttggtg aaaaggtttt gggtaatcct    300
gacacggagc acaaaattct catcccagct gcctctgctt tgtcccttgc taatcaggaa    360
tgcactgata tctcacccaa tcagactccc ttgtccgaat ttgaagctaa acagctttta    420
ttcaaggttg ttggctggag aatagcaaat gaagatgggg tgcttaaact acaatgcaca    480
tggaaattaa aagactttga ttgtggggtt gaactaatca atagaatagg caaggtggtg    540
gaaggcacag ggcacctccc cactcttcat caattacaac agtctaatca agttcatgct    600
gagctgtgga ctccttcgat tggaggtttg agcatgaatg atttcatcat agctgctaaa    660
atagatcaag taaagacatc ggacctcgta ccaagaaaaa gagtctgggc atgatcagat    720
aatagcatta cgaatcacac caaatacagt ttccagaatt gcagtcgctt atcgtcagtg    780
ataaaaacct tgtctgataa gcaatggcaa gggaattttt actgcatgat tctgtattag    840
ttttatgatt gattctaaac aaagccacct ttattatcaa atcctctgaa ctatgggatt    900
caaatcccaa ggtataacaa tcctcatatc atgtatttaa tttttgttta ttattttaat    960
gtaaaaggta attatttttt ctaaaa                                         986
```

<210>  393
<211>  210
<212>  PRT
<213>  Solanum lycopersicum

<400>  393

```
Met Ala Ala Ala Thr His Leu Ser Phe Ser Pro Pro Ile Ser Val Ser
1               5                   10                  15
Ser Leu Pro Leu Arg Lys Pro Asn Phe Val Thr Pro Asn Leu Gln Thr
            20                  25                  30
Ser Gln Ser Arg Ile Cys Thr Arg Ile Arg Cys Asn Thr Asn Leu Leu
        35                  40                  45
Gly Asp Phe Gly Ala Arg Asp Pro Phe Pro Ala Glu Val Glu Ser Lys
    50                  55                  60
Phe Gly Glu Lys Val Leu Gly Asn Pro Asp Thr Glu His Lys Ile Leu
65                  70                  75                  80
Ile Pro Ala Ala Ser Ala Leu Ser Leu Ala Asn Gln Glu Cys Thr Asp
                85                  90                  95
Ile Ser Pro Asn Gln Thr Pro Leu Ser Glu Phe Glu Ala Lys Gln Leu
            100                 105                 110
Leu Phe Lys Val Val Gly Trp Arg Ile Ala Asn Glu Asp Gly Val Leu
        115                 120                 125
Lys Leu Gln Cys Thr Trp Lys Leu Lys Asp Phe Asp Cys Gly Val Glu
    130                 135                 140
Leu Ile Asn Arg Ile Gly Lys Val Val Glu Gly Thr Gly His Leu Pro
145                 150                 155                 160
Thr Leu His Gln Leu Gln Gln Ser Asn Gln Val His Ala Glu Leu Trp
                165                 170                 175
Thr Pro Ser Ile Gly Gly Leu Ser Met Asn Asp Phe Ile Ile Ala Ala
            180                 185                 190
Lys Ile Asp Gln Val Lys Thr Ser Asp Leu Val Pro Arg Lys Arg Val
        195                 200                 205
Trp Ala
    210
```

<210>  394
<211>  668
<212>  DNA
<213>  Saccharum officinarum

<220>
<221>  misc_feature
<222>  (425)..(425)

<223> n is a, c, g, or t

<220>
<221> misc_feature
<222> (520)..(520)
<223> n is a, c, g, or t

<400> 394
caaagcggac tccgttttcc ctctccggat ccggctccgg cgaagcagcg ggagccaccg 60
atgctccggg ccgcaacggt gcacgtgcgt ctcctcctat cgcgctccta cgtatggcag 120
gcgaaggtag cttgtcgctg gcctctcgtc cgcaggaagc cttcgcatct tggatctgta 180
cgcccttttct atcagatgga cactagaggg caagacgaaa ataaaatttt gactgcaaga 240
gggtgccata gctcccctga gagtcaagaa ttagcaatga aaagctgtgt tccatgcaac 300
tctaaggatt tacatcccat gtcagaagat tctgctaaaa agttgcttga acaggtgaat 360
ggttgggaac tgatcactga aggtggtatt ctgaaattac atagagcatg gaaggtgaag 420
aactntgtta aaggacttga gttctttcaa cttgttgctg ctatcgctga ggaacaaggt 480
caccatccag atcttcatct tgttggttgg aataacgtgn aaattgatgt ttggactcat 540
tctgtcagaa ggtttaacaa gtaatgaatt tcatcccttg ctggcgaaag aatccaaatg 600
ggaaatttct tctttttttt tttacaaaca aacaactttt tttttttttt ttttttttta 660
aaaaaaaa 668

<210> 395
<211> 167
<212> PRT
<213> Saccharum officinarum

<220>
<221> misc_feature
<222> (122)..(122)
<223> Xaa can be any naturally occurring amino acid

<220>
<221> misc_feature
<222> (154)..(154)
<223> Xaa can be any naturally occurring amino acid

<400> 395
Met Leu Arg Ala Ala Thr Val His Val Arg Leu Leu Leu Ser Arg Ser
1 5 10 15
Tyr Val Trp Gln Ala Lys Val Ala Cys Arg Trp Pro Leu Val Arg Arg
20 25 30
Lys Pro Ser His Leu Gly Ser Val Arg Pro Phe Tyr Gln Met Asp Thr
35 40 45
Arg Gly Gln Asp Glu Asn Lys Ile Leu Thr Ala Arg Gly Cys His Ser
50 55 60
Ser Pro Glu Ser Gln Glu Leu Ala Met Lys Ser Cys Val Pro Cys Asn
65 70 75 80
Ser Lys Asp Leu His Pro Met Ser Glu Asp Ser Ala Lys Lys Leu Leu
85 90 95
Glu Gln Val Asn Gly Trp Glu Leu Ile Thr Glu Gly Gly Ile Leu Lys
100 105 110
Leu His Arg Ala Trp Lys Val Lys Asn Xaa Val Lys Gly Leu Glu Phe
115 120 125
Phe Gln Leu Val Ala Ala Ile Ala Glu Glu Gln Gly His His Pro Asp
130 135 140
Leu His Leu Val Gly Trp Asn Asn Val Xaa Ile Asp Val Trp Thr His
145 150 155 160
Ser Val Arg Arg Phe Asn Lys
165

```
<210>  396
<211>  652
<212>  DNA
<213>  Saccharum officinarum

<400>  396
ccgcaggaag ccttcgcatc ttggatctgt acgccctttc tatcagatgg acactagagg     60
gcaagacgaa aataaaattt tgactgcaag agggtgccat agctcccctg agagtcaaga    120
attagcaatg aaaagctgtg ttccatgcaa ctctaaggat ttacatccca tgtcagaaga    180
ttctgctaaa aagttgcttg tacaggtgaa tggttgggaa ctgatcactg aaggtggtat    240
tctgaaatta catagagcat ggaaggtgaa gaactttgtt aaaggactcg agttctttca    300
acttgttgct gctatcgctg aagaacaagg tcaccatcca gatcttcatc ttgttggttg    360
gaataacgtg aaaattgatg tttggactca ttctgtcaga agtttaacaa gtaatgattt    420
catccttgct gcgaagatca atgatctcac tttagaaggc attataagga agaaagctac    480
atagctaatg tctcagaagt aactcagatc tgtcgaccca agcagtcctt gtttatgtac    540
tacactcaaa atatgtaact cgtatttgct tgggaaaatg tatacagcgg tggagaggtc    600
aaattaaaag gcaccttaac ggaatatgag tttaacttgg tgaaaataga cc            652

<210>  397
<211>  145
<212>  PRT
<213>  Saccharum officinarum

<400>  397
Met Asp Thr Arg Gly Gln Asp Glu Asn Lys Ile Leu Thr Ala Arg Gly
1               5                   10                  15
Cys His Ser Ser Pro Glu Ser Gln Glu Leu Ala Met Lys Ser Cys Val
            20                  25                  30
Pro Cys Asn Ser Lys Asp Leu His Pro Met Ser Glu Asp Ser Ala Lys
        35                  40                  45
Lys Leu Leu Val Gln Val Asn Gly Trp Glu Leu Ile Thr Glu Gly Gly
    50                  55                  60
Ile Leu Lys Leu His Arg Ala Trp Lys Val Lys Asn Phe Val Lys Gly
65                  70                  75                  80
Leu Glu Phe Phe Gln Leu Val Ala Ala Ile Ala Glu Glu Gln Gly His
                85                  90                  95
His Pro Asp Leu His Leu Val Gly Trp Asn Asn Val Lys Ile Asp Val
            100                 105                 110
Trp Thr His Ser Val Arg Ser Leu Thr Ser Asn Asp Phe Ile Leu Ala
        115                 120                 125
Ala Lys Ile Asn Asp Leu Thr Leu Glu Gly Ile Ile Arg Lys Lys Ala
    130                 135                 140
Thr
145

<210>  398
<211>  676
<212>  DNA
<213>  Saccharum officinarum

<220>
<221>  misc_feature
<222>  (79)..(79)
<223>  n is a, c, g, or t

<220>
<221>  misc_feature
<222>  (105)..(105)
```

<223>  n is a, c, g, or t

<400>  398
tgtacgccct ttctataaga tgcacactag agggcaagac gaaaataaaa ttttgactgc    60
aagagggtgc catagctcnc ctgagagtca agaattagca atganaagct gtgttccatg   120
caactctaag gatttacatc ccatgtcaga agattctgct aaaaagttgc ttgtacaggt   180
gaatggttgg gaactgatca ctgaaggtgt aattctgaaa ttacatagag catggaaggt   240
gaagaacttt gttaaaggac tcgagttctt tcaacttgtt gctgctatcg ctgaggaaca   300
aggtcaccat ccagatcttc atcttgttgg ttggaataac gtgaaaattg atgtttggac   360
tcattctgtc agaggtttaa caagtaatga tttcatcctt gctgcgaaga tcaatgatct   420
cactttagaa ggcattataa ggaagaaagc tacatagcta atgtctcaga agtaactcag   480
atctgtcgac ccaagcagtc ctgttttatg tactccactc aaaatatgta actcgtattt   540
gcttgggaaa atgtatacag cgttggagaa gtcaaattta gaggtcacct caccggagta   600
tgagttttaa cttgctgaaa aatagtactt aaatagctca ttgtactttg ctaattagta   660
atttccttga gatgga                                                   676

<210>  399
<211>  145
<212>  PRT
<213>  Saccharum officinarum

<220>
<221>  misc_feature
<222>  (29)..(29)
<223>  Xaa can be any naturally occurring amino acid

<400>  399
Met His Thr Arg Gly Gln Asp Glu Asn Lys Ile Leu Thr Ala Arg Gly
1               5                   10                  15
Cys His Ser Ser Pro Glu Ser Gln Glu Leu Ala Met Xaa Ser Cys Val
            20                  25                  30
Pro Cys Asn Ser Lys Asp Leu His Pro Met Ser Glu Asp Ser Ala Lys
        35                  40                  45
Lys Leu Leu Val Gln Val Asn Gly Trp Glu Leu Ile Thr Glu Gly Val
    50                  55                  60
Ile Leu Lys Leu His Arg Ala Trp Lys Val Lys Asn Phe Val Lys Gly
65                  70                  75                  80
Leu Glu Phe Phe Gln Leu Val Ala Ala Ile Ala Glu Glu Gln Gly His
                85                  90                  95
His Pro Asp Leu His Leu Val Gly Trp Asn Asn Val Lys Ile Asp Val
            100                 105                 110
Trp Thr His Ser Val Arg Gly Leu Thr Ser Asn Asp Phe Ile Leu Ala
        115                 120                 125
Ala Lys Ile Asn Asp Leu Thr Leu Glu Gly Ile Ile Arg Lys Lys Ala
    130                 135                 140
Thr
145

<210>  400
<211>  613
<212>  DNA
<213>  Triticum aestivum

<220>
<221>  misc_feature
<222>  (2)..(4)
<223>  n is a, c, g, or t

<220>

```
<221>  misc_feature
<222>  (484)..(485)
<223>  n is a, c, g, or t

<220>
<221>  misc_feature
<222>  (583)..(590)
<223>  n is a, c, g, or t

<400>  400
cnnncgtccg gcccaccacc gccatgcgga acctcctctt cggcaccacc accagcacca     60
ccagcggtca ccggagcact cgcaaggtgg tggcgatggc ggacatcctg ggcgacttcg    120
gcgcgcggga cccgttcccg gaggagatcg cgagcaactt cggggagaag acgctgggca    180
acgtggacac gctgcaccgc atcctcatcc ccacgctctc cgtgctctcc ctctcccgcg    240
tcccgctcga cgccgacccg gccccgctct ccgaggagga cgcccgcaag ctgctcttca    300
aggtggtcgg ctggcggctc ctcttcccca gcaagggcga ctccgacgtg ctcaagctcg    360
agtgcgtctg gaaagtccgc gaccaggcct gcggcgacga gctcgtcgcc aggatcaaca    420
aggcgctcga cggcgccggc cacgcccccg ccgcgctccg gttcgaggcg cccaaccaag    480
tcanngccca gctctacacg ccgtccgtaa gcgggctgag cgccaacgac ttcatcatcg    540
cggcgaggat cgaccagatc aagaccaaag atctcctccc aannnnnnnn gtctgggcat    600
gctaatggcc gca                                                       613

<210>  401
<211>  193
<212>  PRT
<213>  Triticum aestivum

<220>
<221>  misc_feature
<222>  (154)..(154)
<223>  Xaa can be any naturally occurring amino acid

<220>
<221>  misc_feature
<222>  (187)..(189)
<223>  Xaa can be any naturally occurring amino acid

<400>  401
Met Arg Asn Leu Leu Phe Gly Thr Thr Thr Ser Thr Thr Ser Gly His
1               5                   10                  15
Arg Ser Thr Arg Lys Val Val Ala Met Ala Asp Ile Leu Gly Asp Phe
            20                  25                  30
Gly Ala Arg Asp Pro Phe Pro Glu Glu Ile Ala Ser Asn Phe Gly Glu
        35                  40                  45
Lys Thr Leu Gly Asn Val Asp Thr Leu His Arg Ile Leu Ile Pro Thr
    50                  55                  60
Leu Ser Val Leu Ser Leu Ser Arg Val Pro Leu Asp Ala Asp Pro Ala
65                  70                  75                  80
Pro Leu Ser Glu Glu Asp Ala Arg Lys Leu Leu Phe Lys Val Val Gly
                85                  90                  95
Trp Arg Leu Leu Phe Pro Ser Lys Gly Asp Ser Asp Val Leu Lys Leu
            100                 105                 110
Glu Cys Val Trp Lys Val Arg Asp Gln Ala Cys Gly Asp Glu Leu Val
        115                 120                 125
Ala Arg Ile Asn Lys Ala Leu Asp Gly Ala Gly His Ala Pro Ala Ala
    130                 135                 140
Leu Arg Phe Glu Ala Pro Asn Gln Val Xaa Ala Gln Leu Tyr Thr Pro
145                 150                 155                 160
Ser Val Ser Gly Leu Ser Ala Asn Asp Phe Ile Ile Ala Ala Arg Ile
```

```
                165                 170                 175
Asp Gln Ile Lys Thr Lys Asp Leu Leu Pro Xaa Xaa Xaa Val Trp Ala
            180                 185                 190
Cys
```

<210> 402
<211> 572
<212> DNA
<213> Triticum aestivum

<220>
<221> misc_feature
<222> (376)..(376)
<223> n is a, c, g, or t

<220>
<221> misc_feature
<222> (483)..(483)
<223> n is a, c, g, or t

<220>
<221> misc_feature
<222> (517)..(517)
<223> n is a, c, g, or t

<220>
<221> misc_feature
<222> (566)..(566)
<223> n is a, c, g, or t

<400> 402

```
cattgcttcc cccagccgcg catcatccat ccatccatcc gttcctctct ccttccttcc     60
tgtccactac accatggcgc tcatgctcag cccggccgcg accttcctcc ccgtcgccgg    120
caagcccgcc gccgccatgc ggaacctcct cttcggcacc agcagcaagg gaagaagcgt    180
tcgcaaggtg gtggcgatgg cggacatcct gggcgacttc ggggcgcggg acccgttccc    240
ggaggagatc gcgagcaact tcggggagaa gacgctgggc aacgtggaca cgctgcaccg    300
catcctcatt cccacgctct ccgtgctctc gctctcccgc gtgccgctcg acgccgaccc    360
cgcgccgctc tccgangagg acgcccgcaa gctgctcttc aaggtggtcg gatggcggct    420
cctcttcccc aacaagggcg actccgaacg tgctcaagct cgagtgcgtc tggaaagtcc    480
gcnacaaggc tgcggcgaac aactcatcgc caggatnaac aagacctcga cggcgccggc    540
atgccccgcc gcgctcaatt cgaggngccc aa                                  572
```

<210> 403
<211> 167
<212> PRT
<213> Triticum aestivum

<220>
<221> misc_feature
<222> (101)..(101)
<223> Xaa can be any naturally occurring amino acid

<220>
<221> misc_feature
<222> (137)..(137)
<223> Xaa can be any naturally occurring amino acid

<220>

<221> misc_feature
<222> (148)..(148)
<223> Xaa can be any naturally occurring amino acid

<220>
<221> misc_feature
<222> (165)..(165)
<223> Xaa can be any naturally occurring amino acid

<220>
<221> misc_feature
<222> (167)..(167)
<223> Xaa can be any naturally occurring amino acid

<400> 403
```
Met Ala Leu Met Leu Ser Pro Ala Ala Thr Phe Leu Pro Val Ala Gly
1               5                   10                  15
Lys Pro Ala Ala Ala Met Arg Asn Leu Leu Phe Gly Thr Ser Ser Lys
            20                  25                  30
Gly Arg Ser Val Arg Lys Val Val Ala Met Ala Asp Ile Leu Gly Asp
        35                  40                  45
Phe Gly Ala Arg Asp Pro Phe Pro Glu Glu Ile Ala Ser Asn Phe Gly
    50                  55                  60
Glu Lys Thr Leu Gly Asn Val Asp Thr Leu His Arg Ile Leu Ile Pro
65                  70                  75                  80
Thr Leu Ser Val Leu Ser Leu Ser Arg Val Pro Leu Asp Ala Asp Pro
                85                  90                  95
Ala Pro Leu Ser Xaa Glu Asp Ala Arg Lys Leu Leu Phe Lys Val Val
            100                 105                 110
Gly Trp Arg Leu Leu Phe Pro Asn Lys Gly Asp Ser Glu Arg Ala Gln
        115                 120                 125
Ala Arg Val Arg Leu Glu Ser Pro Xaa Gln Gly Cys Gly Glu Gln Leu
    130                 135                 140
Ile Ala Arg Xaa Asn Lys Thr Ser Thr Ala Pro Ala Cys Pro Ala Ala
145                 150                 155                 160
Leu Asn Ser Arg Xaa Pro Xaa
                165
```

<210> 404
<211> 573
<212> DNA
<213> Triticum aestivum

<220>
<221> misc_feature
<222> (392)..(392)
<223> n is a, c, g, or t

<220>
<221> misc_feature
<222> (452)..(452)
<223> n is a, c, g, or t

<220>
<221> misc_feature
<222> (462)..(462)
<223> n is a, c, g, or t

<220>

<221> misc_feature
<222> (465)..(465)
<223> n is a, c, g, or t

<220>
<221> misc_feature
<222> (489)..(489)
<223> n is a, c, g, or t

<220>
<221> misc_feature
<222> (497)..(497)
<223> n is a, c, g, or t

<220>
<221> misc_feature
<222> (517)..(517)
<223> n is a, c, g, or t

<220>
<221> misc_feature
<222> (524)..(524)
<223> n is a, c, g, or t

<220>
<221> misc_feature
<222> (547)..(547)
<223> n is a, c, g, or t

<220>
<221> misc_feature
<222> (557)..(557)
<223> n is a, c, g, or t

<400> 404
atccatccat ccgttccact gcaccatggc gctcatgctc agcccggccg cgaccttcct 60
ccccgtcgtc gccggcaagc ccaccaccgc catgcggaac ctcctcttcg gcaccaccac 120
cagcaccacc agcggtcacc ggagcactcg caaggtggtg gcgatggcgg acatcctggg 180
cgacttccgg cgcgcgggac ccgttcccgg aggagattcg ccgagcaact ttcggggaga 240
aagacgctgg gcaacgttgg acacgctgca ccgcatcctc atccccacgt ctccgtgtct 300
ccctctcccg cgtcccgctc cgacgccgac cggcccgctc tccgaggagg acgcccgcag 360
ctgctcttca aggtggtccg ttggggtcct cnttcccaag caagggcact ccgactgtca 420
actccatgcg tctggaagtc cgcacaaagc tnccgggaac anttntcccc aagatttaac 480
aagggcccna cgggccngca agccccgccg gctccgnttt aggngccaac aattaaggcc 540
aatctanagc cgtcgtnagc ggctgacgca aca 573

<210> 405
<211> 183
<212> PRT
<213> Triticum aestivum

<220>
<221> misc_feature
<222> (123)..(123)
<223> Xaa can be any naturally occurring amino acid

<220>
<221> misc_feature
<222> (143)..(143)

<223>  Xaa can be any naturally occurring amino acid

<220>
<221>  misc_feature
<222>  (146)..(147)
<223>  Xaa can be any naturally occurring amino acid

<220>
<221>  misc_feature
<222>  (155)..(155)
<223>  Xaa can be any naturally occurring amino acid

<220>
<221>  misc_feature
<222>  (158)..(158)
<223>  Xaa can be any naturally occurring amino acid

<220>
<221>  misc_feature
<222>  (167)..(167)
<223>  Xaa can be any naturally occurring amino acid

<220>
<221>  misc_feature
<222>  (174)..(174)
<223>  Xaa can be any naturally occurring amino acid

<220>
<221>  misc_feature
<222>  (178)..(178)
<223>  Xaa can be any naturally occurring amino acid

<220>
<221>  misc_feature
<222>  (183)..(183)
<223>  Xaa can be any naturally occurring amino acid

<400>  405

```
Met Ala Leu Met Leu Ser Pro Ala Ala Thr Phe Leu Pro Val Val Ala
1               5                   10                  15
Gly Lys Pro Thr Thr Ala Met Arg Asn Leu Leu Phe Gly Thr Thr Thr
            20                  25                  30
Ser Thr Thr Ser Gly His Arg Ser Thr Arg Lys Val Val Ala Met Ala
        35                  40                  45
Asp Ile Leu Gly Asp Phe Arg Arg Ala Gly Pro Val Pro Gly Gly Asp
    50                  55                  60
Ser Pro Ser Asn Phe Arg Gly Glu Arg Arg Trp Ala Thr Leu Asp Thr
65                  70                  75                  80
Leu His Arg Ile Leu Ile Pro Thr Ser Pro Cys Leu Pro Leu Pro Arg
                85                  90                  95
Pro Ala Pro Thr Pro Thr Gly Pro Leu Ser Glu Glu Asp Ala Arg Ser
            100                 105                 110
Cys Ser Ser Arg Trp Ser Val Gly Val Leu Xaa Pro Lys Gln Gly His
        115                 120                 125
Ser Asp Cys Gln Leu His Ala Ser Gly Ser Pro His Lys Ala Xaa Gly
    130                 135                 140
Asn Xaa Xaa Pro Gln Asp Leu Thr Arg Ala Xaa Arg Ala Xaa Lys Pro
145                 150                 155                 160
Arg Arg Leu Arg Phe Arg Xaa Gln Gln Leu Arg Pro Ile Xaa Ser Arg
```

```
                165                 170                 175
Arg Xaa Arg Leu Thr Gln Xaa
            180

<210>  406
<211>  597
<212>  DNA
<213>  Triticum aestivum

<220>
<221>  misc_feature
<222>  (342)..(342)
<223>  n is a, c, g, or t

<220>
<221>  misc_feature
<222>  (361)..(361)
<223>  n is a, c, g, or t

<220>
<221>  misc_feature
<222>  (389)..(389)
<223>  n is a, c, g, or t

<220>
<221>  misc_feature
<222>  (426)..(426)
<223>  n is a, c, g, or t

<220>
<221>  misc_feature
<222>  (431)..(431)
<223>  n is a, c, g, or t

<220>
<221>  misc_feature
<222>  (457)..(457)
<223>  n is a, c, g, or t

<220>
<221>  misc_feature
<222>  (464)..(464)
<223>  n is a, c, g, or t

<220>
<221>  misc_feature
<222>  (469)..(469)
<223>  n is a, c, g, or t

<220>
<221>  misc_feature
<222>  (521)..(521)
<223>  n is a, c, g, or t

<220>
<221>  misc_feature
<222>  (523)..(523)
<223>  n is a, c, g, or t
```

```
<220>
<221>  misc_feature
<222>  (539)..(539)
<223>  n is a, c, g, or t

<220>
<221>  misc_feature
<222>  (552)..(552)
<223>  n is a, c, g, or t

<220>
<221>  misc_feature
<222>  (559)..(559)
<223>  n is a, c, g, or t

<220>
<221>  misc_feature
<222>  (576)..(576)
<223>  n is a, c, g, or t

<220>
<221>  misc_feature
<222>  (581)..(581)
<223>  n is a, c, g, or t

<220>
<221>  misc_feature
<222>  (583)..(583)
<223>  n is a, c, g, or t

<220>
<221>  misc_feature
<222>  (594)..(594)
<223>  n is a, c, g, or t

<400>  406
ccatggcgct catgctcagc ccggccgcga ccttcctccc cgtcgccacc ggcaagccca     60
ccgccaccgc cggcgccatg cgaagcctcc tcttcaccac caccagcggc accgccagcg    120
gccaccggag cagcaggaag gtggtggcga tggcggacat cctgggggac ttcggcgcgc    180
gggacccgtt cccggaggag atcgcgagca acttcgggga gaagacgctg ggcaacgtgg    240
acacgctgca ccgcatcctc atccccacgc tctccgtgct ctccctctcc cgcgtcccgc    300
tcgaagccca cccggcgccg ctctccgagg aggaccccgc angctgctct tcaagtcgtc    360
ngatggcgcc tcctcttccc gggaaaggng atccgaactg ctcaaactcc aatgcgtctg    420
gaaagnccgc nacaaggcct gcgggaacaa gtaatcncaa gatnggaang ggctcaacgg    480
gccggcaagc cccgccgggc tctcttcaac cgccaacaat nangggaaag ttgaaaccnc    540
cgtagtggct angcaagant tatatccggg aggttnacca ntnagcaaag tccncca       597

<210>  407
<211>  180
<212>  PRT
<213>  Triticum aestivum

<220>
<221>  misc_feature
<222>  (114)..(114)
<223>  Xaa can be any naturally occurring amino acid

<220>
<221>  misc_feature
```

```
<222>  (120)..(120)
<223>  Xaa can be any naturally occurring amino acid

<220>
<221>  misc_feature
<222>  (142)..(142)
<223>  Xaa can be any naturally occurring amino acid

<220>
<221>  misc_feature
<222>  (152)..(152)
<223>  Xaa can be any naturally occurring amino acid

<220>
<221>  misc_feature
<222>  (154)..(154)
<223>  Xaa can be any naturally occurring amino acid

<220>
<221>  misc_feature
<222>  (156)..(156)
<223>  Xaa can be any naturally occurring amino acid

<220>
<221>  misc_feature
<222>  (173)..(174)
<223>  Xaa can be any naturally occurring amino acid

<400>  407
Met Ala Leu Met Leu Ser Pro Ala Ala Thr Phe Leu Pro Val Ala Thr
1               5                   10                  15
Gly Lys Pro Thr Ala Thr Ala Gly Ala Met Arg Ser Leu Leu Phe Thr
            20                  25                  30
Thr Thr Ser Gly Thr Ala Ser Gly His Arg Ser Ser Arg Lys Val Val
        35                  40                  45
Ala Met Ala Asp Ile Leu Gly Asp Phe Gly Ala Arg Asp Pro Phe Pro
    50                  55                  60
Glu Glu Ile Ala Ser Asn Phe Gly Glu Lys Thr Leu Gly Asn Val Asp
65                  70                  75                  80
Thr Leu His Arg Ile Leu Ile Pro Thr Leu Ser Val Leu Ser Leu Ser
                85                  90                  95
Arg Val Pro Leu Glu Ala His Pro Ala Pro Leu Ser Glu Glu Asp Pro
            100                 105                 110
Ala Xaa Cys Ser Ser Ser Arg Xaa Met Ala Pro Pro Leu Pro Gly Lys
        115                 120                 125
Gly Asp Pro Asn Cys Ser Asn Ser Asn Ala Ser Gly Lys Xaa Ala Thr
    130                 135                 140
Arg Pro Ala Gly Thr Ser Asn Xaa Lys Xaa Gly Xaa Gly Ser Thr Gly
145                 150                 155                 160
Arg Gln Ala Pro Pro Gly Ser Leu Gln Pro Pro Thr Xaa Xaa Gly Lys
                165                 170                 175
Leu Lys Pro Pro
            180

<210>  408
<211>  493
<212>  DNA
<213>  Triticum aestivum
```

<220>
<221> misc_feature
<222> (345)..(345)
<223> n is a, c, g, or t

<220>
<221> misc_feature
<222> (354)..(354)
<223> n is a, c, g, or t

<220>
<221> misc_feature
<222> (356)..(356)
<223> n is a, c, g, or t

<220>
<221> misc_feature
<222> (398)..(398)
<223> n is a, c, g, or t

<220>
<221> misc_feature
<222> (459)..(459)
<223> n is a, c, g, or t

<220>
<221> misc_feature
<222> (465)..(465)
<223> n is a, c, g, or t

<220>
<221> misc_feature
<222> (478)..(478)
<223> n is a, c, g, or t

<220>
<221> misc_feature
<222> (489)..(489)
<223> n is a, c, g, or t

<400> 408
acctcctccg tccgtccatt gcctccccca gccgcgcatc atccatccat ccgtcccagt 60
gcgccatggc gctcatgctc agcccggccg cgaccttcct ccccgtcgcc accggcaagc 120
ccaccgccac cgccggcgcc atgcgaagcc tcctcttcac caccaccagc ggcaccgcca 180
gcggccaccg gagcagcagg aaggtggtgg cgatggcgga catcctgggg gacttcgggc 240
gcgcgggacc cgttccccgg agggagatcg cgaagcaact tcgggggaga aagacgctgg 300
gcaacgtggg acacggctgc aacgcaatcc tcaatcccca aggcntctcc gtgntncccc 360
tctcccggcg tccccggctc cgaaagccca acccgggngc cggcttcttc cgaaggaggg 420
aacgccccgg caagggctgc tccttcaaag ggtcggtcng ggatnggggg cctccctntt 480
ccccggggna ggg 493

<210> 409
<211> 143
<212> PRT
<213> Triticum aestivum

<220>
<221> misc_feature
<222> (94)..(94)

<223> Xaa can be any naturally occurring amino acid

<220>
<221> misc_feature
<222> (97)..(97)
<223> Xaa can be any naturally occurring amino acid

<220>
<221> misc_feature
<222> (132)..(132)
<223> Xaa can be any naturally occurring amino acid

<220>
<221> misc_feature
<222> (134)..(134)
<223> Xaa can be any naturally occurring amino acid

<220>
<221> misc_feature
<222> (138)..(138)
<223> Xaa can be any naturally occurring amino acid

<220>
<221> misc_feature
<222> (142)..(142)
<223> Xaa can be any naturally occurring amino acid

<400> 409
```
Met Ala Leu Met Leu Ser Pro Ala Ala Thr Phe Leu Pro Val Ala Thr
1               5                   10                  15
Gly Lys Pro Thr Ala Thr Ala Gly Ala Met Arg Ser Leu Leu Phe Thr
            20                  25                  30
Thr Thr Ser Gly Thr Ala Ser Gly His Arg Ser Ser Arg Lys Val Val
        35                  40                  45
Ala Met Ala Asp Ile Leu Gly Asp Phe Gly Arg Ala Gly Pro Val Pro
    50                  55                  60
Arg Arg Glu Ile Ala Lys Gln Leu Arg Gly Arg Lys Thr Leu Gly Asn
65                  70                  75                  80
Val Gly His Gly Cys Asn Ala Ile Leu Asn Pro Gln Gly Xaa Ser Val
                85                  90                  95
Xaa Pro Leu Ser Arg Arg Pro Arg Leu Arg Lys Pro Asn Pro Gly Ala
            100                 105                 110
Gly Phe Phe Arg Arg Arg Glu Arg Pro Gly Lys Gly Cys Ser Phe Lys
        115                 120                 125
Gly Ser Val Xaa Asp Xaa Gly Pro Pro Xaa Ser Pro Gly Xaa Gly
    130                 135                 140
```

<210> 410
<211> 622
<212> DNA
<213> Triticum aestivum

<220>
<221> misc_feature
<222> (502)..(505)
<223> n is a, c, g, or t

<220>
<221> misc_feature

<222> (581)..(583)
<223> n is a, c, g, or t

<400> 410
ccgcggtcgc agccgcagcc gcagccgact ggacgccgca agccgcgcgc agcgccggtg 60
aagagccgga cctgtcacct cctccgctcc cgtcagttcc ggccacttca ctgcgagccc 120
cactgaccct caaaggcctc aacatggcag gtctgctgct gcgtagattc atcgggatcc 180
agccccgggc gccgatgacc gccgccggtg gagccgcgct cttctacgcc tcgtgtccct 240
cgtcgccccg caccgtcgcg cacctggagg tgaggggga acaggccgct gctgaagctg 300
aattatcaaa gaagagttgt atcccatgca attcaaagga tctgcacgcc atgtcagaag 360
attctgctaa aaagtccctt gaacaggtga ctggttggga actgaaaaat gaaggtgaca 420
ttttgaaatt acacagagca tggaaggtga agaattttgt aaaaggactt gagttctttc 480
agcttgttgc tgctgttgct gnnnnagaag gtcaccaccc agatcttcat ctcgtcagtt 540
ggaacaatgt gaaaatcgat gtctggactc attcagtcag nnntttaaca gataatgact 600
tcatccttgc tgcaaagatc aa 622

<210> 411
<211> 160
<212> PRT
<213> Triticum aestivum

<220>
<221> misc_feature
<222> (120)..(121)
<223> Xaa can be any naturally occurring amino acid

<220>
<221> misc_feature
<222> (146)..(147)
<223> Xaa can be any naturally occurring amino acid

<220>
<221> misc_feature
<222> (160)..(160)
<223> Xaa can be any naturally occurring amino acid

<400> 411
Met Ala Gly Leu Leu Leu Arg Arg Phe Ile Gly Ile Gln Pro Arg Ala
1 5 10 15
Pro Met Thr Ala Ala Gly Gly Ala Ala Leu Phe Tyr Ala Ser Cys Pro
20 25 30
Ser Ser Pro Arg Thr Val Ala His Leu Glu Val Arg Gly Glu Gln Ala
35 40 45
Ala Ala Glu Ala Glu Leu Ser Lys Lys Ser Cys Ile Pro Cys Asn Ser
50 55 60
Lys Asp Leu His Ala Met Ser Glu Asp Ser Ala Lys Lys Ser Leu Glu
65 70 75 80
Gln Val Thr Gly Trp Glu Leu Lys Asn Glu Gly Asp Ile Leu Lys Leu
85 90 95
His Arg Ala Trp Lys Val Lys Asn Phe Val Lys Gly Leu Glu Phe Phe
100 105 110
Gln Leu Val Ala Ala Val Ala Xaa Xaa Glu Gly His His Pro Asp Leu
115 120 125
His Leu Val Ser Trp Asn Asn Val Lys Ile Asp Val Trp Thr His Ser
130 135 140
Val Xaa Xaa Leu Thr Asp Asn Asp Phe Ile Leu Ala Ala Lys Ile Xaa
145 150 155 160

<210> 412

<211> 1071
<212> DNA
<213> Triticum aestivum

<220>
<221> misc_feature
<222> (7)..(7)
<223> n is a, c, g, or t

<220>
<221> misc_feature
<222> (757)..(757)
<223> n is a, c, g, or t

<400> 412
```
gctaggncga cccacgcgtc cgacaaattg tgtggtgata aaaggaagag aaggcttcaa      60
gcgacggcca gataagaagc aacctcgtcc gtccgtccat tgcctccccc agccgcgcat     120
catccatcca tccgtcccag tgcgccatgg cgctcatgct cagcccggcc gcgaccttcc     180
tccccgtcgc caccggcaag cccaccgcca ccgccggcgc catgcgaagc ctcctcttca     240
ccaccaccag cggcaccgcc agcggccacc ggagcagcag gaaggtggtg gcgatggcgg     300
acatcctggg ggacttcggc gcgcgggacc cgttcccgga ggagatcgcg agcaacttcg     360
gggagaagac gctgggcaac gtggacacgc tgcaccgcat cctcatcccc acgctctccg     420
tgctctccct ctcccgcgtc ccgctcgaag cccacccggc gccgctctcc gaggaggacg     480
cccgcaggct gctcttcaag gtcgtcggat ggcgcctcct cttccccggc aagggcgact     540
ccgacgtgct caagctcgag tgcgtctgga aggtccgcga ccaggcctgc ggcgacgagc     600
tcatcgccag gatcggcaag gcgctcgacg gcgccggcca tgcccccgcc gcgctcctct     660
tcgagccgcc caacaagtca gggcacagct ctgcacgccc tccgtaggtg ggctgagcgc     720
ccacgacttc atcatcgcgg cgaggatcga ccagatncag accaaagatc tcctccccaa     780
gaagagggtc tgggcatgct aatccatggc gtcttgtccc tatttctttg cattgcacac     840
atacacagtt ttgtttcatg ggaagtttgt agctctactg ctaatgtttg atgatgatga     900
tggcgatcgc cattttgtcc aatcaaatca aatgatttct caaaaaaaaa aaaaagggcg     960
gccgccctaa aagtatccct cgagggggccc cagctttagc gtaccccgct ttcttggtac    1020
aagggggggcc ccattggggg gcgtattttt aagcaagggc cgggccgccg t            1071
```

<210> 413
<211> 186
<212> PRT
<213> Triticum aestivum

<400> 413
```
Met Ala Leu Met Leu Ser Pro Ala Ala Thr Phe Leu Pro Val Ala Thr
1               5                   10                  15
Gly Lys Pro Thr Ala Thr Ala Gly Ala Met Arg Ser Leu Leu Phe Thr
            20                  25                  30
Thr Thr Ser Gly Thr Ala Ser Gly His Arg Ser Ser Arg Lys Val Val
        35                  40                  45
Ala Met Ala Asp Ile Leu Gly Asp Phe Gly Ala Arg Asp Pro Phe Pro
    50                  55                  60
Glu Glu Ile Ala Ser Asn Phe Gly Glu Lys Thr Leu Gly Asn Val Asp
65                  70                  75                  80
Thr Leu His Arg Ile Leu Ile Pro Thr Leu Ser Val Leu Ser Leu Ser
                85                  90                  95
Arg Val Pro Leu Glu Ala His Pro Ala Pro Leu Ser Glu Glu Asp Ala
            100                 105                 110
Arg Arg Leu Leu Phe Lys Val Val Gly Trp Arg Leu Leu Phe Pro Gly
        115                 120                 125
Lys Gly Asp Ser Asp Val Leu Lys Leu Glu Cys Val Trp Lys Val Arg
    130                 135                 140
Asp Gln Ala Cys Gly Asp Glu Leu Ile Ala Arg Ile Gly Lys Ala Leu
```

```
145                 150                 155                 160
Asp Gly Ala Gly His Ala Pro Ala Ala Leu Leu Phe Glu Pro Pro Asn
                165                 170                 175
Lys Ser Gly His Ser Ser Ala Arg Pro Pro
            180                 185

<210>  414
<211>  1007
<212>  DNA
<213>  Triticum aestivum

<400>  414
gatcgacatt gtcatcatca agggtgagca gtagagctac aaactttcca tgaaacaaaa      60
ctgtgtatgt gtgcaatgca aagaaatagg aacaagacgc catggattag catgcccaga     120
ccctcttctt tgggaggaga tctttggtct tgatctggtc gatcctcgcc gcgatgatga     180
agtcgttggc gctcagccca cctacggagg gcgtgcagag ctgtggccgg gcttggttgg     240
gcggctcgaa gaggagcgcg gcgggggcat ggccggcgcc gtcgagcgcc ttgccgatcc     300
tggcgatgag ctcgtcgccg caggcctggt cgcggacctt ccagacgcac tcgagcttga     360
gcacgtcgga gtcgcccttg ccggggaaga ggaggcgcca tccgacgacc ttgaagagca     420
gcctgcgggc gtcctcctcg gagagcggcg ccgggtgggc ttcgagcggg acgcgggaga     480
gggagagcac ggagagcgtg gggatgagga tgcggtgcag cgtgtccacg ttgcccagcg     540
tcttctcccc gaagttgctc gcgatctcct ccgggaacgg gtcccgcgcg ccgaagtccc     600
ccaggatgtc cgtcatcgcc accaccttcc tgctgctccg gtggccgctg gcggtgccgc     660
tggtggtggt gaagaggagg cttcgcatgg cgccggcggt ggcggtgggc ttgccggtgg     720
cgacggggag gaaggtcgcg gccgggctga gcatgagcgc catggcgcac tgggacggat     780
ggatggatga tgcgcggctg gggaggcaa tggacggacg gaggaggttg cttcttatct     840
ttcctactct tcagtccatg tcagtgtcct cgtgccccaa tcgcgaaacc ttgggtcgaa     900
gattttccgg ggattccgga ccggttccag ccgtgctttt ttgttcaaac ttgttctata     960
gtgttaccta aaaaggccta gggctatata ctgtttccgg ggggaaa                  1007

<210>  415
<211>  218
<212>  PRT
<213>  Triticum aestivum

<400>  415
Met Ala Leu Met Leu Ser Pro Ala Ala Thr Phe Leu Pro Val Ala Thr
1               5                   10                  15
Gly Lys Pro Thr Ala Thr Ala Gly Ala Met Arg Ser Leu Leu Phe Thr
            20                  25                  30
Thr Thr Ser Gly Thr Ala Ser Gly His Arg Ser Ser Arg Lys Val Val
        35                  40                  45
Ala Met Thr Asp Ile Leu Gly Asp Phe Gly Ala Arg Asp Pro Phe Pro
    50                  55                  60
Glu Glu Ile Ala Ser Asn Phe Gly Glu Lys Thr Leu Gly Asn Val Asp
65                  70                  75                  80
Thr Leu His Arg Ile Leu Ile Pro Thr Leu Ser Val Leu Ser Leu Ser
                85                  90                  95
Arg Val Pro Leu Glu Ala His Pro Ala Pro Leu Ser Glu Glu Asp Ala
            100                 105                 110
Arg Arg Leu Leu Phe Lys Val Val Gly Trp Arg Leu Leu Phe Pro Gly
        115                 120                 125
Lys Gly Asp Ser Asp Val Leu Lys Leu Glu Cys Val Trp Lys Val Arg
    130                 135                 140
Asp Gln Ala Cys Gly Asp Glu Leu Ile Ala Arg Ile Gly Lys Ala Leu
145                 150                 155                 160
Asp Gly Ala Gly His Ala Pro Ala Ala Leu Leu Phe Glu Pro Pro Asn
                165                 170                 175
Gln Ala Arg Pro Gln Leu Cys Thr Pro Ser Val Gly Gly Leu Ser Ala
```

```
            180                 185                 190
Asn Asp Phe Ile Ile Ala Ala Arg Ile Asp Gln Ile Lys Thr Lys Asp
        195                 200                 205
Leu Leu Pro Lys Lys Arg Val Trp Ala Cys
    210                 215

<210>  416
<211>  1069
<212>  DNA
<213>  Triticum aestivum

<400>  416
cagaaccgaa gaagttgcca aatcatttgt tttgattggc aaaatggcga tggcatcatc     60
atcatcgtca ttaaacacta ggggtagagc tacaatggtg gataggttgg cttccattcc    120
atgaaacaaa actgtgtcca ggtatgcaat gcaaagaaag aggaacaaga tggcatgggc    180
ggtcgccatt agcatgccca gaccctcttc tttgggagga gatctttggt cttgatctgg    240
tcgatcctgg cggtgatgat gaagtcgttg gcgctcagcc cagctacgga gggcgtgtag    300
agctgggccc tgacttggtt gggcgcctcg aactggagcg cggcgggggc atggccggcg    360
ccgtcgaggg tcttgttgat cctggcgatg agctcgtcgc cgcaggcctg gtcgcggacc    420
ttccagacgc actcgagctt gagcacgtcg gagtcgccct tgctggggaa gaggagccgc    480
catccgacca ccttgaagag cagcttgcgg gcatcctcct cggagagcgg cgcggggtcg    540
gcgtcgagcg gcacgcggga tagcgagagc acggagagcg tgggaatgag gatgcggtgc    600
accgtgtcca cgttgcccag cgtcttctcc ccgaagttgc tcgcgatctc ctccgggaac    660
gggtcccggg ccccgtagtc ccccaggatg tccggcatcg ccaccacctt gcgaacgctt    720
cttcccttgc tgctggtgcc gaaaaagaag ttccgcatgg gcggccgcgg gcttgccggc    780
gaccgggagg aaggtcccgg ccggcctgac cattaacgcc atggttttgt ggacaagaag    840
gaaggaaaat tgaacgaatg gattgatgat taacgcccgg ttgggggaag caaatgcccg    900
gaggacttgc ttcgtaatct tcccacccct tcggccaagg aaaggtcctc gtgctccaat    960
ctctggcacc ctgtgggtca gaaatattca cgggaaattc gggacccggg gccaacgcgg   1020
gctttgttgg accaaaaagg tgtctataac tttcacacaa aactgggcc               1069

<210>  417
<211>  207
<212>  PRT
<213>  Triticum aestivum

<400>  417
Trp Ser Gly Arg Pro Gly Pro Ser Ser Arg Ser Pro Ala Ser Pro Arg
1               5                   10                  15
Pro Pro Met Arg Asn Phe Phe Phe Gly Thr Ser Ser Lys Gly Arg Ser
            20                  25                  30
Val Arg Lys Val Val Ala Met Pro Asp Ile Leu Gly Asp Tyr Gly Ala
        35                  40                  45
Arg Asp Pro Phe Pro Glu Glu Ile Ala Ser Asn Phe Gly Glu Lys Thr
    50                  55                  60
Leu Gly Asn Val Asp Thr Val His Arg Ile Leu Ile Pro Thr Leu Ser
65                  70                  75                  80
Val Leu Ser Leu Ser Arg Val Pro Leu Asp Ala Asp Pro Ala Pro Leu
                85                  90                  95
Ser Glu Glu Asp Ala Arg Lys Leu Leu Phe Lys Val Val Gly Trp Arg
            100                 105                 110
Leu Leu Phe Pro Ser Lys Gly Asp Ser Asp Val Leu Lys Leu Glu Cys
        115                 120                 125
Val Trp Lys Val Arg Asp Gln Ala Cys Gly Asp Glu Leu Ile Ala Arg
    130                 135                 140
Ile Asn Lys Thr Leu Asp Gly Ala Gly His Ala Pro Ala Ala Leu Gln
145                 150                 155                 160
Phe Glu Ala Pro Asn Gln Val Arg Ala Gln Leu Tyr Thr Pro Ser Val
                165                 170                 175
```

```
Ala Gly Leu Ser Ala Asn Asp Phe Ile Ile Thr Ala Arg Ile Asp Gln
            180                 185                 190
Ile Lys Thr Lys Asp Leu Leu Pro Lys Lys Arg Val Trp Ala Cys
        195                 200                 205

<210>  418
<211>  1033
<212>  DNA
<213>  Triticum aestivum

<400>  418
gcccgcgggg cggagtacca tttttcacac cgggaacagg ttattgcccc ttaggcctat     60
ttagggtgac aataatagaa caagtttgga caaataagca cggctggtac cggttccgga    120
tttcccggga tatcgttgac ccacgcgtcc gcgacttggg cacgaggacc cctggacatg    180
gactgaaggg gtagaaaagc cgcgcatcat ccatccatcc cgttccactg caccatggcg    240
ctcatgatca gcccgcccgc gactttcctc cccgtggtcg ccggcaagcc caccaccgcc    300
atgcggaacc tccttttcgg caccaccacc agcaccacca gcggtcaccg gagcactcgc    360
aaggtggtgg cgatggcgga catcctgggc gacttcggcg cgcgggaccc gttcccggag    420
gagatcgcga gcaacttcgg ggagaagacg ctgggctacg tggacacgct gcaccgcatc    480
ctcatcccca cgctctccgt gctctccctc tcccgcgtcc cgctcgacgc tgacccggcc    540
ccgctctccg aggaggacgc ccgcaggctg ctcttcaagg tggtcggctg gcggctcctc    600
ttccccagca agggcgactc cgacgtgctc aagctcgagt gcgtctggaa ggtccgcgac    660
caggcctgcg gcgacgagct cgtcgccagg atcaacaagg cgctcgacgg cgccggccac    720
gcccccgccg cgctccggtt cgaggcgccc aaccaagtca gggcccagct ctacaagccg    780
tccgtaggcg ggctgagcgc caacgacttc atcatcgcgg cgaggatcga ccagatcaag    840
accaaagatc tcctcccaaa gaagagggtc tgggcatgct aatggccgca gattcccctc    900
ttcttgctcc tctttgcata cacatacaca gttttgtttc atggaaagcc aatggatcca    960
tgtttgtagc tctactgcta gtgtctgatg atgatgatgg cgtagccatt ttgtgcaatc   1020
aaatgatttc tcg                                                      1033

<210>  419
<211>  215
<212>  PRT
<213>  Triticum aestivum

<400>  419
Met Ala Leu Met Ile Ser Pro Pro Ala Thr Phe Leu Pro Val Val Ala
1               5                   10                  15
Gly Lys Pro Thr Thr Ala Met Arg Asn Leu Leu Phe Gly Thr Thr Thr
            20                  25                  30
Ser Thr Thr Ser Gly His Arg Ser Thr Arg Lys Val Val Ala Met Ala
        35                  40                  45
Asp Ile Leu Gly Asp Phe Gly Ala Arg Asp Pro Phe Pro Glu Glu Ile
    50                  55                  60
Ala Ser Asn Phe Gly Glu Lys Thr Leu Gly Tyr Val Asp Thr Leu His
65                  70                  75                  80
Arg Ile Leu Ile Pro Thr Leu Ser Val Leu Ser Leu Ser Arg Val Pro
                85                  90                  95
Leu Asp Ala Asp Pro Ala Pro Leu Ser Glu Glu Asp Ala Arg Arg Leu
            100                 105                 110
Leu Phe Lys Val Val Gly Trp Arg Leu Leu Phe Pro Ser Lys Gly Asp
        115                 120                 125
Ser Asp Val Leu Lys Leu Glu Cys Val Trp Lys Val Arg Asp Gln Ala
    130                 135                 140
Cys Gly Asp Glu Leu Val Ala Arg Ile Asn Lys Ala Leu Asp Gly Ala
145                 150                 155                 160
Gly His Ala Pro Ala Ala Leu Arg Phe Glu Ala Pro Asn Gln Val Arg
                165                 170                 175
Ala Gln Leu Tyr Lys Pro Ser Val Gly Gly Leu Ser Ala Asn Asp Phe
```

```
            180                 185                 190
Ile Ile Ala Ala Arg Ile Asp Gln Ile Lys Thr Lys Asp Leu Leu Pro
        195                 200                 205
Lys Lys Arg Val Trp Ala Cys
    210                 215
```

<210> 420
<211> 746
<212> DNA
<213> Triticum aestivum

<400> 420

```
gagggcagcc gactggacgc cgcaagcagc gcagcgccgg tctgctgctg cgtacattca     60
tccggatcca gccccgggcg ccgatgagcg ccgccggtgg agccgcgccc ttctacgcct    120
cgtgtccctc gtcgccccgc accgtcgcgc acctggaggt gaggggggag caggccgctg    180
ctgaagttga attatcaaag aagagttgta tcccatgcat ttcaaaggat ctgcatgcca    240
tgtcagaaga ttctgctaaa aagtcacttg aacaggtgac tggttgggaa ctgaaaaatg    300
aaggtgacat tttgaaatta cacagagcat ggaaggtgaa gaattttgta aaaggtcttg    360
agttctttca gcttgttgct gctgttgctg aggaagaagg tcaccaccca gatcttcatc    420
tcgtcagttg gaacaatgtg aaaatcgatg tctggactca ttcagtcaga ggtttaacag    480
ataatgactt catccttgct gcaaagatca atgaactcaa gctagaaggc cttttgagca    540
agaaaaaggc tacttcccag gagtagcttt gggagatcaa gcagataact tttaatgtgt    600
agatgccaag aaatgccctt ttatattgtc tgcgatgatt gtacatgccg ttccaaaatg    660
agtacttccg atgtgaattc cacttaacct ttggtgagat ctatctgttc tgggcgatgc    720
gtttctctga tgatacgaat ctgggt                                          746
```

<210> 421
<211> 160
<212> PRT
<213> Triticum aestivum

<400> 421

```
Met Ser Ala Ala Gly Gly Ala Ala Pro Phe Tyr Ala Ser Cys Pro Ser
1               5                   10                  15
Ser Pro Arg Thr Val Ala His Leu Glu Val Arg Gly Glu Gln Ala Ala
            20                  25                  30
Ala Glu Val Glu Leu Ser Lys Lys Ser Cys Ile Pro Cys Ile Ser Lys
        35                  40                  45
Asp Leu His Ala Met Ser Glu Asp Ser Ala Lys Lys Ser Leu Glu Gln
    50                  55                  60
Val Thr Gly Trp Glu Leu Lys Asn Glu Gly Asp Ile Leu Lys Leu His
65                  70                  75                  80
Arg Ala Trp Lys Val Lys Asn Phe Val Lys Gly Leu Glu Phe Phe Gln
                85                  90                  95
Leu Val Ala Ala Val Ala Glu Glu Glu Gly His His Pro Asp Leu His
            100                 105                 110
Leu Val Ser Trp Asn Asn Val Lys Ile Asp Val Trp Thr His Ser Val
        115                 120                 125
Arg Gly Leu Thr Asp Asn Asp Phe Ile Leu Ala Ala Lys Ile Asn Glu
    130                 135                 140
Leu Lys Leu Glu Gly Leu Leu Ser Lys Lys Lys Ala Thr Ser Gln Glu
145                 150                 155                 160
```

<210> 422
<211> 738
<212> DNA
<213> Triticum aestivum

<400> 422

```
ccacgcgtcc gcccactccc acggcactgc gccgccgtct ccccgaagct cagggatgga      60
ggggcgtacc ctactcctcg accaggcggc acccagcgcg tccgccccta ctggccgctc     120
cgcctcgcag gcgacggggg acgaggccgc ggctaaagtc gaattatcaa aaaggagctg     180
tgtctcatgc aattcaaagg atttacaagc catgtcagaa gattctgcta aaacgttgct     240
agagcaggtg gctggctggg aggtgaaaaa tgagggcgac attctgaaat tgcacagggc     300
atggaaggtg aaaaactttg ccaaagggct tgagttcttt cagcttgttg ctgctgttgc     360
ggaggaagaa ggtcaccacc cagatcttca tcttgttggt tggaataatg tgaaaattga     420
tgtgtggact cactcagtca gtggtttaac agataatgat ttcatccttg ctgcaaagat     480
caatgaactc aagctaggag gccttttgag caagaaaaaa gctactgccc agaagtagct     540
cttgttaatc aaagatatcc ttttgtattt ggggaaaatt gcatatgccg ctccaaaagc     600
aggattcgtc cagtatatat tctgggataa atgatggttt ggcaatgcta ttgggtcctc     660
ctgagaaagt ccagggagga tccccgaaat taatttaatg ggtaatgagg tctgccacaa     720
aacctttctg gattttgg                                                   738
```

<210> 423
<211> 160
<212> PRT
<213> Triticum aestivum

<400> 423

```
Met Glu Gly Arg Thr Leu Leu Leu Asp Gln Ala Ala Pro Ser Ala Ser
1               5                   10                  15
Ala Pro Thr Gly Arg Ser Ala Ser Gln Ala Thr Gly Asp Glu Ala Ala
            20                  25                  30
Ala Lys Val Glu Leu Ser Lys Arg Ser Cys Val Ser Cys Asn Ser Lys
        35                  40                  45
Asp Leu Gln Ala Met Ser Glu Asp Ser Ala Lys Thr Leu Leu Glu Gln
    50                  55                  60
Val Ala Gly Trp Glu Val Lys Asn Glu Gly Asp Ile Leu Lys Leu His
65                  70                  75                  80
Arg Ala Trp Lys Val Lys Asn Phe Ala Lys Gly Leu Glu Phe Phe Gln
                85                  90                  95
Leu Val Ala Ala Val Ala Glu Glu Glu Gly His His Pro Asp Leu His
            100                 105                 110
Leu Val Gly Trp Asn Asn Val Lys Ile Asp Val Trp Thr His Ser Val
        115                 120                 125
Ser Gly Leu Thr Asp Asn Asp Phe Ile Leu Ala Ala Lys Ile Asn Glu
    130                 135                 140
Leu Lys Leu Gly Gly Leu Leu Ser Lys Lys Lys Ala Thr Ala Gln Lys
145                 150                 155                 160
```

<210> 424
<211> 726
<212> DNA
<213> Triticum aestivum

<220>
<221> misc_feature
<222> (514)..(514)
<223> n is a, c, g, or t

<220>
<221> misc_feature
<222> (692)..(692)
<223> n is a, c, g, or t

<400> 424

```
ccacgcgtcc gcatccatcc atccgttcca ctgcaccatg gcgctcatgc tcagcccggc      60
cgcgaccttc ctccccgtcg tcgccggcaa gcccaccacc gccatgcgga acctcctctt     120
```

```
cggcaccacc accagcacca ccagcggtca ccggagcact cgcaaggtgg tggcgatggc    180
ggacatcctg ggcgacttcg gcgcgcggga cccgttcccg gaggagatcg cgagcaactt    240
cggggagaag acgctgggca acgtggacac gctgcaccgc atcctcatcc ccacgctctc    300
cgtgctctcc ctctcccgcg tcccgctcga cgccgacccg gccccgctct ccgaggagga    360
cgcccgcagg ctgctcttca aggtggtcgg ctggcggctc ctcttcccca gcaagggcga    420
ctccgacgtg ctcaagctcg agtgcgtctg gaaggtccgc gaccaggcct gcggcgacga    480
gctcgtcgcc aggatcaaca aggcgctcga cggngccggc cacgcccccg gcgcgcttcg    540
gttcgaggcg cccaaccaag tcagggccca gctctacacg cccgtcgtag gcgggctgag    600
cgccaacgac ttcatcatcg cggcgaggat cgaccagatc aagacaaaga tctcctccaa    660
aaaagaaggt ctgggcatgc taatggccgc anaatcccct ccttcttgct cctccttgca    720
tacaca                                                               726

<210>  425
<211>  230
<212>  PRT
<213>  Triticum aestivum

<220>
<221>  misc_feature
<222>  (219)..(219)
<223>  Xaa can be any naturally occurring amino acid

<220>
<221>  misc_feature
<222>  (230)..(230)
<223>  Xaa can be any naturally occurring amino acid

<400>  425
Met Ala Leu Met Leu Ser Pro Ala Ala Thr Phe Leu Pro Val Val Ala
1               5                   10                  15
Gly Lys Pro Thr Thr Ala Met Arg Asn Leu Leu Phe Gly Thr Thr Thr
            20                  25                  30
Ser Thr Thr Ser Gly His Arg Ser Thr Arg Lys Val Val Ala Met Ala
        35                  40                  45
Asp Ile Leu Gly Asp Phe Gly Ala Arg Asp Pro Phe Pro Glu Glu Ile
    50                  55                  60
Ala Ser Asn Phe Gly Glu Lys Thr Leu Gly Asn Val Asp Thr Leu His
65                  70                  75                  80
Arg Ile Leu Ile Pro Thr Leu Ser Val Leu Ser Leu Ser Arg Val Pro
                85                  90                  95
Leu Asp Ala Asp Pro Ala Pro Leu Ser Glu Glu Asp Ala Arg Arg Leu
            100                 105                 110
Leu Phe Lys Val Val Gly Trp Arg Leu Leu Phe Pro Ser Lys Gly Asp
        115                 120                 125
Ser Asp Val Leu Lys Leu Glu Cys Val Trp Lys Val Arg Asp Gln Ala
    130                 135                 140
Cys Gly Asp Glu Leu Val Ala Arg Ile Asn Lys Ala Leu Asp Gly Ala
145                 150                 155                 160
Gly His Ala Pro Gly Ala Leu Arg Phe Glu Ala Pro Asn Gln Val Arg
                165                 170                 175
Ala Gln Leu Tyr Thr Pro Val Val Gly Gly Leu Ser Ala Asn Asp Phe
            180                 185                 190
Ile Ile Ala Ala Arg Ile Asp Gln Ile Lys Thr Lys Ile Ser Ser Lys
        195                 200                 205
Lys Glu Gly Leu Gly Met Leu Met Ala Ala Xaa Ser Pro Pro Ser Cys
    210                 215                 220
Ser Ser Leu His Thr Xaa
225                 230
```

<210> 426
<211> 788
<212> DNA
<213> Triticum aestivum

<400> 426
ccacgcgtcc gcatccatcc gttccactgc accatggcgc tcatgctcag cccggccgcg 60
accttcctcc ccgtcgtcgc cggcaagccc accaccgcca tgcggaacct cctcttcggc 120
accaccacca gcaccaccag cggtcaccgg agcactcgca aggtggtggc gatggcggac 180
atcctgggcg acttcggcgc gcgggacccg ttcccggagg agatcgcgag caacttcggg 240
gagaagacgc tgggcaacgt ggacacgctg caccgcatcc tcatccccac gctctccgtg 300
ctctccctct cccgcgtccc gctcgacgcc gacccggccc cgctctccga ggaggacgcc 360
cgcaggctgc tcttcaaggt ggtcggctgg cggctcctct ttcccagcaa gggcgactcc 420
gacgtgctca agctcgagtg cgtctggaag gtccgcgacc aggcctgcgg cgacgagctc 480
gtcgccagga tcaacaaggg gcttcgaggc gccgggccaa gcccccgacg cgtttcgggt 540
tcaaggcgcc aaacaagtca ggggcccact ctacaccccg tccgtaaggg ggatgaaacc 600
caaaaaactt cctcatccgg ggggaggatc gaacccaaat caggaactaa gtatccccct 660
cccaaaaaaa gaagggtctg ggcaaatgct aatgggccga caaattcccc ctctttcttt 720
ggctcctcct tttggaataa acaccaccca gatttttggt tttcctggga aaagcccata 780
gggtatcc 788

<210> 427
<211> 252
<212> PRT
<213> Triticum aestivum

<400> 427
Met Ala Leu Met Leu Ser Pro Ala Ala Thr Phe Leu Pro Val Val Ala
1 5 10 15
Gly Lys Pro Thr Thr Ala Met Arg Asn Leu Leu Phe Gly Thr Thr Thr
20 25 30
Ser Thr Thr Ser Gly His Arg Ser Thr Arg Lys Val Val Ala Met Ala
35 40 45
Asp Ile Leu Gly Asp Phe Gly Ala Arg Asp Pro Phe Pro Glu Glu Ile
50 55 60
Ala Ser Asn Phe Gly Glu Lys Thr Leu Gly Asn Val Asp Thr Leu His
65 70 75 80
Arg Ile Leu Ile Pro Thr Leu Ser Val Leu Ser Leu Ser Arg Val Pro
85 90 95
Leu Asp Ala Asp Pro Ala Pro Leu Ser Glu Glu Asp Ala Arg Arg Leu
100 105 110
Leu Phe Lys Val Val Gly Trp Arg Leu Leu Phe Pro Ser Lys Gly Asp
115 120 125
Ser Asp Val Leu Lys Leu Glu Cys Val Trp Lys Val Arg Asp Gln Ala
130 135 140
Cys Gly Asp Glu Leu Val Ala Arg Ile Asn Lys Gly Leu Arg Gly Ala
145 150 155 160
Gly Pro Ser Pro Arg Arg Val Ser Gly Ser Arg Arg Gln Thr Ser Gln
165 170 175
Gly Pro Thr Leu His Pro Val Arg Lys Gly Asp Glu Thr Gln Lys Thr
180 185 190
Ser Ser Ser Gly Gly Arg Ile Glu Pro Lys Ser Gly Thr Lys Tyr Pro
195 200 205
Pro Pro Lys Lys Arg Arg Val Trp Ala Asn Ala Asn Gly Pro Thr Asn
210 215 220
Ser Pro Ser Phe Phe Gly Ser Ser Phe Trp Asn Lys His His Pro Asp
225 230 235 240
Phe Trp Phe Ser Trp Glu Lys Pro Ile Gly Tyr Pro
245 250

<210> 428
<211> 800
<212> DNA
<213> Triticum aestivum

<400> 428
ggacaaaatg gcgacgccat catcatcatc aaacactagc agtagagcta caaacatgga 60
tccattggct ttccatgaaa caaaactgtg tgtgtgtatg caaagaggag caagaagagg 120
ggaatctgcg gccattagca tgcccagacc ctcttctttg ggaggagatc tttggtcttg 180
atctggtcga tcctcgccgc gatgatgaag tcgttggcgc tcagcccgcc tacggacggc 240
gtgtagagct gggccctgac ttggttgggc gcctcgaacc ggagcgcggc gggggcgtgg 300
ccggcgccgt cgagcgcctt gttgatcctg gcgacgagct cgtcgccgca ggcctggtcg 360
cggaccttcc agacgcactc gagcttgagc acgtcggagt cgcccttgct ggggaagagg 420
agccgccagc cgaccacctt gaagagcagc ctgcgggcgt cctcctcgga gagcggggcc 480
gggtcggcgt cgagcgggac gcgggagagg gagagcacgg agagcgtggg gatgaggatg 540
cggtgcagcg tgtccacgtt gcccagcgtc ttctccccga agttgctcgc gatctcctcc 600
gggaacgggt cccgcgcgcc gaagtcgccc aggatgtccg ccatcgccac caccttgcga 660
gtgctccggt gaccgctggt ggtgctggtg gtggtgccga agaggaggtt ccgcatggcg 720
gtggtgggct tgccggcgac gacggggagg aaggtcgcgg ccgggctgag catgagcgcc 780
atggtgcagc ggacgcgtgg 800

<210> 429
<211> 215
<212> PRT
<213> Triticum aestivum

<400> 429
Met Ala Leu Met Leu Ser Pro Ala Ala Thr Phe Leu Pro Val Val Ala
1               5                   10                  15
Gly Lys Pro Thr Thr Ala Met Arg Asn Leu Leu Phe Gly Thr Thr Thr
            20                  25                  30
Ser Thr Thr Ser Gly His Arg Ser Thr Arg Lys Val Val Ala Met Ala
        35                  40                  45
Asp Ile Leu Gly Asp Phe Gly Ala Arg Asp Pro Phe Pro Glu Glu Ile
    50                  55                  60
Ala Ser Asn Phe Gly Glu Lys Thr Leu Gly Asn Val Asp Thr Leu His
65                  70                  75                  80
Arg Ile Leu Ile Pro Thr Leu Ser Val Leu Ser Leu Ser Arg Val Pro
                85                  90                  95
Leu Asp Ala Asp Pro Ala Pro Leu Ser Glu Glu Asp Ala Arg Arg Leu
            100                 105                 110
Leu Phe Lys Val Val Gly Trp Arg Leu Leu Phe Pro Ser Lys Gly Asp
        115                 120                 125
Ser Asp Val Leu Lys Leu Glu Cys Val Trp Lys Val Arg Asp Gln Ala
    130                 135                 140
Cys Gly Asp Glu Leu Val Ala Arg Ile Asn Lys Ala Leu Asp Gly Ala
145                 150                 155                 160
Gly His Ala Pro Ala Ala Leu Arg Phe Glu Ala Pro Asn Gln Val Arg
                165                 170                 175
Ala Gln Leu Tyr Thr Pro Ser Val Gly Gly Leu Ser Ala Asn Asp Phe
            180                 185                 190
Ile Ile Ala Ala Arg Ile Asp Gln Ile Lys Thr Lys Asp Leu Leu Pro
        195                 200                 205
Lys Lys Arg Val Trp Ala Cys
    210                 215

<210> 430
<211> 656

<212> DNA
<213> Triticum aestivum

<400> 430
ctcctccgtc cattgcttcc cccagccgcg catcatccat ccatccatcc gttcctctct 60
ccttccttcc tgtccactac accatggcgc tcatgctcag cccggccgcg accttcctcc 120
ccgtcgccgg caagcccgcc gccgccatgc ggaacctcct cttcggcacc agcagcaagg 180
gaagaagcgt tcgcaaggtg gtggcgatgg cggacatcct gggcgacttc ggggcgcggg 240
acccgttccc ggaggagatc gcgagcaact tcggggagaa gacgctgggc aacgtggaca 300
cgctgcaccg catcctcatt cccacgctct ccgtgctctc gctctcccgc gtgccgctcg 360
acgccgaccc cgcgccgctc tccgaggagg acgcccgcaa gctgctcttc aaggtggtcg 420
gatggcggct cctcttcccc agcaagggcg actccgacgt gctcaagctc gagtgcgtct 480
ggaaggtccg cgaccaggcc tgcggcgacg agctcatcgc caggatcaac aagaccctcg 540
acggcgccgg ccatgccccc gccgcgctcc agttcgaggc gcccaaccaa gtcagggccc 600
agctctacac gccctccgta ggtgggctga gcgccaacga cttcatcatc gccgcg 656

<210> 431
<211> 191
<212> PRT
<213> Triticum aestivum

<400> 431
Met Ala Leu Met Leu Ser Pro Ala Ala Thr Phe Leu Pro Val Ala Gly
1 5 10 15
Lys Pro Ala Ala Ala Met Arg Asn Leu Leu Phe Gly Thr Ser Ser Lys
20 25 30
Gly Arg Ser Val Arg Lys Val Val Ala Met Ala Asp Ile Leu Gly Asp
35 40 45
Phe Gly Ala Arg Asp Pro Phe Pro Glu Glu Ile Ala Ser Asn Phe Gly
50 55 60
Glu Lys Thr Leu Gly Asn Val Asp Thr Leu His Arg Ile Leu Ile Pro
65 70 75 80
Thr Leu Ser Val Leu Ser Leu Ser Arg Val Pro Leu Asp Ala Asp Pro
85 90 95
Ala Pro Leu Ser Glu Glu Asp Ala Arg Lys Leu Leu Phe Lys Val Val
100 105 110
Gly Trp Arg Leu Leu Phe Pro Ser Lys Gly Asp Ser Asp Val Leu Lys
115 120 125
Leu Glu Cys Val Trp Lys Val Arg Asp Gln Ala Cys Gly Asp Glu Leu
130 135 140
Ile Ala Arg Ile Asn Lys Thr Leu Asp Gly Ala Gly His Ala Pro Ala
145 150 155 160
Ala Leu Gln Phe Glu Ala Pro Asn Gln Val Arg Ala Gln Leu Tyr Thr
165 170 175
Pro Ser Val Gly Gly Leu Ser Ala Asn Asp Phe Ile Ile Ala Ala
180 185 190

<210> 432
<211> 757
<212> DNA
<213> Triticum aestivum

<400> 432
tccgtcccag tgcgccatgg cgctcatgct cagcccggcc gcgaccttcc tccccgtcgc 60
caccggcaag cccaccgcca ccgccggcgc catgcgaaac ctcctcttca ccaccaccag 120
cggcaccgcc agcggccacc ggagcagcag gaaggtggtg gcgatggcgg acatcctggg 180
ggacttcggc gcgcgggacc cgttcccgga ggagatcgcg agcaacttcg ggagaagac 240
gctgggcaac gtggacacgc tgcaccgcat cctcatcccc acgctctccg tgctctccct 300
ctcccgcgtc ccgctcgaag cccacccggc gccgctctcc gaggaggacg cccgcaggct 360

```
gctcttcaag gtcgtcggat ggcgcctcct cttccccggc aagggcgact ccgacgtgct    420
caagctcgag tgcgtctgga aggtccgcga ccaggcctgc ggcgacgagc tcatcgccag    480
gatcggcaag gcgctcgacg gcgccggcca tgcccccgcc gcgctcctct tcgagccgcc    540
caaccaagtc agggcacagc tctgcacgcc ctccgtaggt gggctgagcg ccaacgactt    600
catcatcgcg gcgaggatcg accagatcaa gaccaaagat ctcctcccaa agaagagggt    660
ctgggcatgc taatccatgg cgtcttgttc ctatttcttt gcattgcaca catacacagt    720
tttgtttcat ggaaagtttg tagctctact gctagtg                             757

<210>  433
<211>  218
<212>  PRT
<213>  Triticum aestivum

<400>  433
Met Ala Leu Met Leu Ser Pro Ala Ala Thr Phe Leu Pro Val Ala Thr
1               5                   10                  15
Gly Lys Pro Thr Ala Thr Ala Gly Ala Met Arg Asn Leu Leu Phe Thr
            20                  25                  30
Thr Thr Ser Gly Thr Ala Ser Gly His Arg Ser Ser Arg Lys Val Val
        35                  40                  45
Ala Met Ala Asp Ile Leu Gly Asp Phe Gly Ala Arg Asp Pro Phe Pro
    50                  55                  60
Glu Glu Ile Ala Ser Asn Phe Gly Glu Lys Thr Leu Gly Asn Val Asp
65                  70                  75                  80
Thr Leu His Arg Ile Leu Ile Pro Thr Leu Ser Val Leu Ser Leu Ser
                85                  90                  95
Arg Val Pro Leu Glu Ala His Pro Ala Pro Leu Ser Glu Glu Asp Ala
            100                 105                 110
Arg Arg Leu Leu Phe Lys Val Val Gly Trp Arg Leu Leu Phe Pro Gly
        115                 120                 125
Lys Gly Asp Ser Asp Val Leu Lys Leu Glu Cys Val Trp Lys Val Arg
    130                 135                 140
Asp Gln Ala Cys Gly Asp Glu Leu Ile Ala Arg Ile Gly Lys Ala Leu
145                 150                 155                 160
Asp Gly Ala Gly His Ala Pro Ala Ala Leu Leu Phe Glu Pro Pro Asn
                165                 170                 175
Gln Val Arg Ala Gln Leu Cys Thr Pro Ser Val Gly Gly Leu Ser Ala
            180                 185                 190
Asn Asp Phe Ile Ile Ala Ala Arg Ile Asp Gln Ile Lys Thr Lys Asp
        195                 200                 205
Leu Leu Pro Lys Lys Arg Val Trp Ala Cys
    210                 215

<210>  434
<211>  727
<212>  DNA
<213>  Triticum aestivum

<220>
<221>  misc_feature
<222>  (588)..(588)
<223>  n is a, c, g, or t

<220>
<221>  misc_feature
<222>  (598)..(598)
<223>  n is a, c, g, or t

<220>
```

<221>  misc_feature
<222>  (622)..(622)
<223>  n is a, c, g, or t

<220>
<221>  misc_feature
<222>  (635)..(635)
<223>  n is a, c, g, or t

<220>
<221>  misc_feature
<222>  (662)..(662)
<223>  n is a, c, g, or t

<220>
<221>  misc_feature
<222>  (687)..(687)
<223>  n is a, c, g, or t

<220>
<221>  misc_feature
<222>  (692)..(692)
<223>  n is a, c, g, or t

<220>
<221>  misc_feature
<222>  (723)..(723)
<223>  n is a, c, g, or t

<400>  434
gccgcgcatc atccatccat ccgttccact gcaccatggc gctcatgctc agcccggccg     60
cgaccttcct ccccgtcgtc gccggcaagc ccaccaccgc catgcggaac ctcctcttcg    120
gcaccaccac cagcaccacc agcggtcacc ggagcactcg caaggtggtg gcgatggcgg    180
acatcctggg cgacttcggc gcgcgggacc cgttcccgga ggagatcgcg agcaacttcg    240
gggagaagac gctgggcaac gtggacacgc tgcaccgcat cctcatcccc acgctctccg    300
tgctctccct ctcccgcgtc ccgctcgacg ccgacccggc cccgctctcc gaggaggacg    360
cccgcaggct gctcttcaag gtggtcggct ggcggctcct cttccccagc aagggcgact    420
ccgacgtgct caagctcgag tgcgtctgga aggtccgcga ccaggcctgc ggcgacgagc    480
tcgtcgccag gatcaacaag gcgctcgacg gcgccggcca cgcccccgcc gcgctccggt    540
tcgaggcgcc caaccaagtc agggcccagc tctacacgcc gtccgtangc gggctgancg    600
ccaacgactt catcatcgcg gngaggatcg accanatcaa gaccaaagat ctcctcccaa    660
anaagagggt ctgggcatgc taatggncgc anattcccct cttcttgctc ctcttttgca    720
tanacac                                                              727

<210>  435
<211>  215
<212>  PRT
<213>  Triticum aestivum

<220>
<221>  misc_feature
<222>  (185)..(185)
<223>  Xaa can be any naturally occurring amino acid

<220>
<221>  misc_feature
<222>  (188)..(188)
<223>  Xaa can be any naturally occurring amino acid

<220>
<221> misc_feature
<222> (196)..(196)
<223> Xaa can be any naturally occurring amino acid

<220>
<221> misc_feature
<222> (200)..(200)
<223> Xaa can be any naturally occurring amino acid

<220>
<221> misc_feature
<222> (209)..(209)
<223> Xaa can be any naturally occurring amino acid

<400> 435
```
Met Ala Leu Met Leu Ser Pro Ala Ala Thr Phe Leu Pro Val Val Ala
1               5                   10                  15
Gly Lys Pro Thr Thr Ala Met Arg Asn Leu Leu Phe Gly Thr Thr Thr
            20                  25                  30
Ser Thr Thr Ser Gly His Arg Ser Thr Arg Lys Val Val Ala Met Ala
        35                  40                  45
Asp Ile Leu Gly Asp Phe Gly Ala Arg Asp Pro Phe Pro Glu Glu Ile
    50                  55                  60
Ala Ser Asn Phe Gly Glu Lys Thr Leu Gly Asn Val Asp Thr Leu His
65                  70                  75                  80
Arg Ile Leu Ile Pro Thr Leu Ser Val Leu Ser Leu Ser Arg Val Pro
                85                  90                  95
Leu Asp Ala Asp Pro Ala Pro Leu Ser Glu Glu Asp Ala Arg Arg Leu
            100                 105                 110
Leu Phe Lys Val Val Gly Trp Arg Leu Leu Phe Pro Ser Lys Gly Asp
        115                 120                 125
Ser Asp Val Leu Lys Leu Glu Cys Val Trp Lys Val Arg Asp Gln Ala
    130                 135                 140
Cys Gly Asp Glu Leu Val Ala Arg Ile Asn Lys Ala Leu Asp Gly Ala
145                 150                 155                 160
Gly His Ala Pro Ala Ala Leu Arg Phe Glu Ala Pro Asn Gln Val Arg
                165                 170                 175
Ala Gln Leu Tyr Thr Pro Ser Val Xaa Gly Leu Xaa Ala Asn Asp Phe
            180                 185                 190
Ile Ile Ala Xaa Arg Ile Asp Xaa Ile Lys Thr Lys Asp Leu Leu Pro
        195                 200                 205
Xaa Lys Arg Val Trp Ala Cys
    210                 215
```

<210> 436
<211> 946
<212> DNA
<213> Triticum aestivum

<400> 436
```
catccatcca tccgtccact ctccttcctt cctttcacta caccatggcg ctcatgctca      60
gcccggccgc gaccttcttc tccgtcgccg gcggcaagcc ctcctccgcc acgaggagcc     120
tcttcttcac caccaccagc accaccaagg gcagaagcgt tcgcaaggtg gtggcgatgg     180
cggacatcct gggcgacttc ggcgcgcggg acccgttccc ggaggagatc gcgagcaact     240
tcggggagaa gacgctgggc aacgtggaca cgctgcaccg catcctcatc cccacgctct     300
ccgtgctctc gctctcccgc gtgccgctcg acgccgaccc ggcgccgctc tccgaggagg     360
acgcccgcaa gctgctcttc aaggtggtcg gatggcggct cctcttcccc agcaagggcg     420
actccgacgt gctcaagctc gagtgcgtct ggaaggtccg cgaccaggcc tgcggcgacg     480
```

```
agctcatcgc caggatcaac aagacgctcg acggcgccgg ccatgccccc gccgcgctcc     540
agttcgaggc gcccaaccaa gtcagggccc agctctacac gccctccgta ggtgggctga     600
gcgccaacga cttcatcatc gccgcgagga tcgaccagat caagaccaaa gatctcctcc     660
caaagaagag ggtctgggca tgctaatggc gaccgcccat gccatcttgt tcctctttct     720
ttgcattgca tacatacaca gttttgtttc atggaatgga agccaaccta tccaccattg     780
tagctctacc cctagtgttt aatgatgatg atgatgccat cgccattttg tcaatcaaat     840
caaatgattt ctcaacttct taggttcttc cgaatgcgag gcacgattac aacaacactt     900
gtataattac taaaatgtca agaaattcat tagctattta aatgct                    946
```

```
<210>  437
<211>  213
<212>  PRT
<213>  Triticum aestivum

<400>  437
Met Ala Leu Met Leu Ser Pro Ala Ala Thr Phe Phe Ser Val Ala Gly
1               5                   10                  15
Gly Lys Pro Ser Ser Ala Thr Arg Ser Leu Phe Phe Thr Thr Thr Ser
            20                  25                  30
Thr Thr Lys Gly Arg Ser Val Arg Lys Val Val Ala Met Ala Asp Ile
        35                  40                  45
Leu Gly Asp Phe Gly Ala Arg Asp Pro Phe Pro Glu Glu Ile Ala Ser
    50                  55                  60
Asn Phe Gly Glu Lys Thr Leu Gly Asn Val Asp Thr Leu His Arg Ile
65                  70                  75                  80
Leu Ile Pro Thr Leu Ser Val Leu Ser Leu Ser Arg Val Pro Leu Asp
                85                  90                  95
Ala Asp Pro Ala Pro Leu Ser Glu Glu Asp Ala Arg Lys Leu Leu Phe
            100                 105                 110
Lys Val Val Gly Trp Arg Leu Leu Phe Pro Ser Lys Gly Asp Ser Asp
        115                 120                 125
Val Leu Lys Leu Glu Cys Val Trp Lys Val Arg Asp Gln Ala Cys Gly
    130                 135                 140
Asp Glu Leu Ile Ala Arg Ile Asn Lys Thr Leu Asp Gly Ala Gly His
145                 150                 155                 160
Ala Pro Ala Ala Leu Gln Phe Glu Ala Pro Asn Gln Val Arg Ala Gln
                165                 170                 175
Leu Tyr Thr Pro Ser Val Gly Gly Leu Ser Ala Asn Asp Phe Ile Ile
            180                 185                 190
Ala Ala Arg Ile Asp Gln Ile Lys Thr Lys Asp Leu Leu Pro Lys Lys
        195                 200                 205
Arg Val Trp Ala Cys
    210

<210>  438
<211>  810
<212>  DNA
<213>  Triticum aestivum

<400>  438
gccacttcac cgcgagcccc actgaccctc aaaggcctca acatggcagg tctgctgctg      60
cgtacattca tccggatcca gccccgggcg ccgatgagcg ccgccggtgg agccgcgccc     120
ttctacgcct cgtgtccctc gtcgccccgc accgtcgcgc acctggaggt gaggggggag     180
caggccgctg ctgaagttga attatcaaag aagagttgta tcccatgcat ttcaaaggat     240
ctgcatgcca tgtcagaaga ttctgctaaa aagtcacttg aacaggtgac tggttgggaa     300
ctgaaaaatg aaggtgacat tttgaaatta cacagagcat ggaaggtgaa gaattttgta     360
aaaggtcttg agttctttca gcttgttgct gctgttgctg aggaagaagg tcaccaccca     420
gatcttcatc tcgtcagttg gaacaatgtg aaaatcgatg tctggactca ttcagtcaga     480
ggtttaacag ataatgactt catccttgct gcaaagatca atgaactcaa gctagaaggc     540
```

```
cttttgagca agaaaaaggc tacttcccag gagtagcttt gggagatcaa gcagataact    600
tttaatgtgt agatgccaag aaatgccctt ttatattgtc tgcgatgatt gtacatgccg    660
ttccaaaatg agtacttccg atgtgaattc cacttaccat ttggtgagat ctatccgttc    720
tgggcgatgc gtttctccga tgttgcgaat ctaggatcat cctaatactt gtctgtatct    780
tctgaatata taccgtagtt gaaggtgtca                                     810

<210>  439
<211>  177
<212>  PRT
<213>  Triticum aestivum

<400>  439
Met Ala Gly Leu Leu Leu Arg Thr Phe Ile Arg Ile Gln Pro Arg Ala
1               5                   10                  15
Pro Met Ser Ala Ala Gly Gly Ala Ala Pro Phe Tyr Ala Ser Cys Pro
            20                  25                  30
Ser Ser Pro Arg Thr Val Ala His Leu Glu Val Arg Gly Glu Gln Ala
        35                  40                  45
Ala Ala Glu Val Glu Leu Ser Lys Lys Ser Cys Ile Pro Cys Ile Ser
    50                  55                  60
Lys Asp Leu His Ala Met Ser Glu Asp Ser Ala Lys Lys Ser Leu Glu
65                  70                  75                  80
Gln Val Thr Gly Trp Glu Leu Lys Asn Glu Gly Asp Ile Leu Lys Leu
                85                  90                  95
His Arg Ala Trp Lys Val Lys Asn Phe Val Lys Gly Leu Glu Phe Phe
            100                 105                 110
Gln Leu Val Ala Ala Val Ala Glu Glu Glu Gly His His Pro Asp Leu
        115                 120                 125
His Leu Val Ser Trp Asn Asn Val Lys Ile Asp Val Trp Thr His Ser
    130                 135                 140
Val Arg Gly Leu Thr Asp Asn Asp Phe Ile Leu Ala Ala Lys Ile Asn
145                 150                 155                 160
Glu Leu Lys Leu Glu Gly Leu Leu Ser Lys Lys Lys Ala Thr Ser Gln
                165                 170                 175
Glu


<210>  440
<211>  173
<212>  PRT
<213>  Artificial sequence

<220>
<223>  PCD-domain in SEQ ID NO: 2

<400>  440
Gln Leu Ala Val Arg Arg Lys Gln Lys Ser Val Val Val Ala Met Ala
1               5                   10                  15
Asp Leu Leu Gly Asp Phe Gly Ala Arg Asp Pro Phe Pro Glu Glu Ile
            20                  25                  30
Glu Ser Asn Phe Gly Glu Arg Val Leu Gly Asn Val Asp Thr Leu His
        35                  40                  45
Asn Ile Leu Ile Pro Thr Leu Ser Val Leu Ser Ile Ala Arg Leu Pro
    50                  55                  60
Leu Glu Pro Asn Pro Ala Pro Val Asp Ala Ala Asp Ala Arg Arg Leu
65                  70                  75                  80
Leu His Lys Val Val Gly Trp Arg Leu Leu Asp Asp Ala Asp Gly Met
                85                  90                  95
Arg Leu Gln Cys Val Trp Lys Val Arg Asp Glu Ala Cys Gly His Glu
```

```
            100                 105                 110
Leu Val Ala Arg Ile Asn Ala Ala Val Asp Gly Ala Pro Ala Thr Val
        115                 120                 125
Val Phe Glu Ala Pro Asn Gln Val Arg Ala Glu Leu Gln Thr Pro Ser
    130                 135                 140
Ala Gly Gly Leu Thr Val Asn Asp Phe Ile Val Ala Ala Arg Ile Asp
145                 150                 155                 160
Lys Val Lys Thr Val Asp Leu Ile Pro Lys Lys Arg Val
                165                 170
```

<210> 441
<211> 20
<212> PRT
<213> Artificial sequence

<220>
<223> motif 12

<220>
<221> VARIANT
<222> (1)..(1)
<223> / replace = "Glu"

<220>
<221> VARIANT
<222> (2)..(2)
<223> / replace = "Asn"

<220>
<221> VARIANT
<222> (3)..(3)
<223> / replace = "Leu"

<220>
<221> UNSURE
<222> (9)..(11)
<223> Unknown amino acid

<220>
<221> UNSURE
<222> (13)..(16)
<223> Unknown amino acid

<220>
<221> VARIANT
<222> (19)..(19)
<223> / replace = "Glu"

<400> 441

```
Gly Asp Phe Gly Ala Arg Asp Pro Xaa Xaa Xaa Glu Xaa Xaa Xaa Xaa
1               5                   10                  15
Phe Gly Asp Lys
            20
```

<210> 442
<211> 40
<212> PRT
<213> Artificial sequence

```
<220>
<223>  motif 13

<220>
<221>  VARIANT
<222>  (1)..(1)
<223>  / replace = "Asp" / replace = "Lys" / replace = "His" / replace =
       "Gln" / replace = "Asn"

<220>
<221>  UNSURE
<222>  (2)..(4)
<223>  Unkown amino acid

<220>
<221>  VARIANT
<222>  (6)..(6)
<223>  / replace = "Cys" / replace = "Ser"

<220>
<221>  UNSURE
<222>  (8)..(12)
<223>  Unkown amino acid

<220>
<221>  UNSURE
<222>  (13)..(13)
<223>  Unkown amino acid or gap

<220>
<221>  VARIANT
<222>  (14)..(14)
<223>  / replace = "Trp" / replace = "Phe" / replace = "His"

<220>
<221>  UNSURE
<222>  (15)..(23)
<223>  Unkown amino acid

<220>
<221>  VARIANT
<222>  (24)..(24)
<223>  / replace = "Trp"

<220>
<221>  UNSURE
<222>  (25)..(32)
<223>  Unkown amino acid

<220>
<221>  UNSURE
<222>  (33)..(39)
<223>  Unkown amino acid or a gap

<400>  442
Glu Xaa Xaa Xaa His His Pro Xaa Xaa Xaa Xaa Xaa Xaa Tyr Xaa Xaa
1               5                   10                  15
Xaa Xaa Xaa Xaa Xaa Xaa Xaa His Xaa Xaa Xaa Xaa Xaa Xaa Xaa Xaa
            20                  25                  30
```

```
Xaa Xaa Xaa Xaa Xaa Xaa Xaa Asp
        35                  40

<210>  443
<211>  4
<212>  PRT
<213>  Artificial sequence

<220>
<223>  motif 14

<220>
<221>  VARIANT
<222>  (3)..(3)
<223>  / replace = "Leu"

<220>
<221>  VARIANT
<222>  (4)..(4)
<223>  / replace = "Lys"

<400>  443
Trp Lys Val Arg
1

<210>  444
<211>  4
<212>  PRT
<213>  Artificial sequence

<220>
<223>  motif 15

<400>  444
Thr Asp Phe Ile
1

<210>  445
<211>  6
<212>  PRT
<213>  Artificial sequence

<220>
<223>  motif 16

<220>
<221>  VARIANT
<222>  (1)..(1)
<223>  / replace = "Val"

<220>
<221>  VARIANT
<222>  (2)..(2)
<223>  / replace = "Ile"

<220>
<221>  VARIANT
<222>  (3)..(3)
<223>  / replace = "Arg" / replace = "Ser" / replace = "Ala"
```

```
<220>
<221>  VARIANT
<222>  (6)..(6)
<223>  / replace = "Ser"

<400>  445
Ser Val Gly Gly Leu Thr
1               5

<210>  446
<211>  9
<212>  PRT
<213>  Artificial sequence

<220>
<223>  motif 17

<220>
<221>  VARIANT
<222>  (4)..(4)
<223>  / replace = "Arg"

<220>
<221>  VARIANT
<222>  (5)..(5)
<223>  / replace = "Arg"

<220>
<221>  VARIANT
<222>  (6)..(6)
<223>  / replace = "Ala"

<220>
<221>  VARIANT
<222>  (7)..(7)
<223>  / replace = "Gly"

<400>  446
Leu Gly Asp Phe Gly Ala Arg Asp Pro
1               5

<210>  447
<211>  7
<212>  PRT
<213>  Artificial sequence

<220>
<223>  motif 18

<220>
<221>  VARIANT
<222>  (2)..(2)
<223>  / replace = "Lys"

<220>
<221>  VARIANT
<222>  (7)..(7)
<223>  / replace = "Ala"
```

<400> 447
His Arg Ile Leu Ile Pro Thr
1 5

<210> 448
<211> 51
<212> DNA
<213> Artificial sequence

<220>
<223> primer 3

<400> 448
ggggacaagt ttgtacaaaa aagcaggctt aaacaatggc gctcaccaac c 51

<210> 449
<211> 52
<212> DNA
<213> Artificial sequence

<220>
<223> primer 4

<400> 449
ggggaccact ttgtacaaga aagctgggta aggggatata tgtgaatgaa ga 52

<210> 450
<211> 2194
<212> DNA
<213> Oryza sativa

<400> 450
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacaggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560

```
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccatttttta attatatgaa atgaactgta gcataagcag tattcatttg  1920
gattattttt tttattagct ctcacccctt cattattctg agctgaaagt ctggcatgaa   1980
ctgtcctcaa ttttgttttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                               2194
```

<210> 451
<211> 1683
<212> DNA
<213> Lycopersicon esculentum

<400> 451

```
atgatttgca ttgagaatga attattgggt tggaaagatt tcccaaaggg gcttaaagtc     60
ctacttcttg atgaagatag caactctgct gctgagatga aatcaaggct tgagaaaatg    120
gactacatag tctactcgtt ctgcaacgag agcgaagctt tgactgcaat ctcaagcaaa    180
tccgagggct ttcatgttgc cattgtggag gtaagtgcag gcaacagtga tggggttcta    240
cggtttcttg aaagtgccaa agatctacca actataatga catcaaatat acattctctt    300
agtaccatga tgaaatgtat tgcgctaggc gcagttgagt tccttcagaa accattgtca    360
gatgataaac tcaaaaatat atggcagcat gtagttcaca aggcattcaa tactagaaag    420
gatgtgtcca aatcacttga gccggtaaaa gattctgtcc tctcgatgct gcagttacaa    480
ctagaaatgg gtgaagcaga tgacaaaagt tcaaatggaa cagaacctcc cactgcagta    540
gcggaaagca atactgaaca gtcatcgggc tgtgataaat accctgctcc ctcaatccca    600
caattgaaac aaggagtgcg atccgtcgat gatggtgact gccatgatca tactatcttc    660
tcaactgacc aagacagtgg ggaacatgat gctgacacta aatccgtcga aactacttat    720
aacaattcac ttgctgagaa taatgtccaa acaagtccta ctgtacagca aggagatatt    780
attttgaaag aggataatgt ttcatctcct gatctaaaga cggagactga tatcgctacc    840
acttcacgaa gtaacgactg ccctgacaat agcattatgc attctgctga acctagtaaa    900
gcatctggtc ctcatagttc aaatgggact aaatccaata ggaagaagat aaaggtagat    960
tggacacctg aactacacaa gaagtttgtt caagcagtag agcaactcgg tatagatcaa   1020
gccattcctt ctcgaatact ggacctgatg aaagtagagg gcttaacgag acataacgta   1080
gctagccatc tccagaaata cagaatgcat cgaaagcaaa ttttgccaaa ggaagtagaa   1140
agaagatggc ctaatccgca accaatagat tcagtccaaa gaagttacta tcctcataaa   1200
cctatcatga cattcccaca atatcattct aatcatgttg ccccaggtgg tcagttctat   1260
cctgcttggg taacaccagc aagttatccg aacggtttac aagtgtgggg ttcaccttac   1320
tatccgggat ggaaacctgc agagacttgg cactggacgc ctcgtccaga gctgcatgct   1380
gatacatggg gctcccctat catgtcaccg tcgcttggat catatccacc atatcctcag   1440
aatgctggag tgtaccggcc acatggaaca cataacagat atagcatgct agagaagtcg   1500
tttgatcttc acccggcgga tgaggtgatt gataaagtag taaaagaggc aataaccaaa   1560
ccatggttac cacttccttt gggcctaaaa gctccttcaa cggagagcgt tcttgacgaa   1620
ctttctagac aaggatctc aaccattcct tcacaaatca acgactcccg ttgtcggaga   1680
tga                                                                 1683
```

<210> 452
<211> 560
<212> PRT
<213> Lycopersicon esculentum

<400> 452

```
Met Ile Cys Ile Glu Asn Glu Leu Leu Gly Trp Lys Asp Phe Pro Lys
1               5                   10                  15
Gly Leu Lys Val Leu Leu Leu Asp Glu Asp Ser Asn Ser Ala Ala Glu
            20                  25                  30
Met Lys Ser Arg Leu Glu Lys Met Asp Tyr Ile Val Tyr Ser Phe Cys
```

```
        35                  40                  45
Asn Glu Ser Glu Ala Leu Thr Ala Ile Ser Ser Lys Ser Glu Gly Phe
    50                  55                  60
His Val Ala Ile Val Glu Val Ser Ala Gly Asn Ser Asp Gly Val Leu
65                  70                  75                  80
Arg Phe Leu Glu Ser Ala Lys Asp Leu Pro Thr Ile Met Thr Ser Asn
                85                  90                  95
Ile His Ser Leu Ser Thr Met Met Lys Cys Ile Ala Leu Gly Ala Val
            100                 105                 110
Glu Phe Leu Gln Lys Pro Leu Ser Asp Asp Lys Leu Lys Asn Ile Trp
        115                 120                 125
Gln His Val Val His Lys Ala Phe Asn Thr Arg Lys Asp Val Ser Lys
    130                 135                 140
Ser Leu Glu Pro Val Lys Asp Ser Val Leu Ser Met Leu Gln Leu Gln
145                 150                 155                 160
Leu Glu Met Gly Glu Ala Asp Asp Lys Ser Ser Asn Gly Thr Glu Pro
                165                 170                 175
Pro Thr Ala Val Ala Glu Ser Asn Thr Glu Gln Ser Ser Gly Cys Asp
            180                 185                 190
Lys Tyr Pro Ala Pro Ser Ile Pro Gln Leu Lys Gln Gly Val Arg Ser
        195                 200                 205
Val Asp Asp Gly Asp Cys His Asp His Thr Ile Phe Ser Thr Asp Gln
    210                 215                 220
Asp Ser Gly Glu His Asp Ala Asp Thr Lys Ser Val Glu Thr Thr Tyr
225                 230                 235                 240
Asn Asn Ser Leu Ala Glu Asn Asn Val Gln Thr Ser Pro Thr Val Gln
                245                 250                 255
Gln Gly Asp Ile Ile Leu Lys Glu Asp Asn Val Ser Ser Pro Asp Leu
            260                 265                 270
Lys Thr Glu Thr Asp Ile Ala Thr Thr Ser Arg Ser Asn Asp Cys Pro
        275                 280                 285
Asp Asn Ser Ile Met His Ser Ala Glu Pro Ser Lys Ala Ser Gly Pro
    290                 295                 300
His Ser Ser Asn Gly Thr Lys Ser Asn Arg Lys Lys Ile Lys Val Asp
305                 310                 315                 320
Trp Thr Pro Glu Leu His Lys Lys Phe Val Gln Ala Val Glu Gln Leu
                325                 330                 335
Gly Ile Asp Gln Ala Ile Pro Ser Arg Ile Leu Asp Leu Met Lys Val
            340                 345                 350
Glu Gly Leu Thr Arg His Asn Val Ala Ser His Leu Gln Lys Tyr Arg
        355                 360                 365
Met His Arg Lys Gln Ile Leu Pro Lys Glu Val Glu Arg Arg Trp Pro
    370                 375                 380
Asn Pro Gln Pro Ile Asp Ser Val Gln Arg Ser Tyr Tyr Pro His Lys
385                 390                 395                 400
Pro Ile Met Thr Phe Pro Gln Tyr His Ser Asn His Val Ala Pro Gly
                405                 410                 415
Gly Gln Phe Tyr Pro Ala Trp Val Thr Pro Ala Ser Tyr Pro Asn Gly
            420                 425                 430
Leu Gln Val Trp Gly Ser Pro Tyr Tyr Pro Gly Trp Lys Pro Ala Glu
        435                 440                 445
Thr Trp His Trp Thr Pro Arg Pro Glu Leu His Ala Asp Thr Trp Gly
    450                 455                 460
Ser Pro Ile Met Ser Pro Ser Leu Gly Ser Tyr Pro Pro Tyr Pro Gln
465                 470                 475                 480
Asn Ala Gly Val Tyr Arg Pro His Gly Thr His Asn Arg Tyr Ser Met
                485                 490                 495
Leu Glu Lys Ser Phe Asp Leu His Pro Ala Asp Glu Val Ile Asp Lys
            500                 505                 510
```

```
Val Val Lys Glu Ala Ile Thr Lys Pro Trp Leu Pro Leu Pro Leu Gly
        515                 520                 525
Leu Lys Ala Pro Ser Thr Glu Ser Val Leu Asp Glu Leu Ser Arg Gln
    530                 535                 540
Gly Ile Ser Thr Ile Pro Ser Gln Ile Asn Asp Ser Arg Cys Arg Arg
545                 550                 555                 560

<210>  453
<211>  1656
<212>  DNA
<213>  Aquilegia formosa x Aquilegia pubescens

<400>  453
atggtttgca ctgctgatga tttattaggg tggaaggatt ttccaaaagg actaaaagtc      60
cttctactta aagaagacgg catttctgct acagaaataa aatcaaagct tgaagaaatg     120
gactatatcg tgtctacact tttcaatttg gatgatgctt taatggaaat ttcaaacaag     180
cctggaagct tccatgttgc tattgtagag gttagcacaa acaatcaaca cgaatgtttc     240
aagtttctag aaaccgtgag agatttaccc actattatga tttcaaacat ccaatgtctt     300
agcaccatga tgaaatgcat cgctcttggt gcggctgaat tccttcaaca accactgtcc     360
gaagacaaac tcaggaatat atggcagcat gttgttcata aggcctttaa tgctaaagaa     420
agtgacctca ccaaatcatt gaagcccatc aaagacactg tcgtatccat gcttcagctc     480
ccacgagaaa caaatgacac tgaggattac aatttgttgg aaacagagaa ttctgcccaa     540
tcccgggaat gtgagaatga gcaatcaaca gtcagtgata agttcccagc tccttcaacc     600
ccacagttga aacaaggatg ccgattttca gatgacgggg actgccaaca ccatactaac     660
ttcttggtag acaaagcaca tagaggcatc gaaagagaat caaaatctgt cgacactact     720
tgcaactatt tggttgctga aactacttcg gacaattcac tcgcagaagt tactgtcaca     780
gtcgatccat caacaactgc tagtgaggct atgatcaaag aggaagactc gcttgagaat     840
tctaggagtg gaagcagctt agtttctcat ccgcagagga aagagaataa aacagactcc     900
attagcaagg tcaaaaaccc aaggagagca tctcttcacc ctaatccatg tgggaaaaaa     960
tcaaatagga agatgaaggt ggactggaca ccagacctgc acaaacgttt tgtgcaagca    1020
gttgagcaac taggtgttga ccaagccatt ccttctcgga tattagactt gatgaaggta    1080
gaaggattga caaggcataa tgtcgccagc cacctgcaga agtatcggat gcaaaggaga    1140
catattgcgc caaaggatga cgatcgaaga tggcagcatc caagagactc catccaaagg    1200
gtttatccac aaaaacccat aatggcatat cctccatacc atcctaatca tggattttca    1260
acaaatcagt tctaccctgt atggggtcat cctagtaatc acctgcagaa catccagatg    1320
tggggtcatc ctggtttcca tacatggcat ccagggatgc atgcggatgc atggggccgc    1380
cctatcttgc cattatctca ggggccatat tctaactacc ctcagaatgc ctctggattt    1440
caaaatgtgt atgcatatga ggataataac aacgttccga agaactcatt cgacttcaat    1500
ccggctgaag aagtaattga caaggttgta agagaggcaa taaacaagcc atggttaccc    1560
ttaccattag gtcttaaatc tccatctaca gaaatggttc tctctgagct ccacagacaa    1620
ggcatatgca acatccctcc acacaatcaa tgctga                              1656

<210>  454
<211>  551
<212>  PRT
<213>  Aquilegia formosa x Aquilegia pubescens

<400>  454
Met Val Cys Thr Ala Asp Asp Leu Leu Gly Trp Lys Asp Phe Pro Lys
1               5                   10                  15
Gly Leu Lys Val Leu Leu Leu Lys Glu Asp Gly Ile Ser Ala Thr Glu
            20                  25                  30
Ile Lys Ser Lys Leu Glu Glu Met Asp Tyr Ile Val Ser Thr Leu Phe
        35                  40                  45
Asn Leu Asp Asp Ala Leu Met Glu Ile Ser Asn Lys Pro Gly Ser Phe
    50                  55                  60
His Val Ala Ile Val Glu Val Ser Thr Asn Asn Gln His Glu Cys Phe
65                  70                  75                  80
Lys Phe Leu Glu Thr Val Arg Asp Leu Pro Thr Ile Met Ile Ser Asn
```

```
                85                  90                  95
Ile Gln Cys Leu Ser Thr Met Met Lys Cys Ile Ala Leu Gly Ala Ala
            100                 105                 110
Glu Phe Leu Gln Gln Pro Leu Ser Glu Asp Lys Leu Arg Asn Ile Trp
        115                 120                 125
Gln His Val Val His Lys Ala Phe Asn Ala Lys Glu Ser Asp Leu Thr
    130                 135                 140
Lys Ser Leu Lys Pro Ile Lys Asp Thr Val Val Ser Met Leu Gln Leu
145                 150                 155                 160
Pro Arg Glu Thr Asn Asp Thr Glu Asp Tyr Asn Leu Leu Glu Thr Glu
                165                 170                 175
Asn Ser Ala Gln Ser Arg Glu Cys Glu Asn Glu Gln Ser Thr Val Ser
            180                 185                 190
Asp Lys Phe Pro Ala Pro Ser Thr Pro Gln Leu Lys Gln Gly Cys Arg
        195                 200                 205
Phe Ser Asp Asp Gly Asp Cys Gln His His Thr Asn Phe Leu Val Asp
    210                 215                 220
Lys Ala His Arg Gly Ile Glu Arg Glu Ser Lys Ser Val Asp Thr Thr
225                 230                 235                 240
Cys Asn Tyr Leu Val Ala Glu Thr Thr Ser Asp Asn Ser Leu Ala Glu
                245                 250                 255
Val Thr Val Thr Val Asp Pro Ser Thr Thr Ala Ser Glu Ala Met Ile
            260                 265                 270
Lys Glu Glu Asp Ser Leu Glu Asn Ser Arg Ser Gly Ser Ser Leu Val
        275                 280                 285
Ser His Pro Gln Arg Lys Glu Asn Lys Thr Asp Ser Ile Ser Lys Val
    290                 295                 300
Lys Asn Pro Arg Arg Ala Ser Leu His Pro Asn Pro Cys Gly Lys Lys
305                 310                 315                 320
Ser Asn Arg Lys Met Lys Val Asp Trp Thr Pro Asp Leu His Lys Arg
                325                 330                 335
Phe Val Gln Ala Val Glu Gln Leu Gly Val Asp Gln Ala Ile Pro Ser
            340                 345                 350
Arg Ile Leu Asp Leu Met Lys Val Glu Gly Leu Thr Arg His Asn Val
        355                 360                 365
Ala Ser His Leu Gln Lys Tyr Arg Met Gln Arg Arg His Ile Ala Pro
    370                 375                 380
Lys Asp Asp Asp Arg Arg Trp Gln His Pro Arg Asp Ser Ile Gln Arg
385                 390                 395                 400
Val Tyr Pro Gln Lys Pro Ile Met Ala Tyr Pro Pro Tyr His Pro Asn
                405                 410                 415
His Gly Phe Ser Thr Asn Gln Phe Tyr Pro Val Trp Gly His Pro Ser
            420                 425                 430
Asn His Leu Gln Asn Ile Gln Met Trp Gly His Pro Gly Phe His Thr
        435                 440                 445
Trp His Pro Gly Met His Ala Asp Ala Trp Gly Arg Pro Ile Leu Pro
    450                 455                 460
Leu Ser Gln Gly Pro Tyr Ser Asn Tyr Pro Gln Asn Ala Ser Gly Phe
465                 470                 475                 480
Gln Asn Val Tyr Ala Tyr Glu Asp Asn Asn Asn Val Pro Lys Asn Ser
                485                 490                 495
Phe Asp Phe Asn Pro Ala Glu Glu Val Ile Asp Lys Val Val Arg Glu
            500                 505                 510
Ala Ile Asn Lys Pro Trp Leu Pro Leu Pro Leu Gly Leu Lys Ser Pro
        515                 520                 525
Ser Thr Glu Met Val Leu Ser Glu Leu His Arg Gln Gly Ile Cys Asn
    530                 535                 540
Ile Pro Pro His Asn Gln Cys
545                 550
```

<210> 455
<211> 1608
<212> DNA
<213> Arabidopsis thaliana

<400> 455
atggtcatta ccgctaacga tttatcaaaa tgggaaaatt ttcctaaagg acttaaggtt 60
cttcttctcc tcaacggctg tgacagcgac ggagatggct cctcagccgc cgagactcga 120
tcagagctcg aatcaatgga ctatatcgtt actacattca ccgatgaaac tgaagcactc 180
tctgcggttg tcaagaaccc ggagagcttc cacattgcca tcgtcgaggt gaatatgagc 240
gctgagagtg agagtttcaa gtttcttgag gctgccaaag acgttcttcc tactataatg 300
atttcaaccg atcattgcat cactactaca atgaaatgca tagcgcttgg tgcagttgag 360
ttcctacaaa aaccgctctc accggagaaa ttaaagaaca tttggcagca tgttgttcat 420
aaggcattca atgatggtgg aagtaatgtt tcgatatcac ttaagccagt gaaagaatcc 480
gttgtctcga tgcttcatct tgagaccgac atgacaatcg aggagaaaga tccagcgcca 540
tcaacaccgc aattgaaaca agattcacgg ttactagacg gtgattgcca agagaacata 600
aatttctcga tggaaaatgt aaattcctcg accgagaaag ataacatgga agatcatcaa 660
gacatcggtg aatctaaatc agtcgacact acaaaccgca aattagatga cgacaaagtg 720
gttgtcaaag aagagagagg agacagtgaa aaagaagaag aaggtgaaac cggagatctc 780
ataagcgaga agacagattc agttgatatt cataagaaag aagatgagac taaaccgatt 840
aataaatcat ccgggatcaa gaacgtgtct ggtaacaaaa ctagtcgaaa gaaggtggat 900
tggacaccag agctgcacaa gaagtttgtg caagcagttg agcaactcgg cgttgatcaa 960
gcgataccct cgcggattct tgagttgatg aaagtaggca ccttaacaag acacaatgta 1020
gctagtcacc ttcagaaatt tcggcagcat aggaagaata ttcttccaaa ggatgatcat 1080
aaccatagat ggatacaatc tagagagaac catagaccaa atcaacgtaa ttataacgtt 1140
tttcaacagc aacaccgtcc cgtgatggct tatcccgttt ggggtcttcc cggtgtttat 1200
ccgccaggag cgattccacc tttgtggcca ccgccgctgc agtccattgg tcaaccacct 1260
ccgtggcatt ggaaaccacc ttatccaacg gtgagcggta atgcatgggg ttgtccggtt 1320
ggaccgcctg tgaccggatc atatattact ccttcgaata ctaccgccgg cggatttcaa 1380
tatcccaacg gagctgaaac cggcttcaaa ataatgccgg cgagtcagcc ggacgaggaa 1440
atgttagatc aagtggttaa agaagcgata agcaaaccgt ggctgccgct accgctcggg 1500
ctaaaaccgc cgtccgcgga gagcgttttg gctgagctaa cgcgtcaagg catctcagcc 1560
gtcccttctt cttcttgtct aatcaacggc tctcatcgtc tccgctga 1608

<210> 456
<211> 535
<212> PRT
<213> Arabidopsis thaliana

<400> 456
Met Val Ile Thr Ala Asn Asp Leu Ser Lys Trp Glu Asn Phe Pro Lys
1               5                   10                  15
Gly Leu Lys Val Leu Leu Leu Leu Asn Gly Cys Asp Ser Asp Gly Asp
            20                  25                  30
Gly Ser Ser Ala Ala Glu Thr Arg Ser Glu Leu Glu Ser Met Asp Tyr
        35                  40                  45
Ile Val Thr Thr Phe Thr Asp Glu Thr Glu Ala Leu Ser Ala Val Val
    50                  55                  60
Lys Asn Pro Glu Ser Phe His Ile Ala Ile Val Glu Val Asn Met Ser
65                  70                  75                  80
Ala Glu Ser Glu Ser Phe Lys Phe Leu Glu Ala Ala Lys Asp Val Leu
                85                  90                  95
Pro Thr Ile Met Ile Ser Thr Asp His Cys Ile Thr Thr Thr Met Lys
            100                 105                 110
Cys Ile Ala Leu Gly Ala Val Glu Phe Leu Gln Lys Pro Leu Ser Pro
        115                 120                 125
Glu Lys Leu Lys Asn Ile Trp Gln His Val Val His Lys Ala Phe Asn
    130                 135                 140

```
Asp Gly Gly Ser Asn Val Ser Ile Ser Leu Lys Pro Val Lys Glu Ser
145                 150                 155                 160
Val Val Ser Met Leu His Leu Glu Thr Asp Met Thr Ile Glu Glu Lys
                165                 170                 175
Asp Pro Ala Pro Ser Thr Pro Gln Leu Lys Gln Asp Ser Arg Leu Leu
            180                 185                 190
Asp Gly Asp Cys Gln Glu Asn Ile Asn Phe Ser Met Glu Asn Val Asn
        195                 200                 205
Ser Ser Thr Glu Lys Asp Asn Met Glu Asp His Gln Asp Ile Gly Glu
    210                 215                 220
Ser Lys Ser Val Asp Thr Thr Asn Arg Lys Leu Asp Asp Asp Lys Val
225                 230                 235                 240
Val Val Lys Glu Glu Arg Gly Asp Ser Glu Lys Glu Glu Glu Gly Glu
                245                 250                 255
Thr Gly Asp Leu Ile Ser Glu Lys Thr Asp Ser Val Asp Ile His Lys
            260                 265                 270
Lys Glu Asp Glu Thr Lys Pro Ile Asn Lys Ser Ser Gly Ile Lys Asn
        275                 280                 285
Val Ser Gly Asn Lys Thr Ser Arg Lys Lys Val Asp Trp Thr Pro Glu
    290                 295                 300
Leu His Lys Lys Phe Val Gln Ala Val Glu Gln Leu Gly Val Asp Gln
305                 310                 315                 320
Ala Ile Pro Ser Arg Ile Leu Glu Leu Met Lys Val Gly Thr Leu Thr
                325                 330                 335
Arg His Asn Val Ala Ser His Leu Gln Lys Phe Arg Gln His Arg Lys
            340                 345                 350
Asn Ile Leu Pro Lys Asp Asp His Asn His Arg Trp Ile Gln Ser Arg
        355                 360                 365
Glu Asn His Arg Pro Asn Gln Arg Asn Tyr Asn Val Phe Gln Gln Gln
    370                 375                 380
His Arg Pro Val Met Ala Tyr Pro Val Trp Gly Leu Pro Gly Val Tyr
385                 390                 395                 400
Pro Pro Gly Ala Ile Pro Pro Leu Trp Pro Pro Pro Leu Gln Ser Ile
                405                 410                 415
Gly Gln Pro Pro Pro Trp His Trp Lys Pro Pro Tyr Pro Thr Val Ser
            420                 425                 430
Gly Asn Ala Trp Gly Cys Pro Val Gly Pro Pro Val Thr Gly Ser Tyr
        435                 440                 445
Ile Thr Pro Ser Asn Thr Thr Ala Gly Gly Phe Gln Tyr Pro Asn Gly
    450                 455                 460
Ala Glu Thr Gly Phe Lys Ile Met Pro Ala Ser Gln Pro Asp Glu Glu
465                 470                 475                 480
Met Leu Asp Gln Val Val Lys Glu Ala Ile Ser Lys Pro Trp Leu Pro
                485                 490                 495
Leu Pro Leu Gly Leu Lys Pro Pro Ser Ala Glu Ser Val Leu Ala Glu
            500                 505                 510
Leu Thr Arg Gln Gly Ile Ser Ala Val Pro Ser Ser Ser Cys Leu Ile
        515                 520                 525
Asn Gly Ser His Arg Leu Arg
    530                 535
```

<210> 457
<211> 1674
<212> DNA
<213> Eucalypus grandis

<400> 457
```
atggtttgca ctgccagtga tctacaagaa tggaaggact tcccaaaggg actcaaggtc     60
cttcttcttg accaggactc tgattctgct tcagagataa gatcaaagct ggagttaatg    120
```

```
gactatgttg tttttacatt ctgcaatgag gacgaggctc tatcggcaat agctagcaaa    180
cctgatagct tccacatagc tatggtcgag gtgagtacaa gcagtaatgg gagtttcgag    240
ttcctcgaag ctgctagaga cttgcctacc atcatgatgt caaataacca ttgtctcagc    300
accatcatga agtgtatagc tcttggtgcg gtcgagttcc tccacaaacc gctctgcgag    360
gacaaactca ggaatatttg gcagcatgtc gcccacaagg catttaatgc aggggtcagc    420
gaggtctcgg agtctttgaa acttgtcaaa gaaacttcgg tctccatgtt gcagcaccaa    480
ttgaaaaatg aggagcagaa caatgacgag tcaagagaaa tgagaaaact gtctgttcat    540
gaaactgatc aagagatgtc cgctgcgagt gataagtatc ctgcaccatc gactccacag    600
ttgaagggag gtgcacggct cctagatgat ggggattgcc aagatcaaac gaatggaacg    660
gtcgagaagg agagtgggga gcaagatgga gagtcaaaat ttgtcgaaac aacttgtgat    720
gattcaatgt ctgagaatat ccaggaagct caaggccaga gagataggga tgttctggta    780
aaagaagagg atgattcagc caacaattcc aagtgcgaga gcatgatatc tccgctgtcc    840
gaaaacaaag ataggtccca cgatgtacat ggttgcaatg ataatcaaag caaagcatcc    900
agtcttcata attctgccag aacacgggtt aacaggaaga agatgaaggt tgactggacc    960
ccagaactgc acaagaaatt tgtgcaggcg gtggagcagc taggcgtgga ccaagccata   1020
ccttcttgca tactcgacct tatgaaagtc gaaggcctga cgaggcacaa tgtggcgagt   1080
catctccaga aataccgaat gcataggaga cacatcttgc ccaaggaaga tgaacgcaga   1140
tggccacatt ctcgcgatgg tgtcaccagg agttactatc cgcatcgacc tatcatggcc   1200
tatccgactc accatcctgg tcacccctt ccaccaggag cagcctatcc tgtgtggggg   1260
gcaccgccca gtaatcatcc agccggcatg cagatgtggg ggccgcctgg ctatccgcca   1320
tggccatctc cagaaaactg gcattggaag actgcctatc ctgcgatgca agccgacgtg   1380
tggggttgcc cagtgatgcc atcttctcat cacggtccca gctttgcctt tcctcagaat   1440
gcacccgttt gccatccccc cgacggattg gaaaacagct tcgctggaaa ttcgttcgac   1500
tatcatccgg acgaggaagt gattgacaac atcgtgaaag aggcgataag caagccgtgg   1560
ctgcccttgc cgctggggct caagccgcct tccaccgact gcgtcctcgc cgagctctcc   1620
aggcaaggca tccccagcat tcctccccat gccgaccagc tcggcccatg ctga          1674

<210>  458
<211>  557
<212>  PRT
<213>  Eucalypus grandis

<400>  458
Met Val Cys Thr Ala Ser Asp Leu Gln Glu Trp Lys Asp Phe Pro Lys
1               5                   10                  15
Gly Leu Lys Val Leu Leu Leu Asp Gln Asp Ser Asp Ser Ala Ser Glu
            20                  25                  30
Ile Arg Ser Lys Leu Glu Leu Met Asp Tyr Val Val Phe Thr Phe Cys
        35                  40                  45
Asn Glu Asp Glu Ala Leu Ser Ala Ile Ala Ser Lys Pro Asp Ser Phe
    50                  55                  60
His Ile Ala Met Val Glu Val Ser Thr Ser Ser Asn Gly Ser Phe Glu
65                  70                  75                  80
Phe Leu Glu Ala Ala Arg Asp Leu Pro Thr Ile Met Met Ser Asn Asn
                85                  90                  95
His Cys Leu Ser Thr Ile Met Lys Cys Ile Ala Leu Gly Ala Val Glu
            100                 105                 110
Phe Leu His Lys Pro Leu Cys Glu Asp Lys Leu Arg Asn Ile Trp Gln
        115                 120                 125
His Val Ala His Lys Ala Phe Asn Ala Gly Val Ser Glu Val Ser Glu
    130                 135                 140
Ser Leu Lys Leu Val Lys Glu Thr Ser Val Ser Met Leu Gln His Gln
145                 150                 155                 160
Leu Lys Asn Glu Glu Gln Asn Asn Asp Glu Ser Arg Glu Met Arg Lys
                165                 170                 175
Leu Ser Val His Glu Thr Asp Gln Glu Met Ser Ala Ala Ser Asp Lys
            180                 185                 190
Tyr Pro Ala Pro Ser Thr Pro Gln Leu Lys Gly Gly Ala Arg Leu Leu
        195                 200                 205
```

```
Asp Asp Gly Asp Cys Gln Asp Gln Thr Asn Gly Thr Val Glu Lys Glu
    210                 215                 220
Ser Gly Glu Gln Asp Gly Glu Ser Lys Phe Val Glu Thr Thr Cys Asp
225                 230                 235                 240
Asp Ser Met Ser Glu Asn Ile Gln Glu Ala Gln Gly Gln Arg Asp Arg
                245                 250                 255
Asp Val Leu Val Lys Glu Glu Asp Asp Ser Ala Asn Asn Ser Lys Cys
            260                 265                 270
Glu Ser Met Ile Ser Pro Leu Ser Glu Asn Lys Asp Arg Ser His Asp
        275                 280                 285
Val His Gly Cys Asn Asp Asn Gln Ser Lys Ala Ser Ser Leu His Asn
    290                 295                 300
Ser Ala Arg Thr Arg Val Asn Arg Lys Lys Met Lys Val Asp Trp Thr
305                 310                 315                 320
Pro Glu Leu His Lys Lys Phe Val Gln Ala Val Glu Gln Leu Gly Val
                325                 330                 335
Asp Gln Ala Ile Pro Ser Cys Ile Leu Asp Leu Met Lys Val Glu Gly
            340                 345                 350
Leu Thr Arg His Asn Val Ala Ser His Leu Gln Lys Tyr Arg Met His
        355                 360                 365
Arg Arg His Ile Leu Pro Lys Glu Asp Glu Arg Arg Trp Pro His Ser
    370                 375                 380
Arg Asp Gly Val Thr Arg Ser Tyr Tyr Pro His Arg Pro Ile Met Ala
385                 390                 395                 400
Tyr Pro Thr His His Pro Gly His Pro Leu Pro Pro Gly Ala Ala Tyr
                405                 410                 415
Pro Val Trp Gly Ala Pro Pro Ser Asn His Pro Ala Gly Met Gln Met
            420                 425                 430
Trp Gly Pro Pro Gly Tyr Pro Pro Trp Pro Ser Pro Glu Asn Trp His
        435                 440                 445
Trp Lys Thr Ala Tyr Pro Ala Met Gln Ala Asp Val Trp Gly Cys Pro
    450                 455                 460
Val Met Pro Ser Ser His His Gly Pro Ser Phe Ala Phe Pro Gln Asn
465                 470                 475                 480
Ala Pro Val Cys His Pro Pro Asp Gly Leu Glu Asn Ser Phe Ala Gly
                485                 490                 495
Asn Ser Phe Asp Tyr His Pro Asp Glu Glu Val Ile Asp Asn Ile Val
            500                 505                 510
Lys Glu Ala Ile Ser Lys Pro Trp Leu Pro Leu Pro Leu Gly Leu Lys
        515                 520                 525
Pro Pro Ser Thr Asp Cys Val Leu Ala Glu Leu Ser Arg Gln Gly Ile
    530                 535                 540
Pro Ser Ile Pro Pro His Ala Asp Gln Leu Gly Pro Cys
545                 550                 555

<210>  459
<211>  1713
<212>  DNA
<213>  Glycine max

<400>  459
atggtttgca ctgccaatga tttacaagga tggaaagatt tcccaaaggg acttagagtt      60
cttctgcttg aaggagatag tagttctgct gctgagataa gagaacaact tgaggccatg     120
gactataagg tttccacttt ctacgacgag aatgaagcct tgtcagcact ctcaagtagt     180
cctaaaggct tccatgttgc catagtggag gtgagtacaa gctgtagcct tggaggtttc     240
aaatttcttg agaatgcaaa ggacttgcct accattatga cttcaaagga ccagtgcctg     300
aacaccatga tgaagtgcat tgcgcttggt gcagttgagt ttctcagtaa accactctct     360
gaggacaagc tcaaaaatat ttggcagcat gttgttcaca aggcatttaa tgctggggca     420
aatgtcctgt ctgaatcact aaaacctgtc aaagaatctg tagaatccat gctgcagctt     480
```

```
caaacagaaa atggacaaga taaaagtagg atttcaattg atttagaaaa tgtgtcgagg    540
tttagtgata ttgaccatga gcagtctgct gtatgtgata aatatccggc tccgtcaacc    600
cctcaattaa agcaggggac aaggttactg gatgatggag attgccatga tcagactaat    660
tgctcaacag aaaaagaaag tggggaacat gatagggaat gtaaatctgt cgaaacttca    720
tgtgggaatt tgaatgctga aagttctcct cagccaaggg aacctgataa gactctgatt    780
agggaggaag atgattttgc caatgtttcc aagggtgaga gcgctgtttc actgaatcca    840
cataataaaa agtttctcag caatgccgat ggtaatacat ctccaaataa aacgggtgta    900
cttaatgatt catgtgagat taaagctaat cggaagaagg tgaaagtaga ctggacacct    960
gagctgcata aaaaatttgt aaaggctgtc gaacaactag gtattgatca agccattcct   1020
tctagaatat tggagataat gaaagtggaa ggtttgacaa ggcacaatgt ggcaagccat   1080
cttcagaaat atagaataca caagagacaa agtgcaccca gagaagaaga tcggaaatgg   1140
cataatcaag ggatgcaat gcaaaggaac tattatatgc aaagaccaat catggcatac    1200
cctccatatc attcacatca caccctttct ccagctccta tatatcctat gtggggacaa   1260
cctggcagtc aaacagccgg tgtacagatt tggggccatc ctggttatcc catatggcat   1320
cctacagaaa gttggcactg gaagccttat ccggggggtgc atgtggatgc ttggggatgc  1380
ccgttggtgc cacctcctca agctccttgt ttccctaca atcaaaatac acctggattg    1440
cacaatccta aagcagttga ctatagattc agcatgccac gaagttcctt tgagcatcat   1500
ccggcagagg aggtggtaga caaggtagtg aaggaagcaa tgagccagcc atggctacca   1560
ttgcccttag gactcaaacc tccttccatg gatagtgtgt tggctgaact ctccaaacaa   1620
ggcataccca gcatccctct tggcaacaag ggttcttcta ctcctaaacc ccgtcgagat   1680
gtacgaccga ccccgccacc gggtcccaca tag                                1713
```

<210> 460
<211> 570
<212> PRT
<213> Glycine max

<400> 460

```
Met Val Cys Thr Ala Asn Asp Leu Gln Gly Trp Lys Asp Phe Pro Lys
1               5                   10                  15
Gly Leu Arg Val Leu Leu Leu Glu Gly Asp Ser Ser Ser Ala Ala Glu
            20                  25                  30
Ile Arg Glu Gln Leu Glu Ala Met Asp Tyr Lys Val Ser Thr Phe Tyr
        35                  40                  45
Asp Glu Asn Glu Ala Leu Ser Ala Leu Ser Ser Ser Pro Lys Gly Phe
    50                  55                  60
His Val Ala Ile Val Glu Val Ser Thr Ser Cys Ser Leu Gly Gly Phe
65                  70                  75                  80
Lys Phe Leu Glu Asn Ala Lys Asp Leu Pro Thr Ile Met Thr Ser Lys
                85                  90                  95
Asp Gln Cys Leu Asn Thr Met Met Lys Cys Ile Ala Leu Gly Ala Val
            100                 105                 110
Glu Phe Leu Ser Lys Pro Leu Ser Glu Asp Lys Leu Lys Asn Ile Trp
        115                 120                 125
Gln His Val Val His Lys Ala Phe Asn Ala Gly Ala Asn Val Leu Ser
    130                 135                 140
Glu Ser Leu Lys Pro Val Lys Glu Ser Val Glu Ser Met Leu Gln Leu
145                 150                 155                 160
Gln Thr Glu Asn Gly Gln Asp Lys Ser Arg Ile Ser Ile Asp Leu Glu
                165                 170                 175
Asn Val Ser Arg Phe Ser Asp Ile Asp His Glu Gln Ser Ala Val Cys
            180                 185                 190
Asp Lys Tyr Pro Ala Pro Ser Thr Pro Gln Leu Lys Gln Gly Thr Arg
        195                 200                 205
Leu Leu Asp Asp Gly Asp Cys His Asp Gln Thr Asn Cys Ser Thr Glu
    210                 215                 220
Lys Glu Ser Gly Glu His Asp Arg Glu Cys Lys Ser Val Glu Thr Ser
225                 230                 235                 240
Cys Gly Asn Leu Asn Ala Glu Ser Ser Pro Gln Pro Arg Glu Pro Asp
```

```
                245                 250                 255
Lys Thr Leu Ile Arg Glu Glu Asp Asp Phe Ala Asn Val Ser Lys Gly
            260                 265                 270
Glu Ser Ala Val Ser Leu Asn Pro His Asn Lys Lys Phe Leu Ser Asn
        275                 280                 285
Ala Asp Gly Asn Thr Ser Pro Asn Lys Thr Gly Val Leu Asn Asp Ser
    290                 295                 300
Cys Glu Ile Lys Ala Asn Arg Lys Lys Val Lys Val Asp Trp Thr Pro
305                 310                 315                 320
Glu Leu His Lys Lys Phe Val Lys Ala Val Glu Gln Leu Gly Ile Asp
                325                 330                 335
Gln Ala Ile Pro Ser Arg Ile Leu Glu Ile Met Lys Val Glu Gly Leu
            340                 345                 350
Thr Arg His Asn Val Ala Ser His Leu Gln Lys Tyr Arg Ile His Lys
        355                 360                 365
Arg Gln Ser Ala Pro Arg Glu Glu Asp Arg Lys Trp His Asn Gln Gly
    370                 375                 380
Asp Ala Met Gln Arg Asn Tyr Tyr Met Gln Arg Pro Ile Met Ala Tyr
385                 390                 395                 400
Pro Pro Tyr His Ser His His Thr Leu Ser Pro Ala Pro Ile Tyr Pro
                405                 410                 415
Met Trp Gly Gln Pro Gly Ser Gln Thr Ala Gly Val Gln Ile Trp Gly
            420                 425                 430
His Pro Gly Tyr Pro Ile Trp His Pro Thr Glu Ser Trp His Trp Lys
        435                 440                 445
Pro Tyr Pro Gly Val His Val Asp Ala Trp Gly Cys Pro Leu Val Pro
    450                 455                 460
Pro Pro Gln Ala Pro Cys Phe Pro Tyr Asn Gln Asn Thr Pro Gly Leu
465                 470                 475                 480
His Asn Pro Lys Ala Val Asp Tyr Arg Phe Ser Met Pro Arg Ser Ser
                485                 490                 495
Phe Glu His His Pro Ala Glu Glu Val Val Asp Lys Val Val Lys Glu
            500                 505                 510
Ala Met Ser Gln Pro Trp Leu Pro Leu Pro Leu Gly Leu Lys Pro Pro
        515                 520                 525
Ser Met Asp Ser Val Leu Ala Glu Leu Ser Lys Gln Gly Ile Pro Ser
    530                 535                 540
Ile Pro Leu Gly Asn Lys Gly Ser Ser Thr Pro Lys Pro Arg Arg Asp
545                 550                 555                 560
Val Arg Pro Thr Pro Pro Pro Gly Pro Thr
                565                 570
```

<210> 461
<211> 1674
<212> DNA
<213> Lotus japonica

<400> 461

```
atggtttgca ctgccagtga tttacagcaa tggaaagatt ttccaaaagg gcttcgggtt      60
cttcttcttg aaggagatag cggttctgct accgagataa gagcaaagct tgaggccatg     120
gactatattg tttttacttt ctacaatgag aatgaggcct tgtcagcaat ctctagtgat     180
cctgaaggct tccatgttgc cgtggtggag gtgtctacaa gcagcaacca gggtggtttc     240
aaattccttg agaatgtgaa ggacttgcct accattatga cttccaacag tcaatgcctg     300
aatacaatga tgaagtgcat cgcgcttggc gcagttgaat tcctcactaa accactctct     360
gaggataagc tcaaaaatat ttggcagcat gtagttcaca aggcattcaa tgctgggaca     420
aatgctccgt ctgaatcact aagacctgtc aaagaatctg tagagtccat tttacagctc     480
caaactgata atggacagca tgaaagtaat atttcaatag atatagagaa cgtgtccagg     540
ttaggtgata atgaccatga gcagtcggtt ggaagtgata aatatccggc tccttcaacc     600
ccgcaattaa aacaaggggc aaggttacta gatgatggtg attgccatga gcaaactaat     660
```

```
tgctcaacag aaaaagaaag tggggaacat gatgaggaat ctaaatctgt cgaaatttct     720
tgtgagaatt taaacacgga aagttcttct cagcttagta aacctgaaaa gactctgatt     780
aaggaggaag aggcttttgc cgatggttcc aagggtgaga ttgctgtttc acagaatgaa     840
gataatagaa agtttctcgg cagtgctgag ggtaatgaat ctccaaataa aacaggtgtt     900
cttagtgatt cctgtgagaa gaaagctaat cggaagaaga tgaaagttga ctggacacct     960
gaattgcaca agaaatttgt aaaggcagtg gaacagttgg gtattgatca tgccatcccg    1020
tctcgaatat tggagatcat gaaagtggag ggcttgacga ggcacaatgt tgcaagccat    1080
cttcagaaat atagaataca caagaaacaa atcatgcctg ggaagacga ccgaaaatgt     1140
cagaatccaa gagatccaat gcaaaggagc tattgtttgc aaagaccaat catggcctac    1200
ccaccatatc attctaacca cacccttcct ccggctccta tgtatccaat gtggggacaa    1260
cctggcagtc aaacagctgg tgtgcagatt tggggtaatc atggttatcc cctttggcat    1320
cctacagaaa gttggcactg gaagccatat cccggaatgc atgtggatgc atgggggtgt    1380
ccagtgttgc catctcctca agcttctcct tttccctaca ctcacaatgt ggctgggtgg    1440
cacaatgcta aagcaatgga ttatagattc agcatgccac agagttctgt agagaattat    1500
ccggcggagg aggtggtgga caaggttgtg aaggaggcaa tgagcaagcc atggctcccc    1560
ttgcccttag gcctcaaacc tccttccatg gatactgtct tgtctgagct ctccagccaa    1620
ggcatatccg gcatcatccc ttttagcagc accaagggct ctataccccg ctga          1674
```

```
<210>  462
<211>  557
<212>  PRT
<213>  Lotus japonica

<400>  462
Met Val Cys Thr Ala Ser Asp Leu Gln Gln Trp Lys Asp Phe Pro Lys
1               5                   10                  15
Gly Leu Arg Val Leu Leu Leu Glu Gly Asp Ser Gly Ser Ala Thr Glu
            20                  25                  30
Ile Arg Ala Lys Leu Glu Ala Met Asp Tyr Ile Val Phe Thr Phe Tyr
        35                  40                  45
Asn Glu Asn Glu Ala Leu Ser Ala Ile Ser Ser Asp Pro Glu Gly Phe
    50                  55                  60
His Val Ala Val Val Glu Val Ser Thr Ser Ser Asn Gln Gly Gly Phe
65                  70                  75                  80
Lys Phe Leu Glu Asn Val Lys Asp Leu Pro Thr Ile Met Thr Ser Asn
                85                  90                  95
Ser Gln Cys Leu Asn Thr Met Met Lys Cys Ile Ala Leu Gly Ala Val
            100                 105                 110
Glu Phe Leu Thr Lys Pro Leu Ser Glu Asp Lys Leu Lys Asn Ile Trp
        115                 120                 125
Gln His Val Val His Lys Ala Phe Asn Ala Gly Thr Asn Ala Pro Ser
    130                 135                 140
Glu Ser Leu Arg Pro Val Lys Glu Ser Val Glu Ser Ile Leu Gln Leu
145                 150                 155                 160
Gln Thr Asp Asn Gly Gln His Glu Ser Asn Ile Ser Ile Asp Ile Glu
                165                 170                 175
Asn Val Ser Arg Leu Gly Asp Asn Asp His Glu Gln Ser Val Gly Ser
            180                 185                 190
Asp Lys Tyr Pro Ala Pro Ser Thr Pro Gln Leu Lys Gln Gly Ala Arg
        195                 200                 205
Leu Leu Asp Asp Gly Asp Cys His Glu Gln Thr Asn Cys Ser Thr Glu
    210                 215                 220
Lys Glu Ser Gly Glu His Asp Glu Glu Ser Lys Ser Val Glu Ile Ser
225                 230                 235                 240
Cys Glu Asn Leu Asn Thr Glu Ser Ser Ser Gln Leu Ser Lys Pro Glu
                245                 250                 255
Lys Thr Leu Ile Lys Glu Glu Glu Ala Phe Ala Asp Gly Ser Lys Gly
            260                 265                 270
Glu Ile Ala Val Ser Gln Asn Glu Asp Asn Arg Lys Phe Leu Gly Ser
```

```
        275                 280                 285
Ala Glu Gly Asn Glu Ser Pro Asn Lys Thr Gly Val Leu Ser Asp Ser
    290                 295                 300
Cys Glu Lys Lys Ala Asn Arg Lys Lys Met Lys Val Asp Trp Thr Pro
305                 310                 315                 320
Glu Leu His Lys Lys Phe Val Lys Ala Val Glu Gln Leu Gly Ile Asp
                325                 330                 335
His Ala Ile Pro Ser Arg Ile Leu Glu Ile Met Lys Val Glu Gly Leu
            340                 345                 350
Thr Arg His Asn Val Ala Ser His Leu Gln Lys Tyr Arg Ile His Lys
        355                 360                 365
Lys Gln Ile Met Pro Gly Glu Asp Asp Arg Lys Cys Gln Asn Pro Arg
    370                 375                 380
Asp Pro Met Gln Arg Ser Tyr Cys Leu Gln Arg Pro Ile Met Ala Tyr
385                 390                 395                 400
Pro Pro Tyr His Ser Asn His Thr Leu Pro Pro Ala Pro Met Tyr Pro
                405                 410                 415
Met Trp Gly Gln Pro Gly Ser Gln Thr Ala Gly Val Gln Ile Trp Gly
            420                 425                 430
Asn His Gly Tyr Pro Leu Trp His Pro Thr Glu Ser Trp His Trp Lys
        435                 440                 445
Pro Tyr Pro Gly Met His Val Asp Ala Trp Gly Cys Pro Val Leu Pro
    450                 455                 460
Ser Pro Gln Ala Ser Pro Phe Pro Tyr Thr His Asn Val Ala Gly Trp
465                 470                 475                 480
His Asn Ala Lys Ala Met Asp Tyr Arg Phe Ser Met Pro Gln Ser Ser
                485                 490                 495
Val Glu Asn Tyr Pro Ala Glu Glu Val Val Asp Lys Val Val Lys Glu
            500                 505                 510
Ala Met Ser Lys Pro Trp Leu Pro Leu Pro Leu Gly Leu Lys Pro Pro
        515                 520                 525
Ser Met Asp Thr Val Leu Ser Glu Leu Ser Ser Gln Gly Ile Ser Gly
    530                 535                 540
Ile Ile Pro Phe Ser Ser Thr Lys Gly Ser Ile Pro Arg
545                 550                 555
```

```
<210>  463
<211>  1680
<212>  DNA
<213>  Lycopersicon esculentum

<400>  463
atggtttgca ctgagaatga attattggag tggaaagatt tcccaaaagg acttaaagtt      60
cttcttcttg atacagactc caattttgcc tcacagatga gatcaaggct tcaacaaatg     120
gactatatag tttacacatt ctgcaatgag aatgaggctc tgtctgcaat atcaagcaag     180
tcggaggtgt tccatgttgc cattgtagag gtaagtgctg gcaacagcga tggaggattc     240
aaatttctcg aaagtgctaa agatctacca actataatgg tttcagatat tcactctatt     300
aacatcatga tgaagtgtat agcacttggt gcagttgagt tccttcagaa accattatca     360
gatgataaac tgagaaatat atggcagcat gtagttcaca aggcatttca ttctggagga     420
aagagtgtct ctgagtcact aaagccggtt aaagaatctc ttttgtccat tctagagctc     480
caaccagtaa aacacgaagc agataatgag aatactaatg aagcagaacc cttgatatca     540
gtggtggaaa accaaaaagc atcatcatcc tgctgcgata aatatccagc tccttcaacc     600
ccacaacaga aacaaggagt gaggtcagtg gatgatgttg attaccaaga tcatactatc     660
ttgtcaaatg aacaagatag tgggatgcat gaaggggaca caaaatctgt cgaaactact     720
tcttgcgatt ctgttgctga gactactgtc ttggcagatt cttccgagcg tctaggagag     780
gctatcacaa aggaggaaca ctattctgct gctgatcaac atatggagga tcctattgct     840
acttgttcac caagtaatga caatggcagt acttgttctg ctgacccgaa caaagcttct     900
ggtctccata gttcaagtgg gacaaaagct aataagaaga aaatgaaggt agactggaca     960
cctgaactac acaagaaatt tgttaaagca gtagagaaaa ttggtataga tcaagccatt    1020
```

```
ccttctcgaa tactggagct gatgaaagta gaaggcctga cgagacataa tatagctagc   1080
catctccaga aattcaggat gcagcggaag caaatcttgc caaaggaaga cgaaagaagg   1140
tggcctcgtc ctcaacctag agatcctgta cagaggacct attatccaca taaacctgtc   1200
atggcctttc caacccatca ttccaaccat gcaaccacag ctggtcaatt ctatcctgcc   1260
tggataccac caggaggtta cccgaatggt gcacatatgt ggaattcgcc ttattatcat   1320
ggatggcaac cgcctgagac gtggcactgg aatcctcaac cagggctgta tgctgatgta   1380
tggggttgcc ccgttacgcc tccatcttta ggatcatgta caccataccc tcagaatgca   1440
tctggatttc acagggctga gggaatgctg aatggatata gcataataca gaaatcagtc   1500
gatcttcacc cggcagagga ggtgatagat aaagtggtga aggaggcaat aaacaagcca   1560
tggttgcctc taccttggg  cctaaaacct ccttcaacgg agagtgttct cgacgcgctt   1620
tctaaacaag gcatccccgc cgtccctcca cgccaccacc gctctcatcg cccacactga   1680

<210>  464
<211>  559
<212>  PRT
<213>  Lycopersicon esculentum

<400>  464
Met Val Cys Thr Glu Asn Glu Leu Leu Glu Trp Lys Asp Phe Pro Lys
1               5                   10                  15
Gly Leu Lys Val Leu Leu Leu Asp Thr Asp Ser Asn Phe Ala Ser Gln
            20                  25                  30
Met Arg Ser Arg Leu Gln Gln Met Asp Tyr Ile Val Tyr Thr Phe Cys
        35                  40                  45
Asn Glu Asn Glu Ala Leu Ser Ala Ile Ser Ser Lys Ser Glu Val Phe
    50                  55                  60
His Val Ala Ile Val Glu Val Ser Ala Gly Asn Ser Asp Gly Gly Phe
65                  70                  75                  80
Lys Phe Leu Glu Ser Ala Lys Asp Leu Pro Thr Ile Met Val Ser Asp
                85                  90                  95
Ile His Ser Ile Asn Ile Met Met Lys Cys Ile Ala Leu Gly Ala Val
            100                 105                 110
Glu Phe Leu Gln Lys Pro Leu Ser Asp Asp Lys Leu Arg Asn Ile Trp
        115                 120                 125
Gln His Val Val His Lys Ala Phe His Ser Gly Gly Lys Ser Val Ser
    130                 135                 140
Glu Ser Leu Lys Pro Val Lys Glu Ser Leu Leu Ser Ile Leu Glu Leu
145                 150                 155                 160
Gln Pro Val Lys His Glu Ala Asp Asn Glu Asn Thr Asn Glu Ala Glu
                165                 170                 175
Pro Leu Ile Ser Val Val Glu Asn Gln Lys Ala Ser Ser Ser Cys Cys
            180                 185                 190
Asp Lys Tyr Pro Ala Pro Ser Thr Pro Gln Gln Lys Gln Gly Val Arg
        195                 200                 205
Ser Val Asp Asp Val Asp Tyr Gln Asp His Thr Ile Leu Ser Asn Glu
    210                 215                 220
Gln Asp Ser Gly Met His Glu Gly Asp Thr Lys Ser Val Glu Thr Thr
225                 230                 235                 240
Ser Cys Asp Ser Val Ala Glu Thr Thr Val Leu Ala Asp Ser Ser Glu
                245                 250                 255
Arg Leu Gly Glu Ala Ile Thr Lys Glu Glu His Tyr Ser Ala Ala Asp
            260                 265                 270
Gln His Met Glu Asp Pro Ile Ala Thr Cys Ser Pro Ser Asn Asp Asn
        275                 280                 285
Gly Ser Thr Cys Ser Ala Asp Pro Asn Lys Ala Ser Gly Leu His Ser
    290                 295                 300
Ser Ser Gly Thr Lys Ala Asn Lys Lys Lys Met Lys Val Asp Trp Thr
305                 310                 315                 320
Pro Glu Leu His Lys Lys Phe Val Lys Ala Val Glu Lys Ile Gly Ile
```

```
                325                 330                 335
Asp Gln Ala Ile Pro Ser Arg Ile Leu Glu Leu Met Lys Val Glu Gly
            340                 345                 350
Leu Thr Arg His Asn Ile Ala Ser His Leu Gln Lys Phe Arg Met Gln
        355                 360                 365
Arg Lys Gln Ile Leu Pro Lys Glu Asp Glu Arg Arg Trp Pro Arg Pro
    370                 375                 380
Gln Pro Arg Asp Pro Val Gln Arg Thr Tyr Tyr Pro His Lys Pro Val
385                 390                 395                 400
Met Ala Phe Pro Thr His His Ser Asn His Ala Thr Thr Ala Gly Gln
                405                 410                 415
Phe Tyr Pro Ala Trp Ile Pro Pro Gly Gly Tyr Pro Asn Gly Ala His
            420                 425                 430
Met Trp Asn Ser Pro Tyr Tyr His Gly Trp Gln Pro Pro Glu Thr Trp
        435                 440                 445
His Trp Asn Pro Gln Pro Gly Leu Tyr Ala Asp Val Trp Gly Cys Pro
    450                 455                 460
Val Thr Pro Pro Ser Leu Gly Ser Cys Thr Pro Tyr Pro Gln Asn Ala
465                 470                 475                 480
Ser Gly Phe His Arg Ala Glu Gly Met Leu Asn Gly Tyr Ser Ile Ile
                485                 490                 495
Gln Lys Ser Val Asp Leu His Pro Ala Glu Glu Val Ile Asp Lys Val
            500                 505                 510
Val Lys Glu Ala Ile Asn Lys Pro Trp Leu Pro Leu Pro Leu Gly Leu
        515                 520                 525
Lys Pro Pro Ser Thr Glu Ser Val Leu Asp Ala Leu Ser Lys Gln Gly
    530                 535                 540
Ile Pro Ala Val Pro Pro Arg His His Arg Ser His Arg Pro His
545                 550                 555
```

<210> 465
<211> 1647
<212> DNA
<213> Medicago truncatula

<400> 465

```
atggtttgca ctgccaatga tttacaagga tggaaagatt tcccaaaagg acttagggtt     60
cttctccttg aaggagacaa caattctgct tctgaaataa gaacaaagct tgaatctatg    120
gactataatg tttctacgtt ctacaatgaa aatgaagcct tgtcagcaat ctcaagtagt    180
cccaaatgct tccatgttgc catagtggag gtgagtataa gctgccctga tggaggtttc    240
aaatttctcg agaatgcaaa agacttgcca accattatga cgtcgaatag tcagtgcata    300
aacacaatga tgaagtgcat cgcgcttggt gcagttgagt tcctcactaa accactttct    360
gaggacaagc tgaaaaatat ttggcagcat gtggttcaca aggcattcaa tgccgaggca    420
agcgctctgt ctgaatcact taaaccggtc aaagaatctg tagagtccat gttgcatctc    480
caaacagaca acacactaca tgaaagtaca atatcaatag atttagataa ggtgtccaag    540
tttagtgata atgaacatga gcattctgcg gcaagcgata aatatccggc tccttccacc    600
ccgcaattaa aacaaggggc aaggttagtg gatgatggtg attgccacga gcagactaat    660
tgcttaatag aaaaagaaag tggggaacat gacagtggtg aatgtaaatt tgtcgacact    720
tcatgtgaga atttgaatgc tgaaagttct cctcaaccta caaaacatct gattaaggag    780
gaagaagatt ttgccaaggg ttccaagggt gagggtggtg cttcattgaa tccacttaat    840
aaaaattttc tcggcgacgc tggtggcaat acatctctga ataaaacaag ggtttttaac    900
gatccgtgtg agaataaagc taatcggaag aagatgaaag tagactggac agctgagctg    960
cacaaaaaat ttgtgaaggc agtggaacaa ctaggtattg atcaagccat tccttctcgt   1020
atattggagt taatgaaagt cgacggtttg acaaggcata atgtggcaag ccatcttcag   1080
atttttgttg aacagaaata tagaatgcac aagagacaga tcatccacac ggacgaagat   1140
cgaaaatggc caaatcgaag agatcctatg caaaggaact attgtatgca aagaccgatt   1200
atggcttatc caccatatca ttctaatcac acctttcctc cagctcctgc atatcccatg   1260
tggggccaac atggaagtca aacagctggt gtaccgattt ggagtcctcc gggttatcct   1320
ctatggcagc ctacagaaag ttggcattgg aagccatatc cgccaggggt gcatgtggat   1380
```

```
gcatggggta acccaatgtt gcgcccacct caaactcact gtattccata cactcaaaat   1440
atggctggga tgcacaatgc taaagcagtg gattatagtt atccggcaga ggaagtggtg   1500
gacaaggctg tgaaggaggc aataaacaag ccatggctac ctttacctat aggcctcaaa   1560
cctccttcaa tggatagtgt gttggatgaa ctttccaaac aaggcattcc ttttagcaag   1620
aagagcaagg gctctagacc atgctga                                       1647

<210>  466
<211>  548
<212>  PRT
<213>  Medicago truncatula

<400>  466
Met Val Cys Thr Ala Asn Asp Leu Gln Gly Trp Lys Asp Phe Pro Lys
1               5                   10                  15
Gly Leu Arg Val Leu Leu Leu Glu Gly Asp Asn Asn Ser Ala Ser Glu
            20                  25                  30
Ile Arg Thr Lys Leu Glu Ser Met Asp Tyr Asn Val Ser Thr Phe Tyr
        35                  40                  45
Asn Glu Asn Glu Ala Leu Ser Ala Ile Ser Ser Ser Pro Lys Cys Phe
    50                  55                  60
His Val Ala Ile Val Glu Val Ser Ile Ser Cys Pro Asp Gly Gly Phe
65                  70                  75                  80
Lys Phe Leu Glu Asn Ala Lys Asp Leu Pro Thr Ile Met Thr Ser Asn
                85                  90                  95
Ser Gln Cys Ile Asn Thr Met Met Lys Cys Ile Ala Leu Gly Ala Val
            100                 105                 110
Glu Phe Leu Thr Lys Pro Leu Ser Glu Asp Lys Leu Lys Asn Ile Trp
        115                 120                 125
Gln His Val Val His Lys Ala Phe Asn Ala Glu Ala Ser Ala Leu Ser
    130                 135                 140
Glu Ser Leu Lys Pro Val Lys Glu Ser Val Glu Ser Met Leu His Leu
145                 150                 155                 160
Gln Thr Asp Asn Thr Leu His Glu Ser Thr Ile Ser Ile Asp Leu Asp
                165                 170                 175
Lys Val Ser Lys Phe Ser Asp Asn Glu His Glu His Ser Ala Ala Ser
            180                 185                 190
Asp Lys Tyr Pro Ala Pro Ser Thr Pro Gln Leu Lys Gln Gly Ala Arg
        195                 200                 205
Leu Val Asp Asp Gly Asp Cys His Glu Gln Thr Asn Cys Leu Ile Glu
    210                 215                 220
Lys Glu Ser Gly Glu His Asp Ser Gly Glu Cys Lys Phe Val Asp Thr
225                 230                 235                 240
Ser Cys Glu Asn Leu Asn Ala Glu Ser Ser Pro Gln Pro Thr Lys His
                245                 250                 255
Leu Ile Lys Glu Glu Glu Asp Phe Ala Lys Gly Ser Lys Gly Glu Gly
            260                 265                 270
Gly Ala Ser Leu Asn Pro Leu Asn Lys Asn Phe Leu Gly Asp Ala Gly
        275                 280                 285
Gly Asn Thr Ser Leu Asn Lys Thr Arg Val Phe Asn Asp Pro Cys Glu
    290                 295                 300
Asn Lys Ala Asn Arg Lys Lys Met Lys Val Asp Trp Thr Ala Glu Leu
305                 310                 315                 320
His Lys Lys Phe Val Lys Ala Val Glu Gln Leu Gly Ile Asp Gln Ala
                325                 330                 335
Ile Pro Ser Arg Ile Leu Glu Leu Met Lys Val Asp Gly Leu Thr Arg
            340                 345                 350
His Asn Val Ala Ser His Leu Gln Ile Phe Val Glu Gln Lys Tyr Arg
        355                 360                 365
Met His Lys Arg Gln Ile Ile His Thr Asp Glu Asp Arg Lys Trp Pro
```

```
    370                 375                 380
Asn Arg Arg Asp Pro Met Gln Arg Asn Tyr Cys Met Gln Arg Pro Ile
385                 390                 395                 400
Met Ala Tyr Pro Pro Tyr His Ser Asn His Thr Phe Pro Pro Ala Pro
                405                 410                 415
Ala Tyr Pro Met Trp Gly Gln His Gly Ser Gln Thr Ala Gly Val Pro
            420                 425                 430
Ile Trp Ser Pro Pro Gly Tyr Pro Leu Trp Gln Pro Thr Glu Ser Trp
        435                 440                 445
His Trp Lys Pro Tyr Pro Pro Gly Val His Val Asp Ala Trp Gly Asn
    450                 455                 460
Pro Met Leu Arg Pro Pro Gln Thr His Cys Ile Pro Tyr Thr Gln Asn
465                 470                 475                 480
Met Ala Gly Met His Asn Ala Lys Ala Val Asp Tyr Ser Tyr Pro Ala
                485                 490                 495
Glu Glu Val Val Asp Lys Ala Val Lys Glu Ala Ile Asn Lys Pro Trp
            500                 505                 510
Leu Pro Leu Pro Ile Gly Leu Lys Pro Pro Ser Met Asp Ser Val Leu
        515                 520                 525
Asp Glu Leu Ser Lys Gln Gly Ile Pro Phe Ser Lys Lys Ser Lys Gly
    530                 535                 540
Ser Arg Pro Cys
545

<210>  467
<211>  1671
<212>  DNA
<213>  Populus tremuloides

<400>  467
atggtttgca ccactaatga tttatcagca tggaaggatt ttccaaaggg cctcagagtc      60
cttctccttg atgaggacag catgtctgct gccgagataa aatcgaaact ggaagcaatg     120
gactatattg tttatacatt ctgcaatgag actgaagcgc tctctgcaat ttcaaatgaa     180
cctggaagct tccatgttgc tatagtggag gtgagcatga gcaatagcag caggagtttc     240
aagtttctag aaacttcgaa ggacttgcca accattatga cttcaagcat tgattgcctg     300
aacaccatga tgaagtgcat agcgcttggt gcagttgaat tccttcggaa accactctct     360
gaagataaac tgaggaatat atggcagcac gtcgttcata aggcatttaa tgcagggggg     420
agtgttcagt ccaaatcact gaagcctgtc aaagattctg tagtttccat gctggagctc     480
aaagatggac tcgaagaaaa tgagaataag aacatggaga aaacagaaaa tgtgtctcag     540
gctcaggaaa gtaatgagca gcagtcacca gctagtgaca agtatccggc tccttcaacc     600
cctcaattga aacaaggaga gagactgctg gatgatggag attgccaaga ccatattaac     660
tgtttaatag aaaaggagag tgtggagcaa gagggagatt ctaaatccgt tgaaactact     720
tgtgccatgt ctgaagaaac tctacaggca ggggatcctc agagctttac agagactgtg     780
atcaaagagg aggatgactc aactgatggt gtcaagagtg agaataacat gtgtcccaat     840
tcacaaaata aagacactct caaccattca aatggttgtg ctgaaaaagc ttctagcctt     900
cataattccc atgggactag agctaataga aagaagatga agtttcaggt agactggaca     960
ccagagcttc acagaaagtt tgtgcaggca gtggaaaaac taggtgtaga tcaagcgatt    1020
ccttcaagaa tattagaggt aatgaaagtg gaaggcttga cgaggcataa tgtagcaagt    1080
catctgcagc agaaatatcg aatgcacagg agacatattt tgcccaagga agatgaacgg    1140
cagtggacgc agcatagaga tcaagtacaa aggagttact atccacataa acctatcatg    1200
gcgtatcctc catatcattc taaccatgct ctcccacctg gtccggtcta tcctatgtgg    1260
ggagcaacag gtagtcacac agctggggtg catatgtggg gcccccccgg ctactcccca    1320
tggcccccga cagaaagttg tcattggaag ccttatccag ggatgcatgc agatgcgtgg    1380
ggttgccctg tgatgccacc acataatcct tattctacca ttcctcatta tgcatctggg    1440
tttcagagtg ccagcatggt caataacagc tgtggtatgc cacaaaaact gtttgaccta    1500
cagccggccg aggaggttat cgacaaggta gtgaaagagg caatcaacaa gccatggcta    1560
cctttgccct tgggcctcaa gcccccgtct gccgatagcg tcctagcaga gctctccagg    1620
caaggcgtct catcaattcc tccccgcatt aatggctcta attccttcta a             1671
```

<210> 468
<211> 556
<212> PRT
<213> Populus tremuloides

<400> 468
```
Met Val Cys Thr Thr Asn Asp Leu Ser Ala Trp Lys Asp Phe Pro Lys
1               5                   10                  15
Gly Leu Arg Val Leu Leu Leu Asp Glu Asp Ser Met Ser Ala Ala Glu
            20                  25                  30
Ile Lys Ser Lys Leu Glu Ala Met Asp Tyr Ile Val Tyr Thr Phe Cys
        35                  40                  45
Asn Glu Thr Glu Ala Leu Ser Ala Ile Ser Asn Glu Pro Gly Ser Phe
    50                  55                  60
His Val Ala Ile Val Glu Val Ser Met Ser Asn Ser Ser Arg Ser Phe
65                  70                  75                  80
Lys Phe Leu Glu Thr Ser Lys Asp Leu Pro Thr Ile Met Thr Ser Ser
                85                  90                  95
Ile Asp Cys Leu Asn Thr Met Met Lys Cys Ile Ala Leu Gly Ala Val
            100                 105                 110
Glu Phe Leu Arg Lys Pro Leu Ser Glu Asp Lys Leu Arg Asn Ile Trp
        115                 120                 125
Gln His Val Val His Lys Ala Phe Asn Ala Gly Gly Ser Val Gln Ser
    130                 135                 140
Lys Ser Leu Lys Pro Val Lys Asp Ser Val Val Ser Met Leu Glu Leu
145                 150                 155                 160
Lys Asp Gly Leu Glu Glu Asn Glu Asn Lys Asn Met Glu Lys Thr Glu
                165                 170                 175
Asn Val Ser Gln Ala Gln Glu Ser Asn Glu Gln Gln Ser Pro Ala Ser
            180                 185                 190
Asp Lys Tyr Pro Ala Pro Ser Thr Pro Gln Leu Lys Gln Gly Glu Arg
        195                 200                 205
Leu Leu Asp Asp Gly Asp Cys Gln Asp His Ile Asn Cys Leu Ile Glu
    210                 215                 220
Lys Glu Ser Val Glu Gln Glu Gly Asp Ser Lys Ser Val Glu Thr Thr
225                 230                 235                 240
Cys Ala Met Ser Glu Glu Thr Leu Gln Ala Gly Asp Pro Gln Ser Phe
                245                 250                 255
Thr Glu Thr Val Ile Lys Glu Glu Asp Asp Ser Thr Asp Gly Val Lys
            260                 265                 270
Ser Glu Asn Asn Met Cys Pro Asn Ser Gln Asn Lys Asp Thr Leu Asn
        275                 280                 285
His Ser Asn Gly Cys Ala Glu Lys Ala Ser Ser Leu His Asn Ser His
    290                 295                 300
Gly Thr Arg Ala Asn Arg Lys Lys Met Lys Phe Gln Val Asp Trp Thr
305                 310                 315                 320
Pro Glu Leu His Arg Lys Phe Val Gln Ala Val Glu Lys Leu Gly Val
                325                 330                 335
Asp Gln Ala Ile Pro Ser Arg Ile Leu Glu Val Met Lys Val Glu Gly
            340                 345                 350
Leu Thr Arg His Asn Val Ala Ser His Leu Gln Gln Lys Tyr Arg Met
        355                 360                 365
His Arg Arg His Ile Leu Pro Lys Glu Asp Glu Arg Gln Trp Thr Gln
    370                 375                 380
His Arg Asp Gln Val Gln Arg Ser Tyr Tyr Pro His Lys Pro Ile Met
385                 390                 395                 400
Ala Tyr Pro Pro Tyr His Ser Asn His Ala Leu Pro Pro Gly Pro Val
                405                 410                 415
Tyr Pro Met Trp Gly Ala Thr Gly Ser His Thr Ala Gly Val His Met
```

```
            420                 425                 430
Trp Gly Pro Pro Gly Tyr Ser Pro Trp Pro Pro Thr Glu Ser Cys His
        435                 440                 445
Trp Lys Pro Tyr Pro Gly Met His Ala Asp Ala Trp Gly Cys Pro Val
    450                 455                 460
Met Pro Pro His Asn Pro Tyr Ser Thr Ile Pro His Tyr Ala Ser Gly
465                 470                 475                 480
Phe Gln Ser Ala Ser Met Val Asn Asn Ser Cys Gly Met Pro Gln Lys
                485                 490                 495
Leu Phe Asp Leu Gln Pro Ala Glu Glu Val Ile Asp Lys Val Val Lys
            500                 505                 510
Glu Ala Ile Asn Lys Pro Trp Leu Pro Leu Pro Leu Gly Leu Lys Pro
        515                 520                 525
Pro Ser Ala Asp Ser Val Leu Ala Glu Leu Ser Arg Gln Gly Val Ser
    530                 535                 540
Ser Ile Pro Pro Arg Ile Asn Gly Ser Asn Ser Phe
545                 550                 555

<210>  469
<211>  1650
<212>  DNA
<213>  Populus tremuloides

<400>  469
atggtttgta ccactaatga tttatcagca tggaaggatt ttccaaaggg cctcagtgtc      60
cttctccttg atgaggacaa cagttctgct gctgagataa aatcgaaact ggaagcactg     120
gactatatcg tttatacatt ctgcaatgag aatgaagcgc tcttagcaat ttcaaatgaa     180
cctggaagct tccatgttgc tatagtggag gtgagtacga gcaatagcaa tgggagtttc     240
aagtttctag aaactgccaa ggacttgcct accattatga cttcaaacat tcattgcctg     300
aacaccatga tgaagtgcat agcgcttggt gcagttgagt tccttcggaa accactctct     360
gaagataaac taaggaatat atggcagcat gtggttcata aggcatttaa tgcaggtggg     420
agtgttcaat ccgaatcact aaagcctgtc aaagattcta tagtttctat gcttgagctc     480
aaagtggaac ttgaagaaaa cgaggatgag aacatggaga aaacagaaaa tgcgtctttg     540
gctcaggaaa gcaatgagca gcagtcacca gctagtgata agcatccagc tccttcaact     600
tggcaattta aacaagtagg gcggctgctg gatgatggag attgccaaga ccatattaac     660
tgctcagtag aaaaggatag tggggagcaa gaggggggaat ctaaatctgt cgaaacaact     720
tgtgccatgt ctcaagaaac tctaaaagca gggcatcctc catgctttat agagactgtg     780
atcaaagaga caggtgactt aactgatggt gccaagagtg agaataacat acatcccaat     840
ccacaaaata aagacagtct caaccattct aatgatgttg cttctgatct tcatacttcc     900
aatgggacta gagctaatcg aaagaagatg aaggtagact ggacaccaga gcttcacaaa     960
aaatttgtgc aagtggtgga aaaactaggt gtagatcaag caattccttc acgagtatta    1020
gagctaatga aagtggaaag cttgactagg cataatgtag caagtcatct gcagaaatat    1080
cgaatgcgca ggaggcctat tttgcccaag gaagatgacc ggcgatggcc acaccataga    1140
gagcaagtac aaaggagcta ttatccatat aaacctatca tggcataccc tccatatcat    1200
tctaaccatg atcttccaac caacccggtc tatcctatgt ggggagcaac aggtagtcac    1260
acagctagca tgcatacgtg gggcacaccc agctatctcc catggccccc aacagaaatt    1320
tggcacagga agccttatct agggatgcat gcggatgcat ggggttgccc tgtgatgcca    1380
ccattgcata gtcctttttc ttcttttcct caaaatgcat ctgggtttca aagtgccagc    1440
atggtcgata acagctgtgg catgccacaa aaacctttttg acctacaacc ggccgaggag    1500
gtgatcaaca aggtagtgaa agaggtaatc aacaagcctt ggctaccgtt gcccataggc    1560
ctcaagcccc cttctacaga tagcgtccta gcggagctct ccaggcaagg catctcatca    1620
attcctcccc tttgctctaa ttccttctaa                                     1650

<210>  470
<211>  549
<212>  PRT
<213>  Populus tremuloides

<400>  470
```

```
Met Val Cys Thr Thr Asn Asp Leu Ser Ala Trp Lys Asp Phe Pro Lys
1               5                   10                  15
Gly Leu Ser Val Leu Leu Leu Asp Glu Asp Asn Ser Ser Ala Ala Glu
            20                  25                  30
Ile Lys Ser Lys Leu Glu Ala Leu Asp Tyr Ile Val Tyr Thr Phe Cys
        35                  40                  45
Asn Glu Asn Glu Ala Leu Leu Ala Ile Ser Asn Glu Pro Gly Ser Phe
    50                  55                  60
His Val Ala Ile Val Glu Val Ser Thr Ser Asn Ser Asn Gly Ser Phe
65                  70                  75                  80
Lys Phe Leu Glu Thr Ala Lys Asp Leu Pro Thr Ile Met Thr Ser Asn
                85                  90                  95
Ile His Cys Leu Asn Thr Met Met Lys Cys Ile Ala Leu Gly Ala Val
            100                 105                 110
Glu Phe Leu Arg Lys Pro Leu Ser Glu Asp Lys Leu Arg Asn Ile Trp
        115                 120                 125
Gln His Val Val His Lys Ala Phe Asn Ala Gly Gly Ser Val Gln Ser
    130                 135                 140
Glu Ser Leu Lys Pro Val Lys Asp Ser Ile Val Ser Met Leu Glu Leu
145                 150                 155                 160
Lys Val Glu Leu Glu Glu Asn Glu Asp Glu Asn Met Glu Lys Thr Glu
                165                 170                 175
Asn Ala Ser Leu Ala Gln Glu Ser Asn Glu Gln Gln Ser Pro Ala Ser
            180                 185                 190
Asp Lys His Pro Ala Pro Ser Thr Trp Gln Phe Lys Gln Val Gly Arg
        195                 200                 205
Leu Leu Asp Asp Gly Asp Cys Gln Asp His Ile Asn Cys Ser Val Glu
    210                 215                 220
Lys Asp Ser Gly Glu Gln Glu Gly Glu Ser Lys Ser Val Glu Thr Thr
225                 230                 235                 240
Cys Ala Met Ser Gln Glu Thr Leu Lys Ala Gly His Pro Pro Cys Phe
                245                 250                 255
Ile Glu Thr Val Ile Lys Glu Thr Gly Asp Leu Thr Asp Gly Ala Lys
            260                 265                 270
Ser Glu Asn Asn Ile His Pro Asn Pro Gln Asn Lys Asp Ser Leu Asn
        275                 280                 285
His Ser Asn Asp Val Ala Ser Asp Leu His Thr Ser Asn Gly Thr Arg
    290                 295                 300
Ala Asn Arg Lys Lys Met Lys Val Asp Trp Thr Pro Glu Leu His Lys
305                 310                 315                 320
Lys Phe Val Gln Val Val Glu Lys Leu Gly Val Asp Gln Ala Ile Pro
                325                 330                 335
Ser Arg Val Leu Glu Leu Met Lys Val Glu Ser Leu Thr Arg His Asn
            340                 345                 350
Val Ala Ser His Leu Gln Lys Tyr Arg Met Arg Arg Arg Pro Ile Leu
        355                 360                 365
Pro Lys Glu Asp Asp Arg Arg Trp Pro His His Arg Glu Gln Val Gln
    370                 375                 380
Arg Ser Tyr Tyr Pro Tyr Lys Pro Ile Met Ala Tyr Pro Pro Tyr His
385                 390                 395                 400
Ser Asn His Asp Leu Pro Thr Asn Pro Val Tyr Pro Met Trp Gly Ala
                405                 410                 415
Thr Gly Ser His Thr Ala Ser Met His Thr Trp Gly Thr Pro Ser Tyr
            420                 425                 430
Leu Pro Trp Pro Pro Thr Glu Ile Trp His Arg Lys Pro Tyr Leu Gly
        435                 440                 445
Met His Ala Asp Ala Trp Gly Cys Pro Val Met Pro Pro Leu His Ser
    450                 455                 460
Pro Phe Ser Ser Phe Pro Gln Asn Ala Ser Gly Phe Gln Ser Ala Ser
```

```
465                 470                 475                 480
Met Val Asp Asn Ser Cys Gly Met Pro Gln Lys Pro Phe Asp Leu Gln
                485                 490                 495
Pro Ala Glu Glu Val Ile Asn Lys Val Val Lys Glu Val Ile Asn Lys
            500                 505                 510
Pro Trp Leu Pro Leu Pro Ile Gly Leu Lys Pro Pro Ser Thr Asp Ser
        515                 520                 525
Val Leu Ala Glu Leu Ser Arg Gln Gly Ile Ser Ser Ile Pro Pro Leu
    530                 535                 540
Cys Ser Asn Ser Phe
545
```

<210>  471
<211>  1674
<212>  DNA
<213>  Vitis vinifera

<400>  471

```
atggtttgca ctgctaatga tttacaggag tggaaggatt ttcctaaggg acttagggtc     60
cttctccttg atgatgacac tacttctgct gctgagataa gatcaaagct tgaggaaatg    120
gactatattg tttctacatt ctgcaatgag aatgaagctt tgtcagcaat ttcaagcaaa    180
cctgagagct tccatgttgc tattgtagag gtcagtacag gcaacaatgg gagtttcaag    240
tttctcgaaa ctgctaagga tttacccacc attatgattt caagcatcca ttgcctgagc    300
accatgatga agtgcatagc actcggtgca gttgagttcc ttcgaaaacc actgtccgag    360
gacaagctca ggaatatttg gcagcatgta gttcataagg catttaatgc aggagggagt    420
gtccttcctg aatcactaaa gcccgtcaaa gaatctgtgg cttccatgct gcagctccaa    480
atggaaaatg aagaaccaag gaatgaaagc tcagcagaaa cactgaatgt gtccaatgtc    540
catgaaaatg accatatgca gtcagctgga actgataaat acccggcacc ttcaacccca    600
caattaaaac aaggagggag atcattggat gatggggatt gcctagatca aactaactgc    660
tcaactgaga aagagagtgg ggagcaggat ggggaatcta aatctgtcga aactacatgt    720
ggcacttcag ttgctgaagt tactgctcag gtgtctccac ctcaaggact gggcgagagc    780
gttatcaaag aggaagatga ttcagctgat ggctgtaaga gtgaaagtaa tatgtctcct    840
catccacaga ataaagacag tctcagtgag tttggtggtg atgctagaaa cccsagaaag    900
gcatctggtg ttcatagtcc ttgtgggaca agagccaatc ggaagaaaat gaaggtagac    960
tggacgcctg aactgcacaa gaaatttgtg caggcagtgg agcagctggg tgtggatcaa   1020
gccattccat ctcgaatatt agagctgatg aaagtggaag gcttgacgag gcataatgta   1080
gcaagtcatc tccagaaata cagaatgcat cggagacaca ttctgccaaa ggaagatgat   1140
agaagatggc ctcatcagag agacccaatg cagaggaatt attatccaca gaaacctgtc   1200
atggccttcc ctccatatca ttccagtcac accctcccag ctgctcagct ctaccctgta   1260
tggggccaac ccagcagcca ccctgctcag atgtggagca cacctggcta tcatacatgg   1320
cagcccgcag aaagttggat ttggaagcct tatccaggga tgaatgccga tgcatggggc   1380
tgccctgtga tgacaccaac acatactcca tgctcttcct ttcctcaaaa tccatctggg   1440
tttgatcata ataatggaag tggaatatac aacactggca taccacagag ccctattgac   1500
ctttatccgg cagaggaggt aattgacagg gtagtgaagg aggcaataag caagccgtgg   1560
atgccactgc cattgggcct gaaaccgccg gccaccgaga gcgtgctggc ggagctctcc   1620
cgccagggca tctccaccat cccacctcac atcaacaccc cccgtcccta ctga         1674
```

<210>  472
<211>  557
<212>  PRT
<213>  Vitis vinifera

<400>  472

```
Met Val Cys Thr Ala Asn Asp Leu Gln Glu Trp Lys Asp Phe Pro Lys
1               5                   10                  15
Gly Leu Arg Val Leu Leu Leu Asp Asp Asp Thr Thr Ser Ala Ala Glu
            20                  25                  30
Ile Arg Ser Lys Leu Glu Glu Met Asp Tyr Ile Val Ser Thr Phe Cys
        35                  40                  45
```

```
Asn Glu Asn Glu Ala Leu Ser Ala Ile Ser Ser Lys Pro Glu Ser Phe
    50                  55                  60
His Val Ala Ile Val Glu Val Ser Thr Gly Asn Asn Gly Ser Phe Lys
65                  70                  75                  80
Phe Leu Glu Thr Ala Lys Asp Leu Pro Thr Ile Met Ile Ser Ser Ile
                85                  90                  95
His Cys Leu Ser Thr Met Met Lys Cys Ile Ala Leu Gly Ala Val Glu
            100                 105                 110
Phe Leu Arg Lys Pro Leu Ser Glu Asp Lys Leu Arg Asn Ile Trp Gln
        115                 120                 125
His Val Val His Lys Ala Phe Asn Ala Gly Gly Ser Val Leu Pro Glu
    130                 135                 140
Ser Leu Lys Pro Val Lys Glu Ser Val Ala Ser Met Leu Gln Leu Gln
145                 150                 155                 160
Met Glu Asn Glu Glu Pro Arg Asn Glu Ser Ser Ala Glu Thr Leu Asn
                165                 170                 175
Val Ser Asn Val His Glu Asn Asp His Met Gln Ser Ala Gly Thr Asp
            180                 185                 190
Lys Tyr Pro Ala Pro Ser Thr Pro Gln Leu Lys Gln Gly Gly Arg Ser
        195                 200                 205
Leu Asp Asp Gly Asp Cys Leu Asp Gln Thr Asn Cys Ser Thr Glu Lys
    210                 215                 220
Glu Ser Gly Glu Gln Asp Gly Glu Ser Lys Ser Val Glu Thr Thr Cys
225                 230                 235                 240
Gly Thr Ser Val Ala Glu Val Thr Ala Gln Val Ser Pro Pro Gln Gly
                245                 250                 255
Leu Gly Glu Ser Val Ile Lys Glu Glu Asp Asp Ser Ala Asp Gly Cys
            260                 265                 270
Lys Ser Glu Ser Asn Met Ser Pro His Pro Gln Asn Lys Asp Ser Leu
        275                 280                 285
Ser Glu Phe Gly Gly Asp Ala Arg Asn Pro Arg Lys Ala Ser Gly Val
    290                 295                 300
His Ser Pro Cys Gly Thr Arg Ala Asn Arg Lys Lys Met Lys Val Asp
305                 310                 315                 320
Trp Thr Pro Glu Leu His Lys Lys Phe Val Gln Ala Val Glu Gln Leu
                325                 330                 335
Gly Val Asp Gln Ala Ile Pro Ser Arg Ile Leu Glu Leu Met Lys Val
            340                 345                 350
Glu Gly Leu Thr Arg His Asn Val Ala Ser His Leu Gln Lys Tyr Arg
        355                 360                 365
Met His Arg Arg His Ile Leu Pro Lys Glu Asp Asp Arg Arg Trp Pro
    370                 375                 380
His Gln Arg Asp Pro Met Gln Arg Asn Tyr Tyr Pro Gln Lys Pro Val
385                 390                 395                 400
Met Ala Phe Pro Pro Tyr His Ser Ser His Thr Leu Pro Ala Ala Gln
                405                 410                 415
Leu Tyr Pro Val Trp Gly Gln Pro Ser Ser His Pro Ala Gln Met Trp
            420                 425                 430
Ser Thr Pro Gly Tyr His Thr Trp Gln Pro Ala Glu Ser Trp Ile Trp
        435                 440                 445
Lys Pro Tyr Pro Gly Met Asn Ala Asp Ala Trp Gly Cys Pro Val Met
    450                 455                 460
Thr Pro Thr His Thr Pro Cys Ser Ser Phe Pro Gln Asn Pro Ser Gly
465                 470                 475                 480
Phe Asp His Asn Asn Gly Ser Gly Ile Tyr Asn Thr Gly Ile Pro Gln
                485                 490                 495
Ser Pro Ile Asp Leu Tyr Pro Ala Glu Glu Val Ile Asp Arg Val Val
            500                 505                 510
Lys Glu Ala Ile Ser Lys Pro Trp Met Pro Leu Pro Leu Gly Leu Lys
```

```
        515                 520                 525
Pro Pro Ala Thr Glu Ser Val Leu Ala Glu Leu Ser Arg Gln Gly Ile
    530                 535                 540
Ser Thr Ile Pro Pro His Ile Asn Thr Pro Arg Pro Tyr
545                 550                 555
```

<210> 473
<211> 1863
<212> DNA
<213> Oryza sativa

<400> 473

```
atggcggaga acaacggcgc ggtgcctccc ggctgcaagc tcccggcggg aggattcttc      60
gggaggctgc acgtgctcgt cgtcgacgac gacgccgcct acctcgagga gctcaagctt     120
atgctgctcc tcgccggcta cgcagtgacg ggcaagacga cggcggagga agcgctgaaa     180
gaggtggacc agaatccgga ggattacttc cacatcgtca tgaccgacgt ccacatgtca     240
ggaatggacg gcttcgatct cctccacagg atcaacggcc gggtccctgt catcatgttc     300
tccgagggcg aagatgtggt gatggtgatg aggacggtga tgaacggcgc atgcgactac     360
atggtgaagc cgatgacatc agaggcgatc aagttcatat ggaagcacgt cctgcgctgg     420
aggctcagcg ccctccccgc gaatgcctca tcatccctgc agccaagcga tcacctggcg     480
gcggctctag cggctgtggc gccgccgccg ccgccggcgg tgcagctgcc ggccgcaccg     540
gcgcaggctg ggaacaggga tggcgaagct catgaggagg cagagttgtc gacacagcct     600
cctgcgctgg tgccatctgg cgttcaagag gcggctgcag cggtatggag cagccgtggt     660
gatggacaag aggcgccgcc gccggcggtg gctgcggcgg cgaaggcccc ctcgaagaag     720
aggggcgcgt cagaagtgtc ggacaggggc tccaacaatt tggaggcgac gacgggggagg     780
aagaaggtga ggacaaggtt cacatggacg acggtgtcgc acacatcgtt tgtcagggca     840
tacgagcagc tgaaggacca agaaggtcca aagaagataa agcaactgat ggaacttgat     900
ggtatatttg taactaaaac tcaagtctca agccatctac agaaatacag aagctggtta     960
gaaaatgaaa gaaaaaagga agaagcaaca agcagctcac catgcaaccc actgagttac    1020
accaactgcc tagatagagg ttatagtact tggaagcaaa gttcagtcat aactgaagga    1080
cagcaaagtt caagtttttc tggacggcct attcattcca tggccacatc aaatggttgt    1140
ctaactacga ctgataccca agcaggcaac tatgtgggag tgggagctaa agaaatcgag    1200
aatttcattt ccagccatca gagatctcta ggaactgcaa ttggtcaaga atcaactatt    1260
gaacaggcaa gccttcatag tgagattaca tcagtcagtc gtgatgctca tgaaaatgga    1320
aacagccagg ctagaggaag tgcgatgagc aatggtactt caggtactcg tggtgtgctt    1380
gttacaaatg agaacctcct gcatgttgtt agtgcatcgt tgccatcaaa catgggacag    1440
ccaacgcaac caagtcaaag cttctgcacc aatgaattgg cagctaacta cagtattatt    1500
agtgatcaaa accctgggac tagccatcca actagctcta gtgctatcaa taatcaaaac    1560
tctaagaccc aagaaatgtc tgtttcacaa acagtagaac ttgggtgtgg caacgatgtg    1620
atgttggatt ggccagaatt agttggactg gaagatcagc tggataatga tgtgctaatg    1680
aacagcttct ttgatggaga cttactccag cagggtgtag ttactgctat agatgggacg    1740
caagagatgc ttgcttttga ttctaccggt gacttgggct cagtgccgcc aagaggcctt    1800
aacaacgaaa ttgccagcca tgagaatacc aatggaaaga acggcgcatc tagtggacct    1860
tga                                                                  1863
```

<210> 474
<211> 620
<212> PRT
<213> Oryza sativa

<400> 474

```
Met Ala Glu Asn Asn Gly Ala Val Pro Pro Gly Cys Lys Leu Pro Ala
1               5                   10                  15
Gly Gly Phe Phe Gly Arg Leu His Val Leu Val Val Asp Asp Asp Ala
            20                  25                  30
Ala Tyr Leu Glu Glu Leu Lys Leu Met Leu Leu Leu Ala Gly Tyr Ala
        35                  40                  45
Val Thr Gly Lys Thr Thr Ala Glu Glu Ala Leu Lys Glu Val Asp Gln
    50                  55                  60
```

```
Asn Pro Glu Asp Tyr Phe His Ile Val Met Thr Asp Val His Met Ser
65                  70                  75                  80
Gly Met Asp Gly Phe Asp Leu Leu His Arg Ile Asn Gly Arg Val Pro
                85                  90                  95
Val Ile Met Phe Ser Glu Gly Glu Asp Val Val Met Val Met Arg Thr
            100                 105                 110
Val Met Asn Gly Ala Cys Asp Tyr Met Val Lys Pro Met Thr Ser Glu
        115                 120                 125
Ala Ile Lys Phe Ile Trp Lys His Val Leu Arg Trp Arg Leu Ser Ala
    130                 135                 140
Leu Pro Ala Asn Ala Ser Ser Ser Leu Gln Pro Ser Asp His Leu Ala
145                 150                 155                 160
Ala Ala Leu Ala Ala Val Ala Pro Pro Pro Pro Pro Ala Val Gln Leu
                165                 170                 175
Pro Ala Ala Pro Ala Gln Ala Gly Asn Arg Asp Gly Glu Ala His Glu
            180                 185                 190
Glu Ala Glu Leu Ser Thr Gln Pro Pro Ala Leu Val Pro Ser Gly Val
        195                 200                 205
Gln Glu Ala Ala Ala Ala Val Trp Ser Ser Arg Gly Asp Gly Gln Glu
    210                 215                 220
Ala Pro Pro Pro Ala Val Ala Ala Ala Ala Lys Ala Pro Ser Lys Lys
225                 230                 235                 240
Arg Gly Ala Ser Glu Val Ser Asp Arg Gly Ser Asn Asn Leu Glu Ala
                245                 250                 255
Thr Thr Gly Arg Lys Lys Val Arg Thr Arg Phe Thr Trp Thr Thr Val
            260                 265                 270
Ser His Thr Ser Phe Val Arg Ala Tyr Glu Gln Leu Lys Asp Gln Glu
        275                 280                 285
Gly Pro Lys Lys Ile Lys Gln Leu Met Glu Leu Asp Gly Ile Phe Val
    290                 295                 300
Thr Lys Thr Gln Val Ser Ser His Leu Gln Lys Tyr Arg Ser Trp Leu
305                 310                 315                 320
Glu Asn Glu Arg Lys Lys Glu Glu Ala Thr Ser Ser Ser Pro Cys Asn
                325                 330                 335
Pro Leu Ser Tyr Thr Asn Cys Leu Asp Arg Gly Tyr Ser Thr Trp Lys
            340                 345                 350
Gln Ser Ser Val Ile Thr Glu Gly Gln Gln Ser Ser Ser Phe Ser Gly
        355                 360                 365
Arg Pro Ile His Ser Met Ala Thr Ser Asn Gly Cys Leu Thr Thr Thr
    370                 375                 380
Asp Thr Gln Ala Gly Asn Tyr Val Gly Val Gly Ala Lys Glu Ile Glu
385                 390                 395                 400
Asn Phe Ile Ser Ser His Gln Arg Ser Leu Gly Thr Ala Ile Gly Gln
                405                 410                 415
Glu Ser Thr Ile Glu Gln Ala Ser Leu His Ser Glu Ile Thr Ser Val
            420                 425                 430
Ser Arg Asp Ala His Glu Asn Gly Asn Ser Gln Ala Arg Gly Ser Ala
        435                 440                 445
Met Ser Asn Gly Thr Ser Gly Thr Arg Gly Val Leu Val Thr Asn Glu
    450                 455                 460
Asn Leu Leu His Val Val Ser Ala Ser Leu Pro Ser Asn Met Gly Gln
465                 470                 475                 480
Pro Thr Gln Pro Ser Gln Ser Phe Cys Thr Asn Glu Leu Ala Ala Asn
                485                 490                 495
Tyr Ser Ile Ile Ser Asp Gln Asn Pro Gly Thr Ser His Pro Thr Ser
            500                 505                 510
Ser Ser Ala Ile Asn Asn Gln Asn Ser Lys Thr Gln Glu Met Ser Val
        515                 520                 525
Ser Gln Thr Val Glu Leu Gly Cys Gly Asn Asp Val Met Leu Asp Trp
```

```
    530                 535                 540
Pro Glu Leu Val Gly Leu Glu Asp Gln Leu Asp Asn Asp Val Leu Met
545                 550                 555                 560
Asn Ser Phe Phe Asp Gly Asp Leu Leu Gln Gln Gly Val Val Thr Ala
                565                 570                 575
Ile Asp Gly Thr Gln Glu Met Leu Ala Phe Asp Ser Thr Gly Asp Leu
            580                 585                 590
Gly Ser Val Pro Pro Arg Gly Leu Asn Asn Glu Ile Ala Ser His Glu
        595                 600                 605
Asn Thr Asn Gly Lys Asn Gly Ala Ser Ser Gly Pro
    610                 615                 620

<210>  475
<211>  112
<212>  PRT
<213>  Artificial sequence

<220>
<223>  pseudo response receiver domain from <SEQIDNO2, PF00072
       (integrated in IPR001789)

<400>  475
Leu Lys Val Leu Leu Leu Asp Glu Asp Ser Asn Ser Ala Ala Glu Met
1               5                   10                  15
Lys Ser Arg Leu Glu Lys Met Asp Tyr Ile Val Tyr Ser Phe Cys Asn
            20                  25                  30
Glu Ser Glu Ala Leu Thr Ala Ile Ser Ser Lys Ser Glu Gly Phe His
        35                  40                  45
Val Ala Ile Val Glu Val Ser Ala Gly Asn Ser Asp Gly Val Leu Arg
    50                  55                  60
Phe Leu Glu Ser Ala Lys Asp Leu Pro Thr Ile Met Thr Ser Asn Ile
65                  70                  75                  80
His Ser Leu Ser Thr Met Met Lys Cys Ile Ala Leu Gly Ala Val Glu
                85                  90                  95
Phe Leu Gln Lys Pro Leu Ser Asp Asp Lys Leu Lys Asn Ile Trp Gln
            100                 105                 110

<210>  476
<211>  56
<212>  PRT
<213>  Artificial sequence

<220>
<223>  myb-like DNA binding region, SHAQKYF class(IPR006447)

<400>  476
Lys Ile Lys Val Asp Trp Thr Pro Glu Leu His Lys Lys Phe Val Gln
1               5                   10                  15
Ala Val Glu Gln Leu Gly Ile Asp Gln Ala Ile Pro Ser Arg Ile Leu
            20                  25                  30
Asp Leu Met Lys Val Glu Gly Leu Thr Arg His Asn Val Ala Ser His
        35                  40                  45
Leu Gln Lys Tyr Arg Met His Arg
    50                  55

<210>  477
<211>  42
<212>  PRT
<213>  Artificial sequence
```

<220>
<223> C-terminal conserved domain

<400> 477
Pro Ala Asp Glu Val Ile Asp Lys Val Val Lys Glu Ala Ile Thr Lys
1 5 10 15
Pro Trp Leu Pro Leu Pro Leu Gly Leu Lys Ala Pro Ser Thr Glu Ser
20 25 30
Val Leu Asp Glu Leu Ser Arg Gln Gly Ile
35 40

<210> 478
<211> 2194
<212> DNA
<213> Oryza sativa

<400> 478
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aatttttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

<210> 479
<211> 56
<212> DNA

<213> Artificial sequence

<220>
<223> primer: prm10843

<400> 479
ggggacaagt ttgtacaaaa aagcaggctt aaacaatgat ttgcattgag aatgaa 56

<210> 480
<211> 50
<212> DNA
<213> Artificial sequence

<220>
<223> primer: prm10844

<400> 480
ggggaccact ttgtacaaga aagctgggta catgtcatct catctccgac 50

## Description

The present invention relates generally to the field of molecular biology and concerns a method for enhancing various economically important yield-related traits in plants. More specifically, the present invention concerns a method for enhancing yield-related traits in plants by modulating expression in a plant of a nucleic acid encoding a bHLH9 (basic-Helix-Loop-Helix group 9) polypeptide. The present invention also concerns plants having modulated expression of a nucleic acid encoding a bHLH9 polypeptide, which plants have enhanced yield-related traits relative to control plants. The invention also provides hitherto unknown BHLH9-encoding nucleic acids and constructs comprising the same, useful in performing the methods of the invention.

Furthermore, the present invention relates generally to the field of molecular biology and concerns a method for improving various plant growth characteristics by modulating expression in a plant of a nucleic acid encoding an IMB1 (Imbibition-inducible 1) polypeptide. The present invention also concerns plants having modulated expression of a nucleic acid encoding an IMB1 polypeptide, which plants have improved growth characteristics relative to corresponding wild type plants or other control plants. The invention also provides constructs useful in the methods of the invention.

Yet furthermore, the present invention relates generally to the field of molecular biology and concerns a method for improving various plant growth characteristics by modulating expression in a plant of a nucleic acid encoding a PCD-like (Pterin-4-alpha-carbinolamine dehydratase-like). The present invention also concerns plants having modulated expression of a nucleic acid encoding a PCD-like, which plants have improved growth characteristics relative to corresponding wild type plants or other control plants. The invention also provides constructs useful in the methods of the invention.

Furthermore the present invention relates generally to the field of molecular biology and concerns a method for increasing various plant yield-related traits by increasing expression in a plant of a nucleic acid sequence encoding a pseudo response regulator type 2 (PRR2) polypeptide. The present invention also concerns plants having increased expression of a nucleic acid sequence encoding a PRR2 polypeptide, which plants have increased yield-related traits relative to control plants. The invention additionally relates to nucleic acid sequences, nucleic acid constructs, vectors and plants containing said nucleic acid sequences.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

Another trait of importance is that of improved abiotic stress tolerance. Abiotic stress is a primary cause of crop loss worldwide, reducing average yields for most major crop plants by more than 50% (Wang et al. (2003) Planta 218: 1-14). Abiotic stresses may be caused by drought, salinity, extremes of temperature, chemical toxicity, excess or deficiency of nutrients (macroelements and/or microelements), radiation and oxidative stress. The ability to increase plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increase yield-related traits (seed yield and/or biomass) in plants may be through modification of the inherent growth mechanisms of a plant, such as the cell cycle or various signalling pathways involved in plant growth or in defense mechanisms.

Concerning bHLH9 polypeptides, it has now been found that various yield-related traits may be improved in plants by modulating expression in a plant of a nucleic acid encoding a bHLH9 (basic-Helix-Loop-Helix group 9) polypeptide in a plant.

Concerning IMB1 polypeptides, it has now been found that various growth characteristics may be improved in plants by modulating expression in a plant of a nucleic acid encoding an IMB1 (Imbibition-inducible 1) polypeptide in a plant.

Concerning PCD-like polypeptides, it has now been found that various growth characteristics may be improved in plants by modulating expression in a plant of a nucleic acid encoding a PCD-like (Pterin-4-alpha-carbinolamine dehydratase-like) in a plant.

Concerning PRR2 polypeptides, it has now been found that various yield-related traits may be increased in plants relative to control plants, by increasing expression in a plant of a nucleic acid sequence encoding a pseudo response regulator type 2 (PRR2) polypeptide. The increased yield-related traits comprise one or more of: increased aboveground biomass, increased early vigor, earlier flowering, increased root biomass, increased plant height, increased seed yield per plant, increased number of filled seeds, increased total number of seeds, increased number of primary panicles, increased number of flowers per panicles, increased harvest index (HI), and increased Thousand Kernel Weight (TKW).

**Background**

1. basic-Helix-Loop-Helix group 9 (bHLH9)

Transcription factors are usually defined as proteins that show sequence-specific DNA binding and that are capable of activating and/or repressing transcription. The basic/helix-loop-helix (bHLH) proteins are a superfamily of transcription factors that bind as dimers to specific DNA target sites and that have been well characterized in non-plant eukaryotes as important regulatory components in diverse biological processes. The distinguishing characteristic of the bHLH family is a bipartite domain consisting of approximately 60 amino acids. This bipartite domain comprises a DNA-binding basic region, which binds to a consensus hexanucleotide E-box and two $\alpha$-helices separated by a variable loop region. The two $\alpha$-helices promote dimerization, allowing the formation of homo- and heterodimers between different family members. While the bHLH domain is evolutionarily conserved, sequence similarity outside of the domain between members in different clades is lower. The Arabidopsis genome codes for at least 1533 transcriptional regulators, which account for approximately 5.9% of its estimated total number of genes (Riechmann et al., 2000 (Science Vol. 290, 2105-2109)). Bailey et al., 2003 (The Plant Cell, Vol. 15, 2497-2501) report the total number of detected bHLH genes in Arabidopsis thaliana to be 162, making bHLH genes one of the largest families of transcription factors in Arabidopsis; the rice genome reportedly contains 131 bHLH genes (Buck and Atchley, 2003 (J. Mol. Evol. 56:742-750)). Heim et al., 2003 (Mol. Biol. Evol. 20(5):735-747) identified 12 subfamilies of bHLH genes in Arabidopsis thaliana based on structural similarities. Within each of these main groups, there are conserved amino acid sequence motifs outside the DNA binding domain. It has been suggested that structurally related genes belonging to the same group are likely to share similar functions (Heim et al. 2003).

Animal and plant bHLH proteins bind to the palindromic hexanucleotide sequence CANNTG (E-box motif), wherein N represents any nucleotide and/or the G-box motif: CACGTG. Patterns of amino acids were found at three positions within the basic region of the bHLH motif (the 5-9-13 configuration) that defined the bHLH subgroups (clades), with an H-E-R configuration as the ancestral sequence. The most frequent 5-9-13 amino acid configuration of bHLH proteins falling within group IX (bHLH9) of Heim et al. 2003 is R-E-R.

In plants, the biological function of a number of bHLH proteins from different groups has been reported. For example the proteins of group III, Arabidopsis gene AtbHLH042 (TT8: Transparant Testa 8) and its homologous from Zea mays, gene Intensifier I and Antohcyanin I from petunia hybrida, PhAN1, play a role in the synthesis of precursors of plant pigments such as flavonoids and anthocyanins (Heim et al. 2003) and three members of group XII have been linked to pathways regulating plant hormonal response (Friedrichsen et al 2002, Genetics 162: 1445-1456). Other functions played by bHLH proteins include phytochrome signaling, globulin expression, fruit dehiscence, carpel and epidermal development (Buck and Atchley, 2003 J Mol Evol. 2003; 56(6):742-50.). However no biological role has been assigned yet to any of the bHLH proteins of group IX of Heim et al. 2003.

2. Imbibition-inducible 1 (IMB1)

The bromodomain, a conserved domain present in many chromatin-associated proteins and in most nuclear acetyltransferases, is known to function as an acetyl-lysine binding domain. The bromodomain is composed of four left-handed helix bundles ($\alpha_z$, $\alpha_A$, $\alpha_B$, and $\alpha_c$), with a long loop (ZA) connecting helices Z and A oriented against the small loop (BC) connecting helices B and C. These loops are organized to form an accessible hydrophobic pocket in which the interaction with the acetylated lysine residue occurs (for a review, see Zheng and Zhou, FEBS Letters 513, 124-128, 2002; Loyola and Almouzni, Trends Cell Biol. 14, 279-281, 2004). NMR studies have shown that the acetyl-lysine binding site is localised between the ZA and BC loops in this hydrophobic pocket. The bromodomain thus allows protein-protein interactions that can be regulated by acetylation of lysine.

In yeast, bromodomain-proteins are known to play a role in chromatin remodelling. Other activities in which bromodomain-proteins are suggested to play a role include transcriptional activiation, memory of the transcriptionally activated chromosomal regions and anti-silencing. Little is known however about role of bromodomain protein on the phenotype of plants: Chua et al. (Genes & Development 19, 2245-2254, 2005) report that overexpression of the bromodomain-protein GTE6 gives elongated juvenile leaves in Arabidopsis. Lightner et al. (WO 2005/058021) described that overexpression of the bromodomain-protein At3g52280 resulted in increased seed oil content. IMB1 of *Arabidopsis thaliana* reportedly is induced during seed imbibition and is thought to regulate ABA- and phytochrome A-mediated responses of germination (Duque and Chua, Plant Journal 35, 787-799, 2003).

3. PCD-LIKE

Pterin-4a-carbinolamine dehydratases (PCDs) acts as an enzyme in intermediary metabolism playing a relevant role in for example the synthesis of amino acids. PCDs recycle oxidized pterin cofactors generated by aromatic amino acid hydroxylases (AAHs). PCD having a systematic nomenclature of (6R)-6-(L-erythro-1,2-dihydroxypropyl)-5,6,7,8-tetrahydro-4a-hydroxypterin hydro-lyase [(6R)-6-(L-erythro-1,2-dihydroxypropyl)-7,8-dihydro-6H-pterin-forming] catalyses the dehydration of 4a-hydroxytetrahydrobiopterins according to the reaction: (6R)-6-(L-erythro-1,2-dihydroxypropyl)-5,6,7,8-tetrahydro-4a-hydroxypterin = (6R)-6-(L-erythro-1,2-dihydroxypropyl)-7,8-dihydro-6H-pterin + H2O.

PCDs are known to be encoded in a wide variety of organism including animals, plants, and microbes. Pterin 4 alpha carbinolamine dehydratase is also known as PCD-like (dimerisation cofactor of hepatocyte nuclear factor 1-alpha, HNF-1). PCD-like is a bifunctional protein that function as 4a-hydroxytetrahydrobiopterin dehydratases and as coactivators of HNF1alpha-dependent transcription (Cronk et al. Protein Sci. 1996 Oct;5(10):1963-72).

Pterin-4a-carbinolamine dehydratase activity of PCD proteins from plants and diverse microorganisms has been determined in vivo and in vitro which allowed the definition of a catalytic motif, [EDKHQN]-x(3)-H-[HN]-[PCS]-x (5,6)-[YWFH]-x(9)-[HW]-x(8,15)-D, that can be used as a signature for PCD activity (Naponelli et al. 2008. Plant Physiol. 2008; 146(4): 1515-1527).

Phylogenomic and functional analysis of plant PCDS, reportedly established that plants have two types of PCDs proteins. Type 1 is characterized by the presence of the catalytic motif as defined above, it has PCD activity, and is located in mitochondria. Type 2 is unique to plants, is localized in plastids, and apparently arose from type 1 early in plant evolution. Type 2 proteins have a characteristic subterminal domain that includes the motif [GE]-[DN]-[FL]-G-A-R-D-P-x(3)-E-x(4)-F-G-[DE]K and typically lack the catalytic motif. Reportedly, overexpressing the type 2 gene (*At5g51110*) in Arabidopsis thaliana had no apparent phenotypic effect, and its suppression by RNA interference resulted in a small reduction in leaf pigment content and a larger reduction in chloroplast number per mesophyll cell (Naponelli et al. 2008).

4. Pseudo Response Regulator type 2 (PRR2)

Common signal transduction mechanisms, generally referred to as histidine-to-aspartate (His-to-Asp) phosphorelay systems, are involved in a wide variety of cellular responses to environmental and developmental stimuli many prokaryotes, fungi, slime molds, and plants. A His-to-Asp phosphorelay system consists of two or more common signal transducers: (1) a membrane-localized sensor exhibiting His-kinase activity (HK, that senses the output); (2) a response regulator (RR) transcription factor usually containing a phospho-accepting Asp in its receiver domain (that mediates the output); and (3) and optionally a His-containing phosphotransmitter (HP) (Hwang et al. (2002) Plant Phys 129: 500-515, and references therein).

The completion of the *Arabidopsis* genome sequence has revealed 54 genes encoding putative His kinase (AHK), His phosphotransfer (AHP), response regulator (ARR, type-A and type-B), and related proteins. Among these related proteins, is a set of unique genes encoding proteins very similar to, but clearly distinct from the ARR family of response regulators. Notably, these lack the phospho-accepting aspartate site that is invariantly conserved in the receiver domain of the classical response regulators (Makino et al. (2000) Plant Cell Physiol 41:791-803). The three invariant amino acid residues (D-D-K) in typical bacteria, yeast and plants RRsis not conserved: the phosphate-accepting Asp (D) of the receiver-like domain is replaced by Glu (E), Asn (N), or Gln (Q). However, despite significant sequence divergence, many APRRs have other Asp residues in the conserved motifs and could still act as the final outputs of two-component phosphorelay in plants. Therefore, these novel proteins are collectively designated as 'Arabidopsis pseudo-response regulators (APRRs, APRR1 to APRR9)', and pseudo-response regulators (PRR) in other plants.

The APRR polypeptides are further classified into two subgroups based in particular on their distinctive C-terminal domain: one is represented by APRR1 and the other by APRR2. Members of the APRR1 family of pseudo-response regulators contain a characteristic signature domain named the C-motif, and also found CONSTANS transcription factor polypeptides. The C motif (or CCT motif) is rich in basic amino acids (Arg and Lys) and contains a putative nuclear localization signal. In contrast, members belonging to the APRR2 family have another signature domain named the B-motif, which is common to the type-B family of classical response regulators (Imamura et al. (1999) Plant Cell Physiol 40: 733-742; representative of the plant single Myb-related domains, which belong to the GARP subfamily). This B-type motif is further found in Golden2-like (GLK2) transcription factor polypeptides in involved in photosynthetic development and plastid biogenesis. APRR1 is identical to TOC1 (Strayer et al. (2000) Science 289: 768-771), and has been shown to be involved in phytochrome-mediated plant circadian regulation. APRR2 (also named TOC2), although also belonging to this family implicated in circadian rhythm regulation, is not itself regulated by light and its function has not yet been further characterized in higher plants.

In US patent US 7,193,129 "Stress-related polynucleotides and polypeptides in plants", are described nucleic acid sequences encoding PRR2 polypeptides (SEQ ID NO: 25), and constructs comprising these. Transgenic plants overexpressing a PRR2 polypeptide with increased tolerance to nitrogen limiting conditions as compared to a wild-type plant of the same species, are shown.

**Summary**

1. basic-Helix-Loop-Helix group 9 (bHLH9)

Surprisingly, it has now been found that modulating expression of a nucleic acid encoding a bHLH9 polypeptide gives plants having enhanced yield-related traits, in particular increased yield relative to control plants.
According one embodiment, there is provided a method for enhancing yield related traits of a plant relative to control plants, comprising modulating expression of a nucleic acid encoding a bHLH9 polypeptide in a plant.

2. Imbibition-inducible 1 (IMB1)

Surprisingly, it has now been found that modulating expression of a nucleic acid encoding an IMB1 polypeptide gives plants having enhanced yield-related traits, in particular increased yield relative to control plants, with the proviso that increased yield does not comprise increased oil content of a plant.
According one embodiment, there is provided a method for improving yield related traits of a plant relative to control plants, comprising modulating expression of a nucleic acid encoding an IMB1 polypeptide in a plant.

3. PCD-LIKE

Surprisingly, it has now been found that modulating expression of a nucleic acid encoding a PCD-like polypeptide gives plants having enhanced yield-related traits relative to control plants.
According one embodiment, there is provided a method for enhancing yield related traits of a plant relative to control plants, comprising modulating expression of a nucleic acid encoding a PCD-like polypeptide in a plant.

4. Pseudo Response Regulator type 2 (PRR2)

Surprisingly, it has now been found that increasing expression in a plant of a nucleic acid sequence encoding a PRR2 polypeptide as defined herein, gives plants having increased yield-related traits relative to control plants.
According to one embodiment, there is provided a method for increasing yield-related traits in plants relative to control plants, comprising increasing expression in a plant of a nucleic acid sequence encoding a PRR2 polypeptide as defined herein. The increased yield-related traits comprise one or more of: increased aboveground biomass, increased early vigor, earlier flowering, increased root biomass, increased plant height, increased seed yield per plant, increased number of filled seeds, increased total number of seeds, increased number of primary panicles, increased number of flowers per panicles, increased harvest index (HI), and increased Thousand Kernel Weight (TKW).

Definitions

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.
A deletion refers to removal of one or more amino acids from a protein.
An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.
A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$
a or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions. For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or-10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| **Gene Source** | **Reference** |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987; |
| | Baszczynski et al., Plant Mol. Biol. 14: 633, 1990. |
| Brazil Nut albumin | Pearson et al., Plant Mol. Biol. 18: 235-245, 1992. |

(continued)

| Gene source | Reference |
|---|---|
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147 trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; akaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Methods for decreasing expression are known in the art and the skilled person would readily be able to adapt the known methods for silencing so as to achieve reduction of expression of an endogenous gene in a whole plant or in parts thereof through the use of an appropriate promoter, for example.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

Examples of various methods for the reduction or substantial elimination of expression in a plant of an endogenous gene, or for lowering levels and/or activity of a protein, are known to the skilled in the art. A skilled person would readily be able to adapt the known methods for silencing, so as to achieve reduction of expression of an endogenous gene in a whole plant or in parts thereof through the use of an appropriate promoter, for example.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), and g) increased number of primary panicles, which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased seed yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp.,

*Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticale sp., Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

## Detailed description of the invention

Surprisingly, it has now been found that modulating expression in a plant of a nucleic acid encoding a bHLH9 polypeptide gives plants having enhanced yield-related traits relative to control plants. According to a first embodiment, the present invention provides a method for enhancing yield-related traits in plants relative to control plants, comprising modulating expression in a plant of a nucleic acid encoding a bHLH9 polypeptide.

Furthermore, surprisingly, it has now been found that modulating expression in a plant of a nucleic acid encoding an IMB1 polypeptide gives plants having enhanced yield-related traits relative to control plants. According to a first embodiment, the present invention provides a method for enhancing yield-related traits in plants relative to control plants, comprising modulating expression in a plant of a nucleic acid encoding an IMB1 polypeptide.

Even furthermore, surprisingly, it has now been found that modulating expression in a plant of a nucleic acid encoding a PCD-like polypeptide gives plants having enhanced yield-related traits relative to control plants. According to a first embodiment, the present invention provides a method for enhancing yield-related traits in plants relative to control plants, comprising modulating expression in a plant of a nucleic acid encoding a PCD-like polypeptide and optionally selecting for plants having enhanced yield-related traits.

Yet furthermore, surprisingly, it has now been found that increasing expression in a plant of a nucleic acid sequence encoding a PRR2 polypeptide as defined herein, gives plants having increased yield-related traits relative to control plants. According to a first embodiment, the present invention provides a method for increasing yield-related traits in plants relative to control plants, comprising increasing expression in a plant of a nucleic acid sequence encoding a PRR2 polypeptide.

The invention also provides hitherto unknown nucleic acid sequences encoding PRR2 polypeptides, and PRR2 polypeptides.

According to one embodiment of the present invention, there is therefore provided an isolated nucleic acid molecule selected from:

(i) a nucleic acid sequence as represented by SEQ ID NO: 459;
(ii) the complement of a nucleic acid sequence as represented by SEQ ID NO: 459;
(iii) a nucleic acid sequence encoding a PRR2 polypeptide having, in increasing order of preference, at least 50%, 55%, 60%, 65%, 70%, 75%, 80%, 85%, 90%, 95%, 96%, 97%, 98%, 99% or more amino acid sequence identity to the polypeptide sequence represented by SEQ ID NO: 460.

According to a further embodiment of the present invention, there is also provided an isolated polypeptide selected from:

(i) a polypeptide sequence as represented by SEQ ID NO: 460;

(ii) a polypeptide sequence having, in increasing order of preference, at least 50%, 55%, 60%, 65%, 70%, 75%, 80%, 85%, 90%, 95%, 96%, 97%, 98%, 99% or more amino acid sequence identity to a polypeptide sequence as represented by SEQ ID NO: 460;

(iii) derivatives of any of the polypeptide sequences given in (i) or (ii) above.

Concerning bHLH9 polypeptides, a preferred method for modulating (preferably, increasing) expression of a nucleic acid encoding a bHLH9 polypeptide is by introducing and expressing in a plant a nucleic acid encoding a bHLH9 polypeptide.

Concerning IMB1 polypeptides, a preferred method for modulating (preferably, increasing) expression of a nucleic acid encoding an IMB1 polypeptide is by introducing and expressing in a plant a nucleic acid encoding an IMB1 polypeptide.

Concerning PCD-like polypeptides, a preferred method for modulating (preferably, increasing) expression of a nucleic acid encoding a PCD-like polypeptide is by introducing and expressing in a plant a nucleic acid encoding a PCD-like polypeptide.

Concerning PRR2 polypeptides, a preferred method for increasing expression in a plant of a nucleic acid sequence encoding a PRR2 polypeptide is by introducing and expressing in a plant a nucleic acid sequence encoding a PRR2 polypeptide.

Concerning bHLH9 polypeptides, any reference hereinafter to a "protein useful in the methods of the invention" is taken to mean a bHLH9 polypeptide as defined herein. Any reference hereinafter to a "nucleic acid useful in the methods of the invention" is taken to mean a nucleic acid capable of encoding such a bHLH9 polypeptide. The nucleic acid to be introduced into a plant (and therefore useful in performing the methods of the invention) is any nucleic acid encoding the type of protein which will now be described, hereafter also named *"bHLH9* nucleic acid" or *"bHLH9* gene".

Concerning IMB1 polypeptides, any reference hereinafter to a "protein useful in the methods of the invention" is taken to mean an IMB1 polypeptide as defined herein. Any reference hereinafter to a "nucleic acid useful in the methods of the invention" is taken to mean a nucleic acid capable of encoding such an IMB1 polypeptide. The nucleic acid to be introduced into a plant (and therefore useful in performing the methods of the invention) is any nucleic acid encoding the type of protein which will now be described, hereafter also named *"IMB1* nucleic acid" or *"IMB1* gene".

Concerning PCD-like polypeptides, any reference hereinafter to a "protein useful in the methods of the invention" is taken to mean a PCD-like polypeptide as defined herein. Any reference hereinafter to a "nucleic acid useful in the methods of the invention" is taken to mean a nucleic acid capable of encoding such a PCD-like polypeptide. The nucleic acid to be introduced into a plant (and therefore useful in performing the methods of the invention) is any nucleic acid encoding the type of protein which will now be described, hereafter also named *"PCD* nucleic acid" or *"PCD* gene".

Concerning PRR2 polypeptides, any reference hereinafter to a "protein useful in the methods of the invention" is taken to mean a PRR2 polypeptide as defined herein. Any reference hereinafter to a "nucleic acid sequence useful in the methods of the invention" is taken to mean a nucleic acid sequence capable of encoding such a PRR2 polypeptide. The nucleic acid sequence to be introduced into a plant (and therefore useful in performing the methods of the invention) is any nucleic acid sequence encoding the type of polypeptide, which will now be described, hereafter also named *"PRR2* nucleic acid sequence" or *"PRR2* gene".

A "bHLH9-like polypeptide" as defined herein refers to any polypeptide comprising an HLH domain ((HMM) PFam PF00010, ProfileScan PS50888, SMART SM00353) thereby forming a basic helix-loop-helix domain (Interpro IPR001092), said HLH domain having in increasing order of preference at least 80%, 81 %, 82%, 83%, 84%, 85%, 86%, 87%, 88%, 89%, 90%, 91 %, 92%, 93%, 94%, 95%, 96%, 97%, 98%, 99% or 100% sequence identity to any of SEQ ID NO: 181 to SEQ ID NO: 268.

Preferably the bHLH9 polypeptide comprises a motif having at least 50%, 51 %, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98%, 99% or 100% to any one, preferably to both motifs: Motif 1 (SEQ ID NO: 269): VPCKIRAKRGXXXXXXXXCATHPRSIAERVRRTRISERMRKLQELVPNMDKQTNXTADM, wherein X represents independently any (naturally occurring) amino acid or a gap (that is X is optionally present); and Motif 2 (SEQ ID NO: 269): LDLAVEYIKXLQKQVQXXXLXEXRXK CTCXX, wherein X represents independently any (naturally occurring) amino acid or a gap (i.e. X is optionally present).

bHLH transcription factor polypeptides have conserved patterns of amino acids found at three positions within the basic region of the bHLH motif, (the 5-9-13 configuration) (Heim at al. 2003). A bHLH9 poypeptide useful in the methods of the invention has preferably a 5-9-13 configuration represented by R-E-R.

Alternatively a bHLH9 polypeptide useful in the methods of the invention is any polypeptide falling within the Group IX as defined by Heim et al. 2003.

Preferably, the homologue of a bHLH9 protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid of any of the polypeptides of Table A1, preferably to SEQ ID NO: 2. In addition the homologue of a bHLH9 protein comprises an HLH domain as described above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

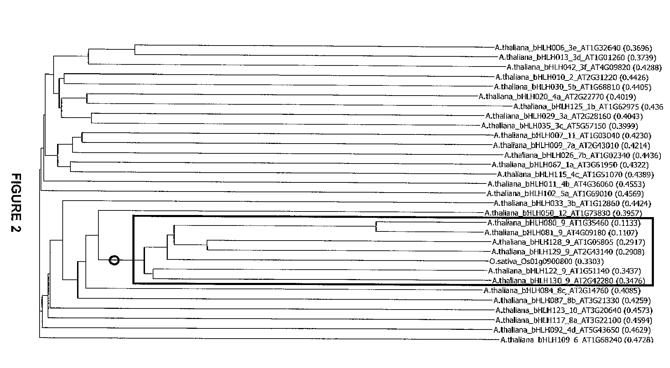

Alternatively, a bHLH9 polypeptide useful in the methods of the invention has an amino acid sequence which when used in the construction of a phylogenetic tree, such as the one depicted in Figure 2, clusters within the clade (group) defined by A.thaliana bHLH130 9 AT2G42280; A.thaliana bHLH122 9 AT1G51140; A.thaliana bHLH081 9 AT4G09180; A.thaliana bHLH080 9 AT1 G35460; A.thaliana bHLH128 9 AT1 G05805; and A.thaliana bHLH129 9 AT2G43140 comprising the amino acid sequence of Arabidopsis thaliana bHLH9 polypeptides rather than with any other group.

An "IMB1 polypeptide" as defined herein refers to a transcriptional activator protein of the BET family (Bromodomain and ET domain), one of the three bromodomain-protein families (Loyola and Almouzni, 2004). An IMB1 polypeptide comprises a single bromodomain (Pfam PF00439) followed by a NET domain (Duque and Chua, 2003). The NET domain is a part of a larger ET domain, which is involved in protein-protein interactions and which consists of three separate regions: the NET domain (for N-terminal ET), an intervening sequence and the C-terminal SEED motif. Only the NET domain is conserved in all BET proteins (Florence and Faller, Frontiers in BioScience 6, d1008-1018, 2001). The sequence of the NET domain suggests it is bipartite: i.e., two conserved motifs separated by a spacer of variable length. The N-terminal portion is predicted to form two alpha helices and the C-terminal region an extended beta sheet followed by an alpha helix. The spacer separates these two regions and is likely to form a loop. The conservation of the NET domain and bromodomain(s) suggests that all BET proteins have some function or functions in common and so at least partially redundant within individual species (Florence and Faller, 2001). The IMB1 polypeptide furthermore comprises a nuclear targeting signal.

In addition, the IMB1 polypeptide comprises one or more the following motifs:

Motif 3 (part of the bromodomain, SEQ ID NO: 304)

A(W/Q/E/H/G)PF(L/M)(D/Q/K/E/H)PV(D/N)V(E/V/K)(G/T)L(G/Q/H/C/R)(L/I)(Y/D/P/H/R)DY(Y/H/F/N/)(Q/K/N/E/D)(I/V)(I/V)(E/T/D/Q)(K/Q/R)PMD(F/L)(S/G/R)TI

Motif 4 (SEQ ID NO: 305)
D(V/M)RL(I/V)FXNAM(K/R/N/T)YN
wherein X in position 7 may be any amino acid, preferably one of K, T, A, E, S, Q or N;
Motif 5 (SEQ ID NO: 306)
M(A/S)(K/E/R)(T/F/S/K)L(L/M/S)(E/D/G/A)(K/R)FE(G/E/D)(C/K)
Motif 6 (SEQ ID NO: 307)
TL(F/W)(R/K)L

Preferably, the IMB1 polypeptide comprises also one or more the following motifs:

Motif 7 (SEQ ID NO: 308)
EAKDG
Motif 8 (SEQ ID NO: 309)
(Q/H/K)LEEL
Motif 9 (SEQ ID NO: 310)
LSNEL
Motif 10 (SEQ ID NO: 311)
KAL(E/L)(I/L/M)V
Motif 11 (SEQ ID NO: 312)
VIQII

Alternatively, the homologue of an IMB1 protein has in increasing order of preference at least 30%, 31%, 32%, 33%, 34%, 35%, 36%, 37%, 38%, 39%, 40%, 41 %, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 301, provided that the homologous protein comprises the bromodomain, the NET domain and conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

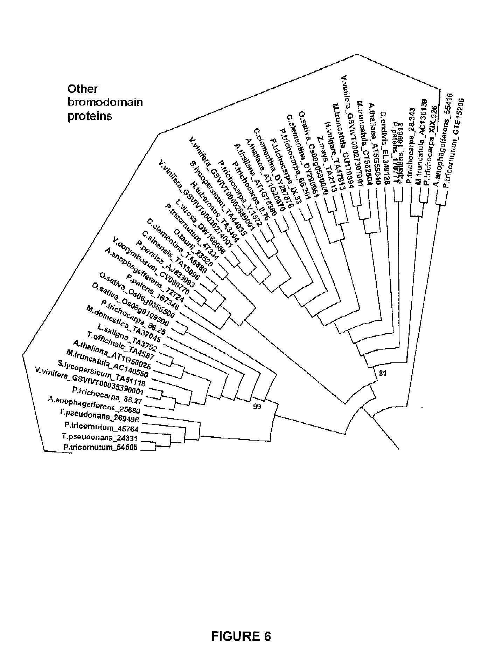

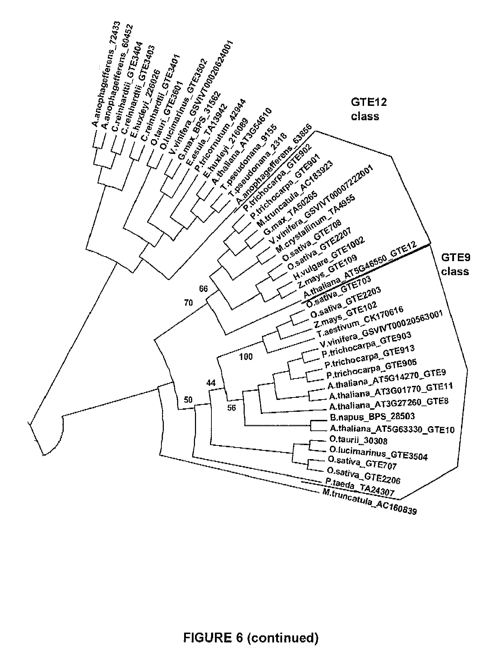

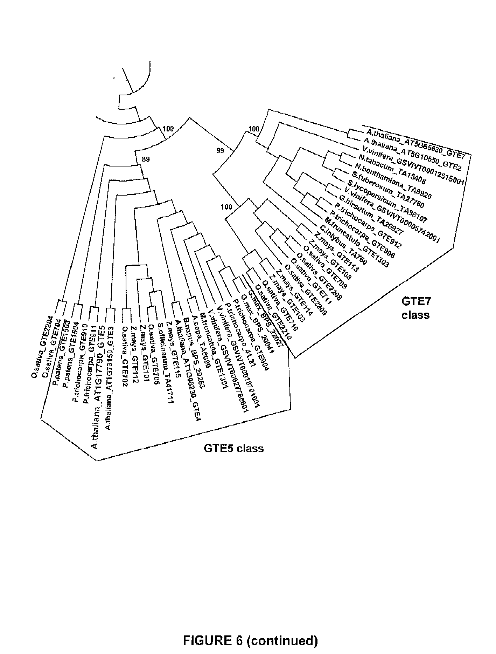

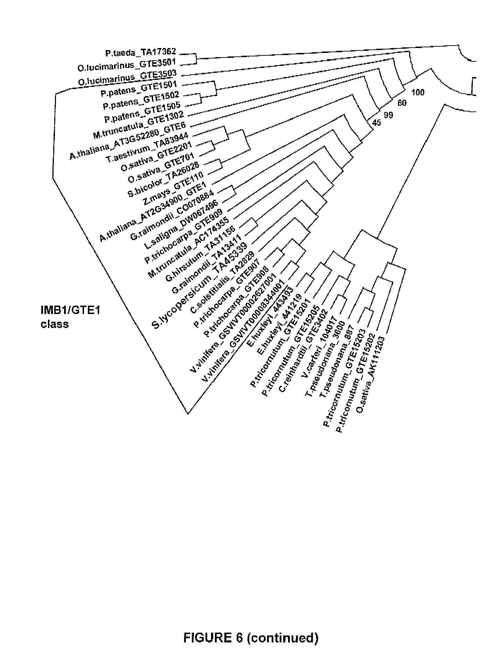

Preferably, the polypeptide sequence which when used in the construction of a phylogenetic tree, such as the one depicted in Figure 6, clusters with the group of IMB1/GTE1 class polypeptides comprising the amino acid sequence represented by SEQ ID NO: 301 rather than with any other group.

A "PCD-like polypeptide" as defined herein refers to any polypeptide comprising a Pterin-4-alpha-carbinolamine dehydratase (PCD) domain (Interpro accession number: IPR001533; pfam accession number: PF01329), said PCD domain preferably having in increasing order of preference at least 50%, 51 %, 52%, 53%, 54%, 55%, 56%, 57%, 58%, 59%, 60%, 61 %, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 83 which represents the PCD domain as present in SEQ ID NO: 359. A "PCD-like polypeptide" has optionally Pterin-4-alpha-carbinolamine dehydratase acitivity (EC:4.2.1.96).

Preferably the PCD-like polypeptide comprises one or more motifs having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, 99% or 100% to any one or more of the following motifs:

(i) Motif 12 (SEQ ID NO: 441): [G/E]-[D/N]-[F/L]-G-A-R-D-P-x(3)-E-x(4)-F-G-[D/E]K;
(ii) Motif 13 (SEQ ID NO: 442): [E/D/K/H/Q/N]-x(3)-H-[H/N]-[P/C/S]-x(5,6)-[Y/W/F/H]-x(9)-[H/W]-x(8,15)-D;
(iii) Motif 14 (SEQ ID NO: 443): WK(V/L)(R/K) wherein amino acids in brackets represent alternative amino acids at the same location;
(iv) Motif 15 (SEQ ID NO: 444): TDFI;
(v) Motif 16 (SEQ ID NO: 445): (S/V)(V/I)(G/R/S/A)GL(T/S);
(vi) Motif 17 (SEQ ID NO: 446): LGDF(G/R)(A/R)(R/A)(D/G)P;
(vii) Motif 18 (SEQ ID NO: 447): H(R/K)ILIP(T/A)

wherein amino acids in brackets represent alternative amino acids at that position, wherein the number in brackets indicate the number of X amino acids at a given location, and wherein X may be optionally present and when present represents independently any amino acid. Numbers between brackets separated by a comma represent alternatives number of X amino acids at that location.

Preferably, the homologue of a PCD-like polypeptide useful in the methods of the invention is homologue or an orthologue of any of the polypeptides of Table A3, said homologue or orthologue having in increasing order of preference at least 25%, 26%, 27%, 28%, 29%, 30%, 31 %, 32%, 33%, 34%, 35%, 36%, 37%, 38%, 39%, 40%, 41%, 42%, 43%, 44%, 45%, 46%, 47%, 48%, 49%, 50%, 51 %, 52%, 53%, 54%, 55%, 56%, 57%, 58%, 59%, 60%, 61 %, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% overall sequence identity to the amino acid of any of the polypeptides of Table A3, preferably to SEQ ID NO: 359. In addition the homologue of a PCD-like protein comprises a PCD domain as described above. The sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

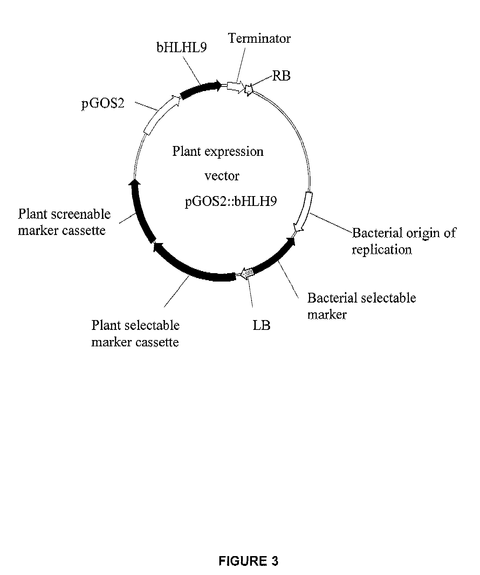

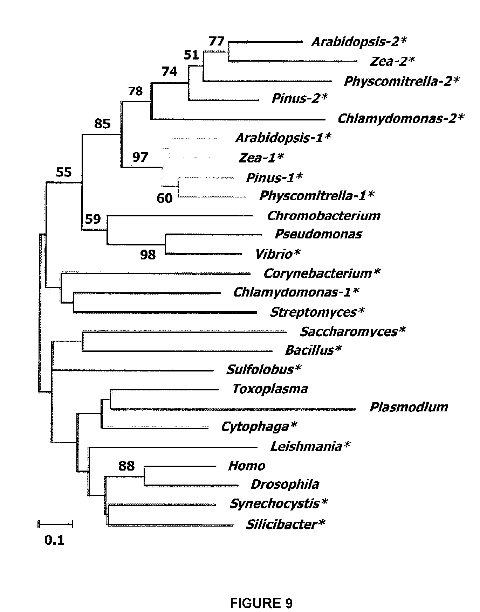

Alternatively, a PCD polypeptide useful in the methods of the invention has an amino acid sequence which when used in the construction of a phylogenetic tree, such as the one depicted in Figure 3B of Naponelli et al. 2008 (herein reproduced in Figure 9), clusters within the clade defined by the PCD polypeptides originating from organisms of the viridiplantae kingdom (green plants), i.e. with Arabidopsis-1; Arabidopsis-2; Pinus-1; Pinus-2; Physcomitrella-1; Physcomitrella-2, Zea-1 and Zea-2, rather than with any other group.

A "PRR2 polypeptide" as defined herein refers to any polypeptide comprising (i) a signal transduction response regulator receiver region with an InterPro entry IPR001789; and (ii) a Myb-like DNA-binding region (SHAQKYF class) with an InterPro entry IPR006447.

Alternatively or additionally, "PRR2 polypeptide" as defined herein refers to any polypeptide comprising (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476.

Additionally, a "PRR2 polypeptide" as defined herein further comprises in increasing order of preference at least 50%, 55%, 60%, 65%, 70%, 75%, 80%, 85%, 90%, 95%, 98%, 99% or more amino acid sequence identity to a C-terminal conserved domain as represented by SEQ ID NO: 477.

Alternatively or additionally, a "PRR2 polypeptide" as defined herein refers to any polypeptide sequence having in increasing order of preference at least 50%, 55%, 60%, 65%, 70%, 75%, 80%, 85%, 90%, 95%, 98%, 99% or more amino acid sequence identity to a polypeptide as represented by SEQ ID NO: 452.

Alternatively or additionally, a "PRR2 polypeptide" as defined herein refers to any polypeptide having in increasing order of preference at least 50%, 55%, 60%, 65%, 70%, 75%, 80%, 85%, 90%, 95%, 98%, 99% or more amino acid sequence identity to any of the polypeptide sequences given in Table A4 herein.

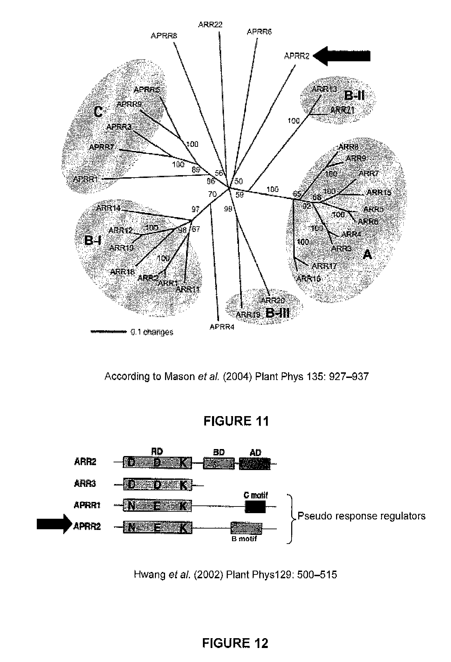

Alternatively or additionally, a "PRR2 polypeptide" as defined herein refers to any polypeptide sequence which when used in the construction of a response receiver domain phylogenetic tree, such as the one depicted in Figure 11, clusters with the APRR2 (pseudo response regulator type 2) group of polypeptides comprising the polypeptide sequences as represented by SEQ ID NO: 452 and SEQ ID NO: 456, rather than with any other group.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

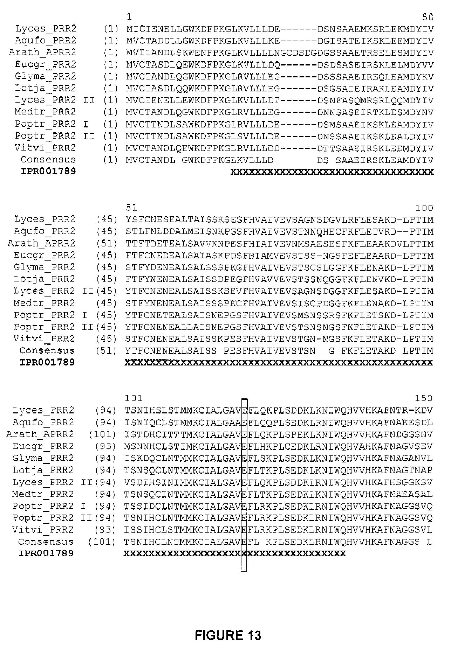

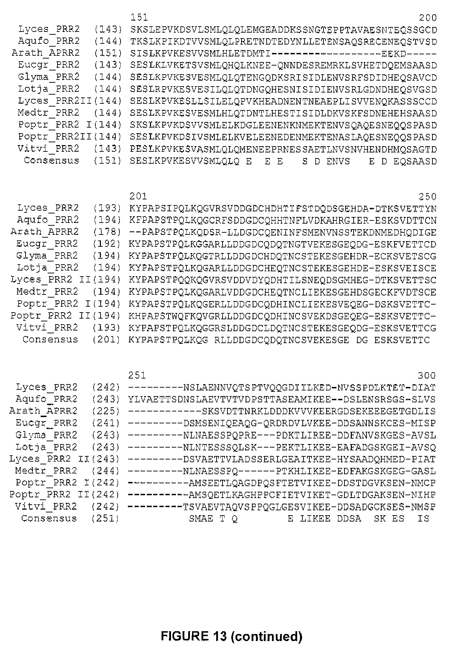

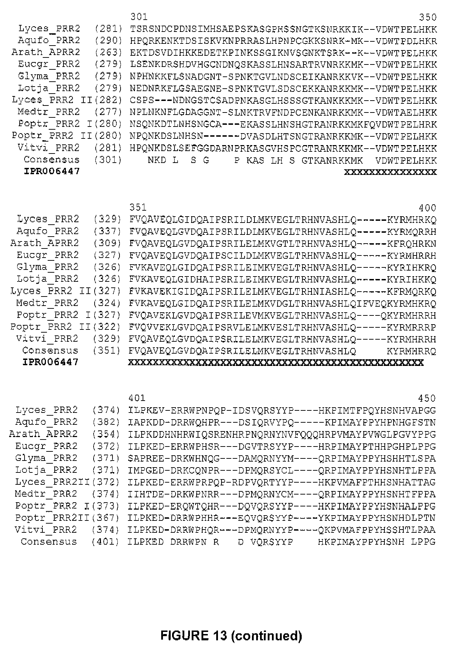

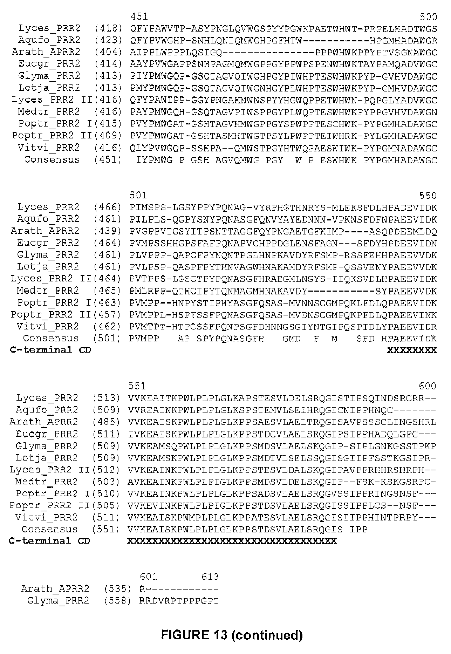

Concerning PRR2 polypeptides, an alignment of the polypeptides of Table A4 herein, is shown in Figure 13. Such alignments are useful for identifying the most conserved domains or motifs between the PRR2 polypeptides as defined herein. Two such domains are (i) a signal transduction response regulator receiver region with an InterPro entry IPROO1789 (marked by X's in Figure 13); and (ii) a Myb-like DNA-binding region (SHAQKYF class) with an InterPro entry IPROO6447 (marked by X's in Figure 13). Another such domain is a C-terminal conserved domain as represented by SEQ ID NO: 477, also marked by X's in Figure 13.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Concerning PRR2 polypeptides, the examples section herein describes in Table B4 the percentage identity between the PRR2 polypeptide as represented by SEQ ID NO: 452 and the PRR2 polypeptides listed in Table A4, which can be as low as 46% amino acid sequence identity. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified.

The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others. Furthermore, bHLH9 polypeptides (at least in their native form) typically have DNA binding activity. Tools and techniques for measuring DNA binding activity are well known in the art. In addition, as shown in the present invention, a bHLH9 protein, such as SEQ ID NO: 2, when overexpressed in rice, gives plants having enhanced yield-related traits, in particular emergence (early) vigour and/or increased number of flowers per panicle. Other bioassays are provided in Dombrecht et al. (Plant Cell 19, 2225-2245, 2007). Further details are provided in Examples section.

Preferably a bHLH9 polypeptide useful in the methods of the invention is able to bind a DNA molecule of in increasing order of preference at least 10, 20, 30, 40, 50, 100, 200, 300, 400, 500, 600, 700, 800, 900, 1000, 2000, 3000 or more nucleotides in length comprising one or more repeats of an E-box motif as represented by SEQ ID NO: 273 (CANNTG), preferably by SEQ ID NO: 274 (CACGTG: G-box). More preferably the E-box and/or G-box motifs are comprised within a promoter, that is, within a polynucleotide capable of driving the expression of a gene, preferably in a plant cell.

The E-box and G-box DNA-binding motifs as well as methods to assay the binding of a polypeptide to polynucleotide molecules comprising such E-box and G-box motif are well known in the art (Heim et al. 2003; Kim et al. 2007 Plant Mol Biol. 64(4):453-66; Ouwerkek Mol Gen Genet. 1999, 261(4-5):635-43).

Furthermore, IMB1 polypeptides (at least in their native form) transcription factors and typically have DNA binding activity. Tools and techniques for measuring DNA binding activity are well known in the art and include for example electrophoretic mobility shift assays (EMSA). Further details are provided in the examples section.

In addition, IMB1 polypeptides, when expressed in rice according to the methods of the present invention as outlined in the examples section, give plants having increased yield related traits, in particular increased seed yield.

Concerning PCD-like polypeptides, preferably a PCD polypeptide useful in the methods of the invention has Pterin-4-alpha-carbinolamine dehydratase activity (EC:4.2.1.96). Techniques and methods to measure PCD activity are well known in the art, for example in Hauer et al. 1993 J. Biol. Chem. 268 (1993) 4828-4831 or Cronk et al. 1996. Alternatively, a PCD polypeptide is able to complement the growth phenotype of E. coli strain JP2255 (aroF363 pheA361 pheO352 tyrA382 thi-1 strR712 lacY1 xyl-15; Zhao et al., 1994) using the method described by Naponelli et al. 2008.

In addition, PCD polypeptides, when expressed in rice according to the methods of the present invention as outlined in the Examples section, give plants having increased yield related traits, in particular increased total seed weight (seed yield) per plant, increased number of filled seeds per plant, increased harvest index, increased total number of seeds per plant.

Concerning bHLH9 polypeptides, the present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any bHLH9-encoding nucleic acid or bHLH9 polypeptide as defined herein.

Examples of nucleic acids encoding bHLH9 polypeptides are given in Table A1 of The Examples section herein. Such nucleic acids are useful in performing the methods of the invention. The amino acid sequences given in Table A1 of The Examples section are example sequences of orthologues and paralogues of the bHLH9 polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A1 of The Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning IMB1 polypeptides, the present invention is illustrated by transforming plants with the nucleic acid sequence represented by SEQ ID NO: 300, encoding the polypeptide sequence of SEQ ID NO: 301. However, performance of the invention is not restricted to these sequences; the methods of the invention may advantageously be performed using any IMB1-encoding nucleic acid or IMB1 polypeptide as defined herein.

Examples of nucleic acids encoding IMB1 polypeptides are given in Table A2 of The Examples section herein. Such nucleic acids are useful in performing the methods of the invention. The amino acid sequences given in Table A2 of The Examples section are example sequences of orthologues and paralogues of the IMB1 polypeptide represented by SEQ ID NO: 301, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A2 of The Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 300 or SEQ ID NO: 301, the second BLAST would therefore be against *Solanum lycopersicum* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning PCD-like polypeptides, the present invention is illustrated by transforming plants with the nucleic acid sequence represented by SEQ ID NO: 358, encoding the polypeptide sequence of SEQ ID NO: 359. However, performance of the invention is not restricted to these sequences; the methods of the invention may advantageously be performed using any PCD-encoding nucleic acid or PCD polypeptide as defined herein.

Examples of nucleic acids encoding PCD polypeptides are given in Table A3 of The Examples section herein. Such nucleic acids are useful in performing the methods of the invention. The amino acid sequences given in Table A3 of The Examples section are example sequences of orthologues and paralogues of the PCD polypeptide represented by SEQ ID NO: 359, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A of The Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 358 or SEQ ID NO: 359, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning PRR2 polypeptides, the present invention is illustrated by transforming plants with the nucleic acid sequence represented by SEQ ID NO: 451, encoding the PRR2 polypeptide sequence of SEQ ID NO: 452. However, performance of the invention is not restricted to these sequences; the methods of the invention may advantageously be performed using any nucleic acid sequence encoding a PRR2 polypeptide as defined herein.

Examples of nucleic acid sequences encoding PRR2 polypeptides are given in Table A4 of Example 1 herein. Such nucleic acid sequences are useful in performing the methods of the invention. The polypeptide sequences given in Table A4 of Example 1 are example sequences of orthologues and paralogues of the PRR2 polypeptide represented by SEQ ID NO: 452, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A4 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 451 or SEQ ID NO: 452, the second BLAST would therefore be against *Lycopersicon esculentum* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A1 to A4 of The Examples section, the terms "homologue" and "derivative" being as defined herein and also include variants in which codon usage is optimised or in which miRNA target sites are removed. Also useful in the methods of the invention are nucleic acids encoding homologues and derivatives of orthologues or paralogues of any one of the amino acid sequences given in Table A1 to A4 The Examples section. Homologues and derivatives useful in the methods of the present invention have substantially the same biological and functional activity as the unmodified protein from which they are derived.

Further nucleic acid variants useful in practising the methods of the invention include portions of nucleic acids encoding bHLH9 polypeptides, or IMB1 polypeptides, or PCD polypeptides, or PRR2 polypeptides, nucleic acids hybridising to nucleic acids encoding bHLH9 polypeptides, or IMB1 polypeptides, or PCD polypeptides, or PRR2 polypeptides, splice variants of nucleic acids encoding bHLH9 polypeptides, or IMB1 polypeptides, or PCD polypeptides, or PRR2 polypeptides, allelic variants of nucleic acids encoding bHLH9 polypeptides, or IMB1 polypeptides, or PCD polypeptides, or PRR2 polypeptides, and variants of nucleic acids encoding bHLH9 polypeptides obtained by gene shuffling. The terms hybridising sequence, splice variant, allelic variant and gene shuffling are as described herein.

Nucleic acids encoding bHLH9 polypeptides, or IMB1 polypeptides, or PCD polypeptides, or PRR2 polypeptides, need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A1 to A4 of The Examples section, or a portion of a nucleic acid encoding an orthologue, paralogue or homologue of any of the amino acid sequences given in Table A1 to A4 of The Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Concerning bHLH9 polypeptides, portions useful in the methods of the invention, encode a bHLH9 polypeptide as defined herein, and have substantially the same biological activity as the amino acid sequences given in Table A1 of The Examples section. Preferably, the portion is a portion of any one of the nucleic acids given in Table A1 of The Examples section, or is a portion of a nucleic acid encoding an orthologue or paralogue of any one of the amino acid sequences given in Table A1 of The Examples section. Preferably the portion is at least 150, 200, 250, 300, 350, 400 450, 500, 550, 600, 650, 700, 750, 800, 850, 900, 950, 1000 consecutive nucleotides in length, the consecutive nucleotides being of any one of the nucleic acid sequences given in Table A1 of The Examples section, or of a nucleic acid encoding an orthologue or paralogue of any one of the amino acid sequences given in Table A1 of The Examples section. Most preferably the portion is a portion of the nucleic acid of SEQ ID NO: 1. Preferably, the portion encodes a fragment of an amino acid sequence which, when used in the construction of a phylogenetic tree, such as the one depicted in Figure 2, clusters within the clade (group) defined by A.thaliana bHLH130 9 AT2G42280; A.thaliana bHLH122 9 AT1G51140; A.thaliana bHLH081 9 AT4G09180; A.thaliana bHLH080 9 AT1G35460; A.thaliana bHLH128 9 AT1G05805; and A.thaliana bHLH129 9 AT2G43140 comprising the amino acid sequence of Arabidopsis thaliana bHLH9 polypeptides rather than with any other group.

Concerning IMB1 polypeptides, portions useful in the methods of the invention, encode an IMB1 polypeptide as defined herein, and have substantially the same biological activity as the amino acid sequences given in Table A2 of The Examples section. Preferably, the portion is a portion of any one of the nucleic acids given in Table A2 of The Examples section, or is a portion of a nucleic acid encoding an orthologue or paralogue of any one of the amino acid sequences given in Table A2 of The Examples section. Preferably the portion is at least 400, 450, 500, 550, 600, 650, 700, 750, 800, 850, 900, 950, 1000, 1050, 1100, 1150, 1200, 1250, 1300, 1350, 1400, 1450,1500, 1550 consecutive nucleotides in length, the consecutive nucleotides being of any one of the nucleic acid sequences given in Table A2 of The Examples section, or of a nucleic acid encoding an orthologue or paralogue of any one of the amino acid sequences given in Table A2 of The Examples section. Most preferably the portion is a portion of the nucleic acid of SEQ ID NO: 300. Preferably, the portion encodes a fragment of an amino acid sequence which, when used in the construction of a phylogenetic tree, such as the one depicted in Figure 6, clusters with the group of IMB1/GTE1 class polypeptides comprising the amino acid sequence represented by SEQ ID NO: 301 rather than with any other group.

Concerning PCD-like polypeptides, portions useful in the methods of the invention, encode a PCD-like polypeptide as defined herein, and have substantially the same biological activity as the amino acid sequences given in Table A3 of The Examples section. Preferably, the portion is a portion of any one of the nucleic acids given in Table A3 of The Examples section, or is a portion of a nucleic acid encoding an orthologue or paralogue of any one of the amino acid sequences given in Table A3 of The Examples section. Preferably the portion is at least 100, 200, 300, 400, 500, 550, 600, 650, 700, 750, 800, 850, 900, 950, 1000 consecutive nucleotides in length, the consecutive nucleotides being of any one of the nucleic acid sequences given in Table A3 of The Examples section, or of a nucleic acid encoding an orthologue or paralogue of any one of the amino acid sequences given in Table A3 of The Examples section. Most preferably the portion is a portion of the nucleic acid of SEQ ID NO: 358. Preferably, the portion encodes a fragment of an amino acid sequence which, when used in the construction of a phylogenetic tree, such as the one depicted in Figure 3B of Naponelli et al. 2008 (herein reproduced in Figure 9), clusters within the clade defined by the PCD polypeptides originating from organisms of the viridiplantae kingdom (green plants), i.e. with Arabidopsis-1; Arabidopsis-2; Pinus-1; Pinus-2; Physcomitrella-1; Physcomitrella-2, Zea-1 and Zea-2, rather than with any other group. Concerning PRR2 polypeptides, portions useful in the methods of the invention, encode a PRR2 polypeptide as defined herein, and have substantially the same biological activity as the polypeptide sequences given in Table A4 of The Examples section. Preferably, the portion is a portion of any one of the nucleic acid sequences given in Table A4 of The Examples section, or is a portion of a nucleic acid sequence encoding an orthologue or paralogue of any one of the polypeptide sequences given in Table A4 of The Examples section. Preferably the portion is, in increasing order of preference at least 800, 900, 1000, 1100, 1150, 1200, 1250, 1300, 1350, 1400, 1450, 1500, 1550, 1600, 1650 or more consecutive nucleotides in length, the consecutive nucleotides being of any one of the nucleic acid sequences given in Table A4 of The Examples section, or of a nucleic acid sequence encoding an orthologue or paralogue of any one of the polypeptide sequences given in Table A4 of The Examples section. Preferably, the portion is a portion of a nucleic sequence encoding a polypeptide sequence comprising (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476. More preferably, the portion is a portion of a nucleic sequence encoding a polypeptide sequence having in increasing order of preference at least 50%, 55%, 60%, 65%, 70%, 75%, 80%, 85%, 90%, 95%, 98%, 99% or more amino acid sequence identity to the PRR2 polypeptide as represented by SEQ ID NO: 452 or to any of the polypeptide sequences given in Table A4 herein. Most preferably, the portion is a portion of the nucleic acid sequence of SEQ ID NO: 451.

Another nucleic acid variant useful in the methods of the invention is a nucleic acid capable of hybridising, under reduced stringency conditions, preferably under stringent conditions, with a nucleic acid encoding a bHLH9 polypeptide, or an IMB1 polypeptide, or a PCD polypeptide, or a PRR2 polypeptide, as defined herein, or with a portion as defined herein. According to the present invention, there is provided a method for enhancing and/or increasing yield-related traits in plants, comprising introducing and expressing in a plant a nucleic acid capable of hybridizing to any one of the nucleic acids given in Table A1 to A4 of The Examples section, or comprising introducing and expressing in a plant a nucleic acid capable of hybridising to a nucleic acid encoding an orthologue, paralogue or homologue of any of the nucleic acid sequences given in Table A1 to A4 of The Examples section.

Concerning bHLH9 polypeptides, hybridising sequences useful in the methods of the invention encode a bHLH9 polypeptide as defined herein, having substantially the same biological activity as the amino acid sequences given in Table A1 of The Examples section. Preferably, the hybridising sequence is capable of hybridising to the complement of any one of the nucleic acids given in Table A1 of The Examples section, or to a portion of any of these sequences, a portion being as defined above, or the hybridising sequence is capable of hybridising to the complement of a nucleic acid encoding an orthologue or paralogue of any one of the amino acid sequences given in Table A1 of The Examples section. Most preferably, the hybridising sequence is capable of hybridising to the complement of a nucleic acid as represented by SEQ ID NO: 1 or to a portion thereof.

Preferably, the hybridising sequence encodes a polypeptide with an amino acid sequence which, when full-length and used in the construction of a phylogenetic tree, such as the one depicted in Figure 2, clusters within the clade (group) defined by A.thaliana bHLH130 9 AT2G42280; A.thaliana bHLH122 9 AT1G51140; A.thaliana bHLH081 9 AT4G09180; A.thaliana bHLH080 9 AT1G35460; A.thaliana bHLH128 9 AT1G05805; and A.thaliana bHLH129 9 AT2G43140 comprising the amino acid sequence of Arabidopsis thaliana bHLH9 polypeptides rather than with any other group.

Concerning IMB1 polypeptides, hybridising sequences useful in the methods of the invention encode an IMB1 polypeptide as defined herein, having substantially the same biological activity as the amino acid sequences given in Table A2 of The Examples section. Preferably, the hybridising sequence is capable of hybridising to the complement of any one of the nucleic acids given in Table A2 of The Examples section, or to a portion of any of these sequences, a portion being as defined above, or the hybridising sequence is capable of hybridising to the complement of a nucleic acid encoding an orthologue or paralogue of any one of the amino acid sequences given in Table A2 of The Examples section. Most preferably, the hybridising sequence is capable of hybridising to the complement of a nucleic acid as represented by SEQ ID NO: 300 or to a portion thereof.

Preferably, the hybridising sequence encodes a polypeptide with an amino acid sequence which, when full-length and used in the construction of a phylogenetic tree, such as the one depicted in Figure 6, clusters with the group of IMB1/GTE1 class polypeptides comprising the amino acid sequence represented by SEQ ID NO: 301 rather than with any other group. Concerning PCD-like polypeptides, hybridising sequences useful in the methods of the invention encode a PCD-like polypeptide as defined herein, having substantially the same biological activity as the amino acid sequences given in Table A3 of The Examples section. Preferably, the hybridising sequence is capable of hybridising to the complement of any one of the nucleic acids given in Table A3 of The Examples section, or to a portion of any of these sequences, a portion being as defined above, or the hybridising sequence is capable of hybridising to the complement of a nucleic acid encoding an orthologue or paralogue of any one of the amino acid sequences given in Table A3 of The Examples section. Most preferably, the hybridising sequence is capable of hybridising to the complement of a nucleic acid as represented by SEQ ID NO: 358 or to a portion thereof.

Preferably, the hybridising sequence encodes a polypeptide with an amino acid sequence which, when full-length and used in the construction of a phylogenetic tree, such as the one depicted in Figure 3B of Naponelli et al. 2008 (herein reproduced in Figure 2), clusters within the clade defined by the PCD polypeptides originating from organisms of the viridiplantae kingdom (green plants), i.e. with Arabidopsis-1; Arabidopsis-2; Pinus-1; Pinus-2; Physcomitrella-1; Physcomitrella-2, Zea-1 and Zea-2, rather than with any other group.

Concerning PRR2 polypeptides, hybridising sequences useful in the methods of the invention encode a PRR2 polypeptide as defined herein, and have substantially the same biological activity as the polypeptide sequences given in Table A4 of The Examples section. Preferably, the hybridising sequence is capable of hybridising to any one of the nucleic acid sequences given in Table A4 of The Examples section, or to a complement thereof, or to a portion of any of these sequences, a portion being as defined above, or wherein the hybridising sequence is capable of hybridising to a nucleic acid sequence encoding an orthologue or paralogue of any one of the polypeptide sequences given in Table A4 of The Examples section, or to a complement thereof. Preferably, the hybridising sequence is capable of hybridising to a nucleic acid sequence encoding a polypeptide sequence comprising (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476. More preferably, the hybridising sequence is capable of hybridising to a nucleic acid sequence encoding a polypeptide sequence having in increasing order of preference at least 50%, 55%, 60%, 65%, 70%, 75%, 80%, 85%, 90%, 95%, 98%, 99% or more amino acid sequence identity to the PRR2 polypeptide as represented by SEQ ID NO: 452 or to any of the polypeptide sequences given in Table A4 herein. Most preferably, the hybridising sequence is capable of hybridising to a nucleic acid sequence as represented by SEQ ID NO: 451 or to a portion thereof.

Another nucleic acid variant useful in the methods of the invention is a splice variant encoding a bHLH9 polypeptide, or an IMB1 polypeptide, or a PCD polypeptide, or a PRR2 polypeptide, as defined hereinabove, a splice variant being as defined herein.

According to the present invention, there is provided a method for enhancing and/or increasing yield-related traits in plants, comprising introducing and expressing in a plant a splice variant of any one of the nucleic acid sequences given in Table A1 to A4 of The Examples section, or a splice variant of a nucleic acid encoding an orthologue, paralogue or homologue of any of the amino acid sequences given in Table A1 to A4 of The Examples section.

Concerning bHLH9 polypeptides, preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant, when used in the construction of a phylogenetic tree, such as the one depicted in Figure 2, clusters within the clade (group) defined by A.thaliana bHLH130 9 AT2G42280; A.thaliana bHLH122 9 AT1G51140; A.thaliana bHLH081 9 AT4G09180; A.thaliana bHLH080 9 AT1G35460; A.thaliana bHLH128 9 AT1G05805; and A.thaliana bHLH129 9 AT2G43140 comprising the amino acid sequence of Arabidopsis thaliana bHLH9 polypeptides rather than with any other group.

Concerning IMB1 polypeptides, preferred splice variants are splice variants of a nucleic acid represented by SEQ ID NO: 300, or a splice variant of a nucleic acid encoding an orthologue or paralogue of SEQ ID NO: 301. Preferably, the amino acid sequence encoded by the splice variant, when used in the construction of a phylogenetic tree, such as the one depicted in Figure 6, clusters with the group of IMB1/GTE1 class polypeptides comprising the amino acid sequence represented by SEQ ID NO: 301 rather than with any other group.

Concerning PCD-like polypeptides, preferred splice variants are splice variants of a nucleic acid represented by SEQ ID NO: 358, or a splice variant of a nucleic acid encoding an orthologue or paralogue of SEQ ID NO: 359. Preferably, the amino acid sequence encoded by the splice variant, when used in the construction of a phylogenetic tree, such as the one depicted in Figure 3B of Naponelli et al. 2008 (herein reproduced in Figure 9), clusters within the clade defined by the PCD polypeptides originating from organisms of the viridiplantae kingdom (green plants), i.e. with Arabidopsis-1; Arabidopsis-2; Pinus-1; Pinus-2; Physcomitrella-1; Physcomitrella-2, Zea-1 and Zea-2, rather than with any other group.

Concerning PRR2 polypeptides, preferred splice variants are splice variants of a nucleic acid sequence represented by SEQ ID NO: 451, or a splice variant of a nucleic acid sequence encoding an orthologue or paralogue of SEQ ID NO: 452. Preferably, the splice variant is a splice variant of a nucleic acid sequence encoding a polypeptide sequence comprising (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476. More preferably, the splice variant is a splice variant of a nucleic acid sequence encoding a polypeptide sequence having in increasing order of preference at least 50%, 55%, 60%, 65%, 70%, 75%, 80%, 85%, 90%, 95%, 98%, 99% or more amino acid sequence identity to the PRR2 polypeptide as represented by SEQ ID NO: 452 or to any of the polypeptide sequences given in Table A4 herein. Most preferably, the splice variant is a splice variant of a nucleic acid sequence as represented by SEQ ID NO: 451, or of a nucleic acid sequence encoding a polypeptide sequence as represented by SEQ ID NO: 452.

Another nucleic acid variant useful in performing the methods of the invention is an allelic variant of a nucleic acid encoding a bHLH9 polypeptide, or an IMB1 polypeptide, or a PCD polypeptide, or a PRR2 polypeptide, as defined hereinabove, an allelic variant being as defined herein.

According to the present invention, there is provided a method for enhancing and/or increasing yield-related traits in plants, comprising introducing and expressing in a plant an allelic variant of any one of the nucleic acids given in Table A1 to A4 of The Examples section, or comprising introducing and expressing in a plant an allelic variant of a nucleic acid encoding an orthologue, paralogue or homologue of any of the amino acid sequences given in Table A1 to A4 of The Examples section.

Concerning bHLH9 polypeptides, the polypeptides encoded by allelic variants useful in the methods of the present invention have substantially the same biological activity as the bHLH9 polypeptide of SEQ ID NO: 2 and any of the amino acids depicted in Table A1 of The Examples section. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant, when used in the construction of a phylogenetic tree, such as the one depicted in Figure 2, clusters within the clade (group) defined by A.thaliana bHLH130 9 AT2G42280; A.thaliana bHLH122 9 AT1G51140; A.thaliana bHLH081 9 AT4G09180; A.thaliana bHLH080 9 AT1G35460; A.thaliana bHLH128 9 AT1G05805; and A.thaliana bHLH129 9 AT2G43140 comprising the amino acid sequence of Arabidopsis thaliana bHLH9 polypeptides rather than with any other group.

Concerning IMB1 polypeptides, the polypeptides encoded by allelic variants useful in the methods of the present invention have substantially the same biological activity as the IMB1 polypeptide of SEQ ID NO: 301 and any of the amino acids depicted in Table A2 of The Examples section. Allelic variants exist in nature, and encompassed within the methods of the present invention is the use of these natural alleles. Preferably, the allelic variant is an allelic variant of SEQ ID NO: 300 or an allelic variant of a nucleic acid encoding an orthologue or paralogue of SEQ ID NO: 301. Preferably, the amino acid sequence encoded by the allelic variant, when used in the construction of a phylogenetic tree, such as the one depicted in Figure 6, clusters with the group of IMB1/GTE1 class polypeptides comprising the amino acid sequence represented by SEQ ID NO: 301 rather than with any other group.

Concerning PCD-like polypeptides, the polypeptides encoded by allelic variants useful in the methods of the present invention have substantially the same biological activity as the PCD polypeptide of SEQ ID NO: 359 and any of the amino acids depicted in Table A3 of The Examples section. Allelic variants exist in nature, and encompassed within the methods of the present invention is the use of these natural alleles. Preferably, the allelic variant is an allelic variant of SEQ ID NO: 358 or an allelic variant of a nucleic acid encoding an orthologue or paralogue of SEQ ID NO: 359. Preferably, the amino acid sequence encoded by the allelic variant, when used in the construction of a phylogenetic tree, such as the one depicted in Figure 3B of Naponelli et al. 2008 (herein reproduced in Figure 9), clusters within the clade defined by the PCD polypeptides originating from organisms of the viridiplantae kingdom (green plants), i.e. with Arabidopsis-1; Arabidopsis-2; Pinus-1; Pinus-2; Physcomitrella-1; Physcomitrella-2, Zea-1 and Zea-2, rather than with any other group.

Concerning PRR2 polypeptides, the allelic variants useful in the methods of the present invention have substantially the same biological activity as the PRR2 polypeptide of SEQ ID NO: 452 and any of the polypeptide sequences depicted in Table A4 of The Examples section. Allelic variants exist in nature, and encompassed within the methods of the present invention is the use of these natural alleles. Preferably, the allelic variant is an allelic variant of a polypeptide sequence comprising (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476. More preferably the allelic variant is an allelic variant encoding a polypeptide sequence having in increasing order of preference at least 50%, 55%, 60%, 65%, 70%, 75%, 80%, 85%, 90%, 95%, 98%, 99% or more amino acid sequence identity to the PRR2 polypeptide as represented by SEQ ID NO: 452 or to any of the polypeptide sequences given in Table A4 herein. Most preferably, the allelic variant is an allelic variant of SEQ ID NO: 451 or an allelic variant of a nucleic acid sequence encoding an orthologue or paralogue of SEQ ID NO: 452.

Gene shuffling or directed evolution may also be used to generate variants of nucleic acids encoding bHLH9 polypeptides, or IMB1 polypeptides, or PCD polypeptides, or PRR2 polypeptides, as defined above; the term "gene shuffling" being as defined herein. According to the present invention, there is provided a method for enhancing yield-related traits in plants, comprising introducing and expressing in a plant a variant of any one of the nucleic acid sequences given in Table A1 to A4 of The Examples section, or comprising introducing and expressing in a plant a variant of a nucleic acid encoding an orthologue, paralogue or homologue of any of the amino acid sequences given in Table A1 to A4 of The Examples section, which variant nucleic acid is obtained by gene shuffling.

Concerning bHLH9 polypeptides, preferably, the amino acid sequence encoded by the variant nucleic acid obtained by gene shuffling, when used in the construction of a phylogenetic tree such as the one depicted in Figure 2, clusters within the clade (group) defined by A.thaliana bHLH130 9 AT2G42280; A.thaliana bHLH122 9 AT1G51140; A.thaliana bHLH081 9 AT4G09180; A.thaliana bHLH080 9 AT1G35460; A.thaliana bHLH128 9 AT1G05805; and A.thaliana bHLH129 9 AT2G43140 comprising the amino acid sequence of Arabidopsis thaliana bHLH9 polypeptides rather than with any other group.

Concerning IMB1 polypeptides, treferably, the amino acid sequence encoded by the variant nucleic acid obtained by gene shuffling, when used in the construction of a phylogenetic tree such as the one depicted in Figure 6, clusters with the group of IMB1/GTE1 class polypeptides comprising the amino acid sequence represented by SEQ ID NO: 301 rather than with any other group.

Concerning PCD-like polypeptides, preferably, the amino acid sequence encoded by the variant nucleic acid obtained by gene shuffling, when used in the construction of a phylogenetic such as the one depicted in Figure 3B of Naponelli et al. 2008 (herein reproduced in Figure 9), clusters within the clade defined by the PCD polypeptides originating from organisms of the viridiplantae kingdom (green plants), i.e. with Arabidopsis-1; Arabidopsis-2; Pinus-1; Pinus-2; Physcomitrella-1; Physcomitrella-2, Zea-1 and Zea-2, rather than with any other group.

Concerning PRR2 polypeptides, preferably, the variant nucleic acid sequence obtained by gene shuffling encodes a polypeptide sequence comprising (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476. More preferably, the variant nucleic acid sequence obtained by gene shuffling encodes a polypeptide sequence having in increasing order of preference at least 50%, 55%, 60%, 65%, 70%, 75%, 80%, 85%, 90%, 95%, 98%, 99% or more amino acid sequence identity to the PRR2 polypeptide as represented by SEQ ID NO: 452 or to any of the polypeptide sequences given in Table A1 herein. Most preferably, the nucleic acid sequence obtained by gene shuffling encodes a polypeptide sequence as represented by SEQ ID NO: 452.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding bHLH9 polypeptides may be derived from any natural or artificial source. The nucleic acid may be modified from its native form in composition and/or genomic environment through deliberate human manipulation. Preferably the bHLH9 polypeptide-encoding nucleic acid is from a plant, further preferably from a monocotyledonous plant, more preferably from the family Poaceae, most preferably the nucleic acid is from *Oryza sativa.*

Advantageously, the invention also provides hitherto unknown bHLH9-encoding nucleic acids and bHLH9 polypeptides.

According to a further embodiment of the present invention, there is therefore provided an isolated nucleic acid molecule selected from:

(i) a nucleic acid represented by any one of SEQ ID NO: 19, 45, 47, 165, 167 and 169;
(ii) the complement of a nucleic acid represented by any one of SEQ ID NO: 19, 45, 47, 165, 167 and 169;
(iii) a nucleic acid encoding the polypeptide as represented by any one of SEQ ID NO: 20, 46, 48, 166, 168 and 170, preferably as a result of the degeneracy of the genetic code, said isolated nucleic acid can be derived from a polypeptide sequence as represented by any one of SEQ ID NO: 20, 46, 48, 166, 168 and 170 and further preferably confers enhanced yield-related traits relative to control plants;

(iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of Table A1 and Table C2 and further preferably conferring enhanced yield-related traits relative to control plants;

(v) a nucleic acid molecule which hybridizes with a nucleic acid molecule of (i) to (iv) under stringent hybridization conditions and preferably confers enhanced yield-related traits relative to control plants;

(vi) a nucleic acid encoding a bHLH9 polypeptide having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 20, 46, 48, 166, 168, 170 and any of the other amino acid sequences in Table A1 and Table C2 and preferably conferring enhanced yield-related traits relative to control plants.

According to a further embodiment of the present invention, there is also provided an isolated polypeptide selected from:

(i) an amino acid sequence represented by any one of SEQ ID NO: 20, 46, 48, 166, 168 and 170;

(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 20, 46, 48, 166, 168 and 170, and any of the other amino acid sequences in Table A1 and Table C2 and preferably conferring enhanced yield-related traits relative to control plants.

(iii) derivatives of any of the amino acid sequences given in (i) or (ii) above.

Nucleic acids encoding IMB1 polypeptides may be derived from any natural or artificial source. The nucleic acid may be modified from its native form in composition and/or genomic environment through deliberate human manipulation. Preferably the IMB1 polypeptide-encoding nucleic acid is from a plant, further preferably from a dicotyledonous plant, more preferably from the family Solanaceae, most preferably the nucleic acid is from *Solanum lycopersicum.*

Nucleic acids encoding PCD polypeptides may be derived from any natural or artificial source. The nucleic acid may be modified from its native form in composition and/or genomic environment through deliberate human manipulation. Preferably the PCD polypeptide-encoding nucleic acid is from a plant, further preferably from a monocotyledonous plant, more preferably from the family Poaceae, most preferably the nucleic acid is from *Oryza sativa.*

Nucleic acid sequences encoding PRR2 polypeptides may be derived from any natural or artificial source. The nucleic acid sequence may be modified from its native form in composition and/or genomic environment through deliberate human manipulation. The nucleic acid sequence encoding a PRR2 polypeptide is from a plant, further preferably from a dicotyledonous plant, more preferably from the family Solanaceae, most preferably the nucleic acid sequence is from *Lycopersicon esculentum.*

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants. Concerning IMB1 polypeptides, the term "yield- related traits", "yield" or "seed yield" as used in the present invention does not encompass increased oil content of a plant or a plant seed.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing yield, especially seed yield of plants, relative to control plants, which method comprises modulating expression in a plant of a nucleic acid encoding a bHLH9 polypeptide, or an IMB1 polypeptide, or a PCD polypeptide, as defined herein.

The present invention also provides a method for increasing yield-related traits of plants relative to control plants, which method comprises increasing expression in a plant of a nucleic acid sequence encoding a PRR2 polypeptide as defined herein.

Since the transgenic plants according to the present invention have increased yield and/or yield-related traits, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression in a plant of a nucleic acid encoding a bHLH9 polypeptide, or an IMB1 polypeptide, or a PCD-like polypeptide, or a PRR2 polypeptide, as defined herein.

An increase in yield and/or growth rate and/or increased yield-related traits occur whether the plant is under non-stress conditions or whether the plant is exposed to various stresses compared to control plants grown under comparable conditions. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, and/or pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes, and insects. The term "non-stress" conditions as used herein are those environmental conditions that allow optimal growth of plants. Persons skilled in the art are aware of normal soil conditions and climatic conditions for a given location.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Performance of the methods of the invention gives plants grown under non-stress conditions or under mild drought conditions increased yield and/or yield-related traits relative to control plants grown under comparable conditions. Therefore, according to the present invention, there is provided a method for increasing yield in plants grown under non-stress conditions or under mild drought conditions, which method comprises modulating expression in a plant of a nucleic acid encoding a bHLH9 polypeptide, or an IMB1 polypeptide, or a PCD-like polypeptide, or a PRR2 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises modulating expression in a plant of a nucleic acid encoding a bHLH9 polypeptide, or an IMB1 polypeptide, or a PCD-like polypeptide. Nutrient deficiency may result from a lack of nutrients such as nitrogen, phosphates and other phosphorous-containing compounds, potassium, calcium, cadmium, magnesium, manganese, iron and boron, amongst others.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCl), but may be any stress caused by one or more of: NaCl, KCl, LiCl, $MgCl_2$, $CaCl_2$, amongst others.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding a bHLH9 polypeptide, or an IMB1 polypeptide, or a PCD-like polypeptide. The term salt stress is not restricted to common salt (NaCl), but may be any one or more of: NaCl, KCl, LiCl, $MgCl_2$, $CaCl_2$, amongst others.

Performance of the methods of the invention gives plants having increased yield-related traits, under abiotic stress conditions relative to control plants grown in comparable stress conditions. Therefore, according to the present invention, there is provided a method for increasing yield-related traits, in plants grown under abiotic stress conditions, which method comprises increasing expression in a plant of a nucleic acid sequence encoding a PRR2 polypeptide. According to one aspect of the invention, the abiotic stress is an osmotic stress, selected from one or more of the following: water stress, salt stress, oxidative stress and ionic stress.

Another example of abiotic environmental stress is the reduced availability of one or more nutrients that need to be assimilated by the plants for growth and development. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor for crop plant growth and production (Frink et al. (1999) Proc Natl Acad Sci USA 96(4): 1175-1180), and has as well a major impact on protein accumulation and amino acid composition. Therefore, of great interest are crop plants with increased yield-related traits, when grown under nitrogen-limiting conditions.

Performance of the methods of the invention gives plants grown under conditions of reduced nutrient availability, particularly under conditions of reduced nitrogen availablity, having increased yield-related traits relative to control plants grown under comparable conditions. Therefore, according to the present invention, there is provided a method for increasing yield-related traits in plants grown under conditions of reduced nutrient availablity, preferably reduced nitrogen availability, which method comprises increasing expression in a plant of a nucleic acid sequence encoding a PRR2 polypeptide. Reduced nutrient availability may result from a deficiency or excess of nutrients such as nitrogen, phosphates and other phosphorous-containing compounds, potassium, calcium, cadmium, magnesium, manganese, iron and boron, amongst others. Preferably, reduced nutrient availablity is reduced nitrogen availability.

The present invention encompasses plants or parts thereof (including seeds) or cells thereof obtainable by the methods according to the present invention. The plants or parts thereof or cells thereof comprise a nucleic acid transgene encoding a bHLH9 polypeptide, or an IMB1 polypeptide, or a PCD-like polypeptide, or a PRR2 polypeptide, as defined above, operably linked to a promoter functioning in plants.

The invention also provides genetic constructs and vectors to facilitate introduction and/or expression in plants of nucleic acids encoding bHLH9 polypeptides, or IMB1 polypeptides, or PCD-like polypeptides, or PRR2 polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a bHLH9 polypeptide, or an IMB1 polypeptide, or a PCD-like polypeptide, or a PRR2 polypeptide, as defined above;
(b) one or more control sequences capable of driving expression of the nucleic acid sequence of (a); and optionally
(c) a transcription termination sequence.

Preferably, the nucleic acid encoding a bHLH9 polypeptide, or an IMB1 polypeptide, or a PCD-like polypeptide, or a PRR2 polypeptide, is as defined above. The term "control sequence" and "termination sequence" are as defined herein.

Concerning PRR2 polypeptides, preferably, one of the control sequences of a construct is a consitituve promoter isolated from a plant genome. An example of a constitutive promoter is a GOS2 promoter, preferably a GOS2 promoter from rice, most preferably a GOS2 sequence as represented by SEQ ID NO: 478.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is also a ubiquitous promoter of medium strength. See the "Definitions" section herein for definitions of the various promoter types.

Concerning PRR2 polypeptides, advantageously, any type of promoter, whether natural or synthetic, may be used to increase expression of the nucleic acid sequence. A constitutive promoter is particularly useful in the methods, preferably a constitutive promoter isolated from a plant genome. The plant constitutive promoter drives expression of a coding sequence at a level that is in all instances below that obtained under the control of a 35S CaMV viral promoter. An example of such a promoter is a GOS2 promoter as represented by SEQ ID NO: 478.

Concerning PRR2 polypeptides, organ-specific promoters, for example for preferred expression in leaves, stems, tubers, meristems, seeds, are useful in performing the methods of the invention. Developmentally-regulated and inducible promoters are also useful in performing the methods of the invention. See the "Definitions" section herein for definitions of the various promoter types.

Concerning bHLH9 polypeptides, it should be clear that the applicability of the present invention is not restricted to the bHLH9 polypeptide-encoding nucleic acid represented by SEQ ID NO: 1, nor is the applicability of the invention restricted to expression of a bHLH9 polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 275, most preferably the constitutive promoter is as represented by SEQ ID NO: 275. See the "Definitions" section herein for further examples of constitutive promoters.

Concerning IMB1 polypeptides, it should be clear that the applicability of the present invention is not restricted to the IMB1 polypeptide-encoding nucleic acid represented by SEQ ID NO: 300, nor is the applicability of the invention restricted to expression of an IMB1 polypeptide-encoding nucleic acid when driven by a constitutive promoter.

The constitutive promoter is preferably a medium strength promoter, more preferably a plant derived medium strength promoter, such as a GOS2 promoter, most preferably the promoter is the GOS2 promoter from rice. Further preferably the constitutive promoter is represented by a nucleic acid sequence substantially similar to SEQ ID NO: 313, most preferably the constitutive promoter is as represented by SEQ ID NO: 313. See the "Definitions" section herein for further examples of constitutive promoters.

Optionally, one or more terminator sequences may be used in the construct introduced into a plant. Preferably, the construct comprises an expression cassette comprising the rice GOS2 promoter, operably linked to the nucleic acid encoding the IMB1 polypeptide.

Concerning PCD-like polypeptides, it should be clear that the applicability of the present invention is not restricted to the PCD polypeptide-encoding nucleic acid represented by SEQ ID NO: 358, nor is the applicability of the invention restricted to expression of a PCD-like polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 450, most preferably the constitutive promoter is as represented by SEQ ID NO: 450. See the "Definitions" section herein for further examples of constitutive promoters.

Concerning PRR2 polypeptides, it should be clear that the applicability of the present invention is not restricted to a nucleic acid sequence encoding the PRR2 polypeptide, as represented by SEQ ID NO: 451, nor is the applicability of the invention restricted to expression of a PRR2 polypeptide-encoding nucleic acid sequence when driven by a constitituve promoter. Optionally, one or more terminator sequences may be used in the construct introduced into a plant.

Additional regulatory elements may include transcriptional as well as translational increasers. Those skilled in the art will be aware of terminator and increaser sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, increaser, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having enhanced yield-related traits relative to control plants, comprising introduction and expression in a plant of any nucleic acid encoding a bHLH9 polypeptide, or an IMB1 polypeptide, or a PCD-like polypeptide, or a PRR2 polypeptide, as defined hereinabove.

Concerning bHLH9 polypeptides, more specifically, the present invention provides a method for the production of transgenic plants having enhanced yield-related traits, particularly increased early vigour and/or increased biomass and/or increased seed yield relative to control plants, comprising:

(i) introducing and expressing in a plant a nucleic acid encoding a bHLH9 polypeptide; and
(ii) cultivating the plant cell under conditions promoting plant growth and development; and optionally
(iii) selecting for plants having increased yield.

The nucleic acid of (i) may be any of the nucleic acids capable of encoding a bHLH9 polypeptide as defined herein.

Concerning IMB1 polypeptides, more specifically, the present invention provides a method for the production of transgenic plants having enhanced yield-related traits, particularly increased (seed) yield, which method comprises:

(i) introducing and expressing in a plant or plant cell an IMB1 polypeptide-encoding nucleic acid; and
(ii) cultivating the plant cell under conditions promoting plant growth and development.

The nucleic acid of (i) may be any of the nucleic acids capable of encoding an IMB1 polypeptide as defined herein.
Concerning PCD-like polypeptides, more specifically, the present invention provides a method for the production of transgenic plants having enhanced yield-related traits, particularly increased (seed) yield, which method comprises:

(i) introducing and expressing in a plant or plant cell a PCD-like polypeptide-encoding nucleic acid; and
(ii) cultivating the plant cell under conditions promoting plant growth and development.

The nucleic acid of (i) may be any of the nucleic acids capable of encoding a PCD-like polypeptide as defined herein.
Concerning PRR2 polypeptides, more specifically, the present invention provides a method for the production of transgenic plants having increased yield-related traits relative to control plants, which method comprises:

(i) introducing and expressing in a plant, plant part, or plant cell a nucleic acid sequence encoding a PRR2 polypeptide; and
(ii) cultivating the plant cell, plant part or plant under conditions promoting plant growth and development.

The nucleic acid sequence of (i) may be any of the nucleic acid sequences capable of encoding a PRR2 polypeptide as defined herein.
The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.
The genetically modified plant cells can be regenerated via all methods with which the skilled worker is familiar. Suitable methods can be found in the abovementioned publications by S.D. Kung and R. Wu, Potrykus or Höfgen and Willmitzer.
Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.
Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.
The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).
The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.
The invention also includes host cells containing an isolated nucleic acid encoding a bHLH9 polypeptide, or an IMB1 polypeptide, or a PCD-like polypeptide, or a PRR2 polypeptide, as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding a bHLH9 polypeptide, or an IMB1 polypeptide, or a PCD-like polypeptide, or a PRR2 polypeptide. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a bHLH9 polypeptide, or an IMB1 polypeptide, or a PCD-like polypeptide, or a PRR2 polypeptide, is by introducing and expressing in a plant a nucleic acid encoding a bHLH9 polypeptide, or an IMB1 polypeptide, or a PCD-like polypeptide, or a PRR2 polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acids encoding bHLH9 polypeptides as described herein and use of these bHLH9 polypeptides, or IMB1 polypeptides, or PCD-like polypeptides, in enhancing any of the aforementioned yield-related traits in plants.

The present invention also encompasses use of nucleic acid sequences encoding PRR2 polypeptides as described herein and use of these PRR2 polypeptides in increasing any of the aforementioned yield-related traits in plants, under normal growth conditions, under abiotic stress growth (preferably osmotic stress growth conditions) conditions, and under growth conditions of reduced nutrient availability, preferably under conditions of reduced nitrogen availability.

Nucleic acids encoding bHLH9 polypeptides, or IMB1 polypeptides, or PCD-like polypeptides, or PRR2 polypeptides, described herein, or the bHLH9 polypeptides themselves, may find use in breeding programmes in which a DNA marker is identified which may be genetically linked to a gene encoding a bHLH9 polypeptide, or an IMB1 polypeptide, or a PCD-like polypeptide, or a PRR2 polypeptide. The nucleic acids/genes, or the bHLH9 polypeptides, or IMB1 polypeptides, or PCD-like polypeptides, or PRR2 polypeptides, themselves may be used to define a molecular marker. This DNA or protein marker may then be used in breeding programmes to select plants having enhanced yield-related traits as defined hereinabove in the methods of the invention.

Allelic variants of a nucleic acid/gene encoding a bHLH9 polypeptide, or an IMB1 polypeptide, or a PCD-like polypeptide, or a PRR2 polypeptide, may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding bHLH9 polypeptides, or IMB1 polypeptides, or PCD-like polypeptides, or PRR2 polypeptides, may also be used as probes for genetically and physically mapping the genes that they are a part of, and as markers for traits linked to those genes. Such information may be useful in plant breeding in order to develop lines with desired phenotypes. Such use of nucleic acids encoding bHLH9 polypeptides, or IMB1 polypeptides, or PCD-like polypeptides, or PRR2 polypeptides, requires only a nucleic acid sequence of at least 15 nucleotides in length. The nucleic acids encoding bHLH9 polypeptides, or IMB1 polypeptides, or PCD-like polypeptides, or PRR2 polypeptides, may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding bHLH9 polypeptides, or IMB1 polypeptides, or PCD-like polypeptides, or PRR2 polypeptides. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding bHLH9 polypeptides, or IMB1 polypeptides, or PCD-like polypeptides, or PRR2 polypeptides, in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Concerning bHLH9 polypeptides, or IMB1 polypeptides, or PCD-like poypeptides, the methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

Concerning PRR2 polypeptides, the methods according to the present invention result in plants having increased yield-related traits, as described hereinbefore. These traits may also be combined with other economically advantageous traits, such as further yield-increasing traits, tolerance to abiotic and biotic stresses, tolerance to herbicides, insectides, traits modifying various architectural features and/or biochemical and/or physiological features.

Items

The present invention will now be described in reference to the following items:

1. <u>basic-Helix-Loop-Helix group 9 (bHLH9)</u>

1. A method for enhancing yield-related traits in plants relative to control plants, comprising modulating expression in a plant of a nucleic acid encoding a bHLH9 (basic-Helix-Loop-Helix group 9) polypeptide comprising an HLH domain (Pfam accession number: PF00010) having in increasing order of preference at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to any of SEQ ID NO: 181 to SEQ ID NO: 268, preferably to SEQ ID NO: 225.

2. Method according to item 1, wherein said bHLH9 binds to an E-box motif as represented in increasing order of preference by SEQ ID NO: 273 (CANNTG) or SEQ ID NO: 274 (CACGTG).

3. Method according to item 1 or 2, wherein said modulated expression is effected by introducing and expressing in a plant a nucleic acid encoding a bHLH9 polypeptide.

4. Method according to any one of items 1 to 3, wherein said nucleic acid encoding a bHLH9 polypeptide encodes any one of the proteins listed in Table A1 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A1.

6. Method according to any preceding item, wherein said enhanced yield-related traits comprise increased yield, preferably increased early vigour and/or increased biomass and/or increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress, salt stress or nitrogen deficiency.

9. Method according to any one of items 3 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding a bHLH9 polypeptide is of plant origin, preferably from a monocotyledonous plant, further preferably from the family Poaceae, more preferably from the genus Oryza, most preferably from *Oryza sativa.*

11. Plant or part thereof, including seeds, obtainable by a method according to any one of items 1 to 10, wherein said plant or part thereof comprises a recombinant nucleic acid encoding a bHLH9 polypeptide.

12. Construct comprising:

(i) nucleic acid encoding a bHLH9 polypeptide as defined in items 1 or 2;
(ii) one or more control sequences capable of driving expression of the nucleic acid sequence of (a); and optionally
(iii) a transcription termination sequence.

13. Construct according to item 12, wherein one of said control sequences is a constitutive promoter, preferably a GOS2 promoter, most preferably a GOS2 promoter from rice.

14. Use of a construct according to item 12 or 13 in a method for making plants having increased yield, particularly increased early vigour and/or increased biomass and/or increased seed yield relative to control plants.

15. Plant, plant part or plant cell transformed with a construct according to item 12 or 13.

16. Method for the production of a transgenic plant having increased yield, particularly increased early vigour and/or increased biomass and/or increased seed yield relative to control plants, comprising:

(i) introducing and expressing in a plant a nucleic acid encoding a bHLH9 polypeptide as defined in item 1 or 2; and
(ii) cultivating the plant cell under conditions promoting plant growth and development; and optionally
(iii) selecting for plants having increased yield.

17. Transgenic plant having increased yield, particularly increased early vigour and/or increased biomass and/or increased seed yield, relative to control plants, resulting from modulated expression of a nucleic acid encoding a bHLH9 polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding a bHLH9 polypeptide in increasing yield, particularly in increasing early vigour and/or in increasing biomass and/or increasing seed yield in plants, relative to control plants.

22. An isolated nucleic acid molecule selected from:

(i) a nucleic acid represented by any one of SEQ ID NO: 19, 45, 47, 165, 167 and 169;
(ii) the complement of a nucleic acid represented by any one of SEQ ID NO: 19, 45, 47, 165, 167 and 169;
(iii) a nucleic acid encoding the polypeptide as represented by any one of SEQ ID NO: 20, 46, 48, 166, 168 and 170, preferably as a result of the degeneracy of the genetic code, said isolated nucleic acid can be derived from a polypeptide sequence as represented by any one of SEQ ID NO: 20, 46, 48, 166, 168 and 170 and further preferably confers enhanced yield-related traits relative to control plants;
(iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of Table A 1and Table C2 and further preferably conferring enhanced yield-related traits relative to control plants;
(v) a nucleic acid molecule which hybridizes with a nucleic acid molecule of (i) to (iv) under stringent hybridization conditions and preferably confers enhanced yield-related traits relative to control plants;
(vi) a nucleic acid encoding a bHLH9 polypeptide having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 20, 46, 48, 166, 168, 170 and any of the other amino acid sequences in Table A and Table C2 and preferably conferring enhanced yield-related traits relative to control plants.

23. An isolated polypeptide selected from:

(i) an amino acid sequence represented by any one of SEQ ID NO: 20, 46, 48, 166, 168 and 170;
(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by any one of SEQ ID NO: 20, 46, 48, 166, 168 and 170, and any of the other amino acid sequences in Table A 1and Table C2 and preferably conferring enhanced yield-related traits relative to control plants.
(iii) derivatives of any of the amino acid sequences given in (i) or (ii) above.

2. Imbibition-inducible 1 (IMB1)

1. A method for enhancing yield-related traits in plants relative to control plants, comprising modulating expression in a plant of a nucleic acid encoding an IMB1 polypeptide, wherein said IMB1 polypeptide comprises a single bromodomain.

2. Method according to item 1, wherein said IMB1polypeptide comprises one or more of the following motifs: Motif 3 (SEQ ID NO: 304), Motif 4 (SEQ ID NO: 305), Motif 5 (SEQ ID NO: 306), and Motif 6 (SEQ ID NO: 307).

3. Method according to item 2, wherein said IMB1 polypeptide additionally comprises one or more of the following motifs: Motif 7 (SEQ ID NO: 308), Motif 8 (SEQ ID NO: 309), Motif 9 (SEQ ID NO: 310), Motif 10 (SEQ ID NO: 311), and Motif 11 (SEQ ID NO: 312).

4. Method according to any of items 1 to 3, wherein said modulated expression is effected by introducing and expressing in a plant a nucleic acid encoding an IMB1 polypeptide.

5. Method according to any one of items 1 to 4, wherein said nucleic acid encoding an IMB1 polypeptide encodes any one of the proteins listed in Table A2 or is a portion of such a nucleic acid, or a nucleic acid capable of hybridising with such a nucleic acid.

6. Method according to any one of items 1 to 5, wherein said nucleic acid sequence encodes an orthologue or paralogue of any of the proteins given in Table A2.

7. Method according to any preceding item, wherein said enhanced yield-related traits comprise increased yield, preferably increased biomass and/or increased seed yield relative to control plants.

8. Method according to any one of items 1 to 7, wherein said enhanced yield-related traits are obtained under non-stress conditions.

9. Method according to any one of items 4 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding an IMB1 polypeptide is of plant origin, preferably from a dicotyledonous plant, further preferably from the family Solanaceae, more preferably from the genus *Solanum,* most preferably from *Solanum lycopersicon.*

11. Plant or part thereof, including seeds, obtainable by a method according to any one of items 1 to 10, wherein said plant or part thereof comprises a recombinant nucleic acid encoding an IMB1 polypeptide.

12. Construct comprising:

(i) nucleic acid encoding an IMB1 polypeptide as defined in any of items 1 to 3;
(ii) one or more control sequences capable of driving expression of the nucleic acid sequence of (a); and optionally
(iii) a transcription termination sequence.

13. Construct according to item 12, wherein one of said control sequences is a medium strength constitutive promoter, preferably a GOS2 promoter, most preferably a GOS2 promoter from rice.

14. Use of a construct according to item 12 or 13 in a method for making plants having increased yield, particularly increased seed yield relative to control plants.

15. Plant, plant part or plant cell transformed with a construct according to item 12 or 13.

16. Method for the production of a transgenic plant having increased yield, particularly increased biomass and/or increased seed yield relative to control plants, comprising:

(i) introducing and expressing in a plant a nucleic acid encoding an IMB1 polypeptide as defined in any of items 1 to 3; and
(ii) cultivating the plant cell under conditions promoting plant growth and development.

17. Transgenic plant having increased yield, particularly increased biomass and/or increased seed yield, relative to control plants, resulting from modulated expression of a nucleic acid encoding an IMB1 polypeptide as defined in any of items 1 to 3, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding an IMB1 polypeptide in increasing yield, particularly in increasing seed yield and/or shoot biomass in plants, relative to control plants.

3. PCD-LIKE

1. A method for enhancing yield-related traits in plants relative to control plants, comprising modulating expression in a plant of a nucleic acid encoding a PCD polypeptide comprising a Pterin-4-alpha-carbinolamine dehydratase (PCD) domain (Interpro accession number: IPR001533; pfam accession number: PF01329), said PCD domain preferably having in increasing order of preference at least 50%, 51 %, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 440.

2. Method according to item 1, wherein said PCD polypeptide comprises one or more motifs having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to any one or more of the following motifs:

(i) Motif 12 (SEQ ID NO: 441): [G/E]-[D/N]-[F/L]-G-A-R-D-P-x(3)-E-x(4)-F-G-[D/E]K;
(ii) Motif 13 (SEQ ID NO: 442): [E/D/K/H/Q/N]-x(3)-H-[H/N]-[P/C/S]-x(5,6)-[Y/W/F/H]-x(9)-[H/W]-x(8,15)-D;
(iii) Motif 14 (SEQ ID NO: 443): WK(V/L)(R/K) wherein amino acids in brackets represent alternative amino acids at the same location;
(iv) Motif 15 (SEQ ID NO: 444): TDFI;
(v) Motif 16 (SEQ ID NO: 445): (S/V)(V/I)(G/R/S/A)GL(T/S);
(vi) Motif 17 (SEQ ID NO: 446): LGDF(G/R)(A/R)(R/A)(D/G)P;
(vii) Motif 18 (SEQ ID NO:447): H(R/K)ILIP(T/A)

3. Method according to item 1 or 2, wherein said modulated expression is effected by introducing and expressing in a plant a nucleic acid encoding a PCD polypeptide.

4. Method according to any one of items 1 to 3, wherein said nucleic acid encoding a PCD polypeptide encodes any one of the proteins listed in Table A3 or is a portion of such a nucleic acid, or a nucleic acid capable of hybridising with such a nucleic acid.

5. Method according to any one of items 1 to 4, wherein said nucleic acid sequence encodes an orthologue or paralogue of any of the proteins given in Table A3.

6. Method according to any preceding item, wherein said enhanced yield-related traits comprise increased yield, preferably increased early vigour and/or increased biomass and/or increased seed yield relative to control plants.

7. Method according to any one of items 1 to 6, wherein said enhanced yield-related traits are obtained under non-stress conditions.

8. Method according to any one of items 1 to 6, wherein said enhanced yield-related traits are obtained under conditions of drought stress, salt stress or nitrogen deficiency.

9. Method according to any one of items 3 to 8, wherein said nucleic acid is operably linked to a constitutive promoter, preferably to a GOS2 promoter, most preferably to a GOS2 promoter from rice.

10. Method according to any one of items 1 to 9, wherein said nucleic acid encoding a PCD polypeptide is of plant origin, preferably from a monocotyledonous plant, further preferably from the family Poaceae, more preferably from the genus Oryza, most preferably from Oryza sativa.

11. Plant or part thereof, including seeds, obtainable by a method according to any one of items 1 to 10, wherein said plant or part thereof comprises a recombinant nucleic acid encoding a PCD polypeptide.

12. Construct comprising:

(i) nucleic acid encoding a PCD polypeptide as defined in items 1 or 2;
(ii) one or more control sequences capable of driving expression of the nucleic acid sequence of (a); and optionally
(iii) a transcription termination sequence.

13. Construct according to item 12, wherein one of said control sequences is a constitutive promoter, preferably a GOS2 promoter, most preferably a GOS2 promoter from rice.

14. Use of a construct according to item 12 or 13 in a method for making plants having increased yield, particularly increased early vigour and/or increased biomass and/or increased seed yield relative to control plants.

15. Plant, plant part or plant cell transformed with a construct according to item 12 or 13.

16. Method for the production of a transgenic plant having increased yield, particularly increased early vigour and/or increased biomass and/or increased seed yield relative to control plants, comprising:

(i) introducing and expressing in a plant a nucleic acid encoding a PCD polypeptide as defined in item 1 or 2; and
(ii) cultivating the plant cell under conditions promoting plant growth and development; and optionally
(iii) selecting for plants having increased yield.

17. Transgenic plant having increased yield, particularly increased early vigour and/or increased biomass and/or increased seed yield, relative to control plants, resulting from modulated expression of a nucleic acid encoding a PCD polypeptide as defined in item 1 or 2, or a transgenic plant cell derived from said transgenic plant.

18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.

19. Harvestable parts of a plant according to item 18, wherein said harvestable parts are preferably shoot biomass and/or seeds.

20. Products derived from a plant according to item 18 and/or from harvestable parts of a plant according to item 19.

21. Use of a nucleic acid encoding a PCD polypeptide in increasing yield, particularly in increasing early vigour and/or in increasing biomass and/or increasing seed yield in plants, relative to control plants.

4. Pseudo Response Regulator type 2 (PRR2)

1. A method for increasing yield-related traits in plants relative to control plants, comprising increasing expression in a plant of a nucleic acid sequence encoding a pseudo response regulator type 2 (PRR2) polypeptide, which PRR2 polypeptide comprises (i) a signal transduction response regulator receiver region with an InterPro entry IPR001789; and (ii) a Myb-like DNA binding region (SHAQKYF class) with an InterPro entry IPR006447, and optionally selecting for plants having increased yield-related traits.

2. Method according to item 1, wherein said PRR2 polypeptide comprises (i) in increasing order of preference at least 50%, 55%, 60%, 65%, 70%, 75%, 80%, 85%, 90%, 95%, 98%, 99% or more amino acid sequence identity to a pseudo response receiver domain as represented by SEQ ID NO: 475; and (ii) in increasing order of preference at least 50%, 55%, 60%, 65%, 70%, 75%, 80%, 85%, 90%, 95%, 98%, 99% or more amino acid sequence identity to a Myb-like DNA binding domain as represented by SEQ ID NO: 476.

3. Method according to item 2, wherein said PRR2 polypeptide further comprises in increasing order of preference at least 50%, 55%, 60%, 65%, 70%, 75%, 80%, 85%, 90%, 95%, 98%, 99% or more amino acid sequence identity to a C-terminal conserved domain as represented by SEQ ID NO: 477.

4. Method according to any preceding item, wherein said PRR2 polypeptide, when used in the construction of a response receiver domain phylogenetic tree, such as the one depicted in Figure 11, clusters with the APRR2 (pseudo response regulator type 2) group of polypeptides comprising the polypeptide sequences as represented by SEQ ID NO: 452 and SEQ ID NO: 456, rather than with any other group.

5. Method according to any preceding item, wherein said PRR2 polypeptide has in increasing order of preference at least 50%, 55%, 60%, 65%, 70%, 75%, 80%, 85%, 90%, 95%, 98%, 99% or more amino acid sequence identity to a PRR2 polypeptide as represented by SEQ ID NO: 452, or to any of the polypeptide sequences given in Table A4 herein.

6. Method according to any preceding item, wherein said nucleic acid sequence encoding a PRR2 polypeptide is represented by any one of the nucleic acid sequence SEQ ID NOs given in Table A4 or a portion thereof, or a sequence capable of hybridising with any one of the nucleic acid sequences SEQ ID NOs given in Table A4, or to a complement thereof.

7. Method according to any preceding item, wherein said nucleic acid sequence encodes an orthologue or paralogue of any of the polypeptide sequence SEQ ID NOs given in Table A4.

8. Method according to any preceding item, wherein said increased expression is effected by any one or more of: T-DNA activation tagging, TILLING, or homologous recombination.

9. Method according to any preceding item, wherein said increased expression is effected by introducing and expressing in a plant a nucleic acid sequence encoding a PRR2 polypeptide.

10. Method according to any preceding item, wherein said increased yield-related trait is one or more of: increased aboveground biomass, increased early vigor, earlier flowering, increased root biomass, increased plant height, increased seed yield per plant, increased number of filled seeds, increased total number of seeds, increased number of primary panicles, increased number of flowers per panicles, increased harvest index (HI), and increased Thousand Kernel Weight (TKW).

11. Method according to any preceding item, wherein said nucleic acid sequence is operably linked to a constitutive promoter.

12. Method according to item 11, wherein said constitutive promoter is a GOS2 promoter, preferably a GOS2 promoter from rice, most preferably a GOS2 sequence as represented by SEQ ID NO: 478.

13. Method according to any preceding item, wherein said nucleic acid sequence encoding a PRR2 polypeptide is from a plant, further preferably from a dicotyledonous plant, more preferably from the family *Solanaceae,* most preferably the nucleic acid sequence is from *Lycopersicon esculentum.*

14. Plants, parts thereof (including seeds), or plant cells obtainable by a method according to any preceding item, wherein said plant, part or cell thereof comprises an isolated nucleic acid transgene encoding a PRR2 polypeptide.

15. An isolated nucleic acid molecule selected from:

(i) a nucleic acid sequence as represented by SEQ ID NO: 459;
(ii) the complement of a nucleic acid sequence as represented by SEQ ID NO: 459;
(iii) a nucleic acid sequence encoding a PRR2 polypeptide having, in increasing order of preference, at least 50%, 55%, 60%, 65%, 70%, 75%, 80%, 85%, 90%, 95%, 96%, 97%, 98%, 99% or more amino acid sequence identity to the polypeptide sequence represented by SEQ ID NO: 460.

16. An isolated polypeptide selected from:

(i) a polypeptide sequence as represented by SEQ ID NO: 460;
(ii) a polypeptide sequence having, in increasing order of preference, at least 50%, 55%, 60%, 65%, 70%, 75%, 80%, 85%, 90%, 95%, 96%, 97%, 98%, 99% or more amino acid sequence identity to a polypeptide sequence as represented by any one of SEQ ID NO: 460;
(iii) derivatives of any of the polypeptide sequences given in (i) or (ii) above.

17. Construct comprising:

(a) a nucleic acid sequence encoding a PRR2 polypeptide as defined in any one of items 1 to 7,or 15;
(b) one or more control sequences capable of driving expression of the nucleic acid sequence of (a); and optionally
(c) a transcription termination sequence.

18. Construct according to item 17 wherein said control sequence is a consitituve promoter.

19. Construct according to item 18 wherein said consitituve promoter is a GOS2 promoter, preferably a GOS2 promoter from rice, most preferably a GOS2 sequence as represented by SEQ ID NO: 28

20. Use of a construct according to any one of items 17 to 19 in a method for making plants having increased yield-related traits relative to control plants, which increased yield-related traits are one or more of: increased aboveground biomass, increased early vigor, earlier flowering, increased root biomass, increased plant height, increased seed yield per plant, increased number of filled seeds, increased total number of seeds, increased number of primary panicles, increased number of flowers per panicles, increased harvest index (HI), and increased Thousand Kernel Weight (TKW).

21. Plant, plant part or plant cell transformed with a construct according to any one of items 17 to 19.

22. Method for the production of transgenic plants having increased yield-related traits relative to control plants, comprising:

(i) introducing and expressing in a plant, plant part, or plant cell, a nucleic acid sequence encoding a PRR2 polypeptide as defined in any one of items 1 to 7, or 15; and
(ii) cultivating the plant cell, plant part, or plant under conditions promoting plant growth and development.

23. Transgenic plant having increased yield-related traits relative to control plants, resulting from increased expression of an isolated nucleic acid sequence encoding a PRR2 polypeptide as defined in any one of items 1 to 7, or 15, or a transgenic plant cell or transgenic plant part derived from said transgenic plant.

24. Transgenic plant according to item 14, 21, or 23, wherein said plant is a crop plant or a monocot or a cereal, such as rice, maize, wheat, barley, millet, rye, triticale, sorghum and oats, or a transgenic plant cell derived from said transgenic plant.

25. Harvestable parts comprising an isolated nucleic acid sequence encoding a PRR2 polypeptide, of a plant according to item 24, wherein said harvestable parts are preferably seeds.

26. Products derived from a plant according to item 24 and/or from harvestable parts of a plant according to item 25.

27. Use of a nucleic acid sequence encoding a PRR2 polypeptide as defined in any one of items 1 to 7, or 15, in increasing yield-related traits, comprising one or more of:

increased aboveground biomass, increased early vigor, earlier flowering, increased root biomass, increased plant height, increased seed yield per plant, increased number of filled seeds, increased total number of seeds, increased number of primary panicles, increased number of flowers per panicles, increased harvest index (HI), and
increased Thousand Kernel Weight (TKW).

**Description of figures**

The present invention will now be described with reference to the following figures in which:

**Figure 1** represents a multiple sequence alignment of bHLH9 polypeptides. The position of the HLH domain is indicated over the consensus sequence (underlined sequence). The position of the 5-9-13 configuration is boxed in the consensus sequence.

**Figure 2** shows phylogenetic tree of bHLH9 polypeptides.

**Figure 3** represents the binary vector used for increased expression in Oryza sativa of a bHLH9-encoding nucleic acid under the control of a rice GOS2 promoter (pGOS2).

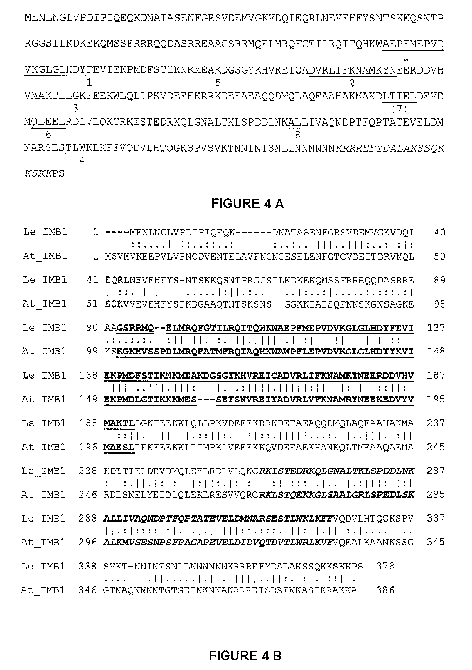

**Figure 4** represents the domain structure of the IMB1 polypeptide. Panel a shows the position of the domains in SEQ ID NO: 301. Domain 7 is not present as such in SEQ ID NO: 301, but occurs in other IMB1 proteins. The predicted Nuclear Localization Signal is shown in italics. Panel b represents an alignment between SEQ ID NO: 301 and IMB1 of Arabidopsis thaliana (SEQ ID NO: 336; Duque and Chua, 2003) with the position of the bromodomain (as identified by Pfam, bold underlined) and the NET domain indicated (as identified in Duque and Chua, 2003, bold).

**Figure 5** represents a multiple alignment of various IMB1 proteins. Conserved regions among the proteins can readily be recognised.

**Figure 6** shows a phylogenetic tree in which the group of IMB1 proteins is indicated as IMB1/GTE1.

**Figure 7** represents the binary vector used for increased expression in Oryza sativa of an IMB1-encoding nucleic acid under the control of a rice GOS2 promoter (pGOS2).

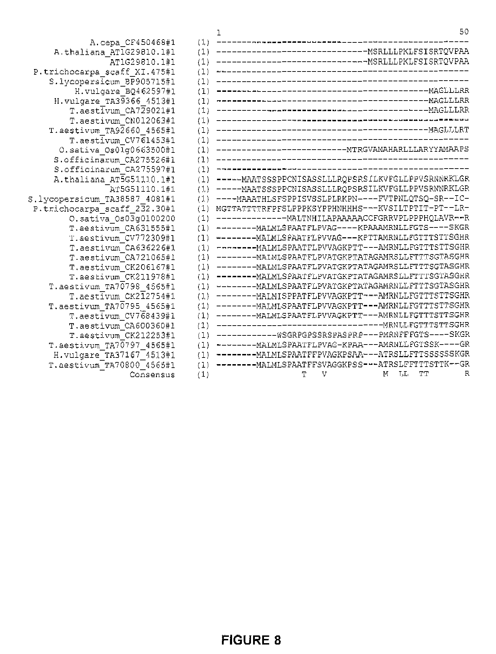

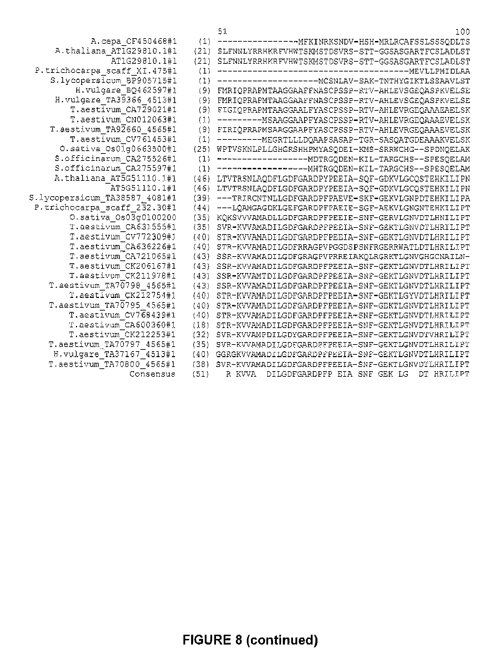

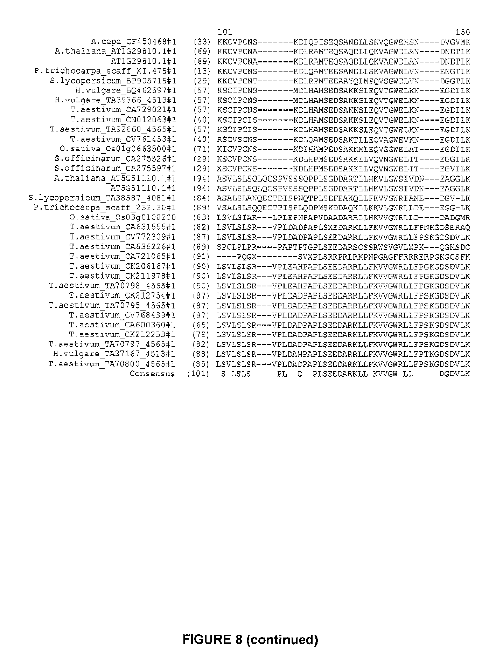

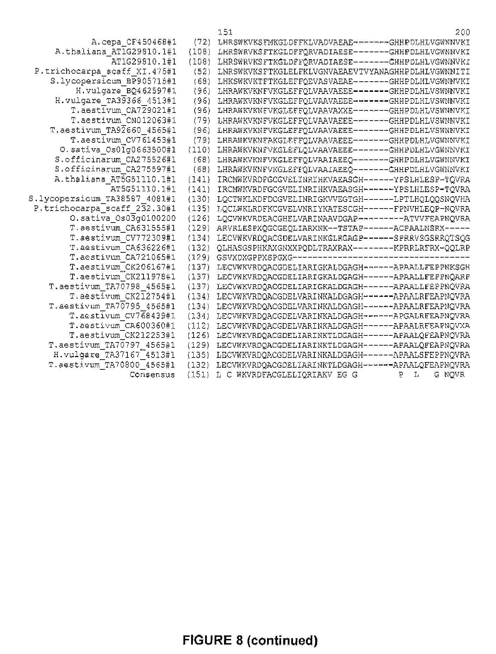

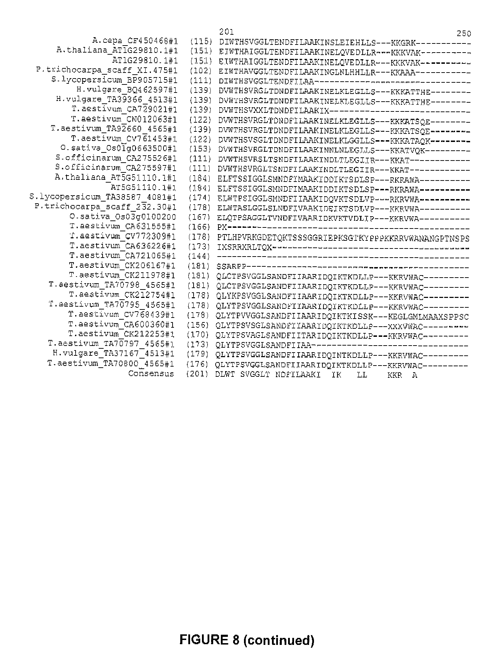

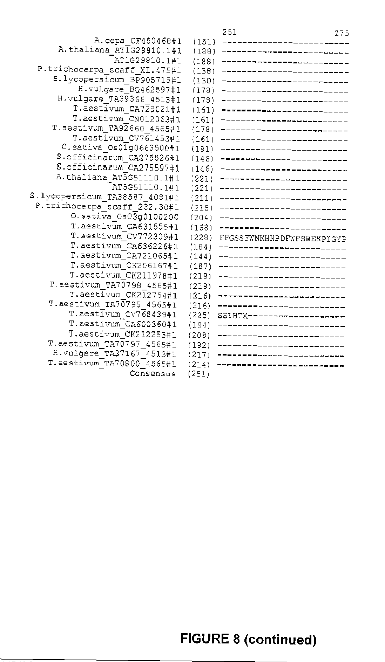

**Figure 8** represents a multiple alignment of PCD polypeptides

**Figure 9** shows a phylogenetic tree of PCD polypeptides as of Figure 3B of Naponelli et al.

**Figure 10** represents the binary vector used for increased expression in Oryza sativa of a PCD-encoding nucleic acid under the control of a rice GOS2 promoter (pGOS2)

**Figure 11** represents the phylogenetic relationship among response regulators and pseudo response regulators from *Arabidopsis thaliana,* based upon amino acid sequence of the receiver domain. Numbers indicate percentage of bootstrapped replicates that give the same branch after 1,000 iterations (according to Mason et al. (2004) Plant Phys 135: 927-937). Polypeptides useful in performing the methods of the invention cluster with APRR2, marked by a black arrow.

**Figure 12** represents a cartoon of a PRR2 polypeptide as represented by SEQ ID NO: 452, which comprises the following features: (i) a signal transduction response regulator receiver region with an InterPro entry IPR001789, where the canonical D residue of response regulator receiver region is replaed by an E residue of a pseudo response regulator receiver region; and (ii) a B motif or GARP domain or Myb-like DNA-binding region (SHAQKYF class) with an InterPro entry IPR006447.

**Figure 13** shows an AlignX (from Vector NTI 10.3, Invitrogen Corporation) multiple sequence alignment of the PRR2 polypeptides from Table A4. A signal transduction response regulator receiver region with an InterPro entry IPR001789, a B motif (or GARP domain or Myb-like DNA-binding region (SHAQKYF class) with an InterPro entry IPR006447), and a C-terminal Conserved Domain, are marked with X's below the consensus sequence. The pseudo response regulator receiver residue E is boxed.

**Figure 14** shows the binary vector for increased expression in *Oryza sativa* plants of a nucleic acid sequence encoding a PRR2 polypeptide under the control of a promoter functioning in plants.

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Identification of sequences related to the nucleic acid sequence used in the methods of the invention*

Sequences (full length cDNA, ESTs or genomic) related to the nucleic acid sequence used in the methods of the present invention were identified amongst those maintained in the Entrez Nucleotides database at the National Center for Biotechnology Information (NCBI) and other databases using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

11. basic-Helix-Loop-Helix group 9 (bHLH9)

Table A1 provides a list of nucleic acid sequences related to the nucleic acid sequence used in the methods of the present invention.

**Table A:** Examples of bHLH9 nucleic acids and polypeptides:

| Name | Nucleic Acid SEQ ID NO: | Polypeptide SEQ ID NO: |
|---|---|---|
| O.sativa_Os01g0900800 | 1 | 2 |
| A.thaliana_bHLH130_9_AT2G42280 | 3 | 4 |
| A.thaliana_bHLH122_9_AT1G51140 | 5 | 6 |
| A.thaliana_bHLH081_9_AT4G09180 | 7 | 8 |
| A.thaliana_bHLH080_9_AT1G35460 | 9 | 10 |
| A.thaliana_bHLH128_9_AT1G05805 | 11 | 12 |
| A.thaliana_bHLH129_9_AT2G43140 | 13 | 14 |
| A.formosa_TA12011_338618 | 15 | 16 |
| A.formosa_TA18296_338618 | 17 | 18 |
| B.napus_BPS_9258 | 19 | 20 |
| B.oleracea_EH428573 | 21 | 22 |
| B.oleracea_TA6610_3712 | 23 | 24 |
| C.clementina_TA5735_85681 | 25 | 26 |
| C.intybus_EH689338 | 27 | 28 |
| C.obtusa_BW987363 | 29 | 30 |
| C.sinensis_TA15236_2711 | 31 | 32 |
| C.sinensis_TA15331_2711 | 33 | 34 |
| C.solstitialis_EH761577 | 35 | 36 |
| C.tinctorius_EL407531 | 37 | 38 |
| E.esula_TA10959_3993 | 39 | 40 |
| G.hirsutum_TA24161_3635 | 41 | 42 |
| G.hybrid_AJ763309 | 43 | 44 |
| G.max_BPS_22716 | 45 | 46 |
| G.max_BPS_39182 | 47 | 48 |
| G.max_TA56453_3847 | 49 | 50 |
| G.max_TA57335_3847 | 51 | 52 |
| G.max_TA63030_3847 | 53 | 54 |
| G.raimondii_TA12344_29730 | 55 | 56 |
| G.soja_CA783858 | 57 | 58 |
| H.ciliaris_EL431737 | 59 | 60 |

(continued)

| Name | Nucleic Acid SEQ ID NO: | Polypeptide SEQ ID NO: |
|---|---|---|
| H.paradoxus_EL487892 | 61 | 62 |
| H.petiolaris_DY944559 | 63 | 64 |
| H.vulgare_TA45248_4513 | 65 | 66 |
| H.vulgare_TA47636_4513 | 67 | 68 |
| I.nil_TA15694_35883 | 69 | 70 |
| I.nil_TA8920_35883 | 71 | 72 |
| I.nil_TA9081_35883 | 73 | 74 |
| I.nil_TA9289_35883 | 75 | 76 |
| L.saligna_TA4923_75948 | 77 | 78 |
| L.sativa_TA5039_4236 | 79 | 80 |
| L.serriola_DW114802 | 81 | 82 |
| M.truncatula_AC141114_16.2 | 83 | 84 |
| M.truncatula_AC149471_36.2 | 85 | 86 |
| M.truncatula_TA37090_3880 | 87 | 88 |
| N.benthamiana_TA8284_4100 | 89 | 90 |
| N.benthamiana_TA8285_4100 | 91 | 92 |
| O.sativa_LOC_Os02g39140.1 | 93 | 94 |
| O.sativa_Os04g0489600 | 95 | 96 |
| O.sativa_Os08g0506700 | 97 | 98 |
| O.sativa_Os09g0487900 | 99 | 100 |
| P.patens_121037_e_gw1.34.393.1 | 101 | 102 |
| P.patens_148201_e_gw1.273.42.1 | 103 | 104 |
| P.patens_TA27139_3218 | 105 | 106 |
| P.patens_TA32785_3218 | 107 | 108 |
| P.taeda_TA15788_3352 | 109 | 110 |
| P.tremula_DN488299 | 111 | 112 |
| P.tremula_TA18674_47664 | 113 | 114 |
| P.trichocarpa_scaff_I.1785 | 115 | 116 |
| P.trichocarpa_scaff_III.1580 | 117 | 118 |
| P.trichocarpa_scaff_IX.1004 | 119 | 120 |
| P.trichocarpa_scaff_XIV.978 | 121 | 122 |
| P.trichocarpa_scaff_XIX.717 | 123 | 124 |
| P.trichocarpa_scaff_XVI.437 | 125 | 126 |
| R.communis_EG683575 | 127 | 128 |
| S.bicolor_TA33134_4558 | 129 | 130 |
| S.lycopersicum_TA38189_4081 | 131 | 132 |
| S.lycopersicum_TA46743_4081 | 133 | 134 |
| S.officinarum_CA143325 | 135 | 136 |

(continued)

| Name | Nucleic Acid SEQ ID NO: | Polypeptide SEQ ID NO: |
|---|---|---|
| S.ofiicinarum_TA45654_4547 | 137 | 138 |
| S.tuberosum_CV506096 | 139 | 140 |
| S.tuberosum_TA26686_4113 | 141 | 142 |
| S.tuberosum_TA29368_4113 | 143 | 144 |
| S.tuberosum_TA37004_4113 | 145 | 146 |
| S.tuberosum_TA40158_4113 | 147 | 148 |
| T.aestivum_TA88301_4565 | 149 | 150 |
| T.officinale_DY832981 | 151 | 152 |
| V.vinifera_GSVIVT00001847001 | 153 | 154 |
| V.vinifera_GSVIVT00005670001 | 155 | 156 |
| V.vinifera_GSVIVT00008845001 | 157 | 158 |
| V.vinifera_GSVIVT00024649001 | 159 | 160 |
| V.vinifera_GSVIVT00025522001 | 161 | 162 |
| V.vinifera_TA49422_29760 | 163 | 164 |
| Z.mays_BPS_3797 | 165 | 166 |
| Z.mays_BPS_30292 | 167 | 168 |
| Z.mays_BPS_52761 | 169 | 170 |
| Z.mays_TA16655_4577999 | 171 | 172 |
| Z.officinale_TA2571_94328 | 173 | 174 |
| Z.officinale_TA334_94328 | 175 | 176 |
| Z.officinale_TA5709_94328 | 177 | 178 |
| Z.officinale_TA6615_94328 | 179 | 180 |

1.2. Imbibition-inducible 1 (IMB1)

Table A2 provides a list of nucleic acid sequences related to the nucleic acid sequence used in the methods of the present invention.

**Table A2:** Examples of IMB1 nucleic acids and polypeptides:

| Plant Source | Nucleic acid SEQ ID NO: | Protein SEQ ID NO: |
|---|---|---|
| Arabidopsis thaliana_AT2G34900_GTE1 | 314 | 336 |
| Arabidopsis thaliana_AT3G52280_GTE6 | 315 | 337 |
| Centaurea solstitialis_TA2829 | 316 | 338 |
| Gossypium hirsutum_TA31156 | 317 | 339 |
| Gossypium raimondii_CO070884 | 318 | 340 |
| Gossypium raimondii_TA13411 | 319 | 341 |
| Lactuca saligna_DW067496 | 320 | 342 |
| Medicago truncatula_AC174355 | 321 | 343 |
| Medicago truncatula_GTE1302 | 322 | 344 |
| Oryza sativa_GTE2201 | 323 | 345 |

(continued)

| Plant Source | Nucleic acid SEQ ID NO: | Protein SEQ ID NO: |
|---|---|---|
| Oryza sativa_GTE701 | 324 | 346 |
| Physcomitrella patens_GTE 1501 | 325 | 347 |
| Physcomitrella patens_GTE1502 | 326 | 348 |
| Physcomitrella patens_GTE1505 | 327 | 349 |
| Populus trichocarpa_GTE907 | 328 | 350 |
| Populus trichocarpa_GTE908 | 329 | 351 |
| Populus trichocarpa_GTE909 | 330 | 352 |
| Sorghum bicolor_TA26028 | 331 | 353 |
| Triticum aestivum_TA83944 | 332 | 354 |
| Vitis vinifera_GSVIVT00002627001 | 333 | 355 |
| Vitis vinifera_GSVIVT00008344001 | 334 | 356 |
| Zea mays_GTE110 | 335 | 357 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

1.3. PCD-LIKE

Table A3 provides a list of nucleic acid sequences related to the nucleic acid sequence used in the methods of the present invention.

**Table A3:** Examples of PCD nucleic acids and polypeptides:

| Name | Nucleic acid SEQ ID NO: | Polypeptide SEQ ID NO: |
|---|---|---|
| O.sativa_Os03g0100200 | 358 | 359 |
| A.cepa_CF450468#1 | 360 | 361 |
| A.thaliana_AT1G29810.1#1 | 362 | 363 |
| A.thaliana_AT5G51110.1#1 | 364 | 365 |
| AT1G29810.1#1 | 366 | 367 |
| AT5G51110.1#1 | 368 | 369 |
| H.vulgare_BQ462597#1 | 370 | 371 |
| H.vulgare_TA37167_4513#1 | 372 | 373 |
| H.vulgare_TA39366_4513#1 | 374 | 375 |
| M.truncatula_TA24237_3880#1 | 376 | 377 |
| O.sativa_Os01g0390600#1 | 378 | 379 |
| O.sativa_Os01g0663500#1 | 380 | 381 |
| P.trichocarpa_DCOH like | 382 | 383 |
| P.trichocarpa_scaff_232.30#1 | 384 | 385 |
| P.trichocarpa_scaff_XI.475#1 | 386 | 387 |
| S-lycopersicum_BP905715#1 | 388 | 389 |

(continued)

| Name | Nucleic acid SEQ ID NO: | Polypeptide SEQ ID NO: |
|---|---|---|
| S.lycopersicum_DB718111#1 | 390 | 391 |
| S.lycopersicum_TA38587_4081#1 | 392 | 393 |
| S.officinarum_CA113975#1 | 394 | 395 |
| S.officinarum_CA275526#1 | 396 | 397 |
| S.officinarum_CA275597#1 | 398 | 399 |
| T.aestivum_CA600360#1 | 400 | 401 |
| T.aestivum_CA631555#1 | 402 | 403 |
| T.aestivum_CA636226#1 | 404 | 405 |
| T.aestivum_CA678224#1 | 406 | 407 |
| T.aestivum_CA721065#1 | 408 | 409 |
| T.aestivum_CA729021#1 | 410 | 411 |
| T.aestivum_CK206167#1 | 412 | 413 |
| T.aestivum_CK211978#1 | 414 | 415 |
| T.aestivum_CK212253#1 | 416 | 417 |
| T.aestivum_CK212754#1 | 418 | 419 |
| T.aestivum_CN012063#1 | 420 | 421 |
| T.aestivum_CV761453#1 | 422 | 423 |
| T.aestivum_CV768439#1 | 424 | 425 |
| T.aestivum_CV772309#1 | 426 | 427 |
| T.aestivum_TA70795_4565#1 | 428 | 429 |
| T.aestivum_TA70797_4565#1 | 430 | 431 |
| T.aestivum_TA70798_4565#1 | 432 | 433 |
| T.aestivum_TA70799_4565#1 | 434 | 435 |
| T.aestivum_TA70800_4565#1 | 436 | 437 |
| T.aestivum_TA92660_4565#1 | 438 | 439 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

1.4. Pseudo Response Regulator type 2 (PRR2)

Table A4 provides a list of nucleic acid sequences related to the nucleic acid sequence used in the methods of the present invention.

**Table A4:** Examples of PRR2 nucleic acids and polypeptide sequences

| Name | Public database accession number | Nucleic acid SEQ ID NO: | Polypeptide SEQ ID NO: |
|---|---|---|---|
| Lyces_PRR2 | NA | 451 | 452 |

(continued)

| Name | Public database accession number | Nucleic acid SEQ ID NO: | Polypeptide SEQ ID NO: |
|---|---|---|---|
| Aqufo_PRR2 | DT734703<br>DR929648.1<br>DR949155.1 | 453 | 454 |
| Arath_APRR2 | At4g18020 | 455 | 456 |
| Eucgr_PRR2 | NA | 457 | 458 |
| Glyma_PRR2 | BE330069.1<br>BE659830.1<br>BE661803.1<br>FK011978.1<br>proprietary | 459 | 460 |
| Lotja_PRR2 | AP004489 | 461 | 462 |
| Lyces_PRR2 II | AC226015 | 463 | 464 |
| Medtr_PRR2 | AC144516.5 | 465 | 466 |
| Poptr_PRR2 I | lcl_scaff_29.152 | 467 | 468 |
| Poptr_PRR2 II | lcl_scaff_118.53 | 469 | 470 |
| Vitvi_PRR2 | AM443941 | 471 | 472 |
| Orysa_PRR2 like | AK242722 | 473 | 474 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

***Example 2: Alignment of sequences related to the polypeptide sequences used in the methods of the invention***

2.1. basic-Helix-Loop-Helix group 9 (bHLH9)

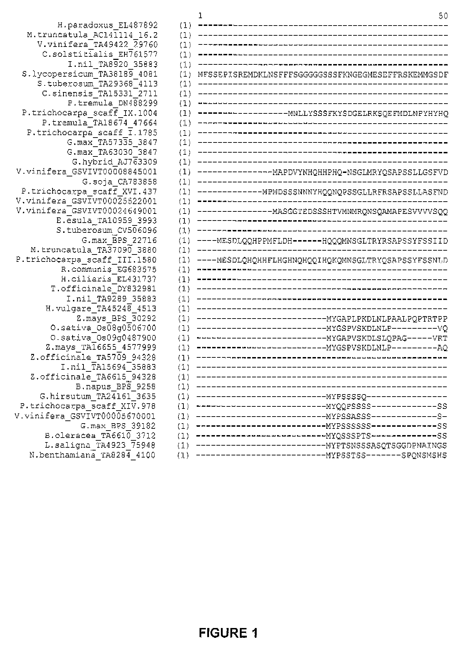

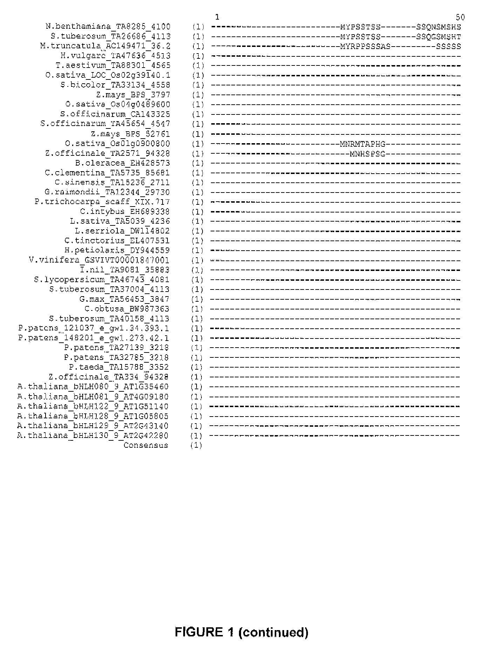

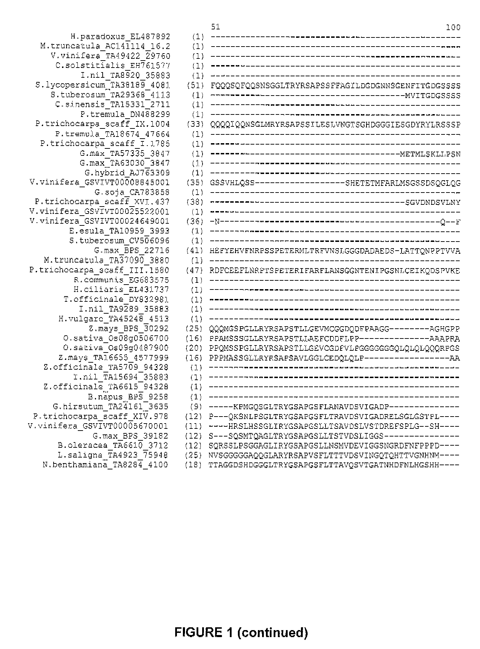

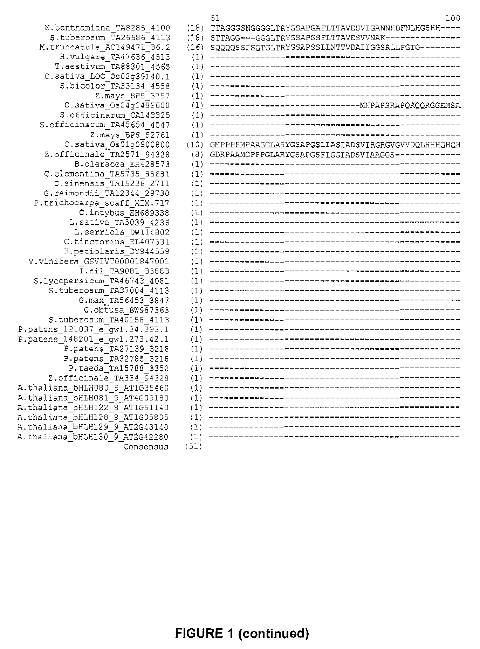

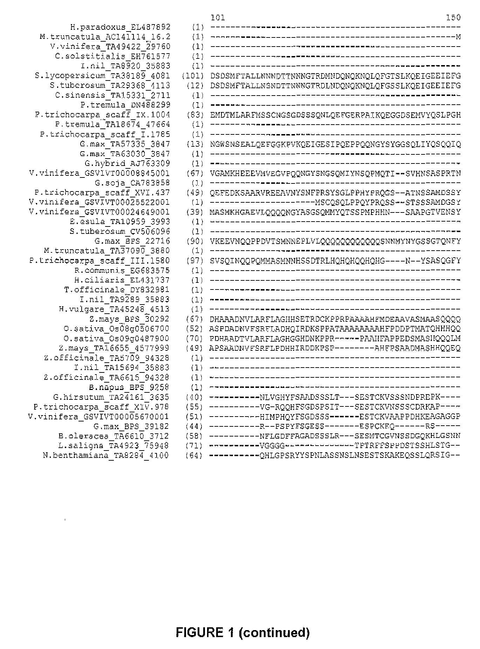

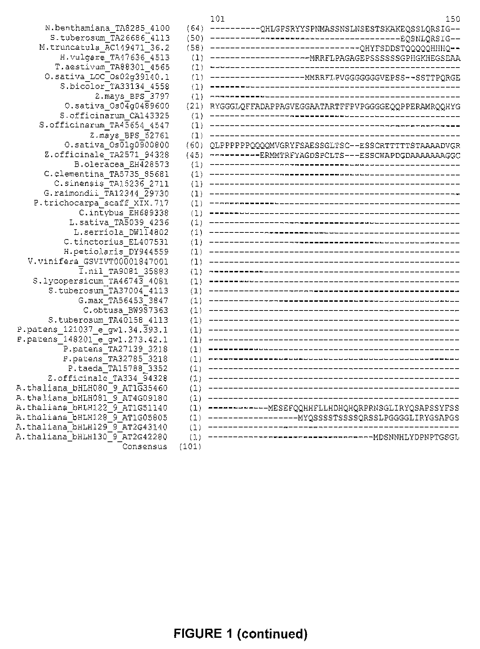

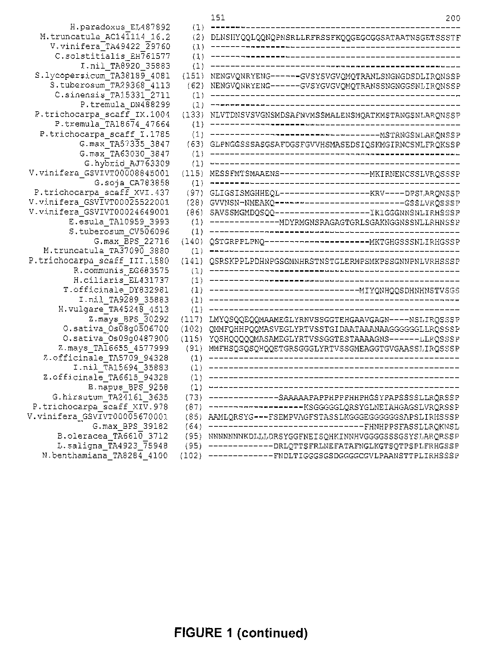

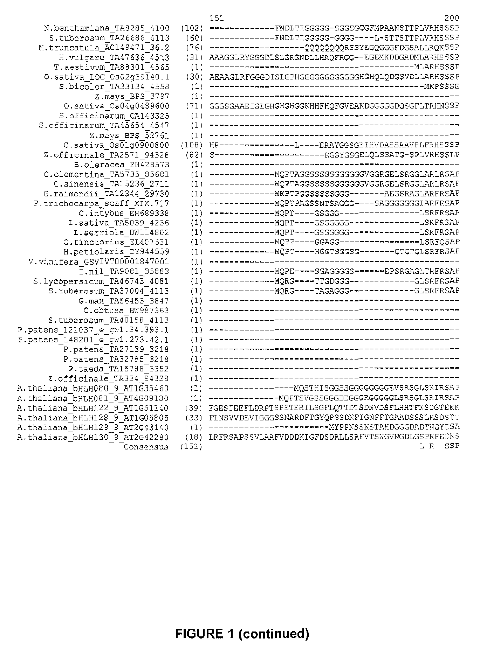

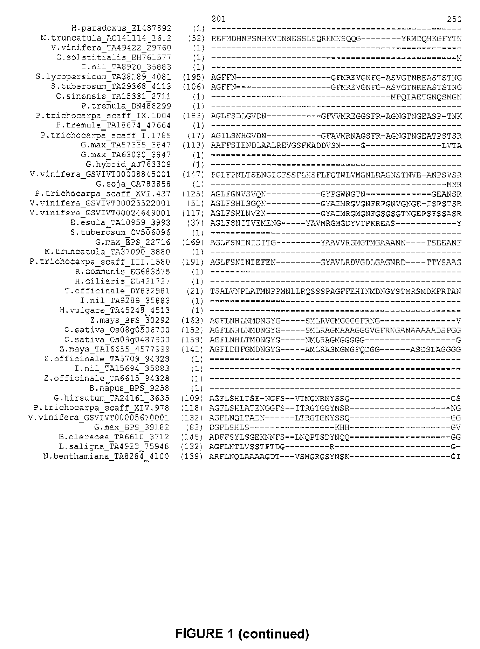

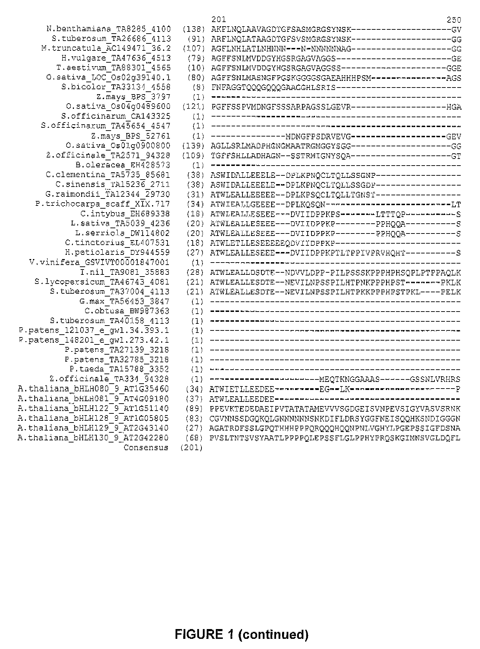

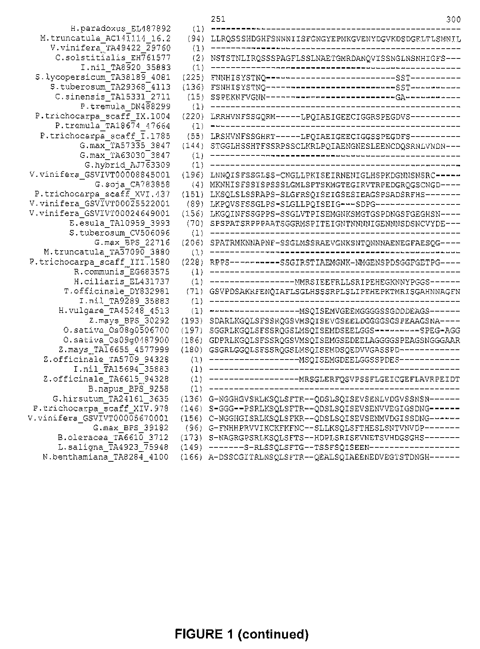

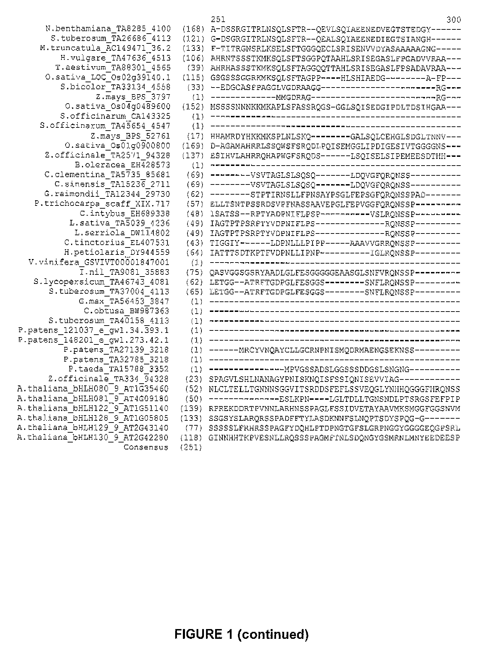

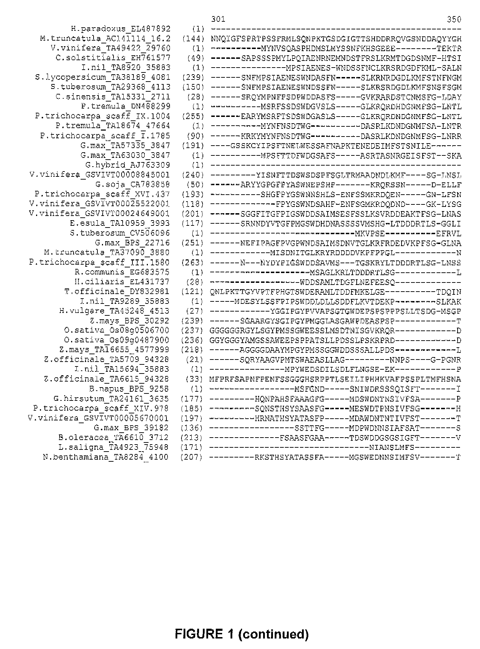

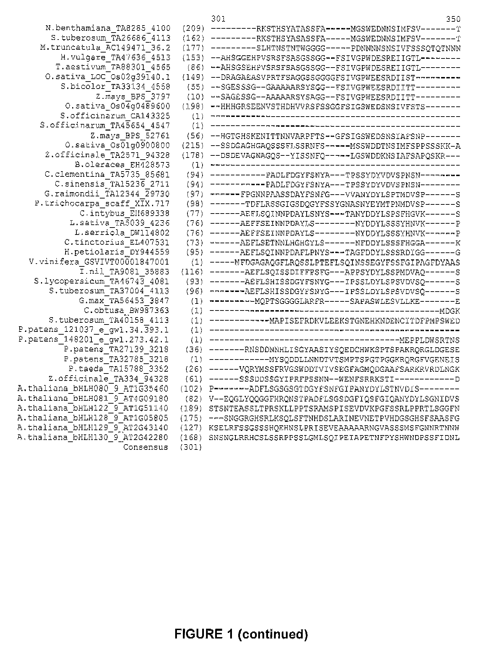

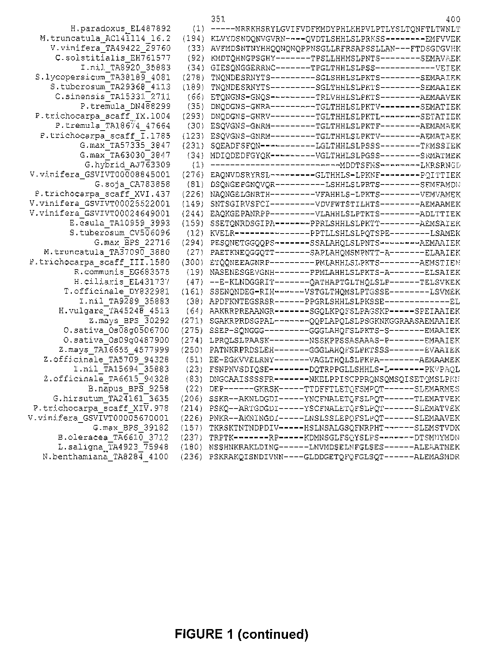

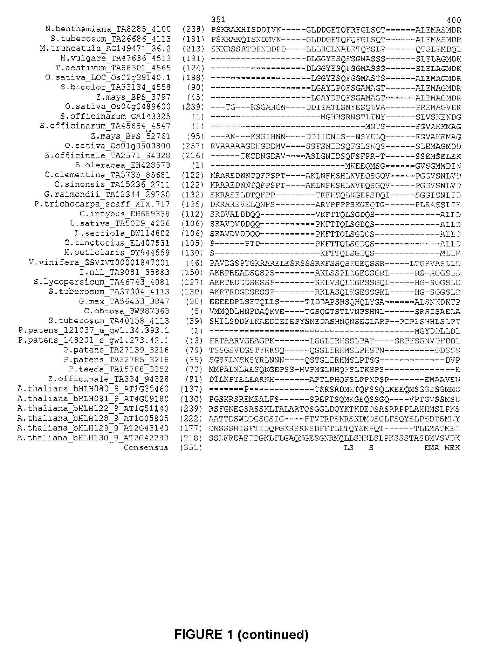

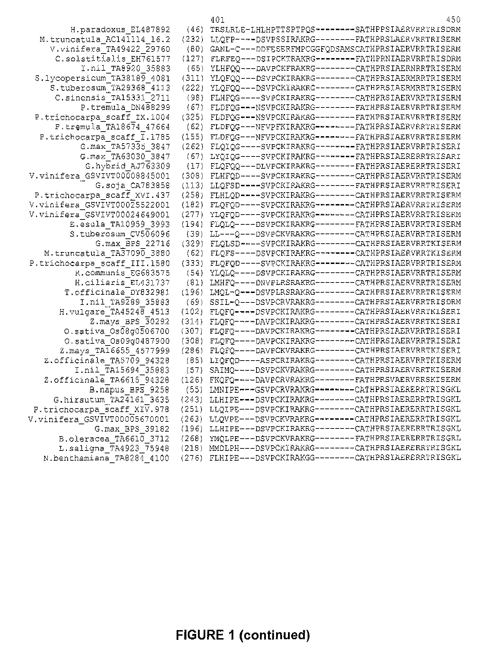

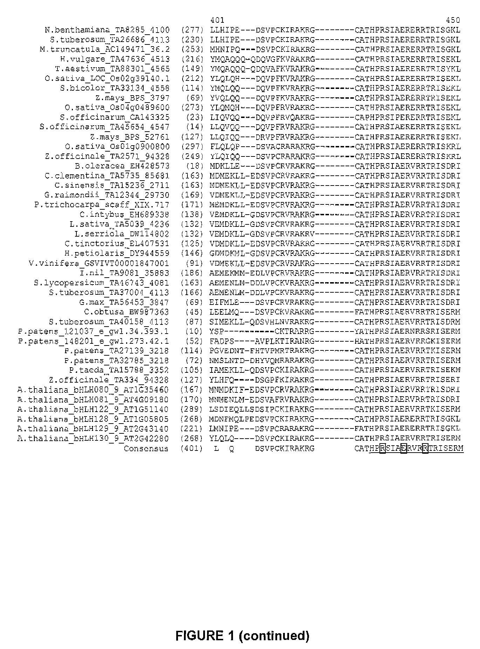

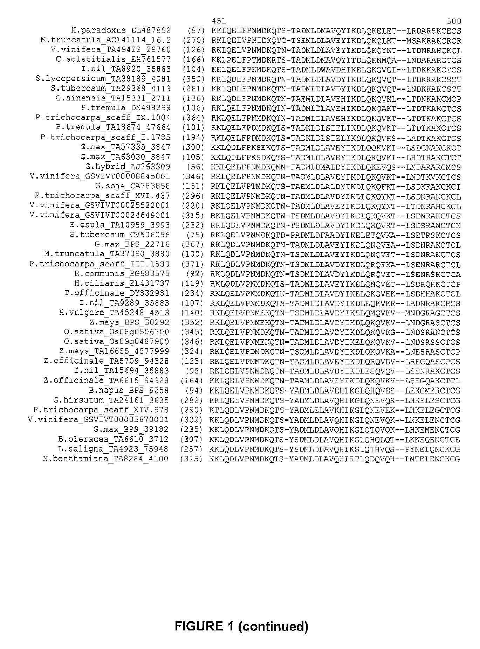

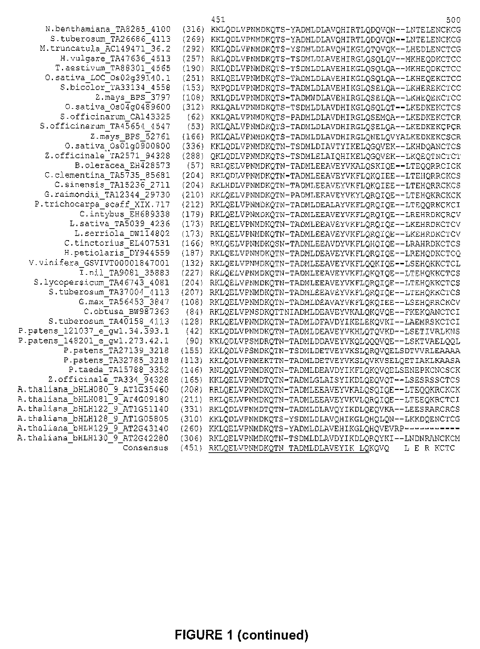

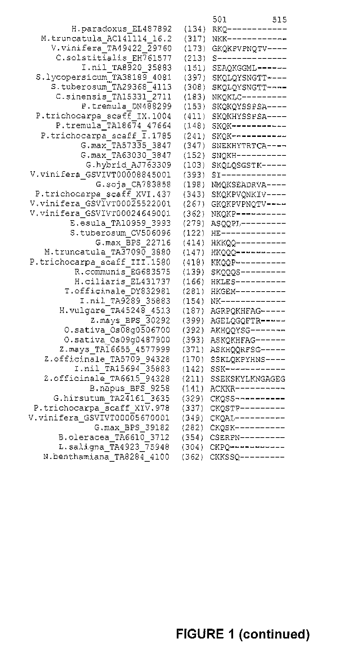

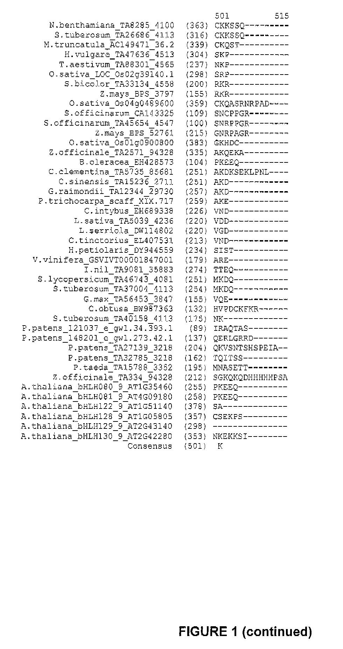

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Sequence conservation among bHLH9 polypeptides is essentially in the HLH domain of the polypeptides. The bHLH9 polypeptides are aligned in Figure 1.

A phylogenetic tree of bHLH9 polypeptides (Figure 2) was constructed using a neighbour-joining clustering algorithm as provided in the AlignX programme from the Vector NTI (Invitrogen). As shown in the tree SEQ ID NO: 2 (O.sativa_Os01g0900800) clusters within the bHLH9 clade (group) defined by defined by A.thaliana bHLH130 9 AT2G42280; A.thaliana bHLH122 9 AT1G51140; A.thaliana bHLH081 9 AT4G09180; A.thaliana bHLH080 9 AT1G35460; A.thaliana bHLH128 9 AT1G05805; and A.thaliana bHLH129 9 AT2G43140.

The sequences used in the construction of the tree are SEQ ID NO: 2, 276, 277, 278, 279, 280, 281, 282, 283, 284, 285, 286, 287, 288, 289, 290, 291, 292, 293, 294, 295, 296, 297, 298 and 299. It should be noted that sequences represented by SEQ ID NO: 276, 277, 278, 279, 280, 281, 282, 283, 284, 285, 286, 287, 288, 289, 290, 291, 292, 293, 294, 295, 296, 297, 298 and 299 represent bHLH proteins that do not belong to the bHLH9 clade (group) and therefore are not part of the invention.

2.2. Imbibition-inducible 1 (IMB1)

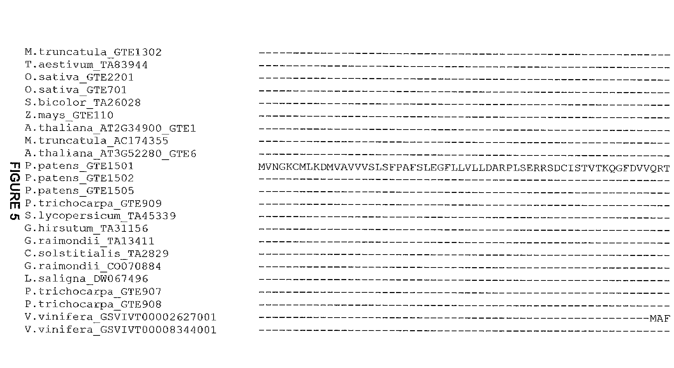

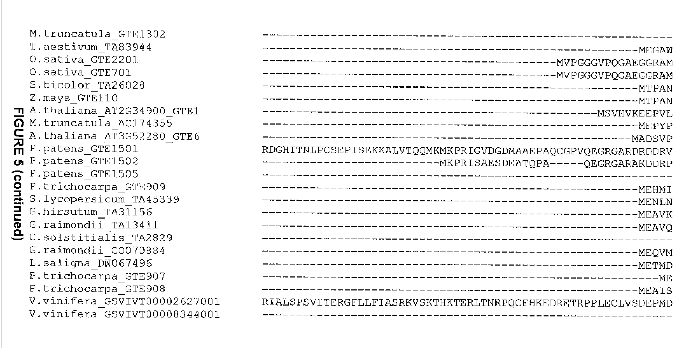

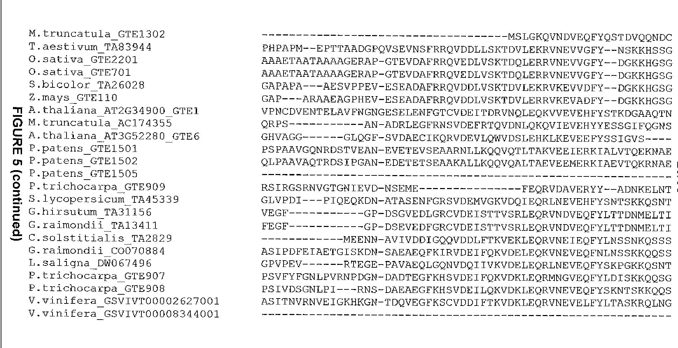

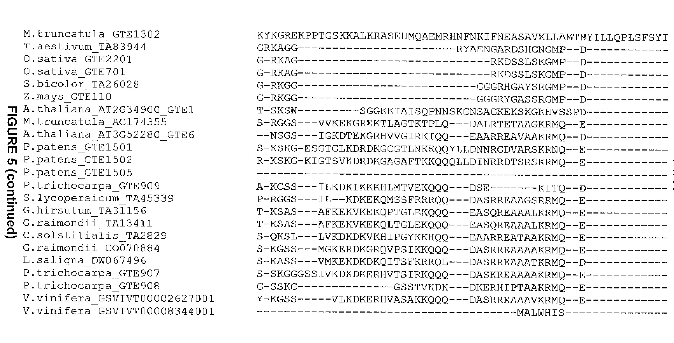

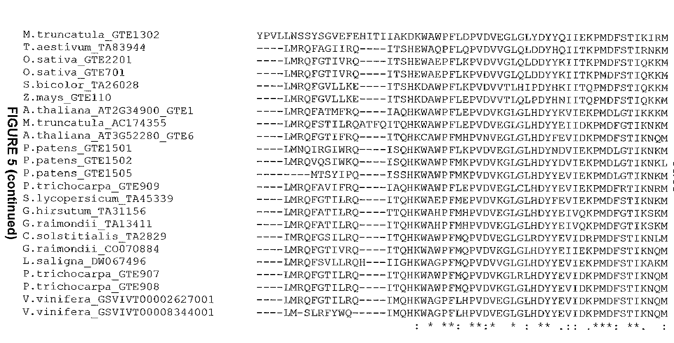

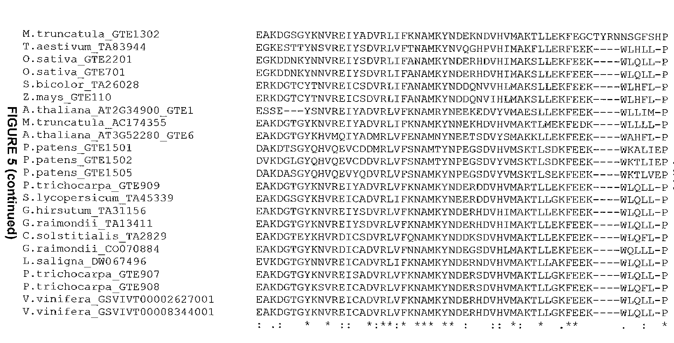

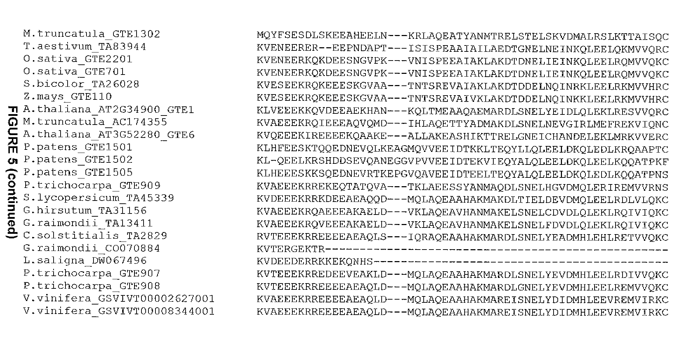

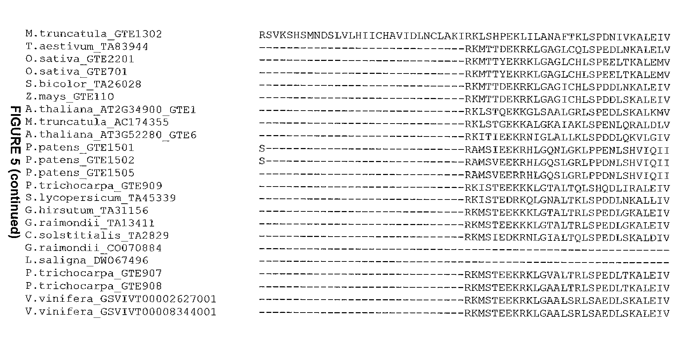

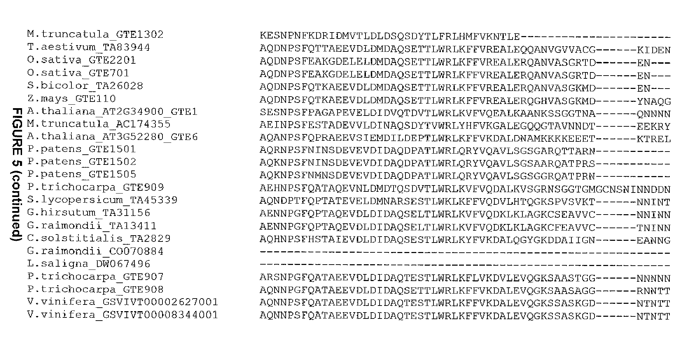

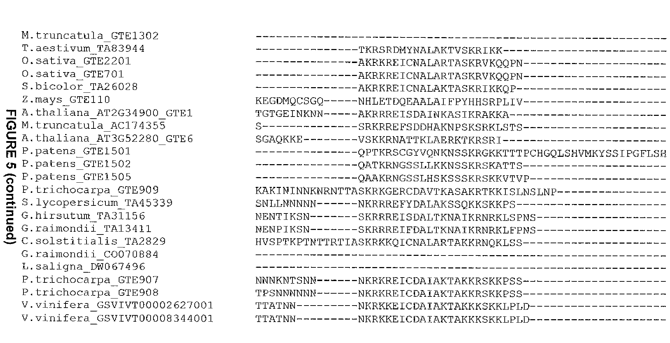

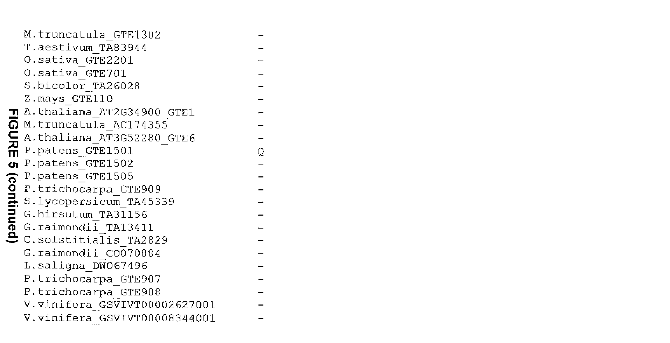

Alignment of polypeptide sequences was performed using the MUSCLE 3.7 program (Edgar, Nucleic Acids Research 32, 1792-1797, 2004). Default values are for the gap open penalty of 10, for the gap extension penalty of 0,1 and the selected weight matrix is Blosum 62 (if polypeptides are aligned). Minor manual editing was done to further optimise the alignment. Sequence conservation among IMB1 polypeptides is essentially in the bromodomain of the polypeptides, and in the NET domain, while the remainder of the protein sequence is usually more variable in sequence length and composition. The IMB1 polypeptides are aligned in Figure 5.

A phylogenetic tree of IMB1 polypeptides (Figure 6) was constructed: The proteins were aligned using MUSCLE (Edgar (2004), Nucleic Acids Research 32(5): 1792-97). A Neighbour-Joining tree was calculated using QuickTree (Howe et al. (2002), Bioinformatics 18(11): 1546-7). Support for the major branching after 100 bootstrap repetitions is indicated. A circular cladogram was drawn using Dendroscope (Huson et al. (2007), BMC Bioinformatics 8(1): 460). 4 different subclasses of GTE can be defined with good statistical support. Other GTEs and bromodomain proteins are difficult to assign to any class with confidence.

A MEME analysis (Bailey et al. Nucleic Acids Research 34, W369-W373, 2006) using the sequences listed in table A provided the following motifs:

```
Motif 3:

Multilevel        QITQHKWAWPFLKPVDVEGLGLHDYYEVIEKPMDFSTIKNKMEAKDGTGY
consensus            S       MQ    K         I D     G   KQ      S
sequence                      E    V           T
                             H

Motif 3 in SEQ ID NO: 301 starts at position 109:

Motif 4:

Multilevel        REICADVRLVFKNAMKYNDERHDVHVMAKTLLEKFEEKWLQLLPKVAEEE
consensus            YS    I          GS   I   S  G        F    E
sequence

Motif 4 in SEQ ID NO: 301 starts at position 162

Motif 5:

Multilevel        MQLAQEAAHAKMAKDLSNELYEIDMQLEELREMVVQKCRKMSTEEKRKLG
consensus         V  S    I  LTRET      VNKH     Q  I R    T    K
sequence                       E

Motif 5 in SEQ ID NO: 301 starts at position 225

Motif 6:

Multilevel        LTRLSPEDLSKALEIVAQNNPSFQATAEEVDLDIDAQSESTLWRLKFFVK
consensus          CK   DN   V     ED        D  E  M     T         Q
sequence           G                                               R

Motif 6 in SEQ ID NO: 301 starts at position 277
```

2.3. PCD-LIKE

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Sequence conservation among PCD polypeptides is essentially in the C-terminal PCD domain of the polypeptides, the N-terminal domain usually being more variable in sequence length and composition. The PCD polypeptides are aligned in Figure 9.

2.4. Pseudo Response Regulator type 2 (PRR2)

Mutliple sequence alignment of all the PRR2 polypeptide sequences in Table A4 was performed using the AlignX algorithm (from Vector NTI 10.3, Invitrogen Corporation). Results of the alignment are shown in Figure 3 of the present application. A signal transduction response regulator receiver region with an InterPro entry IPR001789, a B motif (or GARP domain or Myb-like DNA-binding region (SHAQKYF class) with an InterPro entry IPR006447), and a C-terminal Conserved Domain, are marked with X's below the consensus sequence. The pseudo response regulator receiver residue E is boxed.

***Example 3: Calculation of global percentage identity between polypeptide sequences useful in performing the methods of the invention***

3.1. basic-Helix-Loop-Helix group 9 (bHLH9)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62
First Gap: 12
Extending gap: 2

Results of the software analysis are shown in Table B1 for the global similarity and identity over the full length of the polypeptide sequences. Percentage identity is given below the diagonal and percentage similarity is given above the diagonal.

The percentage identity between the bHLH9 polypeptide sequences of Table B1 can be as low as 21.2 % amino acid identity compared to SEQ ID NO: 2.

**Table B1:** MatGAT results for global similarity and identity over the full length of the polypeptide sequences.

| | | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|---|
| 1 | A.thaliana_ bHLH130_9_AT2G42280 | | 37,4 | 21,7 | 36,5 | 20,7 | 36,8 | 32,5 | 30,9 | 27,6 | 32,2 | 29,8 | 16,4 | 34,2 | 26,8 | 25,3 | 22,2 |
| 2 | A.thaliana_ bHLH122_9_AT1G51140 | 53,6 | | 18,8 | 29,8 | 18,1 | 40,5 | 28,7 | 30,3 | 25,5 | 29,3 | 30,3 | 16,7 | 26 | 24,7 | 23,7 | 21,6 |
| 3 | B.napus_BPS_9258 | 27,9 | 26,9 | | 30,6 | 40,7 | 19,3 | 35 | 27,6 | 46,4 | 28,8 | 21,4 | 34,7 | 31,4 | 35,3 | 41 | 52,8 |
| 4 | G.hirsutum_TA24161_3635 | 50,4 | 45,4 | 35,7 | | 20,4 | 30,5 | 54,3 | 33,5 | 26,7 | 37,7 | 41,1 | 16,1 | 32,1 | 30,9 | 29,1 | 28,7 |
| 5 | G.hybrid_AJ763309 | 24,8 | 23 | 54,5 | 24 | | 16,8 | 22,7 | 19,8 | 45,2 | 20,5 | 17,3 | 47,3 | 28,4 | 24,4 | 32,4 | 35,4 |
| 6 | G.max_BPS_22716 | 52,9 | 55,3 | 25,1 | 44,7 | 20,6 | | 28,6 | 29,2 | 30,1 | 30 | 32,3 | 14,8 | 30,9 | 26,8 | 22,2 | 21,7 |
| 7 | G.max_BPS_39182 | 45,7 | 41,2 | 42 | 64,6 | 27,6 | 41,6 | | 37,1 | 28,3 | 36,2 | 35,8 | 19,1 | 32,4 | 35,5 | 28,7 | 30,1 |
| 8 | H.vulgare_TA47636_4513 | 44,6 | 44,3 | 34,3 | 51,4 | 25,8 | 41,1 | 50,3 | | 26,6 | 61,4 | 33,8 | 16,1 | 30,7 | 72,7 | 28,1 | 39,5 |
| 9 | M.truncatula_TA37090_3880 | 35,4 | 30,6 | 63,6 | 32,4 | 57 | 33,5 | 37,8 | 35,3 | | 26,8 | 22 | 38,6 | 35,6 | 32,8 | 38,8 | 42,5 |
| 10 | O.sativa_LOC_Os02g39140.1 | 43,7 | 40,9 | 35 | 49,5 | 27 | 40,9 | 48,7 | 74,8 | 37,7 | | 35,6 | 17,8 | 30,7 | 53 | 30,3 | 42 |
| 11 | O.sativa_Os01g0900800 | 45 | 45 | 27,4 | 54,5 | 21,2 | 46,4 | 45,5 | 46,8 | 28,7 | 43,9 | | 14,7 | 27,1 | 29,4 | 24,3 | 24 |
| 12 | P.patens_121037_e_ gw1.34.393.1 | 20,3 | 19,5 | 47,6 | 21,6 | 61,6 | 17,7 | 24,8 | 22,5 | 47 | 23 | 17,8 | | 22,3 | 20,6 | 31,4 | 31,1 |
| 13 | P.trichocarpa_scaff_I.1785 | 44,6 | 39,8 | 41 | 44,1 | 36,1 | 39,2 | 48,6 | 46,7 | 45,1 | 45,3 | 37,2 | 29,5 | | 37 | 34,3 | 30,3 |
| 14 | T.aestivum_TA88301_4565 | 37 | 37,2 | 44,4 | 43,5 | 33,1 | 38,5 | 47,6 | 74,5 | 44,4 | 64 | 37,2 | 28 | 56,6 | | 33,5 | 49,4 |
| 15 | V.vinifera_GSVIVT00001 847001 | 34,8 | 31,9 | 56,4 | 38,7 | 44,2 | 30,6 | 40,2 | 38,6 | 53,6 | 41,3 | 30,7 | 40,3 | 49,2 | 47,3 | | 38,5 |
| 16 | Z.mays_BPS_3797 | 29,5 | 28,5 | 66,2 | 35,4 | 51 | 29,4 | 39,2 | 45,1 | 61,1 | 46,7 | 30,2 | 41,4 | 43,9 | 56,9 | 55,8 | |

3.2. Imbibition-inducible 1 (IMB1)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62
First Gap: 12
Extending gap: 2

Results of the software analysis are shown in Table B2 for the global similarity and identity over the full length of the polypeptide sequences. Percentage identity is given above the diagonal in bold and percentage similarity is given below the diagonal (normal face).

The percentage identity between the IMB1 polypeptide sequences useful in performing the methods of the invention can be as low as 31 % amino acid identity compared to SEQ ID NO: 301. In average, the identity within the cluster of IMB1/GTE1 proteins is 47%; the maximal identity with proteins outside this cluster is 30% and the minimal identity is 8%.

**Table B2:** MatGAT results for global similarity and identity over the full length of the polypeptide sequences.

| | 1 | 2 | 3 | 4 | 5 | 6 | 7 | 8 | 9 | 10 |
|---|---|---|---|---|---|---|---|---|---|---|
| **1 A.thaliana_AT2G34900_GTE1** | | **40,1** | **44,6** | **51,4** | **28,4** | **51,4** | **29,2** | **45,6** | **31** | **45,3** |
| **2 A.thaliana_AT3G52280_GTE6** | 58,8 | | **50** | **48,7** | **36,4** | **48,4** | **30,9** | **44,2** | **29,1** | **39,6** |
| **3 C.solstitialis_TA2829** | 65 | 68,9 | | **59,7** | **36,7** | **59,4** | **34,3** | **51,2** | **32,6** | **45,3** |
| **4 G.hirsutum_TA31156** | 69,4 | 67,6 | 77,3 | | **35,3** | **96,5** | **35,8** | **56,6** | **34** | **47,9** |
| **5 G.raimondii_CO070884** | 37,3 | 44,2 | 46,9 | 42,5 | | **36,6** | **61,4** | **35** | **23,5** | **27,6** |
| **6 G.raimondii_TA13411** | 69,7 | 66,3 | 77,3 | 96,8 | 44,1 | | **35,5** | **56,6** | **34,9** | **46,8** |
| **7 L.saligna_DW067496** | 40,7 | 42 | 45,3 | 45,5 | 75,2 | 45,5 | | **34,5** | **25,3** | **28,5** |
| **8 M.truncatula_AC174355** | 66,8 | 64 | 70,2 | 72,7 | 43,4 | 72,7 | 43,1 | | **36** | **45,3** |
| **9 M.truncatula_GTE1302** | 49,2 | 49,1 | 53,4 | 53,7 | 34 | 52,9 | 36,5 | 52,6 | | **30** |
| **10 O.sativa_GTE2201** | 63,5 | 61,5 | 64,9 | 63,6 | 38,1 | 62,6 | 39,2 | 64,5 | 48,6 | |
| **11 O.sativa_GTE701** | 63,5 | 61,5 | 64,9 | 63,6 | 38,1 | 62,6 | 39,2 | 64,5 | 48,6 | 100 |

| | | | | | | | | | | |
|---|---|---|---|---|---|---|---|---|---|---|
| 12 P.patens_GTE1501 | 44,3 | 42,9 | 43,1 | 44,3 | 30,9 | 44,3 | 28,8 | 43,9 | 35,6 | 40,4 |
| 13 P.patens_GTE1502 | 54,8 | 57,1 | 57,6 | 56,8 | 40,4 | 55,8 | 38 | 55,8 | 43,7 | 54,3 |
| 14 P.patens_GTE1505 | 45,9 | 45,5 | 50,1 | 48,4 | 29,9 | 47,9 | 34 | 49,3 | 41,2 | 46,4 |
| 15 P.trichocarpa_GTE907 | 69,2 | 66,9 | 79,4 | 77,3 | 47,1 | 77,6 | 45,8 | 71,6 | 49,5 | 65,1 |
| 16 P.trichocarpa_GTE908 | 71,2 | 66,8 | 80,4 | 75,9 | 47 | 76,5 | 45,9 | 72,7 | 53,8 | 69,5 |
| 17 P.trichocarpa_GTE909 | 66,8 | 62,3 | 72,1 | 76,1 | 40,3 | 75,9 | 41,6 | 70,3 | 51,2 | 59,4 |
| 18 S.bicolor_TA26028 | 62,4 | 61,2 | 65,4 | 61 | 38,4 | 62,3 | 39,3 | 61,8 | 48,2 | 81,7 |
| 19 S.lycopersicum_TA45339 | 70,5 | 67,5 | 78,8 | 77,5 | 46,8 | 78 | 49,5 | 71,2 | 51,3 | 67,7 |
| 20 T.aestivum_TA83944 | 61,4 | 58,5 | 64,1 | 61,5 | 38,2 | 64,4 | 40,2 | 62,1 | 48,5 | 80,3 |
| 21 V.vinifera_GSVIVT02627001 | 63,9 | 57,3 | 70,3 | 68 | 40,4 | 68,7 | 40 | 63,9 | 46,6 | 61,2 |
| 22 V.vinifera_GSVIVT08344001 | 57 | 53,9 | 64,1 | 63,6 | 34,5 | 63,6 | 39,1 | 59,1 | 48,2 | 59,4 |
| 23 Z.mays_GTE110 | 59,6 | 60,4 | 61,9 | 59,1 | 36,9 | 60,7 | 38,3 | 59,6 | 47,8 | 77,2 |

| | 11 | 12 | 13 | 14 | 15 | 16 | 17 | 18 | 19 | 20 |
|---|---|---|---|---|---|---|---|---|---|---|
| 1 A.thaliana_AT2G34900_GTE1 | 45,3 | 30,6 | 32,5 | 32,8 | 51,6 | 52,5 | 47,2 | 42,3 | 49 | 44,2 |
| 2 A.thaliana_AT3G52280_GTE6 | 39,6 | 30,3 | 36,1 | 29,8 | 53,4 | 52,5 | 42 | 42,5 | 50 | 42,4 |
| 3 C.solstitialis_TA2829 | 45,3 | 30,3 | 36,7 | 33,6 | 65,4 | 67,7 | 51,6 | 46,5 | 56,3 | 47,3 |
| 4 G.hirsutum_TA31156 | 47,9 | 29 | 37,3 | 32,5 | 64,5 | 62,1 | 57,7 | 44,7 | 59,5 | 46 |
| 5 G.raimondii_CO070884 | 27,6 | 22,1 | 27,5 | 14,9 | 41,6 | 39,5 | 30,3 | 26,1 | 37,3 | 28,1 |
| 6 G.raimondii_TA13411 | 46,8 | 29,3 | 37,6 | 32,5 | 64,8 | 62,7 | 57,4 | 45,3 | 59,7 | 46,9 |
| 7 L.saligna_DW067496 | 28,5 | 21,2 | 27,3 | 12,8 | 38,7 | 36,6 | 31,6 | 27,9 | 40,2 | 28,9 |
| 8 M.truncatula_AC174355 | 45,3 | 30,3 | 35,6 | 31,8 | 54,9 | 55,2 | 48,7 | 43 | 53,8 | 43,8 |
| 9 M.truncatula_GTE1302 | 30 | 22,7 | 25,6 | 25,6 | 33,5 | 33,9 | 33,6 | 28,7 | 31,9 | 28,6 |
| 10 O.sativa_GTE2201 | 100 | 28,1 | 36,7 | 31,8 | 47,4 | 51,7 | 41,6 | 69,9 | 45,7 | 66,9 |
| 11 O.sativa_GTE701 | | 28,1 | 36,7 | 31,8 | 47,4 | 51,7 | 41,6 | 69,9 | 45,7 | 66,9 |
| 12 P.patens_GTE1501 | 40,4 | | 59 | 41,2 | 31,9 | 33,5 | 28,5 | 26 | 31,9 | 26,9 |
| 13 P.patens_GTE1502 | 54,3 | 65,8 | | 54,8 | 40,3 | 40,2 | 33,9 | 32,8 | 36,9 | 32,4 |
| 14 P.patens_GTE1505 | 46,4 | 46 | 59,1 | | 34,4 | 35,2 | 33,6 | 29,8 | 33,3 | 31,3 |
| 15 P.trichocarpa_GTE907 | 65,1 | 46 | 60,5 | 46,9 | | 82,7 | 54,9 | 48,2 | 67,4 | 48,7 |
| 16 P.trichocarpa_GTE908 | 69,5 | 45,1 | 57,3 | 48,9 | 88,5 | | 55,1 | 53 | 66,5 | 53,2 |
| 17 P.trichocarpa_GTE909 | 59,4 | 42,9 | 54,1 | 48 | 74 | 73,7 | | 41,1 | 51 | 42,9 |
| 18 S.bicolor_TA26028 | 81,7 | 40,4 | 51,9 | 50 | 63,5 | 68,9 | 60,5 | | 45,8 | 66,6 |
| 19 S.lycopersicum_TA45339 | 67,7 | 45,8 | 58,8 | 48,1 | 83,3 | 81,5 | 71,2 | 65,1 | | 45,8 |
| 20 T.aestivum_TA83944 | 80,3 | 41,4 | 50,9 | 48,1 | 62,8 | 67 | 60,7 | 79,2 | 64,6 | |
| 21 V.vinifera_GSVIVT02627001 | 61,2 | 50,9 | 56,4 | 41,1 | 75,3 | 73,1 | 64,6 | 58,4 | 72,1 | 55 |
| 22 V.vinifera_GSVIVT08344001 | 59,4 | 36,2 | 45,2 | 63 | 66,7 | 69,7 | 61 | 61 | 64,8 | 58,1 |
| 23 Z.mays_GTE110 | 77,2 | 41,6 | 51,6 | 46,1 | 62 | 66,2 | 58,4 | 88,9 | 62,2 | 72,5 |

| | 21 | 22 | 23 |
|---|---|---|---|
| 1 A.thaliana_AT2G34900_GTE1 | 45,7 | 42,9 | 40,2 |
| 2 A.thaliana_AT3G52280_GTE6 | 45,4 | 42,7 | 40,8 |
| 3 C.solstitialis_TA2829 | 56,6 | 54,3 | 43,4 |
| 4 G.hirsutum_TA31156 | 56 | 52,7 | 42 |

| | | | |
|---|---|---|---|
| 5 G.raimondii_CO070884 | 35,7 | 23,3 | 25,5 |
| 6 G.raimondii_TA13411 | 55,8 | 52,4 | 43 |
| 7 L.saligna_DW067496 | 34 | 25,5 | 27,7 |
| 8 M.truncatula_AC174355 | 48,9 | 44,8 | 40,9 |
| 9 M.truncatula_GTE1302 | 31,3 | 32,5 | 28 |
| 10 O.sativa_GTE2201 | 44,7 | 43,5 | 63,2 |
| 11 O.sativa_GTE701 | 44,7 | 43,5 | 63,2 |
| 12 P.patens_GTE1501 | 34,2 | 26,3 | 25,7 |
| 13 P.patens_GTE1502 | 37,5 | 30,6 | 30,9 |
| 14 P.patens_GTE1505 | 27,8 | 42,7 | 26,9 |
| 15 P.trichocarpa_GTE907 | 67 | 59,2 | 45,2 |
| 16 P.trichocarpa_GTE908 | 65,5 | 63,9 | 48,8 |
| 17 P.trichocarpa_GTE909 | 49 | 49,3 | 40,3 |
| 18 S.bicolor_TA26028 | 43,4 | 45,1 | 85,8 |
| 19 S.lycopersicum_TA45339 | 57,1 | 51,1 | 43,7 |
| 20 T.aestivum_TA83944 | 42,2 | 43,8 | 59,9 |
| 21 V.vinifera_GSVIVT02627001 | | 63 | 40,7 |
| 22 V.vinifera_GSVIVT08344001 | 63,9 | | 41,8 |
| 23 Z.mays_GTE110 | 55,9 | 55,8 | |

3.3. PCD-LIKE

Global percentages of similarity and identity between full length polypeptide sequences useful in performing the methods of the invention are determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

A MATGAT table for local alignment of a specific domain, or data on % identity/similarity between specific domains may also be generated.

3.4. Pseudo Response Regulator type 2 (PRR2)

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62
First Gap: 12
Extending gap: 2

Results of the software analysis are shown in Table B3 for the global similarity and identity over the full length of the polypeptide sequences (excluding the partial polypeptide sequences).

The percentage identity between the full length polypeptide sequences useful in performing the methods of the invention can be as low as 46% amino acid identity compared to SEQ ID NO: 452.

**Table B3:** MatGAT results for global similarity and identity over the full length of the polypeptide sequences of Table A4.

| | 1 | 2 | 3 | 4 | 5 | 6 | 7 | 8 | 9 | 10 | 11 |
|---|---|---|---|---|---|---|---|---|---|---|---|
| **1. Lyces_ PRR2** | | **53.8** | **46.5** | **56.5** | **54.9** | **54.5** | **52.1** | **58.4** | **56.8** | **68.1** | **60.2** |
| 2. Aqufo_ PRR2 | 69.1 | | 45.2 | 53.6 | 50.9 | 52.2 | 50.3 | 56.9 | 56.5 | 51 | 59.9 |
| 3. Arath_ PRR2 | 62.1 | 61.2 | | 48.3 | 46.1 | 47 | 48.2 | 50.2 | 48.9 | 47.8 | 51.1 |
| 4. Eucgr_ PRR2 | 72.5 | 68.6 | 62.3 | | 57.6 | 58.1 | 56.1 | 63.3 | 61 | 56.6 | 65.8 |
| 5. Glyma_ PRR2 | 70.9 | 67.5 | 61.4 | 71.6 | | 77.1 | 73.8 | 59.4 | 57.5 | 51.7 | 63.7 |
| 6. Lotja_ PRR2 | 69.8 | 68.4 | 63.7 | 72.9 | 84.9 | | 74.5 | 60.9 | 58.9 | 53 | 63.3 |
| 7. Medtr_ PRR2 | 67.1 | 65.9 | 63.7 | 70 | 81.4 | 82.2 | | 58.8 | 57.1 | 53.1 | 61.4 |
| 8. Poptr_ PRR2 I | 73.4 | 71.9 | 65.8 | 74.7 | 72.5 | 74.1 | 71 | | 82.2 | 58.7 | 69.5 |
| 9. Poptr_ PRR2 II | 72.3 | 72.2 | 64.5 | 72.5 | 70.7 | 72.2 | 69.6 | 88.5 | | 57.1 | 68.8 |
| 10. Solly_ PRR2 II | 80.5 | 67.4 | 62.4 | 72.1 | 68.2 | 69.2 | 68.3 | 72.1 | 71.2 | | 61.8 |
| 11. Vitvi_ PRR2 | 75.9 | 73.8 | 66.1 | 76.7 | 76 | 76.8 | 73.8 | 79.9 | 79.5 | 75.3 | |

The percentage amino acid identity can be significantly increased if the most conserved region of the polypeptides are compared. For example, when comparing the amino acid sequence of a pseudo response receiver domain as represented by SEQ ID NO: 475, or of a Myb-like DNA binding domain as represented by SEQ ID NO: 476, or of a C-terminal conserved domain as represented by SEQ ID NO: 477 with the respective corresponding domains of the polypeptides of Table A4, the percentage amino acid identity increases significantly (in order of preference at least 50%, 55%, 60%, 65%, 70%, 75%, 80%, 85%, 90%, 95%, 98%, 99% or more amino acid sequence identity).

***Example 4: Identification of domains comprised in polypeptide sequences useful in performing the methods of the invention***

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

4.1. basic-Helix-Loop-Helix group 9 (bHLH9)

The results of the InterPro scan of the polypeptide sequence as represented by SEQ ID NO: 2 are presented in Table C1.

**Table C1:** InterPro scan results (major accession numbers) of the polypeptide sequence as represented by SEQ ID NO: 2.

| Database | Accession number | Name Domain | Amino acid coordinates T[Start-End] | Evalue |
|---|---|---|---|---|
| InterPro | IPR001092 | Basic helix-loop-helix dimerisation region bHLH | | |
| HMMPfam | PF00010 | HLH or bHLH | T[318-366] | 6.4E-5 |
| HMMSmart | SM00353 | HLH | T[321-371] | 1.2E-12 |
| ProfileScan | PS50888 | HLH | T[309-366] | 0.0 |
| InterPro | IPR011598 | Helix-loop-helix DNA-binding | | |
| Gene3D | G3DSA:4.10.280.10 | HLH_DNA_bd | T[311-377] | 7.4E-5 |
| Superfamily | SSF47459 | HLH_basic | T[311-385] | 2.2E-12 |

Additionally, comparison of SEQ ID NO: 2 (O.sativa_Os01g0900800) with other bHLH9 polypeptides sequences of Table A1 allowed identification of the highly conserved HLH domain in said bHLH9 polypeptides. Table C2 gives the amino acid sequence of the HLH domain as present in bHLH9 polypeptides polypeptides of Table A1.

**Table C2.** Amino acid sequence of the HLH domain in bHLH9 polypeptides.

| bHLH9 polypeptide | bHLH9 polypeptide SEQ ID NO: | HLH domain SEQ ID NO: |
|---|---|---|
| O.sativa_Os01g0900800 | 2 | 225 |
| A.thaliana_bHLH130_9_AT2G42280 | 4 | 181 |
| A.thaliana_bHLH122_9_AT1G51140 | 6 | 182 |
| A.thaliana_bHLH081_9_AT4G09180 | 8 | 183 |
| A.thaliana_bHLH080_9_AT1G35460 | 10 | 184 |
| A.thaliana_bHLH128_9_AT1G05805 | 12 | 185 |
| A.thaliana_bHLH129_9_AT2G43140 | 14 | 186 |
| B.napus_BPS_9258 | 20 | 187 |
| B.oleracea_EH428573 | 22 | 188 |
| B.oleracea_TA6610_3712 | 24 | 189 |

(continued)

| bHLH9 polypeptide | bHLH9 polypeptide SEQ ID NO: | HLH domain SEQ ID NO: |
|---|---|---|
| C.clementina_TA5735_85681 | 26 | 190 |
| C.intybus_EH689338 | 28 | 191 |
| C.obtusa_BW987363 | 30 | 192 |
| C.sinensis_TA15236_2711 | 32 | 193 |
| C.sinensis_TA15331_2711 | 34 | 194 |
| C.solstitialis_EH761577 | 36 | 195 |
| C.tinctorius_EL407531 | 38 | 196 |
| E.esula_TA10959_3993 | 40 | 197 |
| G.hirsutum_TA24161_3635 | 42 | 198 |
| G.hybrid_AJ763309 | 44 | 199 |
| G.max_BPS_22716 | 46 | 200 |
| G.max_BPS_39182 | 48 | 201 |
| G.max_TA56453_3847 | 50 | 202 |
| G.max_TA57335_3847 | 52 | 203 |
| G.max_TA63030_3847 | 54 | 204 |
| G.raimondii_TA12344_29730 | 56 | 205 |
| G.soja_CA783858 | 58 | 206 |
| H.ciliaris_EL431737 | 60 | 207 |
| H.paradoxus_EL487892 | 62 | 208 |
| H.petiolaris_DY944559 | 64 | 209 |
| H.vulgare_TA45248_4513 | 66 | 210 |
| H.vulgare_TA47636_4513 | 68 | 211 |
| I.nil_TA15694_35883 | 70 | 212 |
| I.nil_TA8920_35883 | 72 | 213 |
| I.nil_TA9081_35883 | 74 | 214 |
| I.nil_TA9289_35883 | 76 | 215 |
| L.saligna_TA4923_75948 | 78 | 216 |
| L.sativa_TA5039_4236 | 80 | 217 |
| L.serriola_DW114802 | 82 | 218 |
| M.truncatula_AC141114_16.2 | 84 | 219 |
| M.truncatula_AC149471_36.2 | 86 | 220 |
| M.truncatula_TA37090_3880 | 88 | 221 |
| N.benthamiana_TA8284_4100 | 90 | 222 |
| N.benthamiana_TA8285_4100 | 92 | 223 |
| O.sativa_LOC_Os02g39140.1 | 94 | 224 |
| O.sativa_Os04g0489600 | 96 | 226 |
| O.sativa_Os08g0506700 | 98 | 227 |
| O.sativa_Os09g0487900 | 100 | 228 |

(continued)

| bHLH9 polypeptide | bHLH9 polypeptide SEQ ID NO: | HLH domain SEQ ID NO: |
|---|---|---|
| P.patens_121037_e_gw1.34.393.1 | 102 | 229 |
| P.patens_148201_e_gw1.273.42.1 | 104 | 230 |
| P.patens_TA27139_3218 | 106 | 231 |
| P.patens_TA32785_3218 | 108 | 232 |
| P.taeda_TA15788_3352 | 110 | 233 |
| P.tremula_DN488299 | 112 | 234 |
| P.tremula_TA18674_47664 | 114 | 235 |
| P.trichocarpa_scaff_I.1785 | 116 | 236 |
| P.trichocarpa_scaff_III.1580 | 118 | 237 |
| P.trichocarpa_scaff_IX.1004 | 120 | 238 |
| P.trichocarpa_scaff_XIV.978 | 122 | 239 |
| P.trichocarpa_scaff_XIX.717 | 124 | 240 |
| P.trichocarpa_scaff_XVI.437 | 126 | 241 |
| R.communis_EG683575 | 128 | 242 |
| S.bicolor_TA33134_4558 | 130 | 243 |
| S.lycopersicum_TA38189_4081 | 132 | 244 |
| S.lycopersicum_TA46743_4081 | 134 | 245 |
| S.officinarum_CA143325 | 136 | 246 |
| S.officinarum_TA45654_4547 | 138 | 247 |
| S.tuberosum_CV506096 | 140 | 248 |
| S.tuberosum_TA26686_4113 | 142 | 249 |
| S.tuberosum_TA29368_4113 | 144 | 250 |
| S.tuberosum_TA37004_4113 | 146 | 251 |
| S.tuberosum_TA40158_4113 | 148 | 252 |
| T.aestivum_TA88301_4565 | 150 | 253 |
| T.officinale_DY832981 | 152 | 254 |
| V.vinifera_GSVIVT00001847001 | 154 | 255 |
| V.vinifera_GSVIVT00005670001 | 156 | 256 |
| V.vinifera_GSVIVT00008845001 | 158 | 257 |
| V.vinifera_GSVIVT00024649001 | 160 | 258 |
| V.vinifera_GSVIVT00025522001 | 162 | 259 |
| V.vinifera_TA49422_29760 | 164 | 260 |
| Z.mays_BPS_3797 | 166 | 261 |
| Z.mays_BPS_30292 | 168 | 262 |
| Z.mays_BPS_52761 | 170 | 263 |
| Z.mays_TA16655_4577999 | 172 | 264 |
| Z.officinale_TA2571_94328 | 174 | 265 |
| Z.officinale_TA334_94328 | 176 | 266 |

(continued)

| bHLH9 polypeptide | bHLH9 polypeptide SEQ ID NO: | HLH domain SEQ ID NO: |
|---|---|---|
| Z.officinale_TA5709_94328 | 178 | 267 |
| Z.officinale_TA6615_94328 | 180 | 268 |

4.2. Imbibition-inducible 1 (IMB1)

The results of the InterPro scan of the polypeptide sequence as represented by SEQ ID NO: 301 are presented in Table C3.

**Table C3:** InterPro scan results (major accession numbers) of the polypeptide sequence as represented by SEQ ID NO: 301.

| Database | Accession number | Accession name | Amino acid coordinates on SEQ ID NO 301 |
|---|---|---|---|
| InterPro | IPR001487 | Bromodomain | |
| FPrintScan | PR00503 | BROMODOMAIN | T[114-127] T[130-146] T [167-186] |
| Gene3D | G3DSA:1.20.920.10 | no description | T[75-234] |
| HMMPfam | PF00439 | Bromodomain | T[99-191] |
| HMMSmart | SM00297 | no description | T[92-205] |
| superfamily | SSF47370 | Bromodomain | T[73-216] |

4.3. PCD-LIKE

The protein sequences representing the GRP are used as query to search the InterPro database.

4.4. Pseudo Response Regulator type 2 (PRR2)

The results of the InterPro scan of the polypeptide sequence as represented by SEQ ID NO: 452 are presented in Table C4.

**Table C4:** InterPro scan results of the polypeptide sequence as represented by SEQ ID NO: 452

| InterPro accession number and name | Integrated database name | Integrated database accession number | Integrated database accession name |
|---|---|---|---|
| IPR001789 Signal transduction response regulator, receiver region | ProDOM | PD000039 | Q9LKL2_Arath |
| | PFAM | PF00072 | Response_reg |
| | SMART | SM00448 | REC |
| IPR006447 Myb-like DNA-binding region, SHAKYF | TIGR | TIGR01557 | Myb_SHAQKYF; myb-like DNA binding domain |
| IPR012287 Homeo-domain related | G3DSA | G3DSA/1.10.10.60 | No description |
| IPR014778 Myb, DNA-binding | PFAM | PF00249 | Myb_DNA-binding |

***Example 5: Topology prediction of the polypeptide sequences useful in performing the methods of the invention***

5.1. Imbibition-inducible 1 (IMB1)

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 301 are presented Table D1. The "plant" organism group has been selected, no cutoffs defined, and the predicted length of the transit peptide requested. The subcellular localization of the polypeptide sequence as represented by SEQ ID NO: 2 may be the cytoplasm or nucleus, no transit peptide is predicted.

**Table D1:** TargetP 1.1 analysis of the polypeptide sequence as represented by SEQ ID NO: 301. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|---|---|---|---|---|---|---|---|---|
| Le_IMB1 | 378 | 0.080 | 0.110 | 0.033 | 0.921 | _ | 1 | - |
| cutoff | | 0.000 | 0.000 | 0.000 | 0.000 | | | |

When analysed with other algorithms, a nuclear localisation is predicted, in line with the function as transcription factor.

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

5.2. PCD-LIKE

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The protein sequences representing the GRP are used to query TargetP 1.1. The "plant" organism group is selected, no cutoffs defined, and the predicted length of the transit peptide requested.

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark

***Example 6: Subcellular localisation prediction of the polypeptide sequences useful in performing the methods of the invention***

<u>6.1. Pseudo Response Regulator type 2 (PRR2)</u>

Experimental methods for protein localization range from immunolocalization to tagging of proteins using green fluorescent protein (GFP) or beta-glucuronidase (GUS). Such methods to identify subcellular compartmentalisation of GRF polypeptides are well known in the art.

A predicted nuclear localisation signal (NLS) was found by multiple sequence alignment, followed by eye inspection, in the polypeptide sequences of Table A4 (for SEQ ID NO: 452 KSNRKKIK). An NLS is one or more short sequences of positively charged lysines or arginines.

Computational prediction of protein localisation from sequence data was performed. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, TMpred, and others.

***Example 7: Assay related to the polypeptide sequences useful in performing the methods of the invention***

<u>7.1. Imbibition-inducible 1 (IMB1)</u>

Chua et al. (2005) describe a chromatin immunoprecipitation (ChIP) assay for analysing the functionality of an IMB1 polypeptide (*in casu* GTE6 fused to GFP and the *AS1* promoter). *AS1* transcripts were increased in the *35S::GTE6-GFP* plants, as well as in the *35S::GTE6* plants, indicating that both GTE6-GFP and GTE6 upregulated the expression of *AS1.*

Approximately 0.1-0.8 g leaves of 21-d-old *Arabidopsis* plants grown on 1/2 MS agar in tissue culture plates were fixed with 1% formaldehyde (Sigma) under vacuum for 15 min. The plant material was ground in liquid nitrogen, and chromatin was extracted. The chromatin was sheared into fragments ranging from 400 bp to 1 kbp by sonication. The sonicated chromatin was immunoprecipitated with 10 μL of anti-acetylated histone H4 (Upstate Biotechnology), 10 μL of anti-acetylated histone H3 (Upstate Biotechnology), or 5μ L of anti-GFP (Abcam). DNA was amplified using primers specific for 18S rDNA, and various regions of *AS1.* PCR reactions were first performed with various dilutions of the template DNA to ensure that the PCR conditions were within the quantitative range of the amplification reaction. Co-precipitated DNA was dissolved in 20 μL of TE, and typically 1 μL was used for PCR analyses. For total input samples, DNA was extracted from an aliquot of sonicated chromatin, dissolved in 80 μL of TE, and 1 μL was used for PCR. Twenty-eight cycles were used to amplify the various regions of AS1, and 20 cycles were used to amplify the 18S rDNA sequence. Regions of AS1 were first amplified using AS1 primers for 8 cycles, followed by the addition of 18S rDNA primers, and the PCR was performed for another 20 cycles. In three independently transformed lines of 35S::GTE6-GFP plants, the GFP antibodies coprecipitated the promoter, and the 3' end of the intron and the 5' end of exon 2 of AS1. The GFP antibodies did not coprecipitate regions P3 and P0 of AS1; these sequences were undetected even after forty rounds of PCR. GTE6 thus regulates expression by associating with a 1-kbp region containing the promoter and the start of the transcribed region of *AS1,* which are likely to be important regulatory regions for transcriptional control.

<u>7.2. Pseudo Response Regulator type 2 (PRR2)</u>

PRR2 polypeptides useful in the methods of the present invention (at least in their native form) typically, but not necessarily, have transcriptional regulatory activity and capacity to interact with other proteins. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). PRR2 domains contain a Myb DNA-binding of the SHAQYKF.

*Example 8: Cloning of the nucleic acid sequence used in the methods of the invention*

8.1. basic-Helix-Loop-Helix group 9 (bHLH9)

The nucleic acid sequence used in the methods of the invention was amplified by PCR using as template a custom-made *Oryza sativa* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were 5'-gggga caagtttgtacaaaaaagcaggcttaaacaatgaacaggatgacggc-3' (SEQ ID NO: 271; sense), and 5'-ggggaccactttgtacaagaaagctgggtttacaacattagctcggaga-3' (SEQ ID NO: 272; reverse, complementary) which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pbHLH9. Plasmid pDONR201 was purchased from Invitrogen, as part of the Gateway® technology.

The entry clone comprising the longest ORF (open reading frame) in SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 275) for constitutive specific expression was located upstream of this Gateway cassette.

After the LR recombination step, the resulting expression vector pGOS2::bHLH9 (Figure 3) was transformed into Agrobacterium strain LBA4044 according to methods well known in the art.

8.2. Imbibition-inducible 1 (IMB1)

The nucleic acid sequence used in the methods of the invention was amplified by PCR using as template a custom-made *Solanum lycopersicum* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used were prm10548 (SEQ ID NO: 302; sense, start codon in bold): 5'-ggggacaagtttgt acaaaaaagcaggct taaaca**atg**gagaatctaaacggctta-3' and prm10549 (SEQ ID NO: 303; reverse, complementary): 5'-ggggaccactttgtacaagaaagctgggtgtaaaatcaggatggcttttt-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pIMB1. Plasmid pDONR201 was purchased from Invitrogen, as part of the Gateway® technology.

The entry clone comprising SEQ ID NO: 300 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 313) for constitutive specific expression was located upstream of this Gateway cassette.

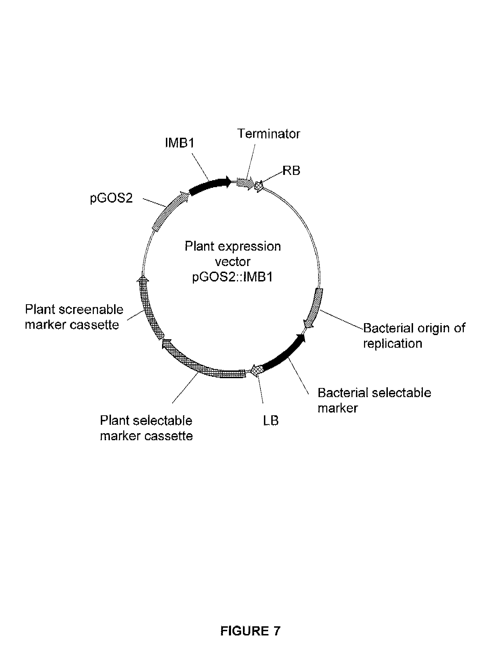

After the LR recombination step, the resulting expression vector pGOS2::IMB1 (Figure 7) was transformed into *Agrobacterium* strain LBA4044 according to methods well known in the art.

8.3. PCD-LIKE

The nucleic acid sequence used in the methods of the invention was amplified by PCR using as template a custom-made *Oryza sativa* seedlings cDNA library (in pCMV Sport 6.0; Invitrogen, Paisley, UK). PCR was performed using Hifi Taq DNA polymerase in standard conditions, using 200 ng of template in a 50 μl PCR mix. The primers used had a sequence as represented by SEQ ID NO: 448; sense, start codon in bold): 5'-ggggacaagtttgtacaa aaaagcaggcttaaacaatggcgctcaccaacc-3' and SEQ ID NO: 449 (reverse, complementary): 5'-ggggaccactttgtacaagaaagctgggtaaggggatatatgtgaatgaaga-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pPCD. Plasmid pDONR201 was purchased from Invitrogen, as part of the Gateway® technology.

The entry clone comprising the longest Open Redeading Frame(ORF) in SEQ ID NO: 358 was then used in an LR reaction with a destination vector used for *Oryza sativa* transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 450) for constitutive specific expression was located upstream of this Gateway cassette.

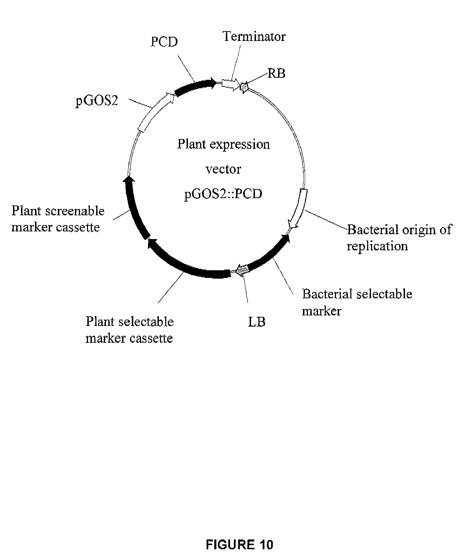

After the LR recombination step, the resulting expression vector pGOS2::PCD (Figure 10) was transformed into Agrobacterium strain LBA4044 according to methods well known in the art.

8.4. Pseudo Response Regulator type 2 (PRR2)

The *Lycopersicon esculentum* nucleic acid sequence encoding a PRR2 polypeptide sequence as represented by SEQ ID NO: 2 was amplified by PCR using as template a cDNA bank constructed using RNA from tomato plants at different developmental stages. The following primers, which include the AttB sites for Gateway recombination, were used for PCR amplification: prm10843 (SEQ ID NO: 479, sense): 5'-ggggacaagtttgtacaaaaaag caggcttaaacaatgatttgcattgagaatgaa-3' and prm10844 (SEQ ID NO: 480, reverse, complementary): 5'-ggggaccactttgtacaagaaagctgggtacatgtcatctcatctccgac-3'. PCR was performed using Hifi Taq DNA polymerase in standard conditions. A PCR fragment of the expected length (including attB sites) was amplified and purified also using standard methods. The first step of the Gateway procedure, the BP reaction, was then performed, during which the PCR fragment recombined in vivo with the pDONR201 plasmid to produce, according to the Gateway terminology, an "entry clone". Plasmid pDONR201 was purchased from Invitrogen, as part of the Gateway® technology.

The entry clone comprising SEQ ID NO: 451 was subsequently used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 478) for constitutive expression was located upstream of this Gateway cassette.

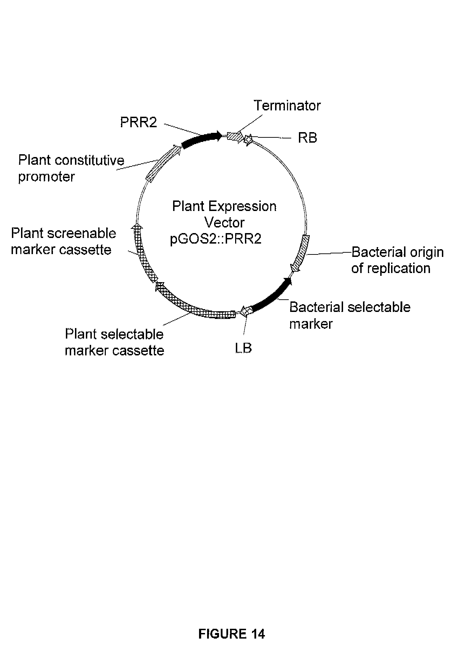

After the LR recombination step, the resulting expression vector pGOS2::PRR2 (Figure 14) for constitutve expression, was transformed into *Agrobacterium* strain LBA4044 according to methods well known in the art.

## *Example 9: Plant transformation*

### *Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

### *Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

***Example 9: Phenotypic evaluation procedure***

<u>10.1 Evaluation setup</u>

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution.

The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

10.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

10.3 Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Examples 11: Results of the phenotypic evaluation of the transgenic plants***

11.1. basic-Helix-Loop-Helix group 9 (bHLH9)

The results of the evaluation under non-stress conditions of T1 transgenic rice plants expressing the longest Open Reading Frame ORF of the O.sativa_Os01g0900800 nucleic acid which encodes SEQ ID NO: 2 are presented below. An increase of at least 5 % was observed for aboveground biomass (AreaMax), emergence vigour (early vigour) and number of total seeds (Table E1).

**Table E1**

| Yield related trait | % increase in transgenic plants relative to control plant |
|---|---|
| AreaMax | 20 |
| early vigour | 32 |
| number of total seeds | 17 |

11.2. Imbibition-inducible 1 (IMB1)

The results of the evaluation of transgenic rice plants expressing an *IMB1* nucleic acid under non-stress conditions are presented below. An increase of at least 5 % (with a p-value ≤0.05) was observed for total weight of seeds, number of filled seeds, flowers per panicle, harvest index and for total number of seeds. The overall increase for these parameters in the confirmation experiment is provided in Table E2. An increase was also observed for above-ground biomass and fill rate.

Table E2:

| Parameter | Overall increase |
|---|---|
| totalwgseeds | 18.2 |
| nrfilledseed | 15.7 |
| flowerperpan | 11.2 |
| harvestindex | 13.9 |
| nrtotalseed | 11.7 |

10.3. PCD-LIKE

The results of the evaluation of transgenic rice plants in the T2 generation and expressing a nucleic acid comprising the longest ORF in SEQ ID NO: 358 under non-stress conditions are presented below. See previous Examples for details on the generations of the transgenic plants.

An increase of at least 5 % was observed for the total seed yield (totalwgseeds), number of filled seeds (nrfilledseed), fill rate, number of seeds per plant (nrtotalseed), harvest index (harvestindex) (Table E3).

**Table E3**

| Parameter | % increased in transgenic plants relative to control plants |
|---|---|
| totalwgseeds | 14,6 |
| nrfilledseed | 13,1 |
| harvestindex | 9,8 |
| nrtotalseed | 10,6 |

10.4. Pseudo Response Regulator type 2 (PRR2)

The results of the evaluation of T2 generation transgenic rice plants expressing the nucleic acid sequence encoding a PRR2 polypeptide as represented by SEQ ID NO: 452, under the control of a constitutive promoter, and grown under normal growth conditions, are presented below.

There was a significant increase in aboveground biomass, in early vigor, flowering time, root biomass, plant height, seed yield per plant, number of filled seeds, total number of seeds, number of primary panicles, number of flowers per panicles, harvest index (HI), and Thousand Kernel Weight (TKW).

**Table E4:** Results of the evaluation of T2 generation transgenic rice plants expressing the nucleic acid sequence encoding a PRR2 polypeptide as represented by SEQ ID NO: 452, under the control of a promoter for constitutive expression.

| **Trait** | **Overall average % increase in 4 events in the T2 generation** |
|---|---|
| Plant aboveground biomass | 18% |
| Early vigor | 10% |
| Flowering time | -4% |
| Root biomass | 14% |
| Plant height | 9% |
| Total seed yield per plant | 38% |
| Number of filled seeds | 32% |
| Total number of seeds | 29% |
| Number of primary panicles | 11% |
| Number of flowers per panicle | 19% |
| Harvest index | 19% |
| Thousand kernel weight | 5% |

## Claims

1. A method for enhancing yield-related traits in plants relative to control plants, comprising introducing and expressing in a plant a nucleic acid encoding a PCD polypeptide comprising a Pterin-4-alpha-carbinolamine dehydratase (PCD) domain (Interpro accession number: IPR001533; pfam accession number: PF01329), said PCD domain preferably having in increasing order of preference at least 50%, 51 %, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 440.

2. Method according to claim 1, wherein said PCD polypeptide comprises one or more motifs having at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to any one or more of the following motifs:

   (i) Motif 12 (SEQ ID NO: 441): [G/E]-[D/N]-[F/L]-G-A-R-D-P-x(3)-E-x(4)-F-G-[D/E]K;
   (ii) Motif 13 (SEQ ID NO: 442): [E/D/K/H/Q/N]-x(3)-H-[H/N]-[P/C/S]-x(5,6)-[Y/W/F/H]-x(9)-[H/W]-x(8,15)-D;
   (iii) Motif 14 (SEQ ID NO: 443): WK(V/L)(R/K) wherein amino acids in brackets represent alternative amino acids at the same location;
   (iv) Motif 15 (SEQ ID NO: 444): TDFI;
   (v) Motif 16 (SEQ ID NO: 445): (S/V)(V/I)(G/R/S/A)GL(T/S);
   (vi) Motif 17 (SEQ ID NO: 446): LGDF(G/R)(A/R)(R/A)(D/G)P;
   (vii) Motif 18 (SEQ ID NO:447): H(R/K)ILIP(T/A)

3. Method according to any one of claims 1 to 3, wherein said nucleic acid encoding a PCD polypeptide encodes any one of the proteins listed in Table A3 or is a portion of such a nucleic acid, or a nucleic acid capable of hybridising with such a nucleic acid.

4. Method according to any preceding claim, wherein said enhanced yield-related traits comprise increased yield, preferably increased early vigour and/or increased biomass and/or increased seed yield relative to control plants.

5. Method according to any one of claims 1 to 4, wherein said enhanced yield-related traits are obtained under non-stress conditions.

6. Method according to any one of claims 1 to 5, wherein said nucleic acid is operably linked to a constitutive promoter, preferably to a GOS2 promoter, most preferably to a GOS2 promoter from rice.

7. Plant or part thereof, including seeds, obtainable by a method according to claim 12, wherein said plant or part thereof comprises a recombinant nucleic acid encoding a PCD polypeptide.

**8.** Construct comprising:

(i) nucleic acid encoding a PCD polypeptide as defined in claims 1 or 2;
(ii) one or more control sequences capable of driving expression of the nucleic acid sequence of (a); and optionally
(iii) a transcription termination sequence.

**9.** Construct according to claim 8, wherein one of said control sequences is a constitutive promoter, preferably a GOS2 promoter, most preferably a GOS2 promoter from rice.

**10.** Use of a construct according to claim 8 or 9, in a method for making plants having increased yield, particularly increased early vigour and/or increased biomass and/or increased seed yield relative to control plants.

**11.** Plant, plant part or plant cell transformed with a construct according to claim 8 or 9.

**12.** Method for the production of a transgenic plant having increased yield, particularly increased early vigour and/or increased biomass and/or increased seed yield relative to control plants, comprising:

(i) introducing and expressing in a plant a nucleic acid encoding a PCD polypeptide as defined in claim 1 or 2; and
(ii) cultivating the plant cell under conditions promoting plant growth and development; and optionally
(iii) selecting for plants having increased yield.

**13.** Transgenic plant having increased yield, particularly increased early vigour and/or increased biomass and/or increased seed yield, relative to control plants, resulting from modulated expression of a nucleic acid encoding a PCD polypeptide as defined in claim 1 or 2, or a transgenic plant cell derived from said transgenic plant.

**14.** Harvestable parts of a plant according to claim 13, wherein said harvestable parts are preferably shoot biomass and/or seeds.

**15.** Products derived from a plant according to claim 13 and/or from harvestable parts of a plant according to claim 15.

**16.** Use of a nucleic acid encoding a PCD polypeptide in increasing yield, particularly in increasing early vigour and/or in increasing biomass and/or increasing seed yield in plants, relative to control plants.

```
                                          1                                                50
            H.paradoxus_EL487892   (1) --------------------------------------------------
       M.truncatula_AC141114_16.2   (1) --------------------------------------------------
         V.vinifera_TA49422_29760   (1) --------------------------------------------------
         C.solstitialis_EH761577   (1) --------------------------------------------------
              I.nil_TA8920_35883   (1) --------------------------------------------------
    S.lycopersicum_TA38189_4081   (1) MFSSEPISREMDKLNSFFFSGGGGGSSSFKNGEGMESEFFRSKEMMGSDF
        S.tuberosum_TA29368_4113   (1) --------------------------------------------------
        C.sinensis_TA15331_2711   (1) --------------------------------------------------
              P.tremula_DN488299   (1) --------------------------------------------------
    P.trichocarpa_scaff_IX.1004   (1) ------------------MNLLYSSSFKYSDGELRKSQEFMDLNPYHYHQ
         P.tremula_TA18674_47664   (1) --------------------------------------------------
     P.trichocarpa_scaff_I.1785   (1) --------------------------------------------------
             G.max_TA57335_3847   (1) --------------------------------------------------
             G.max_TA63030_3847   (1) --------------------------------------------------
              G.hybrid_AJ763309   (1) --------------------------------------------------
  V.vinifera_GSVIVT00008845001   (1) ---------------MAPDVYNHQHHPHQ-NSGLMRYQSAPSSLLGSFVD
                 G.soja_CA783858   (1) --------------------------------------------------
   P.trichocarpa_scaff_XVI.437   (1) -------------MPMDSSSNNNYHQQNQPSSGLLRFRSAPSSLLASFND
  V.vinifera_GSVIVT00025522001   (1) --------------------------------------------------
  V.vinifera_GSVIVT00024649001   (1) ---------------MASGGTEDSSSHTVMNMRQNSQAMAPESVVVVSQQ
           E.esula_TA10959_3993   (1) --------------------------------------------------
           S.tuberosum_CV506096   (1) --------------------------------------------------
                G.max_BPS_22716   (1) ----MESDLQQHPPMFLDH------HQQQMNSGLTRYRSAPSSYFSSIID
      M.truncatula_TA37090_3880   (1) --------------------------------------------------
  P.trichocarpa_scaff_III.1580   (1) ----MESDLQHQHHFLHGHNQHQQIHQKQMNSGLTRYQSAPSSYFSSNLD
              R.communis_EG683575   (1) --------------------------------------------------
              H.ciliaris_EL431737   (1) --------------------------------------------------
          T.officinale_DY832981   (1) --------------------------------------------------
              I.nil_TA9289_35883   (1) --------------------------------------------------
        H.vulgare_TA45248_4513   (1) --------------------------------------------------
              Z.mays_BPS_30292   (1) --------------------------MYGAPLPKDLNLPAALPQPTRTPP
        O.sativa_Os08g0506700   (1) --------------------------MYGSPVSKDLNLP---------VQ
        O.sativa_Os09g0487900   (1) --------------------------MYGAPVSKDLSLQPAG-----VRT
        Z.mays_TA16655_4577999   (1) --------------------------MYGSPVSKDLNLP---------AQ
      Z.officinale_TA5709_94328   (1) --------------------------------------------------
             I.nil_TA15694_35883   (1) --------------------------------------------------
      Z.officinale_TA6615_94328   (1) --------------------------------------------------
                B.napus_BPS_9258   (1) --------------------------------------------------
        G.hirsutum_TA24161_3635   (1) --------------------------MYPSSSSQ----------------
   P.trichocarpa_scaff_XIV.978   (1) --------------------------MYQQPSSSS-------------SS
  V.vinifera_GSVIVT00005670001   (1) --------------------------MYPSSASSS-------------S-
                G.max_BPS_39182   (1) --------------------------MYPSSSSSS-------------SS
        B.oleracea_TA6610_3712   (1) --------------------------MYQSSSPTS-------------SS
        L.saligna_TA4923_75948   (1) --------------------------MYPTSNSSSASQTSGGDPNAINGS
     N.benthamiana_TA8284_4100   (1) --------------------------MYPSSTSS-------SPQNSMSHS
```

FIGURE 1

```
                                         1                                                50
     N.benthamiana_TA8285_4100    (1) --------------------------MYPSSTSS-------SSQNSMSHS
       S.tuberosum_TA26686_4113   (1) --------------------------MYPSSTSS-------SSQGSMSHT
    M.truncatula_AC149471_36.2    (1) --------------------------MYRPPSSSAS---------SSSSS
        H.vulgare_TA47636_4513    (1) --------------------------------------------------
      T.aestivum_TA88301_4565     (1) --------------------------------------------------
   O.sativa_LOC_Os02g39140.1      (1) --------------------------------------------------
        S.bicolor_TA33134_4558    (1) --------------------------------------------------
              Z.mays_BPS_3797     (1) --------------------------------------------------
       O.sativa_Os04g0489600      (1) --------------------------------------------------
      S.officinarum_CA143325      (1) --------------------------------------------------
  S.officinarum_TA45654_4547      (1) --------------------------------------------------
             Z.mays_BPS_52761     (1) --------------------------------------------------
       O.sativa_Os01g0900800      (1) --------------------------MNRMTAPHG---------------
   Z.officinale_TA2571_94328      (1) ----------------------------MNHSPSG---------------
         B.oleracea_EH428573      (1) --------------------------------------------------
   C.clementina_TA5735_85681      (1) --------------------------------------------------
     C.sinensis_TA15236_2711      (1) --------------------------------------------------
   G.raimondii_TA12344_29730      (1) --------------------------------------------------
  P.trichocarpa_scaff_XIX.717     (1) --------------------------------------------------
           C.intybus_EH689338     (1) --------------------------------------------------
        L.sativa_TA5039_4236      (1) --------------------------------------------------
         L.serriola_DW114802      (1) --------------------------------------------------
        C.tinctorius_EL407531     (1) --------------------------------------------------
        H.petiolaris_DY944559     (1) --------------------------------------------------
   V.vinifera_GSVIVT00018447001   (1) --------------------------------------------------
           I.nil_TA9081_35883     (1) --------------------------------------------------
 S.lycopersicum_TA46743_4081      (1) --------------------------------------------------
     S.tuberosum_TA37004_4113     (1) --------------------------------------------------
         G.max_TA56453_3847       (1) --------------------------------------------------
          C.obtusa_BW987363       (1) --------------------------------------------------
     S.tuberosum_TA40158_4113     (1) --------------------------------------------------
P.patens_121037_e_gw1.34.393.1    (1) --------------------------------------------------
P.patens_148201_e_gw1.273.42.1    (1) --------------------------------------------------
        P.patens_TA27139_3218     (1) --------------------------------------------------
        P.patens_TA32785_3218     (1) --------------------------------------------------
         P.taeda_TA15788_3352     (1) --------------------------------------------------
    Z.officinale_TA334_94328      (1) --------------------------------------------------
A.thaliana_bHLH080_9_AT1G35460    (1) --------------------------------------------------
A.thaliana_bHLH081_9_AT4G09180    (1) --------------------------------------------------
A.thaliana_bHLH122_9_AT1G51140    (1) --------------------------------------------------
A.thaliana_bHLH128_9_AT1G05805    (1) --------------------------------------------------
A.thaliana_bHLH129_9_AT2G43140    (1) --------------------------------------------------
A.thaliana_bHLH130_9_AT2G42280    (1) --------------------------------------------------
                     Consensus    (1)
```

FIGURE 1 (continued)

```
                                        51                                               100
H.paradoxus_EL487892               (1)  --------------------------------------------------
M.truncatula_AC141114_16.2         (1)  --------------------------------------------------
V.vinifera_TA49422_29760           (1)  --------------------------------------------------
C.solstitialis_EH761577            (1)  --------------------------------------------------
I.nil_TA8920_35883                 (1)  --------------------------------------------------
S.lycopersicum_TA38189_4081       (51)  FQQQSQFQQSNSGGLTRYRSAPSSFFAGILDGDGNNSGENFITGDGSSSS
S.tuberosum_TA29368_4113           (1)  ---------------------------------------MVITGDGSSSS
C.sinensis_TA15331_2711            (1)  --------------------------------------------------
P.tremula_DN488299                 (1)  --------------------------------------------------
P.trichocarpa_scaff_IX.1004       (33)  QQQQIQQNSGLMRYRSAPSSILESLVNGTSGHDGGGIESGDYRYLRSSSP
P.tremula_TA18674_47664            (1)  --------------------------------------------------
P.trichocarpa_scaff_I.1785         (1)  --------------------------------------------------
G.max_TA57335_3847                 (1)  --------------------------------------METMLSKLLPSN
G.max_TA63030_3847                 (1)  --------------------------------------------------
G.hybrid_AJ763309                  (1)  --------------------------------------------------
V.vinifera_GSVIVT00008845001      (35)  GSSVHLQSS------------------SHETETMFARLMSGSSDSQGLQG
G.soja_CA783858                    (1)  --------------------------------------------------
P.trichocarpa_scaff_XVI.437       (38)  ---------------------------------------SGVDNDSVLNY
V.vinifera_GSVIVT00025522001       (1)  --------------------------------------------------
V.vinifera_GSVIVT00024649001      (36)  -N--------------------------------------------Q--F
E.esula_TA10959_3993               (1)  --------------------------------------------------
S.tuberosum_CV506096               (1)  --------------------------------------------------
G.max_BPS_22716                   (41)  HEFYEHVFNRPSSPETERMLTRFVNSLGGGDADAEDS-LATTQNPPTVVA
M.truncatula_TA37090_3880          (1)  --------------------------------------------------
P.trichocarpa_scaff_III.1580      (47)  RDFCEEFLNRPTSPETERIFARFLANSGGNTENIPGSNLCEIKQDSPVKE
R.communis_EG683575                (1)  --------------------------------------------------
H.ciliaris_EL431737                (1)  --------------------------------------------------
T.officinale_DY832981              (1)  --------------------------------------------------
I.nil_TA9289_35883                 (1)  --------------------------------------------------
H.vulgare_TA45248_4513             (1)  --------------------------------------------------
Z.mays_BPS_30292                  (25)  QQQMGSPGLLRYRSAPSTLLGEVMCGGDQDFPAAGG--------AGHGPP
O.sativa_Os08g0506700             (16)  PPAMSSSGLLRYRSAPSTLLAEFCDDFLPP--------------AAAPRA
O.sativa_Os09g0487900             (20)  PPQMSSPGLLRYRSAPSTLLGEVCGDFVLPGGGGGGGQLQLQLQQQRPGS
Z.mays_TA16655_4577999            (16)  PPPMASSGLLRYRSAPSAVLGGLCEDQLQLP-----------------AA
Z.officinale_TA5709_94328          (1)  --------------------------------------------------
I.nil_TA15694_35883                (1)  --------------------------------------------------
Z.officinale_TA6615_94328          (1)  --------------------------------------------------
B.napus_BPS_9258                   (1)  --------------------------------------------------
G.hirsutum_TA24161_3635            (9)  -----KPMGQSGLTRYGSAPGSFLANAVDSVIGADP--------------
P.trichocarpa_scaff_XIV.978       (12)  P---QKSNLPSGLTRYGSAPGSFLTRAVDSVIGADRELSGLGSTPL----
V.vinifera_GSVIVT00005670001      (11)  ----HRSLHSSGLIRYGSAPGSLLTSAVDSLVSTDREFSPLG--SH----
G.max_BPS_39182                   (12)  S---SQSMTQAGLTRYGSAPGSLLTSTVDSLIGGS---------------
B.oleracea_TA6610_3712            (12)  SQRSSLPSGGAGLIRYGSAPGSLLNSMVDEVIGGSNGRDFNFPPPD----
L.saligna_TA4923_75948            (25)  NVSGGGGGAQQGLARYRSAPVSFLTTTVDSVINGQTQHTTVGNHNM----
N.benthamiana_TA8284_4100         (18)  TTAGGDSHDGGGLTRYGSAPGSFLTTAVQSVTGATNHDFNLHGSHH----
```

FIGURE 1 (continued)

```
                                          51                                               100
         N.benthamiana_TA8285_4100   (18) TTAGGGSNGGGGLTRYGSAPGAFLTTAVESVIGANNHDFNLHGSHH----
          S.tuberosum_TA26686_4113   (18) STTAGG---GGGLTRYGSAPGSFLTTAVESVVNAK---------------
       M.truncatula_AC149471_36.2    (16) SQQQSSISQTGLTRYGSAPSSLLNTTVDAIIGGSRLLPGTG---------
            H.vulgare_TA47636_4513    (1) --------------------------------------------------
          T.aestivum_TA88301_4565     (1) --------------------------------------------------
      O.sativa_LOC_Os02g39140.1       (1) --------------------------------------------------
           S.bicolor_TA33134_4558     (1) --------------------------------------------------
                  Z.mays_BPS_3797     (1) --------------------------------------------------
          O.sativa_Os04g0489600       (1) ------------------------------MNPAPSRAPQRQQRGGEMSA
          S.officinarum_CA143325      (1) --------------------------------------------------
     S.officinarum_TA45654_4547       (1) --------------------------------------------------
                 Z.mays_BPS_52761     (1) --------------------------------------------------
          O.sativa_Os01g0900800      (10) GMPPPPMPAAGGLARYGSAPGSLLASIADSVIRGRGVGVVDQLHHHQHQH
        Z.officinale_TA2571_94328     (8) GDRPAAMGPPPGLARYGSAPGSFLGGIADSVIAAGGS-------------
              B.oleracea_EH428573     (1) --------------------------------------------------
        C.clementina_TA5735_85681     (1) --------------------------------------------------
          C.sinensis_TA15236_2711     (1) --------------------------------------------------
        G.raimondii_TA12344_29730     (1) --------------------------------------------------
     P.trichocarpa_scaff_XIX.717      (1) --------------------------------------------------
               C.intybus_EH689338     (1) --------------------------------------------------
              L.sativa_TA5039_4236    (1) --------------------------------------------------
              L.serriola_DW114802     (1) --------------------------------------------------
            C.tinctorius_EL407531     (1) --------------------------------------------------
            H.petiolaris_DY944559     (1) --------------------------------------------------
       V.vinifera_GSVIVT00001847001   (1) --------------------------------------------------
               I.nil_TA9081_35883     (1) --------------------------------------------------
     S.lycopersicum_TA46743_4081      (1) --------------------------------------------------
         S.tuberosum_TA37004_4113     (1) --------------------------------------------------
               G.max_TA56453_3847     (1) --------------------------------------------------
                C.obtusa_BW987363     (1) --------------------------------------------------
         S.tuberosum_TA40158_4113     (1) --------------------------------------------------
  P.patens_121037_e_gw1.34.393.1      (1) --------------------------------------------------
  P.patens_148201_e_gw1.273.42.1      (1) --------------------------------------------------
            P.patens_TA27139_3218     (1) --------------------------------------------------
            P.patens_TA32785_3218     (1) --------------------------------------------------
             P.taeda_TA15788_3352     (1) --------------------------------------------------
        Z.officinale_TA334_94328      (1) --------------------------------------------------
  A.thaliana_bHLH080_9_AT1G35460      (1) --------------------------------------------------
  A.thaliana_bHLH081_9_AT4G09180      (1) --------------------------------------------------
  A.thaliana_bHLH122_9_AT1G51140      (1) --------------------------------------------------
  A.thaliana_bHLH128_9_AT1G05805      (1) --------------------------------------------------
  A.thaliana_bHLH129_9_AT2G43140      (1) --------------------------------------------------
  A.thaliana_bHLH130_9_AT2G42280      (1) --------------------------------------------------
                        Consensus    (51)
```

FIGURE 1 (continued)

```
                                          101                                              150
             H.paradoxus_EL487892     (1) --------------------------------------------------
       M.truncatula_AC141114_16.2     (1) -------------------------------------------------M
          V.vinifera_TA49422_29760    (1) --------------------------------------------------
        C.solstitialis_EH761577       (1) --------------------------------------------------
              I.nil_TA8920_35883      (1) --------------------------------------------------
     S.lycopersicum_TA38189_4081    (101) DSDSMFTALLNNNDTTNNNGTRDMNDQNQKNQLQFGTSLKQEIGEEIEFG
        S.tuberosum_TA29368_4113     (12) DSDSMFTALLNSNDTTNNNGTRDLNDQNQKNQLQFGSSLKQEIGEEIEFG
         C.sinensis_TA15331_2711      (1) --------------------------------------------------
               P.tremula_DN488299     (1) --------------------------------------------------
     P.trichocarpa_scaff_IX.1004     (83) EMDTMLARFMSSCNGSGDSSSQNLQEFGERPAIKQEGGDSEMVYQSLPGH
          P.tremula_TA18674_47664     (1) --------------------------------------------------
      P.trichocarpa_scaff_I.1785      (1) --------------------------------------------------
              G.max_TA57335_3847     (13) NGWSNSEALQEFGGKPVKQEIGESIPQEPPQQNGYSYGGSQLIYQSQQIQ
              G.max_TA63030_3847      (1) --------------------------------------------------
               G.hybrid_AJ763309      (1) --------------------------------------------------
  V.vinifera_GSVIVT00008845001       (67) VGAMKHEEEVMVEGVPQQNGYSNGSQMIYNSQPMQTI--SVHNSASPRTN
                 G.soja_CA783858      (1) --------------------------------------------------
    P.trichocarpa_scaff_XVI.437      (49) QEFEDKSAARVREEAVNYSNFPRSYSGLPPHYPRQGS--ATNSSAMDSSY
  V.vinifera_GSVIVT00025522001        (1) ---------------------MSCQSQLPPQYPRQSS--STSSSAMDGSY
  V.vinifera_GSVIVT00024649001       (39) MASMKHGAEVLQQQQNGYASGSQMMYQTSSPMPHHN---SAAPGTVENSY
             E.esula_TA10959_3993     (1) --------------------------------------------------
             S.tuberosum_CV506096     (1) --------------------------------------------------
                 G.max_BPS_22716     (90) VKEEVNQQPPDVTSMNNEPLVLQQQQQQQQQQQQSNNMYNYGSSGTQNFY
        M.truncatula_TA37090_3880     (1) --------------------------------------------------
  P.trichocarpa_scaff_III.1580       (97) SVSQINQQPQMMASMNNHSSDTRLHQHQHQQHQHG----N--YSASQGFY
              R.communis_EG683575     (1) --------------------------------------------------
              H.ciliaris_EL431737     (1) --------------------------------------------------
          T.officinale_DY832981       (1) --------------------------------------------------
              I.nil_TA9289_35883      (1) --------------------------------------------------
          H.vulgare_TA45248_4513      (1) --------------------------------------------------
                 Z.mays_BPS_30292    (67) DHAAADNVLARFLAGHHSETRDCKPPRPAAAAHFMDEAAVASMAASQQQQ
           O.sativa_Os08g0506700     (52) ASPDADNVFSRFLADHQIRDKSPPATAAAAAAAAHFPDDPTMATQHHHQQ
           O.sativa_Os09g0487900     (70) PDHAADTVLARFLAGHGGHDNKPPR-----PAAHFAPPEDSMASHQQQLM
          Z.mays_TA16655_4577999     (49) APSAADNVFSRFLPDHHIRDDKPSP---------AHFPSAADMASHHQEQ
        Z.officinale_TA5709_94328     (1) --------------------------------------------------
             I.nil_TA15694_35883      (1) --------------------------------------------------
        Z.officinale_TA6615_94328     (1) --------------------------------------------------
                  B.napus_BPS_9258    (1) --------------------------------------------------
          G.hirsutum_TA24161_3635    (40) ----------NLVGHYFSAADSSSLT---SESTCKVSSSNDPREPK----
    P.trichocarpa_scaff_XIV.978      (55) ----------VG-RQQHFSGDSPSIT---SESTCKVNSSSCDRKAP----
  V.vinifera_GSVIVT00005670001       (51) ----------HIMPHQYFSGDSSS------ESTCKVAAPPDHKEAGAGGP
                 G.max_BPS_39182     (44) ----------R--PSPYFSGESS-------ESPCKEQ------RS-----
          B.oleracea_TA6610_3712     (58) ----------NFLGDFFAGADSSSLR---SESMTCGVNSSDGQKHLGSNN
           L.saligna_TA4923_75948    (71) ----------VGGGG--------------TPTRFFSPPDSTSSHLSTG--
      N.benthamiana_TA8284_4100      (64) ----------QHLGPSRYYSPNLASSNSLNSESTSKAKEQSSLQRSIG--
```

FIGURE 1 (continued)

```
                                          101                                              150
N.benthamiana_TA8285_4100          (64)  ----------QHLGPSRYYSPNMASSNSLNSESTSKAKEQSSLQRSIG--
S.tuberosum_TA26686_4113           (50)  --------------------------------------EQSNLQRSIG--
M.truncatula_AC149471_36.2         (58)  -------------------------------QHYFSDDSTQQQQHHHQ--
H.vulgare_TA47636_4513              (1)  --------------------MRRFLPAGAGEPSSSSSSGPHGKHEGSEAA
T.aestivum_TA88301_4565             (1)  --------------------------------------------------
O.sativa_LOC_Os02g39140.1           (1)  -------------------MMRRFLPVGGGGGGGVEPSS--SSTTPQRGE
S.bicolor_TA33134_4558              (1)  --------------------------------------------------
Z.mays_BPS_3797                     (1)  --------------------------------------------------
O.sativa_Os04g0489600              (21)  RYGGGLQFFADAPPAGVEGGAATARTFFPVPGGGGEQQPPERAMRQQHYG
S.officinarum_CA143325              (1)  --------------------------------------------------
S.officinarum_TA45654_4547          (1)  --------------------------------------------------
Z.mays_BPS_52761                    (1)  --------------------------------------------------
O.sativa_Os01g0900800              (60)  QLPPPPPPQQQQMVGRYFSAESSGLTSC--ESSCRTTTTTSTAAAADVGR
Z.officinale_TA2571_94328          (45)  ----------ERMMTRFYAGDSPCLTS---ESSCWAPDGDAAAAAAAGGC
B.oleracea_EH428573                 (1)  --------------------------------------------------
C.clementina_TA5735_85681           (1)  --------------------------------------------------
C.sinensis_TA15236_2711             (1)  --------------------------------------------------
G.raimondii_TA12344_29730           (1)  --------------------------------------------------
P.trichocarpa_scaff_XIX.717         (1)  --------------------------------------------------
C.intybus_EH689338                  (1)  --------------------------------------------------
L.sativa_TA5039_4236                (1)  --------------------------------------------------
L.serriola_DW114802                 (1)  --------------------------------------------------
C.tinctorius_EL407531               (1)  --------------------------------------------------
H.petiolaris_DY944559               (1)  --------------------------------------------------
V.vinifera_GSVIVT00001847001        (1)  --------------------------------------------------
I.nil_TA9081_35883                  (1)  --------------------------------------------------
S.lycopersicum_TA46743_4081         (1)  --------------------------------------------------
S.tuberosum_TA37004_4113            (1)  --------------------------------------------------
G.max_TA56453_3847                  (1)  --------------------------------------------------
C.obtusa_BW987363                   (1)  --------------------------------------------------
S.tuberosum_TA40158_4113            (1)  --------------------------------------------------
P.patens_121037_e_gw1.34.393.1      (1)  --------------------------------------------------
P.patens_148201_e_gw1.273.42.1      (1)  --------------------------------------------------
P.patens_TA27139_3218               (1)  --------------------------------------------------
P.patens_TA32785_3218               (1)  --------------------------------------------------
P.taeda_TA15788_3352                (1)  --------------------------------------------------
Z.officinale_TA334_94328            (1)  --------------------------------------------------
A.thaliana_bHLH080_9_AT1G35460      (1)  --------------------------------------------------
A.thaliana_bHLH081_9_AT4G09180      (1)  --------------------------------------------------
A.thaliana_bHLH122_9_AT1G51140      (1)  ------------MESEFQQHHFLLHDHQHQRPRNSGLIRYQSAPSSYFSS
A.thaliana_bHLH128_9_AT1G05805      (1)  ------------------MYQSSSSTSSSSQRSSLPGGGGLIRYGSAPGS
A.thaliana_bHLH129_9_AT2G43140      (1)  --------------------------------------------------
A.thaliana_bHLH130_9_AT2G42280      (1)  ---------------------------------MDSNNHLYDPNPTGSGL
Consensus                         (101)
```

FIGURE 1 (continued)

```
                                          151                                              200
          H.paradoxus_EL487892      (1)   --------------------------------------------------
    M.truncatula_AC141114_16.2      (2)   DLNSHYQQLQQNQPNSRLLRFRSSFKQQGEGCGGSATAATNSGETSSSTF
        V.vinifera_TA49422_29760    (1)   --------------------------------------------------
     C.solstitialis_EH761577        (1)   --------------------------------------------------
           I.nil_TA8920_35883       (1)   --------------------------------------------------
  S.lycopersicum_TA38189_4081     (151)   NENGVQNRYENG------GVSYSVGVQMQTRANLSNGNGDSDLIRQNSSP
     S.tuberosum_TA29368_4113      (62)   NENGVQNRYENG------GVSYGVGVQMQTRANSSNGNGGSNLIRQNSSP
      C.sinensis_TA15331_2711       (1)   --------------------------------------------------
          P.tremula_DN488299        (1)   --------------------------------------------------
P.trichocarpa_scaff_IX.1004       (133)   NLVTDNSVSVGNSMDSAFNVMSSMALENSMQATKMSTANGSNLARQNSSP
      P.tremula_TA18674_47664       (1)   --------------------------------------------------
  P.trichocarpa_scaff_I.1785        (1)   ----------------------------------MSTRNGSNLARQNSSP
          G.max_TA57335_3847       (63)   GLPNGGSSSASGSAFDGSFGVVHSMASEDSIQSKMGIRNCSNLFRQKSSP
          G.max_TA63030_3847        (1)   --------------------------------------------------
          G.hybrid_AJ763309         (1)   --------------------------------------------------
V.vinifera_GSVIVT00008845001      (115)   MESSFMTSMAAENS------------------MKIRNENCSSLVRQSSSP
              G.soja_CA783858       (1)   --------------------------------------------------
P.trichocarpa_scaff_XVI.437        (97)   GLIGSISMGHHEQL------------------KRV----DPSLARQNSSP
V.vinifera_GSVIVT00025522001       (28)   GVVNSN-NMEAKQ--------------------------GSSLVRQSSSP
V.vinifera_GSVIVT00024649001       (86)   SAVSSMGMDQSQQ-------------------IKIGGGNNSNLIRHSSSP
        E.esula_TA10959_3993        (1)   --------------MDYRMGNSRAGAGTGRLSGAKNGGNSSNLLRHNSSP
        S.tuberosum_CV506096        (1)   --------------------------------------------------
              G.max_BPS_22716     (140)   QSTGRPPLPNQ---------------------MKTGHGSSSNLIRHGSSP
   M.truncatula_TA37090_3880        (1)   --------------------------------------------------
P.trichocarpa_scaff_III.1580      (141)   QSRSKPPLPDHNPGSGMNHRSTNSTGLERMPSMKPSSGNNPNLVRHSSSP
         R.communis_EG683575        (1)   --------------------------------------------------
         H.ciliaris_EL431737        (1)   --------------------------------------------------
      T.officinale_DY832981         (1)   ------------------------------MIYQNHQQSDHNHNSTVSGS
          I.nil_TA9289_35883        (1)   --------------------------------------------------
       H.vulgare_TA45248_4513       (1)   --------------------------------------------------
             Z.mays_BPS_30292     (117)   LMYQSQQEQQMAAMEGLYRNVSSGGTEHGAAVGAGN----NSLIRQSSSP
       O.sativa_Os08g0506700      (102)   QMMFQHHPQQMASVEGLYRTVSSTGIDAATAAANAAGGGGGGLLRQSSSP
       O.sativa_Os09g0487900      (115)   YQSHQQQQQMASAMEGLYRTVSSGGTESTAAAAGNS------LLRQSSSP
      Z.mays_TA16655_4577999       (91)   MMFHSQSQSQHQQETGRSGGGLYRTVSSGMEAGGTGVGAASSLIRQSSSP
   Z.officinale_TA5709_94328        (1)   --------------------------------------------------
         I.nil_TA15694_35883        (1)   --------------------------------------------------
   Z.officinale_TA6615_94328        (1)   --------------------------------------------------
              B.napus_BPS_9258      (1)   --------------------------------------------------
      G.hirsutum_TA24161_3635      (73)   --------------SAAAAAPAPPHPPPHHPHGSYPAPSSSSLLRQRSSP
P.trichocarpa_scaff_XIV.978        (87)   -------------------KSGGGGGLQRSYGLNEIAHGAGSLVRQRSSP
V.vinifera_GSVIVT00005670001       (85)   AAMLQRSYG---FSEMPVAGFSTASSLKGGGEGGGGGGSAPSLIRHSSSP
              G.max_BPS_39182      (64)   -------------------------------FHNHPPSFASSLLRQKNSL
      B.oleracea_TA6610_3712       (95)   NNNNNNNKDLLLDRSYGGFNEISQHKINNHVGGGGSSSGSYSLARQRSSP
       L.saligna_TA4923_75948      (95)   -------------DRLQTTSFRLNEFATAFNGLKGTSQTPSPLFRHGSSP
 N.benthamiana_TA8284_4100        (102)   -------------FNDLTIGGGSGSDGGGGCGVLPAANSTTPLIRHSSSP
```

FIGURE 1 (continued)

```
                                          151                                              200
       N.benthamiana_TA8285_4100  (102)  -------------FNDLTIGGGGG-SGGSGCGFMPAANSTTPLVRHSSSP
        S.tuberosum_TA26686_4113   (60)  -------------FNDLTIGGGGG-GGGG----L-STTSTTPLVRHSSSP
     M.truncatula_AC149471_36.2    (76)  --------------------QQQQQQQRSSYEGQGGGFDGSALLRQKSSP
          H.vulgare_TA47636_4513   (31)  AAAGGLRYGGGDISLGRGNDLLHAQFRGG--EGEMKDDGADMLARHSSSP
         T.aestivum_TA88301_4565    (1)  -----------------------------------------MLARHSSSP
     O.sativa_LOC_Os02g39140.1     (30)  AEAAGLRFGGGDISLGPHGGGGGGGGGGGGHGHQLQDGSVDLLARHSSSP
          S.bicolor_TA33134_4558    (1)  -------------------------------------------MKPSSSG
                 Z.mays_BPS_3797    (1)  --------------------------------------------------
         O.sativa_Os04g0489600     (71)  GGGSGAAEISLGHGHGHGGKHHFHQFGVEAKDGGGGGDQSGFLTRHNSSP
         S.officinarum_CA143325     (1)  --------------------------------------------------
      S.officinarum_TA45654_4547    (1)  --------------------------------------------------
                Z.mays_BPS_52761    (1)  --------------------------------------------------
         O.sativa_Os01g0900800    (108)  HP---------------L----ERAYGGSGEIHVDASSAAVPLFRHSSSP
      Z.officinale_TA2571_94328    (82)  S----------------------RGSYGSGELQLSSATG-SPLVRHSSLP
             B.oleracea_EH428573    (1)  --------------------------------------------------
      C.clementina_TA5735_85681     (1)  -------------MQPTAGGSSSSSGGGGGGVGGRGELSRGGLARLRSAP
        C.sinensis_TA15236_2711     (1)  -------------MQPTAGGSSSSSGGGGGGVGGRGELSRGGLARLRSAP
     G.raimondii_TA12344_29730      (1)  -------------MKPTPGGSSSSSGGG-------AEGSRAGLARFRSAP
   P.trichocarpa_scaff_XIX.717      (1)  -------------MQPTPAGSSNTSAGGG----SAGGGGGGGIARFRSAP
             C.intybus_EH689338     (1)  -------------MQPT----GSGGG----------------LSRFRSAP
          L.sativa_TA5039_4236      (1)  -------------MQPT----GSGGGGG--------------LSRFRSAP
           L.serriola_DW114802      (1)  -------------MQPT----GSGGGGG--------------LSRFRSAP
          C.tinctorius_EL407531     (1)  -------------MQPP----GGAGG----------------LSRFQSAP
         H.petiolaris_DY944559      (1)  -------------MQPT----HGGTSGGSG-------GTGTGLSRFRSAP
   V.vinifera_GSVIVT00001847001     (1)  --------------------------------------------------
              I.nil_TA9081_35883    (1)  -------------MQPE----SGAGGGGS------EPSRGAGLTRFRSAP
     S.lycopersicum_TA46743_4081    (1)  -------------MQRG----TTGDGGG-------------GLSRFRSAP
        S.tuberosum_TA37004_4113    (1)  -------------MQRG----TAGAGGG-------------GLSRFRSAP
              G.max_TA56453_3847    (1)  --------------------------------------------------
              C.obtusa_BW987363     (1)  --------------------------------------------------
        S.tuberosum_TA40158_4113    (1)  --------------------------------------------------
 P.patens_121037_e_gw1.34.393.1     (1)  --------------------------------------------------
 P.patens_148201_e_gw1.273.42.1     (1)  --------------------------------------------------
          P.patens_TA27139_3218     (1)  --------------------------------------------------
          P.patens_TA32785_3218     (1)  --------------------------------------------------
           P.taeda_TA15788_3352     (1)  --------------------------------------------------
       Z.officinale_TA334_94328     (1)  --------------------------------------------------
A.thaliana_bHLH080_9_AT1G35460      (1)  -----------------MQSTHISGGSSGGGGGGGGEVSRSGLSRIRSAP
A.thaliana_bHLH081_9_AT4G09180      (1)  ---------------MQPTSVGSSGGGDDGGRGGGGGLSRSGLSRIRSAP
A.thaliana_bHLH122_9_AT1G51140     (39)  FGESIEEFLDRPTSPETERILSGFLQTTDTSDNVDSFLHHTFNSDGTEKK
A.thaliana_bHLH128_9_AT1G05805     (33)  FLNSVVDEVIGGGSSNARDFTGYQPSSDNFIGNFFTGAADSSSLRSDSTT
A.thaliana_bHLH129_9_AT2G43140      (1)  ------------------------MYPPNSSKSTAHDGGGDADTNQYDSA
A.thaliana_bHLH130_9_AT2G42280     (18)  LRFRSAPSSVLAAFVDDDKIGFDSDRLLSRFVTSNGVNGDLGSPKFEDKS
                      Consensus   (151)                                            L R  SSP
```

FIGURE 1 (continued)

```
                                        201                                              250
          H.paradoxus_EL487892    (1)   --------------------------------------------------
      M.truncatula_AC141114_16.2 (52)   REFMDHNPSNHKVDNNESSLSQRHMNSQQG--------YRMDQHKGFYTN
        V.vinifera_TA49422_29760  (1)   --------------------------------------------------
      C.solstitialis_EH761577     (1)   -------------------------------------------------M
           I.nil_TA8920_35883     (1)   --------------------------------------------------
  S.lycopersicum_TA38189_4081   (195)   AGFFN-------------------GFMREVGNFG-ASVGTNREASTSTNG
      S.tuberosum_TA29368_4113  (106)   AGFFN-------------------GFMREVGNFG-ASVGTNKEASTSTNG
       C.sinensis_TA15331_2711    (1)   ------------------------------------MPQIAETGNQSMGN
          P.tremula_DN488299      (1)   --------------------------------------------------
  P.trichocarpa_scaff_IX.1004   (183)   AGLFSDLGVDN-----------GFVVMREGGSFR-AGNGTNGEASP-TNK
      P.tremula_TA18674_47664     (1)   --------------------------------------------------
   P.trichocarpa_scaff_I.1785    (17)   AGILSNHGVDN-----------GFAVMRNAGSFR-AGNGTNGEATPSTSR
          G.max_TA57335_3847    (113)   AAFFSIENDLAALREVGSFKADDVSN----G---------------LVTA
          G.max_TA63030_3847      (1)   --------------------------------------------------
          G.hybrid_AJ763309       (1)   --------------------------------------------------
V.vinifera_GSVIVT00008845001    (147)   PGLFPNLTSENGICFSSFLHSFLFQTWLVMGNLRAGNSTNVE-ANPSVSR
             G.soja_CA783858      (1)   -----------------------------------------------MNR
P.trichocarpa_scaff_XVI.437     (125)   AGLFGNVSVQN-----------GYPGWNGTN-------------GEANSR
V.vinifera_GSVIVT00025522001     (51)   AGLFSHLSGQN-----------GYAIMRGVGNFRPGNVGNGE-ISPSTSR
V.vinifera_GSVIVT00024649001    (117)   AGLFSHLNVEN-----------GYAIMRGMGNFGSGSGTNGEPSFSSASR
         E.esula_TA10959_3993    (37)   AGLFSNITVEMENG-----YAVMRGMGDYVTPKREAS------------Y
        S.tuberosum_CV506096      (1)   --------------------------------------------------
             G.max_BPS_22716    (169)   AGLFSNINIDITG---------YAAVVRGMGTMGAAANN----TSEEANF
     M.truncatula_TA37090_3880    (1)   --------------------------------------------------
P.trichocarpa_scaff_III.1580    (191)   AGLFSNINIEFEN---------GYAVLRDVGDLGAGNRD----TTYSAAG
         R.communis_EG683575      (1)   --------------------------------------------------
          H.ciliaris_EL431737     (1)   --------------------------------------------------
        T.officinale_DY832981    (21)   TSALVNPLATMNPPMNLLRQSSSPAGFFEHINMDNGYSTMRSMDKFRTAN
           I.nil_TA9289_35883     (1)   --------------------------------------------------
        H.vulgare_TA45248_4513    (1)   --------------------------------------------------
            Z.mays_BPS_30292    (163)   AGFLNHLNMDNGYG-----SMLRVGMGGGGFRNG---------------V
      O.sativa_Os08g0506700     (152)   AGFLNHLNMDNGYG-----SMLRAGMAAAGGGVGFRNGANAAAAADSPGG
      O.sativa_Os09g0487900     (159)   AGFLNHLTMDNGYG-----NMLRAGMGGGGG------------------G
       Z.mays_TA16655_4577999   (141)   AGFLDHFGMDNGYG-----AMLRASMGMGFQDGG------ASDSLAGGGG
     Z.officinale_TA5709_94328    (1)   --------------------------------------------------
          I.nil_TA15694_35883     (1)   --------------------------------------------------
     Z.officinale_TA6615_94328    (1)   --------------------------------------------------
              B.napus_BPS_9258    (1)   --------------------------------------------------
       G.hirsutum_TA24161_3635  (109)   AGFLSHLTSE-NGFS--VTMGNRNYSSQ--------------------GS
  P.trichocarpa_scaff_XIV.978   (118)   AGFLSHLATENGGFS--ITRGTGGYNSR--------------------NG
V.vinifera_GSVIVT00005670001    (132)   AGFLNQLTADN------LTRGTGNYSSQ--------------------GG
              G.max_BPS_39182    (83)   DGFLSHLS-----------------KHH--------------------GV
       B.oleracea_TA6610_3712   (145)   ADFFSYLSGEKNNFS--LNQPTSDYNQQ--------------------GG
        L.saligna_TA4923_75948  (132)   AGFLNTLVSSTPTDG---------R-----------------------G-
    N.benthamiana_TA8284_4100   (139)   ARFLNQLAAAAGDT---VSMGRGSYNSK--------------------GI
```

FIGURE 1 (continued)

```
                                         201                                              250
      N.benthamiana_TA8285_4100  (138)  AKFLNQLAAVAGDTGFSASMGRGSYNSK--------------------GV
        S.tuberosum_TA26686_4113   (91)  ARFLNQLATAAGDTGFSVSMGRGSYNSK--------------------GG
    M.truncatula_AC149471_36.2  (107)  AGFLNHLATLNHNNN---N-NNNNNNAG--------------------GG
          H.vulgare_TA47636_4513   (79)  AGFFSNLMVDDGYHGSRGAGVAGGS-----------------------GE
         T.aestivum_TA88301_4565   (10)  AGFFSNLMVDDGYHGSRGAGVAGGSS---------------------GGE
     O.sativa_LOC_Os02g39140.1   (80)  AGFFSNLMASNGFPGSKGGGGSGAEAHHHPSM---------------AGS
          S.bicolor_TA33134_4558    (8)  FNFAGGTQQQGQQQGAACGHLSRIS-------------------------
                  Z.mays_BPS_3797    (1)  --------------------------------------------------
        O.sativa_Os04g0489600  (121)  PGFFSSPVMDNGFSSSARPAGSSLGEVR-------------------HGA
      S.officinarum_CA143325    (1)  --------------------------------------------------
   S.officinarum_TA45654_4547    (1)  --------------------------------------------------
                 Z.mays_BPS_52761    (1)  ---------------MDNGFPSDRVEVG-------------------GEV
        O.sativa_Os01g0900800  (139)  AGLLSRLMADPHGNGMAATRGMGGYSGG--------------------GG
     Z.officinale_TA2571_94328  (109)  TGFFSHLLADHAGN--SSTRMIGNYSQA--------------------GT
            B.oleracea_EH428573    (1)  --------------------------------------------------
     C.clementina_TA5735_85681   (38)  ASWIDALLEEELE--DPLKPNQCLTQLLSSGNP-----------------
       C.sinensis_TA15236_2711   (38)  ASWIDALLEEELE--DPLKPNQCLTQLLSSGDP-----------------
     G.raimondii_TA12344_29730   (31)  ATWLEALLEEEEE--DPLKPSQCLTQLLTGNST-----------------
  P.trichocarpa_scaff_XIX.717   (34)  ATWIEALLGEEEE--DPLKQSQN-------------------------LT
             C.intybus_EH689338   (18)  ATWLEALLESEEE---DVIIDPPKPS-------LTTTQP----------S
            L.sativa_TA5039_4236   (20)  ATWLEALLESEEE---DVIIDPPKP--------PPHQQA----------S
            L.serriola_DW114802   (20)  ATWLEALLESEEE---DVIIDPPKP--------PPHQQA----------S
          C.tinctorius_EL407531   (18)  ATWLETLLESEEEEEQDVIIDPPKP-------------------------
          H.petiolaris_DY944559   (27)  ATWLEALLESEEE---DVIIDPPKPTLTPPIVPRVHQHT----------S
 V.vinifera_GSVIVT00001847001    (1)  --------------------------------------------------
             I.nil_TA9081_35883   (28)  ATWLEALLDSDTE--NDVVLDPP-PILPSSSKPPPHPHSQPLPTPPAQLK
   S.lycopersicum_TA46743_4081   (21)  ATWLEALLESDTE--NEVILNPSSPILHTPNKPPPHPST-------PKLK
       S.tuberosum_TA37004_4113   (21)  ATWLEALLESDTE--NEVILNPSSPILHTPKKPPPHPSTPKL----PELK
              G.max_TA56453_3847    (1)  --------------------------------------------------
              C.obtusa_BW987363    (1)  --------------------------------------------------
       S.tuberosum_TA40158_4113    (1)  --------------------------------------------------
P.patens_121037_e_gwl.34.393.1    (1)  --------------------------------------------------
P.patens_148201_e_gwl.273.42.1    (1)  --------------------------------------------------
           P.patens_TA27139_3218    (1)  --------------------------------------------------
           P.patens_TA32785_3218    (1)  --------------------------------------------------
            P.taeda_TA15788_3352    (1)  --------------------------------------------------
      Z.officinale_TA334_94328    (1)  ----------------------MEQTKNGGAAAS------GSSNLVRHRS
A.thaliana_bHLH080_9_AT1G35460   (34)  ATWIETLLEEDEE---------EG--LK---------------------P
A.thaliana_bHLH081_9_AT4G09180   (37)  ATWLEALLEEDEE-------------------------------------
A.thaliana_bHLH122_9_AT1G51140   (89)  PPEVKTEDEDAEIPVTATATAMEVVVSGDGEISVNPEVSIGYVASVSRNK
A.thaliana_bHLH128_9_AT1G05805   (83)  CGVNNSSDGQKQLGNNNNNNSNKDIFLDRSYGGFNEISQQHKSNDIGGGN
A.thaliana_bHLH129_9_AT2G43140   (27)  AGATRDFSSLGPQTHHHPPPQRQQQHQQNPNLVGHYLPGEPSSIGFDSNA
A.thaliana_bHLH130_9_AT2G42280   (68)  PVSLTNTSVSYAATLPPPPQLEPSSFLGLPPHYPRQSKGIMNSVGLDQFL
                        Consensus  (201)
```

**FIGURE 1 (continued)**

```
                                        251                                              300
             H.paradoxus_EL487892    (1) --------------------------------------------------
       M.truncatula_AC141114_16.2   (94) LLRQSSSHDGHFSNNNIISFGNGYEPMKGVENYDGVKDSDGELTLSMNIL
         V.vinifera_TA49422_29760    (1) --------------------------------------------------
        C.solstitialis_EH761577      (2) NSTSTNLIRQSSSPAGFLSSLNAETGMRDANQVISSNGLNSNHIGFS---
              I.nil_TA8920_35883     (1) --------------------------------------------------
     S.lycopersicum_TA38189_4081   (225) FNNHISYSTNQ--------------------------SST----------
       S.tuberosum_TA29368_4113   (136) FSNHISYSTNQ--------------------------SST----------
         C.sinensis_TA15331_2711    (15) SSPEKNFVGNN--------------------------GA-----------
              P.tremula_DN488299     (1) --------------------------------------------------
    P.trichocarpa_scaff_IX.1004   (220) LRRHVNFSSGQRM-----LPQIAEIGEECIGGRSPEGDVS----------
       P.tremula_TA18674_47664      (1) --------------------------------------------------
     P.trichocarpa_scaff_I.1785    (55) LRSHVNFSSGHRT-----LPQIAEIGEECIGGSSPEGDFS----------
             G.max_TA57335_3847   (144) STGGLHSSHTFSSRPSSCLKRLPQIAENGNESLEENCDQSRNLVNDN---
             G.max_TA63030_3847     (1) --------------------------------------------------
             G.hybrid_AJ763309      (1) --------------------------------------------------
 V.vinifera_GSVIVT00008845001   (196) LNNQISFSSGLSS-CNGLLPKISEIRNENIGLHSPKDGNNSNSRC-----
                G.soja_CA783858     (4) MKNHISFSSISPSSSLGMLSPTSKMGTEGIRVTRPEDGRQGSCNGD----
   P.trichocarpa_scaff_XVI.437   (151) LKSQLSLSSRAPS-SLGFRSQISEIGSESIEAGSPSADSRFHS-------
 V.vinifera_GSVIVT00025522001    (89) LKPQVSFSSGLPS-SLGLLPQISEIG---SDPG-----------------
 V.vinifera_GSVIVT00024649001   (156) LKGQINFSSGPPS-SSGLVTPISEMGNKSMGTGSPDNGSFGEGHSN----
          E.esula_TA10959_3993    (70) SPSPATSRPPPAATSGGRMSPITEIGNTNNNNIGENNNSDSNCVYDE---
          S.tuberosum_CV506096     (1) --------------------------------------------------
               G.max_BPS_22716   (206) SPATRMKNNAPNF-SSGLMSSRAEVGNKSNTQNNNAENEGFAESQG----
     M.truncatula_TA37090_3880     (1) --------------------------------------------------
  P.trichocarpa_scaff_III.1580   (228) RPPS----------SSGIRSTIAEMGNK-NMGENSPDSGGFGETPG----
            R.communis_EG683575     (1) --------------------------------------------------
            H.ciliaris_EL431737     (1) -----------------MMRSIEEFRLLSRIPEHEGKNNYPGGS------
         T.officinale_DY832981    (71) GSVPDSAKRFENQIAFLSGLHSSSRPLSLIPEHEPKTMRISGAHNNAGFN
             I.nil_TA9289_35883     (1) --------------------------------------------------
        H.vulgare_TA45248_4513     (1) ------------------MSQISEMVGEEMGGGGSSGDDDEAGS------
              Z.mays_BPS_30292   (193) SDARLKGQLSFSSRQGSVMSQISEVGSEELDGGGGSGSPEAAGSNA----
         O.sativa_Os08g0506700   (197) SGGRLKGQLSFSSRQGSLMSQISEMDSEELGGS---------SPEG-AGG
         O.sativa_Os09g0487900   (186) GDPRLKGQLSFSSRQGSVMSQISEMGSEDEELAGGGGSPEAGSNGGGAAR
        Z.mays_TA16655_4577999   (180) GSGRLGGQLSFSSRQGSLMSQISEMDSQEDVVGASSPD------------
     Z.officinale_TA5709_94328     (1) ------------------MSQISEMGDEELGGSSPDES------------
            I.nil_TA15694_35883     (1) --------------------------------------------------
     Z.officinale_TA6615_94328     (1) ------------------MRSGLERFQSVPSSFLGEICGEFLAVRPEIDT
               B.napus_BPS_9258     (1) --------------------------------------------------
        G.hirsutum_TA24161_3635   (136) G-NGGHGVSRLKSQLSFTR--QDSLSQISEVSENLVDGVSSNSN------
  P.trichocarpa_scaff_XIV.978   (146) S-GGG--PSRLKSQLSFTR--QDSLSQISEVSENVVEGIGSDNG------
 V.vinifera_GSVIVT00005670001   (156) C-NGGHGISRLKSQLSFKR--QDSLSQISEVSENMVDGISSDNG------
               G.max_BPS_39182    (96) G-FNHHPRVVIKCKFKFNC--SLLKSQLSFTHESLSNTVNVDP-------
        B.oleracea_TA6610_3712   (173) S-NAGRGPSRLKSQLSFTS--HDPLSRISEVNETSVHDGSGHS-------
         L.saligna_TA4923_75948   (149) -------S-RLSSQLSFTG--TSSFSQISEEN------------------
   N.benthamiana_TA8284_4100   (166) A-DSSCGITRLNSQLSFTR--QEALSQIAEENEDVEGTSTDNGH------
```

FIGURE 1 (continued)

```
                                         251                                              300
         N.benthamiana_TA8285_4100 (168) A-DSSRGITRLNSQLSFTR--QEVLSQIAEENEDVEGTSTEDGY------
          S.tuberosum_TA26686_4113 (121) G-DSGRGITRLNSQLSFTR--QEALSQIAEENEDIEGTSIANGH------
      M.truncatula_AC149471_36.2   (133) F-TITRGNSRLKSELSFTGGGQECLSRISENVVDYASAAAAAGNG-----
            H.vulgare_TA47636_4513 (106) AHRNTSSSTKMKSQLSFTSGGPQTAAHLSRISEGASLFPGADVVRAA---
          T.aestivum_TA88301_4565   (39) AHRHASSSTKMKSQLSFTAGGQQTTAHLSRISEGASLFPSADAVRAA---
       O.sativa_LOC_Os02g39140.1 (115) GSGSSSGGRKMKSQLSFTAGPP----HLSHIAEDG--------A-FP---
            S.bicolor_TA33134_4558  (33) --EDGCASFPAGGLVGDRAAGG-----------------------RG---
                  Z.mays_BPS_3797    (1) -------------MMGDRAG-------------------------RG---
          O.sativa_Os04g0489600    (152) MSSSSNNNKKMKAPLSFASSRQGS-GGLSQISEDGIPDLTDSIHGAA---
          S.officinarum_CA143325     (1) --------------------------------------------------
       S.officinarum_TA45654_4547    (1) --------------------------------------------------
                 Z.mays_BPS_52761   (17) HHAMRDYHKKMKSPLNLSKQ--------GALSQLCEHGLSDGLTNNV---
          O.sativa_Os01g0900800    (169) D-AGAMAHRRLSSQWSFSRQDLPQISEMGGLIPDIGESIVTGGGGNS---
         Z.officinale_TA2571_94328 (137) ESIHVLAHRRQHAPWGFSRQDS------LSQISELSIPEMEESDTHH---
              B.oleracea_EH428573    (1) --------------------------------------------------
         C.clementina_TA5735_85681  (69) --------VSVTAGLSLSQSQ-------LDQVGFQRQNSS----------
          C.sinensis_TA15236_2711   (69) --------VSVTAGLSLSQSQ-------LDQVGFQRQNSS----------
        G.raimondii_TA12344_29730   (62) --------STPTIRNSLLFPNSAYPSGLFEPSGFQRQNSSPAD-------
      P.trichocarpa_scaff_XIX.717   (57) ELLTSNTPSSRDSVPFNASSAAVEPGLFEPVGGFQRQNSSP---------
               C.intybus_EH689338   (48) ISATSS--RPTYADPNIFLPSP----------VSLRQNSSP---------
             L.sativa_TA5039_4236   (49) IAGTPTPSRPTYVDPNIFLPS--------------RQNSSP---------
              L.serriola_DW114802   (49) IAGTPTPSRPTYVDPNIFLPS--------------RQNSSP---------
             C.tinctorius_EL407531  (43) TIGGIY------LDPNLLLPIPP-----AAAVVGRRQNSSP---------
            H.petiolaris_DY944559   (64) IATTTSDTKPTFVDPNLLIPNP----------IGLRQNSSP---------
    V.vinifera_GSVIVT00001847001     (1) --------------------------------------------------
               I.nil_TA9081_35883   (75) QASVGGSGSRYAADLGLFESGGGGGEAASGLSNFVRQNSSP---------
      S.lycopersicum_TA46743_4081   (62) LETGG--ATRFTGDPGLFESGGS--------SNFLRQNSSP---------
          S.tuberosum_TA37004_4113  (65) LETGG--ATRFTGDPGLFESGGS--------SNFLRQNSSP---------
               G.max_TA56453_3847    (1) --------------------------------------------------
                C.obtusa_BW987363    (1) --------------------------------------------------
          S.tuberosum_TA40158_4113   (1) --------------------------------------------------
   P.patens_121037_e_gw1.34.393.1    (1) --------------------------------------------------
   P.patens_148201_e_gw1.273.42.1    (1) --------------------------------------------------
           P.patens_TA27139_3218     (1) ------MRCYVNQAYCLLGCRNPNISMQDRMAENGSEKNSS---------
           P.patens_TA32785_3218     (1) --------------------------------------------------
            P.taeda_TA15788_3352     (1) ---------------MPVGSSADSLGGSSSDDGSLSNGNG----------
        Z.officinale_TA334_94328    (23) SPAGVLSHLNANAGYPNISKNQISFSSIQNISEVVIAG------------
 A.thaliana_bHLH080_9_AT1G35460     (52) NLCLTELLTGNNNSGGVITSRDDSFEFLSSVEQGLYNHHQGGGFHRQNSS
 A.thaliana_bHLH081_9_AT4G09180     (50) --------------ESLKPN----LGLTDLLTGNSNDLPTSRGSFEFPIP
 A.thaliana_bHLH122_9_AT1G51140    (139) RPREKDDRTPVNNLARHNSSPAGLFSSIDVETAYAAVMKSMGGFGGSNVM
 A.thaliana_bHLH128_9_AT1G05805    (133) SSGSYSLARQRSSPADFFTYLASDKNNFSLNQPTSDYSPQG-G-------
 A.thaliana_bHLH129_9_AT2G43140     (77) SSSSSLFRHRSSPAGFYDQHLPTDPNGTGFSLGRPNGGYGGGGEQGPSRL
 A.thaliana_bHLH130_9_AT2G42280    (118) GINNHHTKPVESNLLRQSSSPAGMFTNLSDQNGYGSMRNLMNYEEDEESP
                        Consensus  (251)
```

**FIGURE 1 (continued)**

```
                                           301                                            350
             H.paradoxus_EL487892    (1) --------------------------------------------------
     M.truncatula_AC141114_16.2  (144) NNQIGFSPRTPSSFRMLSQNPKTGSDGIGTTSHDDRRQVGSNDDAQYYGH
         V.vinifera_TA49422_29760    (1) ----------MYNVSQASPHDMSLMYSSNFKHSGEEE--------TEKTR
      C.solstitialis_EH761577   (49) ------SAPSSSPMYLPQIAENRNEMNDSTFRSLKRMTDGDSNMF-HTSI
            I.nil_TA8920_35883    (1) ---------------MPSIAENES-WNDSSFNCLKRSRDGDFKML-SALN
   S.lycopersicum_TA38189_4081  (239) ------SNFMPSIAENESWNDASFN-----SLKRNRDGDLKMFSTNFNGM
      S.tuberosum_TA29368_4113  (150) ------SNFMPSIAENESWNDSSFN-----SLKRSRDGDLKMFSNSFSGM
       C.sinensis_TA15331_2711   (28) ------SRQYMPNFFSDPWDDASFS-----GVKRARDSTCNMSFG-LDAY
            P.tremula_DN488299    (1) ----------MSRFSSDSWDGVSLS-----GLKRQRDHDGNMFSG-LNTL
  P.trichocarpa_scaff_IX.1004  (255) ------EARYMSRFTSDSWDGASLS-----GLKRQRDNDGNMFSG-LNTL
       P.tremula_TA18674_47664    (1) ----------MYNFNSDTWG----------DASRLKDNDGNMFSA-LNTR
   P.trichocarpa_scaff_I.1785   (90) ------KRKYMYNFNSDTWG----------DASRLKDNDGNMFSG-LNRR
           G.max_TA57335_3847  (191) ----GSSKCYIPSFTNELWESSAFNAPKTENEDEIMFSTSNILE------
           G.max_TA63030_3847    (1) ----------MPSFTTDFWDGSAFS-----ASRTASNRGEISFST--SKA
            G.hybrid_AJ763309    (1) --------------------------------------------------
V.vinifera_GSVIVT00008845001  (240) ---------YISNFTTDSWSDSPFSGLTRMAADNDLKMF----SG-LNSL
              G.soja_CA783858   (50) ------ARYYGPGFPYASWNEPSHP-------KRQRSSN-----D-ELLF
  P.trichocarpa_scaff_XVI.437  (193) ----------SHGFPYGSWNNSHLS-ENFSSMKRDQEN-----GN-LFSN
V.vinifera_GSVIVT00025522001  (118) -------------FPYGSWNDSAHF-ENFSGMKRDQDND----GK-LYSG
V.vinifera_GSVIVT00024649001  (201) ------SGGFITGFPIGSWDDSAIMSESFSSLKSVRDDEAKTFSG-LNAS
         E.esula_TA10959_3993  (117) ------SRNNDYVTGFPMGSWDHDNASSSSVMSHG-LTDDDRTLS-GGLI
        S.tuberosum_CV506096    (1) -----------------------------MKVPSE----------EFRVL
             G.max_BPS_22716  (251) ------NEFIPAGFPVGPWNDSAIMSDNVTGLKRFRDEDVKPFSG-GLNA
     M.truncatula_TA37090_3880    (1) ------------MISDNITGLKRYRDDDDVKPFPPGL------------N
 P.trichocarpa_scaff_III.1580  (263) ------N---NYDYPIGSWDDSAVMS---TGSKRYLTDDDRTLSG-LNSS
          R.communis_EG683575    (1) --------------------MSAGLKRLTDDDRTLSG------------L
          H.ciliaris_EL431737   (28) ------------------WDDSAMLTDGFLNEFEESQ-------------
        T.officinale_DY832981  (121) QNLPKTTGYVPTFPHGTSWDERAMLTDDFMKELGE----------TDQIN
            I.nil_TA9289_35883    (1) -----MDESYLSSFPIPSWDDLDLLSDDFLKVTDEKP--------SLKAK
       H.vulgare_TA45248_4513   (27) ------------YGGIPGYPVVAPSGTGWDEPSPSPPPSLLTSDG-MSGP
              Z.mays_BPS_30292  (239) ------SGAARGYSGIPGYPMGGLASGAWPDEASPSP------------T
        O.sativa_Os08g0506700  (237) GGGGGGRGYLSGYPMSSGWEESSLMSDTNISGVKRQR------------D
        O.sativa_Os09g0487900  (236) GGYGGGYAMGSSAWEEPSPPATSLLPDSSLPSKRPRD------------D
       Z.mays_TA16655_4577999  (218) ------AGGGGDAAYMPGYPMSSGGWDDSSSALLPDS------------L
     Z.officinale_TA5709_94328   (21) ------SQRYAAGVPMTSWAEASLLAG---------NNPS----G-PGNR
           I.nil_TA15694_35883    (1) ---------------MPYWEDSDILSDLFLNGSE-EK------------P
     Z.officinale_TA6615_94328   (33) MFPRFSAPNFPENFSSGGGHSRPPTLSEILIPHHKVAFPSSPLTMFHSNA
              B.napus_BPS_9258    (1) -----------------MSFGND-----SNIWDRSSSQISFT-------I
       G.hirsutum_TA24161_3635  (177) ---------HQNPAHSFAAAGFG-----MDSWDNTNSIVFSA-------P
   P.trichocarpa_scaff_XIV.978  (185) ---------SQNSTHSYSAASFG-----MESWDTPNSIVFSG-------H
V.vinifera_GSVIVT00005670001  (197) ---------HRNATHSYATASFP-----MDAWDNTNTIVFST-------T
              G.max_BPS_39182  (136) -----------------SSTTFG-----MDPWDNNSIAFSAT-------S
       B.oleracea_TA6610_3712  (213) --------------FSAASFGAA-----TDSWDDGSGSIGFT-------V
        L.saligna_TA4923_75948  (171) --------------------------------NIANSLMFS---------
    N.benthamiana_TA8284_4100  (207) ---------RKSTHSYATASSFA-----MGSWEDNNSIMFSV-------T
```

**FIGURE 1 (continued)**

```
                                        301                                              350
     N.benthamiana_TA8285_4100  (209) ---------RKSTHSYATASSFA-----MGSWEDNNSIMFSV-------T
      S.tuberosum_TA26686_4113  (162) ---------RKSTHSYASASSFA-----MGSWEDNNSIMFSV-------T
    M.truncatula_AC149471_36.2  (177) ---------SLHTNSTNTWGGGG-----PDNNNNSNSIVFSSSQTQTNNN
        H.vulgare_TA47636_4513  (153) --AHSGGEHPVSRSFSASGSSGG--FSIVGPWDESREIIGTL--------
      T.aestivum_TA88301_4565    (86) --AHSGSEHPVSRSFSASGSSGG--FSIVGPWDESREIIGTL--------
     O.sativa_LOC_Os02g39140.1  (149) --DRAGAEASVPRTFSAGGSSGGGGFSIVGPWEESRDIIST---------
        S.bicolor_TA33134_4558   (55) --SGESSSG--GAAAAARSYSGG--FSIVGPWEESRDIITT---------
               Z.mays_BPS_3797   (10) --SAGESSG--AAAAARSYSAGG--FSIVGPWEESRDIITT---------
       O.sativa_Os04g0489600    (198) --HHHGRSEENVSTHDHVVRSFSSGGFSIGSWEDSNSIVFSTS-------
       S.officinarum_CA143325     (1) --------------------------------------------------
   S.officinarum_TA45654_4547     (1) --------------------------------------------------
              Z.mays_BPS_52761   (56) --HGTGHSKENITTNNVARPFTS--GFSIGSWEDSNSIAFSNP-------
        O.sativa_Os01g0900800   (215) --SSDGAGHGAQSSSFLSSRNFS-----MSSWDDTNSIMFSPPSSSKK-A
   Z.officinale_TA2571_94328    (178) --DSDEVAGNAGQS--YISSNFQ-----LGSWDDKNSIAFSAPQSKR---
           B.oleracea_EH428573    (1) --------------------------------------------------
    C.clementina_TA5735_85681    (94) ------------PADLFDGYFSNYA---TPSSYDYVDVSPNSN-------
      C.sinensis_TA15236_2711    (94) ------------PADLFDGYFSNYA---TPSSYDYVDVSPNSN-------
    G.raimondii_TA12344_29730    (97) ------FPGNNPAASSDAYFSNFG---VVANYDYLSPTMDVSP------S
   P.trichocarpa_scaff_XIX.717   (98) -------TDFLRSSGIGSDQGYFSSYGNASNYEYMTPNMDVSP------S
            C.intybus_EH689338   (77) ------AEFLSQINNPDAYLSNYS---TANYDDYLSPSFHGVK------S
         L.sativa_TA5039_4236    (76) ------AEFFSEINNPDAYLS--------NYDDYLSSSYHNVK------P
          L.serriola_DW114802    (76) ------AEFFSEINNPDAYLS--------NYDDYLSSSYHNVK------P
         C.tinctorius_EL407531   (73) ------AEFLSETNNLHGHGYLS------NFDDYLSSSFHGGA------K
         H.petiolaris_DY944559   (95) ------AEFLSQINNPDAFLPNYS---TAGFDDYLSSSRDIGG------G
   V.vinifera_GSVIVT00001847001   (1) -----MFDGAGAQGFLRQSSLPTEFLSQINSSEGYFSSFGIPAGFDYAAS
          I.nil_TA9081_35883    (116) -------AEFLSQISSDIFFPSFG---APPSYDYLSSPMDVAQ------S
   S.lycopersicum_TA46743_4081   (93) -------AEFLSHISSDGYFSNYG---IPSSLDYLSPSVDVSQ------S
      S.tuberosum_TA37004_4113   (96) -------AEFLSHISSDGYFSNYG---IPSSLDYLSPSVDVSQ------S
           G.max_TA56453_3847     (1) ---------MQPTSGGGGLARFR-----SAPASWLESVLLKE-------E
             C.obtusa_BW987363    (1) ----------------------------------------------MDGK
      S.tuberosum_TA40158_4113    (1) ------------MAPISEFRDKVLEEKSTGNEHKNDENCITDFPMPSWED
 P.patens_121037_e_gw1.34.393.1   (1) --------------------------------------------------
 P.patens_148201_e_gw1.273.42.1   (1) --------------------------------------------------MEPPLDWSRTNS
         P.patens_TA27139_3218   (36) -------RNSDDNNHLISGYAASIYSQEDCHWKSPTSPAKRQRGLDGESE
         P.patens_TA32785_3218    (1) ------------MYSQDDLLNNDTVTSMPTSPGTPGGKRQRGFVGENEIS
          P.taeda_TA15788_3352   (26) ------VQRYMSSFRVGSWDDTVIVSEGFAGMQDGAAFSARKRVRDLNGK
     Z.officinale_TA334_94328    (61) ------SSSDDSSGYIPRFPSSNN--WENFSRRKSTI------------D
 A.thaliana_bHLH080_9_AT1G35460 (102) P-------ADFLSGSGSGTDGYFSNFGIPANYDYLSTNVDIS--------
 A.thaliana_bHLH081_9_AT4G09180  (82) V--EQGLYQQGGFHRQNSTPADFLSGSDGFIQSFGIQANYDYLSGNIDVS
 A.thaliana_bHLH122_9_AT1G51140 (189) STSNTEASSLTPRSKLLPPTSRAMSPISEVDVKPGFSSRLPPRTLSGGFN
 A.thaliana_bHLH128_9_AT1G05805 (175) ---SNGGRGHSRLKSQLSFTNHDSLARINEVNETPVHDGSGHSFSAASFG
 A.thaliana_bHLH129_9_AT2G43140 (127) KSELRFSSGSSSHQEHNSLPRISEVEAAAAARNGVASSSMSFGNNRTNNW
 A.thaliana_bHLH130_9_AT2G42280 (168) SNSNGLRRHCSLSSRPPSSLGMLSQIPEIAPETNFPYSHWNDPSSFIDNL
                     Consensus  (301)
```

**FIGURE 1 (continued)**

```
                                        351                                              400
            H.paradoxus_EL487892   (1)  -----MRRKHSRYLGVIFVDFKMDYPHLKHPVLPTLYSLTQNFTLTWNLT
      M.truncatula_AC141114_16.2 (194)  KLVYDSNDQNVGVRN----QVDTLSHHLSLPRKSS--------EMFVVEK
        V.vinifera_TA49422_29760  (33)  AVFMDSNTNYHHQQNQNQPPNSGLLRFRSAPSSLLAN---FTDSGDGVHK
        C.solstitialis_EH761577  (92)  KMDTQHNGPSGHY-------TPSLLHHMSLPNTS--------SEMAVAEK
             I.nil_TA8920_35883  (34)  GIESQNGGERRNC-------TPGLTHHLSLPSS------------VEIEK
     S.lycopersicum_TA38189_4081 (278)  TNQNDESRNYTS---------SGLSHHLSLPKTS--------SEMAAIEK
       S.tuberosum_TA29368_4113 (189)  TNQNDESRNYTS---------SGLTHHLSLPKTS--------SEMAAIEK
        C.sinensis_TA15331_2711  (66)  ETQNGNS-GNQS---------TRLVHHLSLPKTS--------AEMAAVEK
            P.tremula_DN488299  (35)  DNQDGNS-GNRA---------TGLTHHLSLPKTV--------SEMATIEK
  P.trichocarpa_scaff_IX.1004 (293)  DNQDGNS-GNRV---------TGLTHHLSLPKTL--------SETATIEK
        P.tremula_TA18674_47664  (30)  ESQVGNS-GNRM---------TGLTHHLSLPKTF--------AEMAMAEK
   P.trichocarpa_scaff_I.1785 (123)  ESQVGNS-GNRM---------TGLTHHLSLPKTV--------AEMATAEK
             G.max_TA57335_3847 (231)  SQEADFSFQN-----------LGLTHHLSLPSSS--------TKMSSIEK
             G.max_TA63030_3847  (34)  MDIQDEDFGYQK---------VGLTHHLSLPGSS--------SRMATMEK
            G.hybrid_AJ763309   (1)  --------------------------MDDTSFNS--------LKRSRNGD
  V.vinifera_GSVIVT00008845001 (276)  EAQNVDSRYRSL---------GLTHHLS-LPKNF--------PQITTIEK
              G.soja_CA783858  (81)  DSQNGEPGNQVQR----------LSHHLSLPRTS--------SEMFAMDN
  P.trichocarpa_scaff_XVI.437 (226)  NAQNGELGNRTH---------VFAHHLS-LPKTS--------VEMVAMEK
  V.vinifera_GSVIVT00025522001 (149)  SNTSGIRVSFCI---------VDVFWTSTILHTS--------AEMAAMEK
  V.vinifera_GSVIVT00024649001 (244)  EAQKGEPANRPP---------VLAHHLSLPTKTS--------ADLTTIEK
          E.esula_TA10959_3993 (159)  SSETQNRDSGIPA-------PPRLSHHLSLPKTT--------AEMSAIEK
         S.tuberosum_CV506096  (12)  KVELR---------------PPTLLSHLSLPQTSPE--------LSAMEK
              G.max_BPS_22716 (294)  PESQNETGGQQPS-------SSALAHQLSLPNTS--------AEMAAIEK
      M.truncatula_TA37090_3880  (27)  PAETKNEQGGQTT-------SAPLAHQMSMPNTT-A-------ELAAIEK
  P.trichocarpa_scaff_III.1580 (300)  ETQQNEEAGNRP---------PMLAHHLSLPKTS--------AEMSTIEN
           R.communis_EG683575  (19)  NASENESGEVGNH-------PPMLAHHLSLPKTS-A-------ELSAIEK
           H.ciliaris_EL431737  (47)  --E-KLNDGGRIT-------QATHAPTGLTHQLSLP------TELSVKEK
         T.officinale_DY832981 (161)  SSENQNDEG-RIH------VSTGLTHQMSLPTGSSE--------LSVMEK
             I.nil_TA9289_35883  (38)  APDFKNTEGSRSR------PPGRLSHHLSLPKSSE-------------EL
        H.vulgare_TA45248_4513  (64)  AAKRRPREAANGR-------SGQLKPQFSLPAGSKP-----SPEIAAIEK
              Z.mays_BPS_30292 (271)  SGAKRPRDSGPAL-------QQPLAPQLSLPSGKNKGGRAASAEMAAIEK
       O.sativa_Os08g0506700 (275)  SSEP-SQNGGG---------GGGLAHQFSLPKTS-S-------EMAAIEK
       O.sativa_Os09g0487900 (274)  LPRQLSLPAASK--------NSSKPPSSASAAAS-P-------EMAAIEK
      Z.mays_TA16655_4577999 (250)  PATNKRPRDSLEH-------GGGLAHQFSLPKTSSS-------EVAAIEK
    Z.officinale_TA5709_94328  (51)  EE-EGKVVELRNY-------VAGLTHQLSLPKPA--------AEMAAMEK
            I.nil_TA15694_35883  (23)  FSNPNVSDIQSE--------DQTRPPGLLSHHLS-L-------PKVPAQL
    Z.officinale_TA6615_94328  (83)  DNGCAAISSSSFR-------NKELPPISCPPRQNSQMSQISETQMSLPKN
               B.napus_BPS_9258  (22)  DEP------GKRSK-----TTDFFTLETQFSMPQT------SLEMARMES
       G.hirsutum_TA24161_3635 (206)  SSKR--AKNLDGDI-----YNCFNALETQFSLPQT------TLEMATVEK
  P.trichocarpa_scaff_XIV.978 (214)  PSKQ--ARTGDGDI-----YSCFNALETQFSLPQT------SLEMATVEK
  V.vinifera_GSVIVT00005670001 (226)  PNKR--AKNINGDI-----LNSLSSLEPQFSLPQT------SLEMAAVEK
              G.max_BPS_39182 (157)  TKRSKTNTNDPDIV-----HSLNSALGSQFNRPHT------SLEMSTVDK
       B.oleracea_TA6610_3712 (237)  TRPTK-------RP-----KDMNSGLFSQYSLPS-------DTSMNYMDN
        L.saligna_TA4923_75948 (180)  NSSHNKRAKLDING------LNVMDSELNFGLSES------ALEAATMEK
    N.benthamiana_TA8284_4100 (236)  PSKRAKQISNDIVNN----GLDDGETQFQFGLSQT------ALEMASMDR
```

FIGURE 1 (continued)

```
                                         351                                              400
     N.benthamiana_TA8285_4100   (238)  PSKRAKHISDDIVN-----GLDDGETQFRFGLSQT------ALEMASMDR
        S.tuberosum_TA26686_4113  (191)  PSKRAKQISNDMVN-----GLDDGETQFQFGLSQT------ALEMASMDR
   M.truncatula_AC149471_36.2   (213)  SKKRSSRTDPNDDPD----LLLHCLNALETQYSLP------QTSLEMDQL
        H.vulgare_TA47636_4513   (191)  -------------------DLGGYESQFSGMASSS------SLELAGMDK
       T.aestivum_TA88301_4565   (124)  -------------------DLGGYESQFSGMASSS------SLELAGMDK
   O.sativa_LOC_Os02g39140.1   (188)  --------------------LGGYESQFGGMASTS------ALEMAGMDR
        S.bicolor_TA33134_4558    (90)  --------------------LGAYDPQFSGAMAGT------ALEMAGMDR
              Z.mays_BPS_3797    (45)  --------------------LGAYDPQFSGAMAGT------ALEMAGMDR
       O.sativa_Os04g0489600   (239)  ---TG---KSGAHGN----DDIIATLSNYESQLVA------PREMAGVEK
       S.officinarum_CA143325     (1)  ----------------------MGHHSRNSTLTNY------SLVSKENDG
  S.officinarum_TA45654_4547     (1)  -------------------------------MNTS------FGVAKKMAG
             Z.mays_BPS_52761    (95)  ---AN---KSGIHNN----DDIIDNIS--NSYELQ------FGVAKEMAG
       O.sativa_Os01g0900800   (257)  RVAAAAAGDHGDDMV----SSFSNIDSQFGLSKQS------SLEMAGMDD
    Z.officinale_TA2571_94328   (216)  ------IKCDNGDAV----ASLGNIDSQFSFPR-T------SSEMSELEK
          B.oleracea_EH428573     (1)  ---------------------------MKEEQMSG------GVSGMMDIN
   C.clementina_TA5735_85681   (122)  KRAREDNNTQFPSPT----AKLNFHSHLKVEQSGQV-----PGGVSNLVD
     C.sinensis_TA15236_2711   (122)  KRAREDNNTQFPSPT----AKLNFHSHLKVEQSGQV-----PGGVSNLVD
   G.raimondii_TA12344_29730   (132)  SKRASELDTQFPP--------TKFHSQLKGEPSDQI-----SGGISNLID
P.trichocarpa_scaff_XIX.717   (135)  DKRAREVELQNPS--------ARYPPPPSKGEQTG------PLRASSLIE
           C.intybus_EH689338   (112)  SRDVALDDQQ-----------VKFTTQLSGDQS-------------ALLD
        L.sativa_TA5039_4236   (106)  SRAVDVDDQQ-----------PKFTTQLSGDQS-------------ALLD
          L.serriola_DW114802   (106)  SRAVDVDDQQ-----------PKFTTQLSGDQS-------------ALLD
        C.tinctorius_EL407531   (105)  P------PTD-----------PKFTTQLSGDQS-------------ALLD
        H.petiolaris_DY944559   (130)  S---------------------KFTTQLSGDQS-------------MLLE
 V.vinifera_GSVIVT00001847001    (46)  PAVDGSPTGKRARELESRSSSRKFSSQSKGEQSSR-----LTGSVASLLD
           I.nil_TA9081_35883   (150)  AKRPREADSQSPS--------AKLSSPLKGEQSGRL-----RS-ACGSLD
  S.lycopersicum_TA46743_4081   (127)  AKRTRDDDSESSP--------RKLVSQLKGESSGQL-----HG-SGGSLD
     S.tuberosum_TA37004_4113   (130)  AKRTRDGDSESSP--------RKLASQLKGESSGKL-----HG-SGGSLD
          G.max_TA56453_3847    (30)  EEEEDPLSFTQLLS-----TIDDAPSHSQHQLYGA------ALSNKDKTP
           C.obtusa_BW987363     (5)  VMMQDLHNPDAQKVE----TGSQGTSTLVNPSHNL------SRSISAELA
     S.tuberosum_TA40158_4113    (39)  SHILSDDFLKAEDIEIEPYSNEDASHNQNSEGLARP--PIPLSHHLSLPT
P.patens_121037_e_gw1.34.393.1     (1)  -----------------------------------------MGYDDLLDL
P.patens_148201_e_gw1.273.42.1    (13)  FRTAARVGEAGPK-------LGGLIRHSSLPAP----SRPFSGNVDFDDL
        P.patens_TA27139_3218    (79)  TSSGSVEGSTYRKSQ-----QGGLIRHMSLPHSTN----------GDSSS
        P.patens_TA32785_3218    (39)  SGSELNSKSYRLNNN----QSTGLIRHMSLPTSG-------------DVP
         P.taeda_TA15788_3352    (70)  MMPALNLAESQKGEPSS-HVPMGLNHQFSLTKSPS--------------E
     Z.officinale_TA334_94328    (91)  DTLNPTELEARNH-------APTLPHQFSLPPKPSP-------EMAAVEN
A.thaliana_bHLH080_9_AT1G35460   (137)  -------P-------------TKRSRDMETQFSSQLKEEQMSGGISGMMD
A.thaliana_bHLH081_9_AT4G09180   (130)  PGSKRSREMEALFS------SPEPTSQMKGEQSSGQ----VPTGVSSMSD
A.thaliana_bHLH122_9_AT1G51140   (239)  RSFGNEGSASSKLTALARTQSGGLDQYKTKDEDSASRRPPLAHHMSLPKS
A.thaliana_bHLH128_9_AT1G05805   (222)  AATTDSWDDGSGSIG----FTVTRPSKRSKDMDSGLFSQYSLPSDTSMNY
A.thaliana_bHLH129_9_AT2G43140   (177)  DNSSSHISFTIDQPGKRSKNSDFFTLETQYSMPQT------TLEMATMEN
A.thaliana_bHLH130_9_AT2G42280   (218)  SSLKREAEDDGKLFLGAQNGESGNRMQLLSHHLSLPKSSSTASDMVSVDK
                     Consensus   (351)                              LS   S          EMA MEK
```

FIGURE 1 (continued)

```
                                         401                                              450
           H.paradoxus_EL487892    (46)  TRSLRLE-LHLHPTTSPTPQS--------SATHPPSIAERVRRTRISDRM
      M.truncatula_AC141114_16.2  (232)  LLQFP----DSVPSSIRAKRG--------FATHPRSLAERVRRTRISERM
        V.vinifera_TA49422_29760   (80)  GANL-C---DDFESERFMPCGGFQDSAMSCATHPRSIAERVRRTRISERM
      C.solstitialis_EH761577     (127)  FLRFEQ---DSIPCKTRAKRG--------FATHPRNIAERVRRTRISDRM
            I.nil_TA8920_35883     (65)  YLHFQQ---DAVPCKFRAKRG--------CATHPRSIAERNRRTRISERM
   S.lycopersicum_TA38189_4081    (311)  YLQFQQ---DSVPCKIRAKRG--------CATHPRSIAERMRRTRISERM
      S.tuberosum_TA29368_4113    (222)  YLQFQQ---DSVPCKIRAKRG--------CATHPRSIAERMRRTRISERM
       C.sinensis_TA15331_2711     (98)  FLHFQG----SVPCKIRAKRG--------CATHPRSIAERVRRTRISERM
             P.tremula_DN488299    (67)  FLDFQG---NSVPCKIRAKRG--------FATHPRSIAERVRRTRISERM
  P.trichocarpa_scaff_IX.1004     (325)  FLDFQG---NSVPCKIRAKRG--------FATHPRSIAERVRRTRISERM
      P.tremula_TA18674_47664      (62)  FLDFQG---NFVPFKIRAKRG--------FATHPRSIAERVRRTRISERM
   P.trichocarpa_scaff_I.1785     (155)  FLDFQG---NFVPCKIRAKRG--------FATHPRSIAERVRRTRISERM
           G.max_TA57335_3847     (262)  FLQIQG----SVPCKIRAKRG--------FATHPRSIAERVRRTRISERI
           G.max_TA63030_3847      (67)  LYQIQG----SVPCKIRAKRG--------FATHPRSIAERERRTRISARI
            G.hybrid_AJ763309      (17)  FLQFQQ---DLVPCKIRAKRG--------FATHPRSIAERERRTRISERI
 V.vinifera_GSVIVT00008845001     (308)  FLHFQD----SVPCKIRAKRG--------CATHPRSIAERVRRTRISERM
              G.soja_CA783858     (113)  LLQFSD----SVPCKIRAKRG--------FATHPRSIAERVRRTRISERI
  P.trichocarpa_scaff_XVI.437     (258)  FLHLQD----SVPCKIRAKRG--------CATHPRSIAERVRRTRISERM
 V.vinifera_GSVIVT00025522001     (182)  FLQFQD----SVPCKIRAKRG--------CATHPRSIAERVRRTRISERM
 V.vinifera_GSVIVT00024649001     (277)  YLQFQD----SVPCKIRAKRG--------CATHPRSIAERVRRTRISERM
          E.esula_TA10959_3993    (194)  FLQLQ----DSVPCKIRAKRG--------FATHPRSIAERVRRTRISERM
         S.tuberosum_CV506096      (39)  LL---Q---DSVPCKVRAKRG--------CATHPRSIAERVRRTRISERM
              G.max_BPS_22716     (329)  FLQLSD----SVPCKIRAKRG--------CATHPRSIAERVRRTKISERM
     M.truncatula_TA37090_3880     (62)  FLQFS----DSVPCKIRAKRG--------CATHPRSIAERVRRTKISERM
 P.trichocarpa_scaff_III.1580     (333)  FLQFQD----SVPCKIRAKRG--------CATHPRSIAERVRRTRISERM
          R.communis_EG683575      (54)  YLQLQ----DSVPCKIRAKRG--------CATHPRSIAERVRRTRISERM
          H.ciliaris_EL431737      (81)  LMHFQ----DNVPLRSRAKRG--------CATHPRSIAERVRRTRISERM
        T.officinale_DY832981     (196)  LMQL-Q---DSVPLRSRAKRG--------CATHPRSIAERVRRTRISERM
           I.nil_TA9289_35883      (69)  SSIL-Q---DSVPCRVRAKRG--------CATHPRSIAERVRRTRISDRM
        H.vulgare_TA45248_4513    (102)  FLQFQ----DSVPCKIRAKRG--------CATHPRSIAERVRRTKISERI
              Z.mays_BPS_30292    (314)  FLQFQ----DAVPCKIRAKRG--------CATHPRSIAERVRRTKISERI
      O.sativa_Os08g0506700       (307)  FLQFQ----DAVPCKIRAKRG--------CATHPRSIAERVRRTRISERI
      O.sativa_Os09g0487900       (308)  FLQFQ----DAVPCKIRAKRG--------CATHPRSIAERVRRTRISERI
      Z.mays_TA16655_4577999      (286)  FLQFQ----DAVPCKVRAKRG--------CATHPRSIAERVRRTKISERI
    Z.officinale_TA5709_94328      (85)  LIQFQD----ASPCRIRAKRG--------CATHPRSIAERVRRTKISERM
          I.nil_TA15694_35883      (57)  SAIMQ----DSVPCKVRAKRG--------CATHPRSIAERVRRTKISERM
    Z.officinale_TA6615_94328     (126)  FKQFQ----DAVPCRVRAKRG--------FATHPRSVAERVRRSKISERM
              B.napus_BPS_9258     (55)  LMNIPE---GSVPCRVRAKRG--------CATHPRSIAERERRTRISGKL
       G.hirsutum_TA24161_3635    (243)  LLHIPE---DSVPCKIRAKRG--------CATHPRSIAERERRTRISGKL
  P.trichocarpa_scaff_XIV.978     (251)  LLQIPE---DSVPCKIRAKRG--------CATHPRSIAERERRTRISGKL
 V.vinifera_GSVIVT00005670001     (263)  LLQVPE---DSVPCKVRAKRG--------CATHPRSIAERERRTRISGKL
              G.max_BPS_39182     (196)  LLHIPE---DSVPCKIRAKRG--------CATHPRSIAERERRTRISGKL
       B.oleracea_TA6610_3712     (268)  YMQLPE---DSVPCKVRAKRG--------FATHPRSIAERERRTRISGRL
        L.saligna_TA4923_75948    (218)  MMDLPH---DSVPCKIRAKRG--------CATHPRSIAERERRTRISGKL
   N.benthamiana_TA8284_4100      (276)  FLHIPE---DSVPCKIRAKGG--------CATHPRSIAERERRTRISGKL
```

FIGURE 1 (continued)

```
                                            401                                              450
         N.benthamiana_TA8285_4100  (277)  LLHIPE---DSVPCKIRAKRG--------CATHPRSIAERERRTRISGKL
          S.tuberosum_TA26686_4113  (230)  LLHIPE---DSVPCKIRAKRG--------CATHPRSIAERERRTRISGKL
       M.truncatula_AC149471_36.2  (253)  MHNIPQ---DSVPCKIRAKRG--------CATHPRSIAERERRTRISGKL
            H.vulgare_TA47636_4513  (216)  YMQAQQQ-QDQVGFKVRAKRG--------CATHPRSIAERERRTRISEKL
           T.aestivum_TA88301_4565  (149)  YMQAQQQ-QDQVAFKVRAKRG--------CATHPRSIAERERRTRISYKL
        O.sativa_LOC_Os02g39140.1  (212)  YLQLQH---DQVPFKVRAKRG--------CATHPRSIAERERRTRISEKL
            S.bicolor_TA33134_4558  (114)  YMQLQQ---DQVPFKVRAKRG--------CATHPRSIAERERRTRISEKL
                   Z.mays_BPS_3797   (69)  YVQLQQ---DQVPFKVRAKRG--------CATHPRSIAERERRTRISEKL
          O.sativa_Os04g0489600  (273)  YLQMQH---DQVPFRVRAKRG--------CATHPRSIAERERRTRISEKL
           S.officinarum_CA143325   (23)  LIQVQQ---DQVPFRVQAKRG--------CAPHPRSIPERERRTRISEKL
      S.officinarum_TA45654_4547   (14)  LLQVQQ---DQVPFRVRAKRG--------CATHPRSIAERERRTRISEKL
                  Z.mays_BPS_52761  (127)  LLQIQQ---DRVPFRVRAKRG--------CATHPRSIAERERRTRISEKL
          O.sativa_Os01g0900800  (297)  FLQLQP---DSVACRARAKRG--------CATHPRSIAERERRTRISKRL
       Z.officinale_TA2571_94328  (249)  YLQIQQ---DSVPCRARAKRG--------CATHPRSIAERERRTRISKRL
             B.oleracea_EH428573   (18)  MDKLLE---DSVPCRVRAKRG--------CATHPRSIAERVRRTRISDRI
     C.clementina_TA5735_85681  (163)  MDMEKLL-EDSVPCRVRAKRG--------CATHPRSIAERVRRTRISDRI
         C.sinensis_TA15236_2711  (163)  MDMEKLL-EDSVPCRVRAKRG--------CATHPRSIAERVRRTRISDRI
      G.raimondii_TA12344_29730  (169)  VDMEKLL-EDSVPCRVRAKRG--------CATHPRSIAERVRRTRISDRI
    P.trichocarpa_scaff_XIX.717  (171)  MEMDKLL-EDSVPCRVRAKRG--------CATHPRSIAERVRRTRISDRI
              C.intybus_EH689338  (138)  VEMDKLL-GDSVPCRVRAKRG--------CATHPRSIAERVRRTRISDRI
            L.sativa_TA5039_4236  (132)  VEMDKLL-GDSVPCRVRAKRG--------CATHPRSIAERVRRTRISDRI
             L.serriola_DW114802  (132)  VEMDKLL-GDSVPCRVRAKRV--------CATHPRSIAERVRRTRISDRI
           C.tinctorius_EL407531  (125)  VDMDKLL-EDSVPCRVRAKRG--------CATHPRSIAERVRRTRISDRI
           H.petiolaris_DY944559  (146)  GDMDKML-GDSVPCRVRAKRG--------CATHPRSIAERVRRTRISDRI
    V.vinifera_GSVIVT00001847001   (91)  VDMEKLL-EDSVPCRVRAKRG--------CATHPRSIAERVRRTRISDRI
               I.nil_TA9081_35883  (186)  AEMEKMM-EDLVPCRVRAKRG--------CATHPRSIAERVRRTRISDRI
    S.lycopersicum_TA46743_4081  (163)  AEMENLM-DDLVPCKVRAKRG--------CATHPRSIAERVRRTRISDRI
         S.tuberosum_TA37004_4113  (166)  AEMENLM-DDLVPCKVRAKRG--------CATHPRSIAERVRRTRISDRI
              G.max_TA56453_3847   (69)  EIFMLE---DSVPCRVRAKRG--------CATHPRSIAERVRRTRISDRI
               C.obtusa_BW987363   (45)  LEELMQ---DSVPCKVRAKRG--------FATHPRSIAERVRRTRISERM
         S.tuberosum_TA40158_4113   (87)  SIMEKLL-QDSVHLNVRAKRG--------CATHPRSIAERVRRTRISDRM
P.patens_121037_e_gw1.34.393.1   (10)  YSP----------CKTRARRG--------YATHPRSIAERNRRSRISERM
P.patens_148201_e_gw1.273.42.1   (52)  FADPS----AVPLKTIRANRG--------HATHPRSIAERVRRCKISERM
           P.patens_TA27139_3218  (114)  PGVEDNT-FHTVPMRTRAKRG--------CATHPRSIAERVRRTKISERM
           P.patens_TA32785_3218   (72)  NMSLNTD-DHYVQMRARAKRG--------CATHPRSIAERVRRTRISERM
             P.taeda_TA15788_3352  (105)  IAMEKLL-QDSVPCKIRAKRG--------CATHPRSIAERVRRTRISEKM
        Z.officinale_TA334_94328  (127)  YLHFQ----DSGPFKIRAKRG--------CATHPRSIAERVRRTRISERI
A.thaliana_bHLH080_9_AT1G35460  (167)  MNMDKIF-EDSVPCRVRAKRG--------CATHPRSIAERVRRTRISDRI
A.thaliana_bHLH081_9_AT4G09180  (170)  MNMENLM-EDSVAFRVRAKRG--------CATHPRSIAERVRRTRISDRI
A.thaliana_bHLH122_9_AT1G51140  (289)  LSDIEQLLSDSIPCKIRAKRG--------CATHPRSIAERVRRTKISERM
A.thaliana_bHLH128_9_AT1G05805  (268)  MDNFMQLPEDSVPCKIRAKRG--------CATHPRSIAERERRTRISGKL
A.thaliana_bHLH129_9_AT2G43140  (221)  LMNIPE---DSVPCRARAKRG--------FATHPRSIAERERRTRISGKL
A.thaliana_bHLH130_9_AT2G42280  (268)  YLQLQ----DSVPCKIRAKRG--------CATHPRSIAERVRRTRISERM
                        Consensus  (401)   L  Q    DSVPCKIRAKRG        CATHPRSIAERVRRTRISERM
```

**FIGURE 1 (continued)**

| | | 451 500 |
|---|---|---|
| H.paradoxus_EL487892 | (87) | KKLQELFPNMDKQTS-TADMLDMAVQYIKDLQKELET--LRDARSKCECS |
| M.truncatula_AC141114_16.2 | (270) | RKLQEIVPNIDKQTC-TSEMLDLAVEYIKDLQKQLKT--MSAKRAKCRCR |
| V.vinifera_TA49422_29760 | (126) | RKLQELVPNMDKQTN-TADMLDLAVEYIKDLQKQYNT--LTDNRAHCKCL |
| C.solstitialis_EH761577 | (166) | KKLPELFPTMDKRTS-TADMLDMAVQYITDLQKNMQA--LNDARARCTCS |
| I.nil_TA8920_35883 | (104) | KKLQELFPKMDKQTS-TADMLDWAVDHIKELQKQVQI--LTDKKAKCTCS |
| S.lycopersicum_TA38189_4081 | (350) | KKLQDLFPNMDKQTN-TADMLDLAVDYIKDLQKQVQT--LTDKKAKCSCT |
| S.tuberosum_TA29368_4113 | (261) | KKLQDLFPNMDKQTN-TADMLDLAVDYIKDLQKQVQT--LNDKKAKCSCT |
| C.sinensis_TA15331_2711 | (136) | RKLQDLFPNMDKQTN-TAEMLDLAVEHIKDLQKQVKL--LTDNKAKCMCP |
| P.tremula_DN488299 | (106) | RKLQELFPNMDKQTN-TADMLDLAVEHIKDLQKQAKT--LTDTKAKCTCS |
| P.trichocarpa_scaff_IX.1004 | (364) | RKLQELFPNMDKQTN-TADMLDLAVEHIKDLQKQVKT--LTDTKAKCTCS |
| P.tremula_TA18674_47664 | (101) | RKLQELFPDMDKQTS-TADKLDLSIELIKDLQKQVKT--LTDTKAKCTCS |
| P.trichocarpa_scaff_I.1785 | (194) | RKLQELFPDMDKQTS-TADKLDLSIELIKDLQKQVKS--LADTKAKCTCS |
| G.max_TA57335_3847 | (300) | KKLQDLFPKSEKQTS-TADMLDLAVEYIKDLQQKVKI--LSDCKAKCKCT |
| G.max_TA63030_3847 | (105) | KKLQDLFPKSDKQTS-TADMLDLAVEYIKDLQKQVKI--LRDTRAKCTCT |
| G.hybrid_AJ763309 | (56) | KKLQELFPNMDKQMN-TADMLDMALDYIKDLQKEVQS--LNDARARCMCS |
| V.vinifera_GSVIVT00008845001 | (346) | RKLQELFPNMDKQTN-TADMLDLAVEYIKDLQKQVKT--LNDTKVKCTCS |
| G.soja_CA783858 | (151) | RKLQELVPTMDKQTS-TAEMLDLALDYIKDLQKQFKT--LSDKRAKCKCI |
| P.trichocarpa_scaff_XVI.437 | (296) | RKLQELVPNMDKQTN-TADMLDLAVDYIKDLQKQYKT--LSDNRANCKCL |
| V.vinifera_GSVIVT00025522001 | (220) | RKLQELVPNMDKQTN-TADMLDLAVEYIKDLQKQYNT--LTDNRAHCKCL |
| V.vinifera_GSVIVT00024649001 | (315) | RKLQELVPNMDKQTN-TSDMLDLAVDYIKDLQKQVKT--LSDNRAKCTCS |
| E.esula_TA10959_3993 | (232) | RKLQDLVPNMDKQTN-TSDMLDLAVDYIKDLQRQVKT--LSDSRANCTCN |
| S.tuberosum_CV506096 | (75) | RKLQELVPNMDKQTD-PADMLDFAADYIKELETQVKA--LSETRSKCTCS |
| G.max_BPS_22716 | (367) | RKLQDLVPNMDKQTN-TADMLDLAVEYIKDLQNQVEA--LSDNRAKCTCL |
| M.truncatula_TA37090_3880 | (100) | RKLQDLVPNMDKQTN-TSDMLDLAVEYIKDLQNQVET--LSDNRAKCTCS |
| P.trichocarpa_scaff_III.1580 | (371) | RKLQDLVPNMDKQTN-TSDMLDLAVDYIKDLQRQFKA--LSENRARCTCL |
| R.communis_EG683575 | (92) | RKLQDLVPNMDKQTN-TSDMLDLAVDYIKDLQRQVET--LSENRSKCTCA |
| H.ciliaris_EL431737 | (119) | RKLQDLVPNMDKQTS-TADMLDLAVEYIKELQNQVET--LSDRQRKCTCP |
| T.officinale_DY832981 | (234) | RKLQELVPNMDKQTN-TADMLDLAVDYIKELQKQVEK--LSDHHAKCTCL |
| I.nil_TA9289_35883 | (107) | RKLQELVPNMDKQTN-TADMLDLAVDYIKDLEQKVKR--LADNRAKCRCS |
| H.vulgare_TA45248_4513 | (140) | RKLQELVPNMEKQTN-TSDMLDLAVDYIKELQMQVKV--MNDGRAGCTCS |
| Z.mays_BPS_30292 | (352) | RKLQELVPNMEKQTN-TADMLDLAVDYIKDLQKQVKV--LNDGRASCTCS |
| O.sativa_Os08g0506700 | (345) | RKLQELVPNMDKQTN-TADMLDLAVDYIKDLQKQVKG--LNDSRANCTCS |
| O.sativa_Os09g0487900 | (346) | RKLQELVPNMEKQTN-TADMLDLAVDYIKELQKQVKV--LNDSRSSCTCS |
| Z.mays_TA16655_4577999 | (324) | RKLQELVPDMDKQTN-TSDMLDLAVDYIKDLQKQVKA--LNESRASCTCP |
| Z.officinale_TA5709_94328 | (123) | RKLQELVPNMDKQTN-TADMLDLAVEYIKDLQRQVDV--LREGQASCPCS |
| I.nil_TA15694_35883 | (95) | RKLQELVPNMDKQTN-TADMLDLAVDYIKDLESQVQV--LSENRAKCTCS |
| Z.officinale_TA6615_94328 | (164) | KKLQELVPNMDKQTN-TAAMLDLAVIYIKDLQKQVKV--LSEGQAKCTCL |
| B.napus_BPS_9258 | (94) | KKLQELVPNMDKQTS-YADMLDLAVEHIKGLQHQVES--LEKGMERCTCG |
| G.hirsutum_TA24161_3635 | (282) | KKLQELVPNMDKQTS-YADMLDLAVQHIKGLQNEVQK--LHKELESCTCG |
| P.trichocarpa_scaff_XIV.978 | (290) | KTLQDLVPNMDKQTS-YADMLELAVKHIKGLQNEVEK--LHKELEGCTCG |
| V.vinifera_GSVIVT00005670001 | (302) | KKLQDLVPNMDKQTS-YADMLDLAVQHIKGLQNEVQK--LNKELENCTCG |
| G.max_BPS_39182 | (235) | KKLQDLVPNMDKQTS-YADMLDLAVQHIKGLQTQVQK--LHKEMENCTCG |
| B.oleracea_TA6610_3712 | (307) | KKLQDLVPNMDKQTS-YSDMLDLAVQHIKGLQHQLQT--LKKEQENCTCE |
| L.saligna_TA4923_75948 | (257) | KKLQDLVPNMDKQTS-YSDMLDLAVQHIKSLQTHVQS--PYNELQNCKCG |
| N.benthamiana_TA8284_4100 | (315) | KKLQDLVPNMDKQTS-YADMLDLAVQHIRTLQDQVQH--LNTELENCKCG |

FIGURE 1 (continued)

```
                                         451                                              500
N.benthamiana_TA8285_4100         (316)  KKLQDLVPNMDKQTS-YADMLDLAVQHIRTLQDQVQN--LNTELENCKCG
S.tuberosum_TA26686_4113          (269)  KKLQDLVPNMDKQTS-YADMLDLAVQHIRTLQDQVQN--LNTELENCKCG
M.truncatula_AC149471_36.2        (292)  KKLQDLVPNMDKQTS-YSDMLDLAVQHIKGLQTQVQK--LHEDLENCTCG
H.vulgare_TA47636_4513            (257)  RKLQDLVPNMDKQTS-TSDMLDLAVEHIRGLQSQLQV--MKHEQDKCTCC
T.aestivum_TA88301_4565           (190)  RKLQDLVPNMDKQTS-TSDMLDLAVEHIKGLQSQLQA--MKHEQDKCTCC
O.sativa_LOC_Os02g39140.1         (251)  RKLQELVPNMDKQTS-TADMLDLAVEHIKGLQSQLQA--LKHEQEKCTCC
S.bicolor_TA33134_4558            (153)  RKPQDLVPNMDKQTS-TADMLDLAVEHIKGLQSELQA--LKHEREKCTCC
Z.mays_BPS_3797                   (108)  RKLQDLVPNMDKQTS-TADMWDLAVEHIRGLQSELQA--LKHEQEKCTCC
O.sativa_Os04g0489600             (312)  RKLQALVPNMDKQTS-TSDMLDLAVDHIKGLQSQLQT--LKEDKEKCTCS
S.officinarum_CA143325             (62)  KKLQALVPNMDKQTS-PADMLDLAVDHIRGLQSEMQA--LKEDKEKCTCR
S.officinarum_TA45654_4547         (53)  RKLQALVPNMDKQTS-TADMLDLAVDHIRGLQSELQA--LKEDKEKCPCR
Z.mays_BPS_52761                  (166)  RKLQALVPNMDKQTS-TADMLDLAVDHIRGLQNELQVYALKEDKEKCSCR
O.sativa_Os01g0900800             (336)  KKLQDLVPNMDKQTN-TSDMLDIAVTYIKELQGQVEK--LKHDQANCTCS
Z.officinale_TA2571_94328         (288)  QKLQDLVPNMDKQTS-TSDMLELAIQHIKELQGQVEK--LKQEQTNCTCT
B.oleracea_EH428573                (57)  RRLQELVPNMDKQTN-TADMLEEAVEYVKALQSKIQE--LTEQQRRCICK
C.clementina_TA5735_85681         (204)  RKLQDLVPNMDKQTN-TADMLEEAVEYVKFLQKQIEE--LTEHQRRCKCS
C.sinensis_TA15236_2711           (204)  RKLHDLVPNMDKQTN-TADMLEEAVEYVKFLQKQIEE--LTEHQRRCKCS
G.raimondii_TA12344_29730         (210)  RKLQELVPNMDKQTN-PADMLEEAVEYVKYLQRQIQE--LTEHQKRCKCK
P.trichocarpa_scaff_XIX.717       (212)  RKLQELVPNMDKQTN-TADMLDEALAYVKFLQRQIQE--LTEQQRKCKCI
C.intybus_EH689338                (179)  RKLQELVPNMDKQTN-TADMLEEAVEYVKFLQRQIQE--LREHRDKCRCV
L.sativa_TA5039_4236              (173)  RKLQELVPNMDKQTN-TADMLEEAVEYVKFLQRQIQE--LKEHRDKCTCV
L.serriola_DW114802               (173)  RKLQELVPNMDKQTN-TADMLEEAVEYVKFLQRQIQE--LKEHRDKCTCV
C.tinctorius_EL407531             (166)  RKLQELVPNMDKQSN-TADMLEEAVDYVKFLQHQIQE--LRAHRDKCTCS
H.petiolaris_DY944559             (187)  RKLQELVPNMDKQTN-TADMLDEAVEYVKFLQRQIQE--LREHQDKCTCQ
V.vinifera_GSVIVT00001847001      (132)  RKLQELVPNMDKQTN-TADMLEEAVEYVKFLQQKIQE--LSEHQKKCTCL
I.nil_TA9081_35883                (227)  RKLQELVPNMDKQTN-TADMLEEAVEYVKFLQKQIQE--LTEHQKKCTCS
S.lycopersicum_TA46743_4081       (204)  RKLQELVPNMDKQTN-TADMLEEAVEYVKFLQRQIQE--LTEHQKKCTCS
S.tuberosum_TA37004_4113          (207)  RKLQELVPNMDKQTN-TADMLEEAVEYVKFLQRQIQE--LTEHQKKCTCS
G.max_TA56453_3847                (108)  RKLQELVPNMDKQTN-TADMLDEAVAYVKFLQKQIEE--LSEHQRRCKCV
C.obtusa_BW987363                  (84)  RKLQELVPNSDKQTTNIADMLDEAVEYVKALQKQVQE--FKEKQANCTCI
S.tuberosum_TA40158_4113          (128)  RKLQELVPNMDKQTN-TADMLDFAVDYIKELEKQVKI--LAEMRSKCTCI
P.patens_121037_e_gwl.34.393.1     (42)  KKLQDLVPNMDKQTN-TADMLDEAVEYVKHLQTQVKD--LSETIVRLKNS
P.patens_148201_e_gwl.273.42.1     (90)  KKLQDLVPSMDRQTN-TADMLDDAVEYVKQLQQQVQE--LSKTVAELQQL
P.patens_TA27139_3218             (155)  KKLQDLVPSMDKQTN-TSDMLDETVEYVKSLQRQVQELSDTVVRLEAAAA
P.patens_TA32785_3218             (113)  KKLQDLVPNMEKTTN-TADMLDETVEYVKSLQVKVSELQETIAKLKAASA
P.taeda_TA15788_3352              (146)  RNLQQLVPNMDKQTN-TADMLDEAVDYIKFLQKQVQELSENEPKCNCSCK
Z.officinale_TA334_94328          (165)  KKLQELVPNMDTQTN-TADMLGLAISYIKDLQEQVQT--LSESRSSCTCS
A.thaliana_bHLH080_9_AT1G35460    (208)  RRLQELVPNMDKQTN-TADMLEEAVEYVKALQSQIQE--LTEQQKRCKCK
A.thaliana_bHLH081_9_AT4G09180    (211)  RKLQELVPNMDKQTN-TADMLEEAVEYVKVLQRQIQE--LTEEQKRCTCI
A.thaliana_bHLH122_9_AT1G51140    (331)  RKLQDLVPNMDTQTN-TADMLDLAVQYIKDLQEQVKA--LEESRARCRCS
A.thaliana_bHLH128_9_AT1G05805    (310)  KKLQDLVPNMDKQTS-YSDMLDLAVQHIKGLQHQLQN--LKKDQENCTCG
A.thaliana_bHLH129_9_AT2G43140    (260)  KKLQELVPNMDKQTS-YADMLDLAVEHIKGLQHQVEVRP-----------
A.thaliana_bHLH130_9_AT2G42280    (306)  RKLQELVPNMDKQTN-TSDMLDLAVDYIKDLQRQYKI--LNDNRANCKCM
Consensus                         (451)  RKLQELVPNMDKQTN TADMLDLAVEYIK LQKQVQ   L E R KCTC
```

FIGURE 1 (continued)

```
                                          501           515
             H.paradoxus_EL487892  (134)  RKQ------------
        M.truncatula_AC141114_16.2  (317)  NKK------------
         V.vinifera_TA49422_29760  (173)  GKQKPVPNQTV----
       C.solstitialis_EH761577  (213)  S--------------
               I.nil_TA8920_35883  (151)  SEAQKGGML------
     S.lycopersicum_TA38189_4081  (397)  SKQLQYSNGTT----
       S.tuberosum_TA29368_4113  (308)  SKQLQYSNGTT----
         C.sinensis_TA15331_2711  (183)  NKQKLC---------
             P.tremula_DN488299  (153)  SKQKQYSSPSA----
    P.trichocarpa_scaff_IX.1004  (411)  SKQKHYSSPSA----
        P.tremula_TA18674_47664  (148)  SKQK-----------
     P.trichocarpa_scaff_I.1785  (241)  SKQK-----------
             G.max_TA57335_3847  (347)  SNEKHYTRTCA----
             G.max_TA63030_3847  (152)  SNQKH----------
             G.hybrid_AJ763309  (103)  SKQLQSGSTK-----
 V.vinifera_GSVIVT00008845001  (393)  SI-------------
                G.soja_CA783858  (198)  NMQKSEADRVA----
    P.trichocarpa_scaff_XVI.437  (343)  SKQKPVQNKIV----
 V.vinifera_GSVIVT00025522001  (267)  GKQKPVPNQTV----
 V.vinifera_GSVIVT00024649001  (362)  NKQKP----------
          E.esula_TA10959_3993  (279)  ASQQPL---------
        S.tuberosum_CV506096  (122)  HE-------------
                G.max_BPS_22716  (414)  HKKQQ----------
    M.truncatula_TA37090_3880  (147)  HKQQQ----------
  P.trichocarpa_scaff_III.1580  (418)  KKQQP----------
           R.communis_EG683575  (139)  SKQQQS---------
           H.ciliaris_EL431737  (166)  HKLES----------
         T.officinale_DY832981  (281)  HKGEM----------
              I.nil_TA9289_35883  (154)  NK-------------
        H.vulgare_TA45248_4513  (187)  AGRPQKHFAG-----
                Z.mays_BPS_30292  (399)  AGELQGQFTR-----
         O.sativa_Os08g0506700  (392)  AKHQQYSG-------
         O.sativa_Os09g0487900  (393)  ASKQKHFAG------
         Z.mays_TA16655_4577999  (371)  ASKHQQRFSG-----
     Z.officinale_TA5709_94328  (170)  SSKLQKPYHNS----
             I.nil_TA15694_35883  (142)  SSK------------
     Z.officinale_TA6615_94328  (211)  SSEKSKYLKNGAGEG
                 B.napus_BPS_9258  (141)  ACKKR----------
        G.hirsutum_TA24161_3635  (329)  CKQSS----------
     P.trichocarpa_scaff_XIV.978  (337)  CKQSTP---------
 V.vinifera_GSVIVT00005670001  (349)  CKQAL----------
                G.max_BPS_39182  (282)  CKQSK----------
       B.oleracea_TA6610_3712  (354)  CSERPN---------
         L.saligna_TA4923_75948  (304)  CKPQ-----------
  N.benthamiana_TA8284_4100  (362)  CKKSSQ---------
```

FIGURE 1 (continued)

```
                                            501           515
         N.benthamiana_TA8285_4100   (363)  CKKSSQ---------
          S.tuberosum_TA26686_4113   (316)  CKKSSQ---------
       M.truncatula_AC149471_36.2    (339)  CKQST----------
           H.vulgare_TA47636_4513    (304)  SKP------------
          T.aestivum_TA88301_4565    (237)  NKP------------
        O.sativa_LOC_Os02g39140.1    (298)  SRP------------
           S.bicolor_TA33134_4558    (200)  RKR------------
                 Z.mays_BPS_3797     (155)  RKR------------
          O.sativa_Os04g0489600      (359)  CKQASRNRPAD----
        S.officinarum_CA143325       (109)  SNCPPGR--------
      S.officinarum_TA45654_4547     (100)  SNRPPGR--------
               Z.mays_BPS_52761      (215)  GNRPAGR--------
          O.sativa_Os01g0900800      (383)  GKHDC----------
      Z.officinale_TA2571_94328      (335)  AKQEKA---------
             B.oleracea_EH428573     (104)  PKEEQ----------
     C.clementina_TA5735_85681       (251)  AKDKSEKLPNL----
         C.sinensis_TA15236_2711     (251)  AKD------------
      G.raimondii_TA12344_29730      (257)  AKD------------
   P.trichocarpa_scaff_XIX.717       (259)  AKE------------
             C.intybus_EH689338      (226)  VND------------
           L.sativa_TA5039_4236      (220)  VDD------------
            L.serriola_DW114802      (220)  VGD------------
          C.tinctorius_EL407531      (213)  VND------------
          H.petiolaris_DY944559      (234)  SIST-----------
   V.vinifera_GSVIVT00001847001      (179)  ARE------------
             I.nil_TA9081_35883      (274)  TTEQ-----------
   S.lycopersicum_TA46743_4081       (251)  MKDQ-----------
       S.tuberosum_TA37004_4113      (254)  MKDQ-----------
             G.max_TA56453_3847      (155)  VQE------------
               C.obtusa_BW987363     (132)  HVPDCKFKR------
       S.tuberosum_TA40158_4113      (175)  NK-------------
P.patens_121037_e_gw1.34.393.1        (89)  IRAQTAS--------
P.patens_148201_e_gw1.273.42.1       (137)  QERLGRRD-------
          P.patens_TA27139_3218      (204)  QKVSNTSHSPEIA--
          P.patens_TA32785_3218      (162)  TQITSS---------
           P.taeda_TA15788_3352      (195)  MNASETT--------
       Z.officinale_TA334_94328      (212)  SGKQKQDHHHHHPSA
A.thaliana_bHLH080_9_AT1G35460       (255)  PKEEQ----------
A.thaliana_bHLH081_9_AT4G09180       (258)  PKEEQ----------
A.thaliana_bHLH122_9_AT1G51140       (378)  SA-------------
A.thaliana_bHLH128_9_AT1G05805       (357)  CSEKPS---------
A.thaliana_bHLH129_9_AT2G43140       (298)  ---------------
A.thaliana_bHLH130_9_AT2G42280       (353)  NKEKKSI--------
                     Consensus       (501)   K
```

FIGURE 1 (continued)

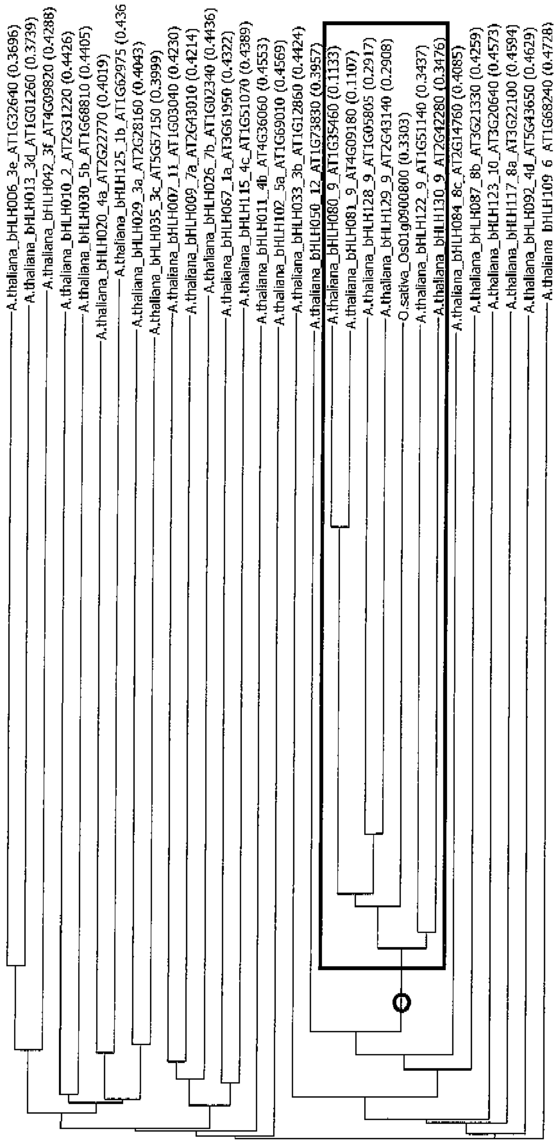

FIGURE 2

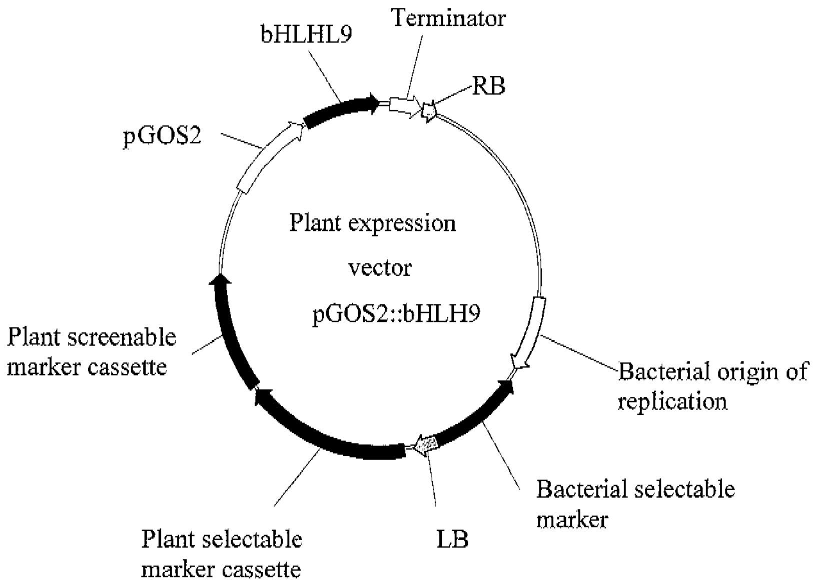
bHLHL9
Terminator
RB
pGOS2
Plant expression
vector
pGOS2::bHLH9
Plant screenable
marker cassette
Bacterial origin of
replication
Bacterial selectable
marker
LB
Plant selectable
marker cassette

FIGURE 3

```
MENLNGLVPDIPIQEQKDNATASENFGRSVDEMVGKVDQIEQRLNEVEHFYSNTSKKQSNTP

RGGSILKDKEKQMSSFRRRQQDASRREAAGSRRMQELMRQFGTILRQITQHKWAEPFMEPVD
                                                                  1
VKGLGLHDYFEVIEKPMDFSTIKNKMEAKDGSGYKHVREICADVRLIFKNAMKYNEERDDVH
          1                    5                   2
VMAKTLLGKFEEKWLQLLPKVDEEEKRRKDEEAEAQQDMQLAQEAAHAKMAKDLTIELDEVD
    3                                                     (7)
MQLEELRDLVLQKCRKISTEDRKQLGNALTKLSPDDLNKALLIVAQNDPTFQPTATEVELDM
  6                                          8
NARSESTLWKLKFFVQDVLHTQGKSPVSVKTNNINTSNLLNNNNNNKRRREFYDALAKSSQK
        4
KSKKPS
```

**FIGURE 4 A**

```
Le_IMB1     1 ----MENLNGLVPDIPIQEQK------DNATASENFGRSVDEMVGKVDQI    40
                  ::....|||:..::..:      :..:..||||..|||:..:|:|:
At_IMB1     1 MSVHVKEEPVLVPNCDVENTELAVFNGNGESELENFGTCVDEITDRVNQL    50

Le_IMB1    41 EQRLNEVEHFYS-NTSKKQSNTPRGGSILKDKEKQMSSFRRRQQDASRRE    89
              ||::.|||||||| .....|:||.:..|   ..|:..:|.....:.:::.:|
At_IMB1    51 EQKVVEVEHFYSTKDGAAQTNTSKSNS--GGKKIAISQPNNSKGNSAGKE    98

Le_IMB1    90 AAGSRRMQ--ELMRQFGTILRQITQHKWAEPFMEPVDVKGLGLHDYFEVI   137
              .:..:.:.  :|||||.|:.|||.|||||.||:||||||||||||||::||
At_IMB1    99 KSKGKHVSSPDLMRQFATMFRQIAQHKWAWPFLEPVDVKGLGLHDYYKVI   148

Le_IMB1   138 EKPMDFSTIKNKMEAKDGSGYKHVREICADVRLIFKNAMKYNEERDDVHV   187
              |||||..|||.|||:   |.|.:||||.|||||:||||||:||||::|||:|
At_IMB1   149 EKPMDLGTIKKKMES---SEYSNVREIYADVRLVFKNAMRYNEEKEDVYV   195

Le_IMB1   188 MAKTLLGKFEEKWLQLLPKVDEEEKRRKDEEAEAQQDMQLAQEAAHAKMA   237
              ||::||.||||||||.::||:.||||::.|||||...:.||..|||.|:||
At_IMB1   196 MAESLLEKFEEKWLLIMPKLVEEEKKQVDEEAEKHANKQLTMEAAQAEMA   245

Le_IMB1   238 KDLTIELDEVDMQLEELRDLVLQKCRKISTEDRKQLGNALTKLSPDDLNK   287
              :||:.||.|:|:||||:|||:.|:|:||||:||||:::|.|..||.:||||:|||:|
At_IMB1   246 RDLSNELYEIDLQLEKLRESVVQRCRKLSTQEKKGLSAALGRLSPEDLSK   295

Le_IMB1   288 ALLIVAQNDPTFQPTATEVELDMNARSESTLWKLKFFVQDVLHTQGKSPV   337
              ||.:|::::|:|...|.|||||::.:::.|||:|||.|||:.|....|||..
At_IMB1   296 ALKMVSESNPSFPAGAPEVELDIDVQTDVTLWRLKVFVQEALKAANKSSG   345

Le_IMB1   338 SVKT-NNINTSNLLNNNNNNKRRREFYDALAKSSQKKSKKPS   378
              ....  ||.||.....|.||.|||||..||:.|:|.|::||.
At_IMB1   346 GTNAQNNNNTGTGEINKNNAKRRREISDAINKASIKRAKKA-   386
```

**FIGURE 4 B**

```
M.truncatula_GTE1302                ------------------------------------------------------------
T.aestivum_TA83944                  ------------------------------------------------------------
O.sativa_GTE2201                    ------------------------------------------------------------
O.sativa_GTE701                     ------------------------------------------------------------
S.bicolor_TA26028                   ------------------------------------------------------------
Z.mays_GTE110                       ------------------------------------------------------------
A.thaliana_AT2G34900_GTE1           ------------------------------------------------------------
M.truncatula_AC174355               ------------------------------------------------------------
A.thaliana_AT3G52280_GTE6           ------------------------------------------------------------
P.patens_GTE1501                    MVNGKCMLKDMVAVVVSLSFPAFSLEGFLLVLLDARPLSERRSDCISTVTKQGFDVVQRT
P.patens_GTE1502                    ------------------------------------------------------------
P.patens_GTE1505                    ------------------------------------------------------------
P.trichocarpa_GTE909                ------------------------------------------------------------
S.lycopersicum_TA45339              ------------------------------------------------------------
G.hirsutum_TA31156                  ------------------------------------------------------------
G.raimondii_TA13411                 ------------------------------------------------------------
C.solstitialis_TA2829               ------------------------------------------------------------
G.raimondii_CO070884                ------------------------------------------------------------
L.saligna_DW067496                  ------------------------------------------------------------
P.trichocarpa_GTE907                ------------------------------------------------------------
P.trichocarpa_GTE908                ------------------------------------------------------------
V.vinifera_GSVIVT00002627001        ---------------------------------------------------------MAF
V.vinifera_GSVIVT00008344001        ------------------------------------------------------------
```

FIGURE 5

```
M.truncatula_GTE1302             ------------------------------------------------------------
T.aestivum_TA83944               -------------------------------------------------------MEGAW
O.sativa_GTE2201                 -------------------------------------------MVPGGGVPQGAEGGRAM
O.sativa_GTE701                  -------------------------------------------MVPGGGVPQGAEGGRAM
S.bicolor_TA26028                -------------------------------------------------------MTPAN
Z.mays_GTE110                    -------------------------------------------------------MTPAN
A.thaliana_AT2G34900_GTE1        -------------------------------------------------MSVHVKEEPVL
M.truncatula_AC174355            -------------------------------------------------------MEPYP
A.thaliana_AT3G52280_GTE6        ------------------------------------------------------MADSVP
P.patens_GTE1501                 RDGHITNLPCSEPISEKKALVTQQMKMKPRIGVDGDMAAEPAQCGPVQEGRGARDRDDRV
P.patens_GTE1502                 --------------------------MKPRISAESDEATQPA-----QEGRGARAKDDRP
P.patens_GTE1505                 ------------------------------------------------------------
P.trichocarpa_GTE909             -------------------------------------------------------MEHMI
S.lycopersicum_TA45339           -------------------------------------------------------MENLN
G.hirsutum_TA31156               -------------------------------------------------------MEAVK
G.raimondii_TA13411              -------------------------------------------------------MEAVQ
C.solstitialis_TA2829            ------------------------------------------------------------
G.raimondii_CO070884             -------------------------------------------------------MEQVM
L.saligna_DW067496               -------------------------------------------------------METMD
P.trichocarpa_GTE907             ----------------------------------------------------------ME
P.trichocarpa_GTE908             -------------------------------------------------------MEAIS
V.vinifera_GSVIVT00002627001     RIALSPSVITERGFLLFIASRKVSKTHKTERLTNRPQCFHKEDRETRPPLECLVSDEPMD
V.vinifera_GSVIVT00008344001     ------------------------------------------------------------
```

FIGURE 5 (continued)

```
M.truncatula_GTE1302                ------------------------------------MSLGKQVNDVEQFYQSTDVQQNDC
T.aestivum_TA83944                  PHPAPM--EPTTAADGPQVSEVNSFRRQVDDLLSKTDVLEKRVNEVVGFY--NSKKHSSG
O.sativa_GTE2201                    AAAETAATAAAAGERAP-GTEVDAFRRQVEDLVSKTDQLERRVNEVVGFY--DGKKHGSG
O.sativa_GTE701                     AAAETAATAAAAGERAP-GTEVDAFRRQVEDLVSKTDQLERRVNEVVGFY--DGKKHGSG
S.bicolor_TA26028                   GAPAPA---AESVPPEV-ESEADAFRRQVDDLVSKTDVLEKRVKEVVDFY--DGKKHGSG
Z.mays_GTE110                       GAP---ARAAEAGPHEV-ESEADAFRRQVDDLVSKTDVLERRVKEVADFY--DGKKHGSG
A.thaliana_AT2G34900_GTE1           VPNCDVENTELAVFNGNGESELENFGTCVDEITDRVNQLEQKVVEVEHFYSTKDGAAQTN
M.truncatula_AC174355               QRPS-----------AN-ADRLEGFRNSVDEFRTQVDNLQKQVIEVEHYYESSGIFQGNS
A.thaliana_AT3G52280_GTE6           GHVAGG------GLQGF-SVDAECIKQRVDEVLQWVDSLEHKLKEVEEFYSSIGVS----
P.patens_GTE1501                    PSPAAVGQNRDSTVEAN-EVETEVSEAARNLLKQQVQTLTAKVEEIERKIALVTQEKNAE
P.patens_GTE1502                    QLPAAVAQTRDSIPGAN-EDETETSEAAKALLKQQVQALTAEVEEMERKIAEVTQKRNAE
P.patens_GTE1505                    ------------------------------------------------------------
P.trichocarpa_GTE909                RSIRGSRNVGTGNIEVD-NSEME---------------FEQRVDAVERYY--ADNKELNT
S.lycopersicum_TA45339              GLVPDI---PIQEQKDN-ATASENFGRSVDEMVGKVDQIEQRLNEVEHFYSNTSKKQSNT
G.hirsutum_TA31156                  VEGF-----------GP-DSGVEDLGRCVDEISTTVSRLEQRVNDVEQFYLTTDNMELTI
G.raimondii_TA13411                 FEGF-----------GP-DSEVEDFGRCVDEISTTVSRLEQRVNDVEQFYLTTDNMELTI
C.solstitialis_TA2829               ------------MEENN-AVIVDDIGQQVDDLFTKVEKLEQRVNEIEQFYLNSSNKQSSS
G.raimondii_CO070884                ASIPDFEIAETGISKDN-SAEAEQFKIRVDEIFQKVDELEQKVNEVEQFNLNSSKKQQSS
L.saligna_DW067496                  GPVPEV------RTEGE-PAVAEQLGQNVDQIIVKVDELEQRLNEVEQFYSKPGKKQSNT
P.trichocarpa_GTE907                PSVFYFGNLPVRNPDGN-DADTEGFKHSVDEIFQKVDKLEQRMNGVEQFYLDISKKQQSG
P.trichocarpa_GTE908                PSIVDSGNLPI---RNS-DAEAEGFKHSVDEILQKVDKLEQRVNEVEQFYSKNTSKKQQS
V.vinifera_GSVIVT00002627001        ASITNVRNVEIGKHKGN-TDQVEGFKSCVDDIFTKVDKLEQRVNEVELFYLTASKRQLNG
V.vinifera_GSVIVT00008344001        ------------------------------------------------------------
```

FIGURE 5 (continued)

```
M.truncatula_GTE1302             KYKGREKPPTGSKKALKRASEDMQAEMRHNFNKIFNEASAVKLLAMTNYILLQPLSFSYI
T.aestivum_TA83944               GRKAGG-----------------------RYAENGARDSHGNGMP--D------------
O.sativa_GTE2201                 G-RKAG----------------------------RKDSSLSKGMP--D------------
O.sativa_GTE701                  G-RKAG----------------------------RKDSSLSKGMP--D------------
S.bicolor_TA26028                G-RKGG--------------------------GGGRHGAYSRGMP--D------------
Z.mays_GTE110                    G-RKGG--------------------------GGGRYGASSRGMP--D------------
A.thaliana_AT2G34900_GTE1        T-SKSN---------SGGKKIAISQPNNSKGNSAGKEKSKGKHVSSPD------------
M.truncatula_AC174355            S-RGGS---VVKEKGREKTLAGTKTPLQ---DALRTETAAGKRMQ--E------------
A.thaliana_AT3G52280_GTE6        --NSGS---IGKDTEKGRHVVGIRKIQQ---EAARREAVAAKRMQ--D------------
P.patens_GTE1501                 S-KSKG-ESGTGLKDRDKGCGTLNKKQQYLLDNNRGDVARSKRNQ--E------------
P.patens_GTE1502                 R-KSKG-KIGTSVKDRDKGAGAFTKKQQQLLDINRRDTSRSKRMQ--E------------
P.patens_GTE1505                 ------------------------------------------------------------
P.trichocarpa_GTE909             A-KCSS---ILKDKIKKKHLMTVEKQQQ---DSE-------KITQ--D------------
S.lycopersicum_TA45339           P-RGGS---IL--KDKEKQMSSFRRRQQ---DASRREAAGSRRMQ--E------------
G.hirsutum_TA31156               T-KSAS---AFKEKVKEKQPTGLEKQQQ---EASQREAAALKRMQ--E------------
G.raimondii_TA13411              T-KSAS---AFKEKVKEKQLTGLEKQQQ---EASQREAAALKRMQ--E------------
C.solstitialis_TA2829            S-QKSL---LVKDKDKVKHIPGYKKHQQ---EAARREATAAKRMQ--E------------
G.raimondii_CO070884             S-KGSS---MGKERDKGRQVPSIKKQQQ---DASRREAAAAKRMQ--E------------
L.saligna_DW067496               S-KASS---VMKEKDKDKQITSFKRRQL---DASRREAAATKRMQ--D------------
P.trichocarpa_GTE907             S-SKGGGSSIVKDKDKERHVTSIRKQQQ---DASKREAAAAKRMQ--E------------
P.trichocarpa_GTE908             G-SSKG--------------GSSTVKDK---DKERHIPTAAKRMQ--E------------
V.vinifera_GSVIVT00002627001     Y-KGSS-----VLKDKERHVASAKKQQQ---DASRREAAAVKRMQ--E------------
V.vinifera_GSVIVT00008344001     --------------------------------------MALWHIS---------------
```

FIGURE 5 (continued)

```
M.truncatula_GTE1302              YPVLLNSSYSGVEFEHITIIAKDKWAWPFLDPVDVEGLGLYDYYQIIEKPMDFSTIKIRM
T.aestivum_TA83944                ----LMRQFAGIIRQ----ITSHEWAQPFLQPVDVVGLQLDDYHQIITKPMDFSTIRNKM
O.sativa_GTE2201                  ----LMRQFGTIVRQ----ITSHEWAEPFLKPVDVVGLQLDDYYKIITKPMDFSTIQKKM
O.sativa_GTE701                   ----LMRQFGTIVRQ----ITSHEWAEPFLKPVDVVGLQLDDYYKIITKPMDFSTIQKKM
S.bicolor_TA26028                 ----LMRQFGVLLKE----ITSHKDAWPFLKPVDVVTLHIPDYHKIITQPMDFSTIQKKM
Z.mays_GTE110                     ----LMRQFGVLLKE----ITSHKDAWPFLEPVDVVTLQLPDYHNIITQPMDFSTIQKKM
A.thaliana_AT2G34900_GTE1         ----LMRQFATMFRQ----IAQHKWAWPFLEPVDVKGLGLHDYYKVIEKPMDLGTIKKKM
M.truncatula_AC174355             ----LMRQFSTILRQATFQITQHKWAWPFLEPVDVEGLGLHDYYEIIDKPMDFGTIKNKM
A.thaliana_AT3G52280_GTE6         ----LMRQFGTIFRQ----ITQHKCAWPFMHPVNVEGLGLHDYFEVIDKPMDFSTIKNQM
P.patens_GTE1501                  ----LMNQIRGIWRQ----ISQHKWAWPFLKPVDVEGLGLHDYNDVIEKPMDLGTIKNKM
P.patens_GTE1502                  ----LMRQVQSIWKQ----ISQHKWAWPFMKPVDVKGLGLHDYYDVIEKPMDLGTIKNKL
P.patens_GTE1505                  --------MTSYIPQ----ISSHKWAWPFMKPVDVKGLGLHDYYEVIEKPMDLGTIKNKM
P.trichocarpa_GTE909              ----LMRQFAVIFRQ----IAQHKWAWPFLEPVDVEGLCLHDYYEVIEKPMDFRTIKNRM
S.lycopersicum_TA45339            ----LMRQFGTILRQ----ITQHKWAEPFMEPVDVKGLGLHDYFEVIEKPMDFSTIKNKM
G.hirsutum_TA31156                ----LMRQFATILRQ----TTQHKWAHPFMHPVDVEGLGLHDYYEIVQKPMDFGTIKSKM
G.raimondii_TA13411               ----LMRQFATILRQ----ITQHKWAHPFMHPVDVEGLGLHDYYEIVQKPMDFGTIKSKM
C.solstitialis_TA2829             ----IMRQFGSILRQ----ITQHKWAWPFMQPVDVEGLGLRDYYEVIDRPMDFSTIKNLM
G.raimondii_CO070884              ----LMRQFGTIVRQ----ITQHKWAWPFMQPVDVKGLGLHDYYEIIEKPMDFSTIKNQM
L.saligna_DW067496                ----LMRQFSVLLRQH---IIGHKWAGPFMQPVDVVGLGLHDYYEVIEKPMDFSTIKAKM
P.trichocarpa_GTE907              ----LMRQFGTILRQ----ITQHKWAWPFMQPVDVKGLRLHDYYEVIDKPMDFSTIKNQM
P.trichocarpa_GTE908              ----LMRQFGTILRQ----ITQHKWAWPFMQPVDVKGLGLHDYYEVIDKPMDFSTIKNQM
V.vinifera_GSVIVT00002627001      ----LMRQFGTILRQ----IMQHKWAGPFLHPVDVEGLGLHDYYEVIDKPMDFSTIKNQM
V.vinifera_GSVIVT00008344001      ----LM-SLRFYWQ-----IMQHKWAGPFLHPVDVEGLGLHDYYEVIDKPMDFSTIKNQM
                                                       : * **: **:*  * : ** .:: .***: **.  :
```

FIGURE 5 (continued)

```
M.truncatula_GTE1302              EAKDGSGYKNVREIYADVRLIFKNAMKYNDEKNDVHVMAKTLLEKFEGCTYRNNSGFSHP
T.aestivum_TA83944                EGKESTTYNSVREIYSDVRLVFTNAMKYNVQGHPVHIMAKFLLERFEEK----WLHLL-P
O.sativa_GTE2201                  EGKDDNKYNNVREIYSDVRLIFANAMKYNDERHDVHIMAKSLLEKFEEK----WLQLL-P
O.sativa_GTE701                   EGKDDNKYNNVREIYSDVRLIFANAMKYNDERHDVHIMAKSLLEKFEEK----WLQLL-P
S.bicolor_TA26028                 ERKDGTCYTNVREICSDVRLIFANAMKYNDDQNVVHLMAKSLLEKFEEK----WLHFL-P
Z.mays_GTE110                     ERKDGTCYTNVREICSDVRLIFANAMKYNDDQNVIHLMAKSLLEKFEEK----WLHFL-P
A.thaliana_AT2G34900_GTE1         ESSE---YSNVREIYADVRLVFKNAMRYNEEKEDVYVMAESLLEKFEEK----WLLIM-P
M.truncatula_AC174355             EAKDGTGYKNVREIYADVRLIFKNAMKYNNEKHDVHVMAKTLMEKFEDK----WLLLL-P
A.thaliana_AT3G52280_GTE6         EAKDGTGYKHVMQIYADMRLVFENAMNYNEETSDVYSMAKKLLEKFEEK----WAHFL-P
P.patens_GTE1501                  DAKDTSGYQHVQEVCDDMRLVFSNAMTYNPEGSDVHVMSKTLSDKFEEK----WKALIEP
P.patens_GTE1502                  DVKDGLGYQHVQEVCDDVRLVFSNAMTYNPEGSDVYVMSKTLSDKFEEK----WKTLIEP
P.patens_GTE1505                  DAKDASGYQHVQEVYQDVRLVFSNAMKYNPEGSDVYVMSKTLSEKFEEK----WKTLVEP
P.trichocarpa_GTE909              EAKDGTGYKNVREIYADVRLVFKNAMKYNDERDDVHVMARTLLEKFEEK----WLQLL-P
S.lycopersicum_TA45339            EAKDGSGYKHVREICADVRLIFKNAMKYNEERDDVHVMAKTLLGKFEEK----WLQLL-P
G.hirsutum_TA31156                EAKDGTGYKNVREIYSDVRLVFKNAMKYNDERHDVHIMAKTLLEKFEEK----WLQLL-P
G.raimondii_TA13411               EAKDGTGYKNVREIYSDVRLVFKNAMKYNDERHDVHIMAKTLLEKFEEK----WLQLL-P
C.solstitialis_TA2829             EAKDGTEYKHVRDICSDVRLVFQNAMKYNDDKSDVHVMAKTLLEKFEEK----WLQFL-P
G.raimondii_CO070884              EAKDGTGYKNVRDICADVRLVFNNAMKYNDEGSDVHLMAKTLLEKFEEK----WQLLL-P
L.saligna_DW067496                EVKDGTGYNNVREICADVRLIFKNAMKYNDERNDVHVMAKTLLAKFEEK----WLQLL-P
P.trichocarpa_GTE907              EAKDGTGYKNVREISADVRLVFKNAMKYNDERSDVHVMAKTLLGKFEEK----WLQLL-P
P.trichocarpa_GTE908              EAKDGTGYKSVREICADVRLVFKNAMKYNDERSDVHVMAKTLLGKFEEK----WLQFL-P
V.vinifera_GSVIVT00002627001      EAKDGTGYKNVREICADVRLVFKNAMKYNDERHDVHVMAKTLLGKFEEK----WLQLL-P
V.vinifera_GSVIVT00008344001      EAKDGTGYKNVREICADVRLVFKNAMKYNDERHDVHVMAKTLLGKFEEK----WLQLL-P
                                  : .:    *  * ::  *:**:* *** ** :   :: *:  *  .**      .  :  *
```

FIGURE 5 (continued)

```
M.truncatula_GTE1302             MQYFSESDLSKEEAHEELN---KRLAQEATYANMTRELSTELSKVDMALRSLKTTAISQC
T.aestivum_TA83944               KVENEERER--EEPNDAPT---ISISPEAAIAILAEDTGNELNEINKQLEELQKMVVQRC
O.sativa_GTE2201                 KVENEERKQKDEESNGVPK---VNISPEEAIAKLAKDTDNELIEINKQLEELRQMVVQKC
O.sativa_GTE701                  KVENEERKQKDEESNGVPK---VNISPEEAIAKLAKDTDNELIEINKQLEELRQMVVQKC
S.bicolor_TA26028                KVESEEKRQKEEESKGVAA---TNTSREVAIAKLAKDTDDELNQINRKLEELRKMVVHRC
Z.mays_GTE110                    KVESEEKRQKEEESKGVAA---TNTSREVAIVKLAKDTDDELNQINKKLEELRKMVVHRC
A.thaliana_AT2G34900_GTE1        KLVEEEKKQVDEEAEKHAN---KQLTMEAAQAEMARDLSNELYEIDLQLEKLRESVVQRC
M.truncatula_AC174355            KVAEEEKRQIEEEAQVQMD---IHLAQETTYADMAKDLSNELNEVGIRLMEFREKVIQNC
A.thaliana_AT3G52280_GTE6        KVQEEEKIREEEEKQAAKE---ALLAKEASHIKTTRELGNEICHANDELEKLMRKVVERC
P.patens_GTE1501                 KLHFEESKTQQEDNEVQLKEAGMQVVEEIDTKKLTEQYLLQLEELDKQLEDLKRQAAPTC
P.patens_GTE1502                 KL-QEELKRSHDDSEVQANEGGVPVVEEIDTEKVIEQYALQLEELDKQLEELKQQATPKF
P.patens_GTE1505                 KLHEEESKKSQEDNEVRTKEPGVQAVEEIDTEELTEQYALQLEELDKQLEDLKQQATPNS
P.trichocarpa_GTE909             KVAEEEKRREKEQTATQVA---TKLAEESSYANMAQDLSNELHGVDMQLERIREMVVRNS
S.lycopersicum_TA45339           KVDEEEKRRKDEEAEAQQD---MQLAQEAAHAKMAKDLTIELDEVDMQLEELRDLVLQKC
G.hirsutum_TA31156               KVAEEEKRQAEEEAKAELD---VKLAQEAVHANMAKELSNELCDVDLQLEKLRQIVIQKC
G.raimondii_TA13411              KVAEEEKRQVEEEAKAELD---VKLAQEAVHANMAKELSNELFDVDLQLEKLRQIVIQKC
C.solstitialis_TA2829            RVTEEEKRREEEEAEAQLS---IQRAQEAAHAKMARDLSNELYEADMHLEHLRETVVQKC
G.raimondii_CO070884             KVTERGEKTR--------------------------------------------------
L.saligna_DW067496               KVDEEDERRKKEKQNHS-------------------------------------------
P.trichocarpa_GTE907             KVTEEEKRREDEEVEAKLD---MQLAQEAAHAKMARDLSNELYEVDMHLEELRDIVVQKC
P.trichocarpa_GTE908             KVTEEEKRREEEEAEAQLD---MQLAQEAAHAKMARDLGNELYEVDMHLEELREMVVQKC
V.vinifera_GSVIVT00002627001     KVAEEEKRREEEEAEAQLD---MQLAQEAAHAKMAREISNELYDIDMHLEEVREMVIRKC
V.vinifera_GSVIVT00008344001     KVAEEEKRREEEEAEAQLD---MQLAQEAAHAKMAREISNELYDIDMHLEEVREMVIRKC
```

FIGURE 5 (continued)

```
M.truncatula_GTE1302             RSVKSHSMNDSLVLHIICHAVIDLNCLAKIRKLSHPEKLILANAFTKLSPDNIVKALEIV
T.aestivum_TA83944               ------------------------------RKMTTDEKRKLGAGLCQLSPEDLNKALELV
O.sativa_GTE2201                 ------------------------------RKMTTYEKRKLGAGLCHLSPEELTKALEMV
O.sativa_GTE701                  ------------------------------RKMTTYEKRKLGAGLCHLSPEELTKALEMV
S.bicolor_TA26028                ------------------------------RKMTTDEKRKLGAGICHLSPDDLNKALEIV
Z.mays_GTE110                    ------------------------------RKMTTDEKRKLGAGICHLSPDDLSKALEIV
A.thaliana_AT2G34900_GTE1        ------------------------------RKLSTQEKKGLSAALGRLSPEDLSKALKMV
M.truncatula_AC174355            ------------------------------RKLSTGEKKALGKAIAKLSPENLQRALDLV
A.thaliana_AT3G52280_GTE6        ------------------------------RKITIEEKRNIGLALLKLSPDDLQKVLGIV
P.patens_GTE1501                 S-----------------------------RAMSIEEKRHLGQNLGKLPPENLSHVIQII
P.patens_GTE1502                 S-----------------------------RAMSVEEKRHLGQSLGRLPPDNLSHVIQII
P.patens_GTE1505                 ------------------------------RAMSVEERRHLGQSLGRLPPDNLSHVIQII
P.trichocarpa_GTE909             ------------------------------RKISTEEKKKLGTALTQLSHQDLIRALEIV
S.lycopersicum_TA45339           ------------------------------RKISTEDRKQLGNALTKLSPDDLNKALLIV
G.hirsutum_TA31156               ------------------------------RKMSTEEKKKLGTALTRLSPEDLGKALEIV
G.raimondii_TA13411              ------------------------------RKMSTEEKKKLGTALTRLSPEDLGKALEIV
C.solstitialis_TA2829            ------------------------------RKMSIEDKRNLGIALTQLSPEDLSKALDIV
G.raimondii_CO070884             ------------------------------------------------------------
L.saligna_DW067496               ------------------------------------------------------------
P.trichocarpa_GTE907             ------------------------------RKMSTEEKRKLGVALTRLSPEDLTKALEIV
P.trichocarpa_GTE908             ------------------------------RKMSTEEKRKLGAALTRLSPEDLTKALEIV
V.vinifera_GSVIVT00002627001     ------------------------------RKMSTEEKRKLGAALSRLSAEDLSKALEIV
V.vinifera_GSVIVT00008344001     ------------------------------RKMSTEEKRKLGAALSRLSAEDLSKALEIV
```

FIGURE 5 (continued)

```
M.truncatula_GTE1302            KESNPNFKDRIDMVTLDLDSQSDYTLFRLHMFVKNTLE----------------------
T.aestivum_TA83944              AQDNPSFQTTAEEVDLDMDAQSETTLWRLKFFVREALEQQANVGVVACG------KIDEN
O.sativa_GTE2201                AQDNPSFEAKGDELELDMDAQSETTLWRLKFFVREALERQANVASGRTD------EN---
O.sativa_GTE701                 AQDNPSFEAKGDELELDMDAQSETTLWRLKFFVREALERQANVASGRTD------EN---
S.bicolor_TA26028               AQDNPSFQTKAEEVDLDMDAQSETTLWRLKFFVREALERQANVASGKMD------EN---
Z.mays_GTE110                   AQDNPSFQTKAEEVDLDMDAQSETTLWRLKFFVREALERQGHVASGKMD------YNAQG
A.thaliana_AT2G34900_GTE1       SESNPSFPAGAPEVELDIDVQTDVTLWRLKVFVQEALKAANKSSGGTNA------QNNNN
M.truncatula_AC174355           AEINPSFESTADEVVLDINAQSDYTVWRLYHFVKGALEGQQGTAVNNDT------EEKRY
A.thaliana_AT3G52280_GTE6       AQANPSFQPRAEEVSIEMDILDEPTLWRLKFFVKDALDNAMKKKKEEET------KTREL
P.patens_GTE1501                AQRNPSFNINSDEVEVDIDAQDPATLWRLQRYVQAVLSGSGARQTTARN-----------
P.patens_GTE1502                AQKNPSFNINSDEVEVDIDAQDPATLWRLQRYVQAVLSGSAARQATPRS-----------
P.patens_GTE1505                AQKNPSFNMNSDEVEVDIDAQDPATLWRLQRYVQAVLSGSGGRQATPRN-----------
P.trichocarpa_GTE909            AEHNPSFQATAQEVNLDMDTQSDVTLWRLKVFVQDALKVSGRNSGGTGMGCNSNINNDDN
S.lycopersicum_TA45339          AQNDPTFQPTATEVELDMNARSESTLWKLKFFVQDVLHTQGKSPVSVKT------NNINT
G.hirsutum_TA31156              AENNPGFQPTAQEVDLDIDAQSELTLWRLKVFVQDKLKLAGKCSEAVVC------NNINN
G.raimondii_TA13411             AENNPGFQPTAQEVDLDIDAQSELTLWRLKVFVQDKLKLAGKCFEAVVC------TNINN
C.solstitialis_TA2829           AQHNPSFHSTAIEVDLDIDAQSESTLWRLKYFVKDALQGYGKDDAIIGN------EANNG
G.raimondii_CO070884            ------------------------------------------------------------
L.saligna_DW067496              ------------------------------------------------------------
P.trichocarpa_GTE907            ARSNPGFQATAEEVDLDIDAQTESTLWRLKFLVKDVLEVQGKSAASTGG------NNNNN
P.trichocarpa_GTE908            AQNNPGFQATAEEVDLDIDAQSETTLWRLKFFVKDALEVQGKSAASAGG------RNNTT
V.vinifera_GSVIVT00002627001    AQNNPSFQATAEEVDLDIDAQTESTLWRLKFFVKDALEVQGKSSASKGD------NTNTT
V.vinifera_GSVIVT00008344001    AQNNPSFQATAEEVDLDIDAQTESTLWRLKFFVKDALEVQGKSSASKGD------NTNTT
```

FIGURE 5 (continued)

```
M.truncatula_GTE1302              ------------------------------------------------------------
T.aestivum_TA83944                ---------------TKRSRDMYNALAKTVSKRIKK------------------------
O.sativa_GTE2201                  ---------------AKRKREICNALARTASKRVKQQPN---------------------
O.sativa_GTE701                   ---------------AKRKREICNALARTASKRVKQQPN---------------------
S.bicolor_TA26028                 ---------------AKRKREICNALAKTASKRIKKQP----------------------
Z.mays_GTE110                     KEGDMQCSGQ-----NHLETDQEAALAIFPYHHSRPLIV---------------------
A.thaliana_AT2G34900_GTE1         TGTGEINKNN-----AKRRREISDAINKASIKRAKKA-----------------------
M.truncatula_AC174355             S--------------SRKKREFSDDHAKNPSKSRKLSTS---------------------
A.thaliana_AT3G52280_GTE6         SGAQKKE--------VSKKRNATTKLAERKTKRSRI------------------------
P.patens_GTE1501                  ---------------QPTKRSCGYVQNKNSSKRGKKTTTPCHGQLSHVMKYSSIPGFLSH
P.patens_GTE1502                  ---------------QATKRNGSSLLKKNSSKRSKATTS---------------------
P.patens_GTE1505                  ---------------QAAKRNGSSLHSKSSSKRSKKVTVP--------------------
P.trichocarpa_GTE909              KAKININNKNRNTTASKRKGERCDAVTKASAKRTKKISLNSLNP----------------
S.lycopersicum_TA45339            SNLLNNNNN------NKRRREFYDALAKSSQKKSKKPS----------------------
G.hirsutum_TA31156                NENTIKSN-------SKRRREISDALTKNAIKRNRKLSPNS-------------------
G.raimondii_TA13411               NENPIKSN-------SKRRREIFDALTKNAIKRNRKLFPNS-------------------
C.solstitialis_TA2829             HVSPTKPTNTTRTIASKRKKQICNALARTAKKRNQKLSS---------------------
G.raimondii_CO070884              ------------------------------------------------------------
L.saligna_DW067496                ------------------------------------------------------------
P.trichocarpa_GTE907              NNNKNTSNN------NKRKREICDAIAKTAKKRSKKPSS---------------------
P.trichocarpa_GTE908              TPSNNNNNN------NKRKREICDAIAKTAKKRSKKPSS---------------------
V.vinifera_GSVIVT00002627001      TTATNN---------NKRKKEICDAIAKTAKKKSKKLPLD--------------------
V.vinifera_GSVIVT00008344001      TTATNN---------NKRKKEICDAIAKTAKKKSKKLPLD--------------------
```

FIGURE 5 (continued)

```
M.truncatula_GTE1302              -
T.aestivum_TA83944                -
O.sativa_GTE2201                  -
O.sativa_GTE701                   -
S.bicolor_TA26028                 -
Z.mays_GTE110                     -
A.thaliana_AT2G34900_GTE1         -
M.truncatula_AC174355             -
A.thaliana_AT3G52280_GTE6         -
P.patens_GTE1501                  Q
P.patens_GTE1502                  -
P.patens_GTE1505                  -
P.trichocarpa_GTE909              -
S.lycopersicum_TA45339            -
G.hirsutum_TA31156                -
G.raimondii_TA13411               -
C.solstitialis_TA2829             -
G.raimondii_CO070884              -
L.saligna_DW067496                -
P.trichocarpa_GTE907              -
P.trichocarpa_GTE908              -
V.vinifera_GSVIVT00002627001      -
V.vinifera_GSVIVT00008344001      -
```

FIGURE 5 (continued)

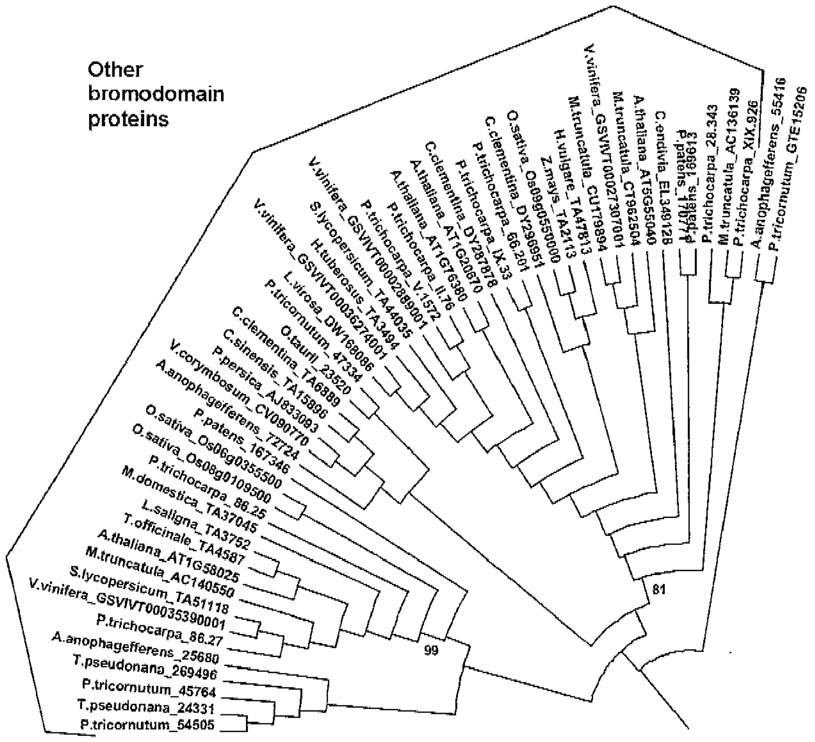
Other bromodomain proteins
V.vinifera_GSVIVT00027307001
M.truncatula_CU179894
H.vulgare_TA47813
Z.mays_TA2113
O.sativa_Os09g0550000
C.clementina_DY296951
P.trichocarpa_66.201
P.trichocarpa_IX.33
C.clementina_DY287878
A.thaliana_AT1G20670
A.thaliana_AT1G76380
P.trichocarpa_II.76
P.trichocarpa_V.1572
V.vinifera_GSVIVT00002889001
S.lycopersicum_TA44035
H.tuberosus_TA3494
V.vinifera_GSVIVT00036274001
L.virosa_DW168086
P.tricornutum_47334
O.tauri_23520
C.clementina_TA6889
C.sinensis_TA15896
P.persica_AJ833093
V.corymbosum_CV090770
A.anophagefferens_72724
P.patens_167346
O.sativa_Os06g0355500
O.sativa_Os08g0109500
P.trichocarpa_86.25
M.domestica_TA37045
L.saligna_TA3752
T.officinale_TA4587
A.thaliana_AT1G58025
M.truncatula_AC140550
S.lycopersicum_TA51118
V.vinifera_GSVIVT00035390001
P.trichocarpa_86.27
A.anophagefferens_25680
T.pseudonana_269496
P.tricornutum_45764
T.pseudonana_24331
P.tricornutum_54505
M.truncatula_CT962504
A.thaliana_AT5G55040
C.endivia_EL349128
P.patens_169613
P.patens_170774
P.trichocarpa_28.343
M.truncatula_AC136139
P.trichocarpa_XIX.926
A.anophagefferens_55416
P.tricornutum_GTE15206
81
99

FIGURE 6

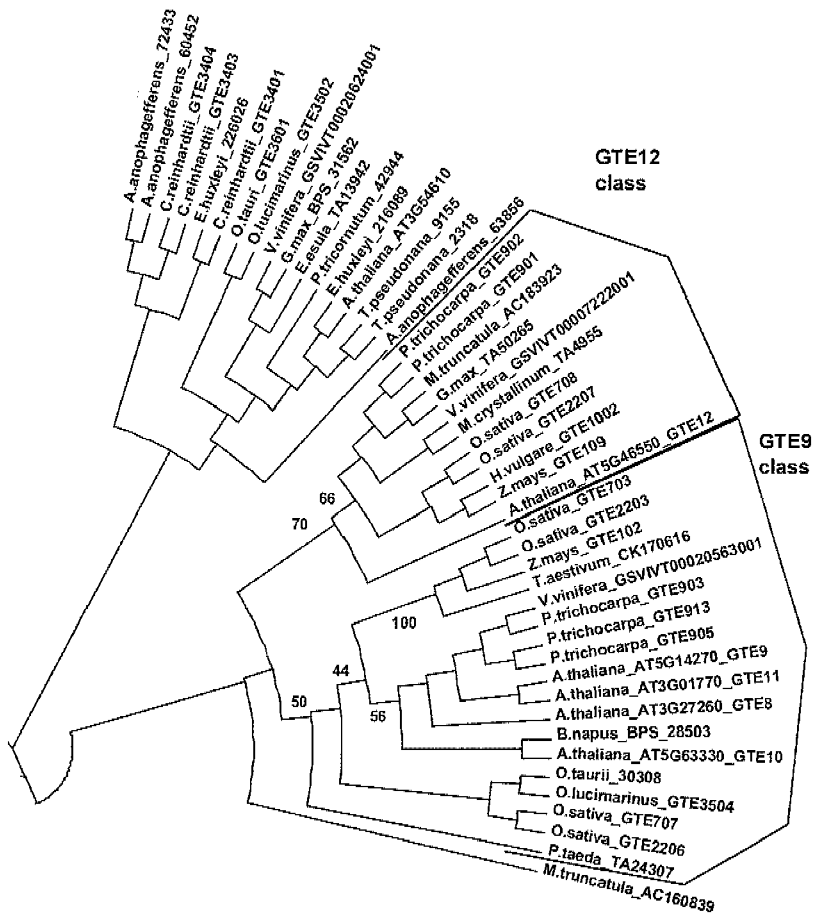


FIGURE 6 (continued)

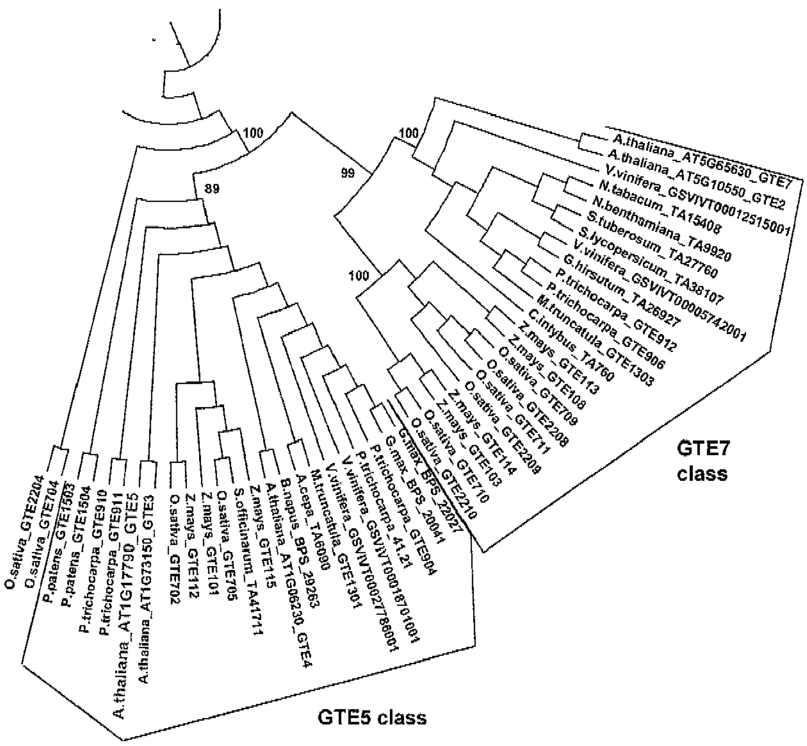


FIGURE 6 (continued)

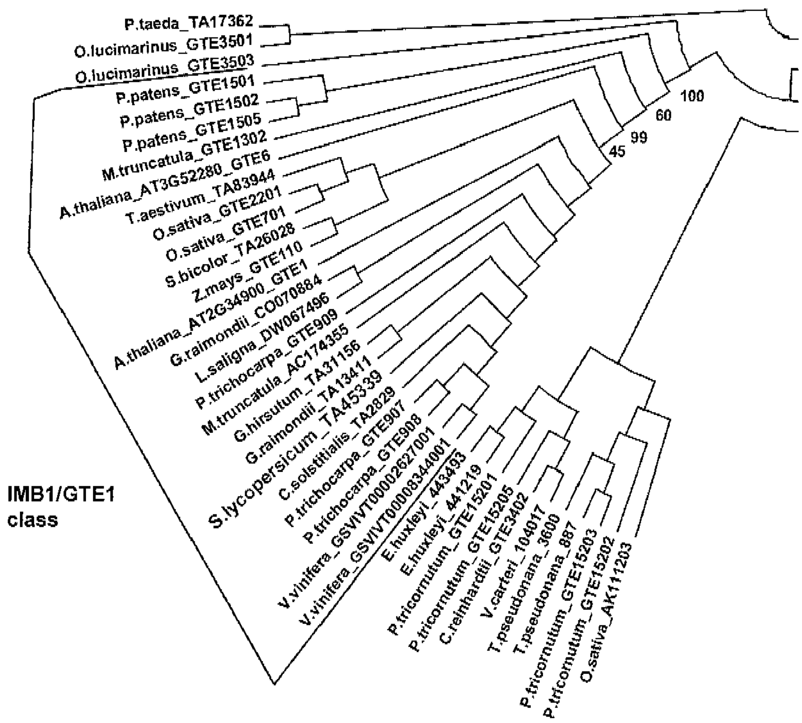


FIGURE 6 (continued)

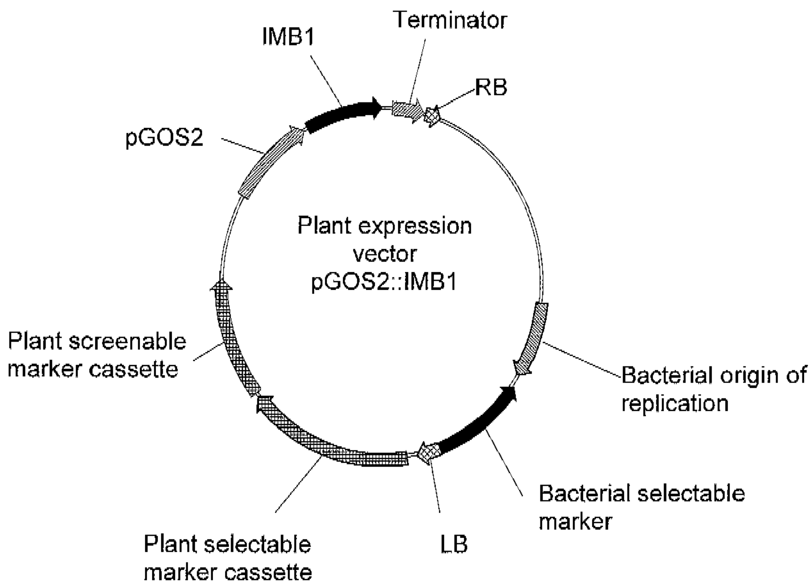
IMB1
Terminator
RB
pGOS2
Plant expression
vector
pGOS2::IMB1
Plant screenable
marker cassette
Bacterial origin of
replication
Bacterial selectable
marker
Plant selectable
marker cassette
LB

FIGURE 7

```
                                          1                                                50
             A.cepa_CF450468#1   (1)  --------------------------------------------------
      A.thaliana_AT1G29810.1#1   (1)  ------------------------------MSRLLLPKLFSISRTQVPAA
                 AT1G29810.1#1   (1)  ------------------------------MSRLLLPKLFSISRTQVPAA
   P.trichocarpa_scaff_XI.475#1  (1)  --------------------------------------------------
    S.lycopersicum_BP905715#1    (1)  --------------------------------------------------
          H.vulgare_BQ462597#1   (1)  ------------------------------------------MAGLLLRR
     H.vulgare_TA39366_4513#1    (1)  ------------------------------------------MAGLLLRR
         T.aestivum_CA729021#1   (1)  ------------------------------------------MAGLLLRR
         T.aestivum_CN012063#1   (1)  --------------------------------------------------
    T.aestivum_TA92660_4565#1    (1)  ------------------------------------------MAGLLLRT
         T.aestivum_CV761453#1   (1)  --------------------------------------------------
      O.sativa_Os01g0663500#1    (1)  --------------------------MTRGVAMAHARLLLARYYAMAAPS
      S.officinarum_CA275526#1   (1)  --------------------------------------------------
      S.officinarum_CA275597#1   (1)  --------------------------------------------------
      A.thaliana_AT5G51110.1#1   (1)  -----MAATSSSPPCNISASSLLLRQPSRSILKVFGLLPPVSRNNRKLGR
                 AT5G51110.1#1   (1)  -----MAATSSSPPCNISASSLLLRQPSRSILKVFGLLPPVSRNNRKLGR
S.lycopersicum_TA38587_4081#1    (1)  ----MAAATHLSFSPPISVSSLPLRKPN----FVTPNLQTSQ-SR--IC-
  P.trichocarpa_scaff_232.30#1   (1)  MGTTATTTTRFPFSLPPPKSYPPHNHHHS---KVSILTPTIT-PT--LR-
        O.sativa_Os03g0100200    (1)  --------------MALTNHILAPAAAAACCFGRRVPLPPPHQLAVR--R
         T.aestivum_CA631555#1   (1)  --------MALMLSPAATFLPVAG----KPAAAMRNLLFGTS----SKGR
         T.aestivum_CV772309#1   (1)  --------MALMLSPAATFLPVVAG---KPTTAMRNLLFGTTTSTTSGHR
         T.aestivum_CA636226#1   (1)  --------MALMLSPAATFLPVVAGKPTT---AMRNLLFGTTTSTTSGHR
         T.aestivum_CA721065#1   (1)  --------MALMLSPAATFLPVATGKPTATAGAMRSLLFTTTSGTASGHR
         T.aestivum_CK206167#1   (1)  --------MALMLSPAATFLPVATGKPTATAGAMRSLLFTTTSGTASGHR
         T.aestivum_CK211978#1   (1)  --------MALMLSPAATFLPVATGKPTATAGAMRSLLFTTTSGTASGHR
    T.aestivum_TA70798_4565#1    (1)  --------MALMLSPAATFLPVATGKPTATAGAMRNLLFTTTSGTASGHR
         T.aestivum_CK212754#1   (1)  --------MALMISPPATFLPVVAGKPTT---AMRNLLFGTTTSTTSGHR
    T.aestivum_TA70795_4565#1    (1)  --------MALMLSPAATFLPVVAGKPTT---AMRNLLFGTTTSTTSGHR
         T.aestivum_CV768439#1   (1)  --------MALMLSPAATFLPVVAGKPTT---AMRNLLFGTTTSTTSGHR
         T.aestivum_CA600360#1   (1)  ---------------------------------MRNLLFGTTTSTTSGHR
         T.aestivum_CK212253#1   (1)  ------------WSGRPGPSSRSPASPRP---PMRNFFFGTS----SKGR
    T.aestivum_TA70797_4565#1    (1)  --------MALMLSPAATFLPVAG-KPAA---AMRNLLFGTSSK----GR
     H.vulgare_TA37167_4513#1    (1)  --------MALMLSPAATFFPVAGKPSAA---ATRSLLFTTSSSSSSKGR
    T.aestivum_TA70800_4565#1    (1)  --------MALMLSPAATFFSVAGGKPSS---ATRSLFFTTTSTTK--GR
                     Consensus   (1)                   T   V           M  LL  TT       R
```

FIGURE 8

```
                                      51                                               100
              A.cepa_CF450468#1   (1) ----------------MFKINRKSNDV-HSH-MRLRCAFSSLSSSQDLTS
        A.thaliana_AT1G29810.1#1 (21) SLFNNLYRRHKRFVHWTSKMSTDSVRS-STT-GGSASGARTFCSLADLST
                   AT1G29810.1#1 (21) SLFNNLYRRHKRFVHWTSKMSTDSVRS-STT-GGSASGARTFCSLADLST
   P.trichocarpa_scaff_XI.475#1   (1) --------------------------------------MEVLLPMIDLAA
      S.lycopersicum_BP905715#1   (1) --------------------MCSNLAV-SAK-TNTHYGIKTLSSAAVLST
            H.vulgare_BQ462597#1  (9) FMRIQPRAPMTAAGGAAFFNASCPSSP-RTV-AHLEVSGEQASPKVELSE
       H.vulgare_TA39366_4513#1   (9) FMRIQPRAPMTAAGGAAFFNASCPSSP-RTV-AHLEVSGEQASPKVELSE
          T.aestivum_CA729021#1   (9) FIGIQPRAPMTAAGGAALFYASCPSSP-RTV-AHLEVRGEQAAAEAELSK
          T.aestivum_CN012063#1   (1) ---------MSAAGGAAPFYASCPSSP-RTV-AHLEVRGEQAAAEVELSK
      T.aestivum_TA92660_4565#1   (9) FIRIQPRAPMSAAGGAAPFYASCPSSP-RTV-AHLEVRGEQAAAEVELSK
          T.aestivum_CV761453#1   (1) ---------MEGRTLLLDQAAPSASAP-TGR-SASQATGDEAAAKVELSK
        O.sativa_Os01g0663500#1  (25) WPTVSKNLPLLGHGRSHHPMYASQDEI-KMS-SRRWCHG--SPDNQELAK
      S.officinarum_CA275526#1    (1) ------------------MDTRGQDEN-KIL-TARGCHS--SPESQELAM
      S.officinarum_CA275597#1    (1) ------------------MHTRGQDEN-KIL-TARGCHS--SPESQELAM
        A.thaliana_AT5G51110.1#1 (46) LTVTRSNLAQDFLGDFGARDPYPEEIA-SQF-GDKVLGCQSTEHKILIPN
                   AT5G51110.1#1 (46) LTVTRSNLAQDFLGDFGARDPYPEEIA-SQF-GDKVLGCQSTEHKILIPN
S.lycopersicum_TA38587_4081#1    (39) ---TRIRCNTNLLGDFGARDPFPAEVE-SKF-GEKVLGNPDTEHKILIPA
   P.trichocarpa_scaff_232.30#1  (44) ---LQAMGAGDKLGEFGARDPFPAEIE-SGF-AEKVLGNGNTEHKILIPT
         O.sativa_Os03g0100200   (35) KQKSVVVAMADLLGDFGARDPFPEEIE-SNF-GERVLGNVDTLHNILIPT
          T.aestivum_CA631555#1  (35) SVR-KVVAMADILGDFGARDPFPEEIA-SNF-GEKTLGNVDTLHRILIPT
          T.aestivum_CV772309#1  (40) STR-KVVAMADILGDFGARDPFPEEIA-SNF-GEKTLGNVDTLHRILIPT
          T.aestivum_CA636226#1  (40) STR-KVVAMADILGDFRRAGPVPGGDSPSNFRGERRWATLDTLHRILIPT
          T.aestivum_CA721065#1  (43) SSR-KVVAMADILGDFGRAGPVPRREIAKQLRGRKTLGNVGHGCNAILN-
          T.aestivum_CK206167#1  (43) SSR-KVVAMADILGDFGARDPFPEEIA-SNF-GEKTLGNVDTLHRILIPT
          T.aestivum_CK211978#1  (43) SSR-KVVAMTDILGDFGARDPFPEEIA-SNF-GEKTLGNVDTLHRILIPT
      T.aestivum_TA70798_4565#1  (43) SSR-KVVAMADILGDFGARDPFPEEIA-SNF-GEKTLGNVDTLHRILIPT
          T.aestivum_CK212754#1  (40) STR-KVVAMADILGDFGARDPFPEEIA-SNF-GEKTLGYVDTLHRILIPT
      T.aestivum_TA70795_4565#1  (40) STR-KVVAMADILGDFGARDPFPEEIA-SNF-GEKTLGNVDTLHRILIPT
          T.aestivum_CV768439#1  (40) STR-KVVAMADILGDFGARDPFPEEIA-SNF-GEKTLGNVDTLHRILIPT
          T.aestivum_CA600360#1  (18) STR-KVVAMADILGDFGARDPFPEEIA-SNF-GEKTLGNVDTLHRILIPT
          T.aestivum_CK212253#1  (32) SVR-KVVAMPDILGDYGARDPFPEEIA-SNF-GEKTLGNVDTVHRILIPT
      T.aestivum_TA70797_4565#1  (35) SVR-KVVAMADILGDFGARDPFPEEIA-SNF-GEKTLGNVDTLHRILIPT
       H.vulgare_TA37167_4513#1  (40) GGRGKVVAMADILGDFGARDPFPEEIA-SNF-GEKTLGNVDTLHRILIPT
      T.aestivum_TA70800_4565#1  (38) SVR-KVVAMADILGDFGARDPFPEEIA-SNF-GEKTLGNVDTLHRILIPT
                      Consensus  (51)   R KVVA  DILGDFGARDPFP EIA SNF GEK LG  DT HRILIPT
```

FIGURE 8 (continued)

```
                                           101                                              150
              A.cepa_CF450468#1     (33)  KKCVPCNS-------KDIQPISEQSANELLSKVQGWEMSN----DVGVMK
       A.thaliana_AT1G29810.1#1     (69)  KKCVPCNA-------KDLRAMTEQSAQDLLQKVAGWDLAN----DNDTLK
                  AT1G29810.1#1     (69)  KKCVPCNA-------KDLRAMTEQSAQDLLQKVAGWDLAN----DNDTLK
 P.trichocarpa_scaff_XI.475#1     (13)  KKCVPCNS-------KDLQAMTEESANDLLSKVAGWNLVN----ENGTLK
   S.lycopersicum_BP905715#1     (29)  KKCVPCNT-------KDLRPMTEEAAYQLMPQVSGWDLVN----DGGTLK
           H.vulgare_BQ462597#1     (57)  KSCIPCNS-------MDLHAMSEDSAKKSLEQVTGWELKN----EGDILK
      H.vulgare_TA39366_4513#1     (57)  KSCIPCNS-------MDLHAMSEDSAKKSLEQVTGWELKN----EGDILK
        T.aestivum_CA729021#1     (57)  KSCIPCNS-------KDLHAMSEDSAKKSLEQVTGWELKN----EGDILK
        T.aestivum_CN012063#1     (40)  KSCIPCIS-------KDLHAMSEDSAKKSLEQVTGWELKN----EGDILK
    T.aestivum_TA92660_4565#1     (57)  KSCIPCIS-------KDLHAMSEDSAKKSLEQVTGWELKN----EGDILK
        T.aestivum_CV761453#1     (40)  RSCVSCNS-------KDLQAMSEDSAKTLLEQVAGWEVKN----EGDILK
     O.sativa_Os01g0663500#1     (71)  KICVPCNS-------KDIHAMPEDSAKKMLEQVGGWELAT----EGDILK
     S.officinarum_CA275526#1     (29)  KSCVPCNS-------KDLHPMSEDSAKKLLVQVNGWELIT----EGGILK
     S.officinarum_CA275597#1     (29)  XSCVPCNS-------KDLHPMSEDSAKKLLVQVNGWELIT----EGVILK
       A.thaliana_AT5G51110.1#1     (94)  ASVLSLSQLQCSPVSSSQPPLSGDDARTLLHKVLGWSIVDN---EAGGLK
                  AT5G51110.1#1     (94)  ASVLSLSQLQCSPVSSSQPPLSGDDARTLLHKVLGWSIVDN---EAGGLK
S.lycopersicum_TA38587_4081#1     (84)  ASALSLANQECTDISPNQTPLSEFEAKQLLFKVVGWRIANE---DGV-LK
 P.trichocarpa_scaff_232.30#1     (89)  VSALSLSQQECTPISPLQDPMSKDDAQKLLKKVLGWRLLDE---EGG-LK
        O.sativa_Os03g0100200     (83)  LSVLSIAR---LPLEPNPAPVDAADARRLLHKVVGWRLLD----DADGMR
        T.aestivum_CA631555#1     (82)  LSVLSLSR---VPLDADPAPLSXEDARKLLFKVVGWRLLFPNKGDSERAQ
        T.aestivum_CV772309#1     (87)  LSVLSLSR---VPLDADPAPLSEEDARRLLFKVVGWRLLFPSKGDSDVLK
        T.aestivum_CA636226#1     (89)  SPCLPLPR----PAPTPTGPLSEEDARSCSSRWSVGVLXPK---QGHSDC
        T.aestivum_CA721065#1     (91)  ----PQGX--------SVXPLSRRPRLRKPNPGAGFFRRRERPGKGCSFK
        T.aestivum_CK206167#1     (90)  LSVLSLSR---VPLEAHPAPLSEEDARRLLFKVVGWRLLFPGKGDSDVLK
        T.aestivum_CK211978#1     (90)  LSVLSLSR---VPLEAHPAPLSEEDARRLLFKVVGWRLLFPGKGDSDVLK
    T.aestivum_TA70798_4565#1     (90)  LSVLSLSR---VPLEAHPAPLSEEDARRLLFKVVGWRLLFPGKGDSDVLK
        T.aestivum_CK212754#1     (87)  LSVLSLSR---VPLDADPAPLSEEDARRLLFKVVGWRLLFPSKGDSDVLK
    T.aestivum_TA70795_4565#1     (87)  LSVLSLSR---VPLDADPAPLSEEDARRLLFKVVGWRLLFPSKGDSDVLK
        T.aestivum_CV768439#1     (87)  LSVLSLSR---VPLDADPAPLSEEDARRLLFKVVGWRLLFPSKGDSDVLK
        T.aestivum_CA600360#1     (65)  LSVLSLSR---VPLDADPAPLSEEDARKLLFKVVGWRLLFPSKGDSDVLK
        T.aestivum_CK212253#1     (79)  LSVLSLSR---VPLDADPAPLSEEDARKLLFKVVGWRLLFPSKGDSDVLK
    T.aestivum_TA70797_4565#1     (82)  LSVLSLSR---VPLDADPAPLSEEDARKLLFKVVGWRLLFPSKGDSDVLK
     H.vulgare_TA37167_4513#1     (88)  LSVLSLSR---VPLDAHPAPLSEEDARRLLFKVVGWRLLFPTKGDSDVLK
    T.aestivum_TA70800_4565#1     (85)  LSVLSLSR---VPLDADPAPLSEEDARKLLFKVVGWRLLFPSKGDSDVLK
                      Consensus    (101)   S LSLS      PL  D  PLSEEDARKLL KVVGW LL      DGDVLK
```

FIGURE 8 (continued)

```
                                        151                                              200
                 A.cepa_CF450468#1   (72) LHRSWKVKSFMKGLDFFKLVADVAEAE-------GHHPDLHLVGWNNVKI
         A.thaliana_AT1G29810.1#1   (108) LHRSWRVKSFTKGLDFFQRVADIAESE-------GHHPDLHLVGWNNVKI
                    AT1G29810.1#1   (108) LHRSWRVKSFTKGLDFFQRVADIAESE-------GHHPDLHLVGWNNVKI
      P.trichocarpa_scaff_XI.475#1   (52) LNRSWKVKSFTKGLELFKLVGNVAEAEVTVYANAGHHPDLHLVGWNNITI
        S.lycopersicum_BP905715#1    (68) LHKSWKVKTFTKGLEFFQEVASVAEAE-------GHHPDLHLVGWNNVKI
             H.vulgare_BQ462597#1    (96) LHRAWKVKNFVKGLEFFQLVAAVAEEE-------GHHPDLHLVSWNNVKI
         H.vulgare_TA39366_4513#1    (96) LHRAWKVKNFVKGLEFFQLVAAVAEEE-------GHHPDLHLVSWNNVKI
           T.aestivum_CA729021#1     (96) LHRAWKVKNFVKGLEFFQLVAAVAXXE-------GHHPDLHLVSWNNVKI
           T.aestivum_CN012063#1     (79) LHRAWKVKNFVKGLEFFQLVAAVAEEE-------GHHPDLHLVSWNNVKI
        T.aestivum_TA92660_4565#1    (96) LHRAWKVKNFVKGLEFFQLVAAVAEEE-------GHHPDLHLVSWNNVKI
           T.aestivum_CV761453#1     (79) LHRAWKVKNFAKGLEFFQLVAAVAEEE-------GHHPDLHLVGWNNVKI
       O.sativa_Os01g0663500#1   (110) LHRAWKVKNFVKGLEFLQLVAAVAEEE-------GHHPDLHLVGWNNVKI
         S.officinarum_CA275526#1    (68) LHRAWKVKNFVKGLEFFQLVAAIAEEQ-------GHHPDLHLVGWNNVKI
         S.officinarum_CA275597#1    (68) LHRAWKVKNFVKGLEFFQLVAAIAEEQ-------GHHPDLHLVGWNNVKI
         A.thaliana_AT5G51110.1#1   (141) IRCMWKVRDFGCGVELINRIHKVAEASGH------YPSLHLESP-TQVRA
                    AT5G51110.1#1   (141) IRCMWKVRDFGCGVELINRIHKVAEASGH------YPSLHLESP-TQVRA
   S.lycopersicum_TA38587_4081#1   (130) LQCTWKLKDFDCGVELINRIGKVVEGTGH------LPTLHQLQQSNQVHA
    P.trichocarpa_scaff_232.30#1   (135) LQCLWKLRDFKCGVELVNRIYKATESCGH------FPNVHLEQP-NQVRA
           O.sativa_Os03g0100200   (126) LQCVWKVRDEACGHELVARINAAVDGAP---------ATVVFEAPNQVRA
            T.aestivum_CA631555#1   (129) ARVRLESPXQGCGEQLIARXNK--TSTAP------ACPAALNSRX-----
            T.aestivum_CV772309#1   (134) LECVWKVRDQACGDELVARINKGLRGAGP------SPRRVSGSRRQTSQG
            T.aestivum_CA636226#1   (132) QLHASGSPHKAXGNXXPQDLTRAXRAX--------KPRRLRFRX-QQLRP
            T.aestivum_CA721065#1   (129) GSVXDXGPPXSPGXG-----------------------------------
            T.aestivum_CK206167#1   (137) LECVWKVRDQACGDELIARIGKALDGAGH------APAALLFEPPNKSGH
            T.aestivum_CK211978#1   (137) LECVWKVRDQACGDELIARIGKALDGAGH------APAALLFEPPNQARP
       T.aestivum_TA70798_4565#1   (137) LECVWKVRDQACGDELIARIGKALDGAGH------APAALLFEPPNQVRA
            T.aestivum_CK212754#1   (134) LECVWKVRDQACGDELVARINKALDGAGH------APAALRFEAPNQVRA
       T.aestivum_TA70795_4565#1   (134) LECVWKVRDQACGDELVARINKALDGAGH------APAALRFEAPNQVRA
            T.aestivum_CV768439#1   (134) LECVWKVRDQACGDELVARINKALDGAGH------APGALRFEAPNQVRA
            T.aestivum_CA600360#1   (112) LECVWKVRDQACGDELVARINKALDGAGH------APAALRFEAPNQVXA
            T.aestivum_CK212253#1   (126) LECVWKVRDQACGDELIARINKTLDGAGH------APAALQFEAPNQVRA
       T.aestivum_TA70797_4565#1   (129) LECVWKVRDQACGDELIARINKTLDGAGH------APAALQFEAPNQVRA
        H.vulgare_TA37167_4513#1   (135) LECVWKVRDQACGDELVARINKALDGAGH------APAALSFEPPNQVRA
       T.aestivum_TA70800_4565#1   (132) LECVWKVRDQACGDELIARINKTLDGAGH------APAALQFEAPNQVRA
                        Consensus   (151) L C WKVRDFACGLELIQRIAKV EG G        P  L   G NQVR
```

FIGURE 8 (continued)

```
                                        201                                              250
                A.cepa_CF450468#1 (115) DIWTHSVGGLTENDFILAAKINSLEIEHLLS---KKGRK-----------
         A.thaliana_AT1G29810.1#1 (151) EIWTHAIGGLTENDFILAAKINELQVEDLLR---KKKVAK----------
                    AT1G29810.1#1 (151) EIWTHAIGGLTENDFILAAKINELQVEDLLR---KKKVAK----------
     P.trichocarpa_scaff_XI.475#1 (102) EIWTHAVGGLTENDFILAAKINGLNLHHLLR---KKAAA-----------
      S.lycopersicum_BP905715#1 (111) DIWTHSVGGLTENDFILAA-------------------------------
           H.vulgare_BQ462597#1 (139) DVWTHSVRGLTDNDFILAAKINELKLEGLLS---KKKATTHE--------
      H.vulgare_TA39366_4513#1 (139) DVWTHSVRGLTDNDFILAAKINELKLEGLLS---KKKATTHE--------
          T.aestivum_CA729021#1 (139) DVWTHSVXXLTDNDFILAAKIX----------------------------
          T.aestivum_CN012063#1 (122) DVWTHSVRGLTDNDFILAAKINELKLEGLLS---KKKATSQE--------
     T.aestivum_TA92660_4565#1 (139) DVWTHSVRGLTDNDFILAAKINELKLEGLLS---KKKATSQE--------
          T.aestivum_CV761453#1 (122) DVWTHSVSGLTDNDFILAAKINELKLGGLLS---KKKATAQK--------
        O.sativa_Os01g0663500#1 (153) DVWTHSVRGLTDNDFILAAKINNLNLEGLLS---KKATVQK---------
      S.officinarum_CA275526#1 (111) DVWTHSVRSLTSNDFILAAKINDLTLEGIIR---KKAT------------
      S.officinarum_CA275597#1 (111) DVWTHSVRGLTSNDFILAAKINDLTLEGIIR---KKAT------------
       A.thaliana_AT5G51110.1#1 (184) ELFTSSIGGLSMNDFIMAAKIDDIKTSDLSP---RKRAWA----------
                  AT5G51110.1#1 (184) ELFTSSIGGLSMNDFIMAAKIDDIKTSDLSP---RKRAWA----------
 S.lycopersicum_TA38587_4081#1 (174) ELWTPSIGGLSMNDFIIAAKIDQVKTSDLVP---RKRVWA----------
   P.trichocarpa_scaff_232.30#1 (178) ELWTASLGGLSLNDFIVAAKIDEIKTSDLVP---KKRVWA----------
         O.sativa_Os03g0100200 (167) ELQTPSAGGLTVNDFIVAARIDKVKTVDLIP---KKRVWA----------
          T.aestivum_CA631555#1 (166) PX------------------------------------------------
          T.aestivum_CV772309#1 (178) PTLHPVRKGDETQKTSSSGGRIEPKSGTKYPPPKKRRVWANANGPTNSPS
          T.aestivum_CA636226#1 (173) IXSRRXRLTQX---------------------------------------
          T.aestivum_CA721065#1 (144) --------------------------------------------------
          T.aestivum_CK206167#1 (181) SSARPP--------------------------------------------
          T.aestivum_CK211978#1 (181) QLCTPSVGGLSANDFIIAARIDQIKTKDLLP---KKRVWAC---------
     T.aestivum_TA70798_4565#1 (181) QLCTPSVGGLSANDFIIAARIDQIKTKDLLP---KKRVWAC---------
          T.aestivum_CK212754#1 (178) QLYKPSVGGLSANDFIIAARIDQIKTKDLLP---KKRVWAC---------
     T.aestivum_TA70795_4565#1 (178) QLYTPSVGGLSANDFIIAARIDQIKTKDLLP---KKRVWAC---------
          T.aestivum_CV768439#1 (178) QLYTPVVGGLSANDFIIAARIDQIKTKISSK---KEGLGMLMAAXSPPSC
          T.aestivum_CA600360#1 (156) QLYTPSVSGLSANDFIIAARIDQIKTKDLLP---XXXVWAC---------
          T.aestivum_CK212253#1 (170) QLYTPSVAGLSANDFIITARIDQIKTKDLLP---KKRVWAC---------
     T.aestivum_TA70797_4565#1 (173) QLYTPSVGGLSANDFIIAA-------------------------------
      H.vulgare_TA37167_4513#1 (179) QLYTPSVGGLSANDFIIAARIDQINTKDLLP---KKRVWAC---------
     T.aestivum_TA70800_4565#1 (176) QLYTPSVGGLSANDFIIAARIDQIKTKDLLP---KKRVWAC---------
                      Consensus (201) DLWT SVGGLT NDFILAAKI  IK   LL    KKR  A
```

FIGURE 8 (continued)

```
                                          251                     275
                 A.cepa_CF450468#1  (151) -------------------------
          A.thaliana_AT1G29810.1#1  (188) -------------------------
                     AT1G29810.1#1  (188) -------------------------
     P.trichocarpa_scaff_XI.475#1  (138) -------------------------
        S.lycopersicum_BP905715#1  (130) -------------------------
             H.vulgare_BQ462597#1  (178) -------------------------
          H.vulgare_TA39366_4513#1  (178) -------------------------
            T.aestivum_CA729021#1  (161) -------------------------
            T.aestivum_CN012063#1  (161) -------------------------
        T.aestivum_TA92660_4565#1  (178) -------------------------
            T.aestivum_CV761453#1  (161) -------------------------
         O.sativa_Os01g0663500#1  (191) -------------------------
         S.officinarum_CA275526#1  (146) -------------------------
         S.officinarum_CA275597#1  (146) -------------------------
          A.thaliana_AT5G51110.1#1  (221) -------------------------
                     AT5G51110.1#1  (221) -------------------------
 S.lycopersicum_TA38587_4081#1  (211) -------------------------
     P.trichocarpa_scaff_232.30#1  (215) -------------------------
          O.sativa_Os03g0100200  (204) -------------------------
            T.aestivum_CA631555#1  (168) -------------------------
            T.aestivum_CV772309#1  (228) FFGSSFWNKHHPDFWFSWEKPIGYP
            T.aestivum_CA636226#1  (184) -------------------------
            T.aestivum_CA721065#1  (144) -------------------------
            T.aestivum_CK206167#1  (187) -------------------------
            T.aestivum_CK211978#1  (219) -------------------------
        T.aestivum_TA70798_4565#1  (219) -------------------------
            T.aestivum_CK212754#1  (216) -------------------------
        T.aestivum_TA70795_4565#1  (216) -------------------------
            T.aestivum_CV768439#1  (225) SSLHTX-------------------
            T.aestivum_CA600360#1  (194) -------------------------
            T.aestivum_CK212253#1  (208) -------------------------
        T.aestivum_TA70797_4565#1  (192) -------------------------
         H.vulgare_TA37167_4513#1  (217) -------------------------
        T.aestivum_TA70800_4565#1  (214) -------------------------
                         Consensus  (251)
```

FIGURE 8 (continued)

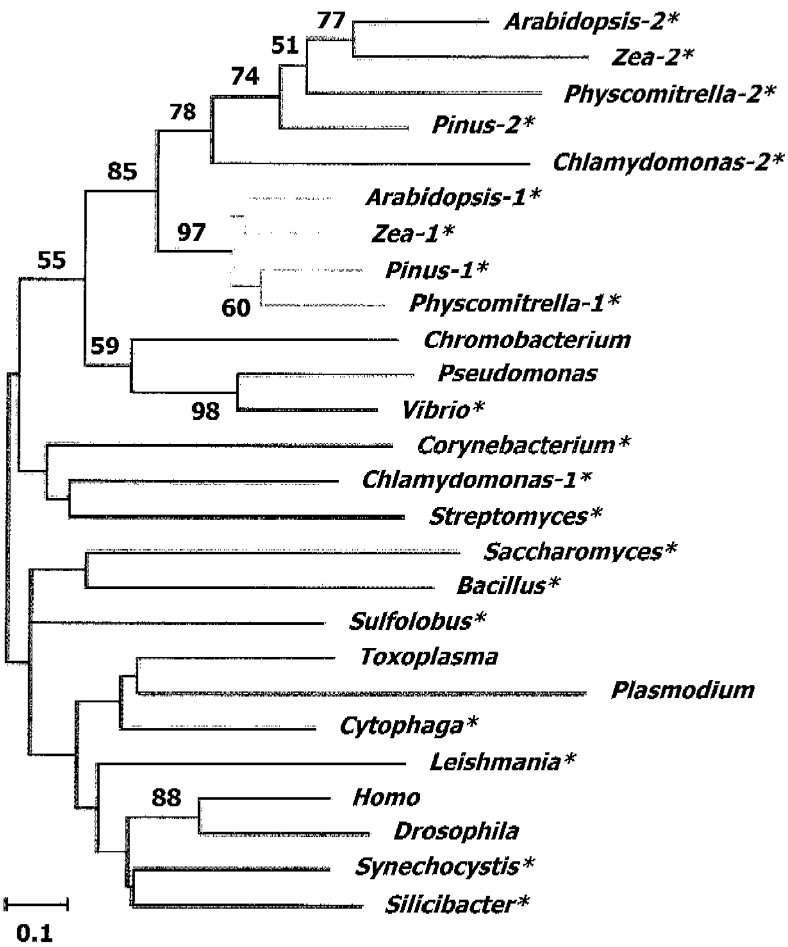
77
51
74
78
85
55
97
60
59
98
88
Arabidopsis-2*
Zea-2*
Physcomitrella-2*
Pinus-2*
Chlamydomonas-2*
Arabidopsis-1*
Zea-1*
Pinus-1*
Physcomitrella-1*
Chromobacterium
Pseudomonas
Vibrio*
Corynebacterium*
Chlamydomonas-1*
Streptomyces*
Saccharomyces*
Bacillus*
Sulfolobus*
Toxoplasma
Plasmodium
Cytophaga*
Leishmania*
Homo
Drosophila
Synechocystis*
Silicibacter*
0.1

FIGURE 9

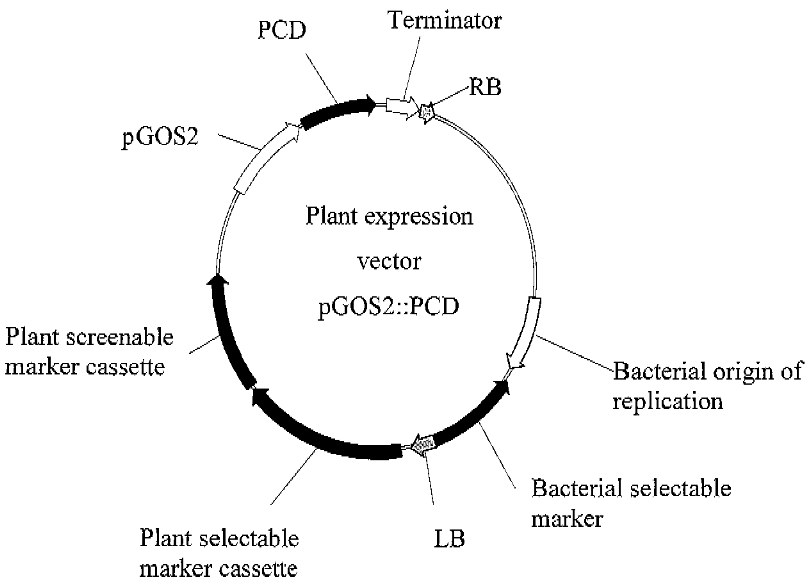
PCD
Terminator
RB
pGOS2
Plant expression
vector
pGOS2::PCD
Plant screenable
marker cassette
Bacterial origin of
replication
Bacterial selectable
marker
LB
Plant selectable
marker cassette

FIGURE 10

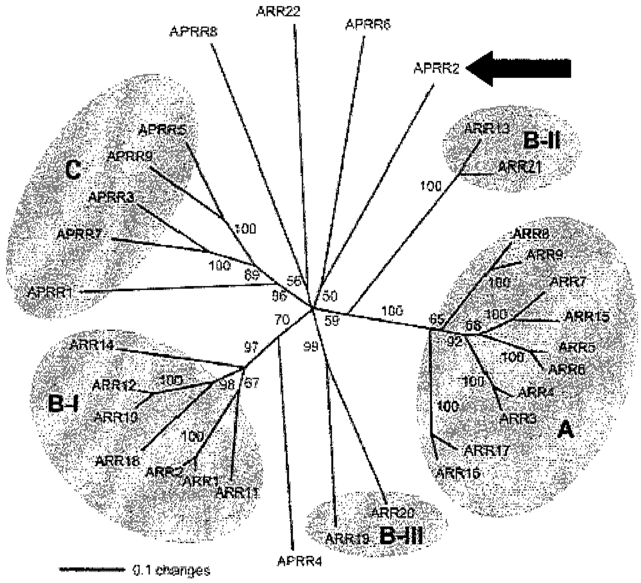


According to Mason *et al.* (2004) Plant Phys 135: 927–937

**FIGURE 11**

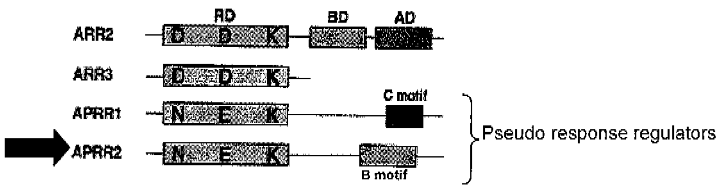


Hwang *et al.* (2002) Plant Phys129: 500–515

**FIGURE 12**

```
                          1                                                50
   Lyces_PRR2      (1) MICIENELLGWKDFPKGLKVLLLDE------DSNSAAEMKSRLEKMDYIV
   Aqufo_PRR2      (1) MVCTADDLLGWKDFPKGLKVLLLKE------DGISATEIKSKLEEMDYIV
  Arath_APRR2      (1) MVITANDLSKWENFPKGLKVLLLLNGCDSDGDGSSAAETRSELESMDYIV
   Eucgr_PRR2      (1) MVCTASDLQEWKDFPKGLKVLLLDQ------DSDSASEIRSKLELMDYVV
   Glyma_PRR2      (1) MVCTANDLQGWKDFPKGLRVLLLEG------DSSSAAEIREQLEAMDYKV
   Lotja_PRR2      (1) MVCTASDLQQWKDFPKGLRVLLLEG------DSGSATEIRAKLEAMDYIV
   Lyces_PRR2 II   (1) MVCTENELLEWKDFPKGLKVLLLDT------DSNFASQMRSRLQQMDYIV
   Medtr_PRR2      (1) MVCTANDLQGWKDFPKGLRVLLLEG------DNNSASEIRTKLESMDYNV
   Poptr_PRR2 I    (1) MVCTTNDLSAWKDFPKGLRVLLLDE------DSMSAAEIKSKLEAMDYIV
   Poptr_PRR2 II   (1) MVCTTNDLSAWKDFPKGLSVLLLDE------DNSSAAEIKSKLEALDYIV
   Vitvi_PRR2      (1) MVCTANDLQEWKDFPKGLRVLLLDD------DTTSAAEIRSKLEEMDYIV
    Consensus      (1) MVCTANDL GWKDFPKGLKVLLLD       DS SAAEIRSKLEAMDYIV
    IPR001789                           XXXXXXXXXXXXXXXXXXXXXXXXXXXXXXXXX

                       51                                               100
   Lyces_PRR2     (45) YSFCNESEALTAISSKSEGFHVAIVEVSAGNSDGVLRFLESAKD-LPTIM
   Aqufo_PRR2     (45) STLFNLDDALMEISNKPGSFHVAIVEVSTNNQHECFKFLETVRD--PTIM
  Arath_APRR2     (51) TTFTDETEALSAVVKNPESFHIAIVEVNMSAESESFKFLEAAKDVLPTIM
   Eucgr_PRR2     (45) FTFCNEDEALSAIASKPDSFHIAMVEVSTSS-NGSFEFLEAARD-LPTIM
   Glyma_PRR2     (45) STFYDENEALSALSSSPKGFHVAIVEVSTSCSLGGFKFLENAKD-LPTIM
   Lotja_PRR2     (45) FTFYNENEALSAISSDPEGFHVAVVEVSTSSNQGGFKFLENVKD-LPTIM
   Lyces_PRR2 II  (45) YTFCNENEALSAISSKSEVFHVAIVEVSAGNSDGGFKFLESAKD-LPTIM
   Medtr_PRR2     (45) STFYNENEALSAISSSPKCFHVAIVEVSISCPDGGFKFLENAKD-LPTIM
   Poptr_PRR2 I   (45) YTFCNETEALSAISNEPGSFHVAIVEVSMSNSSRSFKFLETSKD-LPTIM
   Poptr_PRR2 II  (45) YTFCNENEALLAISNEPGSFHVAIVEVSTSNSNGSFKFLETAKD-LPTIM
   Vitvi_PRR2     (45) STFCNENEALSAISSKPESFHVAIVEVSTGN-NGSFKFLETAKD-LPTIM
    Consensus     (51) YTFCNENEALSAISS PESFHVAIVEVSTSN  G FKFLETAKD LPTIM
    IPR001789          XXXXXXXXXXXXXXXXXXXXXXXXXXXXXXXXXXXXXXXXXXXXXXXXXX

                       101                                              150
   Lyces_PRR2     (94) TSNIHSLSTMMKCIALGAVEFLQKPLSDDKLKNIWQHVVHKAFNTR-KDV
   Aqufo_PRR2     (94) ISNIQCLSTMMKCIALGAAEFLQQPLSEDKLRNIWQHVVHKAFNAKESDL
  Arath_APRR2    (101) ISTDHCITTTMKCIALGAVEFLQKPLSPEKLKNIWQHVVHKAFNDGGSNV
   Eucgr_PRR2     (93) MSNNHCLSTIMKCIALGAVEFLHKPLCEDKLRNIWQHVAHKAFNAGVSEV
   Glyma_PRR2     (94) TSKDQCLNTMMKCIALGAVEFLSKPLSEDKLKNIWQHVVHKAFNAGANVL
   Lotja_PRR2     (94) TSNSQCLNTMMKCIALGAVEFLTKPLSEDKLKNIWQHVVHKAFNAGTNAP
   Lyces_PRR2 II  (94) VSDIHSINIMMKCIALGAVEFLQKPLSDDKLRNIWQHVVHKAFHSGGKSV
   Medtr_PRR2     (94) TSNSQCINTMMKCIALGAVEFLTKPLSEDKLKNIWQHVVHKAFNAEASAL
   Poptr_PRR2 I   (94) TSSIDCLNTMMKCIALGAVEFLRKPLSEDKLRNIWQHVVHKAFNAGGSVQ
   Poptr_PRR2 II  (94) TSNIHCLNTMMKCIALGAVEFLRKPLSEDKLRNIWQHVVHKAFNAGGSVQ
   Vitvi_PRR2     (93) ISSIHCLSTMMKCIALGAVEFLRKPLSEDKLRNIWQHVVHKAFNAGGSVL
    Consensus    (101) TSNIHCLNTMMKCIALGAVEFL KPLSEDKLRNIWQHVVHKAFNAGGS L
    IPR001789          XXXXXXXXXXXXXXXXXXXXXXXXXXXXXXXXXXXX
```

FIGURE 13

```
                      151                                                200
  Lyces_PRR2    (143) SKSLEPVKDSVLSMLQLQLEMGEADDKSSNGTEPPTAVAESNTEQSSGCD
  Aqufo_PRR2    (144) TKSLKPIKDTVVSMLQLPRETNDTEDYNLLETENSAQSRECENEQSTVSD
 Arath_APRR2    (151) SISLKPVKESVVSMLHLETDMTI------------------EEKD-----
  Eucgr_PRR2    (143) SESLKLVKETSVSMLQHQLKNEE-QNNDESREMRKLSVHETDQEMSAASD
  Glyma_PRR2    (144) SESLKPVKESVESMLQLQTENGQDKSRISIDLENVSRFSDIDHEQSAVCD
  Lotja_PRR2    (144) SESLRPVKESVESILQLQTDNGQHESNISIDIENVSRLGDNDHEQSVGSD
  Lyces_PRR2II  (144) SESLKPVKESLLSILELQPVKHEADNENTNEAEPLISVVENQKASSSCCD
  Medtr_PRR2    (144) SESLKPVKESVESMLHLQTDNTLHESTISIDLDKVSKFSDNEHEHSAASD
  Poptr_PRR2 I  (144) SKSLKPVKDSVVSMLELKDGLEENENKNMEKTENVSQAQESNEQQSPASD
  Poptr_PRR2II  (144) SESLKPVKDSIVSMLELKVELEENEDENMEKTENASLAQESNEQQSPASD
  Vitvi_PRR2    (143) PESLKPVKESVASMLQLQMENEEPRNESSAETLNVSNVHENDHMQSAGTD
   Consensus    (151) SESLKPVKESVVSMLQLQ E  E E   S D ENVS   E D EQSAASD

                      201                                                250
  Lyces_PRR2    (193) KYPAPSIPQLKQGVRSVDDGDCHDHTIFSTDQDSGEHDA-DTKSVETTYN
  Aqufo_PRR2    (194) KFPAPSTPQLKQGCRFSDDGDCQHHTNFLVDKAHRGIER-ESKSVDTTCN
 Arath_APRR2    (178) --PAPSTPQLKQDSR-LLDGDCQENINFSMENVNSSTEKDNMEDHQDIGE
  Eucgr_PRR2    (192) KYPAPSTPQLKGGARLLDDGDCQDQTNGTVEKESGEQDG-ESKFVETTCD
  Glyma_PRR2    (194) KYPAPSTPQLKQGTRLLDDGDCHDQTNCSTEKESGEHDR-ECKSVETSCG
  Lotja_PRR2    (194) KYPAPSTPQLKQGARLLDDGDCHEQTNCSTEKESGEHDE-ESKSVEISCE
 Lyces_PRR2 II  (194) KYPAPSTPQQKQGVRSVDDVDYQDHTILSNEQDSGMHEG-DTKSVETTSC
  Medtr_PRR2    (194) KYPAPSTPQLKQGARLVDDGDCHEQTNCLIEKESGEHDSGECKFVDTSCE
  Poptr_PRR2 I  (194) KYPAPSTPQLKQGERLLDDGDCQDHINCLIEKESVEQEG-DSKSVETTC-
 Poptr_PRR2 II  (194) KHPAPSTWQFKQVGRLLDDGDCQDHINCSVEKDSGEQEG-ESKSVETTC-
  Vitvi_PRR2    (193) KYPAPSTPQLKQGGRSLDDGDCLDQTNCSTEKESGEQDG-ESKSVETTCG
   Consensus    (201) KYPAPSTPQLKQG RLLDDGDCQDQTNCSVEKESGE DG ESKSVETTC

                      251                                                300
  Lyces_PRR2    (242) ---------NSLAENNVQTSPTVQQGDIILKED-NVSSPDLKTET-DIAT
  Aqufo_PRR2    (243) YLVAETTSDNSLAEVTVTVDPSTTASEAMIKEE--DSLENSRSGS-SLVS
 Arath_APRR2    (225) ------------SKSVDTTNRKLDDDKVVVKEERGDSEKEEEGETGDLIS
  Eucgr_PRR2    (241) ---------DSMSENIQEAQG-QRDRDVLVKEE-DDSANNSKCES-MISP
  Glyma_PRR2    (243) ---------NLNAESSPQPRE---PDKTLIREE-DDFANVSKGES-AVSL
  Lotja_PRR2    (243) ---------NLNTESSSQLSK---PEKTLIKEE-EAFADGSKGEI-AVSQ
 Lyces_PRR2 II  (243) ---------DSVAETTVLADSSERLGEAITKEE-HYSAADQHMED-PIAT
  Medtr_PRR2    (244) ---------NLNAESSPQ------PTKHLIKEE-EDFAKGSKGEG-GASL
  Poptr_PRR2 I  (242) ----------AMSEETLQAGDPQSFTETVIKEE-DDSTDGVKSEN-NMCP
 Poptr_PRR2 II  (242) ----------AMSQETLKAGHPPCFIETVIKET-GDLTDGAKSEN-NIHP
  Vitvi_PRR2    (242) ---------TSVAEVTAQVSPPQGLGESVIKEE-DDSADGCKSES-NMSP
   Consensus    (251)           SMAE T Q        E LIKEE DDSA  SK ES  IS
```

FIGURE 13 (continued)

```
                         301                                               350
     Lyces_PRR2   (281) TSRSNDCPDNSIMHSAEPSKASGPHSSNGTKSNRKKIK--VDWTPELHKK
     Aqufo_PRR2   (290) HPQRKENKTDSISKVKNPRRASLHPNPCGKKSNRK-MK--VDWTPDLHKR
    Arath_APRR2   (263) EKTDSVDIHKKEDETKPINKSSGIKNVSGNKTSRK--K--VDWTPELHKK
     Eucgr_PRR2   (279) LSENKDRSHDVHGCNDNQSKASSLHNSARTRVNRKKMK--VDWTPELHKK
     Glyma_PRR2   (279) NPHNKKFLSNADGNT-SPNKTGVLNDSCEIKANRKKVK--VDWTPELHKK
     Lotja_PRR2   (279) NEDNRKFLGSAEGNE-SPNKTGVLSDSCEKKANRKKMK--VDWTPELHKK
 Lyces_PRR2 II(282) CSPS---NDNGSTCSADPNKASGLHSSSGTKANKKKMK--VDWTPELHKK
     Medtr_PRR2   (277) NPLNKNFLGDAGGNT-SLNKTRVFNDPCENKANRKKMK--VDWTAELHKK
   Poptr_PRR2 I(280) NSQNKDTLNHSNGCA---EKASSLHNSHGTRANRKKMKFQVDWTPELHRK
  Poptr_PRR2 II(280) NPQNKDSLNHSN------DVASDLHTSNGTRANRKKMK--VDWTPELHKK
     Vitvi_PRR2   (281) HPQNKDSLSEFGGDARNPRKASGVHSPCGTRANRKKMK--VDWTPELHKK
      Consensus   (301)    NKD L  S G    P KAS LH S GTKANRKKMK  VDWTPELHKK
      IPR006447                                            XXXXXXXXXXXXXXX
```

```
                         351                                               400
     Lyces_PRR2   (329) FVQAVEQLGIDQAIPSRILDLMKVEGLTRHNVASHLQ-----KYRMHRKQ
     Aqufo_PRR2   (337) FVQAVEQLGVDQAIPSRILDLMKVEGLTRHNVASHLQ-----KYRMQRRH
    Arath_APRR2   (309) FVQAVEQLGVDQAIPSRILELMKVGTLTRHNVASHLQ-----KFRQHRKN
     Eucgr_PRR2   (327) FVQAVEQLGVDQAIPSCILDLMKVEGLTRHNVASHLQ-----KYRMHRRH
     Glyma_PRR2   (326) FVKAVEQLGIDQAIPSRILEIMKVEGLTRHNVASHLQ-----KYRIHKRQ
     Lotja_PRR2   (326) FVKAVEQLGIDHAIPSRILEIMKVEGLTRHNVASHLQ-----KYRIHKKQ
 Lyces_PRR2 II(327) FVKAVEKIGIDQAIPSRILELMKVEGLTRHNIASHLQ-----KFRMQRKQ
     Medtr_PRR2   (324) FVKAVEQLGIDQAIPSRILELMKVDGLTRHNVASHLQIFVEQKYRMHKRQ
   Poptr_PRR2 I(327) FVQAVEKLGVDQAIPSRILEVMKVEGLTRHNVASHLQ----QKYRMHRRH
  Poptr_PRR2 II(322) FVQVVEKLGVDQAIPSRVLELMKVESLTRHNVASHLQ-----KYRMRRRP
     Vitvi_PRR2   (329) FVQAVEQLGVDQAIPSRILELMKVEGLTRHNVASHLQ-----KYRMHRRH
      Consensus   (351) FVQAVEQLGVDQAIPSRILELMKVEGLTRHNVASHLQ     KYRMHRRQ
      IPR006447         XXXXXXXXXXXXXXXXXXXXXXXXXXXXXXXXXXXXXXXXXXXXXXXX
```

```
                         401                                               450
     Lyces_PRR2   (374) ILPKEV-ERRWPNPQP-IDSVQRSYYP----HKPIMTFPQYHSNHVAPGG
     Aqufo_PRR2   (382) IAPKDD-DRRWQHPR---DSIQRVYPQ-----KPIMAYPPYHPNHGFSTN
    Arath_APRR2   (354) ILPKDDHNHRWIQSRENHRPNQRNYNVFQQQHRPVMAYPVWGLPGVYPPG
     Eucgr_PRR2   (372) ILPKED-ERRWPHSR---DGVTRSYYP----HRPIMAYPTHHPGHPLPPG
     Glyma_PRR2   (371) SAPREE-DRKWHNQG---DAMQRNYYM----QRPIMAYPPYHSHHTLSPA
     Lotja_PRR2   (371) IMPGED-DRKCQNPR---DPMQRSYCL----QRPIMAYPPYHSNHTLPPA
  Lyces_PRR2II(372) ILPKED-ERRWPRPQP-RDPVQRTYYP----HKPVMAFPTHHSNHATTAG
     Medtr_PRR2   (374) IIHTDE-DRKWPNRR---DPMQRNYCM----QRPIMAYPPYHSNHTFPPA
   Poptr_PRR2 I(373) ILPKED-ERQWTQHR---DQVQRSYYP----HKPIMAYPPYHSNHALPPG
   Poptr_PRR2II(367) ILPKED-DRRWPHHR---EQVQRSYYP----YKPIMAYPPYHSNHDLPTN
     Vitvi_PRR2   (374) ILPKED-DRRWPHQR---DPMQRNYYP----QKPVMAFPPYHSSHTLPAA
      Consensus   (401) ILPKED DRRWPN R   D VQRSYYP    HKPIMAYPPYHSNH LPPG
```

**FIGURE 13 (continued)**

```
                         451                                              500
   Lyces_PRR2    (418)   QFYPAWVTP-ASYPNGLQVWGSPYYPGWKPAETWHWT-PRPELHADTWGS
   Aqufo_PRR2    (423)   QFYPVWGHP-SNHLQNIQMWGHPGFHTW-----------HPGMHADAWGR
  Arath_APRR2    (404)   AIPPLWPPPLQSIGQ---------------PPPWHWKPPYPTVSGNAWGC
   Eucgr_PRR2    (414)   AAYPVWGAPPSNHPAGMQMWGPPGYPPWPSPENWHWKTAYPAMQADVWGC
   Glyma_PRR2    (413)   PIYPMWGQP-GSQTAGVQIWGHPGYPIWHPTESWHWKPYP-GVHVDAWGC
   Lotja_PRR2    (413)   PMYPMWGQP-GSQTAGVQIWGNHGYPLWHPTESWHWKPYP-GMHVDAWGC
  Lyces_PRR2 II(416)     QFYPAWIPP-GGYPNGAHMWNSPYYHGWQPPETWHWN-PQPGLYADVWGC
   Medtr_PRR2    (416)   PAYPMWGQH-GSQTAGVPIWSPPGYPLWQPTESWHWKPYPPGVHVDAWGN
   Poptr_PRR2 I(415)     PVYPMWGAT-GSHTAGVHMWGPPGYSPWPPTESCHWK-PYPGMHADAWGC
  Poptr_PRR2 II(409)     PVYPMWGAT-GSHTASMHTWGTPSYLPWPPTEIWHRK-PYLGMHADAWGC
   Vitvi_PRR2    (416)   QLYPVWGQP-SSHPA--QMWSTPGYHTWQPAESWIWK-PYPGMNADAWGC
    Consensus    (451)    IYPMWG P GSH AGVQMWG PGY  W P ESWHWK PYPGMHADAWGC

                         501                                              550
   Lyces_PRR2    (466)   PIMSPS-LGSYPPYPQNAG-VYRPHGTHNRYS-MLEKSFDLHPADEVIDK
   Aqufo_PRR2    (461)   PILPLS-QGPYSNYPQNASGFQNVYAYEDNNN-VPKNSFDFNPAEEVIDK
  Arath_APRR2    (439)   PVGPPVTGSYITPSNTTAGGFQYPNGAETGFKIMP----ASQPDEEMLDQ
   Eucgr_PRR2    (464)   PVMPSSHHGPSFAFPQNAPVCHPPDGLENSFAGN---SFDYHPDEEVIDN
   Glyma_PRR2    (461)   PLVPPP-QAPCFPYNQNTPGLHNPKAVDYRFSMP-RSSFEHHPAEEVVDK
   Lotja_PRR2    (461)   PVLPSP-QASPFPYTHNVAGWHNAKAMDYRFSMP-QSSVENYPAEEVVDK
  Lyces_PRR2 II(464)     PVTPPS-LGSCTPYPQNASGFHRAEGMLNGYS-IIQKSVDLHPAEEVIDK
   Medtr_PRR2    (465)   PMLRPP-QTHCIPYTQNMAGMHNAKAVDY-----------SYPAEEVVDK
   Poptr_PRR2 I(463)     PVMPP--HNPYSTIPHYASGFQSAS-MVNNSCGMPQKLFDLQPAEEVIDK
  Poptr_PRR2 II(457)     PVMPPL-HSPFSSFPQNASGFQSAS-MVDNSCGMPQKPFDLQPAEEVINK
   Vitvi_PRR2    (462)   PVMTPT-HTPCSSFPQNPSGFDHNNGSGIYNTGIPQSPIDLYPAEEVIDR
    Consensus    (501)   PVMPP   AP SPYPQNASGFH   GMD  F  M   SFD HPAEEVIDK
C-terminal CD                                                     XXXXXXXX

                         551                                              600
   Lyces_PRR2    (513)   VVKEAITKPWLPLPLGLKAPSTESVLDELSRQGISTIPSQINDSRCRR--
   Aqufo_PRR2    (509)   VVREAINKPWLPLPLGLKSPSTEMVLSELHRQGICNIPPHNQC-------
  Arath_APRR2    (485)   VVKEAISKPWLPLPLGLKPPSAESVLAELTRQGISAVPSSSCLINGSHRL
   Eucgr_PRR2    (511)   IVKEAISKPWLPLPLGLKPPSTDCVLAELSRQGIPSIPPHADQLGPC---
   Glyma_PRR2    (509)   VVKEAMSQPWLPLPLGLKPPSMDSVLAELSKQGIP-SIPLGNKGSSTPKP
   Lotja_PRR2    (509)   VVKEAMSKPWLPLPLGLKPPSMDTVLSELSSQGISGIIPFSSTKGSIPR-
  Lyces_PRR2 II(512)     VVKEAINKPWLPLPLGLKPPSTESVLDALSKQGIPAVPPRHHRSHRPH--
   Medtr_PRR2    (503)   AVKEAINKPWLPLPIGLKPPSMDSVLDELSKQGIP--FSK-KSKGSRPC-
   Poptr_PRR2 I(510)     VVKEAINKPWLPLPLGLKPPSADSVLAELSRQGVSSIPPRINGSNSF---
  Poptr_PRR2 II(505)     VVKEVINKPWLPLPIGLKPPSTDSVLAELSRQGISSIPPLCS--NSF---
   Vitvi_PRR2    (511)   VVKEAISKPWMPLPLGLKPPATESVLAELSRQGISTIPPHINTPRPY---
    Consensus    (551)   VVKEAISKPWLPLPLGLKPPSTDSVLAELSRQGIS IPP
C-terminal CD            XXXXXXXXXXXXXXXXXXXXXXXXXXXXXXXXX

                          601         613
  Arath_APRR2    (535)    R------------
   Glyma_PRR2    (558)    RRDVRPTPPPGPT
```

**FIGURE 13 (continued)**

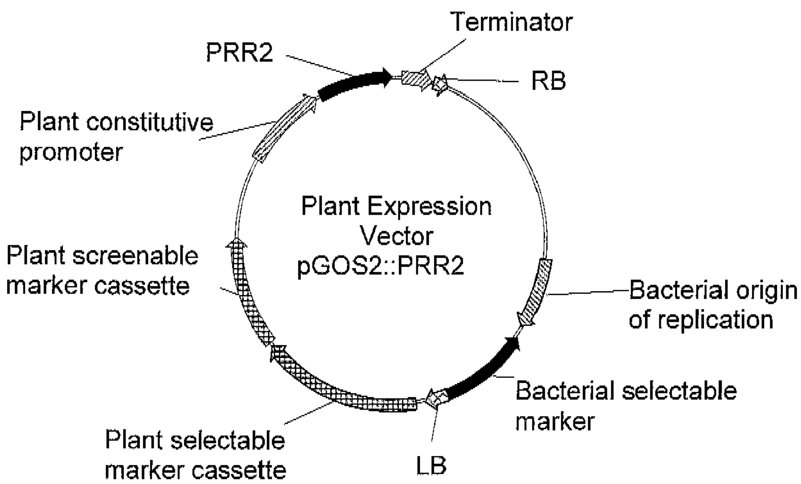
Terminator
PRR2
RB
Plant constitutive promoter
Plant Expression Vector pGOS2::PRR2
Plant screenable marker cassette
Bacterial origin of replication
Bacterial selectable marker
Plant selectable marker cassette
LB

FIGURE 14

Europäisches Patentamt
European Patent Office
Office européen des brevets

# EUROPEAN SEARCH REPORT

Application Number
EP 11 16 5505

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | US 2004/123343 A1 (LA ROSA THOMAS J [US] ET AL LA ROSA THOMAS [US] ET AL) 24 June 2004 (2004-06-24)<br>* claims 1-3; sequences 176069,73586 * | 1-5,7-16<br><br><br>6 | INV.<br>C07K14/415<br>C12N15/82 |
| X | US 2006/123505 A1 (KIKUCHI SHOSHI [JP] ET AL) 8 June 2006 (2006-06-08)<br>* claims 1-26; sequence 35395 * | 7-16 | |
| Y | WO 2004/065596 A2 (CROPDESIGN NV [BE]; HATZFELD YVES [FR]; INZE DIRK [BE]) 5 August 2004 (2004-08-05)<br>* page 2, line 5 - line 17 * | 6 | |
| T | DATABASE Rice [Online]<br>Rice Genome Annotation Project;<br>27 February 2012 (2012-02-27), XP002670413,<br>Database accession no. OS03g01020<br>* * | 1-16 | |
| A | V. NAPONELLI ET AL: "Phylogenomic and Functional Analysis of Pterin-4a-Carbinolamine Dehydratase Family (COG2154) Proteins in Plants and Microorganisms",<br>PLANT PHYSIOLOGY,<br>vol. 146, no. 4, 1 April 2008 (2008-04-01), pages 1515-1527, XP55020366,<br>ISSN: 0032-0889, DOI: 10.1104/pp.107.114090<br>* * | 1-16 | TECHNICAL FIELDS SEARCHED (IPC)<br>C12N |

-/--

The present search report has been drawn up for all claims

| Place of search | Date of completion of the search | Examiner |
|---|---|---|
| Munich | 2 March 2012 | Obel, Nicolai |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 16 5505

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | G Schwarz ET AL: "The Molybdenum Cofactor Biosynthetic Protein Cnx1 Complements Molybdate-Repairable Mutants, Transfers Molybdenum to the Metal Binding Pterin, and Is Associated with the Cytoskeleton", The Plant Cell, 1 January 2000 (2000-01-01), pages 2455-2471, XP55020372, Retrieved from the Internet: URL:http://welonsell.info/content/12/12/2455.full.pdf#page=1&view=FitH [retrieved on 2012-02-27] * * ----- | 1-16 | |
| | | | TECHNICAL FIELDS SEARCHED (IPC) |

1 The present search report has been drawn up for all claims

| Place of search | Date of completion of the search | Examiner |
|---|---|---|
| Munich | 2 March 2012 | Obel, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 16 5505

This annex lists the patent family members relating to the patent documents cited in the above-mentioned European search report. The members are as contained in the European Patent Office EDP file on
The European Patent Office is in no way liable for these particulars which are merely given for the purpose of information.

02-03-2012

| Patent document cited in search report | Publication date | Patent family member(s) | Publication date |
|---|---|---|---|
| US 2004123343 A1 | 24-06-2004 | NONE | |
| US 2006123505 A1 | 08-06-2006 | JP 2005185101 A | 14-07-2005 |
| | | US 2006123505 A1 | 08-06-2006 |
| WO 2004065596 A2 | 05-08-2004 | AT 350481 T | 15-01-2007 |
| | | DE 602004004070 T2 | 16-08-2007 |
| | | EP 1585820 A2 | 19-10-2005 |
| | | ES 2279339 T3 | 16-08-2007 |
| | | US 2006053507 A1 | 09-03-2006 |
| | | US 2008301833 A1 | 04-12-2008 |
| | | WO 2004065596 A2 | 05-08-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005058021 A, Lightner
- US 7193129 B
- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A
- US 5159135 A


### Non-patent literature cited in the description


- **TITTONELL et al.** *Agric Ecosys & Environ,* 2005, vol. 105, 213
- **FASOULA ; TOLLENAAR.** *Maydica,* 2005, vol. 50, 39
- **TER STEEGE et al.** *Plant Physiology,* 2005, vol. 139, 1078
- **HITTALMANI et al.** *Theoretical Applied Genetics,* 2003, vol. 107, 679
- **REBETZKE et al.** *Crop Science,* 2002, vol. 42, 739
- **GARDENER et al.** Physiology of Crop Plants. Iowa State University Press, 1985, 68-73
- **WANG et al.** *Planta,* 2003, vol. 218, 1-14
- **RIECHMANN et al.** *Science,* 2000, vol. 290, 2105-2109
- **BAILEY et al.** *The Plant Cell,* 2003, vol. 15, 2497-2501
- **BUCK ; ATCHLEY.** *J. Mol. Evol.,* 2003, vol. 56, 742-750
- **HEIM et al.** *Mol. Biol. Evol.,* 2003, vol. 20 (5), 735-747
- **FRIEDRICHSEN et al.** *Genetics,* 2002, vol. 162, 1445-1456
- **BUCK ; ATCHLEY.** *J Mol Evol.,* 2003, vol. 56 (6), 742-50
- **ZHENG ; ZHOU.** *FEBS Letters,* 2002, vol. 513, 124-128
- **LOYOLA ; ALMOUZNI.** *Trends Cell Biol.,* 2004, vol. 14, 279-281
- **CHUA et al.** *Genes & Development,* 2005, vol. 19, 2245-2254
- **DUQUE ; CHUA.** *Plant Journal,* 2003, vol. 35, 787-799
- **CRONK et al.** *Protein Sci.,* October 1996, vol. 5 (10), 1963-72
- **NAPONELLI et al.** *Plant Physiol.,* 2008, vol. 146 (4), 1515-1527
- **HWANG et al.** *Plant Phys,* 2002, vol. 129, 500-515
- **MAKINO et al.** *Plant Cell Physiol,* 2000, vol. 41, 791-803
- **IMAMURA et al.** *Plant Cell Physiol,* 1999, vol. 40, 733-742
- **STRAYER et al.** *Science,* 2000, vol. 289, 768-771
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25

- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **WU et al.** *Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38

- **TAKAIWA et al.** *Mol Gen Genet,* 1986, vol. 208, 15-22
- **AKAIWA et al.** *FEBS Letts.,* 1987, vol. 221, 43-47
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handboo. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353

- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **TERADA.** *Curr Opin Biotech,* 2004, vol. 15 (2), 132-8
- **MILLER et al.** *Nature Biotechnol.,* 2007, vol. 25, 778-785
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **DOMBRECHT et al.** *Plant Cell,* 2007, vol. 19, 2225-2245
- **KIM et al.** *Plant Mol Biol.,* 2007, vol. 64 (4), 453-66
- **OUWERKEK.** *Mol Gen Genet.,* 1999, vol. 261 (4-5), 635-43
- **HAUER et al.** *J. Biol. Chem.,* 1993, vol. 268, 4828-4831
- Current Protocols in Molecular Biology
- **RABBANI et al.** *Plant Physiol,* 2003, vol. 133, 1755-1767
- **FRINK et al.** *Proc Natl Acad Sci USA,* 1999, vol. 96 (4), 1175-1180
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet,* 1997, vol. 7, 22-28
- **DEAR ; COOK.** *Nucleic Acid Res.,* 1989, vol. 17, 6795-6807
- **MASON et al.** *Plant Phys,* 2004, vol. 135, 927-937
- **SAMBROOK.** Molecular Cloning: a laboratory manual. Cold Spring Harbor Laboratory Press, 2001
- **AUSUBEL et al.** Current Protocols in Molecular Biology. *Current Protocols,* 1994, vol. 1, 2
- **R.D.D. CROY.** Plant Molecular Biology Labfax. BIOS Scientific Publications Ltd (UK, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **EDGAR.** *Nucleic Acids Research,* 2004, vol. 32, 1792-1797
- **EDGAR.** *Nucleic Acids Research,* 2004, vol. 32 (5), 1792-97
- **HOWE et al.** *Bioinformatics,* 2002, vol. 18 (11), 1546-7
- **HUSON et al.** *BMC Bioinformatics,* 2007, vol. 8 (1), 460
- **BAILEY et al.** *Nucleic Acids Research,* 2006, vol. 34, W369-W373
- *BMC Bioinformatics.,* 2003, vol. 4, 29
- *BMC Bioinformatics,* 2003, vol. 4, 29
- **PARK ; KANEHISA.** *Bioinformatics,* 2003, vol. 19, 1656-1663
- **AUSUBEL et al.** Current Protocols in Molecular Biology. Current Protocols, 1994, vol. 1, 2
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158